# EUROPEAN PATENT APPLICATION

(11) **EP 4 671 250 A1**
(43) Date of publication of application: **31.12.2025**
(21) Application number: 24759760.2
(22) Date of filing: 23.02.2024
(51) Int. Cl.: C07D 495/04, A61K 31/381

(54) **ARYL TRICYCLIC STING AGONIST AND USE THEREOF**

(30) Priority: 24.02.2023 CN 202310196685; 11.08.2023 CN 202311016692; 17.11.2023 CN 202311536799; 08.02.2024 CN 202410178185
(71) Applicant: Shenzhen Genuine Biotechnology Co., Ltd., Shenzhen, Guangdong 518031 (CN)
(72) Inventor: LI, Pan, Shenzhen, Guangdong 518031 (CN); QIN, Zhiyong, Shenzhen, Guangdong 518031 (CN); YANG, Chengyu, Shenzhen, Guangdong 518031 (CN); WANG, Qilin, Shenzhen, Guangdong 518031 (CN); LYU, Yang, Shenzhen, Guangdong 518031 (CN); JIA, Limin, Shenzhen, Guangdong 518031 (CN); WANG, Yujie, Shenzhen, Guangdong 518031 (CN)
(74) Representative: Bayramoglu et al.
(86) International application number: PCT/CN2024/078291
(87) International publication number: WO 2024/175086

(57) **Abstract**

Disclosed herein is an aryl tricyclic STING agonist compound having the structure of formula (I), and a preparation method thereof.

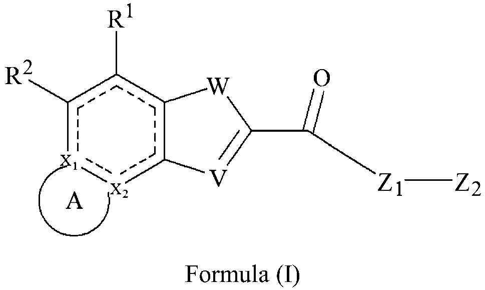

## Description

### TECHNICAL FIELD

The present disclosure is in the field of medicine and, in particular, relates to a STING agonist compound and a use thereof.

### BACKGROUND TECHNOLOGY

STING (Stimulator of Interferon Genes) is currently the latest and most promising immunotherapeutic target in the field of drug development for cancer immunotherapy. Stimulator of Interferon Genes is a transmembrane protein that typically forms a dimer at the junction of regions 152-173 and remains in a self-inhibitory state. When stimulated by certain ligands, its molecular conformation changes and the protein is activated, recruiting cytosolic TANK-binding kinase 1, mediating the phosphorylation of IRF3 by TBKl, leading to the formation of interferon-β and various other cytokines. The production of IFN β is a marker of STING activation. Innate immune signaling in the tumor microenvironment is a critical step in the activation of tumor-specific T cells and the infiltration of tumor-infiltrating lymphocytes. Among them, type I IFN plays a key role in the activation of tumor-activated T cells. As such, STING not only induces the expression of type I interferon genes and plays a significant role in innate immune signaling pathways; STING agonists can also activate immune-stimulatory cells such as dendritic cells, modify the tumor microenvironment, and induce the production of tumor-specific T cells. In murine experiments, DMXAA, a flavonoid vascular disrupting agent, induces the production of IFN-β and other natural cytokines by activating the murine STING protein, and effectively inhibits the growth of various solid tumors. However, in a clinical trial of human non-small cell lung cancer, the drug failed to show significant efficacy when used in combination with standard chemotherapy. Later experiments confirmed that DMXAA fails to activate the human STING protein, although the similarity between human and murine STING proteins is as high as 81%, with the former gene encoding 379 amino acids and the latter gene encoding 378 amino acids. Cyclic dinucleotides are the only class of STING agonists discovered to date that can directly activate both murine and human STING proteins. Direct injection of CDN into B16 melanoma, CT26 colorectal cancer, and 4T1 breast cancer tumors not only leads to significant tumor inhibition until complete regression, but also induces systemic persistent antigen-specific T cell immunity, resulting in the inhibition of tumor growth in other sites of the animal that were not injected with the drug. ML RR-S2 CDA induces changes in the microenvironment of various solid tumors, activating effective tumor-induced CD8⁺ T cells and providing long-lasting therapeutic effects. In recent years, a large number of research reports have shown that the STING pathway can effectively initiate the body's innate immune system and is one of the few signaling pathways that have been verified through multiple studies to induce the production of cytokines such as interferons. This pathway is crucial in innate immunity. Sufficient infiltration of lymphocytes into tumor tissues is the key to the success of immunotherapy. The activation of this target pathway also promotes the infiltration and response of effector T cells in the tumor microenvironment, and thus the target has gradually become an important target for anti-tumor therapy, especially in immunotherapy research. In multiple mouse inoculation models, it has been effective against various refractory and metastatic solid tumors. Not only do the directly injected tumors regress completely, but tumor growth in other sites is also significantly inhibited, and even the occurrence of tumors can be prevented.

Compounds that have been shown to bind to STING and act as agonists induce type 1 interferons and other cytokines when incubated with human PBMCs. Compounds that induce human interferons can be used to treat various conditions, e.g., allergic diseases and other inflammatory conditions, such as allergic rhinitis and asthma, infectious diseases, neurodegenerative diseases, precancerous syndromes, and cancer. They can also be used as immunological compositions or vaccine adjuvants. The activation of STING may be a potential therapeutic approach for diseases associated with the type 1 IFN pathway, including inflammatory, allergic, and autoimmune diseases, infectious diseases, cancer, precancerous syndromes, or as immunological compositions or vaccine adjuvants.

### CONTENT OF THE INVENTION

The present disclosure provides a compound having the structure of formula I or a pharmaceutically acceptable salt thereof: A compound having the structure of formula (I) or a pharmaceutically acceptable salt thereof: 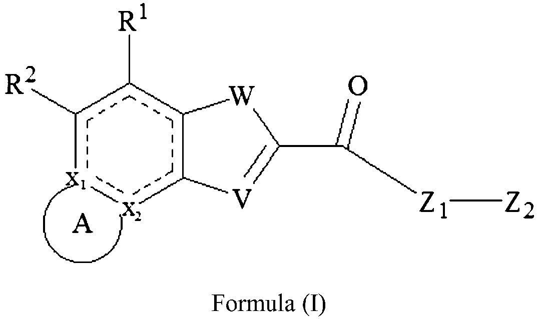 wherein R¹ represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein R² represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
or, R¹ and R², together with the atom to which they are attached, form a 5-8-membered ring, which may optionally contain 0, 1, or 2 heteroatoms selected from O, N, and S;
wherein R³ represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein Z₁ represents -C₁-C₆ alkylene-, -C₂-C₆ alkenylene-, -(C₀-C₆ alkylene)-(C₃-C₆ cycloalkyl)-(C₀-C₆ alkylene)-, -(C₀-C₆ alkylene)-O-(C₀-C₆ alkylene)-, -(C₀-C₆ alkylene)-(3-6-membered heterocycloalkyl)-(C₀-C₆ alkylene)-, -(C₀-C₆ alkylene)-NR^{a}-(C₀-C₆ alkylene)-, -(C₀-C₆ alkylene)-C(O)NR^{a}-(C₀-C₆ alkylene)-, -(C₀-C₆ alkylene)-NR^{a}C(O)-(C₀-C₆ alkylene)-, -(C₀-C₆ alkylene)-S(O)₂NR^{a}-(C₀-C₆ alkylene)-; and any hydrogen atom in the said Z₁ may be substituted with 0-3 substituents selected from halogen, cyano, -OR^{a}, -SR^{a}, -NR^{a}R^{b}, C₁-C₆ alkyl, C₁-C₆ haloalkyl, C₃-C₁₀ cycloalkyl; or any CH₂ in Z₁ is replaced by 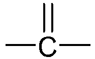 or 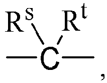 wherein R^{s} and R^{t}, together with the C atom to which they are attached, form a 3-6-membered ring;
wherein W represents S or Se;
wherein V represents CR³ or N;
wherein X₁ and X₂ represent C or N;
wherein Z₂ represents C(O)OR^{L}, C(O)SR^{L}, C(S)OR^{L}, S(O)₂R^{L};
wherein R^{L} represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein ring A represents a substituted or unsubstituted saturated or unsaturated 5-10-membered ring or 5-10-membered heterocyclic ring;
wherein the dotted line represents a single bond or a double bond;
the "substituted or unsubstituted" as defined in the above substituents means optionally substituted with 0, 1, 2, or 3 substituents selected from deuterium, halogen, cyano, C₁-C₆ alkyl, C₂-C₆ alkenyl, C₂-C₆ alkynyl, C₁-C₆ haloalkyl, C₁-C₆ alkoxy, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein R^{a} and R^{b} each independently represent hydrogen, deuterium, C₁-C₆ alkyl, C₁-C₆ haloalkyl, hydroxy-substituted C₁-C₆ alkyl, C₃-C₁₀ cycloalkyl; or R^{a} and R^{b}, together with the atom to which they are attached, form a 3-6-membered ring, which may optionally contain 0, 1, or 2 heteroatoms selected from O, N, and S.

Additionally, the present disclosure discloses a compound or a pharmaceutically acceptable salt thereof, having the structure of formula I-1 below: 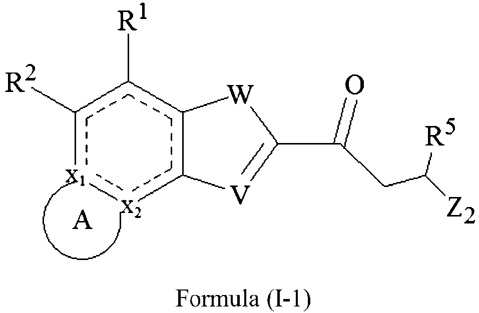 wherein R¹, R², W, V, X₁, X₂, Z₂, and ring A are as defined in formula (I), wherein R⁵ represents C₁-C₆ alkyl, preferably methyl and ethyl; wherein the dotted line represents a single bond or a double bond.

Additionally, the present disclosure provides a compound or a pharmaceutically acceptable salt thereof, having the structure of formula II below: 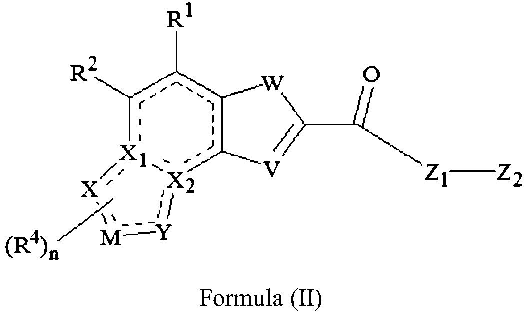 wherein R¹ represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein R² represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
or, R¹ and R², together with the atom to which they are attached, form a 5-8-membered ring, which may optionally contain 0, 1, or 2 heteroatoms selected from O, N, and S;
wherein R³ represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein R⁴ represents hydrogen, deuterium, halogen, cyano, C₁-C₆ alkyl, C₂-C₆ alkenyl, C₂-C₆ alkynyl, C₁-C₆ haloalkyl, C₁-C₆ alkoxy, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein Z₁ represents -C₁-C₆ alkylene-, -C₂-C₆ alkenylene-, -(C₀-C₆ alkylene)-(C₃-C₆ cycloalkyl)-(C₀-C₆ alkylene)-, -(C₀-C₆ alkylene)-O-(C₀-C₆ alkylene)-, -(C₀-C₆ alkylene)-(3-6-membered heterocycloalkyl)-(C₀-C₆ alkylene)-, -(C₀-C₆ alkylene)-NR^{a}-(C₀-C₆ alkylene)-, -(C₀-C₆ alkylene)-C(O)NR^{a}-(C₀-C₆ alkylene)-, -(C₀-C₆ alkylene)-NR^{a}C(O)-(C₀-C₆ alkylene)-, -(C₀-C₆ alkylene)-S(O)₂NR^{a}-(C₀-C₆ alkylene)-; and any hydrogen atom in the said Z₁ may be substituted with 0-3 substituents selected from halogen, cyano, -OR^{a}, -SR^{a}, -NR^{a}R^{b}, C₁-C₆ alkyl, C₁-C₆ haloalkyl, C₃-C₁₀ cycloalkyl; or any CH₂ in Z₁ is replaced by 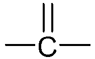 or 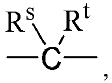 wherein R^{s} and R^{t}, together with the C atom to which they are attached, form a 3-6-membered ring;
wherein Z₂ represents C(O)OR^{L}, C(O)SR^{L}, C(S)OR^{L}, S(O)₂R^{L};
wherein R^{L} represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein W represents S or Se;
wherein V represents CR³ or N;
wherein X, Y, and M each independently represent CR^{X}, O, S, or NR⁵;
wherein X₁ and X₂ represent C or N, provided that at least one is C;
wherein R^{X} represents hydrogen, deuterium, C₁-C₆ alkyl, C₂-C₆ alkenyl, C₂-C₆ alkynyl, C₁-C₆ haloalkyl, hydroxy C₁-C₆ alkyl, halogen, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein R⁵ represents hydrogen, deuterium, C₁-C₆ alkyl, C₂-C₆ alkenyl, C₂-C₆ alkynyl, C₁-C₆ haloalkyl, hydroxy C₁-C₆ alkyl, halogen, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein n is 0, 1, 2;
the "substituted or unsubstituted" as defined in the above substituents means optionally substituted with 0, 1, 2, or 3 substituents selected from halogen, cyano, C₁-C₆ alkyl, C₁-C₆ haloalkyl, C₁-C₆ alkoxy, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein R^{a} and R^{b} each independently represent hydrogen, deuterium, C₁-C₆ alkyl, C₁-C₆ haloalkyl, hydroxy-substituted C₁-C₆ alkyl, C₃-C₁₀ cycloalkyl; or R^{a} and R^{b}, together with the atom to which they are attached, form a 3-6-membered ring, which may optionally contain 0, 1, or 2 heteroatoms selected from O, N, and S;
wherein the dotted line represents a single bond or a double bond.

Additionally, the present disclosure provides a compound or a pharmaceutically acceptable salt thereof, having the structure of formula II-2 below: 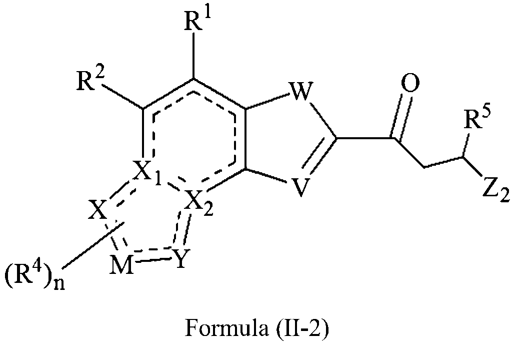 wherein R¹, R², X₁, X₂, X, Y, M, W, V, R₄, n, Z₂ are as defined above, wherein R⁵ represents C₁-C₆ alkyl, preferably methyl and ethyl; wherein the dotted line represents a single bond or a double bond.

In preferred embodiments of the above formula (I), formula (II), and formula (II-2) of the present disclosure, wherein R¹ represents hydrogen, deuterium, halogen, C₁-C₆ alkyl, C₁-C₆ haloalkyl, hydroxy C₁-C₆ alkyl, mercapto C₁-C₆ alkyl, C₂-C₆ alkenyl, C₂-C₆ haloalkenyl, hydroxy C₂-C₆ alkenyl, C₂-C₆ alkenyl, C₂-C₆ haloalkenyl, hydroxy C₂-C₆ alkenyl, mercapto C₂-C₆ alkenyl, C₂-C₆ alkynyl, C₂-C₆ haloalkynyl, hydroxy C₂-C₆ alkynyl, mercapto C₂-C₆ alkynyl, C₃-C₆ saturated or unsaturated cycloalkyl, 3-6-membered saturated or unsaturated heterocycloalkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

In preferred embodiments of the above formula (I), formula (II), and formula (II-2) of the present disclosure, wherein R¹ represents hydrogen, halogen, C₁-C₆ alkyl, C₁-C₆ haloalkyl, hydroxy C₁-C₆ alkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

In preferred embodiments of the above formula (I), formula (II), and formula (II-2) of the present disclosure, wherein R² represents hydrogen, deuterium, halogen, C₁-C₆ alkyl, C₁-C₆ haloalkyl, hydroxy C₁-C₆ alkyl, mercapto C₁-C₆ alkyl, C₂-C₆ alkenyl, C₂-C₆ haloalkenyl, hydroxy C₂-C₆ alkenyl, C₂-C₆ alkenyl, C₂-C₆ haloalkenyl, hydroxy C₂-C₆ alkenyl, mercapto C₂-C₆ alkenyl, C₂-C₆ alkynyl, C₂-C₆ haloalkynyl, hydroxy C₂-C₆ alkynyl, mercapto C₂-C₆ alkynyl, C₃-C₆ saturated or unsaturated cycloalkyl, 3-6-membered saturated or unsaturated heterocycloalkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

In preferred embodiments of the above formula (I), formula (II), and formula (II-2) of the present disclosure, wherein R² represents hydrogen, deuterium, halogen, C₁-C₆ alkyl, C₁-C₆ haloalkyl, hydroxy C₁-C₆ alkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

In preferred embodiments of the above formula (I), formula (II), and formula (II-2) of the present disclosure, wherein R¹ and R², together with the atom to which they are attached, form a 5-6-membered ring, which may optionally contain 0, 1, or 2 heteroatoms selected from O, N, and S.

In preferred embodiments of the above formula (I), formula (II), and formula (II-2) of the present disclosure, wherein R³ represents hydrogen, deuterium, halogen, -OR^{a}, cyano, C₁-C₆ alkyl, hydroxy-substituted C₁-C₆ alkyl, C₁-C₆ haloalkyl, C₃-C₆ cycloalkyl.

In preferred embodiments of the above formula (I), formula (II), and formula (II-2) of the present disclosure, wherein X, Y, and M are each independently selected from O, S, NR⁵, or CR^{X}.

In preferred embodiments of the above formula (I), formula (II), and formula (II-2) of the present disclosure, wherein W represents S.

In preferred embodiments of the above formula (I), formula (II), and formula (II-2) of the present disclosure, wherein R⁴ represents hydrogen, deuterium, halogen, -OR^{a}, cyano, C₁-C₆ alkyl, hydroxy-substituted C₁-C₆ alkyl, C₁-C₆ haloalkyl, C₃-C₆ cycloalkyl.

In preferred embodiments of the above formula (I), formula (II), and formula (II-2) of the present disclosure, wherein Z₁ represents -C₁-C₆ alkylene-, -C₂-C₆ alkenylene-, -(C₀-C₆ alkylene)-(C₃-C₆ cycloalkyl)-(C₀-C₆ alkylene)-, -(C₀-C₆ alkylene)-O-(C₀-C₆ alkylene)-; and any hydrogen atom in the said Z₁ may be substituted with 0-3 substituents selected from halogen, cyano, -OR^{a}, -SR^{a}, -NR^{a}R^{b}, C₁-C₆ alkyl, C₁-C₆ haloalkyl, C₃-C₁₀ cycloalkyl; or any CH₂ in Z₁ is replaced by 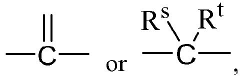 wherein R^{s} and R^{t}, together with the C atom to which they are attached, form a 3-6-membered ring.

In preferred embodiments of the above formula (I), formula (II), and formula (II-2) of the present disclosure, wherein Z₁ represents -C₁-C₆ alkylene- or -(C₀-C₆ alkylene)-(C₃-C₆ cycloalkyl)-(C₀-C₆ alkylene)-; and any hydrogen atom in the said Z₁ may be substituted with 0-3 substituents selected from halogen, cyano, -OR^{a}, -SR^{a}, -NR^{a}R^{b}, C₁-C₆ alkyl, C₁-C₆ haloalkyl, C₃-C₁₀ cycloalkyl; or any CH₂ in Z₁ is replaced by 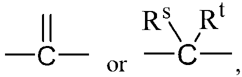 wherein R^{s} and R^{t}, together with the C atom to which they are attached, form a 3-6-membered ring.

In preferred embodiments of the above formula (I), formula (II), and formula (II-2) of the present disclosure, wherein Z₂ represents C(O)OR^{L}, wherein R^{L} represents hydrogen or C₁-C₆ alkyl, preferably hydrogen.

In preferred embodiments of the above formula (I), formula (II), and formula (II-2) of the present disclosure, wherein R^{a} and R^{b} each independently represent hydrogen, C₁-C₆ alkyl, C₁-C₆ haloalkyl, C₃-C₆ cycloalkyl.

Additionally, the present disclosure provides a compound or a pharmaceutically acceptable salt thereof, having the structure of formula II-3 below: 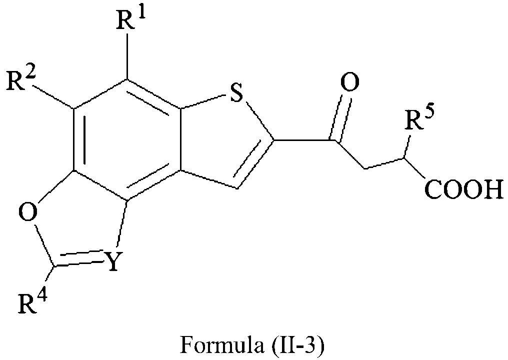
wherein Y represents CH or N;
wherein R¹ and R² each independently represent hydrogen, deuterium, halogen, C₁-C₆ alkyl, C₁-C₆ haloalkyl, hydroxy C₁-C₆ alkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}; or R¹ and R², together with the atom to which they are attached, form a 5-6-membered saturated or unsaturated ring, which may optionally contain 0, 1, or 2 heteroatoms selected from O, S, and N;
wherein Y represents CH or N;
wherein R⁴ represents hydrogen, deuterium, halogen, C₁-C₆ alkyl;
wherein R⁵ represents C₁-C₆ alkyl, preferably methyl and ethyl;
wherein R^{a} and R^{b} each independently represent hydrogen or C₁-C₆ alkyl.

In a preferred embodiment of the above formula (II-3) of the present disclosure, wherein Y represents CH.

In a preferred embodiment of the above formula (II-3) of the present disclosure, wherein Y represents N.

In a preferred embodiment of the above formula (II-3) of the present disclosure, wherein R¹ represents hydrogen, deuterium, halogen, C₁-C₆ alkyl.

In a preferred embodiment of the above formula (II-3) of the present disclosure, wherein R² represents hydrogen, deuterium, halogen, C₁-C₆ alkyl, C₁-C₆ haloalkyl, hydroxy C₁-C₆ alkyl, cyano, -OR^{a};
wherein R^{a} represents hydrogen or C₁-C₆ alkyl.

In a preferred embodiment of the above formula (II-3) of the present disclosure, wherein R² represents hydrogen, halogen, C₁-C₆ haloalkyl, C₁-C₆ alkoxy.

In a preferred embodiment of the above formula (II-3) of the present disclosure, wherein R⁴ represents hydrogen or C₁-C₆ alkyl.

In a preferred embodiment of the above formula (II-3) of the present disclosure, wherein R⁵ represents methyl.

Additionally, the present disclosure provides a compound or a pharmaceutically acceptable salt thereof, having the structure of formula III below: 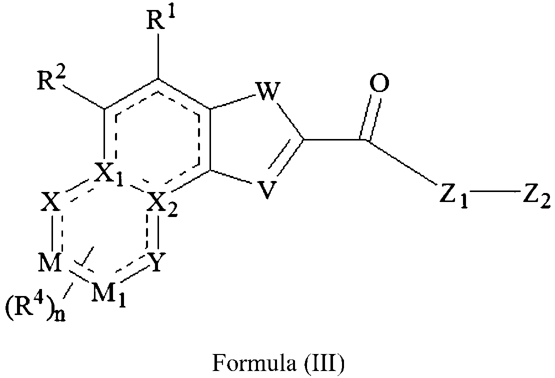
wherein R¹ represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein R² represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
or, R¹ and R², together with the atom to which they are attached, form a 5-8-membered ring, which may optionally contain 0, 1, or 2 heteroatoms selected from O, N, and S;
wherein R³ represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein R⁴ represents hydrogen, deuterium, halogen, cyano, C₁-C₆ alkyl, C₂-C₆ alkenyl, C₂-C₆ alkynyl, C₁-C₆ haloalkyl, C₁-C₆ alkoxy, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein Z₁ represents -C₁-C₆ alkylene-, -C₂-C₆ alkenylene-, -(C₀-C₆ alkylene)-(C₃-C₆ cycloalkyl)-(C₀-C₆ alkylene)-, -(C₀-C₆ alkylene)-O-(C₀-C₆ alkylene)-, -(C₀-C₆ alkylene)-(3-6-membered heterocycloalkyl)-(C₀-C₆ alkylene)-, -(C₀-C₆ alkylene)-NR^{a}-(C₀-C₆ alkylene)-, -(C₀-C₆ alkylene)-C(O)NR^{a}-(C₀-C₆ alkylene)-, -(C₀-C₆ alkylene)-NR^{a}C(O)-(C₀-C₆ alkylene)-, -(C₀-C₆ alkylene)-S(O)₂NR^{a}-(C₀-C₆ alkylene)-; and any hydrogen atom in the said Z₁ may be substituted with 0-3 substituents selected from halogen, cyano, -OR^{a}, -SR^{a}, -NR^{a}R^{b}, C₁-C₆ alkyl, C₁-C₆ haloalkyl, C₃-C₁₀ cycloalkyl; or any CH₂ in Z₁ is replaced by 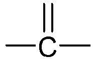 or 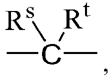 wherein R^{s} and R^{t}, together with the C atom to which they are attached, form a 3-6-membered ring;
wherein Z₂ represents C(O)OR^{L}, C(O)SR^{L}, C(S)OR^{L}, S(O)₂R^{L};
wherein R^{L} represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein W represents S or Se;
wherein V represents CR³ or N;
wherein X, Y, M, and M₁ each independently represent CR^{X}, O, S, or NR⁵;
wherein X₁ and X₂ represent C or N, provided that at least one is C;
wherein R^{X} represents hydrogen, deuterium, C₁-C₆ alkyl, C₂-C₆ alkenyl, C₂-C₆ alkynyl, C₁-C₆ haloalkyl, hydroxy C₁-C₆ alkyl, halogen, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein R⁵ represents hydrogen, deuterium, C₁-C₆ alkyl, C₂-C₆ alkenyl, C₂-C₆ alkynyl, C₁-C₆ haloalkyl, hydroxy C₁-C₆ alkyl, halogen, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein n is 0, 1, 2;
the "substituted or unsubstituted" as defined in the above substituents means optionally substituted with 0, 1, 2, or 3 substituents selected from deuterium, halogen, cyano, C₁-C₆ alkyl, C₁-C₆ haloalkyl, C₁-C₆ alkoxy, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein R^{a} and R^{b} each independently represent hydrogen, C₁-C₆ alkyl, C₁-C₆ haloalkyl, hydroxy-substituted C₁-C₆ alkyl, C₃-C₁₀ cycloalkyl; or R^{a} and R^{b}, together with the atom to which they are attached, form a 3-6-membered ring, which may optionally contain 0, 1, or 2 heteroatoms selected from O, N, and S;
wherein the dotted line represents a single bond or a double bond.

Additionally, the present disclosure provides a compound or a pharmaceutically acceptable salt thereof, having the structure of formula III-2 below: 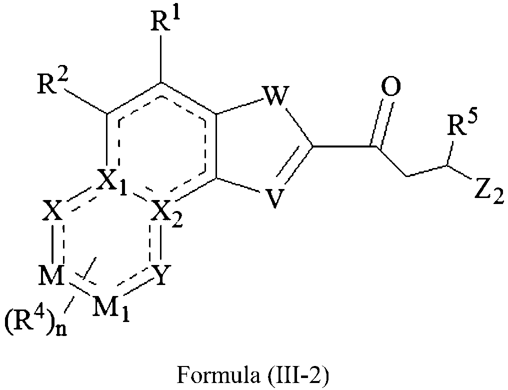 wherein R¹, R², X₁, X₂, R⁴, R⁵, X, Y, M, M₁, W, V, Z₂, and n are as defined in formula (III), wherein the dotted line represents a single bond or a double bond.

In a preferred embodiment of the above formula (III) or formula (III-2) of the present disclosure, wherein R¹ represents hydrogen, halogen, C₁-C₆ alkyl, C₁-C₆ haloalkyl, hydroxy C₁-C₆ alkyl, mercapto C₁-C₆ alkyl, C₂-C₆ alkenyl, C₂-C₆ haloalkenyl, hydroxy C₂-C₆ alkenyl, C₂-C₆ alkenyl, C₂-C₆ haloalkenyl, hydroxy C₂-C₆ alkenyl, mercapto C₂-C₆ alkenyl, C₂-C₆ alkynyl, C₂-C₆ haloalkynyl, hydroxy C₂-C₆ alkynyl, mercapto C₂-C₆ alkynyl, C₃-C₆ saturated or unsaturated cycloalkyl, 3-6-membered saturated or unsaturated heterocycloalkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

In a preferred embodiment of the above formula (III) or formula (III-2) of the present disclosure, wherein R¹ represents hydrogen, halogen, C₁-C₆ alkyl, C₁-C₆ haloalkyl, hydroxy C₁-C₆ alkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

In a preferred embodiment of the above formula (III) or formula (III-2) of the present disclosure, wherein R² represents hydrogen, deuterium, halogen, C₁-C₆ alkyl, C₁-C₆ haloalkyl, hydroxy C₁-C₆ alkyl, mercapto C₁-C₆ alkyl, C₂-C₆ alkenyl, C₂-C₆ haloalkenyl, hydroxy C₂-C₆ alkenyl, C₂-C₆ alkenyl, C₂-C₆ haloalkenyl, hydroxy C₂-C₆ alkenyl, mercapto C₂-C₆ alkenyl, C₂-C₆ alkynyl, C₂-C₆ haloalkynyl, hydroxy C₂-C₆ alkynyl, mercapto C₂-C₆ alkynyl, C₃-C₆ saturated or unsaturated cycloalkyl, 3-6-membered saturated or unsaturated heterocycloalkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

In a preferred embodiment of the above formula (III) or formula (III-2) of the present disclosure, wherein R² represents hydrogen, deuterium, halogen, C₁-C₆ alkyl, C₁-C₆ haloalkyl, hydroxy C₁-C₆ alkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

In a preferred embodiment of the above formula (III) or formula (III-2) of the present disclosure, wherein R¹ and R², together with the atom to which they are attached, form a 5-6-membered ring, which may optionally contain 0, 1, or 2 heteroatoms selected from O, N, and S.

In a preferred embodiment of the above formula (III) or formula (III-2) of the present disclosure, wherein R³ represents hydrogen, halogen, -OR^{a}, cyano, C₁-C₆ alkyl, hydroxy-substituted C₁-C₆ alkyl, C₁-C₆ haloalkyl, C₃-C₆ cycloalkyl.

In a preferred embodiment of the above formula (III) or formula (III-2) of the present disclosure, wherein X, Y, M, and M₁ are each independently selected from O, S, NR⁵, or CR^{X}.

In a preferred embodiment of the above formula (III) or formula (III-2) of the present disclosure, wherein R⁴ represents hydrogen, halogen, -OR^{a}, cyano, C₁-C₆ alkyl, hydroxy-substituted C₁-C₆ alkyl, C₁-C₆ haloalkyl, C₃-C₆ cycloalkyl.

In a preferred embodiment of the above formula (III) or formula (III-2) of the present disclosure, wherein Z₁ represents -C₁-C₆ alkylene-, -C₂-C₆ alkenylene-, -(C₀-C₆ alkylene)-(C₃-C₆ cycloalkyl)-(C₀-C₆ alkylene)-, -(C₀-C₆ alkylene)-O-(C₀-C₆ alkylene)-; and any hydrogen atom in the said Z₁ may be substituted with 0-3 substituents selected from halogen, cyano, -OR^{a}, -SR^{a}, -NR^{a}R^{b}, C₁-C₆ alkyl, C₁-C₆ haloalkyl, C₃-C₁₀ cycloalkyl; or any CH₂ in Z₁ is replaced by 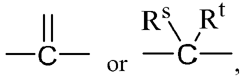 wherein R^{s} and R^{t}, together with the C atom to which they are attached, form a 3-6-membered ring.

In a preferred embodiment of the above formula (III) or formula (III-2) of the present disclosure, wherein Z₁ represents -C₁-C₆ alkylene- or -(C₀-C₆ alkylene)-(C₃-C₆ cycloalkyl)-(C₀-C₆ alkylene)-; and any hydrogen atom in the said Z₁ may be substituted with 0-3 substituents selected from halogen, cyano, -OR^{a}, -SR^{a}, -NR^{a}R^{b}, C₁-C₆ alkyl, C₁-C₆ haloalkyl, C₃-C₁₀ cycloalkyl; or any CH₂ in Z₁ is replaced by 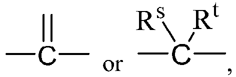 wherein R^{s} and R^{t}, together with the C atom to which they are attached, form a 3-6-membered ring.

In a preferred embodiment of the above formula (III) or formula (III-2) of the present disclosure, wherein Z₂ represents C(O)OR^{L}, wherein R^{L} represents hydrogen or C₁-C₆ alkyl, preferably hydrogen.

In a preferred embodiment of the above formula (III) or formula (III-2) of the present disclosure, wherein R^{a} and R^{b} each independently represent hydrogen, C₁-C₆ alkyl, C₁-C₆ haloalkyl, C₃-C₆ cycloalkyl.

In a preferred embodiment of the present disclosure, the compound of the present disclosure has the following structure:

| Compound No. | Compound structure | Compound No. | Compound structure |
|---|---|---|---|
| 1 | 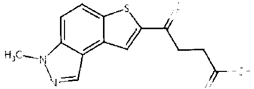 | 2 | 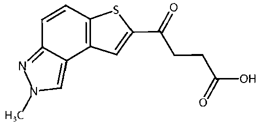 |
| 3 | 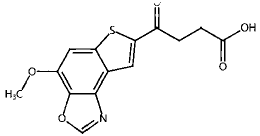 | 4 | 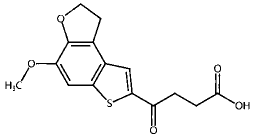 |
| 5 | 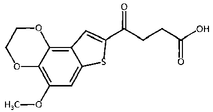 | 6 | 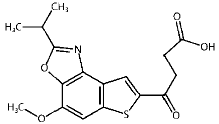 |
| 7 | 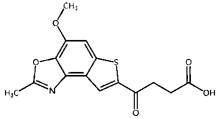 | 8 | 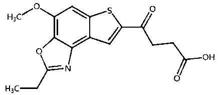 |
| 9 | 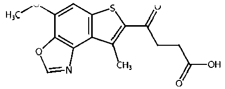 | 10 | 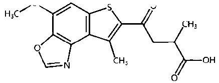 |
| 11 | 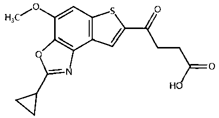 | 12 | 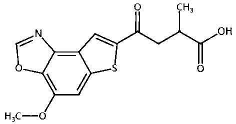 |
| 13 | 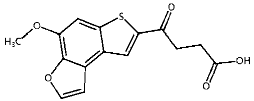 | 14 | 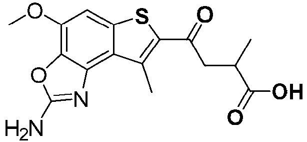 |
| 15 | 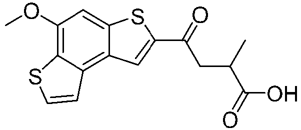 | 16 | 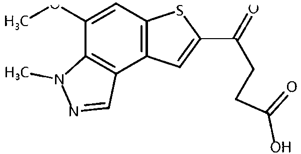 |
| 17 | 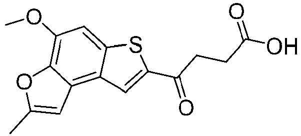 | 18 | 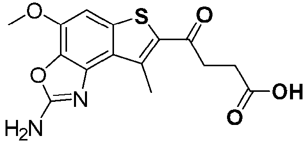 |
| 19 | 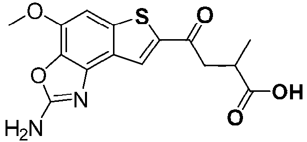 | 20 | 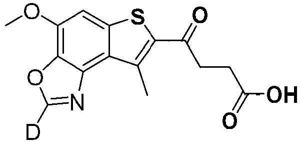 |
| 21 | 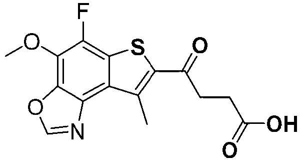 | 22 | 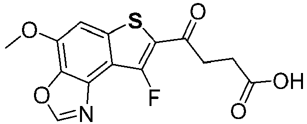 |
| 23 | 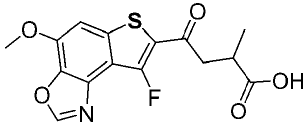 | 24 | 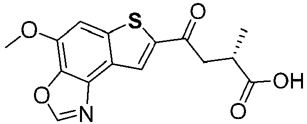 |
| 25 | 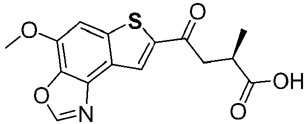 | 26 | 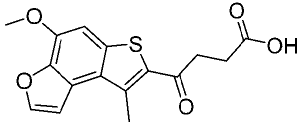 |
| 27 | 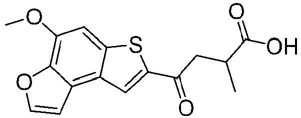 | 28 | 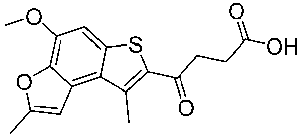 |
| 29 | 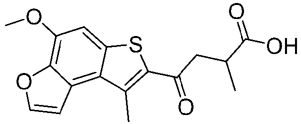 | 30 | 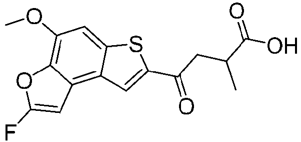 |
| 31 | 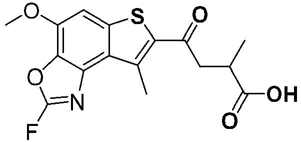 | 32 | 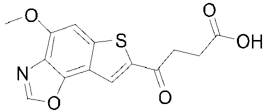 |
| 33 | 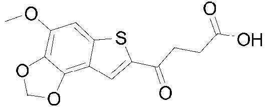 | 34 | 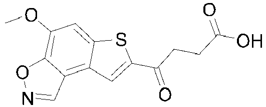 |
| 35 | 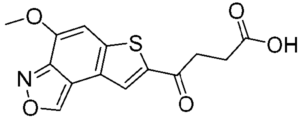 | 36 | 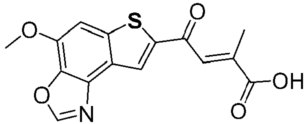 |
| 37 | 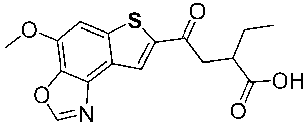 | 38 | 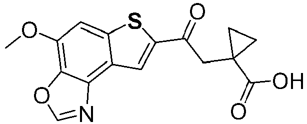 |
| 39 | 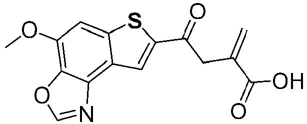 | 40 | 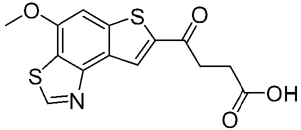 |
| 41 | 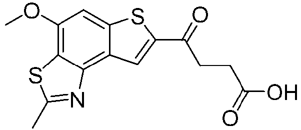 | 42 | 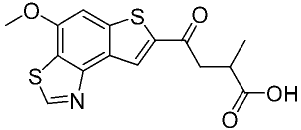 |
| 43 | 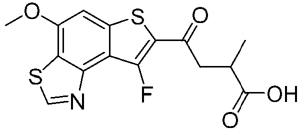 | 44 | 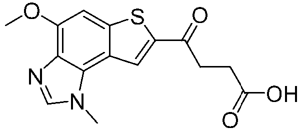 |
| 45 | 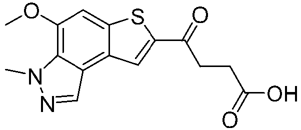 | 46 | 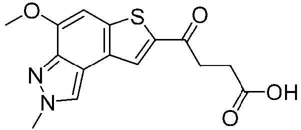 |
| 47 | 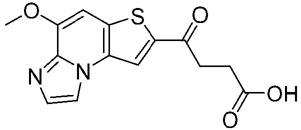 | 48 | 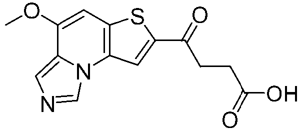 |
| 49 | 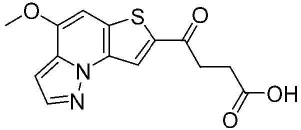 | 50 | 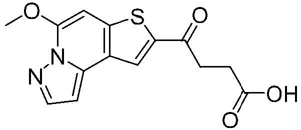 |
| 51 | 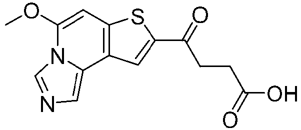 | 52 | 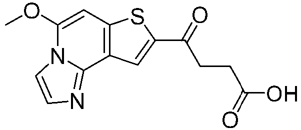 |
| 53 | 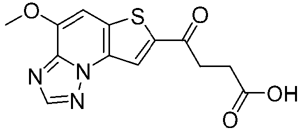 | 54 | 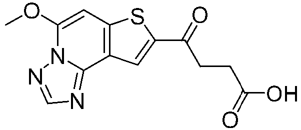 |
| 55 | 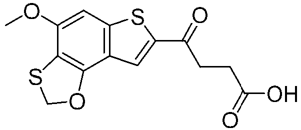 | 56 | 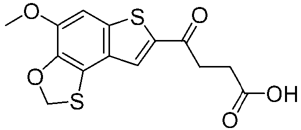 |
| 57 | 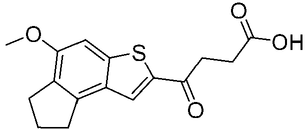 | 58 | 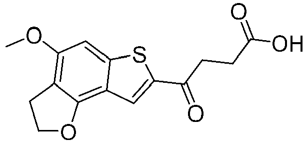 |
| 59 | 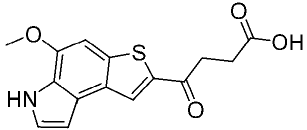 | 60 | 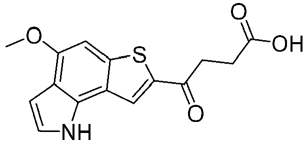 |
| 61 | 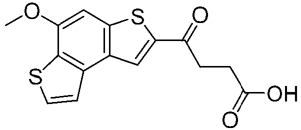 | 62 | 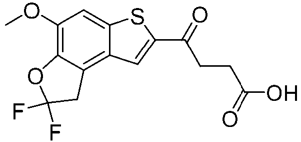 |
| 63 | 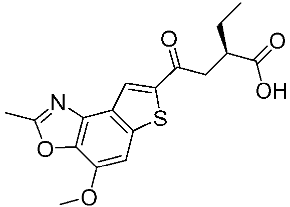 | 64 | 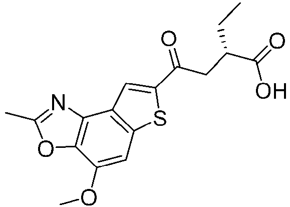 |
| 65 | 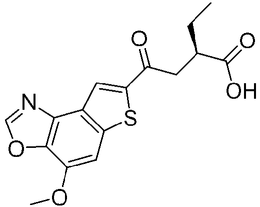 | 66 | 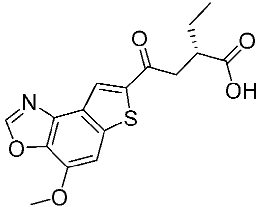 |
| 67 | 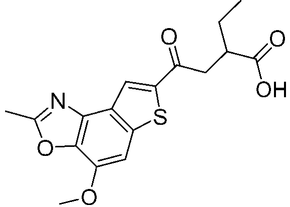 | 68 | 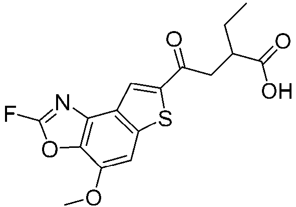 |
| 69 | 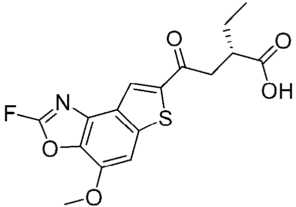 | 70 | 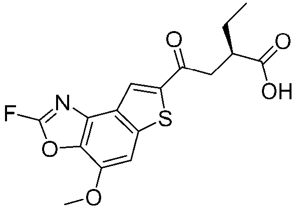 |
| 71 | 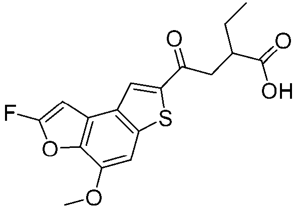 | 72 | 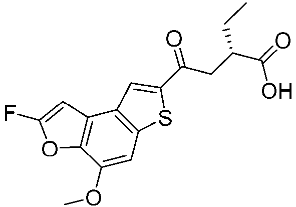 |
| 73 | 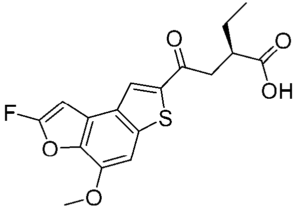 | 74 | 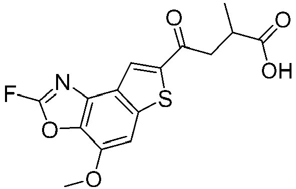 |
| 75 | 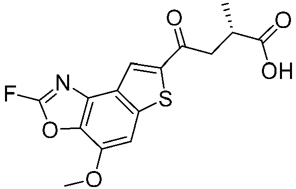 | 76 | 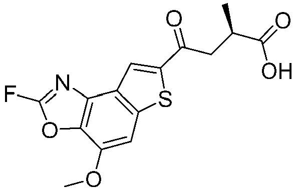 |
| 77 | 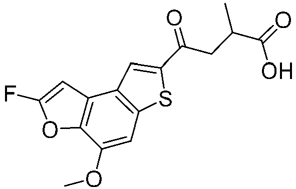 | 78 | 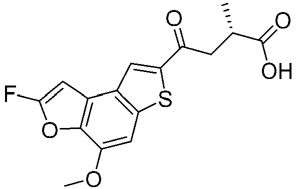 |
| 79 | 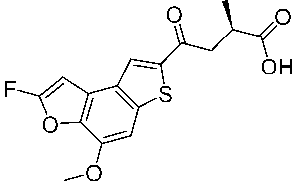 | 80 | 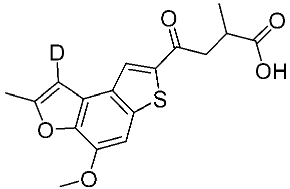 |
| 81 | 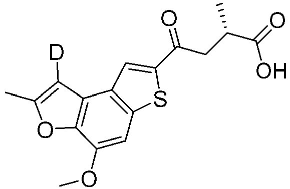 | 82 | 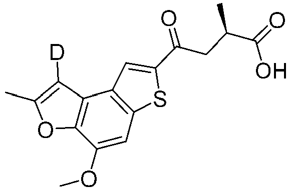 |
| 83 | 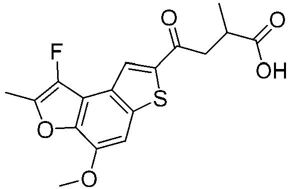 | 84 | 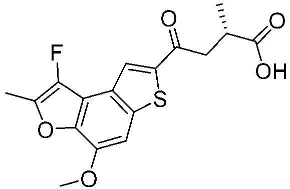 |
| 85 | 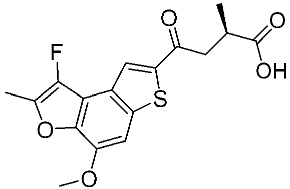 | 86 | 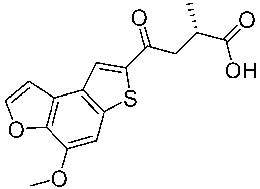 |
| 87 | 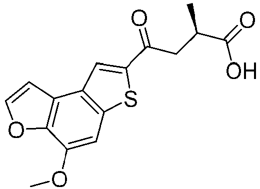 | 88 | 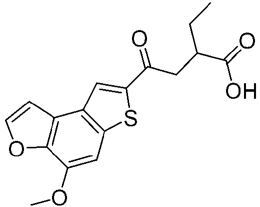 |
| 89 | 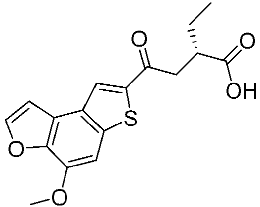 | 90 | 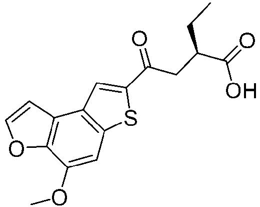 |
| 91 | 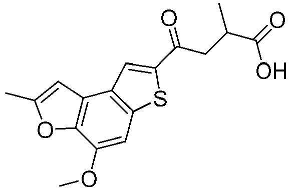 | 92 | 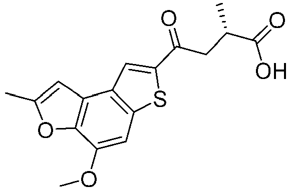 |
| 93 | 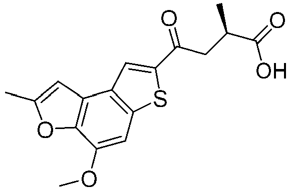 | 94 | 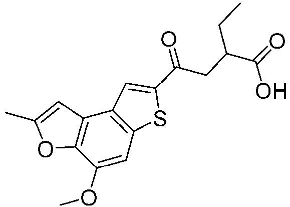 |
| 95 | 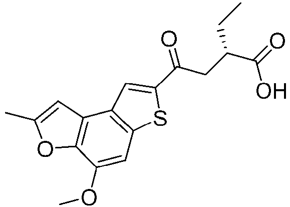 | 96 | 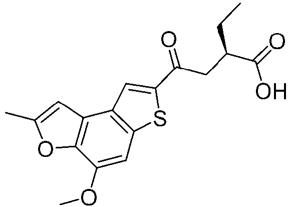 |
| 97 | 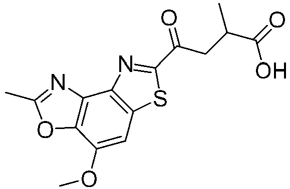 | 98 | 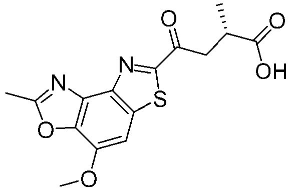 |
| 99 | 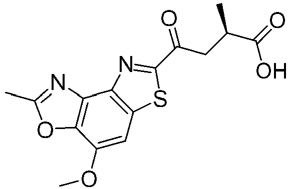 | 100 | 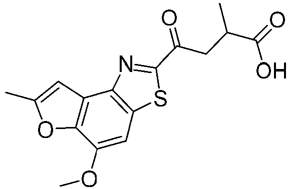 |
| 101 | 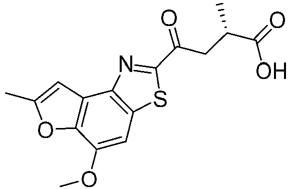 | 102 | 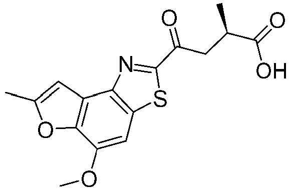 |
| 103 | 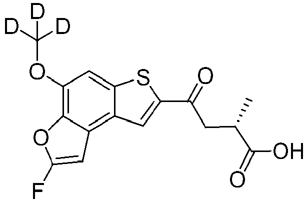 | 104 | 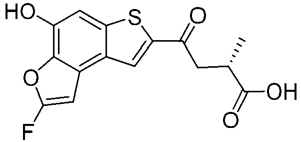 |

### Definitions

It should be noted that, when reference is made herein to a "compound" having a specific structural formula, a stereoisomer, diastereomer, enantiomer, racemic mixture, and isotopic derivative thereof, as well as a medicinal salt, solvate, and hydrate as alternative forms, are also generally encompassed. It is well known to those skilled in the art that a salt, solvate, and hydrate of a compound are alternative forms of the compound that can be converted into the compound under certain conditions. Accordingly, it should be particularly noted that when a compound is referred to herein, its medicinal salt is also generally included, and solvate and hydrate thereof are further included.

Similarly, when a compound is referred to herein, its prodrug, metabolite, and nitrogen oxide are also generally included.

The medicinal salt or pharmaceutically acceptable salt described in the present disclosure may be formed using an inorganic acid or an organic acid. The "medicinal salt" or "pharmaceutically acceptable salt" means a salt that is, within the scope of sound medical judgment, suitable for use in contact with the tissues of humans and lower animals without undue toxicity, irritation, and allergic response, and the like, and commensurate with a reasonable benefit/risk ratio. As outlined below, the salt may be prepared in situ during the final separation and purification of the compound of the present disclosure, or prepared by reacting the free base or free acid with a suitable reagent separately. For example, the free base may be reacted with a suitable acid. In addition, when the compound of the present disclosure carries an acidic moiety, suitable medicinal salts thereof may include metal salts, such as alkali metal salts (e.g., sodium or potassium salts), and alkaline earth metal salts (e.g., calcium or magnesium salts). Examples of pharmaceutically acceptable non-toxic acid addition salts are salts formed by amino groups with inorganic acids (e.g., hydrochloric acid, hydrobromic acid, phosphoric acid, sulfuric acid, and perchloric acid) or organic acids (e.g., acetic acid, oxalic acid, maleic acid, tartaric acid, citric acid, succinic acid, or malonic acid), or formed by using other methods known in the prior art such as ion exchange.

The medicinal salt of the present disclosure may be prepared by a conventional method, for example, by dissolving the compound of the present disclosure in a water-miscible organic solvent (e.g., acetone, methanol, ethanol, and acetonitrile), adding an excess of an aqueous organic or inorganic acid thereto to precipitate the salt from the resulting mixture, removing the solvent and remaining free acid therefrom, and then separating the precipitated salt.

The precursor or metabolite described in the present disclosure may be those known in the art, as long as the precursor or metabolite is converted into the compound through metabolism in vivo. For example, "prodrug" refers to those of the compound of the present disclosure that are, within the scope of sound medical judgment, suitable for use in contact with the tissues of humans and lower animals without undue toxicity, irritation, allergic response, and the like, commensurate with a reasonable benefit/risk ratio, and effective for their intended use. The term "prodrug" refers to a compound that rapidly generates the parent compound of the above formula through transformation in vivo, for example, through metabolism in vivo, or N-demethylation of the compound of the present disclosure.

The "solvate" described in the present disclosure means a physical association of the compound of the present disclosure with one or more solvent molecules (whether organic or inorganic). The physical association includes hydrogen bonding. In some cases, for example, when one or more solvent molecules are incorporated in the crystal lattice of a crystalline solid, the solvate will be capable of being isolated. The solvent molecules in the solvate may be present in a regular and/or disordered arrangement. Solvates may contain stoichiometric or non-stoichiometric solvent molecules. The "solvate" encompasses both solution-phase and isolatable solvates. Exemplary solvates include, but are not limited to, hydrates, ethanolates, methanolates, and isopropanolates. Methods of solvation are well known in the art.

The "stereoisomerism" described in the present disclosure is divided into conformational isomerism and configurational isomerism, and the configurational isomerism can be further divided into cis-trans isomerism and optical isomerism (i.e., optical isomerism). The conformational isomerism refers to a stereoisomerism phenomenon in which the rotation or distortion of the carbon-carbon single bond of an organic molecule with a certain configuration makes the atoms or atomic groups of the molecule produce different arrangements in space, and common examples include the structures of alkanes and cycloalkanes, such as chair and boat conformations as found in the cyclohexane structure. "Stereoisomer" refers to the case where the compound of the present disclosure contains one or more asymmetric centers, thereby existing as racemates and racemic mixtures, single enantiomers, diastereomeric mixtures, and single diastereomers. The compound of the present disclosure may have asymmetric centers, each of which gives rise to two optical isomers. The scope of the present disclosure includes all possible optical isomers, diastereomeric mixtures, and pure or partially pure compounds. The compound of the present disclosure may exist in the form of tautomers, which have different hydrogen attachment points through the displacement of one or more double bonds. For example, ketone and its enol form are keto-enol tautomers. All tautomers and mixtures thereof are included within the compound of the present disclosure. All enantiomers, diastereomers, racemates, mesomers, cis-trans isomers, tautomers, geometric isomers, epimers, and mixtures thereof of the compound of formula (I) are included within the scope of the present disclosure.

The "isotopic derivative" of the present disclosure refers to a molecule in which the compound described in this patent is labeled with an isotope. Isotopes commonly used for isotopic labeling are: hydrogen isotopes, ²H and ³H; carbon isotopes: ¹¹C, ¹³C, and ¹⁴C; chlorine isotopes: ³⁵Cl and ³⁷Cl; fluorine isotopes: ¹⁸F; iodine isotopes: ¹²³I and ¹²⁵I; nitrogen isotopes: ¹³N and ¹⁵N; oxygen isotopes: ¹⁵O, ¹⁷O, and ¹⁸O; and sulfur isotopes: ³⁵S. These isotopically labeled compounds can be used to study the distribution of pharmaceutical molecules in tissues. Deuterium ²H and carbon ¹³C, in particular, are more widely used due to their ease of labeling and detection. Substitution of certain heavy isotopes, such as heavy hydrogen (²H), may enhance metabolic stability, prolong the half-life, and provide therapeutic advantages resulting from reduced dosage. Generally, starting from the labeled starting materials, isotopically labeled compounds are synthesized by using known synthesis techniques in the same way as the synthesis of non-isotopically labeled compounds.

The compound or pharmaceutical composition of the present disclosure may be formulated into dosage forms, such as tablets, granules, powders, capsules, syrups, emulsions, microemulsions, solutions or suspensions, for oral or parenteral administration (including intramuscular, intravenous and subcutaneous routes, and intratumoral injection) according to any of the conventional methods.

Additional features of the present disclosure will become apparent from the description of exemplary embodiments of the present disclosure, which are presented for purposes of illustration and are not intended to be limiting thereof. The following examples are prepared, separated, and characterized using the methods disclosed in the present disclosure.

Unless otherwise specified, terms used in the present disclosure (including the specification and claims) are defined as follows. It must be noted that in the specification and the appended claims, the singular form "a" or "an" includes plural referents unless the context clearly dictates otherwise. Unless otherwise specified, conventional methods such as mass spectrometry, nuclear magnetic resonance, HPLC, protein chemistry, biochemistry, recombinant DNA technology, and pharmacology are used. In this application, unless otherwise specified, the use of "or" or "and" means "and/or".

Throughout the specification and claims, a given chemical formula or name shall encompass all stereoisomers and optical isomers, as well as racemic mixtures in which such isomers exist. Unless otherwise indicated, all chiral (enantiomers and diastereoisomers) and racemic forms are within the scope of the present disclosure. The compound may also exist as numerous geometric isomers of C=C double bonds, C=N double bonds, ring systems, etc., and all such stable isomers are encompassed within the present disclosure. The present disclosure describes the cis- and trans- (or E- and Z-) geometric isomers of the compound of the present disclosure, which can be separated as mixtures of isomers or as separate isomeric forms. The compound of the present disclosure may be separated in optically active form or racemic form. All methods for preparing the compound of the present disclosure and intermediates prepared therein are considered part of the present disclosure. When preparing enantiomeric or diastereomeric products, they can be separated by conventional methods (e.g., chromatography or fractional crystallization). Depending on the process conditions, the final products of the present disclosure are obtained in free (neutral) or salt forms. Both the free forms and salts of these final products are within the scope of the present disclosure. If desired, one form of the compound may be converted into another. The free base or acid may be converted into a salt; the salt may be converted into the free compound or another salt; mixtures of isomeric compounds of the present disclosure may be separated into the individual isomers. The compounds, free forms and salts thereof of the present disclosure, may exist in a variety of tautomeric forms in which a hydrogen atom is transferred to another part of the molecule, thereby resulting in rearrangement of chemical bonds between atoms of the molecule. It is to be understood that all tautomeric forms that may exist are included in the present disclosure.

Unless otherwise defined, the definitions of substituents of the present disclosure are each independent and not interrelated, e.g., for R^{a} (or R⁶) in substituents, they are each independent in the definition of different substituents. Specifically, when a definition of R^{a} (or R^{b}) is selected in a substituent, it does not mean that R^{a} (or R^{b}) has the same definition in other substituents. More specifically, for example (a non-exhaustive list), in the case of NR^{a}R^{b}, when R^{a} (or R^{b}) is defined as hydrogen, it does not mean that in -C(O)-NR^{a}R^{b}, R^{a} (or R^{b}) is necessarily hydrogen.

Unless otherwise defined, when a substituent is labeled "optionally substituted", the substituent is selected from, for example, the following substituents: alkyl, cycloalkyl, aryl, heterocyclyl, halogen, hydroxy, alkoxy, oxo, alkanoyl, aryloxy, alkanoyloxy, amino, alkylamino, arylamino, arylalkylamino, disubstituted amine group (in which two amino substituents are selected from alkyl, aryl, or arylalkyl), alkanoylamino, aroylamino, aralkanoylamino, substituted alkanoylamino, substituted arylamino, substituted aralkanoylamino, thio, alkylthio, arylthio, arylalkylthio, arylthiocarbonyl, arylalkylthiocarbonyl, alkylsulfonyl, arylsulfonyl, arylalkylsulfonyl, sulfonamido such as -SO₂NH₂, substituted sulfonamido, nitro, cyano, carboxy, carbamoyl such as -CONH₂, substituted carbamoyl such as -CONH alkyl, -CONH aryl, -CONH arylalkyl, or the case where there are two substituents selected from alkyl, aryl, or arylalkyl on the nitrogen, alkoxycarbonyl, aryl, substituted aryl, guanidino, heterocyclyl such as indolyl, imidazolyl, furanyl, thienyl, thiazolyl, pyrrolidinyl, pyridyl, pyrimidinyl, pyrrolidinyl, piperidinyl, morpholinyl, piperazinyl, and homopiperazinyl, and substituted heterocyclyl.

As used herein, the term "alkyl" or "alkylene" is intended to include both branched and linear saturated aliphatic hydrocarbon groups having the specified number of carbon atoms. For example, "C₁-C₆ alkyl" denotes an alkyl group having 1 to 6 carbon atoms. Examples of alkyl groups include, but are not limited to, methyl (Me), ethyl (Et), n-propyl, isopropyl, n-butyl, isobutyl, tert-butyl, n-pentyl, isopentyl, and neopentyl.

The term "alkenyl" denotes a linear or branched hydrocarbon group containing one or more double bonds and typically 2 to 20 carbon atoms in length. For example, "C2-C6 alkenyl" contains 2 to 6 carbon atoms. Alkenyl groups include, but are not limited to, for example, ethenyl, propenyl, butenyl, and 1-methyl-2-buten-1-yl.

The term "alkynyl" denotes a linear or branched hydrocarbon group containing one or more triple bonds and typically 2 to 20 carbon atoms in length. For example, "C2-C6 alkynyl" contains 2 to 6 carbon atoms. Representative alkynyl groups include, but are not limited to, for example, ethynyl, 1-propynyl, and 1-butynyl.

The term "alkoxy" or "alkyloxy" refers to -O-alkyl. "C1-C6 alkoxy" (or alkyloxy) is intended to include C1, C2, C3, C4, C5, and C6 alkoxy. Examples of alkoxy groups include, but are not limited to, methoxy, ethoxy, propoxy (e.g., n-propoxy and isopropoxy), and tert-butoxy. Similarly, "alkylthio" or "thioalkoxy" denotes an alkyl group having the specified number of carbon atoms and linked via a sulfur bridge as defined above; for example, methyl-S- and ethyl-S-.

The term "carbonyl" refers to an organic functional group (C=O) composed of carbon and oxygen atoms linked via a double bond.

The term "aryl", alone or as part of a larger moiety such as "aralkyl", "aralkoxy", or "aryloxyalkyl", refers to a monocyclic, bicyclic, or tricyclic ring system having a total of 5 to 12 ring members, where at least one ring in the system is aromatic and where each ring in the system contains 3 to 7 ring members. In certain embodiments of the present disclosure, "aryl" refers to an aromatic ring system including, but not limited to, phenyl, biphenyl, indanyl, 1-naphthyl, 2-naphthyl, and tetrahydronaphthyl. The term "aralkyl" or "arylalkyl" refers to an alkyl residue attached to an aryl ring. Non-limiting examples include benzyl, phenethyl, etc. The fused aryl group may be attached to another group at a suitable position on the cycloalkyl ring or the aromatic ring. For example, a dashed line drawn from a ring system indicates that the bond may be attached to any suitable ring atom.

The term "cycloalkyl" refers to a monocyclic or bicyclic alkyl group. Monocyclic alkyl refers to C3-C8 cyclic alkyl, including, but not limited to, cyclopropyl, cyclobutyl, cyclopentyl, cyclohexyl, and norbornyl. Branched cycloalkyl, such as 1-methylcyclopropyl and 2-methylcyclopropyl, is included in the definition of "cycloalkyl". Bicyclic alkyl includes bridged, spiro, or fused cycloalkyl.

The term "heterocycloalkyl" means that in the structure of the above cycloalkyl, at least one ring carbon atom is substituted with a heteroatom selected from O, N, S, and Se.

The term "cycloalkenyl" refers to a monocyclic or bicyclic alkenyl group. Monocyclic alkenyl refers to C3-C8 cyclic alkenyl, including, but not limited to, cyclopropenyl, cyclobutenyl, cyclopentenyl, cyclohexenyl, and norbornenyl. Branched cycloalkenyl, such as 1-methylcyclopropenyl and 2-methylcyclopropenyl, is included in the definition of "cycloalkenyl". Bicyclic alkenyl includes bridged, spiro, or fused cyclic alkenyl.

"Halo" or "halogen" includes fluorine, chlorine, bromine, and iodine. "Haloalkyl" is intended to include both branched and linear saturated aliphatic hydrocarbon groups having the specified number of carbon atoms and substituted with one or more halogens. Examples of haloalkyl groups include, but are not limited to, fluoromethyl, difluoromethyl, trifluoromethyl, trichloromethyl, pentafluoroethyl, pentachloroethyl, 2,2,2-trifluoroethyl, heptafluoropropyl, and heptachloropropyl. Examples of haloalkyl groups also include "fluoroalkyl" groups intended to include branched and linear saturated aliphatic hydrocarbon groups having the specified number of carbon atoms and substituted with one or more fluorine atoms.

"Haloalkoxy" or "haloalkyloxy" denotes a haloalkyl group having the specified number of carbon atoms and linked via an oxygen bridge as defined above. For example, "C1-C6 haloalkoxy" is intended to include C1, C2, C3, C4, C5, and C6 haloalkoxy. Examples of haloalkoxy groups include, but are not limited to, trifluoromethoxy, 2,2,2-trifluoroethoxy, and pentafluoroethoxy. Similarly, "haloalkylthio" or "thiohaloalkoxy" denotes a haloalkyl group having the specified number of carbon atoms and linked via a sulfur bridge as defined above; for example, trifluoromethyl-S- and pentafluoroethyl-S-.

In the present disclosure, the expression Cx1-Cx2 is used when referring to some substituent groups, which means that the number of carbon atoms in the substituent group may be x1 to x2. For example, C0-C8 means that the group contains 0, 1, 2, 3, 4, 5, 6, 7, or 8 carbon atoms, C1-C8 means that the group contains 1, 2, 3, 4, 5, 6, 7, or 8 carbon atoms, C2-C8 means that the group contains 2, 3, 4, 5, 6, 7, or 8 carbon atoms, C3-C8 means that the group contains 3, 4, 5, 6, 7, or 8 carbon atoms, C4-C8 means that the group contains 4, 5, 6, 7, or 8 carbon atoms, C0-C6 means that the group contains 0, 1, 2, 3, 4, 5, or 6 carbon atoms, C1-C6 means that the group contains 1, 2, 3, 4, 5, or 6 carbon atoms, C2-C6 means that the group contains 2, 3, 4, 5, or 6 carbon atoms, and C3-C6 means that the group contains 3, 4, 5, or 6 carbon atoms.

In the present disclosure, the expression "x1-x2-membered ring" is used when referring to cyclic groups (e.g., aryl, heteroaryl, cycloalkyl, and heterocycloalkyl), which means that the number of ring atoms of the group may be x1 to x2. For example, the 3-12-membered cyclic group may be a 3-, 4-, 5-, 6-, 7-, 8-, 9-, 10-, 11-, or 12-membered ring, the number of ring atoms of which may be 3, 4, 5, 6, 7, 8, 9, 10, 11, or 12; the 3-6-membered ring means that the cyclic group may be a 3-, 4-, 5-, or 6-membered ring, the number of ring atoms of which may be 3, 4, 5, or 6; the 3-8-membered ring means that the cyclic group may be a 3-, 4-, 5-, 6-, 7-, or 8-membered ring, the number of ring atoms of which may be 3, 4, 5, 6, 7, or 8; the 3-9-membered ring means that the cyclic group may be a 3-, 4-, 5-, 6-, 7-, 8-, or 9-membered ring, the number of ring atoms of which may be 3, 4, 5, 6, 7, 8, or 9; the 4-7-membered ring means that the cyclic group may be a 4-, 5-, 6-, or 7-membered ring, the number of ring atoms of which may be 4, 5, 6, or 7; the 5-8-membered ring means that the cyclic group may be a 5-, 6-, 7-, or 8-membered ring, the number of ring atoms of which may be 5, 6, 7, or 8; the 5-12-membered ring means that the cyclic group may be a 5-, 6-, 7-, 8-, 9-, 10-, 11-, or 12-membered ring, the number of ring atoms of which may be 5, 6, 7, 8, 9, 10, 11, or 12; and the 6-12-membered ring means that the cyclic group may be a 6-, 7-, 8-, 9-, 10-, 11-, or 12-membered ring, the number of ring atoms of which may be 6, 7, 8, 9, 10, 11, or 12. The ring atom may be a carbon atom or a heteroatom, for example, a heteroatom selected from N, O, and S. When the ring is a heterocycle, the heterocycle may contain 1, 2, 3, 4, 5, 6, 7, 8, 9, 10, or more ring heteroatoms, e.g., a heteroatom selected from N, O, and S.

In the present disclosure, one or more halogens may each independently be selected from fluorine, chlorine, bromine, and iodine.

The term "heteroaryl" means a stable 3-, 4-, 5-, 6-, or 7-membered aromatic monocyclic or aromatic bicyclic or 7-, 8-, 9-, 10-, 11-, 12-membered aromatic polycyclic heterocycle, which is fully unsaturated or partially unsaturated, and contains carbon atoms and 1, 2, 3, or 4 heteroatoms independently selected from N, O, and S; and includes any polycyclic group in which any heterocycle defined above is fused to a benzene ring. The nitrogen and sulfur heteroatoms may optionally be oxidized. The nitrogen atom is substituted or unsubstituted (i.e., N or NR, where R is H or another substituent if defined). The heterocycle may be attached to its pendant group at any heteroatom or carbon atom that results in a stable structure. If the resulting compound is stable, the heterocyclyl groups described herein may be substituted on a carbon or nitrogen atom. The nitrogen in the heterocycle may be optionally quaternized. Preferably, when the total number of S and O atoms in the heterocycle exceeds 1, then these heteroatoms are not adjacent to each other. Preferably, the total number of S and O atoms in the heterocycle is not greater than 1.

### DESCRIPTION OF THE DRAWINGS

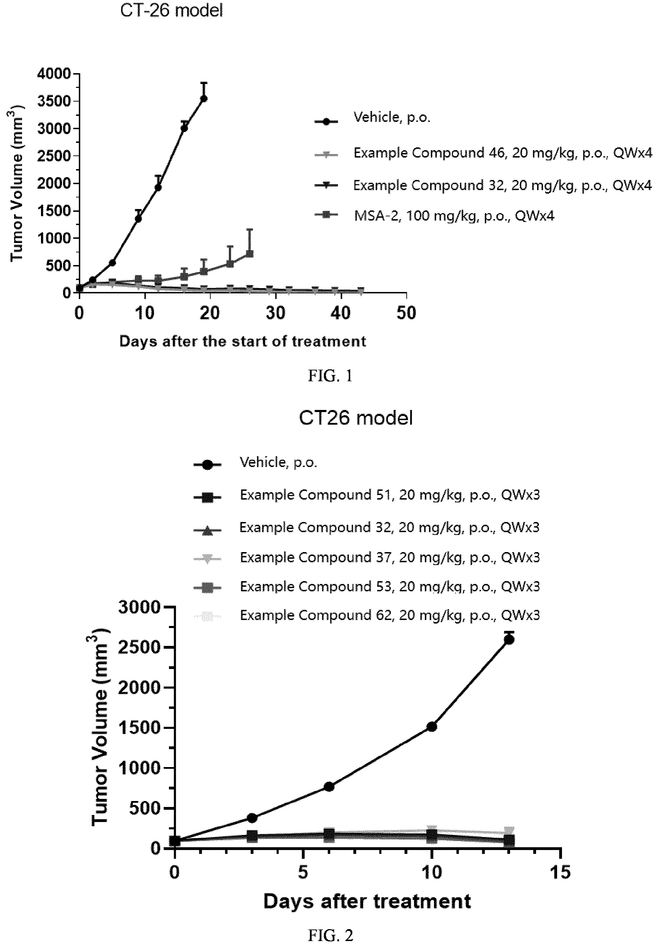

FIG. 1 shows the pharmacodynamic evaluation of test compounds of the present disclosure in a subcutaneous tumor model of female Balb/c mice transplanted with mouse colorectal cancer CT26 cell line.
FIG. 2 shows the pharmacodynamic evaluation of additional test compounds of the present disclosure in a subcutaneous tumor model of female Balb/c mice transplanted with mouse colorectal cancer CT26 cell line.

### SPECIFIC IMPLEMENTATIONS

The following preparations and examples illustrate the present disclosure, but do not limit the present disclosure in any way.

The features and advantages of the subject matter of the present disclosure will become more apparent from the detailed description of selected embodiments. As will be appreciated, the disclosed and claimed subject matter is capable of modifications in various respects, all without departing from the scope of the claims. Accordingly, the description is to be regarded as illustrative in nature and not as restrictive. The full scope of the subject matter of the present disclosure is set forth in the claims.

The present disclosure can be more readily understood by reference to the following examples, which are provided merely for purposes of illustration and are not intended to limit the scope of the present disclosure.

### Example 1

### Synthesis of 4-(4-methoxythieno[2',3':5,6]benzo[1,2-d]oxazol-7-yl)-4-oxobutanoic acid (Example Compound 1)

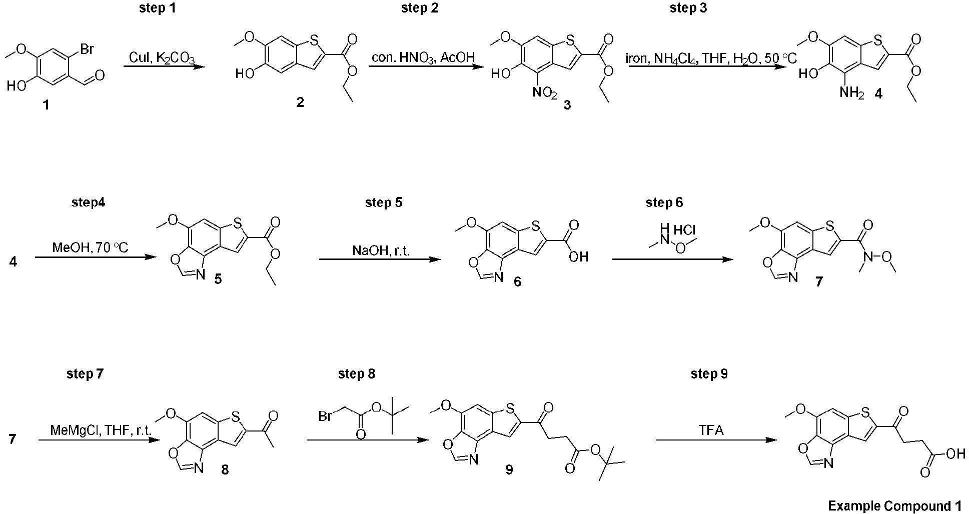

### Step (I): Synthesis of ethyl 5-hydroxy-6-methoxybenzo[b]thiophene-2-carboxylate (Intermediate 2)

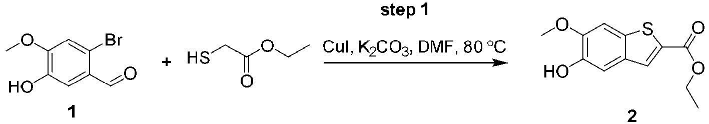

Starting material 2-bromo-5-hydroxy-4-methoxybenzaldehyde (25.0 g, 108.2 mmol) was dissolved in DMF (250 mL). CuI (6.2 g, 32.5 mmol) and ethyl 2-mercaptoacetate (29.9 g, 248.9 mmol) were then added and cooled to 0°C. K₂CO₃ (37.4 g, 270.5 mmol) was added to the mixture at 0°C. The reaction mixture was then sealed and stirred at 80°C under N₂ protection for 3 hours. The reaction mixture was cooled to room temperature, quenched into ice-water (2.5 kg), and then diluted with EtOAc (1000 mL). The resulting mixture was extracted with EtOAc (1000 mL). The organic phase was then washed with water (1000 mL) and brine (500 mL), dried over Na₂SO₄, and filtered. The filtrate was concentrated under vacuum to give a crude sample. The crude product was further purified by column chromatography (SiO₂, petroleum ether:EtOAc = 3:1) to give Intermediate 2 as a yellow solid (20.0 g, 73%), ESI MS [M + H]⁺ for C₁₂H₁₃O₄S, calcd 253.1, found 253.0.

### Step (II): Synthesis of ethyl 5-hydroxy-6-methoxy-4-nitrobenzo[b]thiophene-2-carboxylate (Intermediate 3)

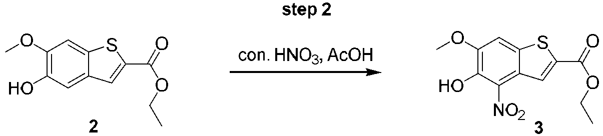

Intermediate 2 (3.0 g, 11.9 mmol) prepared in step (I) was dissolved in AcOH (30 mL), and concentrated nitric acid (0.9 g, 14.3 mmol) was added dropwise. The reaction mixture was then sealed and stirred at room temperature for 2 hours. The reaction mixture was quenched into water (200 g). The resulting mixture was extracted with EtOAc (200 mL). The organic phase was washed twice with water (100 mL), dried over Na₂SO₄, and filtered. The filtrate was concentrated under vacuum to give a crude sample. The crude product was further purified by column chromatography (SiO₂, petroleum ether:EtOAc = 3:1) to give Intermediate 3 as a yellow solid (3.1 g, 88%): ¹H NMR (400 MHz, DMSO-d6) δ 11.02 (s, 1H), 8.04 (s, 1H), 7.99 (s, 1H), 4.34 (q, *J=* 7.1 Hz, 2H), 3.98 (s, 3H), 1.33 (t, *J=* 7.1 Hz, 3H).

### Step (III): Synthesis of ethyl 4-amino-5-hydroxy-6-methoxybenzo[b]thiophene-2-carboxylate (Intermediate 4)

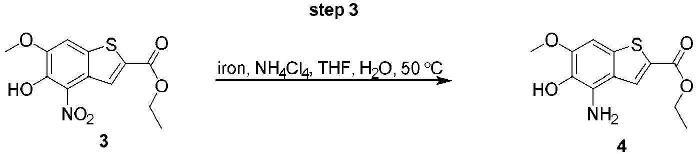

Intermediate 3 (3.1 g, 10.4 mmol) prepared in step (II), iron powder (2.9 g, 52.1 mmol), and NH₄Cl (2.8 g, 52.1 mmol) were dissolved in THF (30 mL) and H₂O (10 mL), and the mixture was purged with N₂ three times. The reaction mixture was then sealed and stirred at 50°C under N₂ protection for 2 hours. The reaction mixture was cooled to room temperature and filtered through celite. The filtrate was diluted with EtOAc (200 mL) and washed with water (50 mL). The organic phase was collected, dried over Na₂SO₄, and concentrated under vacuum to give the crude product Intermediate 4 as a grey solid (2.5 g, 90%), ESI MS [M + H]⁺ for C₁₂H₁₄NO₄S, calcd 268.1, found 268.1.

### Step (IV): Synthesis of ethyl 4-methoxythieno[2',3':5,6]benzo[1,2-d]oxazole-7-carboxylate (Intermediate 5)

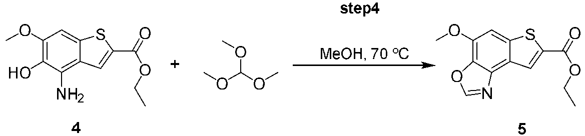

Intermediate 4 (3.0 g, 11.2 mmol) prepared in step (III) was dissolved in MeOH (10 mL) and trimethoxymethane (35.0 g, 336.6 mmol), and then the reaction mixture was stirred at 70°C under N₂ protection for 16 hours. The reaction mixture was concentrated under vacuum to give a crude sample. Water (200 mL) was added to the residue, and the mixture was extracted three times with EtOAc (200 mL). The organic phase was collected, dried over Na₂SO₄, and filtered. The filtrate was concentrated under vacuum to give a crude sample. The crude product was further purified by column chromatography (SiO₂, petroleum ether:EtOAc = 3:1) to give the product 5 as a white solid (1.7 g, 55%), ESI MS [M + H]⁺ for C₁₃H₁₂NO₄S, calcd 278.0, found 278.1.

### Step (V): Synthesis of 4-methoxythieno[2',3':5,6]benzo[1,2-d]oxazole-7-carboxylic acid (Intermediate 6)

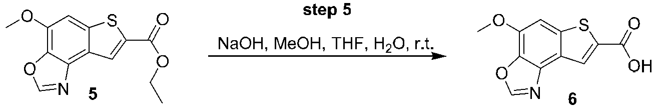

Intermediate 5 (700.0 mg, 2.5 mmol) prepared in step (IV) was dissolved in THF (5 mL), MeOH (5 mL), and H₂O (2 mL), and NaOH (504.0 mg, 12.6 mmol) was added. The reaction mixture was then stirred at room temperature for 1 hour. The reaction mixture was adjusted to pH = 4 and diluted with water (100 mL). The mixture was extracted three times with EtOAc (100 mL). The organic phase was collected, dried over Na₂SO₄, and concentrated under vacuum to give Intermediate Compound 6 as a yellow solid (600.0 mg, 96%), ESI MS [M + H]⁺ for C₁₁H₈NO₄S, calcd 250.0, found 250.1. The reaction product was used in the next step without isolation.

### Step (VI): Synthesis of N,4-dimethoxy-N-methylthieno[2',3':5,6]benzo[1,2-d]oxazole-7-carboxamide (Intermediate 7)

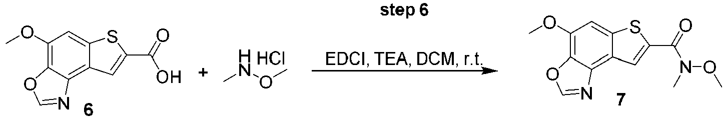

Intermediate 5 (600.0 mg, 2.4 mmol) prepared in step (V), EDCI (923.0 mg, 4.8 mmol), N,O-dimethylhydroxylamine hydrochloride (281.8 mg, 2.9 mmol), and trimethylamine (730.8 mg, 7.2 mmol) were dissolved in DCM (20 mL), and the mixture was purged with N₂ three times. The reaction mixture was stirred at room temperature under N₂ protection for 2 hours. The reaction mixture was quenched into water (100 g) and then diluted with EtOAc (100 mL). The resulting mixture was extracted with EtOAc (200 mL). The organic phase was then washed three times with water (100 mL), dried over Na₂SO₄, and filtered. The filtrate was concentrated under vacuum to give a crude sample. The crude product was further purified with petroleum ether:EtOAc = 1:1 to give the pure title Intermediate Compound 7 as a pale red solid (250.0 mg, 36%), ESI MS [M + H]⁺ for C₁₃H₁₃N₂O₄S, calcd 293.1, found 293.1.

### Step (VII): Synthesis of 1-(4-methoxythieno[2',3':5,6]benzo[1,2-d]oxazol-7-yl)ethan-1-one (Intermediate 8)

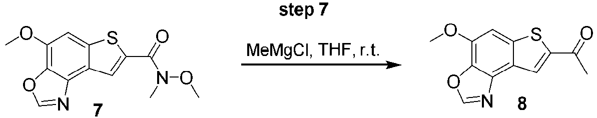

Intermediate 7 (350.0 mg, 1.2 mmol) prepared in step (VI) was dissolved in THF (10 mL), and a 1.0 M solution of methylmagnesium chloride in THF (3.6 mL, 3.6 mmol) was added dropwise at 0°C. The reaction mixture was stirred at room temperature for 1 hour. The reaction mixture was quenched into water (50 g) and then diluted with EtOAc (100 mL). The resulting mixture was extracted with EtOAc (100 mL). The organic phase was then washed three times with water (50 mL), dried over Na₂SO₄, and filtered. The filtrate was concentrated under vacuum to give a crude sample. The crude product was further purified by trituration (petroleum ether:EtOAc = 3:1) to give the pure title Intermediate Compound 8 as a yellow solid (240.0 mg, 81%), ESI MS [M + H]⁺ for C₁₂H₁₀NO₃S, calcd 248.0, found 248.1.

### Step (VIII): Synthesis of tert-butyl 4-(4-methoxythieno[2',3':5,6]benzo[1,2-dioxazol-7-yl)-4-oxobutanoate (Intermediate 9)

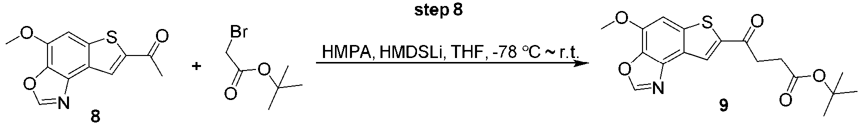

Intermediate 8 (100.0 mg, 0.4 mmol) prepared in step (VII) and HMPA (217.4 mg, 1.2 mmol) were dissolved in anhydrous THF (5 mL), and the mixture was purged with N₂ three times. The reaction mixture was cooled to -78°C under N₂ protection using an ethanol/CO₂(s) bath. Then, 1M HMDSLi in THF (1.2 mL, 1.2 mmol) was added dropwise and stirred at -78°C for 30 minutes. Afterwards, tert-butyl bromoacetate (276.1 mg, 1.4 mmol) was added to the reaction mixture and stirred at room temperature under a N₂ atmosphere for 0.5 hour. The reaction mixture was quenched into water (50 g) and then diluted with EtOAc (100 mL). The resulting mixture was extracted with EtOAc (100 mL). The organic phase was then washed three times with water (50 mL), dried over Na₂SO₄, and filtered. The filtrate was concentrated under vacuum to give a crude sample. The crude product was further purified by trituration (petroleum ether:EtOAc = 3:1) to give the pure title Intermediate Compound 9 as a white solid (100.0 mg, 68%), ESI MS [M + H]⁺ for C₁₈H₂₀NO₅S, calcd 362.1, found 306.1.

### Step (IX): Synthesis of 4-(4-methoxythieno[2',3':5,6]benzo[1,2-d]oxazol-7-yl)-4-oxobutanoic acid

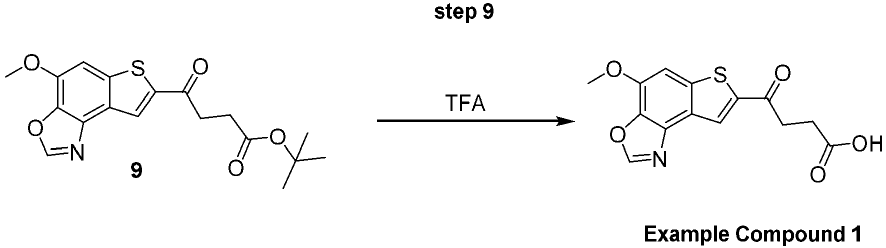

Intermediate 9 (14.5 mg, 0.04 mmol) prepared in step (VIII) was dissolved in TFA (40.0 mg, 0.35 mmol), and the reaction mixture was stirred at room temperature for 3 hours. The reaction mixture was concentrated under vacuum to give a crude sample. The crude product was further purified by reverse-phase HPLC (C18 column, 50-60% gradient of MeCN and water containing 0.1% TFA) to give the final product Example Compound 1 as a white solid (4.7 mg, 50%), ESI MS [M - H]⁻ for C₁₄H₁₀NO₅S, calcd 304.0, found 304.0. ¹H NMR (400 MHz, DMSO-*d*₆) δ 12.21 (s, 1H), 8.93 (s, 1H), 8.56 (s, 1H), 7.79 (s, 1H), 4.07 (s, 3H), 3.38 (t, *J =* 6.3 Hz, 2H), 2.60 (t, *J* = 6.3 Hz, 2H).

### Example 2

### Synthesis of 4-(4-methoxy-1,2-dihydrothieno[3,2-e]benzofuran-7-yl)-4-oxobutanoic acid (Example Compound 2)

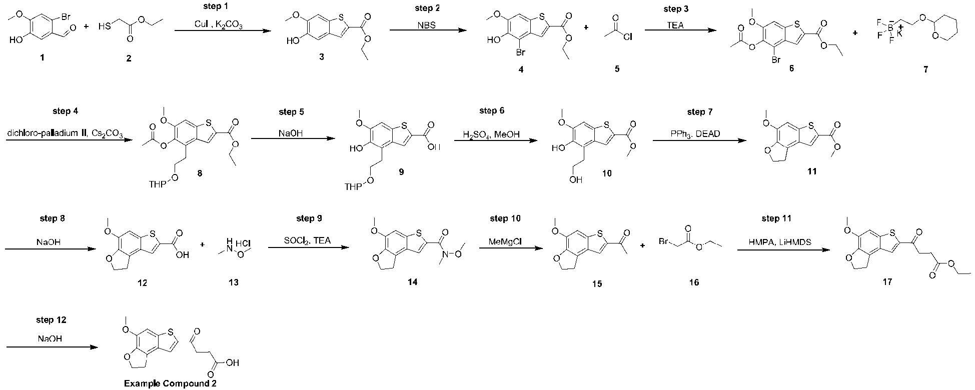

### Step (I): Synthesis of ethyl 5-hydroxy-6-methoxy-1-benzothiophene-2-carboxylate (Intermediate 3)

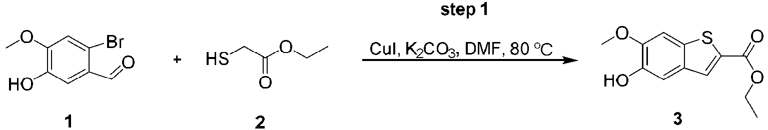

2-Bromo-5-hydroxy-4-methoxybenzaldehyde (6.0 g, 26.0 mmol) was dissolved in DMF (60 mL), and ethyl 2-mercaptoacetate (7.2 g, 59.7 mmol) and λ¹/-copper iodide (1.5 g, 7.8 mmol) were added. The mixture was then cooled to 0°C, and dipotassium carbonate (9.0 g, 64.9 mmol) was slowly added. The mixture was purged with N₂ three times. The reaction mixture was then sealed and stirred at 80°C under N₂ protection for 2 hours. The reaction mixture was quenched into ice-water (600 g) to maintain the internal temperature below 20°C. The resulting mixture was extracted with EtOAc (600 mL). The organic phase was then washed with water (400 mL) and brine (200 mL), dried over Na₂SO₄, and filtered. The filtrate was concentrated under vacuum to give a crude sample. The crude product was further purified by column chromatography (SiO₂, 20% EtOAc/petroleum ether) to give Intermediate 3 as a white solid (5.1 g, 78%), ¹H NMR (400 MHz, DMSO-d6) δ 9.38 (s, 1H), 7.96 (d, *J=* 0.6 Hz, 1H), 7.54 (s, 1H), 7.31 (s, 1H), 4.30 (q, *J =* 7.1 Hz, 2H), 3.86 (s, 3H), 1.31 (t, *J* = 7.1 Hz, 3H).

### Step (II): Synthesis of ethyl 4-bromo-5-hydroxy-6-methoxy-1-benzothiophene-2-carboxylate (Intermediate 4)

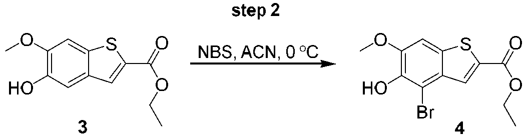

To a solution of Intermediate 3 (4.0 g, 15.9 mmol) prepared in step (I) in ethyl ester (50 mL) was added NBS (3.4 g, 19.0 mmol), and the mixture was purged with N₂ three times. The reaction mixture was then sealed well and stirred at 0°C under N₂ protection for 1 hour.

The reaction mixture was filtered and washed twice with water (50 mL). The filter cake was collected and dried under reduced pressure to give the crude product Intermediate Compound 4 as a brown solid (4.1 g, 78%), ESI MS[M + H]⁺ for C₁₂H₁₂BrO₄S, calcd 332.2, found 333.0.

### Step (III): Synthesis of ethyl 5-(acetoxy)-4-bromo-6-methoxy-1-benzothiophene-2-carboxylate (Intermediate 6)

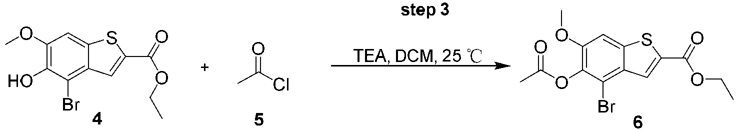

Intermediate Compound 4 (3.6 g, 10.9 mmol) prepared in step (II) was added to acetyl chloride (2.6 g, 32.6 mmol) and triethylamine (3.3 g, 32.6 mmol), and the mixture was purged with N₂ three times. The reaction mixture was then sealed well and stirred at room temperature under N₂ protection for 1 hour.

The reaction mixture was dried under reduced pressure, diluted with EtOAc (200 mL), and then washed with water (200 mL). The organic phase was collected, dried over Na₂SO₄, and concentrated under vacuum to give a crude sample. The crude product was further purified by column chromatography (SiO₂, 30% EtOAc/petroleum ether) to give Intermediate 6 as a white solid (3.5 g, 86%), ESI MS[M + H]⁺ for C₁₄H₁₄BrO₅S, calcd 374.2, found 375.0.

### Step (IV): Synthesis of ethyl 5-(acetyloxy)-6-methoxy-4-[2-(oxa-2-oxy)ethyl]-1-benzothiophene-2-carboxylate (Intermediate 8)

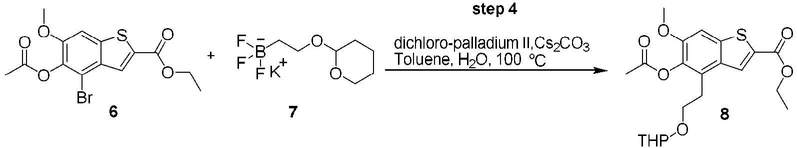

To a solution of Intermediate Compound 6 (1.0 g, 2.7 mmol) prepared in step (III) in toluene (10 mL) and H₂O (2 mL) was added potassium trifluoro[2-(oxa-2-oxy)ethyl]boride (3.2 g, 13.4 mmol), bis[di-tert-butyl(4-dimethylaminophenyl)phosphine]dichloropalladium (II) (1.0 g, 1.3 mmol), and dicesium (1+) carbonate (1.8 g, 5.4 mmol). The mixture was then purged with N₂ three times. The reaction mixture was then sealed well and stirred at 100°C under N₂ protection overnight. The reaction mixture was diluted with ice-water (100 mL) and extracted twice with EtOAc (100 mL). The organic phase was collected, dried over Na₂SO₄, and concentrated under vacuum to give a crude sample. The crude product was further purified by column chromatography (SiO₂, 30% EtOAc/petroleum ether) to give Intermediate 8 as a yellow solid (0.4 g, 35%), ¹H NMR (400 MHz, Chloroform-d) δ 8.13 (s, 1H), 7.22 (s, 1H), 4.55 (dd, *J* = 4.4, 2.7 Hz, 1H), 4.39 (t, *J=* 7.1 Hz, 2H), 3.89 (s, 3H), 3.70 (t, *J=* 7.0 Hz, 2H), 3.16 (t, *J* = 7.1 Hz, 2H), 2.55 (t, *J=* 6.9 Hz, 2H), 2.36 (s, 3H), 1.55 - 1.49 (m, 6H), 1.40 (s, 3H).

### Step (V): Synthesis of 5-hydroxy-6-methoxy-4-[2-(oxa-2-oxy)ethyl]-1-benzothiophene-2-carboxylic acid (Intermediate 9)

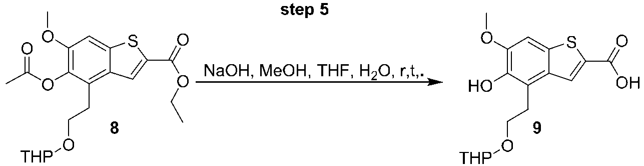

To a solution of Intermediate Compound 8 (2.0 g, 4.7 mmol) prepared in step (IV) in tetrahydrofuran (10 mL), methanol (10 mL), and H₂O (5 mL) was added sodium hydroxide (946.0 mg, 23.7 mmol). The mixture was then stirred at room temperature for 5 hours.

The reaction mixture was diluted with water (100 mL) and extracted with EtOAc (100 mL). The aqueous phase was adjusted to pH = 5-6 and extracted twice with EtOAc (100 mL). The organic phase was collected, dried over Na₂SO₄, and concentrated under vacuum to give crude Intermediate 9 as a yellow solid (1.5 g, 90%).

### Step (VI): Synthesis of methyl 5-hydroxy-4-(2-hydroxyethyl)-6-methoxy-1-benzothiophene-2-carboxylate (Intermediate 10)

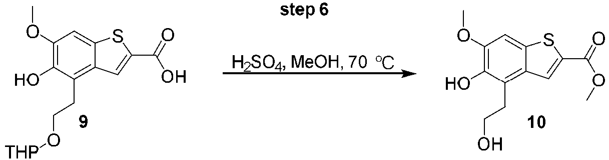

To a solution of Intermediate Compound 9 (1.5 g, 4.3 mmol) prepared in step (V) in methanol (20 mL) was added sulfuric acid (2 mL, 98%, 20.4 mmol). The mixture was then sealed well and stirred at 70°C overnight. The reaction mixture was diluted with water (200 mL) and extracted twice with EtOAc (200 mL). The organic phase was collected, dried over Na₂SO₄, and concentrated under vacuum to give a crude sample. The crude product was further purified by column chromatography (SiO₂, 30% EtOAc/petroleum ether) to give Intermediate 10 as a yellow solid (1.0 g, 83%): ESI MS[M + H]⁺ for C₁₃H₁₅O₅S, calcd 283.3, found 283.0.

### Step (VII): Synthesis of methyl 4-methoxy-1,2-dihydrothieno[3,2-e]benzofuran-7-carboxylate (Intermediate 11)

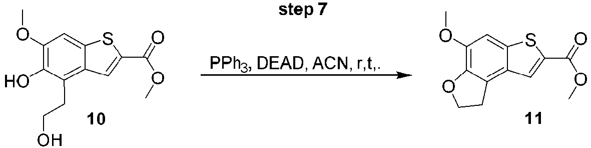

To a solution of Intermediate Compound 10 (1.2 g, 4.3 mmol) prepared in step (VI) in tetrahydrofuran (50 mL) was added triphenylphosphine (2.2 g, 8.5 mmol). The mixture was then purged with N₂ three times and cooled to 0°C. N-[(ethoxycarbonyl)imino]ethoxyformamide (1.5 g, 8.5 mmol) was then added to the mixture. The mixture was purged with N₂ three times. The reaction mixture was then sealed well and stirred at room temperature under N₂ protection for 2 h. The reaction mixture was diluted with water (100 mL) and extracted twice with EtOAc (100 mL). The organic phase was collected, dried over Na₂SO₄, and concentrated under vacuum to give a crude sample. The crude product was further purified by column chromatography (SiO₂, 10% EtOAc/petroleum ether) to give Intermediate 11 as a yellow solid (1.0 g, 89%): ESI MS[M + H]⁺ for C₁₃H₁₃O₄S, calcd 265.3, found 265.0.

### Step (VIII): Synthesis of 4-methoxy-1,2-dihydrothieno[3,2-e]benzofuran-7-carboxylic acid (Intermediate 12)

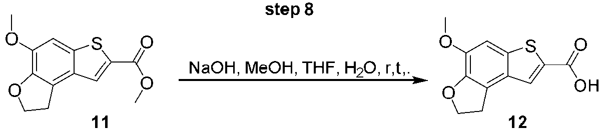

To a solution of Intermediate Compound 11 (1.0 g, 3.8 mmol) prepared in step (VI) in methanol (10 mL), THF (10 mL), and H₂O (5 mL) was added sodium hydroxide (756.0 mg, 18.9 mmol). The mixture was then stirred at room temperature for 2 hours. The reaction mixture was diluted with water (100 mL) and extracted with EtOAc (100 mL). The aqueous phase was adjusted to pH = 5-6 and extracted twice with EtOAc (100 mL). The organic phase was collected, dried over Na₂SO₄, and concentrated under vacuum to give crude Intermediate Compound 12 as a yellow solid (800.0 mg, 85%), ESI MS[M + H]⁺ for C₁₂H₁₁O₄S, calcd 251.3, found 251.0.

### Step (IX): Synthesis of N,4-dimethoxy-N-methyl-1,2-dihydrothieno[3,2-e]benzofuran-7-carboxamide (Intermediate 14)

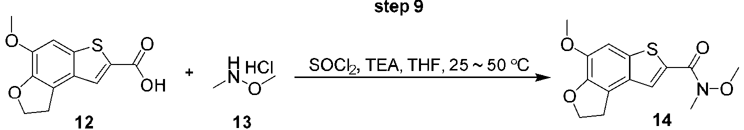

To a solution of Intermediate Compound 12 (1.0 g, 4.0 mmol) prepared in step (VIII) in dichloromethane (20 mL) was added sulfuryl dichloride (2.4 g, 20.0 mmol). The reaction mixture was then sealed well and stirred at 50°C under N₂ protection for 1 hour. Afterwards, the mixture was concentrated under vacuum and then added to a solution of methoxy(methyl)amine hydrochloride (2.0 g, 20.0 mmol) and triethylamine (2.0 g, 20.0 mmol) in THF (20 mL) and water (20 mL). The mixture was then purged with N₂ three times. The reaction mixture was then sealed well and stirred at room temperature under N₂ protection for 2 hours. The reaction mixture was diluted with ice-water (100 mL) and extracted twice with EtOAc (100 mL). The organic phase was collected, dried over Na₂SO₄, and concentrated under vacuum to give a crude sample. The crude product was further purified by column chromatography (SiO₂, 30% EtOAc/petroleum ether) to give the product Intermediate 14 as a white solid (0.8 g, 68%): ESI MS[M + H]⁺ for C₁₄H₁₆NO₄S, calcd 294.1, found 294.1.

### Step (X): Synthesis of 1-(4-methoxy-1,2-dihydrothieno[3,2-e]benzofuran-7-yl)ethan-1-one (Intermediate 15)

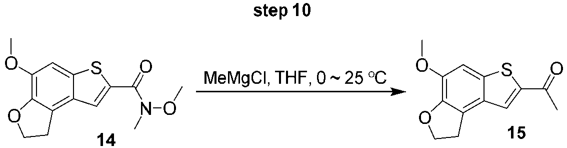

Intermediate Compound 14 (100.0 mg, 0.3 mmol) prepared in step (IX) was dissolved in THF (5 mL). The mixture was purged with N₂ three times and cooled to 0°C. Methylmagnesium chloride (76.5 mg, 1.0 mmol) was then added. The reaction mixture was stirred at room temperature under N₂ protection for 1 hour. The reaction mixture was quenched into water (100 g) and then diluted with EtOAc (100 mL). The resulting mixture was extracted with EtOAc (100 mL). The organic phase was dried over Na₂SO₄ and filtered. The filtrate was concentrated under vacuum to give a crude sample. The crude product was further purified by trituration (SiO₂, 30% EtOAc/petroleum ether) to give pure Intermediate Compound 15 as a white solid (60.0 mg, 71%): ESI MS[M + H]⁺ for C₁₃H₁₃O₃S, calcd 249.3, found 249.0.

### Step (XI): Synthesis of ethyl 4-(4-methoxy-1,2-dihydrothieno[3,2-e]benzofuran-7-yl)-4-oxobutanoate (Intermediate 17)

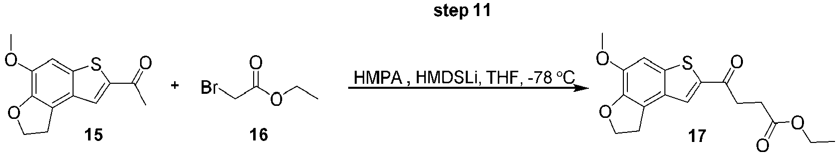

Intermediate Compound 15 (60.0 mg, 0.2 mmol) prepared in step (X) was dissolved in THF (5 mL), and [bis(dimethylamino)phosphoryl]dimethylamine (129.9 mg, 0.7 mmol) was added. The mixture was purged with N₂ three times and cooled to -78°C. The reaction mixture was then added to lithium (1+) bis(trimethylsilyl)amide (60.7 mg, 0.4 mmol) and stirred at -78°C under N₂ protection for 0.5 hour. Finally, ethyl 2-bromoacetate (60.5 mg, 0.4 mmol) was added. The reaction mixture was then sealed well and stirred at room temperature under N₂ protection for 2 hours. The reaction mixture was quenched with ice-water (100 g) and then diluted with EtOAc (100 mL). The resulting mixture was extracted with EtOAc (100 mL). The organic phase was dried over Na₂SO₄ and filtered. The filtrate was concentrated under vacuum to give the crude product Intermediate 17 as a white solid (80.0 mg, 99%): ESI MS[M + H]⁺ for C₁₇H₁₉O₅S, calcd 335.4, found 335.1, which was used in the next step without further purification.

### Step (XII): Synthesis of 4-(4-methoxy-1,2-dihydrothieno[3,2-e]benzofuran-7-yl)-4-oxobutanoic acid (Example Compound 2)

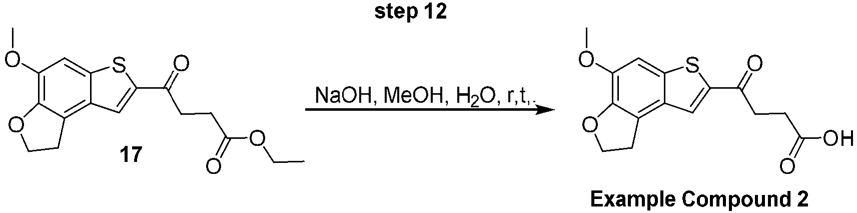

To a solution of Intermediate Compound 17 (80.0 mg, 0.2 mmol) prepared in step (XI) in methanol (5 mL) and H₂O (1 mL) was added NaOH (47.9 mg, 1.2 mmol). The reaction mixture was stirred at room temperature for 2 hours. The reaction mixture was adjusted to pH = 5-6 and diluted with water (100 mL). The mixture was extracted twice with EtOAc (100 mL). The organic phase was collected, dried over Na₂SO₄, and concentrated under vacuum to give a crude sample. The crude product was further purified by reverse-phase HPLC (C18 column, 40-60% gradient of MeCN and water containing 0.1% TFA) to give the product Example Compound 2 as a white solid (25.0 mg, 34%), ESI MS[M + H]⁺ for C₁₅H₁₅O₅S, calcd 307.33, found 307.0. ¹H NMR (400 MHz, DMSO-d6) δ 12.17 (s, 1H), 8.20 (s, 1H), 7.49 (s, 1H), 4.68 (t, *J* = 9.0 Hz, 2H), 3.86 (s, 3H), 3.46 (t, *J* = 9.0 Hz, 2H), 3.28 (t, *J* = 6.4 Hz, 2H), 2.59 (t, *J* = 6.4 Hz, 2H).

### Example 3

### Synthesis of 4-(5-methoxy-2,3-dihydrothieno[3',2':3,4]benzo[1,2-b][1,4]dioxin-8-yl)-4-oxobutanoic acid (Example Compound 3)

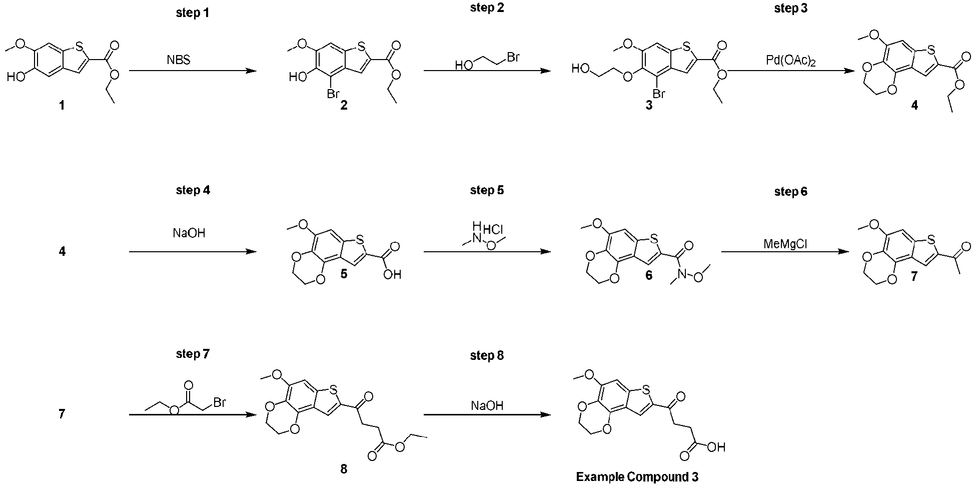

### Step (I): Synthesis of ethyl 4-bromo-5-hydroxy-6-methoxybenzo[b]thiophene-2-carboxylate (Intermediate 2)

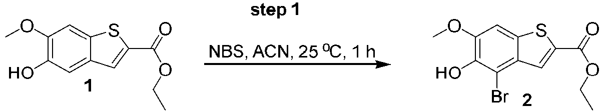

Ethyl 5-hydroxy-6-methoxybenzo[b]thiophene-2-carboxylate (3.0 g, 11.9 mmol) was dissolved in acetonitrile (50 mL) and cooled to 0°C. NBS (2539.7 mg, 14.3 mmol) was then slowly added, and the mixture was purged with N₂ three times. The reaction mixture was stirred at room temperature under N₂ protection for 1 hour. The reaction mixture was quenched into ice-water (500 g) at such a rate that its internal temperature was maintained below 20°C. The mixture was then filtered, and the filter cake was extracted with EA (500 mL). The organic phase was then washed with water (400 mL) and saturated brine (200 mL), dried over Na₂SO₄, and filtered. The filtrate was concentrated under vacuum to give a crude sample. The crude product was added to PE (100 mL). The mixture was stirred at 25°C for 1 h and then filtered. The filter cake was collected and dried under vacuum to give Intermediate 2 as a light brown solid (3.0 g, 76%).

### Step (II): Synthesis of ethyl 4-bromo-5-(2-hydroxyethoxy)-6-methoxybenzo[b]thiophene-2-carboxylate (Intermediate 3)

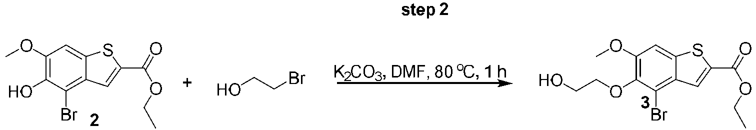

Intermediate 2 (3.3 g, 10.0 mmol) prepared in step (I) and 2-bromo-1-ol (2.5 g, 19.9 mmol) were dissolved in DMF (40 mL), and K₂CO₃ (4.1 g, 29.9 mmol) was added. The mixture was purged with N₂ three times. The reaction mixture was then sealed well and stirred at 80°C under N₂ protection for 1 hour. The reaction mixture was cooled to room temperature and filtered. The filtrate was quenched into ice-water (600 g), and the resulting mixture was extracted with EtOAc (400 mL). The organic phase was washed twice with water (200 mL), dried over Na₂SO₄, and filtered. The filtrate was concentrated under vacuum to give a crude sample. The crude product was further purified by column chromatography (SiO₂, petroleum ether:EtOAc = 5:1) to give Intermediate 3 as a yellow solid (1.8 g, 48%).

### Step (III): Synthesis of ethyl 5-methoxy-2,3-dihydrothieno[3',2':3,4]benzo[1,2-b][1,4]dioxine-8-carboxylate (Intermediate 4)

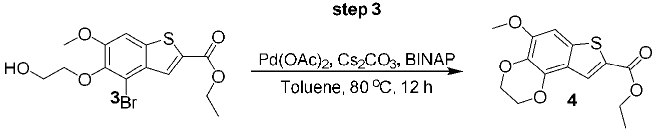

Intermediate 3 (1.8 g, 4.7 mmol) prepared in step (II), Pd(OAc)₂ (319.5 mg, 1.4 mmol), Cs₂CO₃ (4.6 g, 14.2 mmol), and BINAP (1.5 g, 2.4 mmol) were dissolved in toluene (20 mL), and the mixture was purged with N₂ three times. The reaction mixture was then sealed well and stirred at 80°C under N₂ protection for 12 hours. The reaction mixture was cooled to room temperature and filtered through celite. The filtrate was diluted with EtOAc (100 mL) and washed with water (200 mL). The organic phase was collected, dried over Na₂SO₄, and concentrated under vacuum to give a crude sample. The crude product was further purified by column chromatography (SiO₂, petroleum ether:EtOAc = 10:1) to give Intermediate 4 as a yellow solid (1.0 g, 72%).

### Step (IV): Synthesis of 5-methoxy-2,3-dihydrothieno[3',2':3,4]benzo[1,2-b][1,4]dioxine-8-carboxylic acid (Intermediate 5)

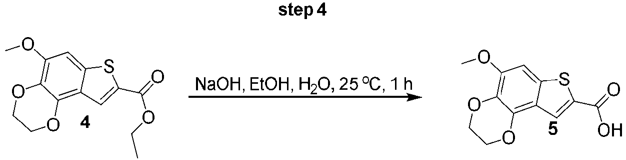

Intermediate 4 (900 mg, 3.1 mmol) prepared in step (III) was dissolved in EtOH (5 mL) and H₂O (3 mL), and NaOH (611.6 mg, 15.3 mmol) was added. The reaction mixture was then stirred at room temperature for 1 hour. The reaction mixture was adjusted to pH = 4 and diluted with water (100 mL). The mixture was extracted three times with EtOAc (100 mL). The organic phase was collected, dried over Na₂SO₄, and concentrated under vacuum to give Intermediate 5 as a yellow solid (580.0 mg, 71%).

### Step (V): Synthesis of N,5-dimethoxy-N-methyl-2,3-dihydrothieno[3',2':3,4]benzo[1,2-b][1,4]dioxine-8-carboxami de

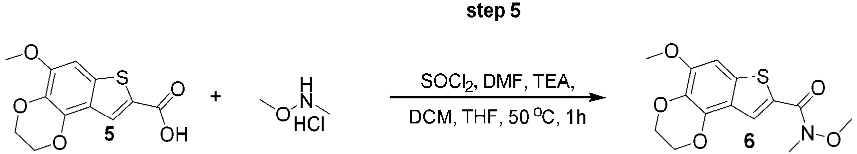

Intermediate 5 (580 mg, 2.2 mmol) prepared in step (IV) was dissolved in SOCl₂ (9.5 mL, 79.7 mmol), DMF (1.0 mL, 13.0 mmol), and DCM (10 mL) to form a solution. The reaction mixture was stirred and refluxed at 50°C for 1 hour. The reaction solution was concentrated and dissolved in tetrahydrofuran (5 ml, 20.0%). N,N-Dimethylhydroxylamine hydrochloride (1062.3 mg, 10.9 mmol), triethylamine (2204.2 mg, 21.8 mmol), and tetrahydrofuran (10 ml) were added to another 25 ml reaction flask. Acyl chloride was diluted. The reaction solution was added to the reaction flask and allowed to react at room temperature for 10 minutes. The reaction mixture was cooled to room temperature and filtered. The filtrate was diluted with EtOAc (50 mL) and washed with water (100 mL). The organic phase was dried over Na₂SO₄ and filtered. The filtrate was concentrated under vacuum to give a crude sample. The crude product was further purified by column chromatography (SiO₂, petroleum ether:EtOAc = 3:1) to give Intermediate 6 as a yellow solid (600.0 mg, 89%): ESI MS [M + H]⁺ for C₁₄H₁₅NO₅S, calcd 310.34, found 309.99. ¹H NMR (400 MHz, Chloroform-d) δ 8.17 (d, *J* = 0.8 Hz, 1H), 6.89 (s, 1H), 4.40 (dq, *J* = 3.5, 2.4, 1.7 Hz, 4H), 3.96 (s, 3H), 3.81 (s, 3H), 3.40 (s, 3H).

### Step (VI): Synthesis of 1-(5-methoxy-2,3-dihydrothieno[3',2':3,4]benzo[1,2-b][1,4]dioxin-8-yl)ethan-1-one

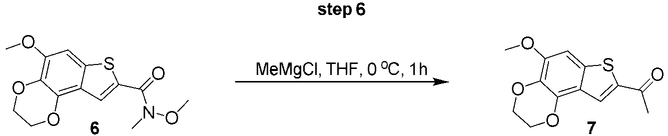

Intermediate 6 prepared in step (V) was dissolved in THF (10 mL). The solution was purged with N₂ three times and cooled to 0°C. Methylmagnesium chloride (5.4 mL, 5.4 mmol) was added to the mixture and then stirred at room temperature for 1 hour. The reaction mixture was quenched by the addition of water (50 mL) and extracted with ethyl acetate (50 mL). The organic phase was dried over Na₂SO₄ and filtered. The filtrate was concentrated under vacuum to give a crude sample. The crude product was further purified by column chromatography (SiO₂, petroleum ether:EtOAc = 5:1) to give Intermediate 7 as a white solid (80.0 mg, 17%): ESI MS [M + H]⁺ for C₁₃H₁₂N₂O₂S, calcd 261.31, found 261.0; ¹H NMR (400 MHz, Chloroform-d) δ 7.91 (d, *J* = 0.7 Hz, 1H), 6.91 (s, 1H), 4.44 - 4.39 (m, 4H), 3.97 (s, 3H), 2.62 (s, 3H).

### Step (VII): Synthesis of ethyl 4-(5-Methoxy-2,3-dihydrothieno[3',2':3,4]benzo[1,2-b][1,4]dioxin-8-yl)-4-oxobutanoate

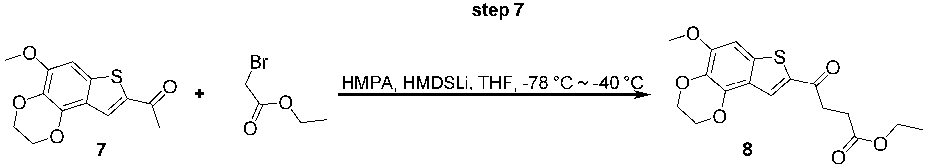

Intermediate 7 (80.0 mg, 0.3 mmol) prepared in step (VI) and HMPA (135.6 mg, 0.8 mmol) were dissolved in anhydrous THF (3 mL), and the mixture was purged with N₂ three times. The reaction mixture was cooled to -78°C under N₂ protection using an ethanol/CO₂(s) bath. HMDSLi (0.1 mL, 0.7 mmol) was then added dropwise and stirred at -40°C for 1 hour. Afterwards, the reaction mixture was cooled to -78°C, and ethyl 2-bromoacetate (75.8 mg, 0.5 mmol) was added dropwise. The mixture was maintained at this temperature for 30 minutes. The reaction mixture was quenched into water (50 g) and then diluted with EtOAc (100 mL). The resulting mixture was extracted with EtOAc (100 mL). The organic phase was then washed three times with water (50 mL), dried over Na₂SO₄, and filtered. The filtrate was concentrated under vacuum to give Intermediate Compound 8 as a yellow solid (110 mg, 83%): ESI MS[M + H]⁺ for C₁₇H₁₈O₆S, calcd 351.4, found 351.0, which was used in the next step without further purification.

### Step (VIII): Synthesis of 4-(5-methoxy-2,3-dihydrothieno[3',2':3,4]benzo[1,2-b][1,4]dioxin-8-yl)-4-oxobutanoic acid

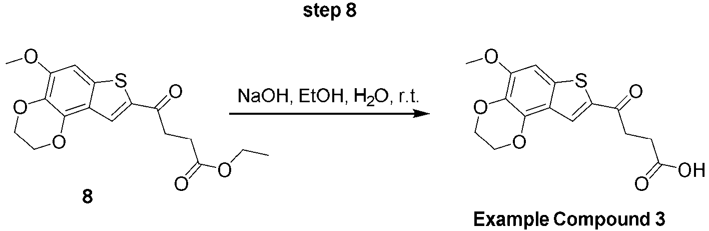

Intermediate 8 (108.6 mg, 0.3 mmol) prepared in step (VII) was dissolved in ethanol (2 mL) and H₂O (2 mL), and then NaOH (62.0 mg, 1.6 mmol) was added to the solution. The reaction mixture was stirred at room temperature for 3 h. The reaction mixture was concentrated under vacuum to give a crude sample. The crude product was further purified by reverse-phase HPLC (C18 column, 50% to 60% gradient of MeCN and water containing 0.1% FA) to give the product Example Compound 3 as a white solid (26.0 mg, 26%): ¹H NMR (400 MHz, DMSO-d6) δ 12.02 (s, 1H), 8.11 (s, 1H), 7.23 (s, 1H), 4.41 (dd, *J =* 5.4, 2.6 Hz, 2H), 4.32 (dd, *J =* 5.4, 2.6 Hz, 2H), 3.85 (s, 3H), 3.28 - 3.24 (t, *J =* 6.4 Hz, 2H), 2.56 (t, *J =* 6.4 Hz, 2H), ESI MS[M + H]⁺ for C₁₅H₁₅O₆S, calcd 323.3, found 323.0.

### Example 4

### Synthesis of 4-(2-cyclopropyl-4-methoxythieno[2',3':5,6]benzo[1,2-d]oxazol-7-yl)-4-oxobutanoic acid (Example Compound 4)

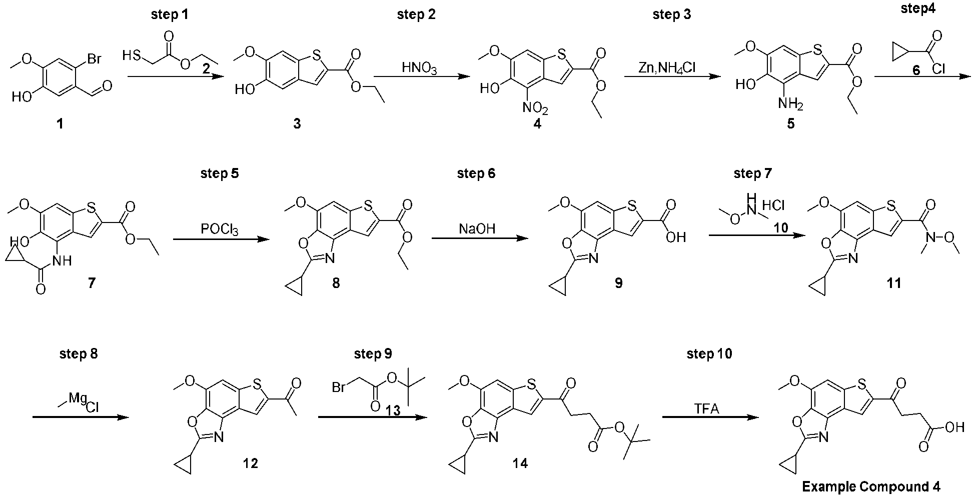

### Step (I): Synthesis of ethyl 5-hydroxy-6-methoxybenzo[b]thiophene-2-carboxylate

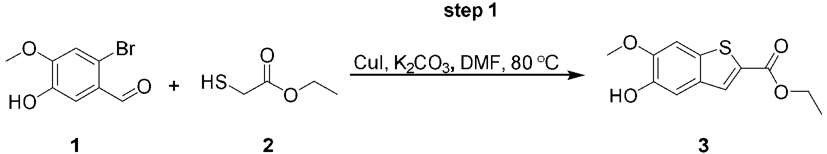

2-Bromo-5-hydroxy-4-methoxybenzaldehyde (25.0 g, 108.2 mmol) was dissolved in DMF (250 mL), and subsequently ethyl 2-mercaptoacetate (29.9 g, 248.9 mmol), CuI (6.2 g, 32.5 mmol), and K₂CO₃ (37.4 g, 270.5 mmol) were added. The reaction mixture was then sealed well and stirred at 80°C under N₂ protection for 5 hours. The reaction mixture was quenched into ice-water (1000 g) while maintaining the internal temperature below 20°C and then diluted with EtOAc (1000 mL). The resulting mixture was extracted with EtOAc (1000 mL). The organic phase was then washed twice with water (1000 mL), followed by brine (1000 mL), dried over Na₂SO₄, and filtered. The filtrate was concentrated under vacuum to give a crude sample. The crude product was further purified by column chromatography (SiO₂, Hex) to give Intermediate Compound 3 (19.4 g, 71%): ESI MS [M + H]⁺ for C₁₂H₁₂O₄S, calcd 253.3, found 253.0.

### Step (II): Synthesis of ethyl 5-hydroxy-6-methoxy-4-nitrobenzo[b]thiophene-2-carboxylate

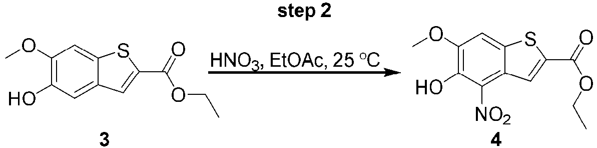

Intermediate Compound 3 (19.4 g, 76.9 mmol) prepared in step (I) was dissolved in EtOAc (200 mL) and HNO₃ (7.3 g, 115.3 mmol) and slowly mixed at 25°C. The reaction mixture was stirred at 25°C for 3 hours. The reaction mixture was quenched into water (400 g). The resulting mixture was extracted with EtOAc (200 mL). The organic phase was washed twice with water (400 mL), dried over Na₂SO₄, and filtered. The filtrate was concentrated under vacuum to give a crude sample. The crude product was further purified by column chromatography (SiO₂, Hex) to give Intermediate Compound 4 (13.2 g, 57.7%):¹ H NMR (400 MHz, DMSO-d6) δ 11.02 (s, 1H), 8.04 (s, 1H), 7.99 (s, 1H), 4.34 (q, J = 7.1 Hz, 2H), 3.98 (s, 3H), 1.33 (t, J = 7.1 Hz, 3H).

### Step (III): Synthesis of ethyl 4-amino-5-hydroxy-6-methoxybenzo[b]thiophene-2-carboxylate

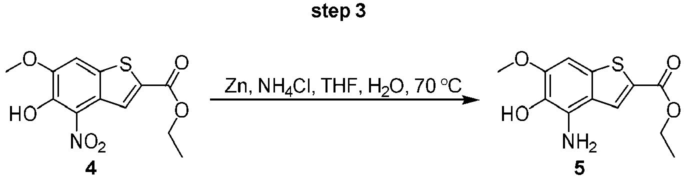

Intermediate Compound 4 (5.0 g, 16.8 mmol) prepared in step (II), zinc (5.5 g, 84.1 mmol), and NH₄Cl (4.5 g, 84.1 mmol) was dissolved in THF (100 mL) and H₂O (50 mL), and the mixture was purged with N₂ three times. The reaction mixture was then sealed well, stirred, and refluxed under N₂ protection for 1 hour. The reaction mixture was cooled to room temperature and filtered through Celite. The filter cake was washed with THF (100 mL), and the filtrate was concentrated under vacuum to give crude Intermediate Compound 5 as a yellow solid (4.2 g, 93.4%): ESI MS [M + H]⁺ for C₁₂H₁₄NO₄S, calcd 268.1, found 268.1, which was used in the next step without further purification.

### Step (IV): Synthesis of ethyl 4-(cyclopropanecarboxamido)-5-hydroxy-6-methoxybenzo[b]thiophene-2-carboxylate

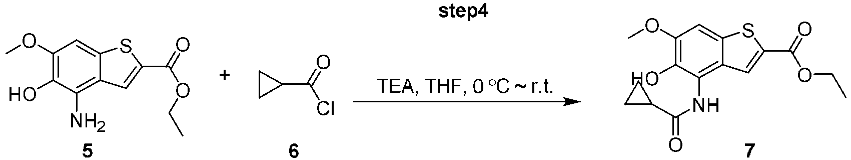

Intermediate Compound 5 prepared in step (III) was dissolved in THF (50 mL), and then TEA (4.8 g, 47.1 mmol) was added. The mixture was purged with N₂ three times. The reaction mixture was cooled to 0°C under N₂ protection using an ice bath. Cyclopropanecarbonyl chloride (1.6 g, 15.7 mmol) was then added dropwise to the reaction mixture. Afterwards, the reaction mixture was stirred at room temperature under a N₂ atmosphere for 1 hour. The reaction mixture was concentrated under vacuum while maintaining the internal temperature below 20°C, transferred into ice-water (200 g), and then diluted with EtOAc (100 mL). The resulting mixture was extracted with EtOAc (200 mL). The organic phase was then washed three times with water (100 mL), dried over Na₂SO₄, and filtered. The crude product was further purified by trituration (petroleum ether:EtOAc = 3:1) to give pure Intermediate Compound 7 as a pale yellow-brown solid (3.1 g, 58.8%): ESI MS [M + H]⁺ for C₁₆H₁₇NO₅S, calcd 336.4, found 336.0.

### Step (V): Synthesis of ethyl 2-cyclopropyl-4-methoxythieno[2',3':5,6]benzo[1,2-d]oxazole-7-carboxylate (Intermediate Compound 8)

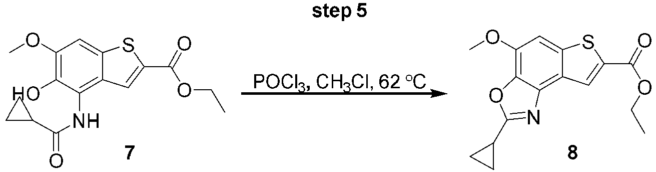

Ethyl 4-(cyclopropanecarboxamido)-5-hydroxy-6-methoxybenzo[b]thiophene-2-carboxylate (2.0 g, 6.0 mmol) was dissolved in chloroform (20 mL), and the mixture was purged with N₂ three times. Phosphorus oxychloride (4.6 g, 29.8 mmol) was then added to the reaction mixture. The reaction mixture was then stirred at 62°C under N₂ protection for 1 hour. The reaction mixture was concentrated under vacuum to give a crude sample. Ice water (50 mL) was added to the residue, and the mixture was extracted with DCM (50 mL). The organic phase was then washed with water (100 mL), dried over Na₂SO₄, and filtered. The crude product was further purified by trituration (petroleum ether:EtOAc = 2:1) to give pure Intermediate Compound 8 as a pale white solid (900 mg, 47.6%): ESI MS [M + H]⁺ for C₁₆H₅NO₄S, calcd 318.4, found 318.0.

### Step (VI): Synthesis of 2-cyclopropyl-4-methoxythieno[2',3':5,6]benzo[1,2-d]oxazole-7-carboxylic acid (Intermediate Compound 9)

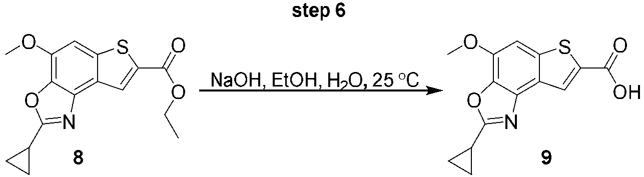

Intermediate Compound 8 (500.0 mg, 1.6 mmol) prepared in step (V) was dissolved in EtOH (20 mL) and H₂O (10 mL), and then NaOH (189.1 mg, 4.7 mmol) was added. The reaction mixture was then stirred at 27°C for 4 hours. The reaction mixture was adjusted to pH = 4 and diluted with water (100 mL). The mixture was extracted three times with EtOAc (100 mL). The organic phase was collected, dried over Na₂SO₄, and concentrated under vacuum to give Intermediate Compound 9 as a white solid (460.0 mg, 96.3%): ESI MS [M + H]⁺ for C₁₄H₁₁NO₄S, calcd 290.3, found 290.0, which was used in the next step without further purification.

### Step (VII): Synthesis of 2-cyclopropyl-N,4-dimethoxy-N-methylthieno[2',3':5,6]benzo[1,2-d]oxazole-7-carboxamide (Intermediate Compound 11)

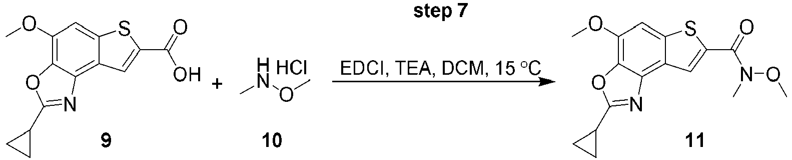

Intermediate Compound 11 (360.0 mg, 1.2 mmol) prepared in step (VI), EDCI (477.1 mg, 2.5 mmol), N,O-dimethylhydroxylamine hydrochloride (364.1 mg, 3.73 mmol), and trimethylamine (629.6 mg, 6.2 mmol) were dissolved in DCM (10 mL), and the mixture was purged with N₂ three times. The reaction mixture was stirred at 15°C under N₂ protection for 1 hour. The reaction mixture was quenched into water (100 g) and then diluted with DCM (100 mL). The resulting mixture was extracted with DCM (200 mL). The organic phase was then washed three times with water (100 mL), dried over Na₂SO₄, and filtered. The filtrate was concentrated under vacuum to give a crude sample. The crude product was further purified by trituration (petroleum ether:EtOAc = 1:1) to give pure Intermediate Compound 11 as a white solid (320 mg, 77.4%): ESI MS [M + H]⁺ for C₁₆H₁₆N₂O₄S, calcd 333.4, found 333.0.

### Step (VIII): Synthesis of 1-(2-cyclopropyl-4-methoxythieno[2',3':5,6]benzo[1,2-d]oxazol-7-yl)ethan-1-one (Intermediate Compound 12)

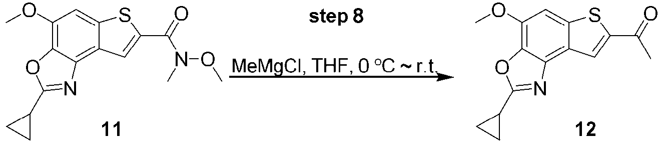

Intermediate Compound 11 (320.0 mg, 1.0 mmol) prepared in step (VII) was dissolved in THF (6 mL), and a 1.0 M solution of methylmagnesium chloride in THF (3.9 mL, 2.9 mmol) was added dropwise at 0°C. The reaction mixture was stirred at 15°C for 0.5 hour. The reaction mixture was quenched into water (50 g) and then diluted with EtOAc (100 mL). The resulting mixture was extracted with EtOAc (100 mL). The organic phase was then washed three times with water (50 mL), dried over Na₂SO₄, and filtered. The filtrate was concentrated under vacuum to give a crude sample. The crude product was further purified by trituration (petroleum ether:EtOAc = 1:1) to give pure Intermediate Compound 12 as a white solid (230.0 mg, 83.4%): ESI MS [M + H]⁺ for C₁₅H₁₃NO₃S, calcd 288.3, found 288.0.

### Step (IX): Synthesis of tert-butyl 4-(2-cyclopropyl-4-methoxythieno[2',3':5,6]benzo[1,2-d]oxazol-7-yl)-4-oxobutanoate (Intermediate Compound)

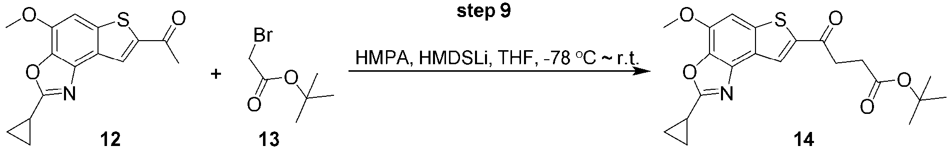

Intermediate Compound 12 (180.0 mg, 0.6 mmol) prepared in step (VIII) and HMPA (392.9 mg, 2.2 mmol) were dissolved in anhydrous THF (2 mL), and the mixture was purged with N₂ three times. The reaction mixture was cooled to -78°C under N₂ protection using an ethanol/CO₂(s) bath. Then, 1 M HMDSLi in THF (0.9 mL, 0.9 mmol) was added dropwise and stirred at -40°C for 1 hour. The reaction mixture was cooled to -78°C under N₂ protection using an ethanol/CO₂(s) bath. Afterwards, tert-butyl bromoacetate (244.4 mg, 1.25 mmol) was added dropwise to the reaction mixture and stirred at room temperature under a N₂ atmosphere for 0.5 hour. The reaction mixture was quenched into water (50 g) and then diluted with EtOAc (100 mL). The resulting mixture was extracted with EtOAc (100 mL). The organic phase was then washed three times with water (50 mL), dried over Na₂SO₄, and filtered. The filtrate was concentrated under vacuum to give a crude sample. The crude product was further purified by trituration (petroleum ether:EtOAc = 10:1) to give pure Intermediate Compound 14 as a white solid (140.0 mg, 55.4%): ESI MS [M + H]⁺ for C₂₁H₂₃NO₅S, calcd 402.5, found 402.1.

### Step (X): Synthesis of 4-(2-cyclopropyl-4-methoxythieno[2',3':5,6]benzo[1,2-d]oxazol-7-yl)-4-oxobutanoic acid (Example Compound 4)

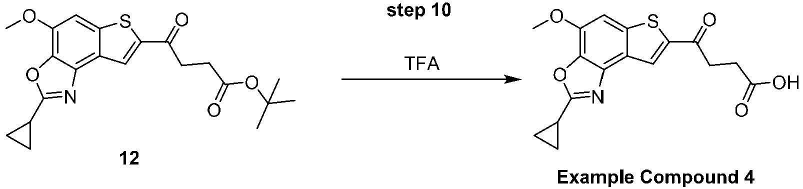

Intermediate Compound 14 (140 mg, 0.35 mmol) prepared in step (IX) was dissolved in TFA (380.1 mg, 3.5 mmol). The reaction mixture was stirred at room temperature for 1 hour. The reaction mixture was concentrated under vacuum to give a crude sample. The crude product was further purified by reverse-phase HPLC (C18 column, 50-60% gradient of MeCN and water containing 0.1% TFA) to give the product Example Compound 4 as a white solid: ESI MS [M + H]⁺ for C₁₇H₁₅NO₅S, calcd 346.4, found 346.1. ¹H NMR (400 MHz, DMSO-*d*₆) δ 12.35 (s, 1H), 8.48 (s, 1H), 7.66 (s, 1H), 4.03 (s, 3H), 3.35 (t, *J=* 6.3 Hz, 2H), 2.59 (t, *J* = 6.3 Hz, 2H), 2.38 (tt, *J=* 8.1, 5.0 Hz, 1H), 1.22 (m, 4H).

### Example 5

### Synthesis of 4-(2-isopropyl-4-methoxythieno[2',3':5,6]benzo[1,2-d]oxazol-7-yl)-4-oxobutanoic acid

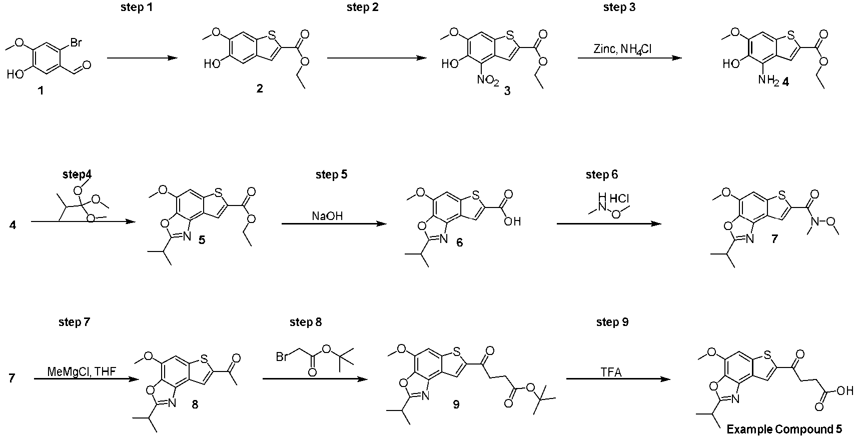

For the synthesis of ethyl 5-hydroxy-6-methoxy-4-nitrobenzo[b]thiophene-2-carboxylate (Intermediate 3), refer to the synthesis of Intermediate 3 in Example 4.

### Step (III): Synthesis of ethyl 4-amino-5-hydroxy-6-methoxybenzo[b]thiophene-2-carboxylate (Intermediate 4)

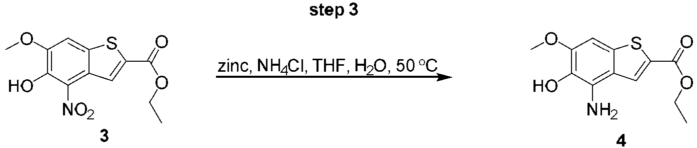

Intermediate Compound 3 (1.0 g, 3.36 mmol), zinc (1.1 g, 16.8 mmol), and NH₄Cl (0.9 g, 16.8 mmol) were dissolved in THF (10 mL) and H₂O (5 mL), and the mixture was purged with N₂ three times. The reaction mixture was then sealed well and stirred at 70°C under N₂ protection for 3 hours. The reaction mixture was cooled to room temperature and filtered through celite. The filtrate was diluted with EtOAc (50 mL) and washed with water (20 mL). The organic phase was collected, dried over Na₂SO₄, and concentrated under vacuum to give the crude product 4 as a yellow solid (850 mg, 94%): ESI MS [M + H]⁺ for C₁₂H₁₄NO₄S, calcd 268.1, found 268.1, which was used in the next step without further purification.

### Step (IV): Synthesis of ethyl 2-isopropyl-4-methoxythieno[2',3':5,6]benzo[1,2-dioxazole-7-carboxylate (Intermediate Compound 5)

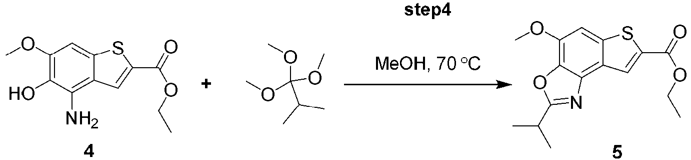

Intermediate Compound 4 (850.0 mg, 3.2 mmol) prepared in step (III) was dissolved in MeOH (10 mL) and 1,1,1-trimethoxy-2-methylpropane (2.36 g, 15.9 mmol). The reaction mixture was then stirred at 65°C under N₂ protection for 12 hours. The reaction mixture was concentrated under vacuum to give a crude sample. Water (200 mL) was added to the residue, and the mixture was extracted three times with EtOAc (200 mL). The organic phase was collected, dried over Na₂SO₄, and filtered. The filtrate was concentrated under vacuum to give a crude sample. The crude product was further purified by column chromatography (petroleum ether:EtOAc = 20:1) to give Intermediate Compound 5 as a yellow solid (680 mg, 66%): ESI MS [M + H]⁺ for C₁₆H₁₇NO₄S, calcd 320.1, found 319.9. ¹H NMR (400 MHz, Chloroform-d) δ 8.44 (d, *J* = 0.8 Hz, 1H), 7.21 (s, 1H), 4.40 (q, *J* = 7.1 Hz, 2H), 4.09 (s, 3H), 3.35 (p, *J* = 7.0 Hz, 1H), 1.52 (s, 3H), 1.50 (s, 3H), 1.41 (t, *J* = 7.1 Hz, 3H).

### Step (V): Synthesis of 2-isopropyl-4-methoxythieno[2',3':5,6]benzo[1,2-d]oxazole-7-carboxylic acid (Intermediate 6)

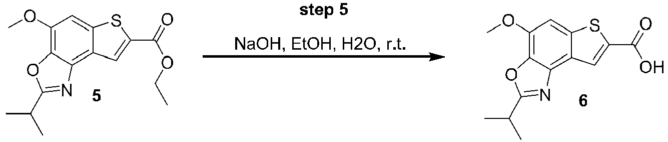

To a solution of Intermediate Compound 5 (580.0 mg, 1.8 mmol) prepared in step (IV) in EtOH (10 mL) and H₂O (10 mL) was added NaOH (290.5 mg, 7.3 mmol). The reaction mixture was then stirred at room temperature for 2 hours. The reaction mixture was adjusted to pH = 4 and diluted with water (100 mL). The mixture was extracted three times with EtOAc (100 mL). The organic phase was collected, dried over Na₂SO₄, and concentrated under vacuum to give Intermediate Compound 6 as a yellow solid (500.0 mg, 94%): ESI MS [M + H]⁺ for C₁₄H₁₄NO₄S, calcd 292.1, found 292.0, which was used in the next step without further purification.

### Step (VI): Synthesis of 2-isopropyl-N,4-dimethoxy-N-methylthieno[2',3':5,6]benzo[1,2-d]oxazole-7-carboxamide (Intermediate 7)

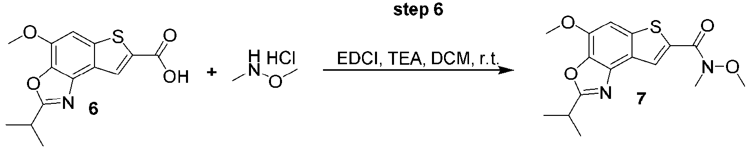

Intermediate Compound 6 (500.0 mg, 1.7 mmol) prepared in step (V), EDCI (658.0 mg, 3.4 mmol), N,N-dimethylhydroxylamine hydrochloride (502.2 mg, 5.2 mmol), and trimethylamine (868.4 mg, 8.6 mmol) were dissolved in DCM (10 mL), and the mixture was purged with N₂ three times. The reaction mixture was stirred at room temperature under N₂ protection for 12 hours. The reaction mixture was quenched into water (100 g) and then diluted with EtOAc (100 mL). The resulting mixture was extracted with EtOAc (200 mL). The organic phase was then washed three times with water (100 mL), dried over Na₂SO₄, and filtered. The filtrate was concentrated under vacuum to give a crude sample. The crude product was further purified by trituration (petroleum ether:EtOAc = 5:1) to give pure Intermediate Compound 7 as a white solid (470 mg, 82%): ESI MS [M + H]⁺ for C₁₆H₁₉N₂O₄S, calcd 335.1, found 335.1. ¹H NMR (400 MHz, Chloroform-d) δ 8.56 (s, 1H), 7.21 (s, 1H), 4.08 (s, 3H), 3.85 (s, 3H), 3.42 (s, 3H), 3.36 (p, *J =* 7.0 Hz, 1H), 1.51 (d, *J* = 7.0 Hz, 7H).

### Step (VII): Synthesis of 2-isopropyl-N,4-dimethoxy-N-methylthieno[2',3':5,6]benzo[1,2-d]oxazole-7-carboxamide (Intermediate 8)

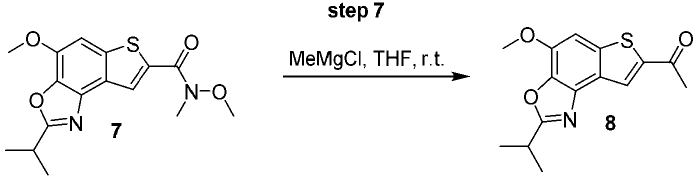

Intermediate Compound 7 (370.0 mg, 1.11 mmol) prepared in step (VI) was dissolved in THF (5 mL), and a 1.0 M solution of methylmagnesium chloride in THF (0.25 mL, 3.33 mmol) was added dropwise at 0°C. The reaction mixture was stirred at room temperature for 1 hour. The reaction mixture was quenched into water (50 g) and then diluted with EtOAc (100 mL). The resulting mixture was extracted with EtOAc (100 mL). The organic phase was then washed three times with water (50 mL), dried over Na₂SO₄, and filtered. The filtrate was concentrated under vacuum to give a crude sample. The crude product was further purified by trituration (petroleum ether:EtOAc = 10:1) to give pure Intermediate Compound 8 as a white solid (150.0 mg, 47%): ¹H NMR (400 MHz, Chloroform-d) δ 8.33 (s, 1H), 7.22 (s, 1H), 4.09 (s, 3H), 3.36 (p, *J* = 7.0 Hz, 1H), 2.67 (s, 3H), 1.52 (d, *J =* 7.0 Hz, 6H).

### Step (VIII): Synthesis of tert-butyl 4-(2-isopropyl-4-methoxythieno[2',3' :5,6]benzo [1,2-d]oxazol-7-yl)-4-oxobutanoate (Intermediate 9)

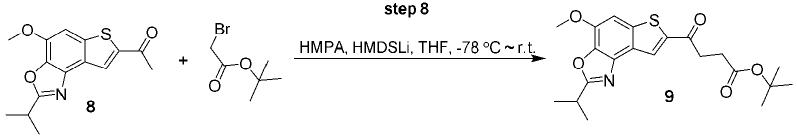

Intermediate Compound 8 (50.0 mg, 0.2 mmol) prepared in step (VII) and HMPA (92.9 mg, 0.5 mmol) were dissolved in anhydrous THF (5 mL), and the mixture was purged with N₂ three times. The reaction mixture was cooled to -78°C under N₂ protection. Then, 1M HMDSLi in THF (0.26 mL, 1.6 mmol) was added dropwise and stirred at -78°C for 1 hour. Afterwards, tert-butyl bromoacetate (67.4 mg, 0.4 mmol) was added to the reaction mixture and stirred at room temperature under a N₂ atmosphere for 0.5 hour. The reaction mixture was quenched into water (50 g) and then diluted with EtOAc (100 mL). The resulting mixture was extracted with EtOAc (100 mL). The organic phase was then washed three times with water (50 mL), dried over Na₂SO₄, and filtered. The filtrate was concentrated under vacuum to give a crude sample, Intermediate Compound 9, as a yellow solid (50.0 mg, 73%): ESI MS [M + H]⁺ for C₂₁H₂₅NO₅S, calcd 404.2, found 348.0, which was used in the next step without further purification.

### Step (IX): Synthesis of 4-(2-isopropyl-4-methoxythieno[2',3':5,6]benzo[1,2-d]oxazol-7-yl)-4-oxobutanoic acid (Example Compound 5)

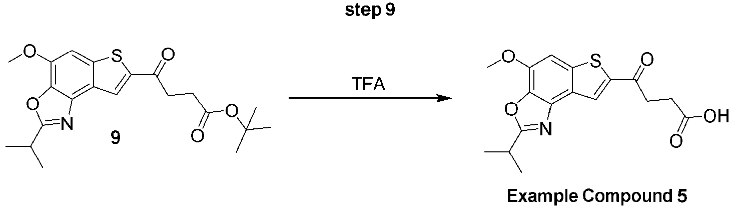

Intermediate Compound 9 (50.0 mg, 0.12 mmol) prepared in step (VIII) was dissolved in TFA (2 mL, 17.54 mmol). The reaction mixture was stirred at room temperature for 1 hour. The reaction mixture was concentrated under vacuum to give a crude sample. The crude product was further purified by reverse-phase HPLC (C18 column, 50-60% gradient of MeCN and water containing 0.1% TFA) to give the product Example Compound 5 as a white solid (16 mg, 38%): ESI MS [M + H]⁺ for C₁₇H₁₈NO₅S, calcd 348.1, found 348.0. ¹H NMR (400 MHz, DMSO-d6) δ 12.13 (s, 1H), 8.55 (s, 1H), 7.71 (s, 1H), 4.05 (s, 3H), 3.37 (td, *J* = 6.5, 3.4 Hz, 3H), 2.60 (t, *J* = 6.3 Hz, 2H), 1.44 (s, 3H), 1.42 (s, 3H).

### Example 6

### Synthesis of 4-(4-methoxy-2-methylthieno[2',3':5,6]benzo[1,2-d]oxazol-7-yl)-4-oxobutanoic acid (Example Compound 6)

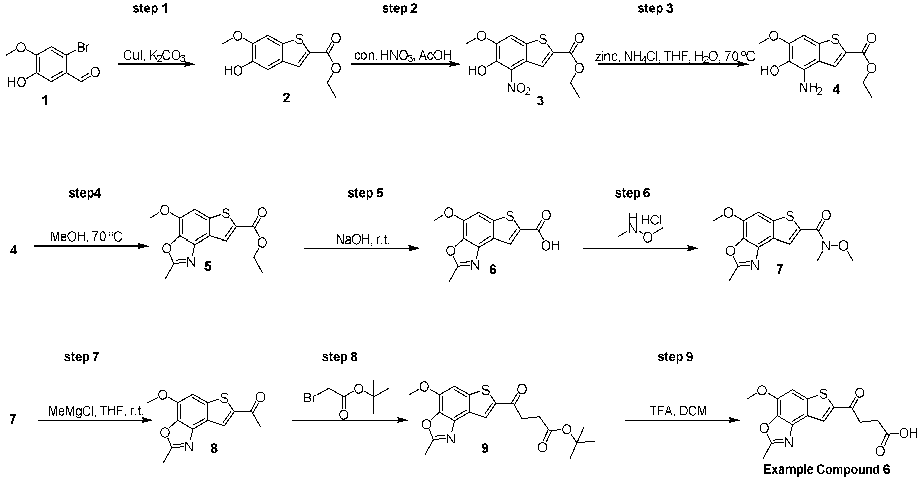

For the synthesis of ethyl 5-hydroxy-6-methoxy-4-nitrobenzo[b]thiophene-2-carboxylate (Intermediate 3), refer to the synthesis of Intermediate 3 in Example 4.

### Step (III): Synthesis of ethyl 4-amino-5-hydroxy-6-methoxybenzo[b]thiophene-2-carboxylate (Intermediate Compound 4)

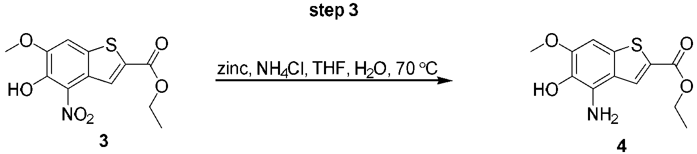

Intermediate Compound 3 (2.0 g, 6.7 mmol), zinc (2.2 g, 33.7 mmol), and NH₄Cl (1.8 g, 33.7 mmol) were dissolved in THF (40 mL) and H₂O (20 mL), and the mixture was purged with N₂ three times. The reaction mixture was then sealed well and stirred at 70°C under N₂ protection for 3 hours. The reaction mixture was cooled to room temperature and filtered through celite. The filtrate was diluted with EtOAc (200 mL) and washed with water (50 mL). The organic phase was collected, dried over Na₂SO₄, and concentrated under vacuum to give the crude product 4 as a yellow solid (1.8 g, 80%): ESI MS [M + H]⁺ for C₁₂H₁₄NO₄S, calcd 268.1, found 268.0, which was used in the next step without further purification.

### Step (IV): Synthesis of ethyl 4-methoxy-2-methylthieno[2',3':5,6]benzo[1,2-d]oxazole-7-carboxylate (Intermediate Compound 5)

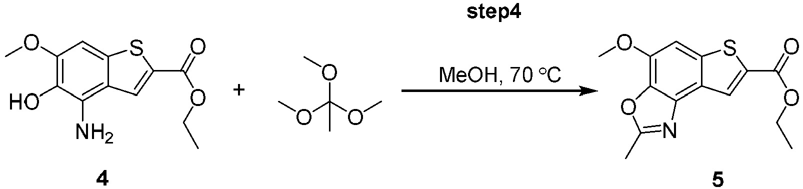

Intermediate Compound 4 (1.8 g, 6.7 mmol) prepared in step (III) was dissolved in MeOH (36 mL) and 1,1,1-trimethoxyethane (5.0 g, 33.7 mmol). The reaction mixture was then stirred at 70°C under N₂ protection for 16 hours. The reaction mixture was concentrated under vacuum to give a crude sample. Water (100 mL) was added to the residue, and the mixture was extracted three times with EtOAc (100 mL). The organic phase was collected, dried over Na₂SO₄, and filtered. The filtrate was concentrated under vacuum to give a crude sample. The crude product was further purified by column chromatography (SiO₂, petroleum ether:EtOAc = 3:1) to give the product Intermediate Compound 5 as a yellow solid (1.49 g, 76%): ESI MS [M + H]⁺ for C₁₄H₁₃NO₄S, calcd 292.1, found 292.0. ¹H NMR (400 MHz, CDCl₃) δ 8.40 (s, 1H), 7.23 (s, 1H), 4.43 (q, *J =* 7.1 Hz, 2H), 4.11 (s, 3H), 2.75 (s, 3H), 1.44 (t, *J =* 7.1 Hz, 3H).

### Step (V): Synthesis of 4-methoxy-2-methylthieno[2',3':5,6]benzo[1,2-d]oxazole-7-carboxylic acid (Intermediate Compound 6)

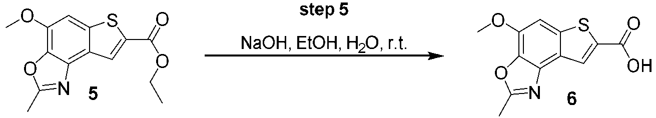

To a solution of Intermediate Compound 5 prepared in step (IV) in EtOH (40 mL) and H₂O (20 mL) was added NaOH (1.0 g, 25.6 mmol). The reaction mixture was then stirred at room temperature for 1 hour. The reaction mixture was adjusted to pH = 4 and diluted with water (100 mL). The mixture was extracted three times with EtOAc (100 mL). The organic phase was collected, dried over Na₂SO₄, and concentrated under vacuum to give Intermediate Compound 6 as a white solid (1.3 g, 97%): ESI MS [M + H]⁺ for C₁₂H₁₀NO₄S, calcd 264.0, found 264.0, which was used in the next step without further purification.

### Step (VI): Synthesis of N,4-dimethoxy-N,2-dimethylthieno[2',3':5,6]benzo[1,2-d]oxazole-7-carboxamide (Intermediate Compound 7)

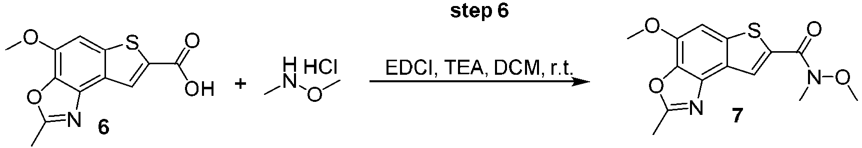

Intermediate Compound 6 (1.3 g, 5.1 mmol) prepared in step (V), EDCI (2.0 g, 10.3 mmol), N,O-dimethylhydroxylamine hydrochloride (1.5 g, 15.4 mmol), and trimethylamine (2.6 g, 25.7 mmol) were dissolved in DCM (20 mL), and the mixture was purged with N₂ three times. The reaction mixture was stirred at room temperature under N₂ protection for 2 hours. The reaction mixture was quenched into water (20 g) and then diluted with DCM (50 mL). The resulting mixture was extracted with DCM (50 mL). The organic phase was then washed three times with water (20 mL), dried over Na₂SO₄, and filtered. The filtrate was concentrated under vacuum to give a crude sample. The crude product was further purified by trituration (dichloromethane:EtOAc = 20:1) to give the pure title Intermediate Compound 7 as a white solid (700.0 mg, 45%): ESI MS [M + H]⁺ for C₁₄H₁₅N₂O₄S, calcd 307.1, found 307.0. ¹H NMR (400 MHz, CDCl₃) δ 8.54 (s, 1H), 7.24 (s, 1H), 4.11 (s, 3H), 3.87 (s, 3H), 3.45 (s, 3H), 2.76 (s, 3H).

### Step (VII): Synthesis of 1-(4-methoxy-2-methylthieno[2',3':5,6]benzo[1,2-d]oxazol-7-yl)ethyl-1-one (Intermediate Compound 8)

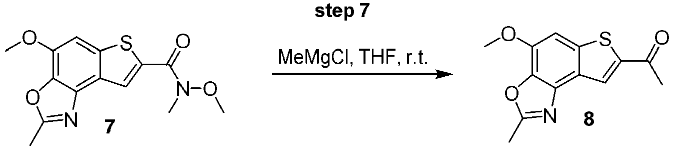

Intermediate Compound 7 (600.0 mg, 2.0 mmol) prepared in step (VI) was dissolved in THF (12 mL), and a 1.0 M solution of methylmagnesium chloride in THF (6.0 mL, 6.0 mmol) was added dropwise at 0°C. The reaction mixture was stirred at room temperature for 1 hour. The reaction mixture was quenched into water (10 g) and then diluted with EtOAc (20 mL). The resulting mixture was extracted with EtOAc (20 mL). The organic phase was washed three times with water (10 mL), dried over Na₂SO₄, and filtered. The filtrate was concentrated under vacuum to give a crude sample. The crude product was further purified by trituration (dichloromethane:EtOAc = 20:1) to give the pure title Intermediate Compound 8 as a white solid (370.0 mg, 72%): ESI MS [M + H]⁺ for C₁₃H₁₂NO₃S, calcd 262.1, found 262.0. ¹H NMR (400 MHz, CDCl₃) δ 8.30 (s, 1H), 7.25 (s, 1H), 4.12 (s, 3H), 2.77 (s, 3H), 2.69 (s, 3H).

### Step (VIII): Synthesis of tert-butyl 4-(4-methoxy-2-methylthieno[2',3':5,6]benzo[1,2-d]oxazol-7-yl)-4-oxobutanoate (Intermediate Compound 9)

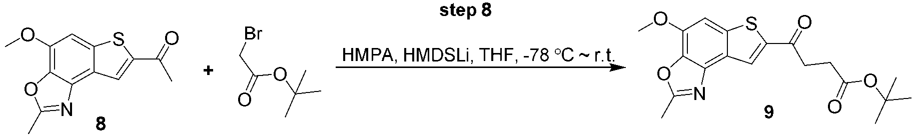

Intermediate Compound 8 (100.0 mg, 0.4 mg) prepared in step (VII) and HMPA (205.7 mg, 1.2 mg) were dissolved in anhydrous THF (3 mL), and the mixture was purged with N₂ three times. The reaction mixture was cooled to -78°C under N₂ protection. Then, 1M HMDSLi in THF (0.6 mL, 0.6 mmol) was added dropwise and stirred at -78°C for 30 minutes. Afterwards, tert-butyl bromoacetate (149.3 mg, 0.8 mg) was added to the reaction mixture and stirred at room temperature under a N₂ atmosphere for 0.5 hour. The reaction mixture was quenched into water (20 g) and then diluted with EtOAc (50 mL). The resulting mixture was extracted with EtOAc (50 mL). The organic phase was then washed three times with water (20 mL), dried over Na₂SO₄, and filtered. The filtrate was concentrated under vacuum to give crude Intermediate Compound 9 as a white solid (143.7 mg, 100%): ESI MS [M + H]⁺ for C₁₉H₂₂NO₅S, calcd 376.1, found 378.1, which was used in the next step without further purification.

### Step (IX): Synthesis of 4-(4-methoxy-2-methylthieno[2',3':5,6]benzo[1,2-d]oxazol-7-yl)-4-oxobutanoic acid

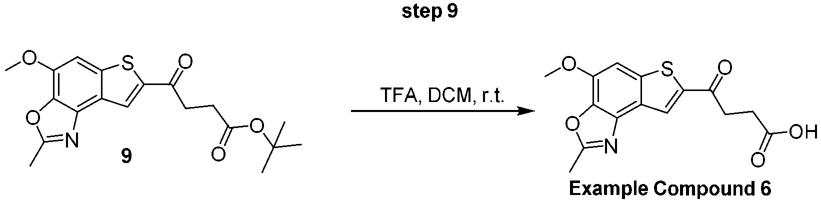

Intermediate Compound 9 (143.7 mg, 0.4 mg) prepared in step (VIII) and trifluoroacetic acid (1 mL, 8.8 mg) were dissolved in DCM (2 mL). The reaction mixture was stirred at room temperature for 3 hours. The reaction mixture was concentrated under vacuum to give a crude sample. The crude product was further purified by reverse-phase HPLC (C18 column, 30-50% gradient of MeCN and water containing 0.1% TFA) to give the product Example Compound 6 as a yellow solid (6.0 mg, 4.9%): ESI MS [M + H]⁺ for C₁₅H₁₄NO₅S, calcd 320.1, found 320.0. ¹H NMR (400 MHz, DMSO-*d*₆) δ 12.19 (s, 1H), 8.50 (s, 1H), 7.69 (s, 1H), 4.04 (s, 3H), 3.38 - 3.34 (t, *J=* 6.4, 2H), 2.70 (s, 3H), 2.61 (t, *J=* 6.4 Hz, 2H).

### Example 7

### Synthesis of 4-(2-ethyl-4-methoxythieno[2',3':5,6]benzo[1,2-d]oxazol-7-yl)-4-oxobutanoic acid (Example Compound 7)

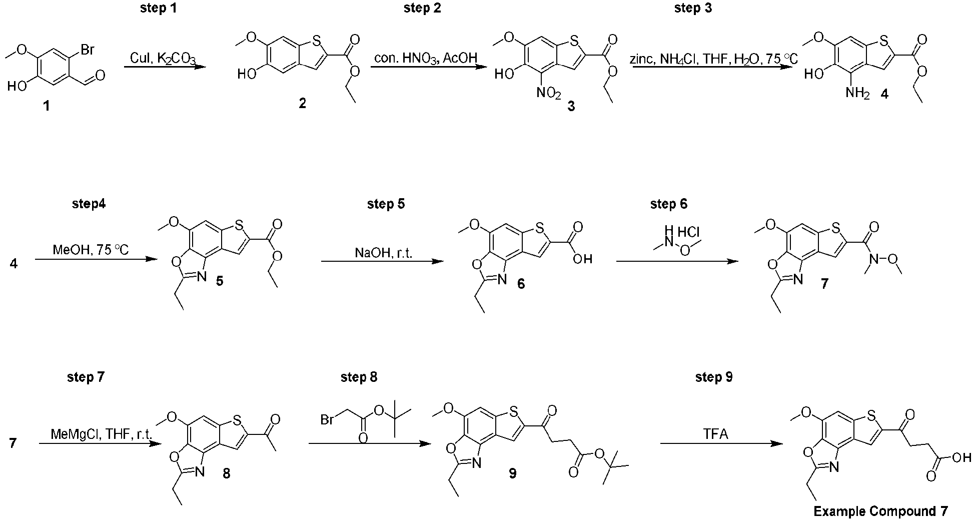

For the synthesis of ethyl 5-hydroxy-6-methoxy-4-nitrobenzo[b]thiophene-2-carboxylate (Intermediate 3), refer to the synthesis of Intermediate 3 in Example 4.

### Step (III): Synthesis of ethyl 4-amino-5-hydroxy-6-methoxybenzo[b]thiophene-2-carboxylate (Intermediate Compound 4)

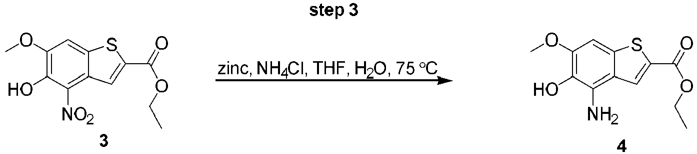

Intermediate Compound 3 (1.0 g, 3.7 mmol), zinc (1.1 g, 16.8 mmol), and NH₄Cl (0.9 g, 16.8 mmol) were dissolved in THF (8 mL) and H₂O (4 mL), and the mixture was purged with N₂ three times. The reaction mixture was then sealed well and stirred at 75°C under N₂ protection for 1 hour. The reaction mixture was cooled to room temperature and filtered through celite. The filtrate was diluted with EtOAc (50 mL) and washed with water (30 mL). The organic phase was collected, dried over Na₂SO₄, and concentrated under vacuum to give the crude product 4 as a grey solid (400 mg, 45%): ESI MS [M + H]⁺ for C₁₂H₁₄NO₄S, calcd 268.1, found 268.0.

### Step (IV): Synthesis of ethyl 2-ethyl-4-methoxythieno[2',3':5,6]benzo[1,2-d]oxazole-7-carboxylate (Intermediate Compound 5)

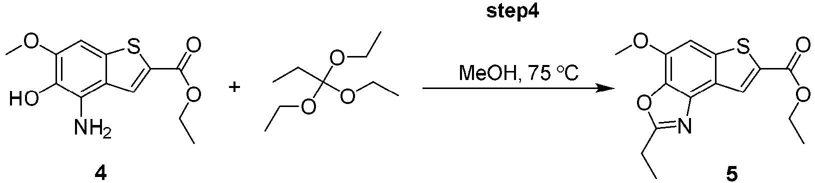

Intermediate Compound 4 (400.0 mg, 1.5 mmol) prepared in step (III) was dissolved in MeOH (20 mL) and triethyl orthopropionate (1.0 g, 7.5 mmol). The reaction mixture was then stirred at 75°C under N₂ protection for 12 hours. The reaction mixture was concentrated under vacuum to give a crude sample. Water (50 mL) was added to the residue, and the mixture was extracted three times with EtOAc (50 mL). The organic phase was collected, dried over Na₂SO₄, and filtered. The filtrate was concentrated under vacuum to give a crude sample. The crude product was further purified by column chromatography (SiO₂, petroleum ether:EtOAc = 10:1) to give the product Intermediate Compound 5 as a white solid (300.0 mg, 65%): ESI MS [M + H]⁺ for C₁₅H₁₆NO₄S, calcd 306.1, found 306.0.

### Step (V): Synthesis of 2-ethyl-4-methoxythieno[2',3':5,6]benzo[1,2-d]oxazole-7-carboxylic acid (Intermediate Compound 6)

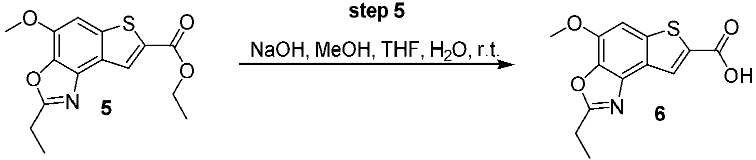

Intermediate Compound 5 (700.0 mg, 2.3 mmol) prepared in step (IV) was dissolved in MeOH (5 mL) and H₂O (5 mL), and NaOH (500.0 mg, 12.5 mmol) was added. The reaction mixture was then stirred at room temperature for 4 hours. The reaction mixture was concentrated under vacuum and adjusted to pH = 4. The residue was washed with water (20 mL) and filtered to give a filter cake. The filter cake was dried to give the title Intermediate Compound 6 as a white solid (500.0 mg, 79%): ESI MS [M + H]⁺ for C₁₃H₁₂NO₄S, calcd 278.0, found 278.0, ¹H NMR (400 MHz, DMSO-d6) δ 13.45 (s, 1H), 8.11 (s, 1H), 7.70 (s, 1H), 4.03 (s, 3H), 3.04 (q, *J* = 7.6 Hz, 2H), 1.39 (t, *J* = 7.6 Hz, 3H).

### Step (VI): Synthesis of 2-ethyl-N,4-dimethoxy-N-methylthieno[2',3':5,6]benzo[1,2-d]oxazole-7-carboxamide (Intermediate Compound 7)

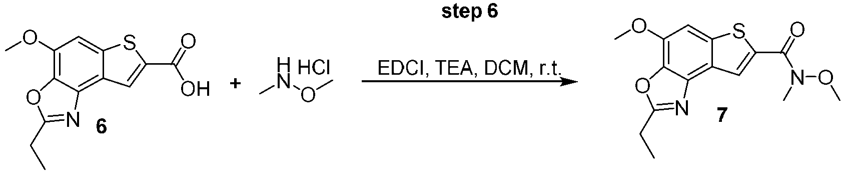

Intermediate Compound 6 (500.0 mg, 1.8 mmol) prepared in step (V), EDCI (691.3 mg, 3.6 mmol), N,O-dimethylhydroxylamine hydrochloride (527.6 mg, 5.4 mmol), and trimethylamine (912.3 mg, 9.0 mmol) were dissolved in DCM (4 mL), and the mixture was purged with N₂ three times. The reaction mixture was stirred at room temperature under N₂ protection for 3 hours. The reaction mixture was quenched into water (50 mL) and then diluted with EtOAc (50 mL). The resulting mixture was extracted with EtOAc (200 mL). The organic phase was then washed three times with water (100 mL), dried over Na₂SO₄, and filtered. The filtrate was concentrated under vacuum to give a crude sample. The crude product was further purified by trituration (petroleum ether:EtOAc = 3:1) to give the pure title Intermediate Compound 7 as a pale yellow oil (300.0 mg, 52%): ESI MS [M + H]⁺ for C₁₅H₁₈N₂O₄S, calcd 321.1, found 321.0.

### Step (VII): Synthesis of 1-(2-ethyl-4-methoxythieno[2',3':5,6]benzo[1,2-d]oxazol-7-yl)ethan-1-one

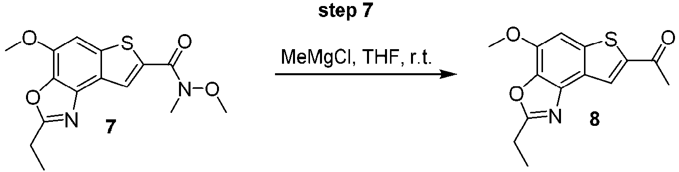

Intermediate Compound 7 (300.0 mg, 0.9 mmol) prepared in step (VI) was dissolved in THF (5 mL), and a 3.0 M solution of methylmagnesium chloride in THF (0.9 mL, 2.7 mmol) was added dropwise at 0°C. The reaction mixture was stirred at room temperature for 30 minutes. The reaction mixture was quenched into water (50 mL) and then diluted with EtOAc (100 mL). The resulting mixture was extracted with EtOAc (100 mL). The organic phase was then washed three times with water (50 mL), dried over Na₂SO₄, and filtered. The filtrate was concentrated under vacuum to give a crude sample. The crude product was further purified by trituration (petroleum ether:EtOAc = 5:1) to give the pure title Intermediate Compound 8 as a white solid (180.0 mg, 70%): ESI MS [M + H]⁺ for C₁₄H₁₄NO₃S, calcd 276.1, found 278.0.

### Step (VIII): Synthesis of tert-butyl 4-(2-ethyl-4-methoxythieno[2',3':5,6]benzo[1,2-d]oxazol-7-yl)-4-oxobutanoate

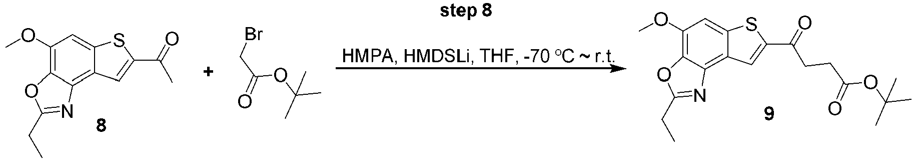

Intermediate Compound 8 (120.0 mg, 0.4 mmol) prepared in step (VII) and HMPA (273.3 mg, 1.5 mmol) were dissolved in anhydrous THF (2 mL), and the mixture was purged with N₂ three times. The reaction mixture was cooled to -70°C under N₂ protection. Then, 1M HMDSLi in THF (1.7 mL, 1.7 mmol) was added dropwise and stirred at -70°C for 1 hour. Afterwards, tert-butyl bromoacetate (255.0 mg, 1.3 mmol) was added to the reaction mixture and stirred at room temperature under a N₂ atmosphere for 10 minutes. The reaction mixture was quenched into water (50 mL) and then diluted with EtOAc (100 mL). The resulting mixture was extracted with EtOAc (100 mL). The organic phase was then washed three times with water (50 mL), dried over Na₂SO₄, and filtered. The filtrate was concentrated under vacuum to give a crude sample. The crude product was further purified by trituration (petroleum ether:EtOAc = 3:1) to give the pure title Intermediate Compound 9 as a yellow solid (25.0 mg, 15%): ESI MS [M + H]⁺ for C₂₀H₂₄NO₅S, calcd 390.1, found 334.0.

### Step (IX): Synthesis of 4-(2-ethyl-4-methoxythieno[2',3':5,6]benzo[1,2-d]oxazol-7-yl)-4-oxobutanoic acid (Example Compound 7)

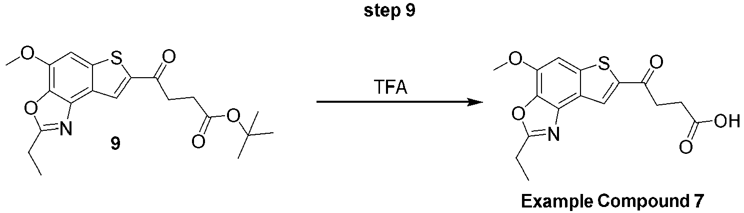

Intermediate Compound 9 (30.0 mg, 0.08 mmol) prepared in step (VIII) was dissolved in TFA (2.0 mL, 17.5 mmol). The reaction mixture was stirred at room temperature for 10 minutes. The reaction mixture was concentrated under vacuum to give a crude sample. The crude product was further purified by reverse-phase HPLC (C18 column, 45-50% gradient of MeCN and water containing 0.1% TFA) to give the product Example Compound 7 as a white solid (2.2 mg, 13%): ESI MS [M + H]⁺ for C₁₆H₁₆NO₅S, calcd 334.1, found 334.0. ¹H NMR (400 MHz, Methanol-*d*₄) δ 8.43 (s, 1H), 7.50 (s, 1H), 4.08 (s, 3H), 3.40 (t, *J* = 6.4 Hz, 2H), 3.08 (q, *J* = 7.6 Hz, 2H), 2.75 (t, *J =* 6.4 Hz, 2H), 1.50 (t, *J* = 7.6 Hz, 3H).

### Example 8

### Synthesis of 4-(4-methoxy-8-methylthieno[2,3:5,6]benzo[1,2-d]oxazol-7-yl)-4-oxobutanoic acid

### Example 9

### Synthesis of 4-(2-amino-4-methoxy-8-methylthieno[2',3':5,6]benzo[1,2-d]oxazol-7-yl)-4-oxobutanoic acid

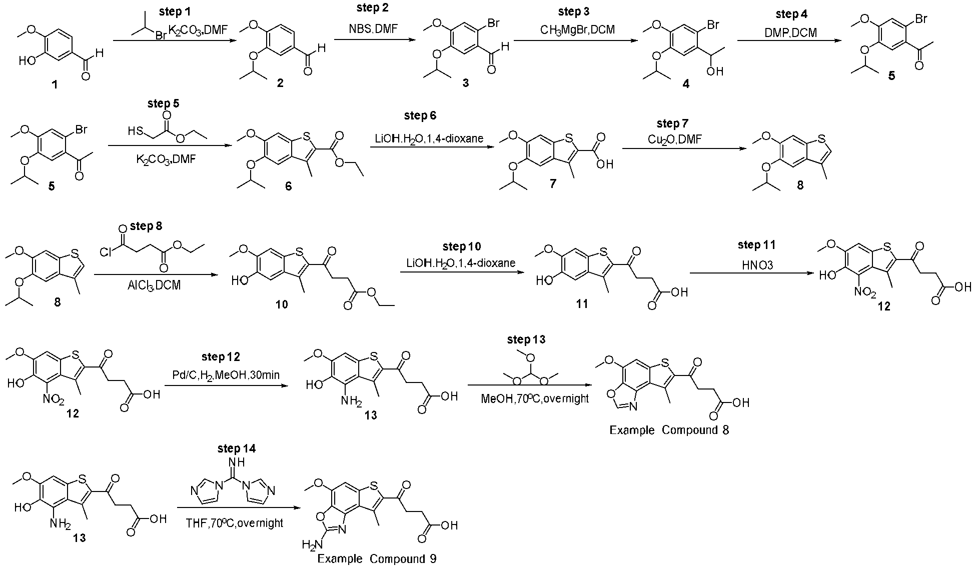

### Step (I): Synthesis of 3-isopropoxy-4-methoxybenzaldehyde (Intermediate 2)

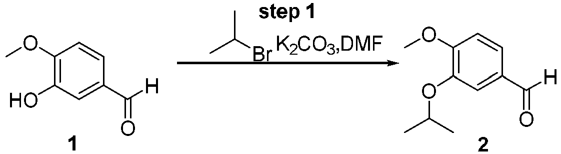

Compound 1 (25 g, 164.31 mmol, 1.0 eq) was added to a dry 500 mL single-necked flask under nitrogen protection, dissolved in 125 mL of DMF, and placed in an ice bath. Potassium carbonate (6.4 g, 262.9 mmol, 1.6 eq) was added, followed by 2-bromopropane (30.3 g, 246.3 mmol, 1.5 eq), and the mixture was reacted at 30°C for 24 h. A sample was collected for TLC, which indicated that the starting material had not completely reacted. An additional 2-bromopropane (10.1 g, 82.1 mmol, 0.5 eq) was added, and the mixture was reacted at 30°C for 24 h. A sample was collected for TLC, which indicated that the starting material had completely reacted. The reaction mixture was filtered, concentrated to remove DMF, diluted with EA, washed with water and brine, and dried over anhydrous sodium sulfate. TLC indicated that the product was pure. The product was concentrated and dried under vacuum to give 30.3 g of a light orange-yellow oil, ESI MS[M + H]⁺ for C₁₁H₁₅O₃, calcd 195.2, found: 195.2 (M+1), which was used directly in the next step. For the second batch with 25 g, 28.5 g of Intermediate Compound 2 as a light orange-yellow oil was obtained and used directly in the next step.

### Step (II): Synthesis of 2-bromo-5-isopropoxy-4-methoxybenzaldehyde (Intermediate 3)

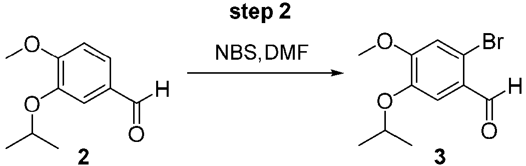

Intermediate Compound 2 (48.5 g, 249.7 mmol, 1.0 eq) prepared in the previous step was added to a dry 500 mL three-necked flask under nitrogen protection and dissolved in 100 mL of anhydrous DMF. NBS (66.66 g, 347.56 mmol, 1.5 eq) was added, and the mixture was reacted at 80°C for 3 h. TLC indicated that the starting material had completely reacted. The reaction was quenched by the addition of water, and the reaction mixture was extracted with EA. The EA phase was washed with water, 5% Na₂S₂O₄ (200 mL), and then brine, dried over anhydrous magnesium sulfate, filtered, concentrated, mixed with an adsorbent, and purified on a silica gel column using PE/EA as the mobile phase and 5% EA to give a product. The product was concentrated to give 56 g of Intermediate Compound 3 as a white solid, ESI MS[M + H]⁺ for C₁₁H₁₄BrO₃, calcd 273.1, found: 273.1 (M+1).

### Step (III): Synthesis of 1-(2-bromo-5-isopropoxy-4-methoxyphenyl)ethan-1-ol (Intermediate 4)

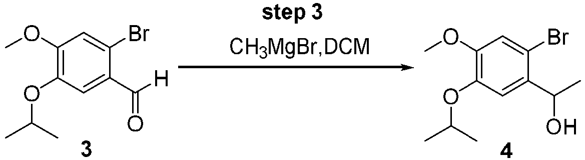

Intermediate Compound 3 (56 g, 205.04 mmol, 1.0 eq) prepared in the previous step was placed under a nitrogen atmosphere, dissolved in 500 mL of anhydrous THF, and placed in an ice bath. Methylmagnesium bromide (206 mL, 615.12 mmol, 3.0 eq) (3.0M/L in 2-methyl tetrahydrofuran) was injected, and the mixture was reacted at 0°C for 2 h. A sample was collected for TLC. After completion of the reaction, the reaction was quenched by the slow dropwise addition of water. The reaction mixture was dissolved in EA, washed with water and brine, dried over anhydrous sodium sulfate, concentrated, and purified on a silica gel column using PE/EA as the mobile phase and 20% EA to give a product. The product was concentrated and dried under vacuum to give 54.22 g of Intermediate Compound 4 as a light yellow oil.

### Step (IV): Synthesis of 1-(2-bromo-5-isopropoxy-4-methoxyphenyl)ethan-1-one (Intermediate 5)

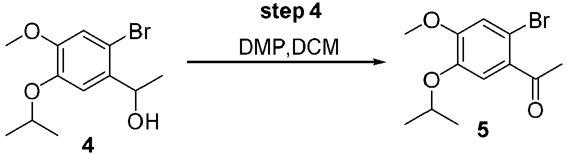

Intermediate Compound 4 (54.22 g, 187.5 mmol, 1.0 eq) prepared in the previous step was placed under a nitrogen atmosphere, dissolved in 500 mL of anhydrous DCM, and placed in an ice bath. DMP (87.5 g, 206.2 mmol, 1.0 eq) was added, and the mixture was reacted at 0°C for 2 h. TLC indicated that the starting material had completely reacted. The reaction mixture was mixed directly with an adsorbent and purified on a silica gel column using PE/EA as the mobile phase and 13%-16% EA to give a product. The product was concentrated to give 30.22 g of Intermediate Compound 5 as a white solid, which was used directly in the next step, ESI MS[M + H]⁺ for C₁₂H₁₆BrO₃, calcd 287.1, found: 287.1 (M+1).

### Step (V): Synthesis of ethyl 5-isopropoxy-6-methoxy-3-methylbenzo[b]thiophene-2-carboxylate (Intermediate 6)

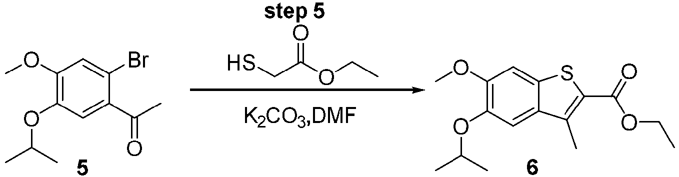

Intermediate Compound 5 (11.27 g, 35.77 mmol, 1.0 eq) prepared in the previous step was added to a dry sealed tube under nitrogen protection and dissolved in 100 mL of anhydrous DMF. Potassium carbonate (14.8 g, 107.3 mmol, 3.0 eq) and ethyl thioglycolate (6.45 g, 53.65 mmol, 1.0 eq) were added, and the mixture was reacted at 90°C overnight. The next day, a yellow solution was obtained. The reaction was quenched by the addition of water. The reaction mixture was diluted with EA, washed with water and brine, dried over anhydrous sodium sulfate, and concentrated. TLC indicated a new spot. The concentrate was purified on a silica gel column using PE/EA as the mobile phase and 4%-6% EA to give a product. The product was concentrated to give 4.55 g of Intermediate Compound 6 as a yellow solid, which was not very pure and was used directly in the subsequent hydrolysis reaction.

### Step (VI): Synthesis of 5-isopropoxy-6-methoxy-3-methylbenzo[b]thiophene-2-carboxylic acid (Intermediate 7)

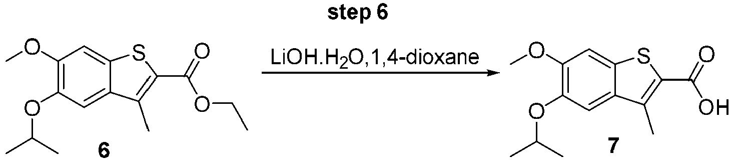

Intermediate Compound 6 (impure) (4.55 g, 8.86 mmol, 1.0 eq) prepared in the previous step was dissolved in 30 mL of 1,4-dioxane. Subsequently, 10 mL of water was added, followed by lithium hydroxide monohydrate (3.72 g, 88.6 mmol, 1.0 eq), and the mixture was reacted at 30°C overnight. The next day, a sample was collected for TLC, which indicated no starting material. The reaction mixture was directly worked up, concentrated to remove the organic solvent, diluted with water, and extracted with EA. The EA phase was washed with water. The aqueous phases were combined and adjusted to pH 4 with IN HCl. A large amount of white solid precipitated. The solid was filtered, washed with water, dissolved in methanol, concentrated, and dried under vacuum to give 2.0 g of Intermediate Compound 7 as a white solid, ESI MS[M + H]⁺ for C₁₄H₁₇O₄S, calcd 281.2, found: 281.2 (M+1).

### Step (VII): Synthesis of 5-isopropoxy-6-methoxy-3-methylbenzothiophene (Intermediate 8)

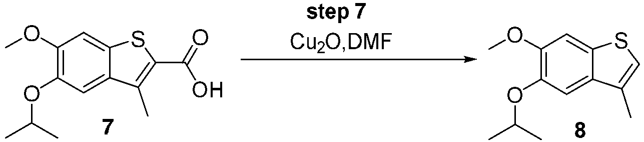

Intermediate Compound 7 (2 g, 7.14 mmol, 1.0 eq) prepared in the previous step was placed under a nitrogen atmosphere and dissolved in 20 mL of anhydrous DMF. Cuprous oxide (4 g, 28.57 mmol, 4.0 eq) was added, and the mixture was reacted at 140°C overnight. TLC indicated that the starting material had completely reacted. The reaction mixture was filtered, washed with EA, concentrated, and purified on a silica gel column using PE/EA as the mobile phase and 0% P-5% EA to give a product. The product was concentrated to give 1.22 g of a colorless oil, which turned to a white solid, Intermediate Compound 8, upon refrigeration, ¹H NMR (400 MHz, CDCl₃) δ 7.32 (s, 1H), 7.19 (s, 1H), 6.93 (d, J = 1.0 Hz, 1H), 4.62 (hept, J = 6.1 Hz, 1H), 3.94 (s, 3H), 2.40 (d, J = 1.0 Hz, 3H), 1.44 (s, 3H), 1.43 (s, 3H).

### Step (VIII): Synthesis of ethyl 4-(5-hydroxy-6-methoxy-3-methylbenzo[b]thiophen-2-yl)-4-oxobutanoate (Intermediate 9)

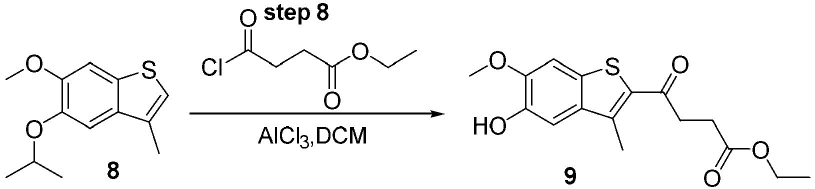

Intermediate Compound 8 (540 mg, 2.28 mmol, 1.0 eq) prepared in the previous step was placed under a nitrogen atmosphere, dissolved in 10 mL of anhydrous DCM, and placed in an ice bath. Acyl chloride (750 mg, 4.56 mmol, 2.0 eq) was added dropwise, followed by aluminum trichloride (610 mg, 4.56 mmol, 2.0 eq), and the mixture was reacted in an ice bath for 30 min and at room temperature (15°C) for 3 h. An additional aluminum trichloride (610 mg, 4.56 mmol, 2.0 eq) was added, and the mixture was reacted at room temperature (15°C) overnight. The next day, TLC indicated that the starting material had completely reacted, with a single new clean spot observed. The reaction was quenched by the addition of water, and the reaction mixture was extracted with EA. The EA phase was washed with water and brine, dried over anhydrous sodium sulfate, concentrated, and purified on a silica gel column using PE/EA as the mobile phase and 30% EA to give a product. The product was concentrated to give 700 mg of Intermediate Compound 9 as a white solid, ESI MS[M + H]⁺ for C₁₆H₁₉O₅S, calcd 323.1, found: 451.3 (also an acyl chloride on the hydroxyl group), 323.2 (M+1), which indicated a single spot on TLC and was used directly in the next step.

### Step (IX): Synthesis of 4-(5-hydroxy-6-methoxy-3-methylbenzothiophen-2-yl)-4-oxobutanoic acid (Intermediate 10)

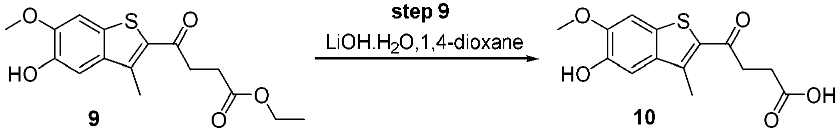

Intermediate Compound 9 (700 mg, 2.17 mmol, 1.0 eq) prepared in the previous step was dissolved in 50 mL of 1,4-dioxane. Subsequently, 30 mL of water was added, followed by lithium hydroxide monohydrate (911 mg, 21.7 mmol, 10.0 eq), and the mixture was reacted at 35°C for 5 h. A sample was collected for TLC, which indicated that the starting material had completely reacted. The reaction mixture was concentrated to remove the organic solvent, diluted with water, and adjusted to pH 4 with IN HCl. A large amount of white solid precipitated. The solid was filtered, washed with water, dissolved in methanol, concentrated, and dried under vacuum to give 600 mg of Intermediate Compound 10 as a white solid, ESI MS[M + H]⁺ for C₁₄H₁₅O₅S, calcd 295.1, found: 295.1 (M+1).

### Step (X): Synthesis of 4-(5-hydroxy-6-methoxy-3-methyl-4-nitrobenzo[b]thiophen-2-yl)-4-oxobutanoic acid (Intermediate 11)

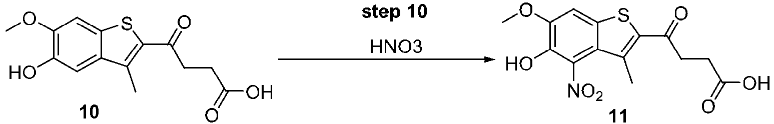

Intermediate Compound 10 (600 mg, 2.04 mmol, 1.0 eq) prepared in the previous step was dissolved in 90 mL of EA, which was not completely dissolved, resulting in a white suspension. The suspension was placed in an ice bath, and 30 drops of concentrated nitric acid were added dropwise. The mixture was slowly warmed to room temperature for 1 h, forming a light pink suspension. MS monitoring indicated that the reaction was not complete. After 2 h, the reaction was just completed. The reaction mixture was diluted with EA, washed with ice water and brine, dried over anhydrous magnesium sulfate, and concentrated at low temperature to give a red solid. The solid was mixed directly with an adsorbent and loaded onto a column using PE/EA as the mobile phase and 60% EA to give 100 mg of Intermediate Compound 11 as a yellow solid, ESI MS[M + H]⁺ for C₁₄H₁₄NO₇S, calcd 340.1, found: 340.1 (M+1), which was used directly in the next step.

### Step (XI): Synthesis of 4-(4-amino-5-hydroxy-6-methoxy-3-methylbenzo[b]thiophen-2-yl)-4-oxobutanoic acid (Intermediate 12)

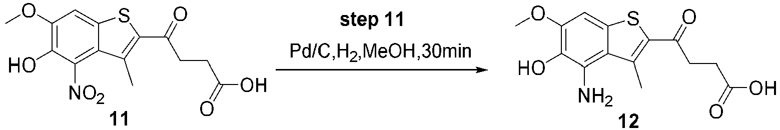

Intermediate Compound 11 (100 mg, 0.28 mmol, 1.0 eq), as the nitration product from the previous step, was dissolved in 30 mL of methanol, and 40 mg of palladium on carbon was added. The mixture was subjected to catalytic hydrogenation at 25°C under a hydrogen atmosphere for 35 min. A sample was collected for TLC, which indicated that the starting material had completely reacted. The reaction mixture was filtered, concentrated at low temperature, and dried under vacuum to give 87 mg of Intermediate Compound 12 as a yellow solid, ESI MS[M + H]⁺ for C₁₄H₁₆NO₅S, calcd 310.1, found: 310.1 (M+1), which was used directly in the next step.

### Step (XII): Synthesis of 4-(4-methoxy-8-methylthieno[2,3:5,6]benzo[1,2-d]oxazol-7-yl)-4-oxobutanoic acid (Example Compound 8)

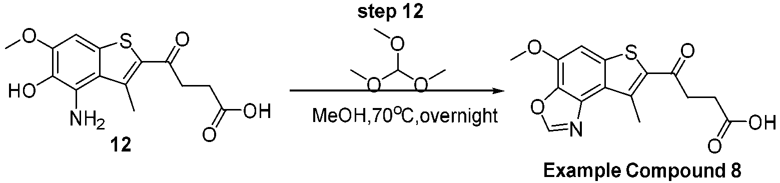

Intermediate Compound 12 (60 mg, 0.19 mmol, 1.0 eq) prepared in the previous step was dissolved in 3 mL of methanol and placed under a nitrogen atmosphere. Trimethoxymethane (31 mg, 0.97 mmol, 5 eq) was added, and the mixture was subjected to microwave reaction for 3 h. The reaction mixture was directly worked up, cooled, concentrated, and subjected to preparative purification (column: C18, 10 µm, 20 * 250 mm; mobile phase: water (containing 0.1% formic acid)/acetonitrile; procedure: 10% acetonitrile for 5 min, gradient from 10% to 90% acetonitrile over 30 min, 90% acetonitrile for 5 min, flow rate: 12 mL/min, 70% acetonitrile) to give a product. The product was lyophilized to give 4.4 mg of Example 8 as a yellow solid, ESI MS[M + H]⁺ for C₁₅H₁₄NO₅S, calcd 320.1, found: 320.0, ¹H NMR (400 MHz, DMSO) δ 12.19 (s, 1H), 8.91 (s, 1H), 7.72 (s, 1H), 4.06 (s, 3H), 3.25 - 3.20 (m, 2H), 3.09 (s, 3H), 2.61 (t, J = 6.2 Hz, 2H).

### Step (XIII): Synthesis of 4-(2-amino-4-methoxy-8-methylthieno[2',3':5,6]benzo[1,2-d]oxazol-7-yl)-4-oxobutanoic acid (Example Compound 9)

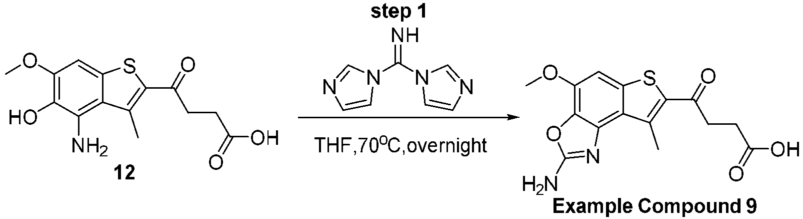

Intermediate Compound 12 (27 mg, 0.087 mmol, 1.0 eq) from the synthesis of Example Compound 8 was weighed and placed under a nitrogen atmosphere. Di(1-imidazolyl)methanimine (98 mg, 0.611 mmol, 7 eq) was added, and the mixture was reacted at room temperature for 2 h and at 70°C in a sealed tube overnight. The next day, the reaction mixture was cooled, concentrated, dissolved in DMF, filtered, and purified on a preparative column (column: C18, 10 µm, 30 * 250 mm; mobile phase: water (containing 0.1% formic acid)/acetonitrile; procedure: 10% acetonitrile for 5 min, gradient from 10% to 90% acetonitrile over 30 min, 90% acetonitrile for 5 min, flow rate: 20 mL/min, 57% acetonitrile) to give a product. The product was lyophilized to give 7 mg of Example Compound 9 as a yellow solid, ESI MS[M + H]⁺ for C₁₅H₁₅N₂O₅S, calcd 335.1, found: 335.1; ¹H NMR (400 MHz, DMSO) δ 12.16 (s, 1H), 7.69 (s, 2H), 7.29 (s, 1H), 3.97 (s, 3H), 3.17 (t, J = 6.2 Hz, 2H), 3.01 (s, 3H), 2.59 (t, J = 6.2 Hz, 2H).

### Example 10

### Synthesis of 4-(4-methoxy-8-methylthieno[2,3:5,6]benzo[1,2-d]oxazol-7-yl)-2-methyl-4-oxobutanoic acid (Example Compound 10)

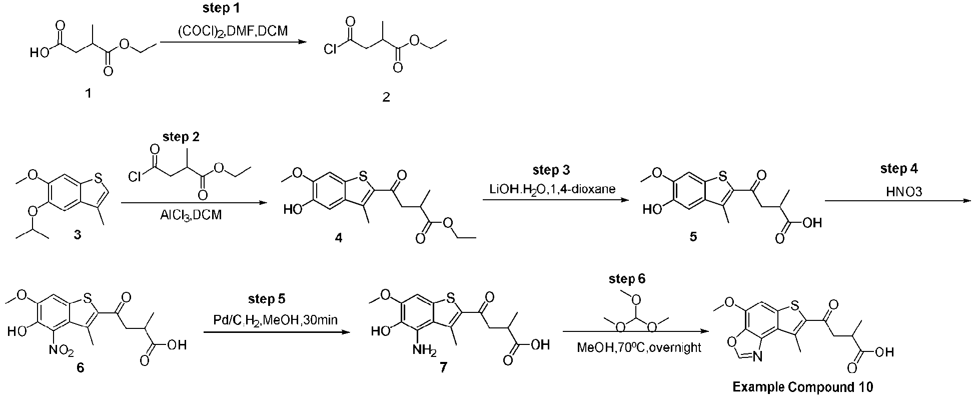

### Step (I): Synthesis of ethyl 4-chloro-2-methyl-4-oxobutanoate (Intermediate 2)

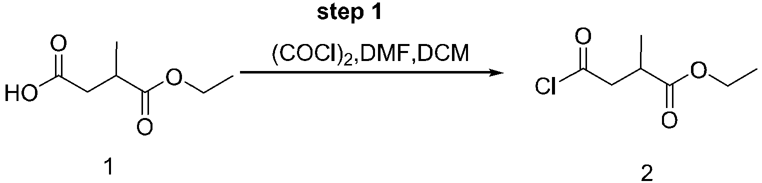

Starting material Compound 1 (760 mg, 4.18 mmol, 1.0 eq) was placed under a nitrogen atmosphere, dissolved in 10 mL of anhydrous DCM, and placed in an ice bath. Oxalyl chloride (2.5 mL, 5.02 mmol, 1.2 eq) (2.0M/L in DCM) was added dropwise, followed by 1 drop of anhydrous DMF. The mixture was placed in an ice bath for 30 min and then slowly warmed to room temperature for 2 h. The reaction mixture was concentrated to remove the solvent and excess oxalyl chloride and placed under a nitrogen atmosphere to give Intermediate Compound 2, which was used directly in the next step.

### Step (II): Synthesis of ethyl 4-(5-hydroxy-6-methoxy-3-methylbenzo[b]thiophen-2-yl)-2-methyl-4-oxobutanoate (Intermediate 4)

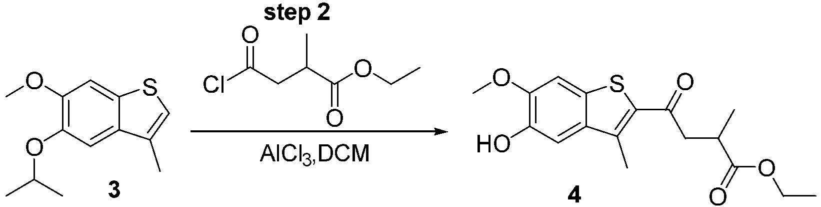

Intermediate Compound 8 (760 mg, 3.22 mmol, 1.0 eq) from the preparation of Example Compound 8 was placed under a nitrogen atmosphere, dissolved in 5 mL of anhydrous DCM, and placed in an ice bath. A solution of Intermediate Compound 2 prepared in step (I) in 5 mL of anhydrous DCM was added dropwise, followed by aluminum trichloride (860 mg, 6.44 mmol, 2.0 eq), and the mixture was reacted in an ice bath for 30 min and at room temperature for 3 h. An additional aluminum trichloride (860 mg, 6.44 mmol, 2.0 eq) was added, and the mixture was reacted at room temperature overnight. The next day, TLC indicated that the starting material had completely reacted, with a single new clean spot observed. The reaction was quenched by the addition of water, and the reaction mixture was extracted with EA. The EA phase was washed with water and brine, dried over anhydrous sodium sulfate, concentrated, and purified on a silica gel column using PE/EA as the mobile phase and 30% EA to give a product, which indicated two spots with close polarity on TLC. The product was concentrated to give 800 mg of Intermediate Compound 4 as a white solid, ESI MS[M + H]⁺ for C₁₇H₂₀O₅S, calcd 337.1, found: 479.6 (also an acyl chloride on the hydroxyl group), 337.1 (M+1), which was used directly in the next step.

### Step (III): Synthesis of 4-(5-hydroxy-6-methoxy-3-methylbenzo[b]thiophen-2-yl)-2-methyl-4-oxobutanoic acid (Intermediate 5)

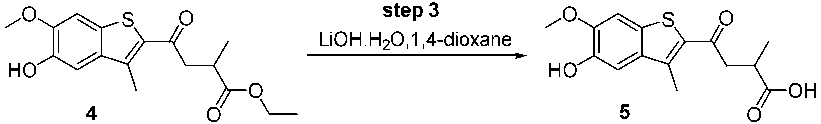

Intermediate Compound 14 (800 mg, 2.38 mmol, 1.0 eq) prepared in step (II) was dissolved in 50 mL of 1,4-dioxane. Subsequently, 30 mL of water was added, followed by lithium hydroxide monohydrate (1.0 g, 23.8 mmol, 10.0 eq), and the mixture was reacted at 35°C for 5 h. A sample was collected for TLC, which indicated that the starting material had completely reacted. The reaction mixture was concentrated to remove the organic solvent, diluted with water, and adjusted to pH 4 with IN HCl. A large amount of white solid precipitated. The solid was filtered, washed with water, dissolved in methanol, concentrated, and dried under vacuum to give 600 mg of Intermediate Compound 5 as a white solid, ESI MS[M + H]⁺ for C₁₅H₁₆O₅S, calcd 309.1, found: 309.3 (M+1).

### Step (IV): Synthesis of 4-(5-hydroxy-6-methoxy-3-methyl-4-nitrobenzothiophen-2-yl)-2-methyl-4-oxobutanoic acid (Intermediate 6)

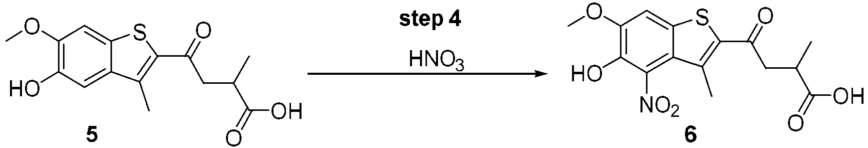

Intermediate Compound 15 (600 mg, 1.94 mmol, 1.0 eq) prepared in the previous step was dissolved in 90 mL of EA, which was not completely dissolved, resulting in a white suspension. The suspension was placed in an ice bath, and 30 drops of concentrated nitric acid were added dropwise. The mixture was slowly warmed to room temperature for 1 h. TLC indicated that the starting material had completely reacted. The reaction mixture was diluted with EA, washed with ice water and brine, dried over anhydrous magnesium sulfate, and concentrated at low temperature to give a red solid. The solid was mixed directly with an adsorbent and loaded onto a column using PE/EA as the mobile phase and 60% EA to give 100 mg of Intermediate Compound 6 as a yellow solid, ESI MS[M + H]⁺ for C₁₅H₁₆NO₇S, calcd 354.1, found: 354.2 (M+1), which was used directly in the next step.

### Step (V): Synthesis of 4-(4-amino-5-hydroxy-6-methoxy-3-methylbenzo[b]thiophen-2-yl)-2-methyl-4-oxobutanoic acid (Intermediate 7)

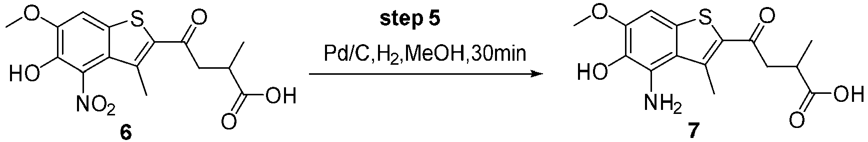

Intermediate Compound 16 (100 mg, 0.28 mmol, 1.0 eq), as the nitration product from the previous step, was dissolved in 30 mL of methanol, and 40 mg of palladium on carbon was added. The mixture was subjected to catalytic hydrogenation at room temperature under a hydrogen atmosphere for 35 min. A sample was collected for TLC, which indicated that the starting material had completely reacted. The reaction mixture was filtered, concentrated at low temperature, and dried under vacuum to give 65 mg of Intermediate Compound 7 as a yellow solid, ESI MS[M + H]⁺ for C₁₅H₁₈NO₅S, calcd 324.1, found: 324.2 (M+1), which was used directly in the next step.

### Step (VI): Synthesis of 4-(4-methoxy-8-methylthieno[2,3:5,6]benzo[1,2-d]oxazol-7-yl)-2-methyl-4-oxobutanoic acid (Example Compound 10)

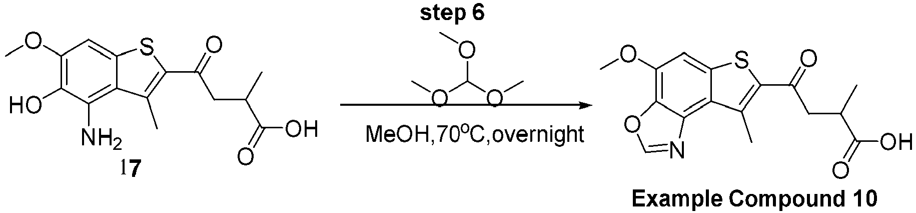

Intermediate Compound 17 (36 mg, 0.11 mmol, 1.0 eq) prepared in the previous step was weighed, placed under a nitrogen atmosphere, and dissolved in 6 mL of methanol. Trimethoxymethane (118 mg, 1.1 mmol, 10 eq) was added, and the mixture was reacted in a sealed vessel for 6 h. A sample was collected for LC-MS monitoring, which indicated that the starting material had completely reacted. The reaction mixture was cooled, concentered, dissolved in DMF, filtered, and purified on a preparative column (column: C18, 10 µm, 20 * 250 mm; mobile phase: water (containing 0.1% formic acid)/acetonitrile; procedure: 10% acetonitrile for 5 min, gradient from 10% to 90% acetonitrile over 30 min, 90% acetonitrile for 5 min, flow rate: 12 mL/min, 70% acetonitrile) to give a product. The product was lyophilized to give 5.7 mg of Example Compound 10 as a yellow solid, ESI MS[M + H]⁺ for C₁₆H₁₆NO₅S, calcd 334.3, found: 334.2; ¹H NMR (400 MHz, DMSO) δ 8.91 (s, 1H), 7.72 (d, J = 8.6 Hz, 1H), 4.06 (s, 3H), 3.32 (dd, J = 17.5, 8.3 Hz, 1H), 3.08 (d, J = 5.3 Hz, 3H), 3.07 (d, J = 2.8 Hz, 1H), 2.91 (dd, J = 12.5, 7.7 Hz, 1H), 1.19 (t, J = 6.3 Hz, 3H).

### Example 11

### Synthesis of 4-(2-amino-4-methoxy-8-methylthieno[2',3':5,6]benzo[1,2-d]oxazol-7-yl)-2-methyl-4-oxobut anoic acid (Example Compound 11)

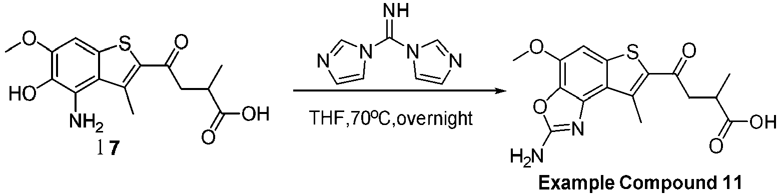

Intermediate Compound 17 (27 mg, 0.084 mmol, 1.0 eq) from the preparation of Example Compound 10 was placed under a nitrogen atmosphere and dissolved in 2 mL of anhydrous THF. Di(1-imidazolyl)methanimine (27 mg, 0.167 mmol, 2 eq) was added, and the mixture was reacted at room temperature for 2 h and at 70°C in a sealed tube overnight. The next day, the reaction mixture was cooled, concentrated, subjected to preparative purification, concentrated, dissolved in DMF, and purified on a preparative column (column: C18, 10 µm, 20 * 250 mm; mobile phase: water (containing 0.1% formic acid)/acetonitrile; procedure: 10% acetonitrile for 5 min, gradient from 10% to 90% acetonitrile over 30 min, 90% acetonitrile for 5 min, flow rate: 12 mL/min, 58% acetonitrile) to give a product. The product was lyophilized to give 7 mg of Example Compound 11 as a yellow solid, ESI MS[M + H]⁺ for C₁₆H₁₇N₂O₅S, calcd 349.1, found: 349.1; ¹H NMR (400 MHz, DMSO) δ 12.17 (s, 1H), 7.69 (s, 2H), 7.30 (d, *J* = 8.7 Hz, 1H), 3.97 (s, 3H), 3.30 - 3.22 (m, 1H), 3.00 (d, *J* = 8.8 Hz, 3H), 2.95 - 2.85 (m, 1H), 2.75 (dd, *J* = 16.9, 9.0 Hz, 1H), 1.18 (t, *J =* 6.7 Hz, 3H)).

### Example 12

### Synthesis of 4-(4-methoxythieno[2,3:5,6]benzo[1,2-d]oxazol-7-yl)-2-methyl-4-oxobutanoic acid (Example Compound 12)

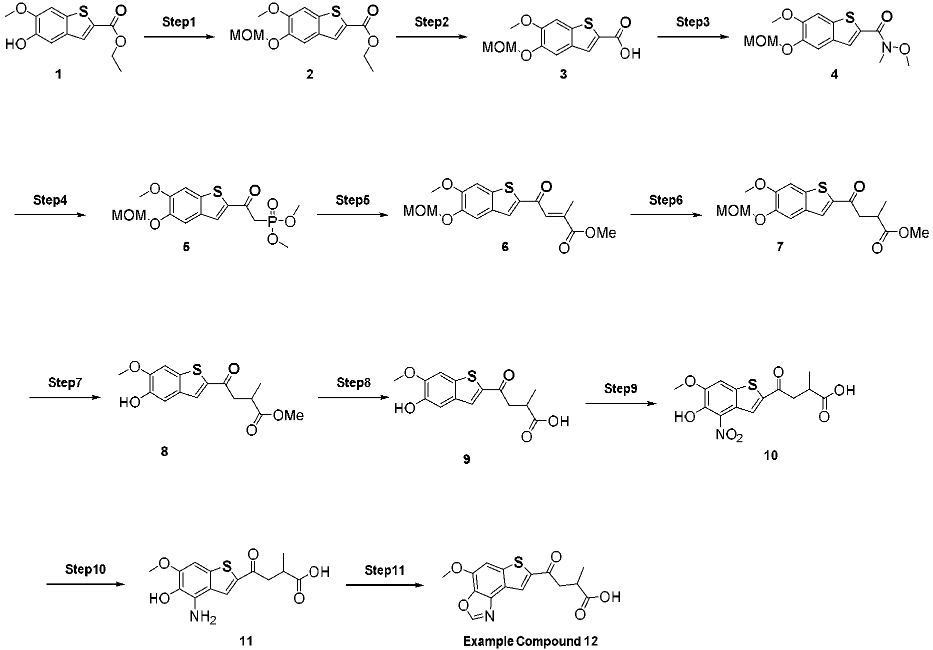

### Step (I): Synthesis of ethyl 6-methoxy-5-(methoxymethoxy)benzo[b]thiophene-2-carboxylate (Intermediate 2)

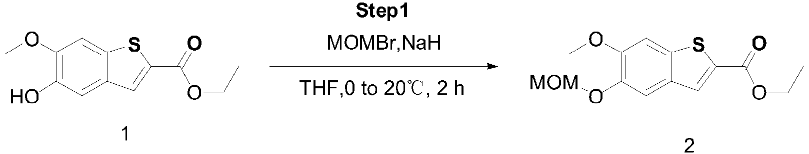

Starting material 1 (5 g, 19.82 mmol) was dissolved in THF (80 mL). NaH (1189.2 mg, 29.73 mmol) was added in an ice bath and stirred for half an hour. MOMBr (2972.05 mg, 23.78 mmol) was then added in an ice bath. The mixture was returned to room temperature and stirred for 1 hour. TLC indicated that the starting material had completely reacted. The reaction solution was poured into ammonium chloride solution, extracted three times with ethyl acetate, washed with saturated brine, dried over anhydrous sodium sulfate, and concentrated. The resulting solid was purified on a silica gel column (EA:PE = 0 to 10%) to give Intermediate Compound 2 (5.2 g) as a solid. Yield: 88.53%.

### Step (II): Synthesis of 6-methoxy-5-(methoxymethoxy)benzo[b]thiophene-2-carboxylic acid (Intermediate 3)

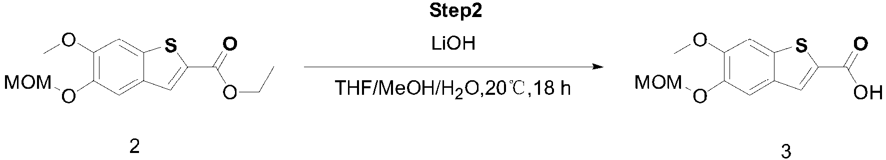

Intermediate Compound 2 (5.2 g, 17.55 mmol) prepared in step (I) was dissolved in a mixed solvent of tetrahydrofuran/water/methanol (2:1:1, 40 mL), and lithium hydroxide (1.1 g, 26.32 mmol) was added. The mixture was reacted at room temperature for 18 h. TLC indicated that the starting material had completely reacted. The reaction mixture was concentrated to remove the organic phase, adjusted to pH 4 with 1 N aqueous hydrochloric acid solution, and extracted with EA. The organic phase was washed with saturated brine, dried over anhydrous sodium sulfate, and concentrated to give Intermediate Compound 3 as a white solid (4.5 g). Yield: 95.57%.

### Step (III): Synthesis of N,6-dimethoxy-5-methoxymethoxy-N-methylbenzothiophene-2-carboxamide (Intermediate 4)

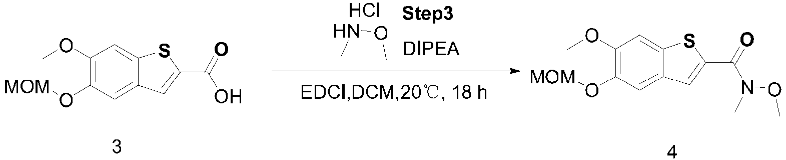

Intermediate Compound 3 (3 g, 11.18 mmol) prepared in step (II) was dissolved in DCM (30 mL). N,O-dimethylhydroxylamine hydrochloride (1308.82 mg, 13.42 mmol), DIPEA (1878.7 mg, 14.54 mmol), and EDCI (3249.25 mg, 16.77 mmol) were added, followed by nitrogen protection. The mixture was stirred at room temperature for 18 hours. TLC indicated that the starting material had completely reacted. The reaction solution was added dropwise to ammonium chloride solution, extracted with EA, washed with brine, dried, concentrated, and purified on a silica gel column (EA:PE = 0% to 30%) to give 2.3 g of solid. Yield: 66.06%.

### Step (IV): Synthesis of dimethyl (2-(6-methoxy-5-(methoxymethoxy)benzo[b]thiophen-2-yl)-2-oxoethyl)phosphonate (Intermediate 5)

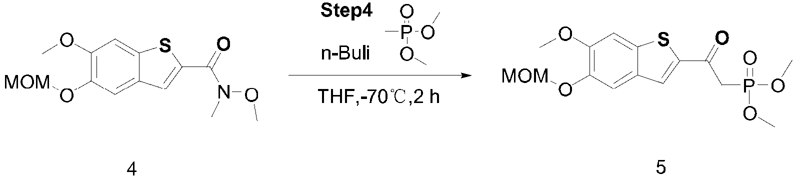

Dimethyl methylphosphonate (1354.95 mg, 10.92 mmol) was dissolved in anhydrous THF (30 mL), and n-BuLi (2.5M, 0.7 mL, 10.92 mmol) was added dropwise at -70°C. After addition, the mixture was stirred at -70°C for 1 hour. Intermediate 4 (1.7 g, 5.46 mmol) prepared in step (III) was then dissolved in THF (5 mL), added dropwise to the system at -70°C, and stirred at -70°C for 1 hour. TLC indicated that most of the starting material had completely reacted. Aqueous ammonium chloride solution was added dropwise to the system, and the mixture was extracted three times with EA. The organic phase was washed with saturated brine, dried, concentrated, and purified on a silica gel column (EA:PE = 0 to 100%) to give Intermediate Compound 4 as a solid (1.3 g). Yield: 63.6%. ESI MS[M+H]⁺ for C₁₅H₂₀O₇PS, calcd 375.15, found: 375.

### Step (V): Synthesis of methyl (E)-4-(6-methoxy-5-methoxymethoxy)benzo[b]thiophen-2-yl)-2-methyl-4-oxobut-2-enoate (Intermediate 6)

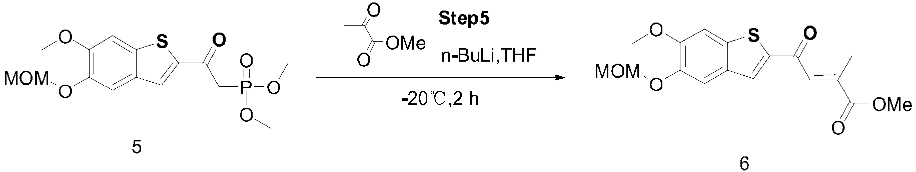

Intermediate Compound 5 (420 mg, 1.12 mmol) prepared in step (IV) was dissolved in THF (10 mL). n-BuLi (2.5M, 0.08 mL, 1.23 mmol) was slowly added at -20°C and stirred for 1 hour. Methyl 2-oxopropanoate (125.99 mg, 1.23 mmol) was then dissolved in THF, added dropwise to the system, and stirred for 30 minutes. The mixture was then returned to room temperature and stirred for 1.5 hours. TLC indicated that the starting material had completely reacted. The reaction solution was added dropwise to aqueous ammonium chloride solution and extracted three times with EA. The organic phase was washed with saturated brine, dried, concentrated, and purified on a silica gel column (EA:PE = 0 to 50%) to give Intermediate Compound 6 as a solid (350 mg). Yield: 89.19%. ESI MS[M+H]⁺ for C₁₇H₁₉O₆S, calcd 351.39, found: 351.

### Step (VI): Synthesis of methyl 4-(6-methoxy-5-methoxymethoxy)benzo[b]thiophen-2-yl)-2-methyl-4-oxobutanoate (Intermediate 7)

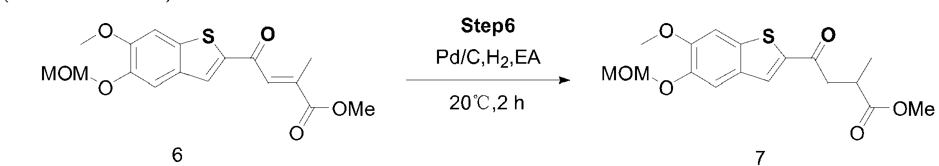

Intermediate Compound 6 (300 mg, 0.86 mmol) prepared in step (V) was dissolved in ethyl acetate (10 mL). Wet Pd/C (91.52 mg, 0.09 mmol) was added, and a hydrogen balloon was connected. The mixture was purged with hydrogen and reacted at room temperature for 2 h. TLC indicated that the starting material had completely reacted. The reaction solution was filtered. The filtrate was concentrated and then purified on a silica gel column (EA:PE = 0 to 30%) to give 130 mg of Intermediate Compound 7 as a solid. Yield: 42.9%. ESI MS[M+H]⁺ for C₁₇H₁₉O₆S, calcd 353.39, found: 353. ¹H NMR (400 MHz, Chloroform-d) δ 7.86 (s, 1H), 7.59 (d, J = 2.5 Hz, 1H), 7.28 (s, 1H), 5.30 (s, 2H), 3.98 (s, 3H), 3.70 (s, 3H), 3.55 (s, 3H), 3.50 - 3.39 (m, 1H), 3.22 - 3.10 (m, 1H), 3.02 (dd, J = 16.8, 5.9 Hz, 1H), 1.29 (d, J = 7.2 Hz, 3H).

### Step (VII): Synthesis of methyl 4-(5-hydroxy-6-methoxybenzo[b]thiophen-2-yl)-2-methyl-4-oxobutanoate (Intermediate 8)

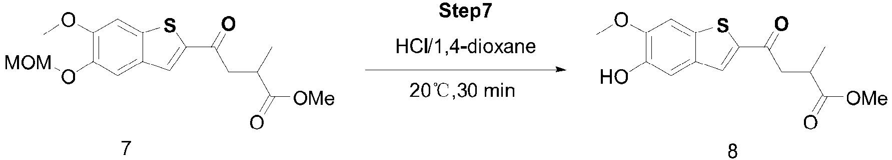

Intermediate Compound 7 (130 mg, 0.37 mmol) prepared in step (VI) was dissolved in 2M HCl/1,4-dioxane (2M, 3 mL, 6 mmol) and stirred at room temperature for 2 hours. TLC indicated that the starting material had completely reacted. The reaction solution was concentrated to give 90 mg of Intermediate Compound 8 as a solid. Yield: 78.38%.

### Step (VIII): Synthesis of 4-(5-hydroxy-6-methoxybenzo[b]thiophen-2-yl)-2-methyl-4-oxobutanoic acid (Intermediate 9)

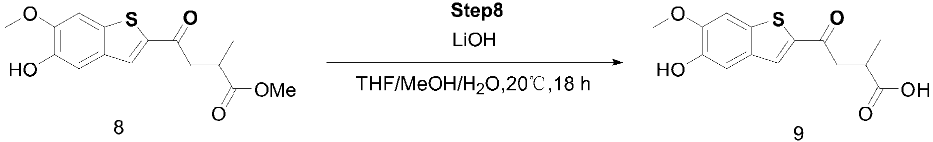

Intermediate Compound 8 (90 mg, 0.29 mmol) prepared in step (VII) was dissolved in a mixed solvent of tetrahydrofuran/water/methanol (2:1:1, 4 mL), and lithium hydroxide (13.89 mg, 0.58 mmol) was added. The reaction solution was stirred at room temperature for 18 hours. TLC indicated that the reaction was complete. The reaction solution was adjusted to pH 3, extracted with EA, washed with saturated brine, dried, and concentrated to give Intermediate Compound 9 as a solid (80 mg). Yield: 93.73%.

### Step (IX): Synthesis of 4-(5-hydroxy-6-methoxy-4-nitrobenzo[b]thiophen-2-yl)-2-methyl-4-oxobutanoic acid (Intermediate 10)

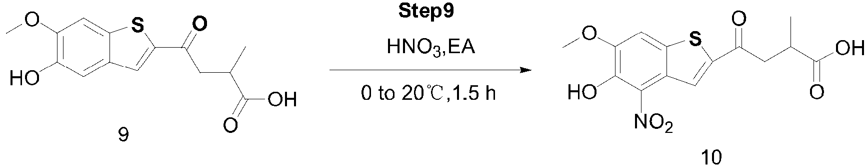

Intermediate Compound 9 (80 mg, 0.27 mmol) prepared in step (VIII) was dissolved in ethyl acetate (10 mL). Concentrated nitric acid (75.98 mg, 0.82 mmol) was added in an ice bath and stirred at room temperature for 1.5 hours. TLC indicated that the starting material had completely reacted. The reaction solution was immediately poured into water and extracted with EA. The organic phase was washed three times with saturated brine and concentrated at room temperature. The resulting solid was purified on a silica gel column (EA:PE = 0 to 100%) to give 45 mg of Intermediate Compound 10 as a solid. Yield: 49.12%.

### Step (X): Synthesis of 4-(4-amino-5-hydroxy-6-methoxybenzothiophen-2-yl)-2-methyl-4-oxobutanoic acid (Intermediate 11)

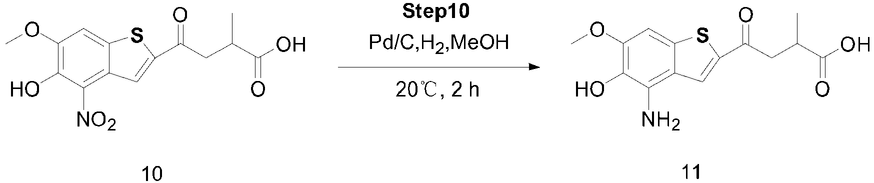

Intermediate 10 (45 mg, 0.13 mmol) prepared in step (IX) was dissolved in methanol (5 mL). Wet Pd/C (31.93 mg, 0.03 mmol) was added, and a hydrogen balloon was connected. The mixture was purged with hydrogen and reacted at room temperature for 2 h. TLC indicated that the starting material had completely reacted. The reaction solution was filtered, and the filtrate was concentrated to give 40 mg of Intermediate Compound 11 as a solid. Yield: 99.47%.

### Step (XI): Synthesis of 4-(4-methoxythieno[2,3:5,6]benzo[1,2-d]oxazol-7-yl)-2-methyl-4-oxobutanoic acid (Example Compound 12)

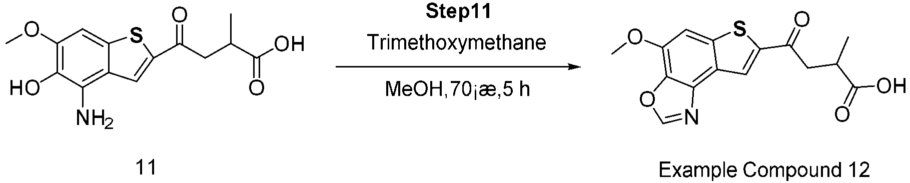

Intermediate 11 prepared in step (X) was dissolved in methanol (40 mg, 0.13 mmol), and trimethoxymethane (137.96 mg, 1.3 mmol) was added. The mixture was reacted at 70°C in a sealed tube for 5 hours. TLC indicated that the starting material had completely reacted. The reaction solution was concentrated at room temperature, and the resulting solid was purified on a column (C18, 10 µm, 20×250 mm; mobile phase: water (containing 0.1% formic acid)/acetonitrile; procedure: gradient from 10% to 50% acetonitrile over 30 min, 50% acetonitrile) to give a product. The product was lyophilized to give 8.5 mg of Example Compound 12 as a white solid. Yield: 20.48%. ESI MS[M+H]⁺ for C₁₅H₁₄NO₅S, calcd 320.05, found: 320.1; ¹H NMR (400 MHz, DMSO-d6) δ 12.19 (s, 1H), 8.92 (s, 1H), 8.57 (s, 1H), 7.78 (s, 1H), 4.07 (s, 3H), 3.53 (dd, J = 17.6, 8.6 Hz, 1H), 3.20 (dd, J = 17.6, 5.1 Hz, 1H), 2.91 (ddd, J = 8.5, 7.0, 4.9 Hz, 1H), 1.21 (d, J = 7.2 Hz, 3H).

### Example 13

### Synthesis of 4-(4-methoxythieno[3,2-e]benzofuran-7-yl)-4-oxobutanoic acid (Example Compound 13)

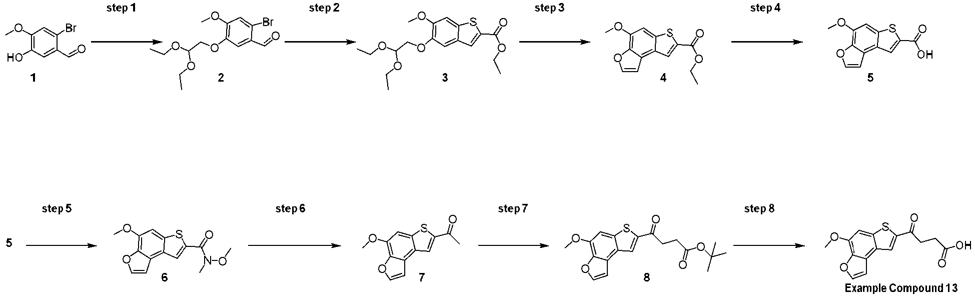

### Step (I): Synthesis of 2-bromo-5-(2,2-diethoxyethoxy)-4-methoxybenzaldehyde (Intermediate 2)

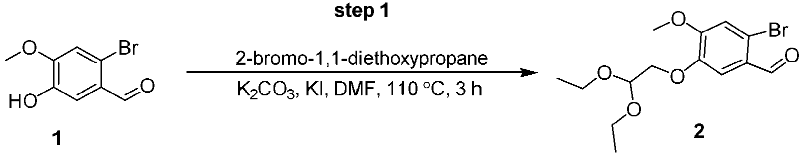

Compound 1 (5.0 g, 21.6 mmol) was dissolved in DMF (25 mL), and then 2-bromo-1,1-diethoxypropane (5.1 g, 26.0 mmol) and K₂CO₃ (5.0 g, 36.2 mmol) were added. The reaction mixture was then sealed well and stirred at 110°C for 3 hours. The reaction mixture was cooled to room temperature, quenched into ice water (200 mL), and then diluted with EtOAc (200 mL). The resulting mixture was extracted with EtOAc (200 mL). The organic phase was then washed with water (100 mL), dried over Na₂SO₄, and filtered. The filtrate was concentrated under vacuum to give crude Intermediate Compound 2 as a pale brown solid (7.0 g, 93%), ESI MS [M + H]⁺ for C₁₄H₂₀BrO₅, calcd 347.0, found 254.9; ¹H NMR (400 MHz, DMSO-*d*₆) δ 10.06 (s, 1H), 7.39 (s, 1H), 7.35 (s, 1H), 4.80 (t, *J* = 5.1 Hz, 1H), 4.01 (d, *J =* 5.1 Hz, 2H), 3.92 (s, 3H), 3.71 - 3.64 (m, 2H), 3.60 - 3.53 (m, 2H), 1.13 (t, *J =* 7.0 Hz, 6H).

### Step (II): Synthesis of ethyl 5-(2,2-diethoxyethoxy)-6-methoxybenzo[b]thiophene-2-carboxylate (Intermediate 3)

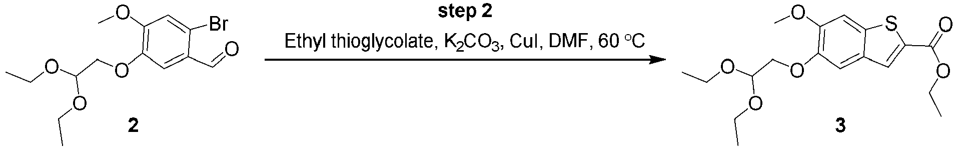

Intermediate Compound 2 (6.5 g, 18.7 mmol) prepared in step (I) was dissolved in DMF (100 mL). CuI (356.6 mg, 1.9 mmol) and ethyl thioglycolate (4.5 g, 37.4 mmol) were then added and cooled to 0°C. K₂CO₃ (7.7 g, 56.2 mmol) was added to the mixture at 0°C. The reaction mixture was then sealed well and stirred at 60°C under N₂ protection for 2 hours. The reaction mixture was cooled to room temperature, quenched into ice-water (500 mL), and then diluted with EtOAc (500 mL). The resulting mixture was extracted with EtOAc (500 mL). The organic phase was then washed with water (500 mL) and brine (500 mL), dried over Na₂SO₄, and filtered. The filtrate was concentrated under vacuum to give a crude sample. The crude product was further purified by column chromatography (SiO₂, petroleum ether:EtOAc = 10:1) to give the product Intermediate Compound 3 as a yellow solid (3.5 g, 51%): ESI MS [M + H]⁺ for C₁₈H₂₅O₆S, calcd 369.1, found 276.9; ¹H NMR (400 MHz, DMSO-d6) δ 8.00 (s, 1H), 7.62 (s, 1H), 7.57 (s, 1H), 4.87 (s, 1H), 4.32 (d, *J* = 7.1 Hz, 2H), 3.98 (d, *J =* 5.2 Hz, 2H), 3.87 (s, 3H), 3.75 - 3.65 (m, 1H), 3.61 (d, *J =* 6.9 Hz, 1H), 1.33 (t, *J =* 7.1 Hz, 3H), 1.15 (t, *J =* 7.0 Hz, 6H).

### Step (III): Synthesis of ethyl 4-methoxythieno[3,2-e]benzofuran-7-carboxylate (Intermediate 4)

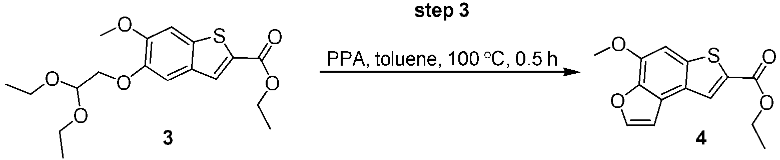

To a solution (5 mL) of Intermediate Compound 3 (500 mg, 1.4 mmol) prepared in Step (II) in toluene was added PPA (3 mL). The reaction mixture was stirred at 100°C for 0.5 hour. The reaction mixture was cooled to room temperature, quenched into ice-water (50 mL), and then diluted with EtOAc (100 mL). The resulting mixture was extracted with EtOAc (100 mL). The organic phase was then washed twice with water (20 mL), dried over Na₂SO₄, and filtered. The filtrate was concentrated under vacuum to give a crude sample. The crude product was further purified by column chromatography (SiO₂, petroleum ether:EtOAc = 20:1) to give the product Intermediate Compound 4 as a white solid (150 mg, 40%): ESI MS [M + H]⁺ for C₁₄H₁₃O₄S, calcd 277.1, found 276.9. ¹H NMR (400 MHz, DMSO-*d*₆) δ 8.47 (s, 1H), 8.14 (d, *J =* 2.0 Hz, 1H), 7.62 (s, 1H), 7.51 (d, *J* = 2.1 Hz, 1H), 4.36 (q, *J =* 7.1 Hz, 2H), 4.03 (s, 3H), 1.35 (t, *J* = 7.1 Hz, 3H).

### Step (IV): Synthesis of 4-methoxythieno[3,2-e]benzofuran-7-carboxylic acid (Intermediate 5)

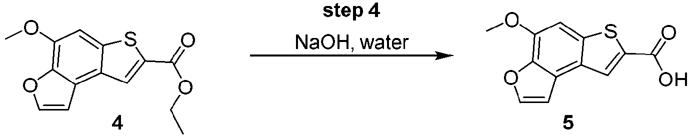

Intermediate Compound 4 (450.0 mg, 1.6 mmol) prepared in step (III) was dissolved in MeOH (10 mL) and H₂O (10 mL), and NaOH (232.6 mg, 5.8 mmol) was added. The reaction mixture was then stirred at 60°C for 3 hours. LCMS at this point indicated no more starting material. The reaction mixture was adjusted to pH = 4 and diluted with water (100 mL). The mixture was extracted three times with EtOAc (100 mL). The organic phase was collected, dried over Na₂SO₄, and concentrated under vacuum to give the title Intermediate Compound 5 as a white solid (400.0 mg, 99%): ESI MS [M + H]⁺ for C₁₂H₉O₄S, calcd 248.1, found 247.9, which was used in the next step without further purification.

### Step (V): Synthesis of N,4-dimethoxy-N-methylthieno[3,2-e]benzofuran-7-carboxamide (Intermediate 6)

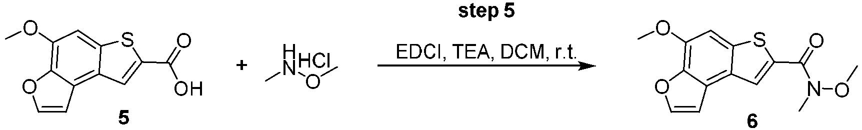

Intermediate Compound 5 (550.0 mg, 2.2 mmol) prepared in step (IV), EDCI (849.4 mg, 4.4 mmol), N,O-dimethylhydroxylamine hydrochloride (648.3 mg, 6.7 mmol), and trimethylamine (1.1 g, 11.1 mmol) were dissolved in DCM (10 mL), and the mixture was purged with N₂ three times. The reaction mixture was stirred at room temperature under N₂ protection for 12 hours. The reaction mixture was quenched into water (100 g) and then diluted with DCM (100 mL). The resulting mixture was extracted with DCM (200 mL). The organic phase was then washed three times with water (100 mL), dried over Na₂SO₄, and filtered. The filtrate was concentrated under vacuum to give a crude sample. The crude product was further purified by trituration (petroleum ether:EtOAc = 5:1) to give the pure title Intermediate Compound 6 as a pale red solid (600.0 mg, 93%): ESI MS [M + H]⁺ for C₁₃H₁₃N₂O₄S, calcd 293.1, found 292.0; ¹H NMR (400 MHz, Chloroform-d) δ 8.41 (s, 1H), 7.78 (d, *J =* 2.1 Hz, 1H), 7.21 (s, 1H), 7.11 (d, *J =* 2.1 Hz, 1H), 4.11 (s, 3H), 3.88 (s, 3H), 3.46 (s, 3H).

### Step (VI): Synthesis of 1-(4-methoxythieno[3,2-e]benzofuran-7-yl)ethan-1-one (Intermediate 7)

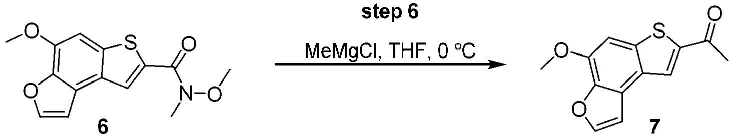

Intermediate Compound 6 (600.0 mg, 2.1 mmol) prepared in step (V) was dissolved in THF (10 mL), and a 1.0 M solution of methylmagnesium chloride in THF (6.2 mL, 6.2 mmol) was added dropwise at 0°C. The reaction mixture was stirred at room temperature for 0.5 hour. The reaction mixture was quenched into water (50 g) and then diluted with EtOAc (100 mL). The resulting mixture was extracted with EtOAc (100 mL). The organic phase was then washed three times with water (50 mL), dried over Na₂SO₄, and filtered. The filtrate was concentrated under vacuum to give a crude sample. The crude product was further purified by trituration (petroleum ether:EtOAc = 5:1) to give the pure title Intermediate Compound 7 as a white solid (400.0 mg, 79%): ESI MS [M + H]⁺ for C₁₃H₁₁O₃S, calcd 247.0, found 247.0; ¹H NMR (400 MHz, Chloroform-d) δ 8.09 (s, 1H), 7.78 (d, *J* = 2.1 Hz, 1H), 7.20 (s, 1H), 7.07 (d, *J* = 2.1 Hz, 1H), 4.10 (s, 3H), 2.68 (s, 3H).

### Step (VII): Synthesis of tert-butyl 4-(4-methoxythieno[3,2-e]benzofuran-7-yl)-4-oxobutanoate (Intermediate 8)

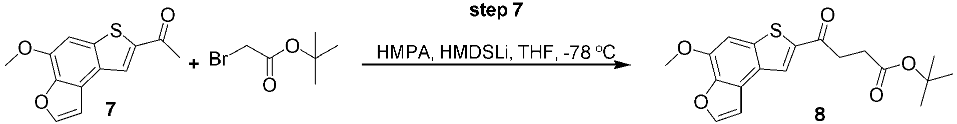

Intermediate Compound 7 (150.0 mg, 0.6 mmol) prepared in step (VI) and HMPA (382.0 mg, 2.1 mmol) were dissolved in anhydrous THF (3 mL), and the mixture was purged with N₂ three times. The reaction mixture was cooled to -78°C under N₂ protection. HMDSLi (152.9 mg, 0.9 mmol) was then added dropwise and stirred at -40°C for 1 hour. Afterwards, the reaction mixture was cooled to -78°C. Tert-butyl 2-bromoacetate (237.6 mg, 1.2 mmol) was added dropwise and stirred at -78°C for 30 minutes. The reaction mixture was quenched into water (50 g) and then diluted with EtOAc (100 mL). The resulting mixture was extracted with EtOAc (100 mL). The organic phase was then washed three times with water (50 mL), dried over Na₂SO₄, and filtered. The filtrate was concentrated under vacuum to give the title Intermediate Compound 8 as a yellow solid (89.7 mg, 41%): ESI MS[M -55 H]⁺ for C₁₉H₂₀O₅S, calcd 360.1, found 304.9, which was used in the next step without further purification.

### Step (VIII): Synthesis of 4-(4-methoxythieno[3, 2-e]benzofuran-7-yl)-4-oxobutanoic acid (Example compound 13)

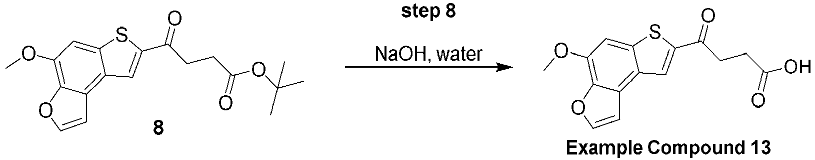

Intermediate Compound 8 (219.0 mg, 0.6 mmol) prepared in step (VII) was dissolved in DCM (2 mL) and TFA (698.0 mg, 6.1 mmol). The reaction mixture was stirred at room temperature for 1 hour. The reaction mixture was concentrated under vacuum to give a crude sample. The crude product was further purified by reverse-phase HPLC (C18 column, 30-50% gradient of MeCN and water containing 0.1% FA) to give the product Example Compound 13 as a white solid: ESI MS [M + H]⁺ for C₁₅H₁₃O₅S, calcd 305.0, found 305.0. ¹H NMR (400 MHz, DMSO-d6) δ 12.19 (s, 1H), 8.68 (s, 1H), 8.16 (d, *J* = 2.1 Hz, 1H), 7.61 (s, 1H), 7.45 (d, *J =* 2.0 Hz, 1H), 4.04 (s, 3H), 3.35 (t, *J =* 6.4 Hz, 2H), 2.64 (t, *J =* 6.4 Hz, 2H).

### Example 14

### Synthesis of 4-(4-methoxy-8-methylthieno[3,2-e]benzofuran-7-yl)-4-oxobutanoic acid (Example Compound 14)

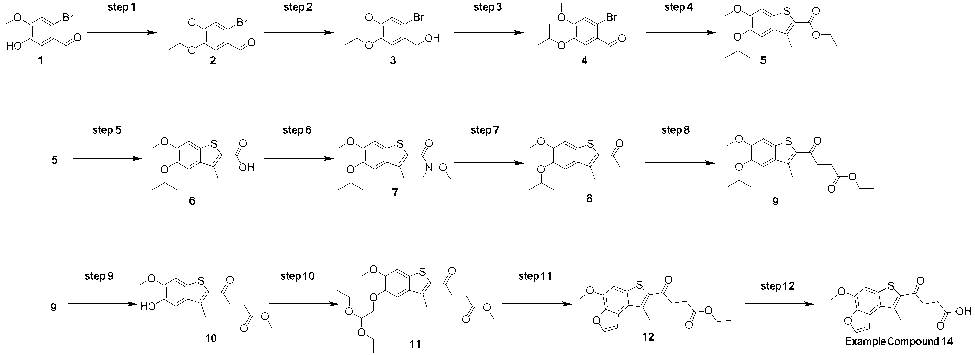

### Step (I): Synthesis of 2-bromo-5-isopropoxy-4-methoxybenzaldehyde (Intermediate 2)

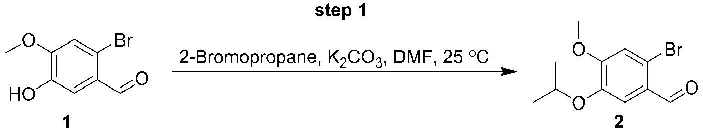

2-Bromo-5-hydroxy-4-methoxybenzaldehyde (10.0 g, 43.3 mmol) was dissolved in DMF (100 mL), and then 2-bromopropane (8.0 g, 64.9 mmol) and K₂CO₃ (12 g, 86.6 mmol) were added at 25°C. The reaction mixture was then sealed well and stirred at 25°C for 16 hours. The reaction mixture was quenched into water (2.0 kg) and then diluted with EtOAc (1000 mL). The resulting mixture was extracted with EtOAc (1000 mL), and the organic phase was washed with water (1000 mL) and brine (500 mL). The organic phase was collected, dried over Na₂SO₄, and concentrated under vacuum to give Intermediate Compound 2 as a white solid (10.6 g, 90%): ESI MS [M + H]⁺ for C₁₁H₁₃BrO₃, calcd 274.1, found 272.9.

### Step (II): Synthesis of 1-(2-bromo-5-isopropoxy-4-methoxyphenyl)ethan-1-ol (Intermediate 3)

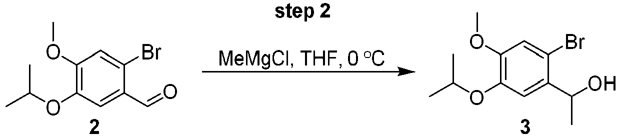

Intermediate Compound 2 (10.6 g, 38.8 mmol) prepared in the previous step was dissolved in THF (110 mL), and the mixture was purged with N₂ three times. Subsequently, 3.0M methylmagnesium chloride in THF (12.9 mL, 38.8 mmol) was added dropwise to the reaction mixture at 0°C. The reaction mixture was stirred at room temperature for 1 hour. The reaction mixture was quenched into 1M HCl (150 g) and then diluted with EtOAc (200 mL). The resulting mixture was extracted with EtOAc (100 mL), and then the organic phase was washed with water (100 mL) and brine (100 mL). The organic phase was collected, dried over Na₂SO₄, and concentrated under vacuum to give the product 3, namely Intermediate Compound 3, as a pale yellow solid (11 g, 98%): ESI MS [M + H]⁺ for C₁₂H₁₇BrO₃, calcd 290.2, found 270.9.

### Step (III): Synthesis of 1-(2-bromo-5-isopropoxy-4-methoxyphenyl)ethan-1-one (Intermediate 4)

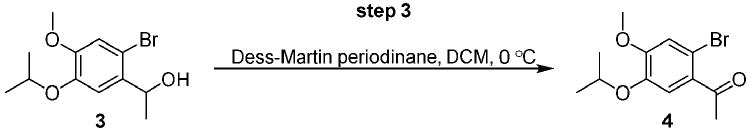

Intermediate Compound 3 (11 g, 38 mmol) prepared in the previous step was dissolved in DCM (150 mL). The reaction mixture was added portionwise to Dess-Martin periodinane (32.3 g, 76.1 mmol) at 0°C, and the mixture was stirred at room temperature for 1 hour. The reaction mixture was quenched into water (500 g). The resulting mixture was extracted with EtOAc (500 mL). The organic phase was washed five times with water (200 mL), dried over Na₂SO₄, and filtered. The filtrate was concentrated under vacuum to give the product Intermediate Compound 4 as a white solid (7.8 g, 92%): ESI MS [M + H]⁺ for C₁₅H₁₉O₄S, calcd 295.1, found 295.0; ¹H NMR (400 MHz, CDCl₃) δ 7.90 (s, 1H), 7.26 (s, 1H), 7.24 (s, 1H), 4.57 (hept, J = 6.1 Hz, 1H), 4.38 (q, J = 7.1 Hz, 2H), 3.93 (s, 3H), 1.40 (dd, J = 8.8, 6.6 Hz, 9H).

### Step (IV): Ethyl 5-isopropoxy-6-methoxy-3-methylbenzo[b]thiophene-2-carboxylate (Intermediate 5)

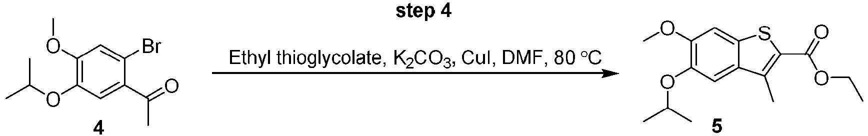

Intermediate Compound 4 (10.0 g, 34.8 mmol) prepared in the previous step was dissolved in DMF (180 mL). CuI (1.3 g, 7.0 mmol) and ethyl thioglycolate (8.4 g, 69.7 mmol) were then added and cooled to 0°C. K₂CO₃ (14.4 g, 104.5 mmol) was added to the mixture at 0°C. The reaction mixture was then sealed well and stirred at 80°C under N₂ protection for 3 hours. The reaction mixture was cooled to room temperature, quenched into ice water (2.0 kg), and then diluted with EtOAc (1000 mL). The resulting mixture was extracted with EtOAc (1000 mL). The organic phase was then washed with water (1000 mL) and brine (500 mL), dried over Na₂SO₄, and filtered. The filtrate was concentrated under vacuum to give a crude sample. The crude product was further purified by column chromatography (SiO₂, petroleum ether:EtOAc = 10:1) to give the product Intermediate Compound 5 as a white solid (6.1 g, 57%): ESI MS [M + H]⁺ for C₁₆H₂₁O₄S, calcd 309.1, found 309.0.

### Step (V): Synthesis of 5-isopropoxy-6-methoxy-3-methylbenzo[b]thiophene-2-carboxylic acid (Intermediate 6)

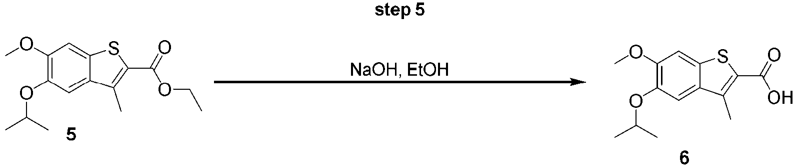

Intermediate Compound 5 (6.0 g, 20.4 mmol) prepared in the previous step was dissolved in water (40 mL), EtOH (40 mL), and NaOH (2.4 g, 61.2 mmol). The reaction mixture was stirred at 40°C for 3 hours. The reaction mixture was adjusted to pH = 6 and diluted with water (500 mL). The mixture was extracted twice with EtOAc (500 mL). The organic phase was collected, dried over Na₂SO₄, and concentrated under vacuum to give crude Intermediate Compound 6 as a pale white solid (4.1 g, 76%): ESI MS [M + H]⁺ for C₁₄H₁₇O₄S, calcd 281.1, found 281.0, which was used in the next step without further purification.

### Step (VI): Synthesis of 5-isopropoxy-N,6-dimethoxy-N,3-dimethylbenzo[b]thiophene-2-carboxamide (Intermediate 7)

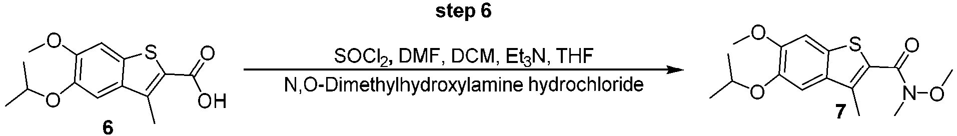

To a solution of Intermediate Compound 6 (4.0 g, 14.3 mmol) prepared in the previous step in DCM (60 mL) was added dropwise thionyl chloride (8.5 g, 71.4 mmol) at room temperature. The reaction mixture was then refluxed at 50°C under N₂ protection for 1 hour. The reaction mixture was concentrated under vacuum to give a light brown sample. The residue was diluted with DCM (10 mL) and quenched into N,O-dimethylhydroxylamine hydrochloride (4.2 g, 42.8 mmol) and Et₃N (7.2 g, 71.4 mmol) in water (2 mL) and THF (38 mL). Water (200 mL) was then added, and the mixture was extracted three times with DCM (200 mL). The organic phase was collected, dried over Na₂SO₄, and filtered. The filtrate was concentrated under vacuum to give a crude sample. The crude product was further purified by column chromatography (SiO₂, petroleum ether:EtOAc = 5:1) to give the product Intermediate Compound 7 as a white solid (4.4 g, 95%): ¹H NMR (400 MHz, Chloroform-d) δ 7.24 (s, 1H), 7.22 (s, 1H), 4.61 (hept, *J* = 6.1 Hz, 1H), 3.93 (s, 3H), 3.71 (s, 3H), 3.37 (s, 3H), 2.63 (s, 3H), 1.42 (d, J = 6.1 Hz, 6H).

### Step (VII): Synthesis of 1-(5-isopropoxy-6-methoxy-3-methylbenzo[b]thiophen-2-yl)ethan-1-one (Intermediate 8)

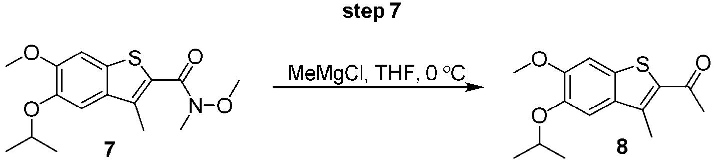

To a solution of Intermediate Compound 7 (4.3 g, 13.3 mmol) prepared in the previous step in THF (50 mL) was added dropwise 3.0M methylmagnesium chloride in THF (13.3 mL, 39.9 mmol) at 0°C. The reaction mixture was stirred at room temperature for 0.5 hour. The reaction mixture was quenched into water (100 g) and then diluted with EtOAc (200 mL). The resulting mixture was extracted with EtOAc (200 mL). The organic phase was washed three times with water (50 mL), dried over Na₂SO₄, and filtered. The filtrate was concentrated under vacuum to give a crude sample. The crude product was further purified by column chromatography (SiO₂, petroleum ether:EtOAc = 10:1) to give the title compound, Intermediate Compound 8, as a white solid (3.0 g, 81%): ¹H NMR (400 MHz, Chloroform-d) δ 7.24 (s, 1H), 7.22 (s, 1H), 4.62 (hept, *J* = 6.2 Hz, 1H), 3.95 (s, 3H), 2.70 (d, *J =* 1.6 Hz, 3H), 2.59 (d, *J =* 1.5 Hz, 3H), 1.42 (d, *J* = 6.1 Hz, 6H).

### Step (VIII): Synthesis of ethyl 4-(5-isopropoxy-6-methoxy-3-methylbenzo[b]thiophen-2-yl)-4-oxobutanoate (Intermediate 9)

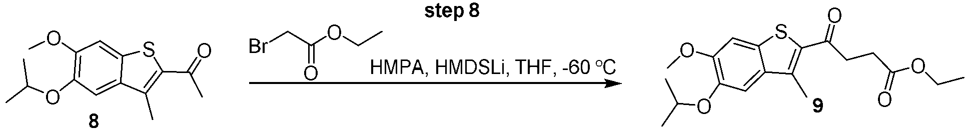

Intermediate Compound 9 (2.9 g, 10.4 mmol) prepared in the previous step and HMPA (6.5 g, 36.5 mmol) were dissolved in anhydrous THF (30 mL), and the reaction mixture was cooled to -78°C under N₂ protection. Then, 1M HMDSLi in THF (15.6 mL, 15.6 mmol) was added dropwise and stirred at -40°C for 1.5 hours. The reaction mixture was then cooled to -78°C, and ethyl 2-bromoacetate (3.5 g, 20.8 mmol) was added dropwise. The mixture was stirred at -78°C under a N₂ atmosphere for 0.5 hour.

The reaction mixture was quenched into water (100 g) and then diluted with EtOAc (200 mL). The resulting mixture was extracted with EtOAc (200 mL). The organic phase was then washed three times with water (100 mL), dried over Na₂SO₄, and filtered. The filtrate was concentrated under vacuum to give a crude sample. The crude product was further purified by column chromatography (SiO₂, petroleum ether:EtOAc = 10:1) to give the title compound, Intermediate Compound 9, as a pale yellow solid (3.2 g, 84%): ¹H NMR (400 MHz, Chloroform-d) δ 7.24 (s, 1H), 7.22 (s, 1H), 4.61 (h, *J =* 6.0 Hz, 1H), 4.17 (q, *J =* 7.1 Hz, 2H), 3.95 (s, 3H), 3.25 (t, *J* = 6.6 Hz, 2H), 2.75 (t, *J =* 6.6 Hz, 2H), 2.71 (s, 3H), 1.42 (d, *J =* 6.1 Hz, 6H), 1.27 (t, *J =* 7.1 Hz, 3H).

### Step (IX): Synthesis of ethyl 4-(5-hydroxy-6-methoxybenzo[b]thiophen-2-yl)-4-oxobutanoate (Intermediate Compound 10)

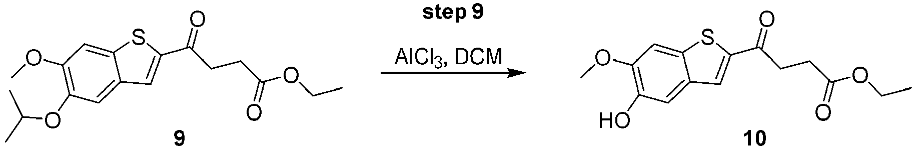

Intermediate Compound 10 (3.0 g, 8.2 mmol) prepared in the previous step was dissolved in DCM (30 mL), and AlCl₃ (1.6 g, 12.4 mmol) was added. The reaction mixture was stirred at room temperature for 12 hours. The reaction mixture was quenched into ice-water (100 g) and then diluted with DCM (200 mL). The resulting mixture was extracted with DCM (200 mL). The organic phase was washed twice with water (100 mL), dried over Na₂SO₄, and filtered. The filtrate was concentrated under vacuum to give a crude sample. The crude product was further purified by column chromatography (SiO₂, petroleum ether:EtOAc = 10:1) to give the title compound, Intermediate Compound 10, as a yellow solid (2.1 g, 79%): ESI MS [M + H]⁺ for C₁₆H₁₉O₅S, calcd 323.1, found 323.0.

### Step (X): Synthesis of ethyl 4-(5-(2,2-diethoxyethoxy)-6-methoxy-3-methylbenzo[b]thiophen-2-yl)-4-oxobutanoate (Intermediate 11)

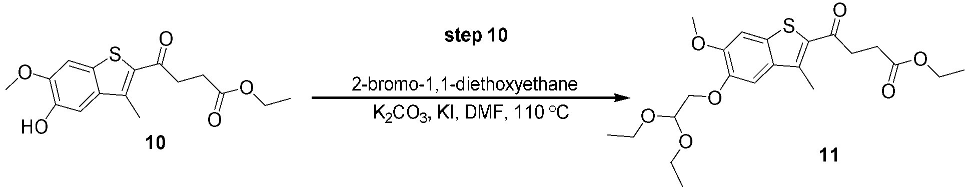

Intermediate Compound 10 (0.5 g, 1.6 mmol) prepared in the previous step was dissolved in DMF (15 mL), and then KI (51.5 mg, 0.3 mmol), 2-bromo-1,1-diethoxyethane (3.1 g, 15.5 mmol), and K₂CO₃ (1.1 g, 7.8 mmol) were added. The reaction mixture was then sealed well and stirred at 110°C for 4 hours. The reaction mixture was cooled to room temperature, quenched into ice water (200 mL), and then diluted with EtOAc (200 mL). The resulting mixture was extracted with EtOAc (200 mL). The organic phase was then washed with water (100 mL), dried over Na₂SO₄, and filtered. The filtrate was concentrated under vacuum to give a crude sample. The crude product was further purified by column chromatography (SiO₂, petroleum ether:EtOAc = 10:1) to give the product Intermediate Compound 11 as a yellow solid (550 mg, 81%): ¹H NMR (400 MHz, DMSO-d6) δ 7.58 (s, 1H), 7.48 (s, 1H), 4.87 (t, *J* = 5.1 Hz, 1H), 4.10 - 4.04 (m, 4H), 3.88 (s, 3H), 3.71 (dq, *J* = 9.6, 7.1 Hz, 2H), 3.60 (dq, *J* = 9.6, 7.0 Hz, 2H), 3.22 (dd, *J =* 7.1, 5.4 Hz, 2H), 2.70 (s, 3H), 2.65 (t, *J* = 6.2 Hz, 2H), 1.18 (dt, *J =* 12.4, 7.1 Hz, 9H).

### Step (XI): Synthesis of ethyl 4-(4-methoxy-8-methylthieno[3,2-e]benzofuran-7-yl)-4-oxobutanoate (Intermediate 12)

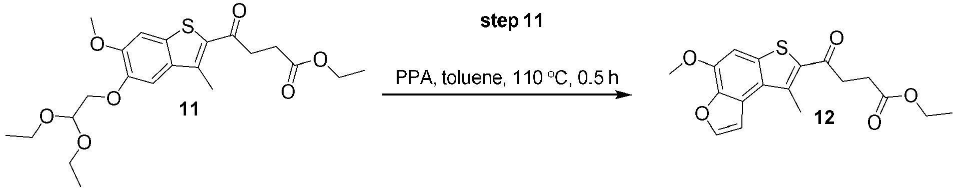

Intermediate Compound 11 (300 mg, 0.7 mmol) prepared in the previous step was dissolved in toluene (3 mL), and PPA (557.6 mg, 6.8 mmol) was added. The reaction mixture was stirred at 110°C for 0.5 hour. The reaction mixture was cooled to room temperature, quenched into ice-water (20 mL), and then diluted with EtOAc (50 mL). The resulting mixture was extracted with EtOAc (50 mL). The organic phase was then washed three times with water (20 mL), dried over Na₂SO₄, and filtered. The filtrate was concentrated under vacuum to give a crude sample. The crude product was further purified by column chromatography (SiO₂, petroleum ether:EtOAc = 5:1) to give the product Intermediate Compound 12 as a white solid (50 mg, 21%): ESI MS [M + H]⁺ for C₁₈H₁₉O₅S, calcd 347.1, found 347.1.

### Step (XII): Synthesis of 4-(4-methoxy-8-methylthieno[3,2-e]benzofuran-7-yl)-4-oxobutanoic acid (Example Compound 14)

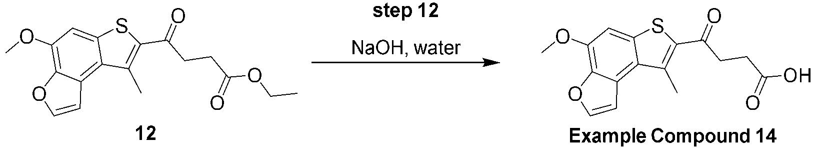

Intermediate Compound 12 (50 mg, 0.1 mmol) prepared in the previous step was dissolved in a solution of water (2 mL), ethanol (4 mL), and NaOH (28 mg, 0.7 mmol). The reaction mixture was stirred at room temperature for 0.5 hour. The reaction mixture was adjusted to pH = 5-6 and diluted with water (20 mL). The mixture was extracted three times with EtOAc (20 mL). The organic phase was collected, dried over Na₂SO₄, and concentrated under vacuum to give a crude sample. The crude product was further purified by reverse-phase HPLC (C18 column, 30-50% gradient of MeCN and water containing 0.1% FA) to give the product as a white solid (8.9 mg, 19%): ESI MS [M + H]⁺ for C₁₆H₁₅O₅S, calcd 319.1, found 319.0. ¹H NMR (400 MHz, DMSO-d6) δ 12.20 (s, 1H), 8.19 (d, *J =* 2.1 Hz, 1H), 7.58 (s, 1H), 7.51 (d, *J =* 2.1 Hz, 1H), 4.03 (s, 3H), 3.20 (dd, *J=* 7.1, 5.4 Hz, 2H), 2.93 (s, 3H), 2.60 (t, *J =* 6.2 Hz, 2H).

### Example 15

### Synthesis of 4-(4-methoxy-3-methyl-3H-thieno[3,2-e]indazol-7-yl)-4-oxobutanoic acid (Example Compound 15)

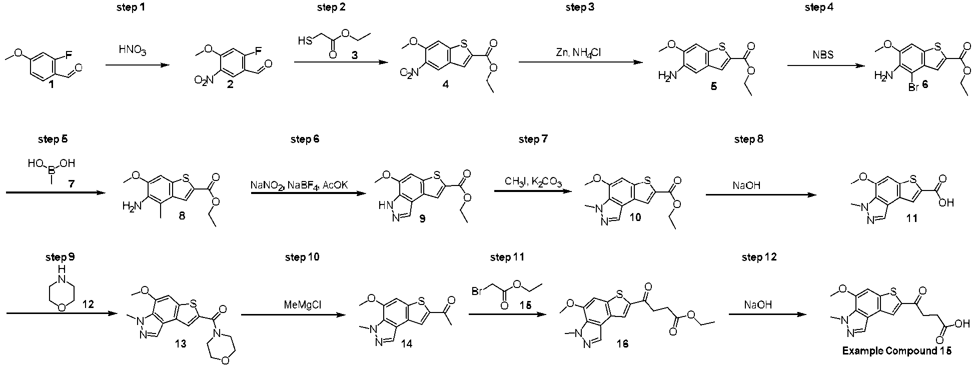

### Step (I): Synthesis of 2-fluoro-4-methoxy-5-nitrobenzaldehyde (Intermediate 2)

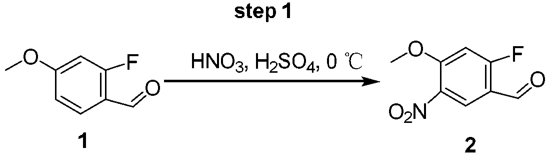

A solution of 2-fluoro-4-methoxybenzaldehyde (5.0 g, 32.4 mmol) in H₂SO₄ (30 mL) was cooled to 0°C, and then HNO₃ (4 mL, 65%, 41.3 mmol) was slowly added. The reaction mixture was stirred at 0°C for 2 minutes. The reaction mixture was quenched into ice-water (500 g) at such a rate that its internal temperature was maintained below 20°C. The mixture was then filtered, and the filter cake was extracted with EA (500 mL). The organic phase was then washed with water (400 mL) and saturated brine (200 mL), dried over Na₂SO₄, and filtered. The filtrate was concentrated under vacuum to give the crude product 2, 2-fluoro-4-methoxy-5-nitrobenzaldehyde, namely Intermediate Compound 2, as a yellow solid (6.0 g, 92.9%): ESI MS [M + H]⁺ for C₈H₆FNO₄, calcd 200.14, found 200.1.

### Step (II): Synthesis of ethyl 6-methoxy-5-nitro-1-benzothiophene-2-carboxylate (Intermediate Compound 4)

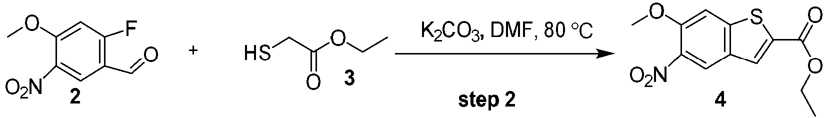

To a solution of Intermediate Compound 2 (5.0 g, 25.1 mmol) prepared in the previous step and ethyl 2-mercaptoacetate (4.5 g, 37.66 mmol) in DMF (50 mL) was added K₂CO₃ (6.9 g, 50.22 mmol), and the mixture was purged with N₂ three times. The reaction mixture was then sealed well and stirred at 80°C under N₂ protection for 3 hours. The reaction mixture was cooled to room temperature and filtered. The filtrate was quenched into ice-water (600 g), and the resulting mixture was extracted with EtOAc (400 mL). The organic phase was washed twice with water (200 mL), dried over Na₂SO₄, and filtered. The filtrate was concentrated under vacuum to give a crude sample. To the crude product was added EtOAc (10 mL) and PE (100 mL). The mixture was stirred at 25°C for 1 hour and then filtered. The filter cake was dried under vacuum to give the product 4, ethyl 6-methoxy-5-nitro-1-benzothiophene-2-carboxylate (Intermediate Compound 4), as a yellow solid (6.0 g, 85.0%): ESI MS[M + H]⁺ for C₁₂H₁₁NO₅S, calcd 282.28, found 282.0.

### Step (III): Synthesis of ethyl 5-amino-6-methoxybenzo[b]thiophene-2-carboxylate (Intermediate 5)

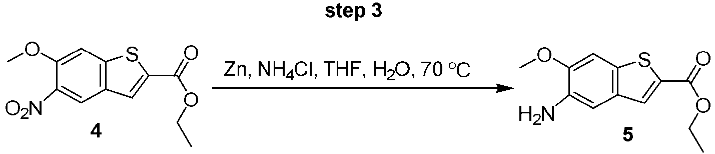

Intermediate Compound 4 (6.0 g, 21.33 mmol) prepared in the previous step, zinc (7.0 g, 106.7 mmol), and NH₄Cl (5.7 g, 106.7 mmol) were dissolved in THF (50 mL) and H₂O (10 mL), and the mixture was purged with N₂ three times. The reaction mixture was then sealed well and stirred at 70°C under N₂ protection for 2 hours. The reaction mixture was cooled to room temperature and filtered through celite. The filtrate was diluted with EtOAc (500 mL) and washed with water (200 mL). The organic phase was collected, dried over Na₂SO₄, and concentrated under vacuum to give a crude sample. The crude product was further purified by column chromatography (SiO₂, 20% EtOAc/petroleum ether) to give the product Intermediate Compound 5 as a yellow solid (5.0 g, 93.3%): ESI MS [M + H]⁺ for C₁₂H₁₃NO₃S, calcd 252.3, found 252.1.

### Step (IV): Synthesis of ethyl 5-amino-4-bromo-6-methoxy-1-benzothiophene-2-carboxylate (Intermediate 6)

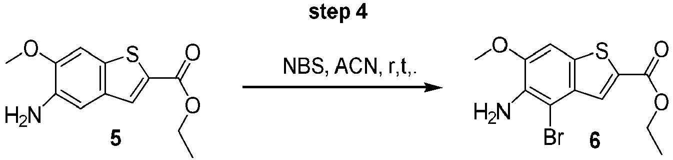

Intermediate Compound 5 (2.7 g, 10.74 mmol) prepared in the previous step was dissolved in acetonitrile (300 mL), and 1-bromopyrrolidine-2,5-dione (1.9 g, 10.7 mmol) was added. The reaction mixture was then stirred at room temperature for 2 minutes. The reaction mixture was diluted with water (300 mL) and extracted twice with EtOAc (500 mL). The organic phase was collected, dried over Na₂SO₄, and concentrated under vacuum to give a crude sample. The crude product was further purified by column chromatography (SiO₂, 10% EtOAc/petroleum ether) to give the product Intermediate Compound 6 as a yellow solid (2.9 g, 81.7%): ESI MS[M + H]⁺ for C₁₂H₁₂BrNO₃S, calcd 331.2, found 332.0.

### Step (V): Synthesis of ethyl 5-amino-6-methoxy-4-methyl-1-benzothiophene-2-carboxylate (Intermediate 8)

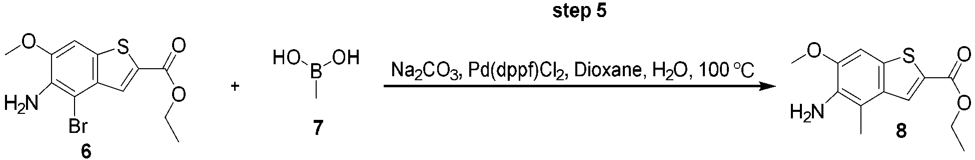

Intermediate Compound (2.6 g, 7.9 mmol) prepared in the previous step, methylboronic acid (1.4 g, 23.6 mmol), disodium carbonate (2.5 g, 23.6 mmol), and [1,1'-bis(diphenylphosphino)ferrocene]dichloropalladium (0.6 g, 0.79 mmol) were dissolved in 1,4-dioxane (50 mL) and water (10 mL), and the mixture was purged with N₂ three times. The reaction mixture was sealed well and stirred at 100°C under N₂ protection overnight.

The reaction mixture was cooled to room temperature and filtered. The filtrate was diluted with EtOAc (500 mL) and washed with water (200 mL). The organic phase was dried over Na₂SO₄ and filtered. The filtrate was concentrated under vacuum to give a crude sample. The crude product was further purified by column chromatography (SiO₂, 10% EtOAc/petroleum ether) to give the product Intermediate Compound 8 as a yellow solid (1.7 g, 79.9%): ESI MS [M + H]⁺ for C₁₃H₁₅NO₃S, calcd 266.33, found 266.2.

### Step (VI): Synthesis of ethyl 4-methoxy-3H-thieno[3,2-e]indazole-7-carboxylate (Intermediate 9)

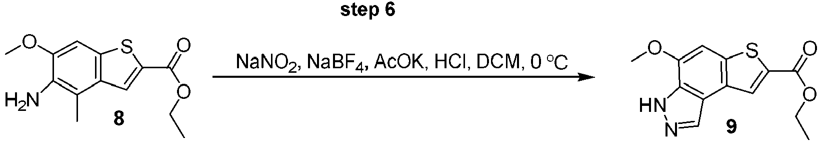

Intermediate Compound 8 (1.7 g, 6.3 mmol) prepared in the previous step was dissolved in hydrogen chloride (15 mL, 36%), and a mixture of sodium nitrite (0.5 g, 7.6 mmol) was added at 0°C. The reaction mixture was then stirred at 0°C for 0.5 hour. Sodium tetrafluoroborate (0.8 g, 7.6 mmol) was then added and stirred at 0°C for 0.5 hour. The reaction mixture was filtered, and dichloromethane (20 mL) and potassium acetate (0.7 g, 7.6 mmol) were added to the filter cake. The reaction mixture was stirred at room temperature for 1 hour. The reaction mixture was quenched into water (100 g). The resulting mixture was extracted with DCM (100 mL). The organic phase was then washed with water (100 mL), dried over Na₂SO₄, and filtered. The filtrate was concentrated under vacuum to give the title Intermediate Compound 9 as a yellow solid (1.3 g, 74.8%): ESI MS[M + H]⁺ for C₁₃H₁₂N₂O₃S, calcd 277.31, found 277.1, which was used in the next step without further purification.

### Step (VII): Synthesis of ethyl 4-methoxy-3-methyl-3H-thieno[3,2-e]indazole-7-carboxylate (Intermediate 10)

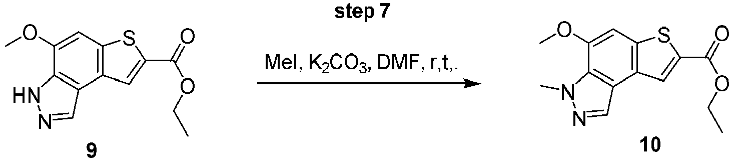

Intermediate Compound 9 (1.3 g, 4.7 mmol) prepared in the previous step was dissolved in DMF (15 mL), and methyl iodide (0.8 g, 5.7 mmol) and dipotassium carbonate (1.3 g, 9.4 mmol) were added to the mixture. The mixture was then stirred at room temperature for 1 hour. The reaction mixture was diluted with EtOAc (200 mL) and washed three times with ice-water (200 mL). The organic phase was dried over Na₂SO₄ and filtered. The filtrate was concentrated under vacuum to give a crude sample. The crude product was further purified by column chromatography (SiO₂, 30% EtOAc/petroleum ether) to give the product Intermediate Compound 10 as a yellow solid (0.6 g, 44.0%): ESI MS [M + H]⁺ for C₁₄H₁₄N₂O₃S, calcd 291.34, found 291.1.

### Step (VIII): Synthesis of 4-methoxy-3-methyl-3H-thieno[3,2-e]indazole-7-carboxylic acid (Intermediate 11)

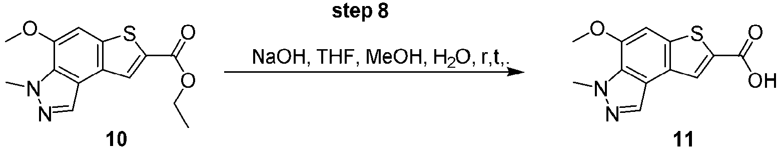

To a solution of Intermediate Compound 10 (1.0 g, 3.4 mmol) prepared in the previous step in THF (10 mL), MeOH (10 mL), and H₂O (2 mL) was added NaOH (688 mg, 17.2 mmol). The reaction mixture was stirred at room temperature for 3 hours. The reaction mixture was adjusted to pH = 5-6 and diluted with water (100 mL). The mixture was extracted three times with EtOAc (100 mL). The organic phase was collected, dried over Na₂SO₄, and concentrated under vacuum to give a crude sample. The crude product was further purified by column chromatography (SiO₂, 10% MeOH/DCM, 1% AcOH) to give the product Intermediate Compound 11 as a yellow solid: ESI MS[M + H]⁺ for C₁₂H₁₀N₂O₃S, calcd 263.3, found 263.0.

### Step (IX): Synthesis of 4-{4-methoxy-3-methyl-3H-thieno[3,2-e]indazole-7-carbonyl}morpholine (Intermediate 13)

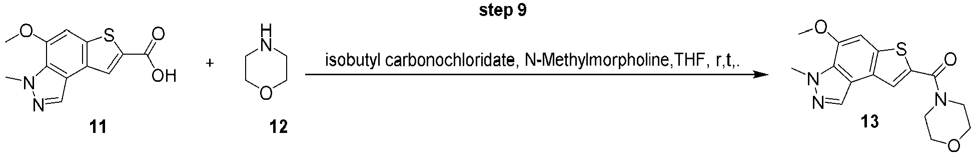

Intermediate Compound 11 (1.2 g, 4.6 mmol) prepared in the previous step was dissolved in THF (20 mL), and 4-methylmorpholine (2.3 g, 22.9 mmol) and 2-methylpropyl chloroformate (1.3 g, 9.2 mmol) were added. The mixture was purged with N₂ three times. The reaction mixture was sealed well and stirred at room temperature for 1 hour. Morpholine (2.0 g, 22.9 mmol) was added to the mixture, and then the mixture was stirred at room temperature for 1 hour.

The reaction mixture was diluted with EtOAc (200 mL) and washed twice with water (200 mL). The organic phase was dried over Na₂SO₄ and filtered. The filtrate was concentrated under vacuum to give a crude sample. The crude product was further purified by column chromatography (SiO₂, 30% EtOAc/petroleum ether) to give the product Intermediate Compound 13 as a yellow solid (200 mg, 13.2%): ESI MS[M + H]⁺ for C₁₆H₁₇N₃O₃S, calcd 332.39, found 332.1.

### Step (X): Synthesis of 1-{4-methoxy-3-methyl-3H-thieno[3,2-e]indazol-7-yl}ethan-1-one (Intermediate 14)

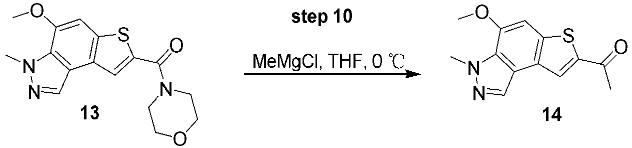

Intermediate Compound 13 (200.0 mg, 0.6 mmol) prepared in the previous step was dissolved in THF (15 mL). The solution was purged with N₂ three times and cooled to 0°C. Magnesium (2+) chloride dimethyl sulfide complex (134.6 mg, 1.8 mmol) was added to the mixture and then stirred at room temperature for 1 hour. The reaction mixture was quenched by the addition of water (100 mL) and extracted with ethyl acetate (100 mL). The organic phase was dried over Na₂SO₄ and filtered. The filtrate was concentrated under vacuum to give a crude sample. The crude product was further purified by column chromatography (SiO₂, 30% EtOAc/petroleum ether) to give the product Intermediate Compound 14 as a white solid (57.0 mg, 36.5%): ESI MS[M + H]⁺ for C₁₃H₁₂N₂O₂S, calcd 261.31, found 261.0.

### Step (XI): Synthesis of ethyl 4-(4-methoxy-3-methyl-3H-thieno[3,2-e]indazol-7-yl)-4-oxobutanoate (Intermediate Compound 16)

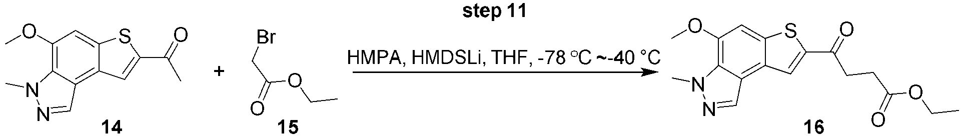

Intermediate Compound 14 (50.0 mg, 0.2 mmol) prepared in the previous step and HMPA (119.2 mg, 0.7 mmol) were dissolved in anhydrous THF (2 mL), and the mixture was purged with N₂ three times. The reaction mixture was cooled to -78°C under N₂ protection using an ethanol/CO₂(s) bath. HMDSLi (47.7 mg, 0.3 mmol) was then added dropwise and stirred at -40°C for 1.5 hours. Afterwards, the reaction mixture was cooled to -78°C. Ethyl 2-bromoacetate (63.5 mg, 0.4 mmol) was added dropwise and stirred at -78°C for 30 minutes. The reaction mixture was quenched into water (50 g) and then diluted with EtOAc (100 mL). The resulting mixture was extracted with EtOAc (100 mL). The organic phase was then washed three times with water (50 mL), dried over Na₂SO₄, and filtered. The filtrate was concentrated under vacuum to give the title compound, Intermediate Compound 16, as a yellow solid (30 mg, 45%): ESI MS[M + H]⁺ for C₁₇H₁₈N₂O₄S, calcd 347.4, found 347.05, which was used in the next step without further purification.

### Step (XII): Synthesis of 4-(4-methoxy-3-methyl-3H-thieno[3,2-e]indazol-7-yl)-4-oxobutanoic acid (Example Compound 15)

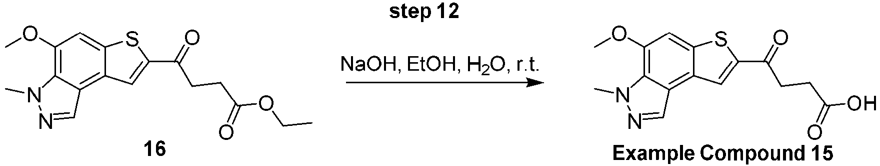

To a solution of Intermediate Compound 15 (30.0 mg, 0.1 mmol) prepared in the previous step in EtOH (6 mL) and H₂O (2 mL) was added NaOH (18.0 mg, 0.5 mmol). The reaction mixture was stirred at room temperature for 30 minutes. The reaction mixture was concentrated under vacuum to give a crude sample. The crude product was further purified by reverse-phase HPLC (C18 column, 30-50% gradient of MeCN and water containing 0.1% FA) to give the product Example Compound 15 as a white solid (8.9 mg, 31%): ESI MS [M + H] ⁺ for C₁₅H₁₄N₂O₄S, calcd 319.4, found 319.1. ¹H NMR (400 MHz, DMSO-d6) δ 12.21 (s, 1H), 8.68 (s, 1H), 8.37 (s, 1H), 7.50 (s, 1H), 4.28 (s, 3H), 4.05 (s, 3H), 3.30 (s, 2H), 2.63 (t, *J =* 6.5 Hz, 2H).

### Example 16

### Synthesis of 4-(4-methoxy-2-methylthieno[3,2-e]benzofuran-7-yl)-4-oxobutanoic acid (Example Compound 16)

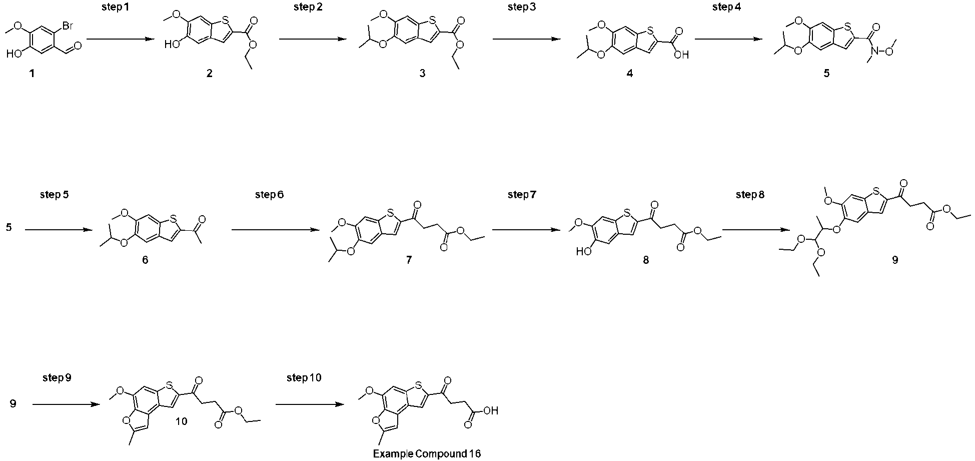

### Step (I): Synthesis of ethyl 5-hydroxy-6-methoxybenzo[b]thiophene-2-carboxylate (Intermediate 2)

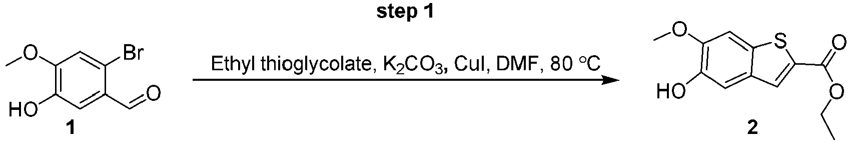

2-Bromo-5-hydroxy-4-methoxybenzaldehyde (10.0 g, 43.3 mmol) was dissolved in DMF (100 mL). CuI (2.5 g, 13.0 mmol) and ethyl 2-mercaptoacetate (12 g, 99.6 mmol) were then added and cooled to 0°C. K₂CO₃ (15 g, 108.2 mmol) was added to the mixture at 0°C. The reaction mixture was then sealed well and stirred at 80°C under N₂ protection for 3 hours.

The reaction mixture was cooled to room temperature, quenched into ice water (2.0 kg), and then diluted with EtOAc (1000 mL). The resulting mixture was extracted with EtOAc (1000 mL). The organic phase was then washed with water (1000 mL) and brine (500 mL), dried over Na₂SO₄, and filtered. The filtrate was concentrated under vacuum to give a crude sample. The crude product was further purified by column chromatography (SiO₂, petroleum ether:EtOAc = 5:1) to give the product Intermediate Compound 2 as a yellow solid (8.3 g, 76%): ESI MS [M + H]⁺ for C₁₂H₁₃O₄S, calcd 253.1, found 253.0.

### Step (II): Synthesis of ethyl 5-isopropoxy-6-methoxybenzo[b]thiophene-2-carboxylate (Intermediate 3)

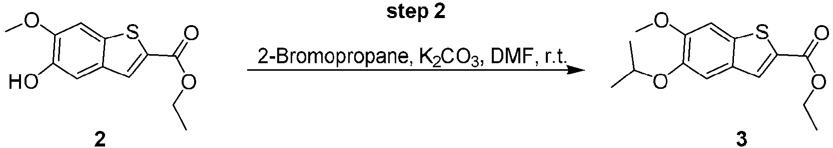

Intermediate Compound 2 (7.3 g, 28.9 mmol) prepared in the previous step was dissolved in DMF (70 mL), and K₂CO₃ (20.0 g, 144.7 mmol) and 2-bromopropane (10.7 g, 86.8 mmol) were added. The mixture was stirred at room temperature under N₂ protection for 48 hours. The reaction mixture was quenched into water (500 g). The resulting mixture was extracted with EtOAc (500 mL). The organic phase was washed five times with water (200 mL), dried over Na₂SO₄, and filtered. The filtrate was concentrated under vacuum to give the product Intermediate Compound 3 as a white solid (7.8 g, 92%): ESI MS [M + H]⁺ for C₁₅H₁₉O₄S, calcd 295.1, found 295.0; ¹H NMR (400 MHz, CDCl₃) δ 7.90 (s, 1H), 7.26 (s, 1H), 7.24 (s, 1H), 4.57 (hept, *J* = 6.1 Hz, 1H), 4.38 (q, *J =* 7.1 Hz, 2H), 3.93 (s, 3H), 1.40 (dd, *J =* 8.8, 6.6 Hz, 9H).

### Step (III): Synthesis of 5-isopropoxy-6-methoxybenzo[b]thiophene-2-carboxylic acid (Intermediate 4)

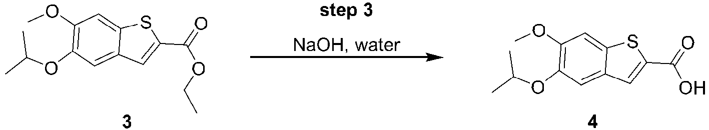

Intermediate Compound 3 (7.5 g, 25.5 mmol) prepared in the previous step was dissolved in water (30 mL), ethyl ethyl (75 mL), and sodium hydroxide (6.1 g, 152.9 mmol). The reaction mixture was stirred at 40°C for 1 hour.

The reaction mixture was adjusted to pH = 2-4 and diluted with water (500 mL). The mixture was extracted three times with EtOAc (500 mL). The organic phase was collected, dried over Na₂SO₄, and concentrated under vacuum to give the crude product Intermediate Compound 4 as a pale yellow solid (6.3 g, 93%): ESI MS [M + H]⁺ for C₁₃H₁₅O₄S, calcd 267.1, found 267.0, which was used in the next step without further purification.

### Step (IV): Synthesis of 5-isopropoxy-N,6-dimethoxy-N-methylbenzo[b]thiophene-2-carboxamide (Intermediate 5)

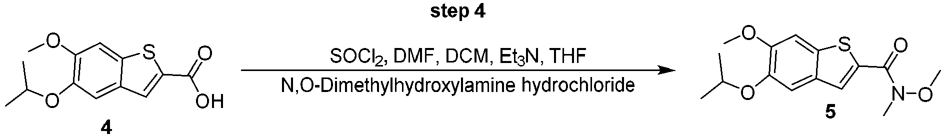

Intermediate Compound 5 (7.0 g, 26.3 mmol) prepared in the previous step was dissolved in DCM (100 mL), and thionyl chloride (15.6 g, 131.5 mmol) was added dropwise at room temperature. The reaction mixture was then refluxed at 50°C under N₂ protection for 1 hour. The reaction mixture was concentrated under vacuum to give a light brown sample. The residue was diluted with THF (70 mL) and quenched into an aqueous solution (7 mL) of N,O-dimethylhydroxylamine hydrochloride (7.7 g, 78.9 mmol). Water (200 mL) was then added, and the mixture was extracted three times with DCM (200 mL). The organic phase was collected, dried over Na₂SO₄, and filtered. The filtrate was concentrated under vacuum to give the product Intermediate Compound 5 as a pale yellow solid (7.3 g, 90%): ESI MS [M + H]⁺ for C₁₅H₂₀NO₄S, calcd 310.1, found 310.0; ¹H NMR (400 MHz, CDCl₃) δ 8.06 (s, 1H), 7.29 (s, 1H), 7.24 (s, 1H), 4.58 (hept, *J =* 6.1 Hz, 1H), 3.93 (s, 3H), 3.81 (s, 3H), 3.40 (s, 3H), 1.41 (d, *J =* 6.1 Hz, 6H), which was used in the next step without further purification.

### Step (V): Synthesis of 1-(5-isopropoxy-6-methoxybenzo[b]thiophen-2-yl)ethan-1-one (Intermediate 6)

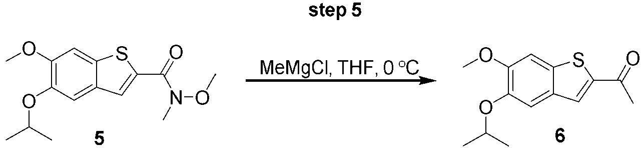

Intermediate Compound 5 (6.9 g, 22.3 mmol) prepared in the previous step was dissolved in THF (70 mL), and 3.0M methylmagnesium chloride in THF (22.3 mL, 66.9 mmol) was added dropwise at 0°C. The reaction mixture was stirred at room temperature for 1 hour. The reaction mixture was quenched into water (100 g) and then diluted with EtOAc (200 mL). The resulting mixture was extracted with EtOAc (200 mL). The organic phase was washed three times with water (50 mL), dried over Na₂SO₄, and filtered. The filtrate was concentrated under vacuum to give a crude sample. The crude product was further purified by column chromatography (SiO₂, petroleum ether:EtOAc = 10:1) to give the title compound, Intermediate Compound 6, as a white solid (5.1 g, 87%): ESI MS [M + H]⁺ for C₁₄H₁₇O₃S, calcd 265.1, found 265.0; ¹H NMR (400 MHz, CDCl₃) δ 7.80 (s, 1H), 7.28 (s, 1H), 7.25 (s, 1H), 4.57 (hept, *J=* 6.1 Hz, 1H), 3.94 (s, 3H), 2.62 (s, 3H), 1.42 (d, *J=* 6.1 Hz, 6H).

### Step (VI): Synthesis of ethyl 4-(5-isopropoxy-6-methoxybenzo[b]thiophen-2-yl)-4-oxobutanoate (Intermediate 7)

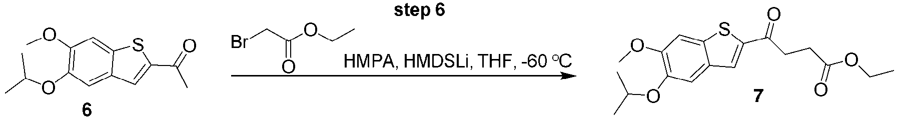

Intermediate Compound 6 (4.6 g, 17.4 mmol) prepared in the previous step and HMPA (60.9 mg, 10.9 mmol) were dissolved in anhydrous THF (50 mL), and the mixture was purged with N₂ three times. The reaction mixture was cooled to -78°C under N₂ protection. Then, 1M HMDSLi in THF (26.1 mL, 26.1 mmol) was added dropwise and stirred at -40°C for 1 hour. The reaction mixture was then cooled to -78°C, and ethyl 2-bromoacetate (5.8 g, 34.8 mmol) was added dropwise. The mixture was stirred at -78°C under a N₂ atmosphere for 1 hour. The reaction mixture was quenched into water (100 g) and then diluted with EtOAc (200 mL). The resulting mixture was extracted with EtOAc (200 mL). The organic phase was then washed three times with water (100 mL), dried over Na₂SO₄, and filtered. The filtrate was concentrated under vacuum to give a crude sample. The crude product was further purified by column chromatography (SiO₂, petroleum ether:EtOAc = 10:1) to give the title compound, Intermediate Compound 7, as a white solid (5.1 g, 84%): ESI MS [M + H]⁺ for C₁₈H₂₃O₅S, calcd 351.1, found 351.0; ¹H NMR (400 MHz, CDCl₃) δ 7.87 (s, 1H), 7.28 (s, 1H), 7.25 (s, 1H), 4.58 (hept, J = 6.1 Hz, 1H), 4.16 (q, J = 7.1 Hz, 2H), 3.94 (s, 3H), 3.31 (t, J = 6.8 Hz, 2H), 2.78 (t, J = 6.8 Hz, 2H), 1.42 (d, J = 6.1 Hz,6H), 1.26 (t, J = 7.1 Hz, 4H).

### Step (VII): Synthesis of ethyl 4-(5-hydroxy-6-methoxybenzo[b]thiophen-2-yl)-4-oxobutanoate (Intermediate 8)

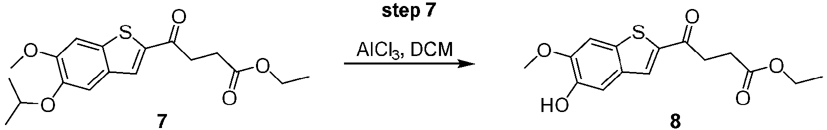

Intermediate Compound 7 (5.1 g, 14.6 mmol) prepared in the previous step was dissolved in DCM (50 mL), and AlCl₃ (2.9 g, 21.8 mmol) was added. The reaction mixture was stirred at room temperature for 16 hours. The reaction mixture was quenched into ice-water (100 g) and then diluted with DCM (200 mL). The resulting mixture was extracted with DCM (200 mL). The organic phase was washed twice with water (100 mL), dried over Na₂SO₄, and filtered. The filtrate was concentrated under vacuum to give a crude sample. The crude product was further purified by column chromatography (SiO₂, petroleum ether:EtOAc = 3:1) to give the title compound, Intermediate Compound 8, as a white solid (3.7 g, 82%): ESI MS [M + H]⁺ for C₁₅H₁₇O₅S, calcd 309.1, found 309.0; ¹H NMR (400 MHz, DMSO) δ 9.37 (s, 1H), 8.13 (s, 1H), 7.49 (s, 1H), 7.27 (s, 1H), 4.01 (q, *J =* 7.1 Hz, 2H), 3.82 (s, 3H), 3.24 (t, *J =* 6.4 Hz, 2H), 2.60 (t, *J=* 6.4 Hz, 2H), 1.13 (t, *J* = 7.1 Hz, 3H).

### Step (VIII): Synthesis of ethyl 4-(5-((1,1-diethoxypropan-2-yl)oxy)-6-methoxybenzo[b]thiophen-2-yl)-4-oxobutanoate (Intermediate 9)

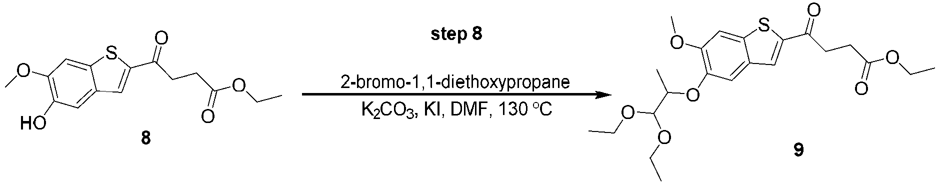

Intermediate Compound 8 (1.0 g, 3.2 mmol) prepared in the previous step was dissolved in DMF (30 mL), and then KI (107.6 g, 0.7 mmol), 2-bromo-1,1-diethoxypropane (6.8 g, 32.4 mmol), and K₂CO₃ (2.2 g, 16.2 mmol) were added. The reaction mixture was then sealed well and stirred at 130°C for 16 hours. The reaction mixture was cooled to room temperature, quenched into ice water (200 mL), and then diluted with EtOAc (200 mL). The resulting mixture was extracted with EtOAc (200 mL). The organic phase was then washed with water (100 mL), dried over Na₂SO₄, and filtered. The filtrate was concentrated under vacuum to give a crude sample. The crude product was further purified by column chromatography (SiO₂, petroleum ether:EtOAc = 3:1) to give the product Intermediate Compound 9 as a white solid (250 mg, 18%): ESI MS [M + H]⁺ for C₂₂H₃₁O₇S, calcd 439.2, found 439.1.

### Step (IX): Ethyl 4-(4-methoxy-2-methylthieno[3,2-e]benzofuran-7-yl)-4-oxobutanoate (Intermediate 10)

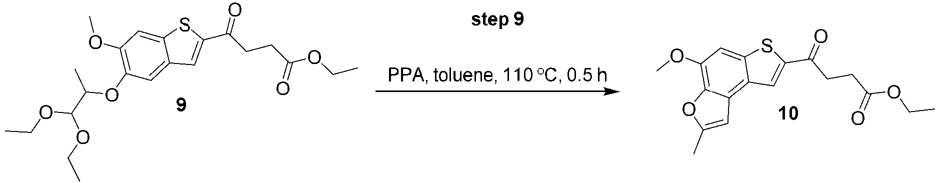

Intermediate Compound 9 (100 mg, 0.2 mmol) prepared in the previous step was dissolved in toluene (2 mL), and PPA (1 mL) was added. The reaction mixture was stirred at 110°C for 0.5 hour. The reaction mixture was cooled to room temperature, quenched into ice-water (20 mL), and then diluted with EtOAc (50 mL). The resulting mixture was extracted with EtOAc (50 mL). The organic phase was then washed twice with water (20 mL), dried over Na₂SO₄, and filtered. The filtrate was concentrated under vacuum to give a crude sample. The crude product was further purified by column chromatography (SiO₂, petroleum ether:EtOAc = 5:1) to give the product Intermediate Compound 10 as a white solid (25 mg, 31%): ESI MS [M + H]⁺ for C₁₈H₁₉O₅S, calcd 347.1, found 347.0.

### Step (X): Synthesis of 4-(4-methoxy-2-methylthieno[3,2-e]benzofuran-7-yl)-4-oxobutanoic acid (Example 16)

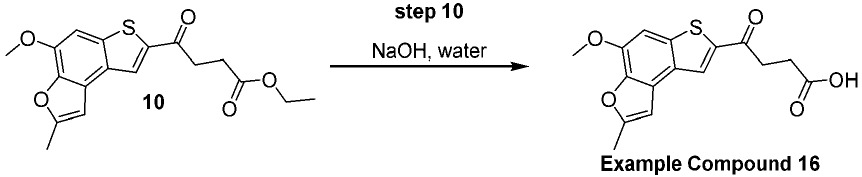

Intermediate Compound 10 (25 mg, 0.07 mmol) prepared in the previous step was dissolved in water (1 mL), EtOH (1 mL), and NaOH (14 mg, 0.4 mmol). The reaction mixture was stirred at room temperature for 1 hour. LCMS at this point indicated no more starting material. The reaction mixture was adjusted to pH = 3-4 and diluted with water (20 mL). The mixture was extracted three times with EtOAc (20 mL). The organic phase was collected, dried over Na₂SO₄, and concentrated under vacuum to give a crude sample. The crude product was further purified by reverse-phase HPLC (C18 column, 40-60% gradient of MeCN and water containing 0.1% TFA) to give the product Example Compound 16 as a white solid: ESI MS [M + H]⁺ for C₁₆H₁₅O₅S, calcd 319.1, found 319.0. ¹H NMR (400 MHz, DMSO-d6) δ 8.59 (s, 1H), 7.51 (s, 1H), 7.04 (s, 1H), 4.00 (s, 3H), 3.30 (t, *J =* 6.4 Hz, 2H), 2.61 (t, *J=* 6.4 Hz, 2H), 2.53 (s, 3H).

### Example 17

### Synthesis of 4-(4-methoxythieno[3,2-e]benzofuran-7-yl)-2-methyl-4-oxobutanoic acid (Example Compound 17)

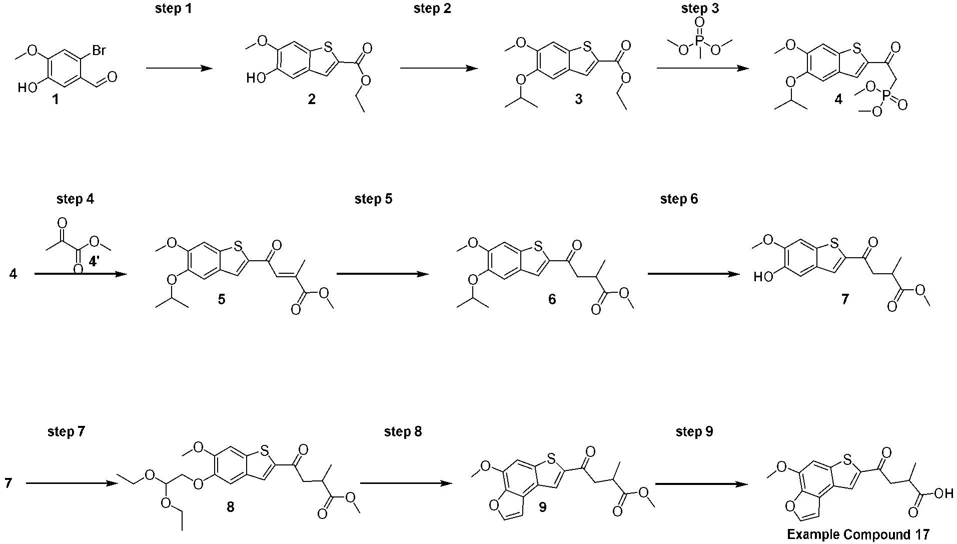

### Step (I): Synthesis of ethyl 5-hydroxy-6-methoxybenzo[b]thiophene-2-carboxylate (Intermediate 2)

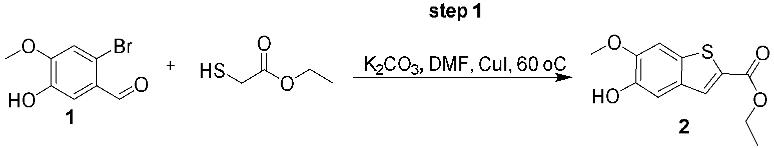

A solution of 2-bromo-5-hydroxy-4-methoxybenzaldehyde (20.0 g, 86.6 mmol), ethyl 2-mercaptoacetate (20.8 g, 173.1 mmol), and CuI (1.7 g, 8.7 mmol) in DMF (300 mL) and K₂CO₃ (35.9 g, 35.9 mmol) was added to the reaction at 0°C. The mixture was then purged with N₂ three times. The reaction mixture was sealed well and stirred at 60°C under N₂ protection for 2 hours. The reaction mixture was cooled to room temperature, quenched with water (600 mL), and extracted three times with EtOAc (500 mL). The organic phase was washed three times with brine (200 mL), dried over Na₂SO₄, and concentrated under vacuum to give a crude sample. The crude product was further purified by column chromatography (SiO₂, petroleum ether:EtOAc = 5:1) to give the product Intermediate Compound 2 as a white solid (17 g, 78%): ESI MS [M + H]⁺ for C₁₂H₁₃NO₄S, calcd 253.2, found 253.0.

### Step (II): Synthesis of ethyl 5-isopropoxy-6-methoxybenzo[b]thiophene-2-carboxylate (Intermediate 3)

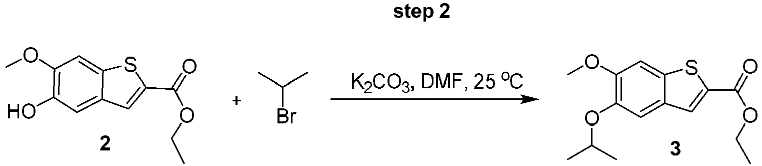

A solution of Intermediate Compound 2 (10.0 g, 40.0 mmol) prepared in the previous step and 2-bromopropane (14.6 g, 118.9 mmol) in DMF (150 mL) and K₂CO₃ (16.4 g, 118.9 mmol) was added to the reaction at 0°C. The mixture was then purged with N₂ three times. The reaction mixture was sealed well and stirred at 25°C under N₂ protection for 18 hours. The reaction mixture was cooled to room temperature, quenched with water (400 mL), and extracted three times with EtOAc (300 mL). The organic phase was washed three times with brine (100 mL), dried over Na₂SO₄, and concentrated under vacuum to give the crude product Intermediate Compound 3 as a brown solid (12.0 g, 102%): ESI MS [M + H]⁺ for C₁₅H₁₉NO₄S, calcd 295.3, found 295.0, which was used in the next step without further purification.

### Step (III): Synthesis of dimethyl (2-(5-isopropoxy-6-methoxybenzo[b]thiophen-2-yl)-2-oxoethyl)phosphonate (Intermediate 4)

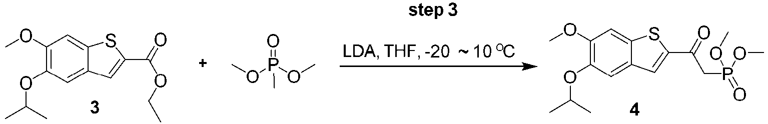

Intermediate Compound 3 (3.0 g, 10.2 mmol) prepared in the previous step and dimethyl methylphosphonate (1.3 g, 10.2 mmol) were added to THF (30 mL), and LDA (2.2 g, 20.4 mmol) was slowly added at -20°C under N₂ protection. The reaction mixture was then stirred at room temperature for 2 hours. The reaction mixture was quenched with water (100 mL) and extracted three times with EtOAc (100 mL). The organic phase was dried over Na₂SO₄ and concentrated under vacuum to give a crude sample. The crude product was further purified by column chromatography (SiO₂, EtOAc) to give the title compound, Intermediate Compound 4, as a yellow solid (2.6 g, 69%): ESI MS [M + H]⁺ for C₁₆H₂₂NO₆PS, calcd 373.3, found 373.1.

### Step (IV): Synthesis of methyl (E)-4-(5-isopropoxy-6-methoxybenzo[b]thiophen-2-yl)-2-methyl-4-oxobut-2-enoate (Intermediate 5)

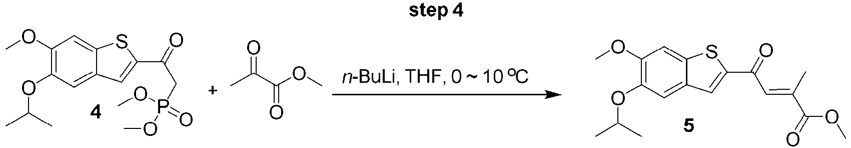

Intermediate Compound 4 (2.7 g, 7.3 mmol) prepared in the previous step was dissolved in THF (35 mL), and n-BuLi (650.2 g, 10.2 mmol) was added dropwise at 0°C. The reaction mixture was stirred at 0°C for 1 hour. Methyl 2-oxopropanoate (1.1 g, 10.9 mmol) was then added to the mixture and stirred at room temperature under N₂ protection for 10 minutes. The reaction mixture was quenched into water (50 mL) and then diluted three times with EtOAc (50 mL). The organic phase was then dried over Na₂SO₄ and concentrated under vacuum to give a crude sample. The crude product was further purified by column chromatography (SiO₂, petroleum ether:EtOAc = 8:1) to give the title compound, Intermediate Compound 5, as a yellow solid (1.5 g, 59%): ESI MS [M + H]⁺ for C₁₈H₂₁O₅S, calcd 349.5, found 349.1.

### Step (V): Synthesis of methyl 4-(5-isopropoxy-6-methoxybenzo[b]thiophen-2-yl)-2-methyl-4-oxobutanoate (Intermediate 6)

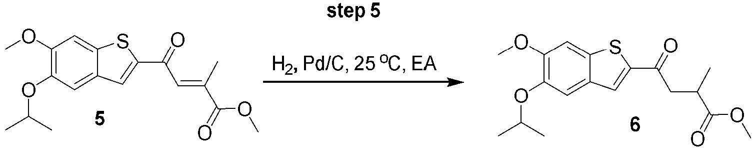

A solution of Intermediate Compound 5 (1.0 g, 2.9 mmol) prepared in the previous step and Pd/C (305.4 mg, 2.9 mmol) was added to EA (30 mL). The mixture was then purged with H₂ three times. The reaction mixture was stirred at 25°C for 4 hours. The reaction mixture was filtered and concentrated under vacuum to give the crude compound, Intermediate Compound 6, as a yellow solid (1.0 g, 99%): ESI MS [M + H]⁺ for C₁₈H₂₃NO₅, calcd 351.4, found 351.1, which was used in the next step without further purification.

### Step (VI): Synthesis of methyl 4-(5-hydroxy-6-methoxybenzo[b]thiophen-2-yl)-2-methyl-4-oxobutanoate (Intermediate 7)

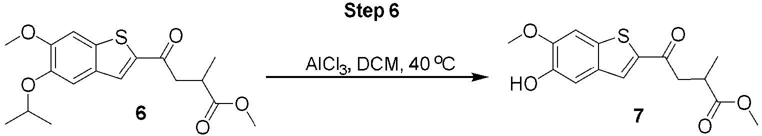

A solution of Intermediate Compound 6 (350.0 mg, 1.0 mmol) prepared in the previous step and AlCl₃ (266.4 mg, 2.0 mmol) was added to DCM (10 mL), and the mixture was purged with N₂ three times. The reaction mixture was stirred at 40°C for 4 hours. The reaction mixture was quenched into water (50 mL) and then extracted three times with EtOAc (50 mL). The organic phase was then dried over Na₂SO₄ and concentrated under vacuum to give the crude compound, Intermediate Compound 7, as a yellow solid (250.0 mg, 81%): ESI MS [M + H]⁺ for C₁₅H₁₇O₅S, calcd 309.1, found 309.0, which was used in the next step without further purification.

### Step (VII): Synthesis of methyl 4-(5-(2,2-diethoxyethoxy)-6-methoxybenzo[b]thiophen-2-yl)-2-methyl-4-oxobutanoate (Intermediate 8)

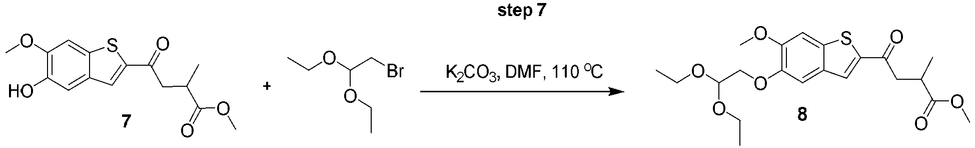

Intermediate Compound 7 (160.0 mg, 0.5 mmol) prepared in the previous step, 2-bromo-1,1-diethoxyethane (122.7 mg, 0.6 mmol), and K₂CO₃ (119.8 mg, 0.87 mmol) were added to DMF (2.0 mL) solution, and the mixture was purged with N₂ three times. The reaction mixture was stirred at 110°C for 4 hours. The reaction mixture was cooled to room temperature, quenched with water (20 mL), and extracted three times with EtOAc (30 mL). The organic phase was washed three times with brine (30 mL), dried over Na₂SO₄, and concentrated under vacuum to give the crude compound, Intermediate Compound 8, as a yellow oil (150.0 mg, 68%): ESI MS [M + H]⁺ for C₂₁H₂₉O₇S, calcd 425.2, found 425.1, which was used in the next step without further purification.

### Step (VIII): Synthesis of methyl 4-(4-methoxythieno[3,2-e]benzofuran-7-yl)-2-methyl-4-oxobutanoate (Intermediate Compound 9)

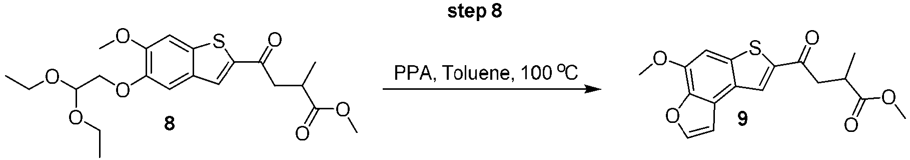

Intermediate Compound 8 (190.0 mg, 0.5 mmol) prepared in the previous step and PPA (0.1 mL, 1.2 mmol) were dissolved in toluene (2.0 mL). The reaction mixture was stirred at 100°C for 0.5 hour.

The reaction mixture was diluted with EA (100 mL), and the EA layer was concentrated under vacuum to give a crude sample. The residue was further purified by column chromatography (SiO₂, petroleum ether:EtOAc = 3:1) to give the title compound, Intermediate Compound 9, as a yellow oil (70.0 mg, 47%): ESI MS [M + H]⁺ for C₁₇H₁₇O₅S, calcd 333.1, found 333.0.

### Step (IX): Synthesis of 4-(4-methoxythieno[3,2-e]benzofuran-7-yl)-2-methyl-4-oxobutanoic acid (Example Compound 17)

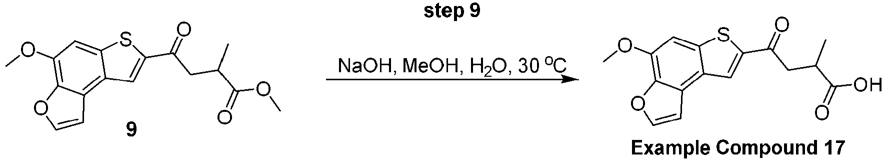

Intermediate Compound 9 (60.0 mg, 0.18 mmol) prepared in the previous step and NaOH (72.0 mg, 1.8 mmol) were dissolved in MeOH (5.0 mL) and H₂O (1.0 mL). The reaction mixture was stirred at 30°C for 3 hours. The reaction mixture was concentrated under vacuum to give a crude sample. The crude product was further purified by reverse-phase HPLC (C18 column, 40-45% gradient of MeCN and water containing 0.1% FA) to give the product Example Compound 17 as a white solid: ESI MS [M + H]⁺ for C₁₆H₁₅NO₅S, calcd 319.1, found 319.0. ¹H NMR (400 MHz, DMSO-*d*₆) δ 12.23 (s, 1H), 8.69 (s, 1H), 8.16 (d, *J=* 2.0 Hz, 1H), 7.61 (s, 1H), 7.44 (d, *J=* 2.1 Hz, 1H), 4.04 (s, 3H), 3.52 - 3.42 (m, 1H), 3.12 (s, 1H), 2.94 (q, *J =* 7.1 Hz, 1H), 1.21 (d, *J =* 7.2 Hz, 3H).

### Example 18

### Synthesis of 4-(4-methoxy-2,8-dimethylthieno[3,2-e]benzofuran-7-yl)-4-oxobutanoic acid (Example Compound 18)

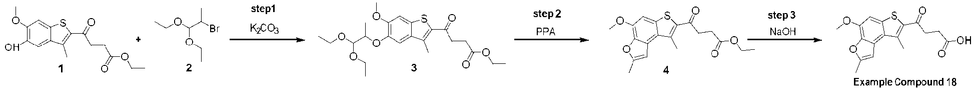

### Step (I): Synthesis of ethyl 4-(5-(2,2-diethoxyethoxy)-6-methoxy-1-benzothiophene)-2-ethyl-4-oxobutanoate (Intermediate 3)

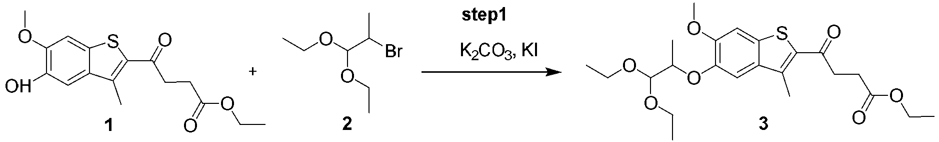

Ethyl 4-(5-hydroxy-6-methoxy-3-methylbenzo-1-thiophen-2-yl)-4-oxobutanoate (1.0 g, 3.1 mmol) was dissolved in DMF (15 mL), and 2-bromo-1,1-diethoxypropane (3.3 g, 15.5 mmol) was added. The mixture was then cooled to 0°C, and dipotassium carbonate (2.1 g, 15.5 mmol) and potassium iodide (103.0 mg, 0.62 mmol) were slowly added. The mixture was purged with N₂ three times. The reaction mixture was then sealed and stirred at 130°C under N₂ protection for 12 hours. The reaction mixture was quenched into ice-water (600 g) to maintain the internal temperature below 20°C. The resulting mixture was extracted with EtOAc (600 mL). The organic phase was then washed with water (400 mL) and brine (200 mL), dried over Na₂SO₄, and filtered. The filtrate was concentrated under vacuum to give a crude sample. The crude product was further purified by column chromatography (SiO₂, 20% EtOAc/petroleum ether) to give Intermediate 3 as a white solid (260.0 mg, 18.5%), ¹H NMR (400 MHz, Chloroform-d) δ 7.38 (s, 1H), 7.21 (s, 1H), 4.60 (d, *J=* 5.4 Hz, 1H), 4.42 (p, *J* = 6.2 Hz, 1H), 4.17 (q, *J* = 7.1 Hz, 4H), 3.93 (s, 3H), 3.24 (t, *J =* 6.6 Hz, 3H), 2.75 (t, *J =* 6.6 Hz, 3H),2.69 (s, 5H), 1.40 (d, *J =* 6.5 Hz, 3H), 1.28 (dd, *J* = 7.3, 1.6 Hz, 3H), 1.17 (t, *J=* 7.1 Hz, 3H).

### Step (II): Synthesis of ethyl 4-(4-methoxy-2,8-dimethylthieno[3,2-e]benzofuran-7-yl)-4-oxobutanoate (Intermediate 4)

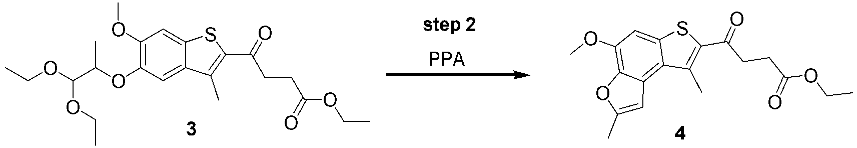

Methyl 4-(5-(2,2-diethoxyethoxy)-6-methoxy-1-benzothiophene)-2-ethyl-4-oxobutanoate (100.0 mg, 0.22 mmol) was dissolved in toluene (5 mL), and polyphosphoric acid (18.10 mg, 0.22 mmol) was added. The mixture was purged with N₂ three times. The reaction mixture was then sealed and stirred at 80°C under N₂ protection for 15 min. The reaction mixture was quenched into ice-water (60 g) to maintain the internal temperature below 20°C. The resulting mixture was extracted with EtOAc (60 mL). The organic phase was then washed with water (40 mL) and brine (20 mL), dried over Na₂SO₄, and filtered. The filtrate was concentrated under vacuum to give a crude sample. The crude product was further purified by column chromatography (SiO₂, 20% EtOAc/petroleum ether) to give Intermediate 4 as a white solid (40 mg, 50.2%), LCMS: ESI MS[M + H]⁺ for C₁₉H₂₀O₅S, calcd 361.4, found 361.06.

### Step (III): Synthesis of 4-(4-methoxy-2,8-dimethylthieno[3,2-e]benzofuran-7-yl)-4-oxobutanoic acid (Example Compound 18)

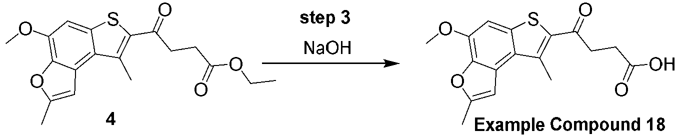

Ethyl 4-(4-methoxy-2,8-dimethylthieno[3,2-e]benzofuran-7-yl)-4-oxobutanoate (40 mg, 0.11 mmol) was dissolved in methanol (5 mL). The mixture was then cooled to 0°C, and a solution of sodium hydroxide (22.0 mg, 0.6 mmol) in water (5 mL) was slowly added. The mixture was purged with N₂ three times. The reaction mixture was then sealed and stirred at 25°C under N₂ protection for 12 hours. The reaction was quenched with ice-water. The reaction mixture was extracted with EA and concentrated to give a crude product. The crude product was then purified by prep-HPLC (column: Hanbang DAC-50, fillers: NanoMicro UniHybrid 10-120 C18, mobile phase: 0.1% aqueous NH₄HCO₃ solution and acetonitrile, eluent: 50% ACN) to give the pure final product as a white solid (2.4 mg, 6.6%), LCMS: ESI MS[M + H]⁺ for C₁₇H₁₆O₅S, calcd 333.4, found 332.90. ¹H NMR (400 MHz, DMSO-d6) δ 12.19 (s, 1H), 7.49 (s, 1H), 7.16 (s, 1H), 4.01 (s, 3H), 3.18 (t, *J =* 6.2 Hz, 2H), 2.90 (s, 3H), 2.59 (t, *J =* 6.2 Hz, 2H), 2.53 (s, 3H).

### Example 19

### Synthesis of 4-(4-methoxy-2-methylthieno[3,2-e]benzofuran-7-yl)-2-methyl-4-oxobutanoic acid (Example Compound 19)

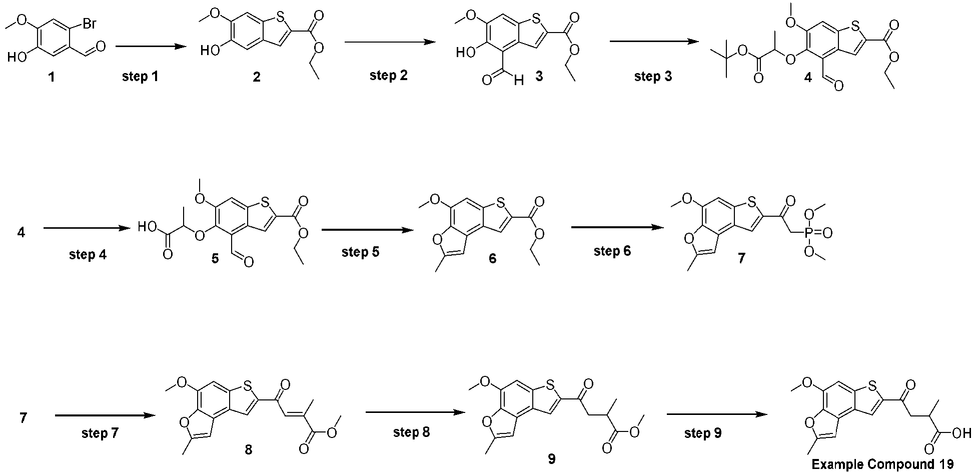

### Step (I): Synthesis of ethyl 5-hydroxy-6-methoxybenzo[b]thiophene-2-carboxylate (Intermediate 2)

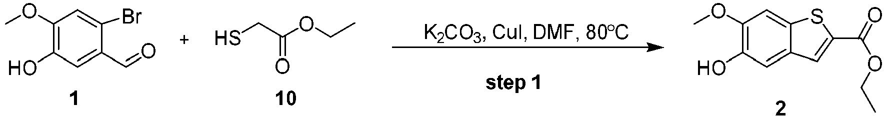

2-Bromo-5-hydroxy-4-methoxybenzaldehyde (50 g, 216.4 mmol) was dissolved in DMF (500 mL), and then ethyl 2-mercaptoacetate (59.8 g, 497.7 mmol) and CuI (12.4 g, 64.9 mmol were added. The temperature was then controlled to 0°C, and K₂CO₃ (74.8 g, 541.0 mmol) was added. The mixture was purged with nitrogen, warmed to 80°C, and stirred for 3 h. After completion of the reaction, the reaction solution was cooled to room temperature, and 5 L of water was added. The mixture was extracted with 3 L of EA, and the layers were separated. The organic phase was washed twice with 3 L of water, washed with 1 L of saturated brine, dried over Na₂SO₄, and filtered. The filtrate was concentrated under reduced pressure to give a crude product. The crude product was further purified by column chromatography (SiO₂, EA/petroleum ether = 3/1) to give Intermediate 2 (46 g, 84%) as a yellow solid. ESI MS[M + H]⁺ for C₁₂H₁₃O₄S, calcd 253.29, found 253.0.

### Step (II): Synthesis of ethyl 4-formyl-5-hydroxy-6-methoxybenzo[b]thiophene-2-carboxylate (Intermediate 3)

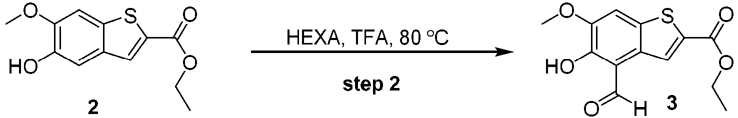

HEXA (76.7 g, 547.0 mmol) was dissolved in TFA (460 mL) in an ice-water bath, and then ethyl 5-hydroxy-6-methoxybenzo[b]thiophene-2-carboxylate (46 g, 182.3 mmol) was added. The mixture was purged with nitrogen, warmed to 70°C, and stirred overnight. The reaction solution was cooled to room temperature, and 5 L of water was added to the reaction solution. The mixture was extracted with 3 L of DCM, and the layers were separated. The aqueous phase was extracted twice with 2 L of DCM. The organic phases were combined, washed twice with 2 L of water, once with 2 L of saturated aqueous ammonium chloride solution, once with 2 L of 5% aqueous NaHCO₃ solution, and finally once with 2 L of pure water. The organic phase was dried over Na₂SO₄ and filtered. The filtrate was concentrated under reduced pressure to give a crude product. The crude product was further purified by trituration with 100 mL of mixed solvent (PE:EA = 1:1) and filtered. The filter cake was collected and dried by rotary evaporation to give Intermediate 3 (37 g, 72%) as a yellow solid, ESI MS[M + H]⁺ for C₁₃H₁₃O₅S, calcd 281.3, found 281.0.

### Step (III): Synthesis of ethyl 5-((1-(tert-butoxy)-1-oxopropan-2-yl)oxy)-4-formyl-6-methoxybenzo[b]thiophene-2-carbox ylate (Intermediate 4)

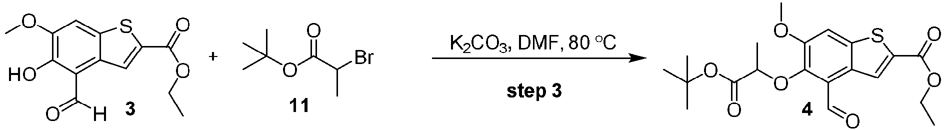

Ethyl 4-formyl-5-hydroxy-6-methoxybenzo[b]thiophene-2-carboxylate (27 g, 96.3 mmol) was dissolved in DMF (270 mL), and then tert-butyl 2-bromopropionate (24.2 g, 115.6 mmol) and K₂CO₃ (39.9 g, 289.0 mmol) were added. The mixture was purged with nitrogen, warmed to 80°C, and stirred for 1 h. After completion of the reaction, the reaction solution was cooled to room temperature and filtered, and 3 L of water was added to the filtrate. The mixture was extracted with 2 L of EA, and the layers were separated. The organic phase was washed twice with 3 L of water and once with 1 L of saturated brine. The organic phase was dried over anhydrous Na₂SO₄ and filtered. The filtrate was concentrated under reduced pressure to give a crude product. The crude product was purified by trituration (PE:EA = 10:1) to give Intermediate 4 (27 g, 69%) as a yellow solid, ESI MS[M + Na]⁺ for C₂₀H₂₅O₇S, calcd 431.47, found 431.06.

### Step (IV): Synthesis of 2-((2-(ethoxycarbonyl)-4-formyl-6-methoxybenzo[b]thiophen-5-yl)oxy)propanoic acid (Intermediate 5)

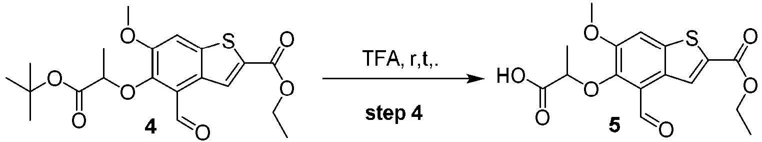

### Ethyl

5-((1-(tert-butoxy)-1-oxopropan-2-yl)oxy)-4-formyl-6-methoxybenzo[b]thiophene-2-carboxylate (38 g, 93.0 mmol) was dissolved in TFA (106.1 g, 930.3 mmol) and stirred at room temperature for 1 h. After the reaction was monitored to be complete by TLC, 1 L of ice water was added to the reaction solution. The mixture was extracted with 1 L of DCM, and the layers were separated. The organic phase was washed twice with 1 L of water, once with 500 mL of saturated aqueous sodium bicarbonate solution, and once with 500 mL of saturated brine, dried over Na₂SO₄, and concentrated under reduced pressure to give a crude product. The crude product was triturated with 100 mL of methyl tert-butyl ether and filtered. The filter cake was collected and dried by rotary evaporation to give Intermediate 5 (30 g, 92%) as a yellow solid, ESI MS[M + H]⁺ for C₁₆H₁₇O₇S, calcd 353.36, found 353.05.

### Step (V): Synthesis of ethyl 4-methoxy-2-methylthieno[3,2-e]benzofuran-7-carboxylate (Intermediate 6)

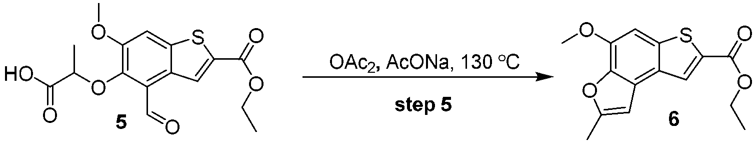

2-((2-(Ethoxycarbonyl)-4-formyl-6-methoxybenzo[b]thiophen-5-yl) oxy)propanoic acid (27 g, 76.6 mmol) was dissolved in acetic anhydride (270 mL), and then sodium acetate (27.9 g, 283.8 mmol) was added. The mixture was purged with nitrogen, warmed to 130°C, and stirred for 2 h. After completion of the reaction, the reaction solution was cooled to room temperature, and 2 L of water was added to the reaction solution. The mixture was extracted with 2 L of EA, and the layers were separated. The organic phase was washed twice with 2 L of water and then once with 1 L of saturated aqueous NaHCO₃ solution. The organic phase was concentrated under reduced pressure to give a crude product. The crude product was further purified by column chromatography (SiO₂, EA/petroleum ether = 1/60) to give Intermediate 6 (20 g, 90%) as a yellow solid, ESI MS[M + H]⁺ for C₁₅H₁₅O₄S, calcd 291.33, found 291.1.

### Step (VI): Synthesis of dimethyl (2-(4-methoxy-2-methylthieno [3,2-e]benzofuran-7-yl)-2-oxoethyl)phosphonate (Intermediate 7)

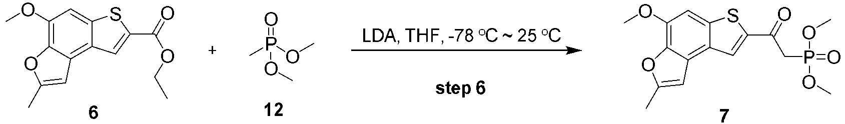

Dimethyl methylphosphonate (7.7 g, 62.0 mmol) and ethyl 4-methoxy-2-methylthieno[3,2-e]benzofuran-7-carboxylate (18.0 g, 62.0 mmol) were dissolved in THF (180 mL). The mixture was purged with nitrogen, cooled to -78°C, and then LDA (62 mL, 2 M) was added. The mixture was slowly returned to room temperature and stirred for 3 h. After completion of the reaction, 500 mL of water was added to the reaction solution. The mixture was extracted with 500 mL of EA, and the layers were separated. The organic phase was washed once with 500 mL of brine and concentrated under reduced pressure to give a crude product. The crude product was further purified by column chromatography (SiO₂, pure EA) to give Intermediate 7 (16.0 g, 70%) as a yellow solid, ESI MS[M + H]⁺ for C₁₆H₁₈O₆PS, calcd 369.34, found 369.1.

### Step (VII): Synthesis of methyl (E)-4-(4-methoxy-2-methylthieno[3,2-E]benzofuran-7-yl)-2-methyl-4-oxobut-2-enoate (Intermediate 8)

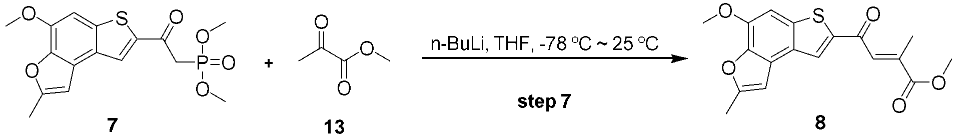

Dimethyl (2-(4-methoxy-2-methylthieno[3,2-e]benzofuran-7-yl)-2-oxoethyl)phosphonate (23.0 g, 62.4 mmol) was dissolved in THF (500 mL). The mixture was purged with nitrogen and cooled to -78°C. n-Butyllithium (25 mL, 2.5 M) was then added and stirred for 0.5 h, followed by methyl 2-oxopropanoate (6.4 g, 62.4 mmol). The mixture was returned to room temperature and stirred for 1 h. After completion of the reaction, 300 mL of saturated aqueous ammonium chloride solution was added to the reaction solution. The mixture was extracted with 500 mL of EA, and the layers were separated. The organic phase was washed once with 500 mL of brine and concentrated under reduced pressure to give a crude product. The crude product was further purified by column chromatography (SiO₂, PE/EA/DCM = 20/1/1) to give Intermediate 8 (12 g, 56%) as a yellow solid, ESI MS[M + H]⁺ for C₁₈H₁₇O₅S, calcd 345.38, found 345.1.

### Step (VIII): Synthesis of methyl 4-(4-methoxy-2-methylthieno[3,2-e]benzofuran-7-yl)-2-methyl-4-oxobutanoate

### (Intermediate 9)

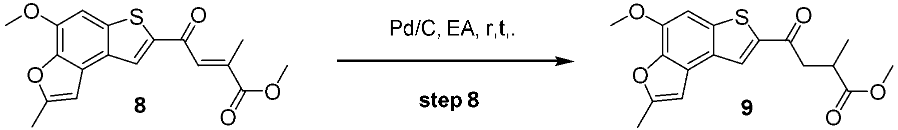

Methyl (E)-4-(4-methoxy-2-methylthieno[3,2-E]benzofuran-7-yl)-2-methyl-4-oxobut-2-enoate (12 g, 34.9 mmol) was dissolved in EA (250 mL), and then Pd/C (1.2 g, 11.3 mmol) was added. The mixture was purged with hydrogen and stirred at room temperature overnight. After completion of the reaction, the reaction solution was filtered through celite, and the celite was rinsed with 500 mL of EA. The filtrate was concentrated under reduced pressure to give a crude product. The crude product was further purified by column chromatography (SiO₂, PE/EA/DCM = 20/1/1) to give Intermediate 9 (6 g, 50%) as a yellow solid, ESI MS[M + H]⁺ for C₁₈H₁₉O₅S, calcd 347.4, found 347.1.

### Step (IX): Synthesis of 4-(4-methoxy-2-methylthieno[3,2-e]benzofuran-7-yl)-2-methyl-4-oxobutanoic acid (Example Compound 19)

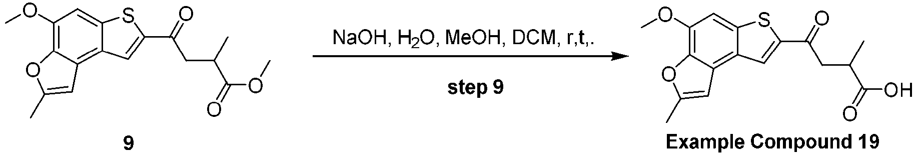

Methyl 4-(4-methoxy-2-methylthieno[3,2-e]benzofuran-7-yl)-2-methyl-4-oxobutanoate (6.5 g, 18.8 mmol) was dissolved in H₂O (20 mL), methanol (50 mL), and dichloromethane (50 mL), and then NaOH (7.5 g, 187.6 mmol) was added. The mixture was stirred at room temperature for 1 h. After completion of the reaction, 500 mL of water was added to the reaction solution. The mixture was adjusted to pH = 4-5 with hydrochloric acid and extracted three times with 500 mL of DCM (with solid retained in the organic phase). The organic phase was concentrated under reduced pressure to give a crude product. The crude product was triturated with 10 mL of tertiary ether and filtered. The filter cake was collected and dried by rotary evaporation to give Example 19 (5.6 g, 90%) as a yellow solid, ESI MS[M + H]⁺ for C₁₇H₁₇O₅S, calcd 333.37, found 333.1. ¹H NMR (400 MHz, DMSO-d6) δ 12.22 (s, 1H), 8.60 (s, 1H), 7.50 (s, 1H), 7.03 (d, J = 1.2 Hz, 1H), 4.00 (s, 3H), 3.45 (dd, J = 17.4, 8.5 Hz, 1H), 3.13 (dd, J = 17.3, 5.3 Hz, 1H), 2.92 (td, J = 8.0, 5.5 Hz, 1H), 2.55 - 2.52 (m, 3H), 1.20 (d, J = 7.2 Hz, 3H).

### Example 20

### Synthesis of 4-(4-methoxy-3H-thieno[3',2':3,4]benzo[1,2-d]imidazol-7-yl)-4-oxobutanoic acid (Example Compound 20)

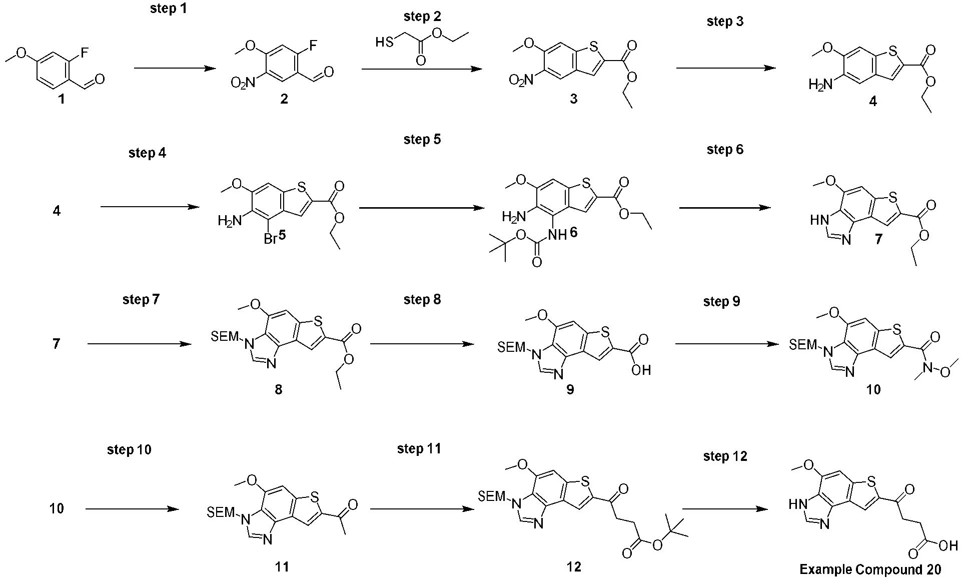

### Step (I): Synthesis of 2-fluoro-4-methoxy-5-nitrobenzaldehyde (Intermediate 1)

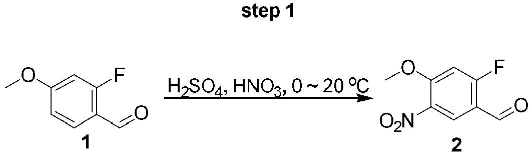

2-Fluoro-4-methoxybenzaldehyde (5.0 g, 32.4 mmol) was dissolved in H₂SO₄ (30 mL), and HNO₃ (4 mL, 63.5 mmol) was added dropwise at 0-20°C. The reaction mixture was then poured into ice water (100 mL) and extracted twice with EtOAc (200 mL). The organic phase was collected, dried over Na₂SO₄, and concentrated under vacuum to give crude Intermediate 2 as a yellow solid (6.0 g, 93%): ESI MS [M + H]⁺ for C₈H₇FNO₄, calcd 200.1, found 200.1.

### Step (II): Synthesis of ethyl 6-methoxy-5-nitrobenzo[b]thiophene-2-carboxylate (Intermediate 3)

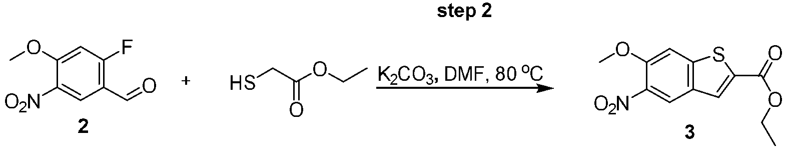

2-Fluoro-4-methoxy-5-nitrobenzaldehyde (5.0 g, 25.1 mmol) was dissolved in DMF (50 mL), and then ethyl 2-mercaptoacetate (4.5 g, 37.7 mmol) and K₂CO₃ (6.9 g, 50.2 mmol) were added. The mixture was purged with N₂ three times. The reaction mixture was then sealed well and stirred at 80°C under N₂ protection for 2 hours. The reaction mixture was cooled to room temperature and filtered. The filtrate was quenched into ice water (200 mL), extracted with EtOAc (300 mL), and washed three times with brine (300 mL). The organic phase was collected, dried over Na₂SO₄, and concentrated under vacuum to give a crude product. The crude product was triturated with petroleum ether:ethyl acetate = 10:1 to give Intermediate 3 as a yellow solid (6.0 g, 85%): ESI MS [M + H]⁺ for C₁₂H₁₂NO₅S, calcd 282.3, found 282.0.

### Step (III): Synthesis of ethyl 5-amino-6-methoxybenzo[b]thiophene-2-carboxylate (Intermediate 4)

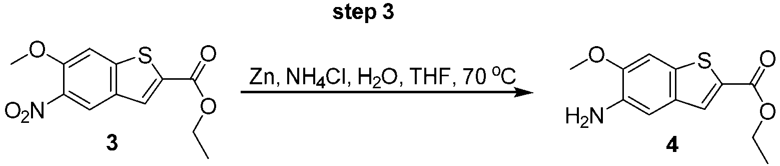

Ethyl 6-methoxy-5-nitrobenzo[b]thiophene-2-carboxylate (15.0 g, 53.3 mmol) was dissolved in THF (150 mL) and H₂O (75 mL), and then Zn (17.4 g, 266.6 mmol) and NH₄Cl (14.3 g, 266.7 mmol) were slowly added. The reaction mixture was purged with N₂ three times. The reaction mixture was then stirred at 70°C under N₂ protection for 2 hours. The reaction mixture was cooled to room temperature and filtered. The filtrate was quenched into water (100 mL) and extracted three times with EtOAc (200 mL). The organic phase was collected, dried over Na₂SO₄, and concentrated under vacuum to give a crude sample. The crude product was further purified by column chromatography (SiO₂, petroleum ether:ethyl acetate = 3:1) to give the pure title Intermediate 4 as a pale yellow solid (11.0 g, 82%): ESI MS [M + H]⁺ for C₁₂H₁₄NO₃S, calcd 252.3, found 252.1.

### Step (IV): Synthesis of ethyl 5-amino-4-bromo-6-methoxybenzo[b]thiophene-2-carboxylate (Intermediate 5)

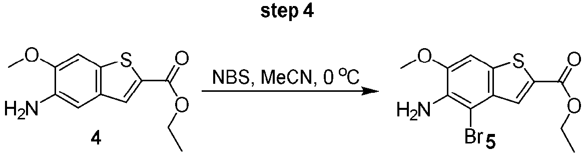

Ethyl 5-amino-6-methoxybenzo[b]thiophene-2-carboxylate (10.0 g, 39.8 mmol) was dissolved in MeCN (400 mL), and a solution of NBS (7.1 g, 39.8 mmol) in MeCN (100 mL) was slowly added at 0°C. The reaction mixture was then stirred at 25°C under N₂ protection for 10 minutes. The reaction mixture was quenched into ice water (300 mL) and extracted three times with EtOAc (400 mL). The organic phase was collected, dried over Na₂SO₄, and concentrated under vacuum to give crude Intermediate 5 as a brown solid (8.8 g, 67%): ESI MS [M + H]⁺ for C₁₂H₁₃BrNO₃S, calcd 331.2, found 331.9.

### Step (V): Synthesis of ethyl 5-amino-4-(tert-butoxycarbonyl)amino-6-methoxybenzo[b]thiophene-2-carboxylate

### (Intermediate 6)

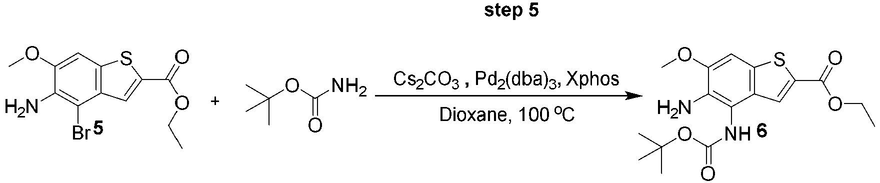

Ethyl 5-amino-4-bromo-6-methoxybenzo[b]thiophene-2-carboxylate (8.8 g, 26.7 mmol) was dissolved in 1,4-dioxane (120 mL), and then Cs₂CO₃ (17.4 g, 53.3 mmol), tert-butylcarbamate (4.7 g, 40.0 mmol), Xphos (2.5 g, 5.3 mmol), and PdCl₂(dba)₃ (2.4 g, 2.7 mmol) were added. The reaction mixture was stirred at 100°C under N₂ protection for 4 hours. The reaction mixture was then concentrated under vacuum to give a crude sample. The crude product was further purified by column chromatography (SiO₂, petroleum ether: ethyl acetate = 5:1) to give pure Intermediate 6 as a light brown solid (5.1 g, 52%): ¹H NMR (400 MHz, Chloroform-d) δ 7.89 (d, *J=* 0.9 Hz, 1H), 7.09 (s, 1H), 6.33 (s, 1H), 4.38 (q, *J =* 7.1 Hz, 2H), 4.25 (d, *J =* 4.8 Hz, 2H), 3.94 (s, 3H), 1.53 (s, 9H), 1.40 (td, *J=* 7.1, 1.5 Hz, 3H).

### Step (VI): Synthesis of ethyl 4-methoxy-3H-thieno[3',2':3,4]benzo[1,2-d]imidazole-7-carboxylate (Intermediate 7)

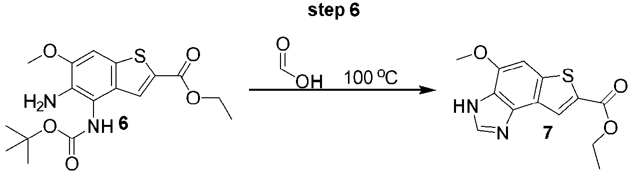

Ethyl 5-amino-4-((tert-butoxycarbonyl)amino)-6-methoxybenzo[b]thiophene-2-carboxylate (5.1 g, 13.9 mmol) was dissolved in formic acid (10 mL, 1.1 mol). The reaction mixture was then stirred at 100°C under N₂ protection for 3 hours. The reaction mixture was concentrated under vacuum and diluted with EtOAc (50 mL) and H₂O (50 mL). The reaction mixture was adjusted to pH = 6. The organic phase was washed three times with water (50 mL) and brine (50 mL), dried over Na₂SO₄, and filtered. The filtrate was concentrated under vacuum to give a crude sample. The crude product was further purified by column chromatography (SiO₂, DCM:MeOH = 50:1) to give Intermediate 7 as a white solid (2.2 g, 57%).

### Step (VII): Synthesis of ethyl 4-methoxy-3-((2-(trimethylsilyl)ethoxy)methyl-3H-thieno[3',2':3,4]benzo[1,2-d]imidazole-7-carboxylate (Intermediate 8)

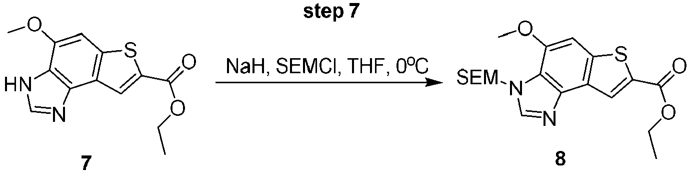

Ethyl 4-methoxy-3H-thieno[3',2':3,4]benzo[1,2-d]imidazole-7-carboxylate (950.0 mg, 3.4 mmol) was dissolved in THF (20 mL), and NaH (158.1 mg, 6.9 mmol) was slowly added at 0°C under N₂ protection. The mixture was stirred at 25°C for 30 minutes. SEMCl (1.1 g, 6.9 mmol) was then added to the mixture and stirred at 25°C for 1 hour. The reaction mixture was quenched into ice water (50 mL) and then extracted three times with EtOAc (50 mL). The organic phase was then washed twice with brine (50 mL), dried over Na₂SO₄, and filtered. The filtrate was concentrated under vacuum to give crude Intermediate 8 as a brown solid (1.1 g, 79%): ESI MS [M + H]⁺ for C₁₉H₂₇N₂O₄SSi, calcd 407.6, found 407.1.

### Step (VIII): Synthesis of 4-methoxy-3-(2-(trimethylsilyl)ethoxy)methyl-3H-thieno[3',2':3,4]benzo[1,2-d]imidazole-7-carboxylic acid (Intermediate 9)

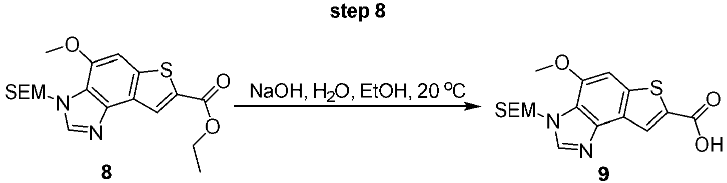

### Ethyl

4-methoxy-3-((2-(trimethylsilyl)ethoxy)methyl)-3H-thieno[3',2':3,4]benzo[1,2-d]imidazole-7-car boxylate (1.0 g, 2.5 mmol) was dissolved in H₂O (5 mL) and EtOH (20 mL), and then NaOH (491.9 mg, 2.5 mmol) was added. The reaction mixture was stirred at 25°C for 2 hours. The reaction mixture was diluted with EtOAc (50 mL) and H₂O (50 mL), adjusted to pH = 6, then extracted three times with EtOAc (50 mL), dried over Na₂SO₄, and filtered. The filtrate was concentrated under vacuum. The crude product was triturated with petroleum ether:ethyl acetate = 5:1. The mixture was filtered, and the filtrate was concentrated under vacuum to give Intermediate 9 as a white solid (850 mg, 91%): ESI MS [M + H]⁺ for C₁₇H₂₃N₂O₄SSi, calcd 379.5, found 379.1.

### Step (IX): Synthesis of N,4-dimethoxy-N-methyl-3-(2-(trimethylsilyl)ethoxy)methyl-3H-thieno[3',2':3,4]benzo[1,2-d]imidazole-7-carboxamide (Intermediate 10)

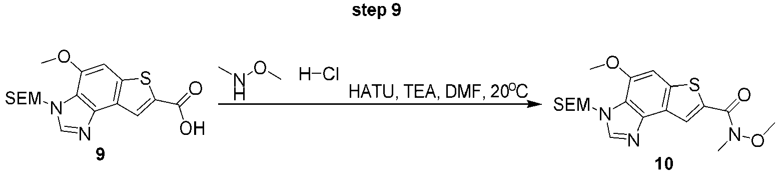

4-Methoxy-3-((2-(trimethylsilyl)ethoxy)methyl)-3H-thieno[3',2':3,4]benzo[1,2-d]imidazole-7-ca rboxylic acid (800.0 mg, 2.1 mmol) was dissolved in DMF (10.0 mL), and then HATU (1.6 g, 4.2 mmol), TEA (1.1 g, 10.6 mmol), and N,O-dimethylhydroxylamine hydrochloride (0.4 g, 4.2 mmol) were added. The reaction mixture was stirred at 20°C under N₂ protection for 2 hours. The reaction mixture was quenched into ice water (50 mL), then extracted three times with DCM (50 mL), washed three times with brine (100 mL), dried over Na₂SO₄, and filtered. The filtrate was concentrated under vacuum to give a crude sample. The crude product was further purified by column chromatography (SiO₂, DCM:MeOH = 50:1) to give Intermediate 10 as a yellow oil (650 mg, 73%): ESI MS [M + H]⁺ for C₁₉H₂₈N₃O₄SSi, calcd 422.6, found 422.1.

### Step (X): Synthesis of 1-(4-methoxy-3-(2-(trimethylsilyl)ethoxy)methyl-3H-thieno[3',2':3,4]benzo[1,2-d]imidazol-7 -yl)ethan-1-one (Intermediate 11)

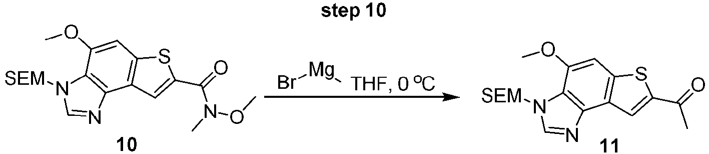

N,4-Dimethoxy-N-methyl-3-((2-(trimethylsilyl)ethoxy)methyl)-3H-thieno[3',2':3,4]benzo[1,2-d]i midazole-7-carboxamide (600.0 mg, 1.4 mmol) was dissolved in anhydrous THF (6 mL), and methylmagnesium bromide (509.1 mg, 4.3 mmol) was added at 0°C under N₂ protection. The reaction mixture was stirred at 25°C for 1 hour. The reaction mixture was quenched into ice water (50 mL) and then diluted with EtOAc (50 mL). The resulting mixture was extracted three times with EtOAc (50 mL), washed three times with brine (100 mL), dried over Na₂SO₄, and filtered. The filtrate was concentrated under vacuum to give a crude sample. The crude product was further purified by column chromatography (SiO₂, DCM:MeOH = 50:1) to give pure Intermediate 11 as a yellow oil (380.0 mg, 71%): ESI MS [M + H]⁺ for C₁₈H₂₅N₃O₃SSi, calcd 377.6, found 377.1.

### Step (XI): Synthesis of tert-butyl 4-(4-methoxy-3-((2-(trimethylsilyl)ethoxy)methyl)-3H-thieno[3',2':3,4]benzo[1,2-d]imidazol -7-yl)-4-oxobutanoate (Intermediate 12)

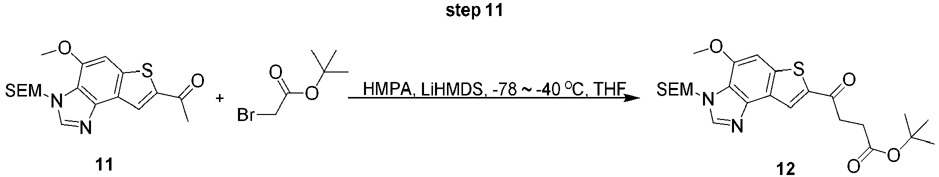

1-(4-Methoxy-3-((2-(trimethylsilyl)ethoxy)methyl)-3H-thieno[3',2':3,4]benzo[1,2-d]imidazol-7-yl)ethan-1-one (100 mg, 0.3 mmol) and HMPA (166.6 mg, 1.0 mmol) were dissolved in anhydrous THF (4 mL). The mixture was purged with N₂ three times. The reaction mixture was cooled to -78°C under N₂ protection using an ethanol/CO₂(s) bath. A 1M solution of HMDSLi in THF (0.4 mL, 0.4 mmol) was then added dropwise and stirred at -40°C for 1 hour. Afterwards, tert-butyl 2-bromoacetate (103.6 mg, 0.5 mmol) was added to the reaction mixture at -78°C and stirred at -78°C under N₂ protection for 30 minutes. The reaction mixture was quenched into ice water (50 mL) and then diluted with EtOAc (50 mL). The resulting mixture was extracted with EtOAc (50 mL). The organic phase was washed three times with brine (50 mL), dried over Na₂SO₄, and filtered. The filtrate was concentrated under vacuum to give crude Intermediate 12 as a yellow solid (100.0 mg, 75%): ESI MS [M + H]⁺ for C₂₄H₃₅N₂O₅SSi, calcd 491.7, found 491.1.

### Step (XII): Synthesis of 4-(4-methoxy-3H-thieno[3',2':3,4]benzo[1,2-d]imidazol-7-yl)-4-oxobutanoic acid (Example Compound 20)

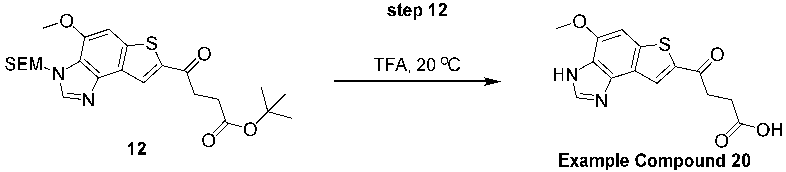

### Tert-butyl

4-(4-methoxy-3-((2-(trimethylsilyl)ethoxy)methyl)-3H-thieno[3',2':3,4]benzo[1,2-d]imidazol-7-y l)-4-oxobutanoate (100.0 mg, 0.2 mmol) was dissolved in TFA (3.0 mL). The reaction mixture was stirred at 20°C for 1 hour. The reaction mixture was concentrated under vacuum to give a crude sample. The crude product was further purified by reverse-phase HPLC (C₁₈ column, 30-60% of MeCN and water containing 0.1% TFA) to give the product as a white solid (9.0 mg, 15%): ESI MS [M + H]⁺ for C₁₄H₁₃N₂O₄S, calcd 305.3, found 305.0. ¹H NMR (400 MHz, DMSO-d₆) δ 8.50 (s, 1H), 8.24 (s, 1H), 7.40 (s, 1H), 4.02 (s, 3H), 2.60 (t, *J =* 6.4 Hz, 2H).

### Example 21

### Synthesis of ethyl 4-(4-methoxy-1-methyl-1H-thieno[3',2':3,4]benzo[1,2-d]imidazol-7-yl)-4-oxobutanoate

### (Example Compound 21)

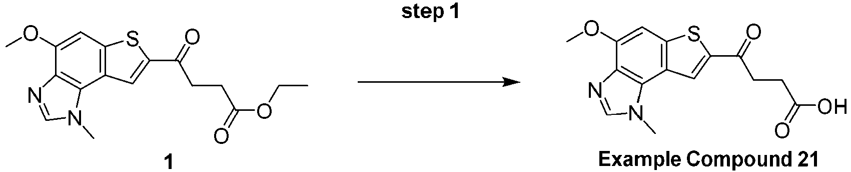

### Step (I): Synthesis of ethyl 4-(4-methoxy-1-methyl-1H-thieno[3',2':3,4]benzo[1,2-d]imidazol-7-yl)-4-oxobutanoate (Example Compound 21)

To a solution of ethyl 4-(4-Methoxy-1-methyl-1H-thieno[3',2':3,4]benzo[1,2-d]imidazol-7-yl)-4-oxobutanoate (30.0 mg, 0.1 mmol) in ethanol (2 mL) and H₂O (2 mL) was added NaOH (18.0 mg, 0.5 mmol). The reaction mixture was then stirred at room temperature for 2 hours. After the reaction was monitored to be complete by LCMS, the reaction mixture was adjusted to pH = 5 with HCl (1 mol/L) and then concentrated under vacuum to give a crude sample. The crude product was further purified by reverse-phase HPLC (C18 column, 30-60% gradient of MeCN, water, and water containing 0.1% TFA) to give Example Compound 21 as a white solid (5.0 mg, 17%), ESI MS [M + H]⁺ for C₁₅H₁₅N₂O₄S, calcd 319.4, found 319.1. ¹H NMR (400 MHz, DMSO-*d*₆) δ 12.24 (s, 1H), 8.62 (s, 1H), 8.13 (s, 1H), 7.38 (s, 1H), 4.19 (s, 3H), 4.00 (s, 3H), 3.38 (t, *J* = 6.4 Hz, 2H), 2.62 (t, *J =* 6.4 Hz, 2H).

### Example 22

### Synthesis of 4-(4-methoxy-3-methyl-3H-thieno[3,2]benzo[1,2-d]imidazol-7-yl)-4-oxobutanoic acid

### (Example compound 22)

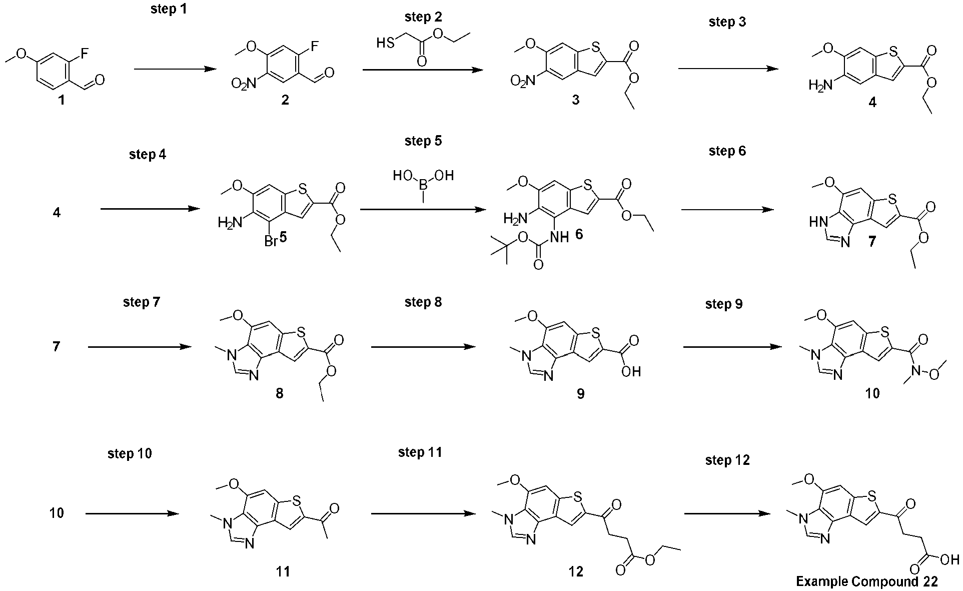

For the synthesis of ethyl 4-methoxy-3H-thieno[3',2':3,4]benzo[1,2-d]imidazole-7-carboxylate, refer to Example 20.

### Step (VII): Synthesis of ethyl 4-methoxy-3-methyl-3H-thieno[3',2':3,4]benzo[1,2-d]imidazole-7-carboxylate

### (Intermediate 8)

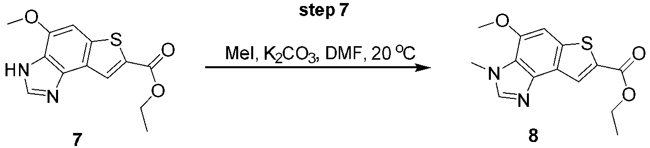

Ethyl 4-methoxy-3H-thieno[3',2':3,4]benzo[1,2-d]imidazole-7-carboxylate (1.2 g, 4.3 mmol) was dissolved in DMF (20 mL), and MeI (0.9 g, 6.5 mmol) and K₂CO₃ (1.8 g, 13.0 mmol) were added. The reaction mixture was then sealed and stirred at 20°C for 1 hour. The reaction mixture was quenched with water (100 mL), extracted with EtOAc (100 mL), and washed three times with brine (100 mL). The organic phase was collected, dried over Na₂SO₄, and concentrated under vacuum to give the crude product 8 as a white solid (1.2 g, 95%): ESI MS [M + H]⁺ for C₁₄H₁₅N₂O₃S, calcd 291.3, found 291.1.

### Step (VIII): Synthesis of 4-methoxy-3-methylthieno[3,2:3,4]benzo[1,2-d]imidazole-7-carboxylic acid (Intermediate 9)

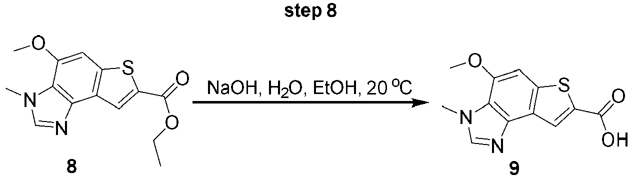

Ethyl 4-methoxy-3-methyl-3H-thieno[3',2':3,4]benzo[1,2-d]imidazole-7-carboxylate (1.2 g, 4.1 mmol) was dissolved in H₂O (20 mL) and EtOH (40 mL), and then NaOH (0.826 g, 20.7 mmol) was added. The reaction mixture was then stirred at 20°C for 3 hours. The reaction mixture was diluted with EtOAc (50 mL) and H₂O (50 mL), adjusted to pH = 6, then extracted three times with EtOAc (100 mL), dried over Na₂SO₄, and filtered. The filtrate was concentrated under vacuum to give crude Intermediate 9 as a white solid (900 mg, 83%): ESI MS [M + H]⁺ for C₁₂H₁₁N₂O₃S, calcd 263.3, found 263.0.

### Step (IX): Synthesis of N,4-dimethoxy-N,3-dimethylthieno[3,2:3,4]benzo[1,2-d]imidazole-7-carboxamide (Intermediate 10)

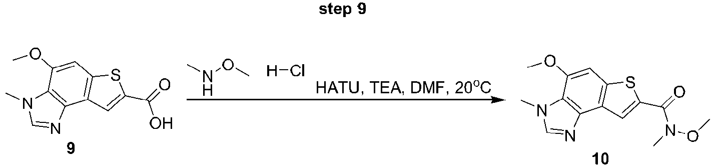

4-Methoxy-3-methyl-3H-thieno[3',2':3,4]benzo[1,2-d]imidazole-7-carboxylic acid (1.0 g, 3.8 mmol) was dissolved in DMF (50 mL), and HATU (2.9 g, 7.6 mmol), TEA (1.9 g, 19.1 mmol), and N,O-dimethylhydroxylamine hydrochloride (0.7 g, 7.6 mmol) were added under a N₂ atmosphere. The reaction mixture was stirred at 20°C for 1 hour. The reaction mixture was quenched into ice water (200 mL), then extracted three times with DCM (200 mL), washed three times with brine (100 mL), dried over Na₂SO₄, and filtered. The filtrate was concentrated under vacuum to give a crude sample. The crude product was further purified by column chromatography (SiO₂, 20% MeOH/DCM) to give Intermediate 10 as a yellow solid (1.0 g, 86%): ESI MS [M + H]⁺ for C₁₄H₁₆N₃O₃S, calcd 306.4, found 306.0.

### Step (X): Synthesis of 1-(4-methoxy-3-methyl-3H-thieno[3,2:3,4]benzo[1,2-d]imidazol-7-yl)ethan-1-one (Intermediate 11)

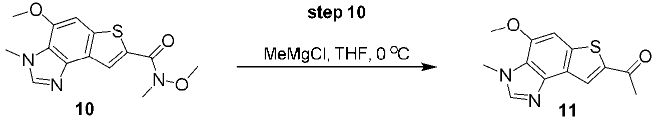

N,4-Dimethoxy-N,3-dimethyl-3H-thieno[3',2':3,4]benzo[1,2-d]imidazole-7-carboxamide (900.0 mg, 3.0 mmol) was dissolved in anhydrous THF (20 mL), and methylmagnesium chloride (661.3 mg, 8.8 mmol) was added at 0°C under a N₂ atmosphere. The reaction mixture was stirred at 25°C for 0.5 hour. The reaction mixture was quenched into ice water (100 mL) and then diluted with EtOAc (100 mL). The resulting mixture was extracted three times with EtOAc (100 mL), washed three times with brine (100 mL), dried over Na₂SO₄, and filtered. The filtrate was concentrated under vacuum to give a crude sample. The crude product was further purified by column chromatography (SiO₂, 20% MeOH/DCM) to give Intermediate 11 as a yellow solid (720.0 mg, 94%): ESI MS [M + H]⁺ for C₁₃H₁₃N₂O₂S, calcd 261.3, found 261.0.

### Step (XI): Synthesis of ethyl 4-(4-methoxy-3-methyl-3H-thieno[3',2':3,4]benzo[1,2-d]imidazol-7-yl)-4-oxobutanoate (Intermediate 12)

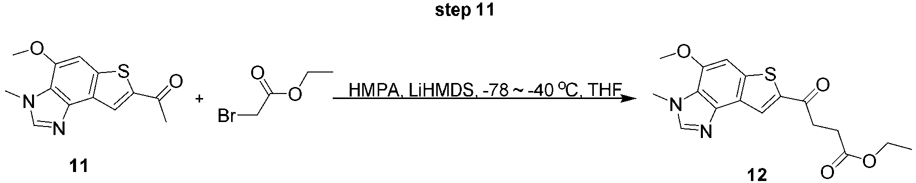

1-(4-Methoxy-3-methyl-3H-thieno[3',2':3,4]benzo[1,2-d]imidazol-7-yl)ethan-1-one (800.0 mg, 3.1 mmol) was dissolved in anhydrous THF (50 mL), and HMPA (1.9 g, 10.8 mmol) was added under a N₂ atmosphere. The reaction mixture was cooled to -78°C under N₂ protection using an ethanol/carbon dioxide(s) bath. A 1 mol/L solution of HMDSLi (4.6 mL, 4.6 mmol) in tetrahydrofuran was then added dropwise and stirred at -40°C for 1 hour. Afterwards, ethyl 2-bromoacetate (1.0 g, 6.2 mmol) was added to the reaction mixture at -78°C and stirred at -78°C under N₂ protection for 30 minutes. The reaction mixture was quenched into ice water (50 mL) and then diluted with EtOAc (50 mL). The resulting mixture was extracted with EtOAc (100 mL). The organic phase was washed three times with brine (100 mL), dried over Na₂SO₄, and filtered. The filtrate was concentrated under vacuum to give a crude sample, namely crude Compound 12 as a yellow solid (800.0 mg, 75%): ESI MS [M + H]⁺ for C₁₇H₁₉N₂O₄S, calcd 347.4, found 347.0.

### Step (XII): Synthesis of 4-(4-methoxy-3-methyl-3H-thieno[3,2]benzo[1,2-d]imidazol-7-yl)-4-oxobutanoic acid (Example Compound 22)

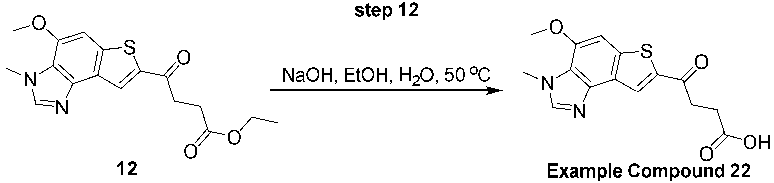

Ethyl 4-(4-methoxy-3-methyl-3H-thieno[3',2':3,4]benzo[1 ,2-d]imidazol-7-yl)-4-oxobutanoate (80.0 mg, 0.2 mmol) was dissolved in H₂O (10 mL) and EtOH (10 mL), and then NaOH (42.6 mg, 1.2 mmol) was added. The reaction mixture was stirred at 50°C for 2 hours, adjusted to pH = 5 with 1 mol/L hydrochloric acid solution, and concentrated under vacuum to give a crude sample. The crude product was further purified by reverse-phase HPLC (C₁₈ column, 30-60% of MeCN and water containing 0.1% TFA) to give the product as a white solid (8.0 mg, 11%): ESI MS [M + H]⁺ for C₁₅H₁₅N₂O₄S, calcd 319.4, found 319.0. ¹H NMR (400 MHz, DMSO- *d*₆) δ 12.05 (s, 1H), 8.49 (s, 1H), 8.20 (s, 1H), 7.44 (s, 1H), 4.07 (s, 3H), 4.02 (s, 3H), 3.36 (t, *J* = 6.3 Hz, 2H), 2.60 (t, *J=* 6.3 Hz, 2H).

### Example 23

### Synthesis of TM073 4-(4-methoxy-2-methyl-2H-thieno[3,2-e]indazol-7-yl)-4-oxobutanoic acid (Example Compound 23)

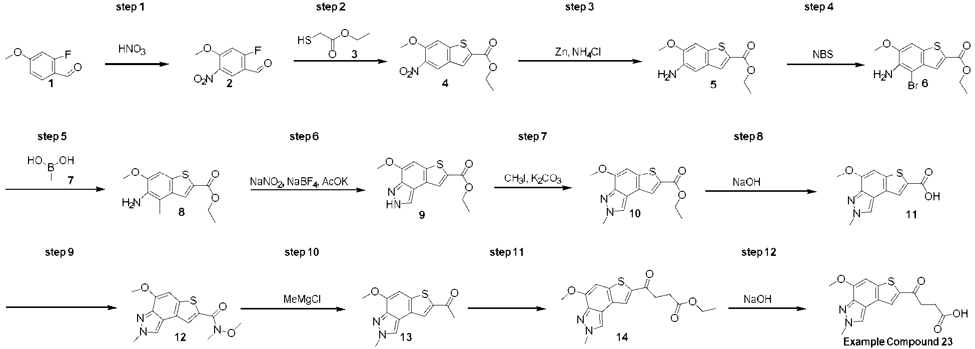

For the synthesis of 4-methoxy-2-methyl-2H-thieno[3,2-e]indazole-7-carboxylic acid (Intermediate 11), refer to Example 15.

### Step (IX): Synthesis of N,4-dimethoxy-N,3-dimethyl-3H-thieno[3,2-e]indazole-7-carboxamide (Intermediate 12)

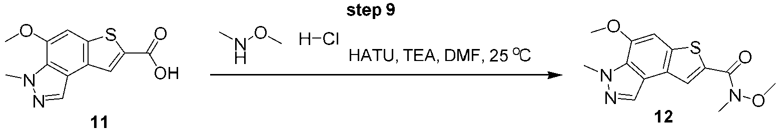

4-Methoxy-3-((2-(trimethylsilyl)ethoxy)methyl)-3H-thieno[3',2':3,4]benzo[1,2-d]imidazole-7-ca rboxylic acid (400.0 mg, 1.5 mmol) was dissolved in DMF (10.0 mL), and HATU (1.2 g, 3.1 mmol), TEA (0.8 g, 7.6 mmol), and N,O-dimethylhydroxylamine hydrochloride (0.3 g, 3.1 mol) were added. The reaction mixture was stirred at 25°C under nitrogen protection for 1 hour.

The reaction mixture was poured into water (50 mL) and then extracted three times with EtOAc (50 mL). The organic phase was dried over Na₂SO₄ and filtered. The filtrate was concentrated under reduced pressure to give a crude product. The crude product was further purified by column chromatography (SiO₂, petroleum ether:EtOAc = 3:1) to give Compound 12 as a yellow solid (300 mg, 64%), ESI MS[M + H]⁺ for C₁₄H₁₆N₃O₃S, calcd 306.4, found 306.1.

### Step (X): Synthesis of 1-(4-methoxy-2-methyl-2H-thieno[3,2-e]indazol-7-yl)ethan-1-one (Intermediate 13)

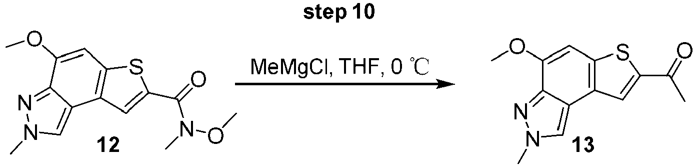

N,4-Dimethoxy-N,3-dimethyl-3H-thieno[3,2-e]indazole-7-carboxamide (300.0 mg, 1.0 mmol) was dissolved in THF (10 mL) and cooled to 0°C under nitrogen protection. Methylmagnesium chloride (367.4 mg, 4.9 mmol) was added to the reaction mixture and stirred at room temperature for 1 hour. The reaction solution was quenched by the addition of water (50 mL) and extracted twice with EtOAc (50 mL). The organic phase was dried over Na₂SO₄ and filtered. The filtrate was concentrated under reduced pressure to give a crude product. The crude product was further purified by column chromatography (SiO₂, petroleum ether:EtOAc = 3:1) to give Intermediate 13 as a yellow solid (250.0 mg, 98%). ESI MS[M + H]⁺ for C₁₃H₁₃N₂O₂S, calcd 261.3, found 261.0.

### Step (XI): Synthesis of ethyl 4-(4-methoxy-2-methyl-2H-thieno[3,2-e]indazol-7-yl)-4-oxobutanoate (Intermediate 14)

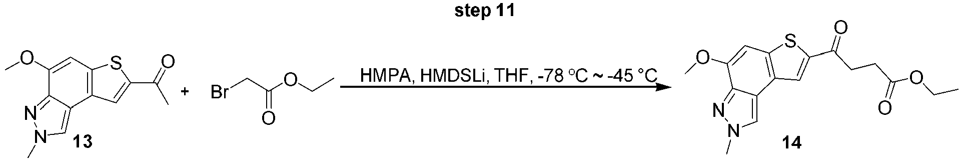

1-(4-Methoxy-2-methyl-2H-thieno[3,2-e]indazol-7-yl)ethan-1-one (70.0 mg, 0.3 mmol) and HMPA (168.7 mg, 1.0 mmol) were dissolved in anhydrous THF (5 mL). The reaction mixture was cooled to -78°C under N2 protection using a dry ice-ethanol bath. HMDSLi (0.4 mL, 0.4 mmol, 1M in THF) was then added dropwise and stirred at -45°C for 1 hour. Afterwards, the reaction mixture was cooled to -78°C. Ethyl 2-bromoacetate (89.8 mg, 0.5 mmol) was added dropwise and stirred at -78°C for 1 hour. The reaction mixture was quenched by the addition of water (50 mL) and then extracted twice with EtOAc (50 mL). The organic phase was dried over Na₂SO₄ and filtered. The filtrate was concentrated under reduced pressure to give crude Intermediate 14 as a yellow solid (90 mg, 96%), ESI MS[M + H]⁺ for C₁₇H₁₉N₂O₄S, calcd 347.4, found 347.0, which was used directly in the next step without purification.

### Step (XII): Synthesis of 4-(4-methoxy-2-methyl-2H-thieno[3,2-e]indazol-7-yl)-4-oxobutanoic acid (Example Compound 23)

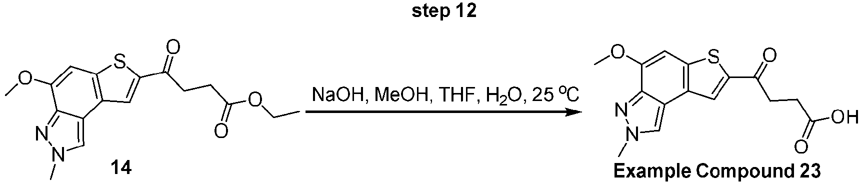

Ethyl 4-(4-methoxy-2-methyl-2H-thieno[3,2-e]indazol-7-yl)-4-oxobutanoate (90.0 mg, 0.3 mmol) was dissolved in a solution of MeOH (3 mL), THF (3 mL), and H₂O (1 mL), and NaOH (52.0 mg, 1.3 mmol) was added. The reaction mixture was stirred at room temperature for 2 hours. EtOAc (20 mL) and H₂O (20 mL) were added to the reaction solution. The mixture was adjusted to pH = 5, then extracted three times with EtOAc (20 mL), dried over sodium sulfate, and filtered. The filtrate was concentrated under reduced pressure to give a crude product. The crude product was further purified by reverse-phase HPLC (C18 column, 30-50% gradient of MeCN and water containing 0.1% FA) to give the product as a white solid (18.0 mg, 22%). ESI MS [M + H] ⁺ for C₁₅H₁₅N₂O₄S, calcd 319.4, found 319.0. ¹H NMR (400 MHz, DMSO-d6) δ 12.19 (s, 1H), 8.57 (s, 1H), 8.55 (s, 1H), 7.26 (s, 1H), 4.19 (s, 3H), 3.99 (s, 3H), 3.29 (t, J = 6.5 Hz, 2H), 2.62 (t, J = 6.4 Hz, 2H).

### Example 24

### Synthesis of 4-(4-methoxy-2,8-dimethylthieno[3,2-e]benzofuran-7-yl)-4-oxobutanoic acid (Example Compound 24)

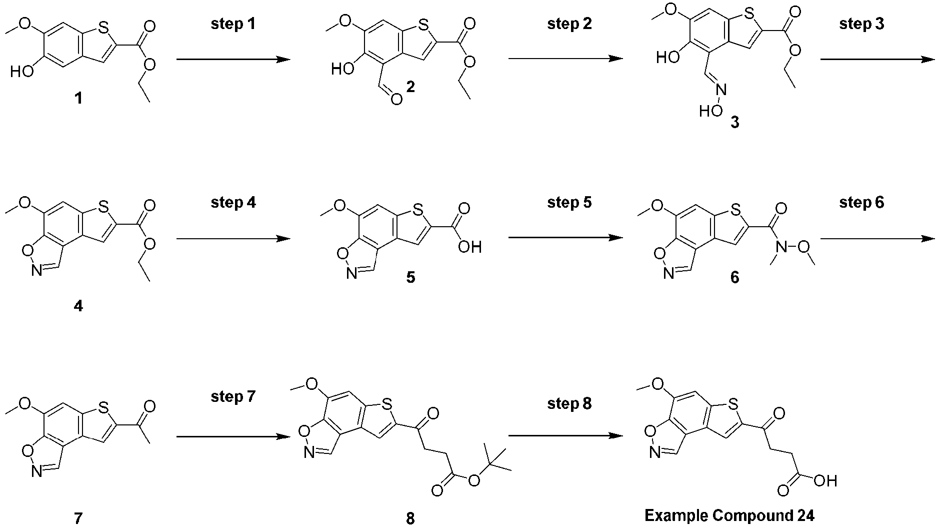

### Step (I): Synthesis of ethyl 4-formyl-5-hydroxy-6-methoxy-1-benzothiophene-2-carboxylate (Intermediate 2)

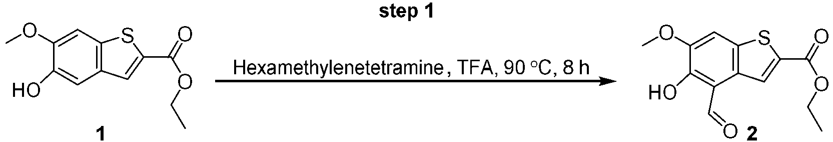

Ethyl 5-hydroxy-6-methoxy-1-benzothiophene-2-carboxylate (4.2 g, 16.7 mmol) was dissolved in TFA (42 mL, 368.3 mmol), and urotropine (11.7 g, 83.2 mmol) was added. The mixture was purged with N₂ three times. The reaction mixture was then sealed and stirred at 90°C under N₂ protection for 8 hours. The reaction mixture was quenched into ice-water (600 g) to maintain the internal temperature below 20°C. The resulting mixture was extracted with EtOAc (600 mL). The organic phase was then washed with water (400 mL) and brine (200 mL), dried over Na₂SO₄, and filtered. The filtrate was concentrated under vacuum to give a crude sample. The crude product was further purified by column chromatography (SiO₂, 20% EtOAc/petroleum ether) to give Intermediate 2 as a white solid (4 g, 85.7%), LCMS: ESI MS[M + H]⁺ for C₁₃H₁₂O₅S, calcd 281.3, found 280.92.

### Step (II): Synthesis of ethyl (E)-5-hydroxy-4-(hydroxyimino)methyl)-6-methoxy-1-benzothiophene-2-carboxylate (Intermediate 3)

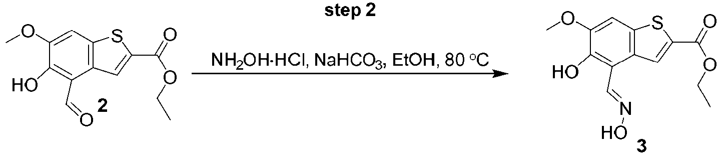

Ethyl 4-formyl-5-hydroxy-6-methoxy-1-benzothiophene-2-carboxylate (2.7 g, 9.6 mmol) was dissolved in ethanol (30 mL), and sodium bicarbonate (1.1 g, 12.9 mmol) was added, followed by hydroxylamine hydrochloride (3.3 g, 48.2 mmol). The mixture was purged with N₂ three times. The reaction mixture was then sealed and stirred at 80°C under N₂ protection for 1 h. The reaction mixture was quenched into ice-water (600 g) to maintain the internal temperature below 20°C. The resulting mixture was extracted with EtOAc (600 mL). The organic phase was then washed with water (400 mL) and brine (200 mL), dried over Na₂SO₄, and filtered. The filtrate was concentrated under vacuum to give a crude sample. The crude product was further purified by column chromatography (SiO₂, 20% EtOAc/petroleum ether) to give Intermediate 3 as a yellow solid (2.1 g, 84.0%), ESI MS [M + H]⁺ for C₁₃H₁₃NO₅S, calcd 296.3, found 296.0.

### Step (III): Synthesis of ethyl 4-methoxythieno[2',3':5,6]benzo[1,2-d]isoxazole-7-carboxylate

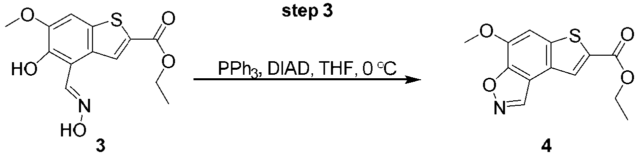

Ethyl (E)-5-hydroxy-4-(hydroxyimino)methyl)-6-methoxy-1-benzothiophene-2-carboxylate (2.3 g, 7.62 mmol) was dissolved in THF (50 mL), and then triphenylphosphine (6 g, 22.9 mmol) was added. Diisopropyl azodicarboxylate (3.1g, 15.2 mmol) was added under nitrogen protection in an ice-water bath. The mixture was purged with N₂ three times. The reaction mixture was then sealed and stirred at 25°C under N₂ protection for 12 hours. The reaction was quenched with ice water. The reaction mixture was extracted with EA and concentrated to give a crude product. The crude product was further purified by column chromatography (SiO₂, 20% EtOAc/petroleum ether) to give Intermediate 3 as a yellow solid (1.35 g, 63.9%), ESI MS [M + H]⁺ for C₁₃H₁₁NO₄S, calcd 278.3, found 278.0.

### Step (IV): Synthesis of 4-methoxythieno[2',3':5,6]benzo[1,2-d]isoxazole-7-carboxylic acid

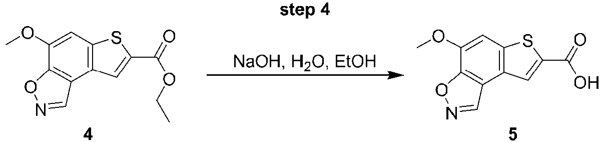

Ethyl 4-methoxythieno[2',3':5,6]benzo[1,2-d]isoxazole-7-carboxylate (1.3 g, 4.7 mmol) was dissolved in water (20 mL), ethanol (20 mL), and NaOH (187.5 mg, 4.7 mmol). The reaction mixture was stirred at room temperature for 2 hours. The reaction was quenched with ice water. The reaction mixture was extracted with EA and concentrated to give a crude product. The crude product was further purified by column chromatography (SiO₂, 20% EtOAc/petroleum ether) to give Intermediate 3 as a yellow solid (1.1 g, 94%), ESI MS [M - H]⁻ for C₁₁H₇NO₄S, calcd 248.2, found 247.9.

### Step (V): Synthesis of N,4-dimethoxy-N-methylthieno[2',3':5,6]benzo[1,2-d]isoxazole-7-carboxamide

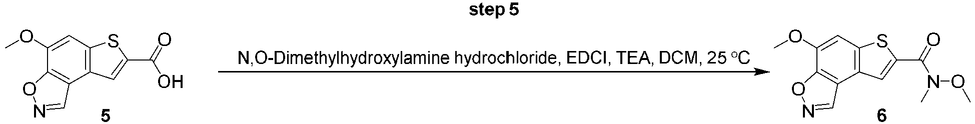

Ethyl (E)-5-hydroxy-4-(hydroxyimino)methyl)-6-methoxy-1-benzothiophene-2-carboxylate (2.3 g, 7.62 mmol) was dissolved in DCM (15 mL), and N,O-dimethylhydroxylamine hydrochloride (1.3 g, 12.9 mmol), triethylamine (2.2 g, 21.5 mmol), and EDCI (1.6 g, 8.6 mmol) were added. The reaction mixture was then reacted at 25°C under N₂ protection for 1 hour. The reaction was quenched with ice water. The reaction mixture was extracted with EA and concentrated to give a crude product. The crude product was further purified by column chromatography (SiO₂, 20% EtOAc/petroleum ether) to give Intermediate 6 as a yellow solid (330 mg, 26.0%), ESI MS [M + H]⁺ for C₁₃H₁₂N₂O₄S, calcd 293.3, found 293.1.

### Step (VI): Synthesis of 1-(4-methoxythieno[2',3':5,6]benzo[1,2-d]isoxazol-7-yl)ethan-1-one

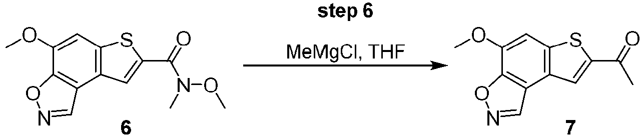

N,4-Dimethoxy-N-methylthieno[2',3':5,6]benzo[1,2-d]isoxazole-7-carboxamide (300.0 mg, 1 mmol) was dissolved in THF (3 mL), and the mixture was purged with a N₂ mixture three times. The reaction mixture was cooled to 0°C under N₂ protection. Then, 1.0M methylmagnesium chloride in THF (3.1 mL, 3.1 mmol) was added dropwise at 0°C. The reaction mixture was stirred at room temperature for 1 hour. The reaction mixture was quenched into ice-water (20 mL) and then diluted with EtOAc (20 mL). The resulting mixture was extracted with EtOAc (20 mL). The organic phase was washed three times with water (20 mL), dried over Na₂SO₄, and filtered. The filtrate was concentrated under vacuum to give a crude sample. The crude product was further purified by column chromatography (SiO₂, petroleum ether:EtOAc = 5:1) to give Intermediate 7 as a yellow solid (180 mg, 71%), ESI MS [M - H]⁻ for C₁₂H₉NO₃S, calcd 246.3, found 246.0.

### Step (VII): Synthesis of tert-butyl 4-(4-methoxythieno[2',3':5,6]benzo[1,2-d]isoxazol-7-yl)-4-oxobutanoate

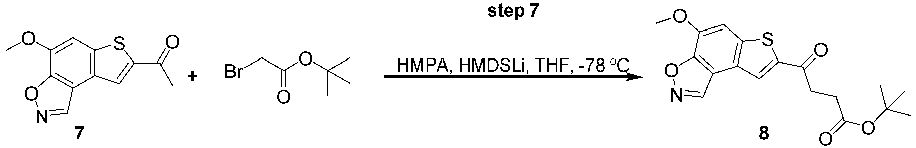

1-(4-Methoxythieno[2',3':5,6]benzo[1,2-d]isoxazol-7-yl)ethan-1-one (100.0 mg, 0.4 mmol) and HMPA (253.7 g, 1.4 mmol) were dissolved in anhydrous THF (1 mL), and the mixture was purged with N₂ three times. The reaction mixture was cooled to -78°C under N₂ protection. Then, 1M HMDSLi in THF (0.6 mL, 0.6 mmol) was added dropwise and stirred at -78°C for 30 minutes. Afterwards, tert-butyl 2-bromoacetate (157.8 mg, 0.8 mmol) was added to the reaction mixture and stirred at -78°C under a N₂ atmosphere for 2 hours. LCMS at this point indicated no more starting material. The reaction mixture was adjusted to pH = 5 and diluted with water (20 mL), followed by EtOAc (20 mL). The organic phase was then washed with water (20 mL) and brine (20 mL), then dried over Na₂SO₄, concentrated under vacuum, and filtered to give crude Compound 8 as a yellow solid (100.0 mg, 68%), ESI MS [M + H]⁺ for C₁₈H₁₉NO₅S, calcd 362.4, found 306.0.

### Step (VIII): Synthesis of 4-(4-methoxythieno[2',3':5,6]benzo[1,2-d]isoxazol-7-yl)-4-oxobutanoic acid

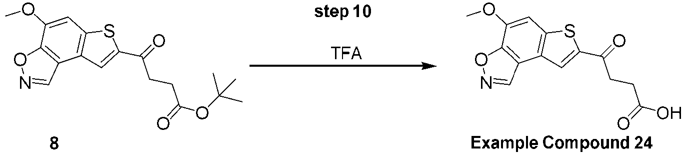

A solution of 1-(4-methoxythieno[2',3':5,6]benzo[1,2-d]isoxazol-7-yl)ethan-1-one (100.0 mg, 0.28 mmol) and TFA (0.5 mL) was prepared. The reaction mixture was stirred at 20°C for 0.5 hour. The reaction mixture was concentrated under vacuum to give a crude sample. The crude product was further purified by reverse-phase HPLC (C18 column, 30-50% gradient of MeCN and water containing 0.1% FA) to give the product as a white solid (16.8 mg, 19.65%), ¹H NMR (400 MHz, DMSO-*d*₆) δ 12.18 (s, 1H), 11.03 (s, 1H), 8.12 (s, 1H), 7.95 (s, 1H), 3.96 (s, 3H), 3.36 (t, *J =* 6.4 Hz, 2H), 2.59 (t, *J =* 6.4 Hz, 2H)).

### Example 25

### Synthesis of 4-(4-methoxy-2-methyl-2H-thieno[3,2-e]indazol-7-yl)-4-oxobutanoic acid (Example Compound 25)

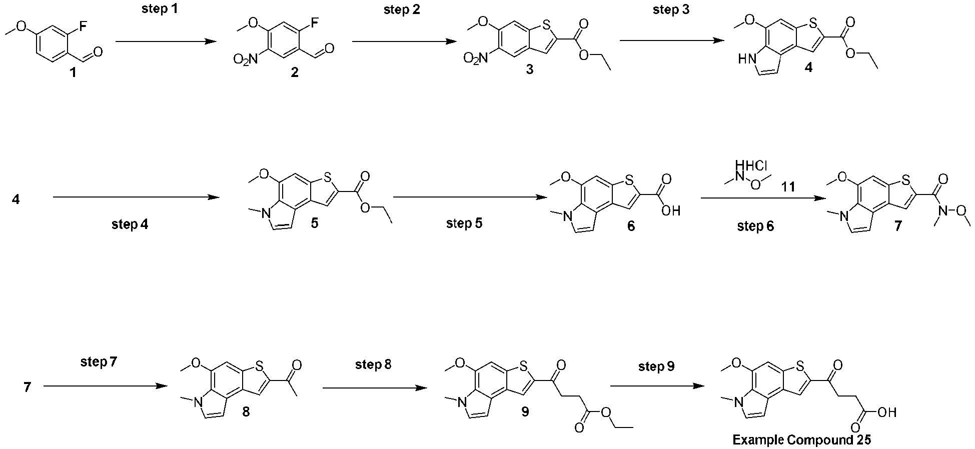

### Step (I): Synthesis of 2-fluoro-4-methoxy-5-nitrobenzaldehyde (Intermediate 2)

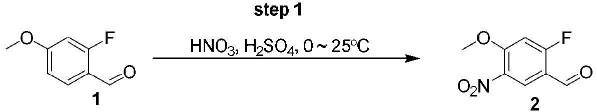

2-Fluoro-4-methoxybenzaldehyde (5 g, 32.4 mmol) was dissolved in sulfuric acid (30 mL) and cooled to 0°C. Nitric acid (4 mL, 63.5 mmol, 64%) was then slowly added. The addition was highly exothermic, so the internal temperature was controlled below 20°C. TLC monitoring was performed immediately after the addition. The reaction solution was dropped into ice water and filtered. The filter cake was then taken up in EA (500 mL) and water (500 mL). The mixture was extracted, and the layers were separated. The organic phase was dried over Na₂SO₄ and filtered. The filtrate was concentrated under reduced pressure to give crude Intermediate 2 as a yellow solid (6 g, 93%). ESI MS[M + H]⁺ for C₈H₇FNO₄, calcd 200.14, found 200.0.

### Step (II): Synthesis of ethyl 6-methoxy-5-nitrobenzo[b]thiophene-2-carboxylate (Intermediate 3)

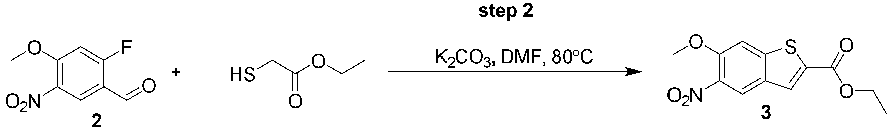

2-Fluoro-4-methoxy-5-nitrobenzaldehyde (6 g, 30.1 mmol) was dissolved in DMF (60 mL) and cooled to 0°C. Ethyl 2-mercaptoacetate (5.4 g, 45.2 mmol) and potassium carbonate (8.3 g, 60.3 mmol) were then added and stirred at 80°C under nitrogen protection for 4 h. The reaction solution was cooled to room temperature and filtered. The filter cake was rinsed with EA (600 mL), and ice water (600 mL) was added to the filtrate. The mixture was extracted, and the layers were separated. The organic phase was dried over Na₂SO₄ and filtered. The filtrate was concentrated under reduced pressure to give a crude product. The crude product was purified by trituration with 10v of mixed solvent (PE:EA = 10:1) and filtered. The filter cake was collected to give Intermediate 3 as a yellow solid (7 g, 83%). ESI MS[M + H]⁺ for C₁₂H₁₂NO₅S, calcd 282.28, found 282.0.

### Step (III): Synthesis of ethyl 5-methoxy-6-H-thieno[3,2-ethyl]indole-2-carboxylate (Intermediate 4)

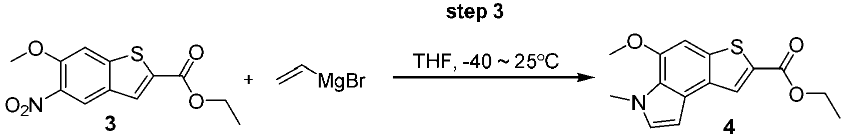

Ethyl 6-methoxy-5-nitrobenzo[b]thiophene-2-carboxylate (3 g, 10.7 mmol) was dissolved in anhydrous THF (50 mL) and cooled to -40°C under nitrogen protection. Vinylmagnesium bromide (53 mL, 53.3 mmol, 1M in THF) was then added and stirred for 1 h. The mixture was warmed to room temperature and stirred overnight. The reaction solution was quenched by the addition of water (500 mL), and EA (500 mL) was then added. The mixture was extracted, and the layers were separated. The organic phase was dried over Na₂SO₄ and filtered. The filtrate was concentrated under reduced pressure to give a crude product. The crude product was further purified by column chromatography (SiO₂, EtOAc/petroleum ether = 1/3) to give Intermediate 4 as a yellow liquid (500 mg, 17%). ESI MS[M + H]⁺ for C₁₄H₁₄NO₃S, calcd 276.32, found 276.1.

### Step (IV): Synthesis of ethyl 5-methoxy-6-methyl-6H-thieno[3,2-e]indole-2-carboxylate (Intermediate 5)

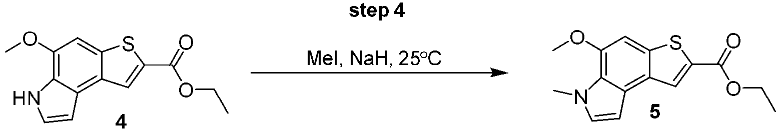

Ethyl 5-methoxy-6-H-thieno[3,2-ethyl]indole-2-carboxylate (400 mg, 1.5 mmol) was dissolved in THF (10 mL), and then sodium hydride (66.8 mg, 2.9 mmol) and iodomethane (247.5 mg, 1.7 mmol) were added under nitrogen protection. The mixture was stirred at room temperature for 1 h. Ice water (50 mL) and EA (50 mL) were added to the reaction solution. The mixture was extracted, and the layers were separated. The organic phase was dried over Na₂SO₄ and filtered. The filtrate was concentrated under reduced pressure to give crude Intermediate 5 as a yellow solid (400 mg, 95%). The crude product was used directly in the next step without purification. ESI MS[M + H]⁺ for C₁₅H₁₆NO₃S, calcd 290.35, found 290.1.

### Step (V): Synthesis of 5-methoxy-6-methyl-6H-thieno[3,2-e]indole-2-carboxylic acid (Intermediate 6)

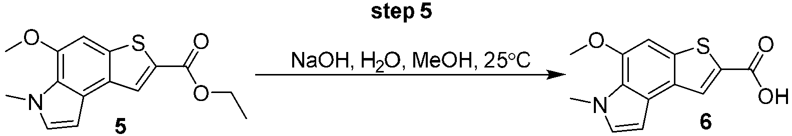

Ethyl 5-methoxy-6-methyl-6H-thieno[3,2-e]indole-2-carboxylate (400 mg, 1.4 mmol) was dissolved in H₂O (2 mL) and methanol (10 mL), and then NaOH (276.5 mg, 6.9 mmol) was added. The mixture was stirred at room temperature overnight. The reaction solution was adjusted to pH = 5, and then EA (100 mL) and water (100 mL) were added. The mixture was extracted, and the layers were separated. The organic phase was dried over Na₂SO₄ and filtered. The filtrate was concentrated under reduced pressure to give a crude product. The crude product was further purified by column chromatography (SiO₂, DCM:MeOH = 20:1) to give Intermediate 6 as a yellow liquid (300 mg, 83%). ESI MS[M + H]⁺ for C₁₃H₁₂NO₃S, calcd 262.3, found 262.0.

### Step (VI): Synthesis of N,5-dimethoxy-N,6-dimethyl-6H-thieno[3,2-e]indole-2-carboxamide (Intermediate 7)

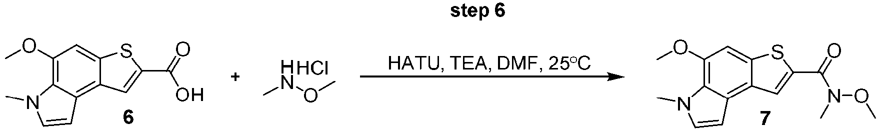

5-Methoxy-6-methyl-6H-thieno[3,2-e]indole-2-carboxylic acid (300 mg, 1.2 mmol) was dissolved in DMF (10 mL), and then HATU (387.1 mg, 2.3 mmol), triethylamine (580.9 mg, 5.7 mmol), and dimethylhydroxylamine hydrochloride (224.0 mg, 2.3mmol) were added. The mixture was stirred at room temperature for 1 h. Water (100 mL) and EA (100 mL) were added to the reaction solution. The mixture was extracted, and the layers were separated. The organic phase was dried over Na₂SO₄ and filtered. The filtrate was concentrated under reduced pressure to give a crude product. The crude product was further purified by column chromatography (SiO₂, PE:EA = 3:1) to give Intermediate 7 as a yellow liquid (300 mg, 86%). ESI MS[M + H]⁺ for C₁₅H₁₇N₂O₃S, calcd 305.36, found 305.1.

### Step (VII): Synthesis of 1-(5-methoxy-6-methyl-6H-thieno[3,2-e]indol-2-yl)ethan-1-one (Intermediate 8)

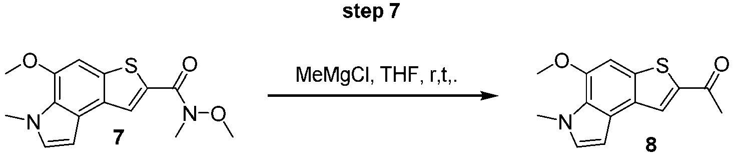

N,5-Dimethoxy-N,6-dimethyl-6H-thieno[3,2-e]indole-2-carboxamide (300 mg, 1.0 mmol) was dissolved in THF (10 mL), and then methylmagnesium chloride (222.1 mg, 3.0 mmol) was added under nitrogen protection. The mixture was stirred at room temperature for 1 h. Water (100 mL) and EA (100 mL) were added to the reaction solution. The mixture was extracted, and the layers were separated. The organic phase was dried over Na₂SO₄ and filtered. The filtrate was concentrated under reduced pressure to give a crude product. The crude product was further purified by column chromatography (SiO₂, PE:EA = 3:1) to give Intermediate 8 as a yellow liquid (150 mg, 58%). ESI MS[M + H]⁺ for C₁₄H₁₄NO₂S, calcd 260.32, found 260.0.

### Step (VIII): Synthesis of ethyl 4-(5-methoxy-6-methyl-6H-thieno[3,2-e]indol-2-yl)-4-oxobutanoate (Intermediate 9)

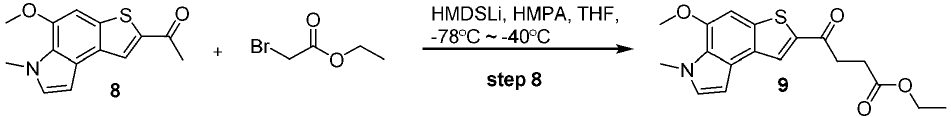

1-(5-Methoxy-6-methyl-6H-thieno[3,2-e]indol-2-yl)ethan-1-one (80 mg, 0.3 mmol) was dissolved in THF (5 mL), and then HMPA (193.5 mg, 1.1 mmol) was added. The mixture was cooled to -78°C under nitrogen protection, and then HMDSLi (154.9 mg, 0.9 mmol) was added. The mixture was warmed to -40°C and stirred for 2 h. Ethyl 2-bromoacetate (154.6 mg, 0.9 mmol) was then added and stirred for 1 h. Water (50 mL) and EA (50 mL) were added to the reaction solution. The mixture was extracted, and the layers were separated. The organic phase was dried over Na₂SO₄ and filtered. The filtrate was concentrated under reduced pressure to give crude Intermediate 9 as a yellow solid (80 mg, 75%). ESI MS[M + H]⁺ for C₁₈H₁₉NO₄S, calcd 346.41, found 346.1.

### Step (IX): Synthesis of 4-(5-methoxy-6-methyl-6H-thieno[3,2-e]indol-2-yl)-4-oxobutanoic acid (Example Compound 25)

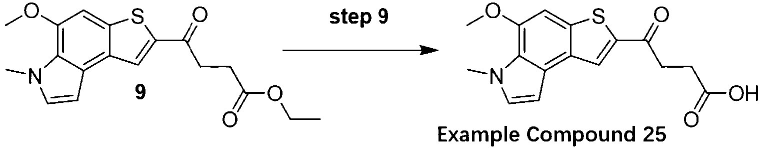

Ethyl 4-(5-methoxy-6-methyl-6H-thieno[3,2-e]indol-2-yl)-4-oxobutanoate (80.0 mg, 0.2 mmol) was dissolved in a solution of MeOH (2 mL), THF (2 mL), and H₂O (1 mL), and NaOH (46.0 mg, 1.2 mmol) was added. The reaction mixture was stirred at room temperature for 2 hours. EtOAc (20 mL) and H₂O (20 mL) were added to the reaction solution. The mixture was adjusted to pH = 5, then extracted three times with EtOAc (20 mL), dried over sodium sulfate, and filtered. The filtrate was concentrated under reduced pressure to give a crude product. The crude product was further purified by reverse-phase HPLC (C18 column, 30-50% gradient of MeCN and water containing 0.1% FA) to give the product as a white solid (2.0 mg, 3%). ESI MS[M + H]⁺ for C₁₆H₁₆NO₄S, calcd 318.36, found 317.95, ¹H NMR (400 MHz, DMSO-d6) δ 8.56 (s, 1H), 7.33 (d, J = 3.0 Hz, 1H), 7.25 (s, 1H), 6.86 (d, J = 2.9 Hz, 1H), 4.06 (s, 3H), 3.99 (s, 3H), 3.27 (t, J = 6.7 Hz, 2H), 2.64 (t, J = 6.7 Hz, 2H).

### Example 26

### Synthesis of 4-(5-methoxy-7,8-dihydro-6H-indeno[5,4-b]thiophen-2-yl)-4-oxobutanoic acid (Example compound 26)

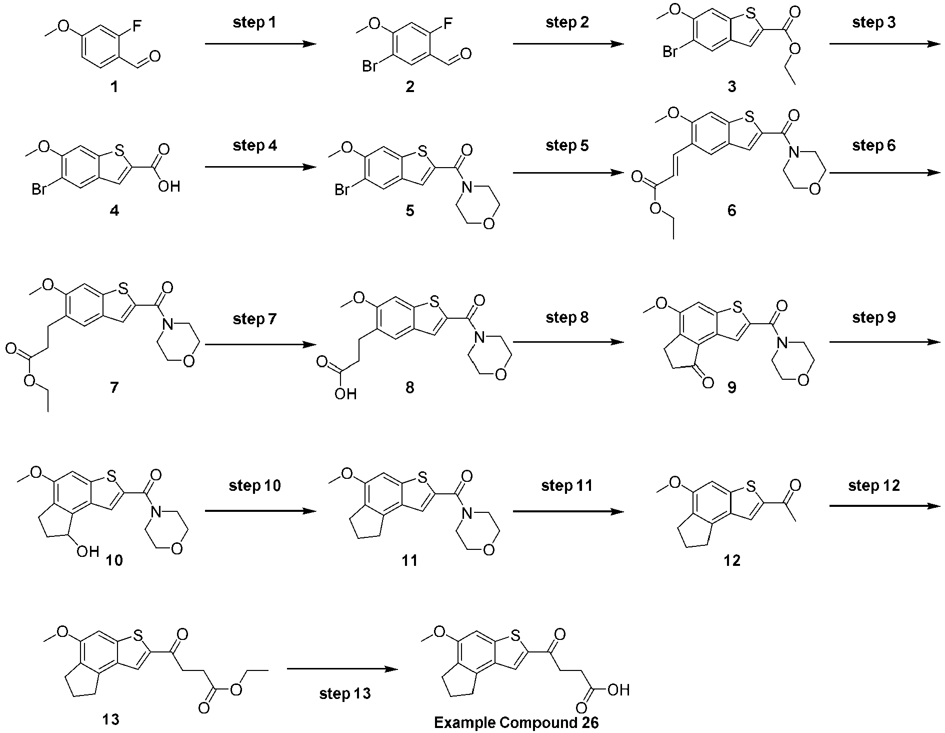

### Step (I): Synthesis of 5-bromo-2-fluoro-4-methoxybenzaldehyde (Intermediate 2)

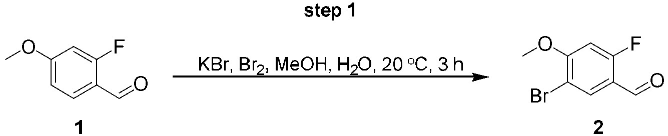

Potassium bromide (19.3 g, 162.2 mmol) was dissolved in H₂O (100 mL) and MeOH (25 mL), and then bromine (10.4 g, 64.9 mmol) was added. The reaction mixture was then cooled to 0°C. 2-Fluoro-4-methoxybenzaldehyde (5.0 g, 34.4 mmol) was dissolved in 20 mL of methanol solution and added dropwise to the above reaction mixture at 0°C. The reaction mixture was stirred at room temperature for 3 hours. A solid precipitated from the reaction solution, and the reaction solution was then filtered. The filter cake was washed with water (500 mL) and concentrated under vacuum to give Compound 2 as a white solid (7.6 g, 93%): ¹H NMR (400 MHz, Chloroform- d) δ 10.16 (s, 1H), 8.06 (d, *J =* 7.4 Hz, 1H), 6.68 (d, *J =* 11.9 Hz, 1H), 3.97 (s, 3H).

### Step (II): Synthesis of ethyl 5-bromo-6-methoxybenzo[b]thiophene-2-carboxylate (Intermediate 3)

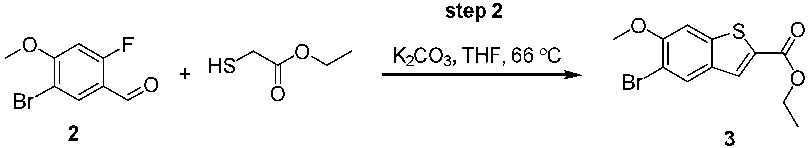

5-Bromo-2-fluoro-4-methoxybenzaldehyde (25.0 g, 108.2 mmol) was dissolved in THF (250 mL), and then Cs₂CO₃ (14.0 g, 42.9 mmol) and ethyl 2-mercaptoacetate (2.8 g, 23.6 mmol) were added. The reaction mixture was then sealed well and stirred at 66°C under N₂ protection overnight. The reaction mixture was cooled to room temperature, quenched into ice water (1000 mL), and then diluted with EtOAc (1000 mL). The resulting mixture was extracted with EtOAc (1000 mL). The organic phase was washed with water (1000 mL) and brine (1000 mL), dried over Na₂SO₄, and filtered. The filtrate was concentrated under vacuum to give a crude sample. The crude product was further purified by column chromatography (SiO₂, petroleum ether:ethyl acetate = 3:1) to give the product 3 as a white solid (6.8 g, 83%): ¹H NMR (400 MHz, Chloroform- *d*) δ 8.03 (s, 1H), 7.89 (s, 1H), 7.29 (s, 1H), 4.39 (q, *J =* 7.1 Hz, 2H), 3.98 (s, 3H), 1.41 (t, *J =* 7.1 Hz, 3H).

### Step (III): Synthesis of 5-bromo-6-methoxybenzo[b]thiophene-2-carboxylic acid (Intermediate 4)

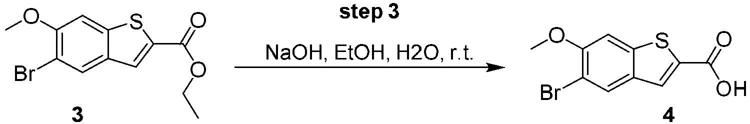

Ethyl 5-bromo-6-methoxybenzo[b]thiophene-2-carboxylate (5.6 g, 17.8 mmol) was dissolved in EtOAC (50 mL) and H₂O (50 mL), and then NaOH (2.1 g, 53.3 mmol) was added. The reaction mixture was stirred at room temperature for 2 hours. The reaction mixture was then adjusted to pH = 6, diluted with water (200 mL), and extracted twice with EtOAc (200 mL). The mixture was dried over Na₂SO₄ and filtered. The filtrate was concentrated under vacuum to give the product 4 as a white solid (5.0 g, 98%): ¹H NMR (400 MHz, DMSO- *d*₆) δ 8.26 (s, 1H), 7.98 (s, 1H), 7.79 (s, 1H), 3.93 (s, 3H).

### Step (IV): Synthesis of (5-bromo-6-methoxybenzothiophen-2-yl)(morpholino)methanone (Intermediate 5)

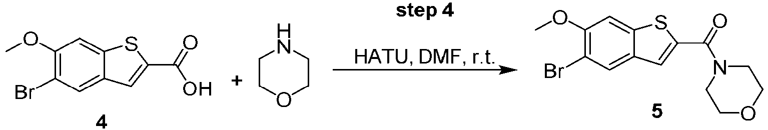

5-Bromo-6-methoxybenzo[b]thiophene-2-carboxylic acid (5.0 g, 17.4 mmol) was dissolved in DMF (100 mL), and then HATU (13.2 g, 34.8 mmol) and morpholine (7.6 g, 87.1 mmol) were added. The reaction mixture was stirred at room temperature for 1 hour. The reaction mixture was quenched into water (500 mL) and then diluted with EtOAc (500 mL). The resulting mixture was extracted three times with EtOAc (100 mL). The organic phase was washed twice with water (500 mL) and twice with brine (500 mL), dried over Na₂SO₄, and filtered. The filtrate was concentrated under vacuum to give a crude sample. The crude product was further purified by column chromatography (SiO₂, petroleum ether:ethyl acetate = 10:1) to give the product 5 as a white solid (6.0 g, 97%): ESI MS [M + H]⁺ for C₁₄H₁₄BrNO₃S, calcd 357.2, found 355.9 and 357.9.

### Step (V): Synthesis of ethyl (E)-3-(6-methoxy-2-(morpholine-4-carbonyl)benzo[b]thiophen-5-yl)acrylate (Intermediate 6)

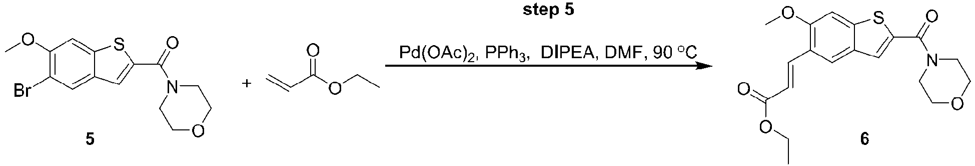

(5-Bromo-6-methoxybenzo[b]thiophen-2-yl)(morpholino)methanone (5.0 g, 14.0 mmol) was dissolved in DMF (100 mL), and then palladium acetate (1.6 g, 7.0 mmol), triphenylphosphine (1.8 g, 7.0 mmol), ethyl acrylate (4.2 g, 42.1 mmol), and DIPEA (5.4 g, 42.1 mmol) were added. The reaction mixture was purged with N₂ three times. The reaction mixture was then sealed well and stirred at 90°C under N₂ protection overnight. The reaction mixture was cooled to room temperature and quenched into ice-water (500 mL). The mixture was extracted with EtOAc (500 mL). The organic phase was washed with water (500 mL) and brine (500 mL), dried over Na₂SO₄, and filtered. The filtrate was concentrated under vacuum to give a crude sample. The crude product was purified by column chromatography (SiO₂, petroleum ether:ethyl acetate = 3:1) to give the product 6 as a yellow solid (5.0 g, 95%): ESI MS [M + H]⁺ for C₁₉H₂₁NO₅S, calcd 376.4, found 376.1.

### Step (VI): Synthesis of ethyl 3-(6-methoxy-2-(morpholine-4-carbonyl)benzo[b]thiophen-5-yl)propanoate (Compound 7)

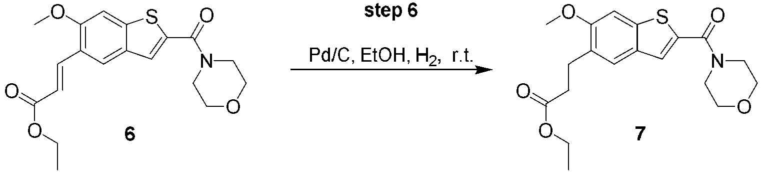

Ethyl (E)-3-(6-methoxy-2-(morpholine-4-carbonyl)benzo[b]thiophen-5-yl)acrylate (5.0 g, 13.3 mmol) was dissolved in EtOH (100 mL), and then Pd/C (15.0 g, 141.0 mmol) was added. The mixture was purged with H₂ three times. The reaction mixture was stirred at room temperature for 72 hours. The reaction solution was filtered, and the filter cake was washed with EtOAc (100 mL). The filtrate was concentrated under vacuum to give the crude product 7 as a yellow solid (4.5 g, 90%): ESI MS [M + H]⁺ for C₁₉H₂₃NO₅S, calcd 378.5, found 378.1.

### Step (VII): Synthesis of 3-(6-methoxy-2-morpholine-4-carbonyl)benzo[b]thiophen-5-yl)propanoic acid (Intermediate 8)

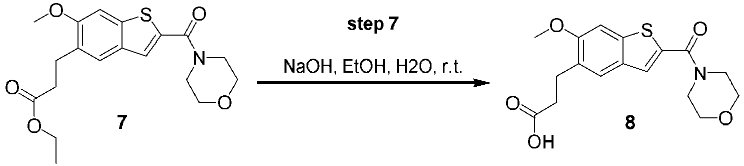

Ethyl 3-(6-methoxy-2-(morpholine-4-carbonyl)benzo[b]thiophen-5-yl)propanoate (4.5 g, 11.9 mmol) was dissolved in EtOH (80 mL) and H₂O (20 mL), and then sodium hydroxide (2.4 g, 59.6 mmol) was added. The reaction mixture was then stirred at room temperature for 2 hours. The reaction mixture was adjusted to pH = 5 and diluted with water (200 mL). The mixture was extracted with EtOAc (200 mL). The organic phase was washed with water (200 mL) and brine (200 mL). The organic phase was collected, dried over Na₂SO₄, and concentrated under vacuum to give the product 8 as a white solid (3.8 g, 91%): ESI MS [M + H]⁺ for C₁₇H₁₉NO₅S, calcd 350.4, found 350.0.

### Step (VIII): Synthesis of 5-methoxy-2-morpholine-4-carbonyl-6,7-dihydro-8H-indeno[5,4-b]thiophen-8-one (Intermediate 9)

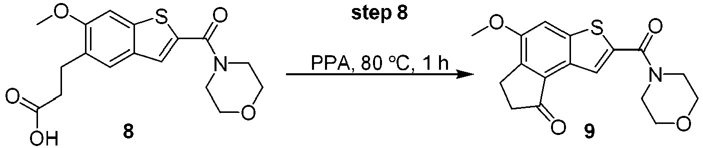

3-(6-Methoxy-2-(morpholine-4-carbonyl)benzo[b]thiophen-5-yl)propanoic acid (2.8 g, 8.0 mmol) was dissolved in PPA (28 mL). The mixture was purged with N₂ three times. The reaction mixture was stirred at 80°C for 1 hour. The reaction mixture was then cooled to room temperature, quenched into ice water (200 mL), and diluted with EtOAc (200 mL). The organic phase was washed with water (200 mL) and brine (200 mL), dried over Na₂SO₄, and filtered. The filtrate was concentrated under vacuum to give a crude sample. The crude product was further purified by column chromatography (SiO₂, petroleum ether:ethyl acetate = 1:1) to give the product 9 as a white solid (1.5 g, 57%): ESI MS [M + H]⁺ for C₁₇H₁₇NO₄S, calcd 332.4, found 332.0.

### Step (IX): Synthesis of 8-hydroxy-5-methoxy-7,8-dihydro-6H-indeno[5,4-b]thiophen-2-yl)(morpholino)methanone (Intermediate 10)

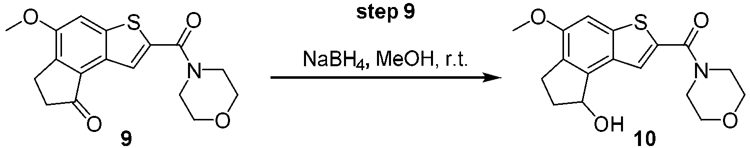

5-Methoxy-2-(morpholine-4-carbonyl)-6,7-dihydro-8H-indeno[5,4-b]thiophen-8-one (1.5 g, 4.5 mmol) was dissolved in MeOH (20 mL). NaBH₄ (856.2 mg, 22.6 mmol) was then added, and the reaction mixture was stirred at room temperature for 30 minutes. The mixture was purged with N₂ three times, and the reaction mixture was stirred at 80°C for 1 hour. The reaction mixture was then diluted with water (100 mL). The resulting mixture was extracted with DCM (100 mL). The organic phase was washed with water (100 mL) and brine (100 mL), dried over Na₂SO₄, and filtered. The filtrate was concentrated under vacuum to give the product 10 as a white solid (1.2 g, 80%): ESI MS [M + H]⁺ for C₁₇H₁₉NO₄S, calcd 334.4, found 334.0.

### Step (X): Synthesis of 5-methoxy-7,8-dihydro-6H-indeno[5,4-b]thiophen-2-yl)(morpholino)methanone (Intermediate 11)

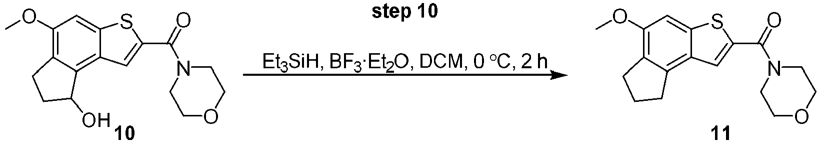

8-Hydroxy-5-methoxy-7,8-dihydro-6H-indeno[5,4-b]thiophen-2-yl)(morpholino)methanone (1.2 g, 3.6 mmol) was dissolved in DCM (24 mL). The mixture was purged with N₂ three times, and the reaction mixture was cooled to 0°C under N₂ protection. Triethylsilane (1.2 g, 10.8 mmol) and boron trifluoride diethyl etherate (47%) (1.6 g, 5.4 mmol) were then added dropwise at 0°C. The reaction mixture was stirred at room temperature for 1 hour. The reaction mixture was quenched with methanol (5 mL) and diluted with water (50 mL). The mixture was extracted with DCM (50 mL). The organic phase was washed with water (50 mL) and brine (50 mL), dried over Na₂SO₄, and filtered. The filtrate was concentrated under vacuum to give a crude sample. The crude product was further purified by column chromatography (SiO₂, petroleum ether:ethyl acetate = 5:1) to give the product 11 as a white solid (520 mg, 46%): ESI MS [M + H]⁺ for C₁₇H₁₉NO₃S, calcd 318.4, found 318.0.

### Step (XI): Synthesis of 1-(5-methoxy-7,8-dihydro-6H-indeno[5,4-b]thiophen-2-yl)ethan-1-one (Intermediate 12)

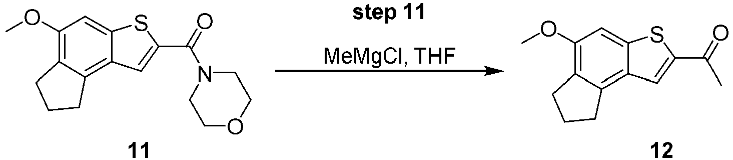

5-Methoxy-7,8-dihydro-6H-indeno[5,4-b]thiophen-2-yl)(morpholino)methanone (510.0 mg, 1.6 mmol) was dissolved in THF (10 mL). The mixture was purged with N₂ three times, and the reaction mixture was cooled to 0°C under N₂ protection. A 1.0M solution of methylmagnesium chloride in THF (4.8 mL, 4.8 mmol) was then added dropwise at 0°C. The reaction mixture was stirred at room temperature for 1 hour. The reaction mixture was quenched into ice-water (20 mL) and then diluted with EtOAc (20 mL). The resulting mixture was extracted with EtOAc (20 mL). The organic phase was then washed with water (20 mL) and brine (20 mL), dried over Na₂SO₄, and filtered. The filtrate was concentrated under vacuum to give a crude sample. The crude product was further purified by column chromatography (SiO₂, petroleum ether:ethyl acetate = 1:1) to give the product 12 as a yellow solid (330 mg, 83%): ¹H NMR (400 MHz, DMSO-*d*₆) δ 8.18 (d, *J=* 0.7 Hz, 1H), 7.41 (s, 1H), 3.87 (s, 3H), 3.17 (t, *J=* 7.5 Hz, 2H), 2.87 (t, *J=* 7.4 Hz, 2H), 2.61 (s, 3H), 2.15 (p, *J =* 7.5 Hz, 2H), ESI MS [M + H]⁺ for C₁₄H₁₄O₂S, calcd 247.3, found 247.0.

### Step (XII): Synthesis of ethyl 4-(5-methoxy-7,8-dihydro-6H-indeno[5,4-b]thiophen-2-yl)-4-oxobutanoate (Intermediate 13)

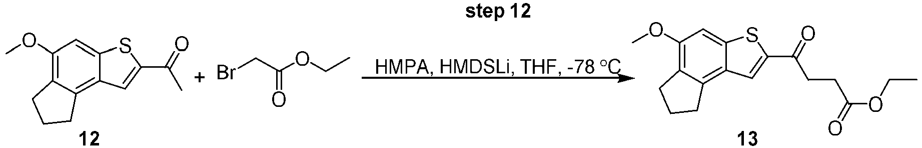

1-(5-Methoxy-7,8-dihydro-6H-indeno[5,4-b]thiophen-2-yl)ethan-1-one (100.0 mg, 0.4 mmol) and HMPA (254.6 mg, 1.4 mmol) were dissolved in anhydrous THF (2 mL). The mixture was purged with N₂ three times. The reaction mixture was cooled to -78°C under N₂ protection using an ethanol/CO₂(s) bath. A 1M solution of HMDSLi in THF (0.6 mL, 0.6 mmol) was then added dropwise and stirred at -40°C for 1.5 hours. The reaction mixture was then cooled to -78°C, and ethyl 2-bromoacetate (135.6 mg, 0.8 mmol) was added dropwise. The mixture was stirred at -78°C under N₂ protection for 2 hours. The reaction mixture was diluted with ice water (20 mL) and then with EtOAc (20 mL). The organic phase was then washed with water (20 mL) and brine (20 mL), dried over Na₂SO₄, and concentrated under vacuum to give the crude product 13 as a yellow solid (80.0 mg, 59%): ESI MS [M + H]⁺ for C₁₈H₂₀O₄S, calcd 333.4, found 333.0.

### Step (XIII): Synthesis of 4-(5-methoxy-7,8-dihydro-6H-indeno[5,4-b]thiophen-2-yl)-4-oxobutanoic acid (Example 26)

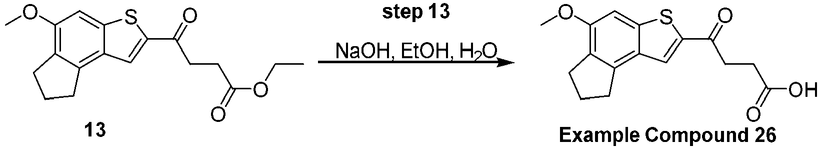

Ethyl 4-(5-methoxy-7,8-dihydro-6H-indeno[5,4-b]thiophen-2-yl)-4-oxobutanoate (100 mg, 0.3 mmol) was dissolved in H₂O (2 mL) and EtOH (2 mL), and then NaOH (60.2 mg, 1.5 mmol) was added. The reaction mixture was stirred at room temperature for 2 hours. The reaction mixture was adjusted to pH = 5-6 and concentrated under vacuum to give a crude sample. The crude product was further purified by reverse-phase HPLC (C₁₈ column, 30-60% of MeCN and water containing 0.1% FA) to give the product ZSSW2104140-01 as an off-white solid (26 mg, 28%): ¹H NMR (400 MHz, DMSO-*d*₆) δ 12.00 (s, 1H), 8.23 (s, 1H), 7.41 (s, 1H), 3.87 (s, 3H), 3.29 (t, *J=* 6.4 Hz, 2H), 3.18 (t, *J=* 7.4 Hz, 2H), 2.88 (t, *J=* 7.4 Hz, 2H), 2.59 (t, *J=* 6.4 Hz, 2H), 2.15 (p, *J* = 7.5 Hz, 2H). ESI MS [M + H]⁺ for C₁₆H₁₆O₄S, calcd 305.4, found 305.1.

### Examples 27

### Synthesis of 4-(4-methoxy-2-methylthieno[3,2-e]benzofuran-7-yl)-2-methyl-4-oxobutanoic acid (Example Compound 27)

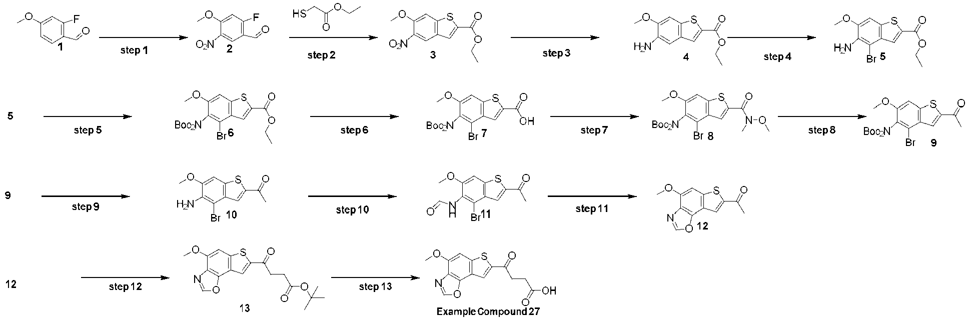

The first four steps refer to Example 25.

### Step (V): Synthesis of ethyl 5-tert-butoxycarbonylamino-4-bromo-6-methoxybenzo[b]thiophene-2-carboxylate

### (Intermediate 6)

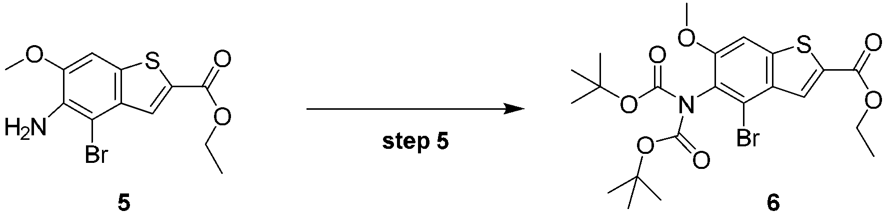

Ethyl 5-amino-4-bromo-6-methoxybenzo[b]thiophene-2-carboxylate (5 g, 15.14 mmol) was dissolved in DMF (50 mL), and then dicarbonyl di-tert-butyl ester (1.6 g, 75.7 mmol), 4-dimethylamino pyridine (1.8 g, 15.4 mmol), and triethylamine (9.1 mg, 90 mmol) were added. The mixture was stirred at 20°C for 2 h. After completion of the reaction, the reaction solution was cooled to room temperature, and the reaction was quenched by the addition of water. The mixture was extracted with EA, and the layers were separated. The organic phase was then washed twice with water, washed with saturated brine, dried over Na₂SO₄, and filtered. The filtrate was concentrated under reduced pressure to give a crude product. The crude product was further purified by column chromatography (SiO₂, EA/petroleum ether = 5/1) to give Intermediate 6 (7.5 g, 94%) as a white solid. ESI MS[M + H]⁺ for C₂₂H₂₈BrNO₇S, calcd 531.4, found 331.8.

### Step (VI): Synthesis of 5-tert-butoxycarbonylamino-4-bromo-6-methoxybenzo[b]thiophene-2-carboxylic acid (Intermediate 7)

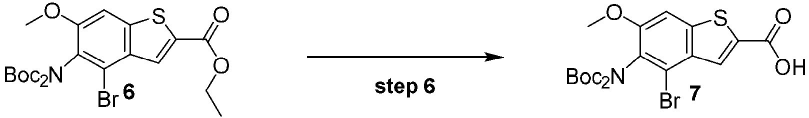

Ethyl 5-tert-butoxycarbonylamino-4-bromo-6-methoxybenzo[b]thiophene-2-carboxylate (7.5 g, 14.14 mmol) and NaOH (3.9 g, 98.9 mmol) were dissolved in ethanol (75 mL) and water (75 mL) and stirred at 25°C for 1 hour. After the reaction was monitored to be complete by LCMS, the reaction mixture was adjusted to pH = 3 and diluted with water (1 L). The mixture was extracted with EtOAc (1 L). The organic phase was washed with water (1 L) and brine (100 mL). The organic phase was collected, dried over Na₂SO₄, and concentrated under vacuum to give the pure compound Intermediate 7 as a white solid (6.5 g, 91.5%), ESI MS [M + H]⁺ for C₂₀H₂₄BrNO₄S, calcd 503.3, found 303.3.

### Step (VII): Synthesis of 5-tert-butoxycarbonylamino-4-bromo-N,6-dimethoxy-N-methylbenzo[b]thiophene-2-carbo xamide (Intermediate 8)

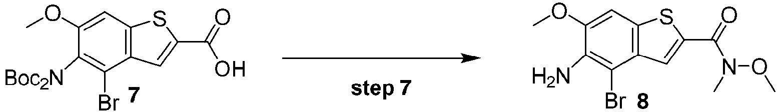

5-Tert-butoxycarbonylamino-4-bromo-6-methoxybenzo[b]thiophene-2-carboxylic acid (6.5 g, 12.94 mmol) and NaOH (3.9 g, 98.9 mmol) were dissolved in DCM (75 mL), and carbodiimide hydrochloride (4.9 g, 59 mmol), dimethylhydroxylamine hydrochloride (3.8 g, 38 mmol), and triethylamine (6.5 g, 64.5 mmol) were added. The mixture was stirred at 30°C under nitrogen protection for 2 hours. After the reaction was monitored to be complete by LCMS, the reaction mixture was adjusted to pH = 3 and diluted with water (1 L). The mixture was extracted with EtOAc (1 L). The organic phase was washed with water (1 L) and brine (100 mL). The organic phase was collected, dried over Na₂SO₄, and concentrated under vacuum to give the pure compound Intermediate 8 as a white solid (5.7 g, 81.5%), ESI MS [M + H]⁺ for C₂₂H₂₉BrN₂O₇S, calcd 546.4, found 390.9 (fragment ion after BOC cleavage).

### Step (VIII): Synthesis of 1-(5-tert-butoxycarbonylamino-4-bromo-6-methoxybenzo[b]thiophen-2-yl)ethan-1-one (Intermediate 9)

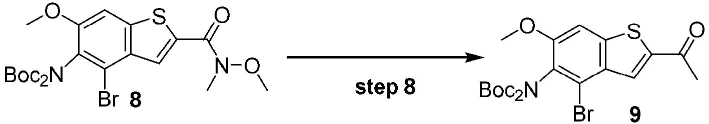

5-Tert-butoxycarbonylamino-4-bromo-N,6-dimethoxy-N-methylbenzo[b]thiophene-2-carboxami de (6.5 g, 12.94 mmol) was dissolved in THF (75 mL), and methylmagnesium chloride (2.3 g, 31.3 mmol) was added in an ice-water bath. The mixture was stirred at 25°C under nitrogen protection for 2 hours. After the reaction was monitored to be complete by LCMS, the reaction mixture was diluted with water (1 L). The mixture was extracted with EtOAc (1 L). The organic phase was washed with water (1 L) and saturated ammonium chloride (100 mL). The organic phase was collected, dried over Na₂SO₄, and concentrated under vacuum to give the pure compound Intermediate 9 as a white solid (4.24 g, 81%), ESI MS [M + H]⁺ for C₂₁H₂₆BrNO₆S, calcd 501.4, found 301.8 (fragment ion after BOC cleavage).

### Step (IX): Synthesis of 1-(5-amino-4-bromo-6-methoxybenzo[b]thiophen-2-yl)ethan-1-one (Intermediate 10)

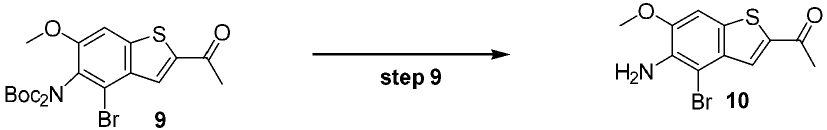

1-(5-Tert-butoxycarbonylamino-4-bromo-6-methoxybenzo[b]thiophen-2-yl)ethan-1-one (4.24 g, 8.5 mmol) was dissolved in DCM (75 mL), and HCl (3.1 g, 87 mmol) was added. The mixture was stirred at 25°C for 16 hours. After the reaction was monitored to be complete by LCMS, the reaction mixture was diluted with water (1 L) and adjusted to pH = 7. The mixture was extracted with EtOAc (1 L). The organic phase was washed with water (1 L) and saturated ammonium chloride (100 mL). The organic phase was collected, dried over Na₂SO₄, and concentrated under vacuum to give the pure compound Intermediate 10 as a white solid (2.2 g, 86%), ESI MS [M + H]⁺ for C₁₁H₁₀BrNO₂S, calcd 301.2, found 301.8.

### Step (X): Synthesis of N-(2-acetyl-4-bromo-6-methoxybenzo[b]thiophen-5-yl)formamide (Intermediate 11)

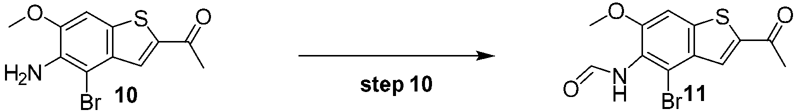

1-(5-Amino-4-bromo-6-methoxybenzo[b]thiophen-2-yl)ethan-1-one (1.2 g, 4 mmol) was dissolved in DCM (100 mL), and formic acid (1.8 g, 40 mmol) and acetoacetic ester (816 g, 8 mmol) were added. The mixture was stirred at 25°C for 3 hours. After the reaction was monitored to be complete by LCMS, the reaction mixture was filtered, and the filter cake was washed with dichloromethane to give the pure compound Intermediate 11 as a white solid (1.2 g, 91%), ESI MS [M + H]⁺ for C₁₂H₁₀BrNO₃S, calcd 329.2, found 329.8.

### Step (XI): Synthesis of 1-(4-methoxythieno[3,2:3,4]benzo[1,2-d]oxazol-7-yl)ethan-1-one (Intermediate 12)

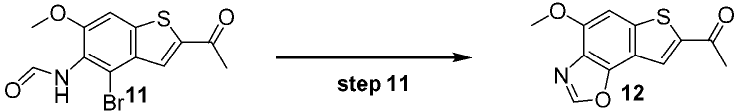

N-(2-Acetyl-4-bromo-6-methoxybenzo[b]thiophen-5-yl)formamide (1.0 g, 3 mmol) was dissolved in 1,4-dioxane (100 mL), and methyldimethylamine ethylamine (288 mg, 3.08 mmol), CuI (290 mg, 1.52 mmol), and cesium carbonate (1.4 g, 4.57 mmol) were added. The mixture was stirred at 90°C for 2 hours. After the reaction was monitored to be complete by LCMS, the reaction mixture was filtered. The filtrate was concentrated and purified by column chromatography (PE:EA = 2:1) to give the pure compound Intermediate 12 as a white solid (300 mg, 40%), ESI MS [M + H]⁺ for C₁₂H₉NO₃S, calcd 248.3, found 247.96, ¹H NMR (400 MHz, DMSO) δ 8.81 (s, 1H), 8.52 (s, 1H), 7.63 (s, 1H), 4.04 (s, 3H), 2.67 (s, 3H).

### Step (XII): Synthesis of tert-butyl 4-(4-methoxythieno[3',2':3,4]benzo[1,2-d]oxazol-7-yl)-4-oxobutanoate (Intermediate 13)

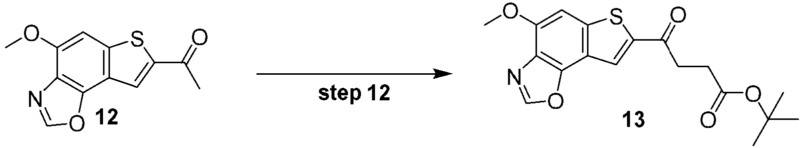

1-(4-Methoxythieno[3,2:3,4]benzo[1,2-d]oxazol-7-yl)ethan-1-one (80 mg, 0.3 mmol) was dissolved in THF (2 mL), and HMPA (202 mg, 1.3 mmol) was added. The mixture was cooled to -78°C, and HMDSLi (81 mg, 0.5 mmol) was added dropwise under nitrogen protection. The mixture was warmed -40°C, stirred for 1 hour, and then cooled to -78°C. Ethyl bromoacetate (126 mg, 0.65 mmol) was added dropwise and then stirred at -78°C for 2 h. After the reaction was monitored to be complete by LCMS, the reaction mixture was diluted with water (100 mL). The mixture was extracted with EtOAc (100 mL). The organic phase was washed with water (100 mL) and brine (100 mL). The organic phase was collected, dried over Na₂SO₄, and concentrated under vacuum to give the pure compound Intermediate 13 as a white solid (100 mg, 41%), ESI MS [M + H]⁺ for C₂₂H₂₈O₇S, calcd 362.41, found 305.92 (signal for deprotection of the tert-butyl ester).

### Step (XIII): Synthesis of 4-(4-methoxythieno[3',2':3,4]benzo[1,2-d]oxazol-7-yl)-4-oxobutanoic acid (Example Compound 27)

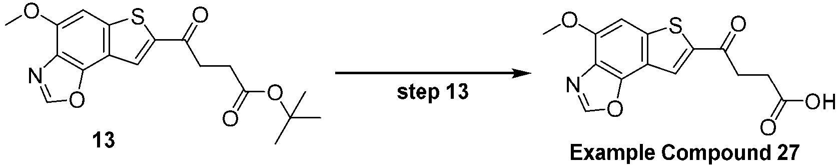

Tert-butyl 4-(4-methoxythieno[3',2':3,4]benzo[1,2-d]oxazol-7-yl)-4-oxobutanoate (100 mg, 0.28 mmol) was dissolved in trifluoroacetic acid (0.35 mg, 3.3 mmol). The reaction mixture was stirred at room temperature for half an hour. LCMS at this point indicated that the reaction was complete. The reaction mixture was concentrated under vacuum to give a crude sample. The crude product was further purified by reverse-phase HPLC (C18 column, 50-60% gradient of MeCN and water containing 0.1% TFA) to give the product as a white solid (25.5 mg, 30%), ESI MS [M + H]⁺ for C₁₄H₁₁NO₅S, calcd 306.3, found 306.07, ¹H NMR (400 MHz, DMSO-d6) δ 8.81 (s, 1H), 8.52 (s, 1H), 7.64 (s, 1H), 6.18 (s, 2H), 4.04 (s, 3H), 3.26 (t, *J =* 6.6 Hz, 2H), 2.45 (t, *J* = 6.5 Hz, 2H).

### Example 28

### Synthesis of 2-ethyl-4-(4-methoxythieno[3,2-e]benzofuran-7-yl)-4-oxobutanoic acid (Example Compound 28)

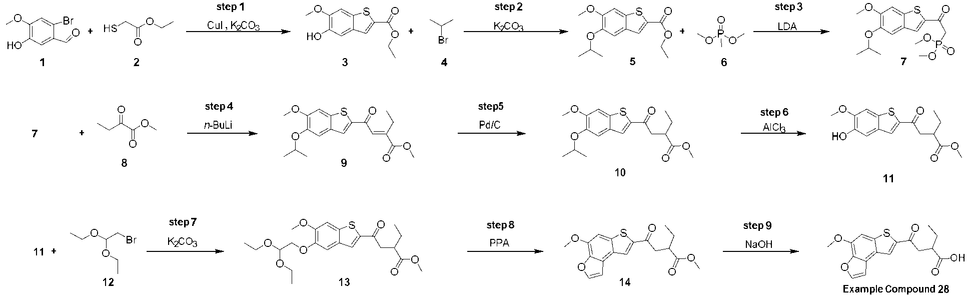

### Step (I): Synthesis of ethyl 5-hydroxy-6-methoxy-1-benzothiophene-2-carboxylate (Intermediate 3)

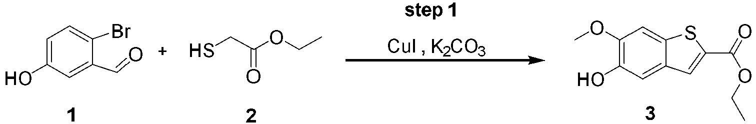

2-Bromo-5-hydroxy-4-methoxybenzaldehyde (6.0 g, 26.0 mmol) was dissolved in DMF (60 mL), and ethyl 2-mercaptoacetate (7.2 g, 59.7 mmol) and λ¹/-copper iodide (1.5 g, 7.8 mmol) were added. The mixture was then cooled to 0°C, and dipotassium carbonate (9.0 g, 64.9 mmol) was slowly added. The mixture was purged with N₂ three times. The reaction mixture was then sealed and stirred at 80°C under N₂ protection for 2 hours. The reaction mixture was quenched into ice-water (600 g) to maintain the internal temperature below 20°C. The resulting mixture was extracted with EtOAc (600 mL). The organic phase was then washed with water (400 mL) and brine (200 mL), dried over Na₂SO₄, and filtered. The filtrate was concentrated under vacuum to give a crude sample. The crude product was further purified by column chromatography (SiO₂, 20% EtOAc/petroleum ether) to give Intermediate 3 as a white solid (5.1 g, 78%), LCMS: ¹H NMR (400 MHz, DMSO-d6) δ 9.38 (s, 1H), 7.96 (d, *J =* 0.6 Hz, 1H), 7.54 (s, 1H), 7.31 (s, 1H), 4.30 (q, *J =* 7.1 Hz, 2H), 3.86 (s, 3H), 1.31 (t, *J =* 7.1 Hz, 3H).

### Step (II): Synthesis of ethyl 5-isopropoxy-6-methoxy-1-benzothiophene-2-carboxylate (Intermediate 5)

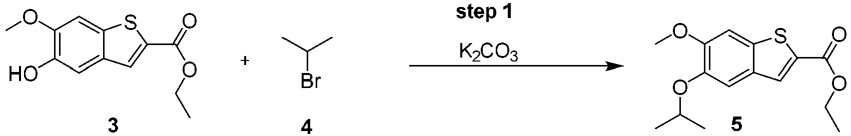

Ethyl 5-hydroxy-6-methoxy-1-benzothiophene-2-carboxylate (5.0 g, 22.0 mmol) was dissolved in DMF (60 mL), and isopropyl bromide (8.0 g, 64.9 mmol) was added. The mixture was then cooled to 0°C, and anhydrous dipotassium carbonate (15 g, 108.2 mmol) was slowly added. The mixture was purged with N₂ three times. The reaction mixture was then sealed and stirred at 45°C under N₂ protection for 12 hours. The reaction mixture was quenched into ice-water (600 g) to maintain the internal temperature below 20°C. The resulting mixture was extracted with EtOAc (600 mL). The organic phase was then washed with water (400 mL) and brine (200 mL), dried over Na₂SO₄, and filtered. The filtrate was concentrated under vacuum to give a crude sample. The crude product was further purified by column chromatography (SiO₂, 20% EtOAc/petroleum ether) to give Intermediate 3 as a white solid (5.1 g, 78%), ESI MS[M + H]⁺ for C₁₅H₁₈O₄S, calcd 295.4, found 295.01.

### Step (III): Synthesis of dimethyl (2-(5-isopropoxy-6-methoxy-1-benzothiophene)-2-oxoethyl)phosphonate (Intermediate 7)

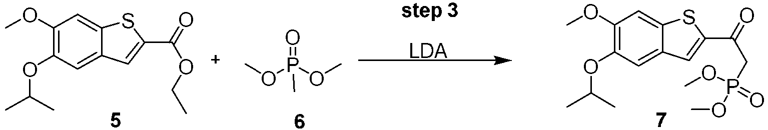

Ethyl 5-isopropoxy-6-methoxy-1-benzothiophene-2-carboxylate (5.15 g, 17.5 mmol) was dissolved in THF (50 mL), and the mixture was purged with N₂ three times. The reaction mixture was then sealed. The mixture was then cooled to -20°C, and LDA (3.8 g, 35.0 mmol) was slowly added dropwise. After addition, the mixture was stirred for 5 min, and then dimethyl methylphosphonate (2.2 g, 17.5 mmol) was added dropwise. The mixture was stirred at 25°C under N₂ protection for 12 hours. The reaction mixture was quenched into ice-water (600 g) to maintain the internal temperature below 20°C. The resulting mixture was extracted with EtOAc (600 mL). The organic phase was then washed with water (400 mL) and brine (200 mL), dried over Na₂SO₄, and filtered. The filtrate was concentrated under vacuum to give a crude sample. The crude product was further purified by column chromatography (SiO₂, 20% EtOAc/petroleum ether) to give Intermediate 7 as a white solid (6.0 g, 92.1%), LCMS: ESI MS[M + H]⁺ for C₁₆H₂₁O₆PS, calcd 373.4, found 373.05, ¹H NMR (400 MHz, DMSO-d6) δ 8.28 (s,1H), 7.61 (s,1H), 7.51 (s, 1H), 4.70 - 4.59 (m,1H), 3.92 - 3.83 (m, 5H), 3.69 (d, J = 11.1 Hz, 6H), 1.31 (d, J = 6.1 Hz, 6H).

### Step (IV): Synthesis of methyl 2-ethyl-4-(5-isopropoxy-6-methoxy-1-benzothiophene)-4-oxobut-2-enoate (Intermediate 9)

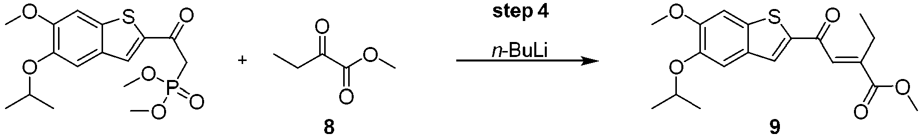

Dimethyl 2-(5-isopropoxy-6-methoxy-1-benzothiophene)-2-oxoethyl)phosphonate (5.2 g, 13.83 mmol) was dissolved in THF (50 mL). The mixture was then cooled to -20°C, and LDA (1.5 g, 13.8 mmol) was slowly added dropwise. After addition, the mixture was stirred for 5 min, and then methyl 2-oxobutanoate (1.6 g, 13.8 mmol) was added dropwise. The mixture was stirred at 25°C under N₂ protection for 1 hour. The reaction mixture was quenched into ice-water (600 g) to maintain the internal temperature below 20°C. The resulting mixture was extracted with EtOAc (600 mL). The organic phase was then washed with water (400 mL) and brine (200 mL), dried over Na₂SO₄, and filtered. The filtrate was concentrated under vacuum to give a crude sample. The crude product was further purified by column chromatography (SiO₂, 20% EtOAc/petroleum ether) to give Intermediate 3 as a white solid (4.5 g, 89.8%), LCMS: ESI MS[M + H]⁺ for C₁₉H₂₂O₅S, calcd 363.4, found 363.08, ¹H NMR (400 MHz, DMSO-d6) δ 8.28 (s,1H), 7.61 (s,1H), 7.51 (s, 1H), 4.70 - 4.59 (m,1H), 3.92 - 3.83 (m, 5H), 3.69 (d, J = 11.1 Hz, 6H), 1.31 (d, J = 6.1 Hz, 6H).

### Step (V): Synthesis of methyl 2-ethyl-4-(5-isopropoxy-6-methoxy-1-benzothiophene)-4-oxobutanoate (Intermediate 10)

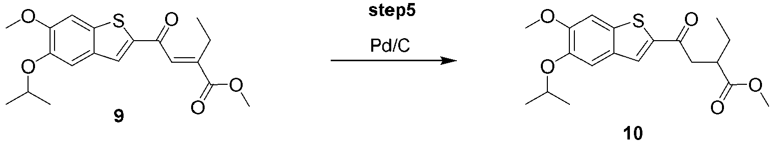

Methyl 2-ethyl-4-(5-isopropoxy-6-methoxy-1-benzothiophene)-4-oxobut-2-enoate (4.5 g, 12.4 mmol) was dissolved in EA (50 mL), and Pd/C (0.45 g, 1.24 mmol) and copper iodide (1.5 g, 7.8 mmol) were added. The mixture was purged with H₂ three times. The reaction mixture was then sealed and stirred at 25°C under H₂ protection for 12 hours. The reaction mixture was filtered to remove the remaining Pd/C. The filtrate was concentrated under vacuum to give a crude sample. The crude product was further purified by column chromatography (SiO₂, 20% EtOAc/petroleum ether) to give Intermediate 10 as a white solid (2.4 g, 53.0%), LCMS: ESI MS[M + H]⁺ for C₁₉H₂₄O₅S, calcd 365.5, found 365.08.

### Step (VI): Synthesis of methyl 2-ethyl-4-(5-hydroxy-6-methoxy-1-benzothiophene)-4-oxobutanoate (Intermediate 11)

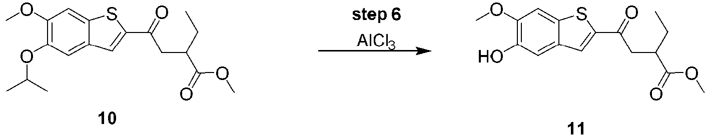

Methyl 2-ethyl-4-(5-isopropoxy-6-methoxy-1-benzothiophene)-4-oxobutanoate (2.6 g, 7.1 mmol) was dissolved in DCM (30 mL), and AlCl₃ (0.95 g, 7.1 mmol) was added. The mixture was purged with N₂ three times. The reaction mixture was then sealed and stirred at 25°C under N₂ protection for 2 hours. The reaction mixture was quenched into ice-water (600 g) to maintain the internal temperature below 20°C. The resulting mixture was extracted with EtOAc (600 mL). The organic phase was then washed with water (400 mL) and brine (200 mL), dried over Na₂SO₄, and filtered. The filtrate was concentrated under vacuum to give a crude sample. The crude product was further purified by column chromatography (SiO₂, 20% EtOAc/petroleum ether) to give Intermediate 11 as a white solid (2.3 g, 99%), LCMS: ESI MS[M + H]⁺ for C₁₆H₁₈O₅S, calcd 323.4, found 322.99.

### Step (VII): Synthesis of methyl 4-(5-(2,2-diethoxyethoxy)-6-methoxy-1-benzothiophene)-2-ethyl-4-oxobutanoate (Intermediate 13)

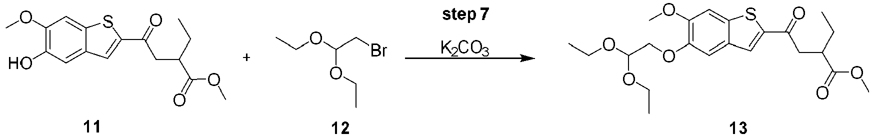

Methyl 2-ethyl-4-(5-hydroxy-6-methoxy-1-benzothiophene)-4-oxobutanoate (1.0 g, 3.1 mmol) was dissolved in DMF (10 mL), and 2-bromo-1,1-diethoxyethane (1.8 g, 9.3 mmol) was added. The mixture was then cooled to 0°C, and dipotassium carbonate (2.1 g, 15.5 mmol) was slowly added. The mixture was purged with N₂ three times. The reaction mixture was then sealed and stirred at 110°C under N₂ protection for 3 hours. The reaction mixture was quenched into ice-water (600 g) to maintain the internal temperature below 20°C. The resulting mixture was extracted with EtOAc (600 mL). The organic phase was then washed with water (400 mL) and brine (200 mL), dried over Na₂SO₄, and filtered. The filtrate was concentrated under vacuum to give a crude sample. The crude product was further purified by column chromatography (SiO₂, 20% EtOAc/petroleum ether) to give Intermediate 13 as a white solid (1.3 g, 95.6%), LCMS: ESI MS[M + H]⁺ for C₂₂H₃₀O₇S, calcd 439.5, found 439.12.

### Step (VIII): Synthesis of methyl 2-ethyl-4-(4-methoxythieno[3,2-e]benzofuran-7-yl)-4-oxobutanoate (Intermediate 14)

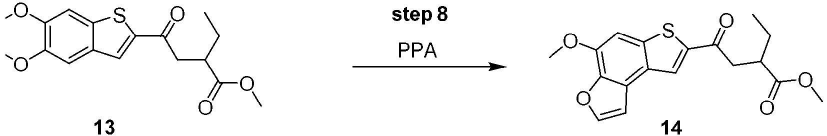

Methyl 4-(5-(2,2-diethoxyethoxy)-6-methoxy-1-benzothiophene)-2-ethyl-4-oxobutanoate (500.0 mg, 1.1 mmol) was dissolved in toluene (15 mL), and polyphosphoric acid (187.0 mg, 2.3 mmol) was added. The mixture was purged with N₂ three times. The reaction mixture was then sealed and stirred at 80°C under N₂ protection for 30 min. The reaction mixture was quenched into ice-water (600 g) to maintain the internal temperature below 20°C. The resulting mixture was extracted with EtOAc (600 mL). The organic phase was then washed with water (400 mL) and brine (200 mL), dried over Na₂SO₄, and filtered. The filtrate was concentrated under vacuum to give a crude sample. The crude product was further purified by column chromatography (SiO₂, 20% EtOAc/petroleum ether) to give Intermediate 14 as a white solid (150 mg, 38%), LCMS: ESI MS[M + H]⁺ for C₁₈H₁₈O₅S, calcd 347.4, found 347.03.

### Step (IX): Synthesis of 2-ethyl-4-(4-methoxythieno[3,2-e]benzofuran-7-yl)-4-oxobutanoic acid (Example Compound 28)

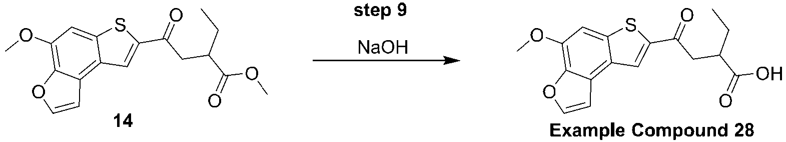

Methyl 2-ethyl-4-(4-methoxythieno[3,2-e]benzofuran-7-yl)-4-oxobutanoate (150 mg, 0.43 mmol) was dissolved in methanol (5 mL). The mixture was then cooled to 0°C, and a solution of sodium hydroxide (44.4 mg, 1.1 mmol) in water (5 mL) was slowly added. The mixture was purged with N₂ three times. The reaction mixture was then sealed and stirred at 25°C under N₂ protection for 12 hours. The reaction was quenched with ice-water. The reaction mixture was extracted with EA and concentrated to give a crude product. The crude product was then purified by prep-HPLC (column: Hanbang DAC-50, fillers: NanoMicro UniHybrid 10-120 C18, mobile phase: 0.1% aqueous NH₄HCO₃ solution and acetonitrile, eluent: 50% ACN) to give the pure final product as a white solid (13.0 mg, 17%), LCMS: ESI MS[M + H]⁺ for C₁₇H₁₆O₅S, calcd 332.4, found 333.00. ¹H NMR (400 MHz, DMSO-d6) δ 8.65 (s, 1H), 8.14 (d, J = 2.0 Hz, 1H), 7.58 (s, 1H), 7.44 (d, J = 2.0 Hz, 1H), 4.02 (s, 3H), 3.44 (dd, J = 16.9, 8.9 Hz, 2H), 3.02 (dd, J = 16.9, 5.1 Hz, 1H), 2.74 (dq, J = 9.0, 6.2 Hz, 1H), 1.60 (ddq, J = 20.4, 13.5, 6.8 Hz, 2H), 0.92 (t, J = 7.4 Hz, 3H).

### Example 29

### Synthesis of (R)-2-ethyl-4-(4-methoxy-2-methylthieno[3,2-e]benzofuran-7-yl)-4-oxobutanoic acid (Example Compound 29)

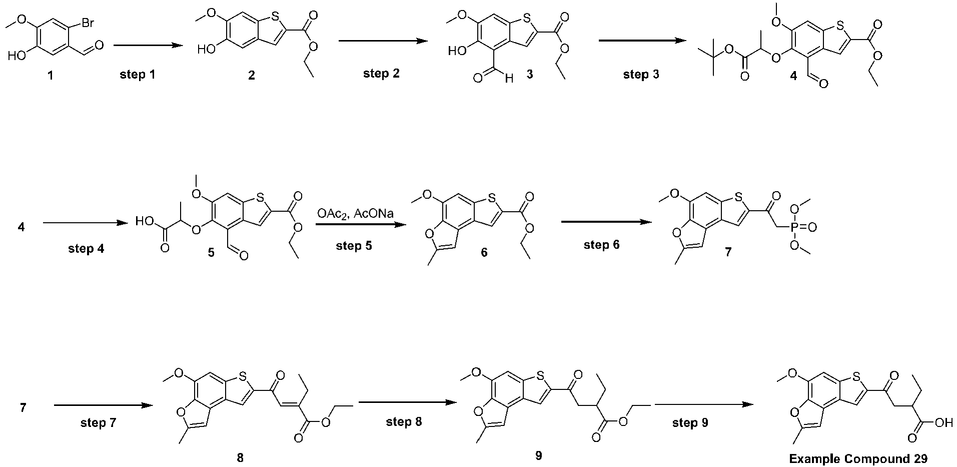

For the synthesis of dimethyl (2-(4-methoxy-2-methylthieno[3,2-e]benzofuran-7-yl)-2-oxoethyl)phosphonate (Intermediate 7), refer to Example 19.

### Step (VII): Synthesis of ethyl (E)-2-ethyl-4-(4-methoxy-2-methylthieno[3,2-e]benzofuran-7-yl)-4-oxobut-2-enoate (Intermediate 8)

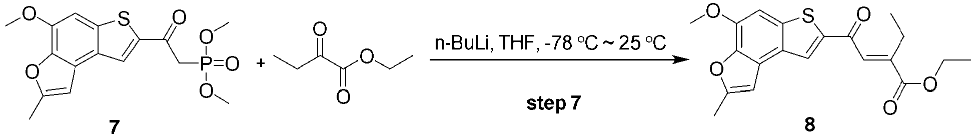

Dimethyl (2-(4-methoxy-2-methylthieno[3,2-e]benzofuran-7-yl)-2-oxoethyl)phosphonate (8.5 g, 23.1 mmol) was dissolved in THF (16 mL). The mixture was purged with nitrogen and cooled to -78°C. n-Butyllithium (9.2 mL, 2.5 M) was then added and stirred for 0.5 h, followed by methyl 2-oxopropanoate (3.3 g, 25.4 mmol). The mixture was returned to room temperature and stirred for 1 h. After completion of the reaction, 300 mL of saturated aqueous ammonium chloride solution was added to the reaction solution. The mixture was extracted with 200 mL of EA, and the layers were separated. The organic phase was washed once with 200 mL of brine and concentrated under reduced pressure to give a crude product. The crude product was further purified by column chromatography (SiO₂, PE/EA/DCM = 20/1/1) to give Intermediate 8 (6.3 g, 73%) as a yellow solid, ESI MS[M + H ]⁺ for C₂₀H₂₁O₅S, calcd 373.43, found 373.1.

### Step (VIII): Synthesis of ethyl 2-ethyl-4-(4-methoxy-2-methylthieno[3,2-e]benzofuran-7-yl)-4-oxobutanoate (Intermediate 9)

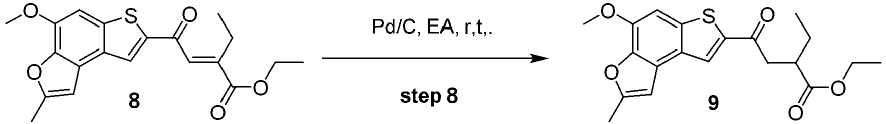

Ethyl (E)-2-ethyl-4-(4-methoxy-2-methylthieno[3,2-e]benzofuran-7-yl)-4-oxobut-2-enoate (5.2 g, 14.0 mmol) was dissolved in EA (100 mL), and then Pd/C (0.5 g, 4.7 mmol) was added. The mixture was purged with hydrogen and stirred at room temperature overnight. After completion of the reaction, the reaction solution was filtered through celite, and the celite was rinsed with 500 mL of EA. The filtrate was concentrated under reduced pressure to give a crude product. The crude product was further purified by column chromatography (SiO₂, PE/EA/DCM = 20/1/1) to give Intermediate 9 (5.0 g, 96%) as a yellow solid, ESI MS[M + H ]⁺ for C₂₀H₂₃O₅S, calcd 375.45, found 375.1.

### Step (IX): Synthesis of 2-ethyl-4-(4-methoxy-2-methylthieno[3,2-e]benzofuran-7-yl)-4-oxobutanoic acid (Example Compound 29)

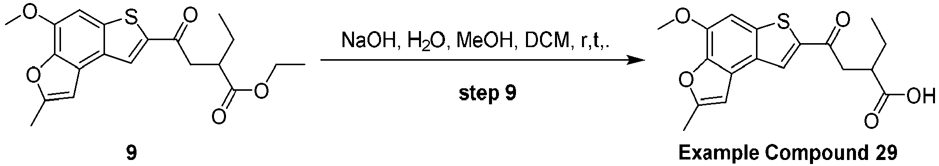

Ethyl 2-ethyl-4-(4-methoxy-2-methylthieno[3,2-e]benzofuran-7-yl)-4-oxobutanoate (3.1 g, 8.3 mmol) was dissolved in H₂O (5 mL), methanol (10 mL), and dichloromethane (10 mL), and then NaOH (1.7 g, 41.4 mmol) was added. The mixture was stirred at room temperature for 1 h. After the completion of the reaction, 200 mL of water was added to the reaction solution. The mixture was adjusted to pH = 4-5 with hydrochloric acid and extracted three times with 200 mL of DCM (with solid retained in the organic phase). The organic phase was concentrated under reduced pressure to give a crude product. The crude product was triturated with 10 mL of methyl tert-butyl ether and filtered. The filter cake was collected and dried by rotary evaporation to give Example Compound 29 (2.6 g, 91%) as a yellow solid, ESI MS[M + H ]⁺ for C₁₈H₁₉O₅S, calcd 347.4, found 347.1.

### Example 30

### Synthesis of 4-(5-methoxybenzo[1,2-b:4,3-b']dithiophen-2-yl)-4-oxobutanoic acid (Example Compound 30)

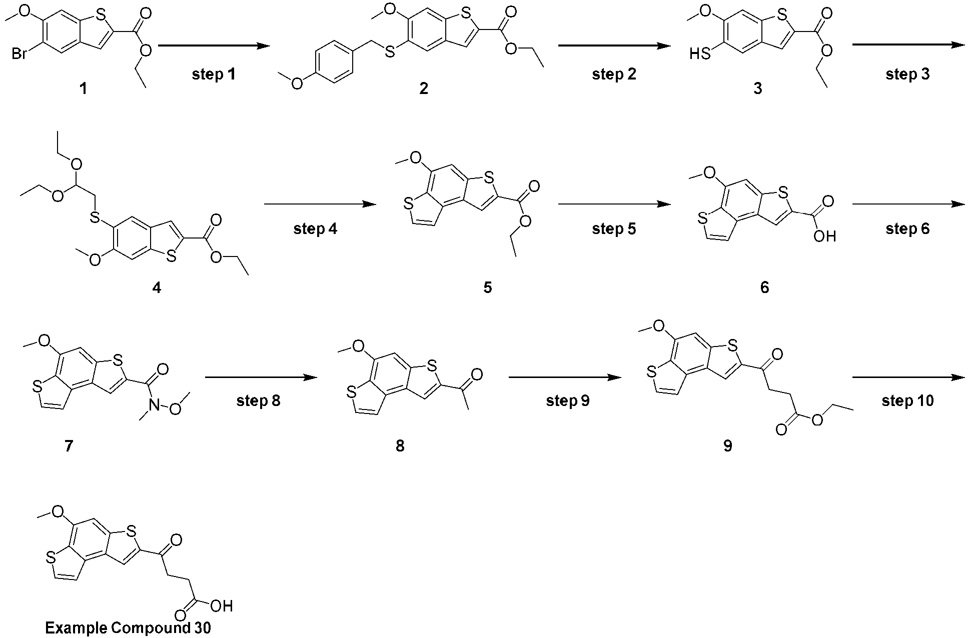

### Step (I): Synthesis of ethyl 6-methoxy-5-((4-methoxybenzyl)thio)-1-benzothiophene-2-carboxylate (Intermediate 2)

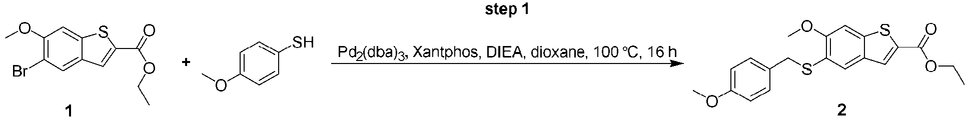

Ethyl 5-bromo-6-methoxy-1-benzothiophene-2-carboxylate (6.0 g, 19.0 mmol), Pd₂(dba)₃ (1.7 g, 1.9 mmol), Xantphos (2.2 g, 3.8 mmol), 4-methoxythiophenol (8.8 g, 57.1 mmol), and DIPEA (7.4 g, 57.1 mmol) were dissolved in 1,4-dioxane (60 mL). The mixture was purged with N₂ three times. The reaction mixture was then sealed and stirred at 100°C under N₂ protection for 16 hours. The reaction mixture was quenched into ice-water (600 g) to maintain the internal temperature below 20°C. The resulting mixture was extracted with EtOAc (600 mL). The organic phase was then washed with water (400 mL) and brine (200 mL), dried over Na₂SO₄, and filtered. The filtrate was concentrated under vacuum to give a crude sample. The crude product was further purified by column chromatography (SiO₂, 20% EtOAc/petroleum ether) to give Intermediate 2 as a white solid (6.2 g, 84%), ¹H NMR (400 MHz, DMSO) δ 8.02 (s, 1H), 7.89 (s, 1H), 7.64 (s, 1H), 7.37 - 7.28 (m, 2H), 6.90 - 6.81 (m, 2H), 4.32 (q, *J =* 7.1 Hz, 2H), 4.15 (s, 2H), 3.91 (s, 3H), 3.71 (s, 3H), 1.32 (t, *J =* 7.1 Hz, 3H).

### Step (II): Synthesis of ethyl 5-mercapto-6-methoxy-1-benzothiophene-2-carboxylate (Intermediate 3)

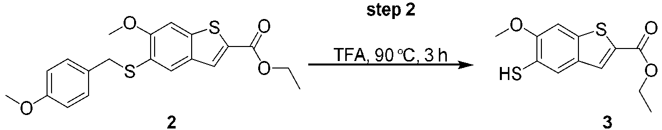

Ethyl 6-methoxy-5-((4-methoxybenzyl)thio)-1-benzothiophene-2-carboxylate (5.0 g, 12.9 mmol) was dissolved in TFA (50 mL). The reaction mixture was then sealed well and stirred at 90°C under N₂ protection for 3 hours. After the reaction was monitored to be complete by LCMS, the reaction mixture was cooled to room temperature, quenched into ice-water (500 mL), then adjusted to pH = 7, and extracted with EtOAc (500 mL). The resulting mixture was extracted with EtOAc (500 mL). The organic phase was then washed with water (500 mL) and brine (500 mL), dried over Na₂SO₄, and filtered. The filtrate was concentrated under vacuum to give a crude sample. The crude product was further purified by column chromatography (petroleum ether:EtOAc = 10:1) to give the product 3 as a white solid (3.0 g, 87%), ESI MS [M + H]⁺ for C₁₂H₁₂O₃S₂, calcd 269.4, found 269.0.

### Step (III): Synthesis of ethyl 5-((2,2-diethoxyethyl)thio)-6-methoxy-1-benzothiophene-2-carboxylate (Intermediate 4)

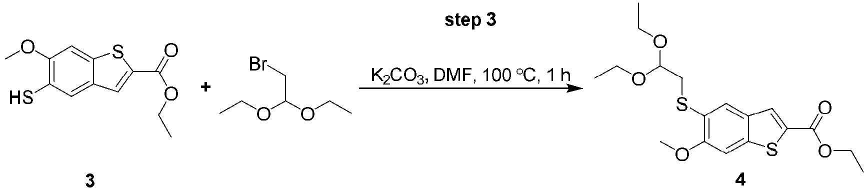

Ethyl 5-mercapto-6-methoxy-1-benzothiophene-2-carboxylate (2.6 g, 9.7 mmol), 2-bromo-1,1-diethoxyethane (5.7 g, 29.1 mmol), and potassium carbonate (4.0 g, 29.1 mmol) were dissolved in DMF (26 mL), and the mixture was purged with N₂ three times. The reaction mixture was then sealed and stirred at 100°C under N₂ protection for 1 hour. After the reaction was monitored to be complete by LCMS, the reaction mixture was quenched into ice-water (200 g) to maintain the internal temperature below 20°C. The resulting mixture was extracted with EtOAc (200 mL). The organic phase was then washed with water (200 mL) and brine (200 mL), dried over Na₂SO₄, and filtered. The filtrate was concentrated under vacuum to give a crude sample. The crude product was further purified by column chromatography (20% EtOAc/petroleum ether) to give Intermediate 4 as a white solid (3.0 g, 81%): ESI MS [M + H]⁺ for C₁₈H₂₄O₅S₂, calcd 385.5, found 292.9.

### Step (IV): Synthesis of ethyl 5-methoxybenzo[1,2-b:4,3-b']dithiophene-2-carboxylate (Intermediate 5)

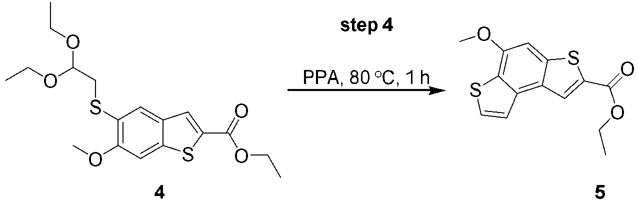

Ethyl 5-((2,2-diethoxyethyl)thio)-6-methoxy-1-benzothiophene-2-carboxylate (3.2 g, 8.3 mmol) was dissolved in toluene (10 mL), and then PPA (10 mL) was added. The mixture was stirred at 100°C under N₂ protection for 30 min. The reaction mixture was quenched into ice-water (600 g). After the reaction was monitored to be complete by LCMS, the reaction mixture was cooled to room temperature, quenched into ice-water (200 mL), and then diluted with EtOAc (100 mL). The organic phase was then washed with water (100 mL) and brine (100 mL), dried over Na₂SO₄, and filtered. The filtrate was concentrated under vacuum to give a crude sample. The crude product was further purified by column chromatography (petroleum ether:EtOAc = 50:1) to give the product 5 as a white solid (1.2 g, 47%): ESI MS [M + H]⁺ for C₁₄H₁₂O₃S₂, calcd 293.4, found 292.9.

### Step (V): Synthesis of 5-methoxybenzo[1,2-b:4,3-b']dithiophene-2-carboxylic acid (Intermediate 6)

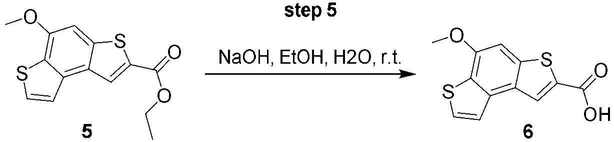

To a solution of ethyl 5-methoxybenzo[1,2-b:4,3-b']dithiophene-2-carboxylate (1.4 g, 4.8 mmol) in ethanol (20 mL) and H₂O(10 mL) was added NaOH (574.6 mg, 14.4 mmol). The reaction mixture was then stirred at room temperature for 2 hours. After the reaction was monitored to be complete by LCMS, the reaction mixture was adjusted to pH = 6 and diluted with water (100 mL). The mixture was extracted with EtOAc (100 mL). The organic phase was washed with water (100 mL) and brine (100 mL). The organic phase was collected, dried over Na₂SO₄, and concentrated under vacuum to give Compound 6 as a white solid (1.2 g, 95%), ¹H NMR (400 MHz, DMSO-d₆) δ 8.53 (s, 1H), 8.06 (d, *J=* 5.3 Hz, 1H), 7.95 (d, *J =* 5.3 Hz, 1H), 7.61 (s, 1H), 4.05 (s, 3H), ESI MS [M + H]⁺ for C₁₂H₈O₃S₂, calcd 265.3, found 264.9.

### Step (VI): Synthesis of N,5-dimethoxy-N-methylbenzo[1,2-b:4,3-b']dithiophene-2-carboxamide (Intermediate 7)

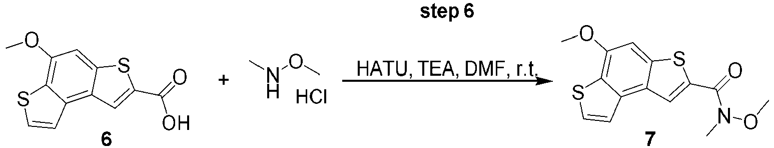

5-Methoxybenzo[1,2-b:4,3-b']dithiophene-2-carboxylic acid (1.1 g, 4.2 mmol), N,O-dimethylhydroxylamine hydrochloride (8.1 g, 8.3 mmol), HATU (3.2 g, 8.3 mmol), and triethylamine (2.1 g, 20.8 mmol) were dissolved in DMF (20 mL), and the mixture was purged with N₂ three times. The reaction mixture was stirred at room temperature for 2 hours. After the reaction was monitored to be complete by LCMS, the reaction mixture was quenched into ice-water (200 mL) and then diluted with EtOAc (200 mL). The resulting mixture was extracted with EtOAc (200 mL). The organic phase was washed twice with water (200 mL) and brine (200 mL), dried over Na₂SO₄, and filtered. The filtrate was concentrated under vacuum to give a crude sample. The crude product was further purified by column chromatography (petroleum ether:EtOAc = 20:1) to give the product 7 as a white solid (1.1 g, 86%), ESI MS [M + H]⁺ for C₁₄H₁₃NO₃S₂, calcd 308.4, found 307.9.

### Step (VII): Synthesis of 1-(5-methoxybenzo[1,2-b:4,3-b']dithiophen-2-yl)ethan-1-one (Intermediate 8)

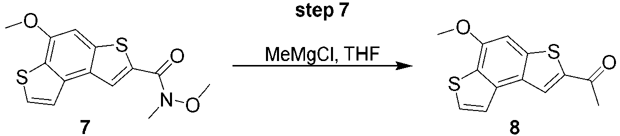

N,5-Dimethoxy-N-methylbenzo[1,2-b:4,3-b']dithiophene-2-carboxamide (1.1 g, 3.6 mmol) was dissolved in THF (20 mL), and the mixture was purged with N₂ three times. The reaction mixture was cooled to 0°C under N₂ protection. A 1.0M solution of methylmagnesium chloride in THF (10.7 mL, 10.7 mmol) was then added dropwise at 0°C. The reaction mixture was stirred at room temperature for 1 hour. After the reaction was monitored to be complete by LCMS, the reaction mixture was quenched into ice-water (200 mL) and then diluted with EtOAc (200 mL). The resulting mixture was extracted with EtOAc (200 mL). The organic phase was washed with water (200 mL) and brine (200 mL), dried over Na₂SO₄, and filtered. The filtrate was concentrated under vacuum to give a crude sample. The crude product was further purified by column chromatography (petroleum ether:EtOAc = 3:1) to give Compound 8 as an off-white solid (900 mg, 96%), ESI MS [M + H]⁺ for C₁₃H₁₀O₂S₂, calcd 263.4, found 262.9.

### Step (VIII): Synthesis of ethyl 4-(5-methoxybenzo[1,2-b:4,3-b']dithiophen-2-yl)-4-oxobutanoate (Intermediate 9)

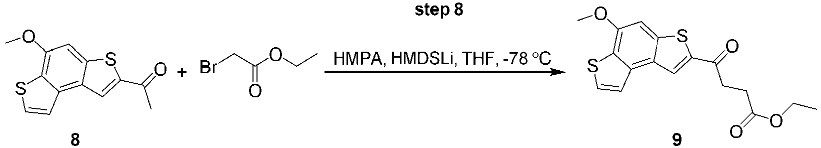

1-(5-Methoxybenzo[1,2-b:4,3-b']dithiophen-2-yl)ethan-1-one (100.0 mg, 0.4 mmol) and HMPA (239.1 mg, 1.3 mmol) were dissolved in anhydrous THF (2 mL), and the mixture was purged with N₂ three times. The reaction mixture was cooled to -78°C under N₂ protection using an ethanol/dry ice bath. Then, 1M HMDSLi in THF (0.6 mL, 0.6 mmol) was added dropwise and stirred at -40°C for 1.5 hours. The reaction mixture was then cooled to -78°C, and ethyl 2-bromoacetate (127.3 mg, 0.8 mmol) was added dropwise. The mixture was then stirred at -78°C for 2 hours. After the reaction was monitored to be complete by LCMS, the reaction mixture was diluted with ice-water (20 mL) and then with EtOAc (20 mL). The organic phase was then washed with water (20 mL) and brine (20 mL), then dried over Na₂SO₄, concentrated under vacuum, and filtered to give Compound 9 as a yellow solid (80.0 mg, 60%), ESI MS [M + H]⁺ for C₁₇H₁₆O₄S₂, calcd 349.4, found 349.0.

### Step (IX): Synthesis of 4-(5-methoxybenzo[1,2-b:4,3-b']dithiophen-2-yl)-4-oxobutanoic acid (Example Compound 30)

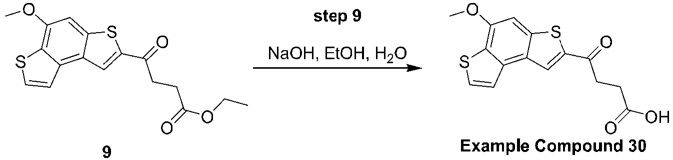

Ethyl 4-(5-methoxybenzo[1,2-b:4,3-b']dithiophen-2-yl)-4-oxobutanoate (80 mg, 0.23 mmol) was dissolved in H₂O (3 mL), ethanol (3 mL), and NaOH (27.6 mg, 0.7 mmol). The reaction mixture was stirred at room temperature for 2 hours. After the reaction was monitored to be complete by LCMS, the reaction mixture was adjusted to pH = 5 and concentrated under vacuum to give a crude sample. The crude product was further purified by reverse-phase HPLC (C18 column, 30-60% gradient of MeCN and water containing 0.1% FA) to give the product as a white solid (24 mg, 33%), ¹H NMR (400 MHz, Acetonitrile-*d₃*) δ 8.52 (s, 1H), 7.85 (d, *J =* 5.3 Hz, 1H), 7.78 (d, *J =* 5.3 Hz, 1H), 7.40 (s, 1H), 4.07 (s, 3H), 3.37 (t, *J =* 6.5 Hz, 2H), 2.72 (t, *J =* 6.5 Hz, 2H). ESI MS [M + H]⁺ for C₁₅H₁₂O₄S₂, calcd 321.4, found 320.9.

### Examples 31

### Chiral separation of 4-(4-methoxy-2-methylthieno[3,2-e]benzofuran-7-yl)-2-methyl-4-oxobutanoic acid (Example Compound 31)

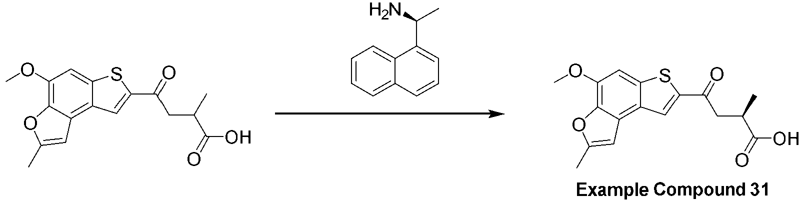

4-(4-Methoxy-2-methylthieno[3,2-e]benzofuran-7-yl)-2-methyl-4-oxobutanoic acid (2.8 g, 8.4 mmol) was added to a reaction flask containing ethyl acetate (420 mL, 100.0%), and then 1-naphthylethan-1-amine (1.5 g, 8.7 mmol) was added. The mixture was heated to 75°C until completely dissolved, followed by slow gradient cooling to room temperature, and crystallized for 12 h. White crystals precipitated. The crystals were filtered by suction and dried to give the product (R)-4-(4-methoxy-2-methylthieno[3,2-e]benzofuran-7-yl)-2-methyl-4-oxobutanoic acid (2 g, 6.0 mmol, 71%) with a purity of 89%.(R)-4-(4-Methoxy-2-methylthieno[3,2-e]benzofuran-7-yl)-2-methyl-4-oxobutanoic acid (2 g, 6.0 mmol, 71%) with a purity of 89% was added to a reaction flask containing ethyl acetate (200 mL, 100.0%). The mixture was heated to 75°C until completely dissolved, followed by slow gradient cooling to room temperature, and crystallized for 12 h. White crystals precipitated. The crystals were filtered by suction and dried. An appropriate amount of EA was then added until completely dissolved, and 2 mol of dilute hydrochloric acid was used to free the amine to give the product (R)-4-(4-methoxy-2-methylthieno[3,2-e]benzofuran-7-yl)-2-methyl-4-oxobutanoic acid (0.6 g, 1.8mmol, 30%) with a purity of 97%. ESI MS[M + H]⁺ for C₁₇H₁₆O₅S, calcd 332.4, found 332.97, ¹H NMR (400 MHz, DMSO-d6) δ 12.21 (s, 1H), 8.59 (s, 1H), 7.50 (s, 1H), 7.02 (s, 1H), 4.00 (s, 3H), 3.45 (dd, J = 17.4, 8.5 Hz, 2H), 3.13 (dd, J = 17.4, 5.3 Hz, 1H), 2.98 - 2.87 (m, 1H), 2.53 (s, 3H), 1.20 (d, J = 7.2 Hz, 3H).

### Example 32

### Chiral separation of 4-(4-methoxy-2-methylthieno[3,2-e]benzofuran-7-yl)-2-methyl-4-oxobutanoic acid (Example Compound 32)

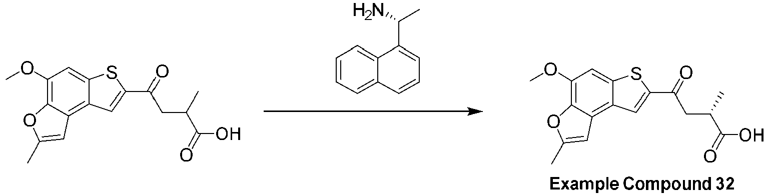

4-(4-Methoxy-2-methylthieno[3,2-e]benzofuran-7-yl)-2-methyl-4-oxobutanoic acid (5 g, 15 mmol) was added to a reaction flask containing ethyl acetate (400 mL), and then 1-naphthylethan-1-amine (2.58 g, 15.04 mmol) was added. The mixture was heated to 75°C until completely dissolved, followed by slow gradient cooling to room temperature, and crystallized for 12 h. White crystals precipitated. The crystals were filtered by suction and dried to give the product (S)-4-(4-methoxy-2-methylthieno[3,2-e]benzofuran-7-yl)-2-methyl-4-oxobutanoic acid (850 mg, 2.56 mmol, 17%) with a purity of 87%. (S)-4-(4-Methoxy-2-methylthieno[3,2-e]benzofuran-7-yl)-2-methyl-4-oxobutanoic acid (850 mg, 2.56 mmol, 17%) with a purity of 87% was added to a reaction flask, and ethyl acetate (80 mL) was added. The mixture was heated to 75°C until completely dissolved, followed by slow gradient cooling to room temperature, and crystallized for 12 h. After completion of the reaction, white crystals precipitated. The crystals were filtered by suction and dried. An appropriate amount of EA was then added until completely dissolved, and 2 mol of dilute hydrochloric acid was used to free the amine to give the product (S)-4-(4-methoxy-2-methylthieno[3,2-e]benzofuran-7-yl)-2-methyl-4-oxobutanoic acid (200 mg, 0.6 mmol, 23.5%) with a purity of 99%. ESI MS[M + H]⁺ for C₁₇H₁₆O₅S, calcd 332.4, found 332.98, ¹H NMR (400 MHz, DMSO-d6) δ 12.21 (s, 1H), 8.59 (s, 1H), 7.50 (s, 1H), 7.02 (s, 1H), 4.00 (s, 3H), 3.45 (dd, J = 17.3, 8.5 Hz, 1H), 3.13 (dd, J = 17.4, 5.3 Hz, 1H), 2.97 - 2.87 (m, 1H), 2.52 (s, 3H), 1.20 (d, J = 7.2 Hz, 3H).

### Example 33

### Synthesis of 4-(4-methoxy-2-methylthiazolo[5',4':5,6]benzo[1,2-d]oxazol-7-yl)-2-methyl-4-oxobutanoic acid (Example Compound 33)

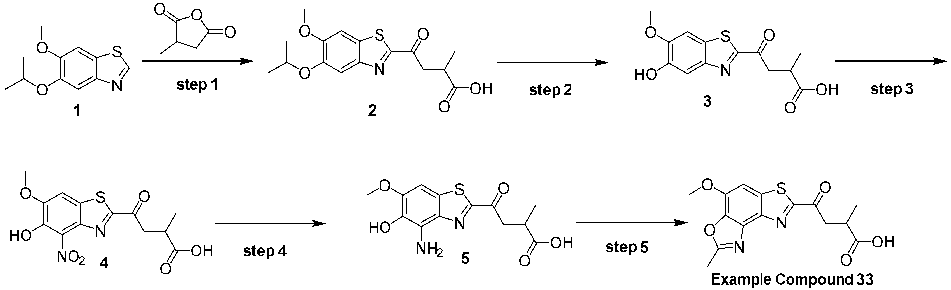

### Step (I): Synthesis of 4-(5-isopropoxy-6-methoxybenzo[d]thiazol-2-yl)-2-methyl-4-oxobutanoic acid (Intermediate 2)

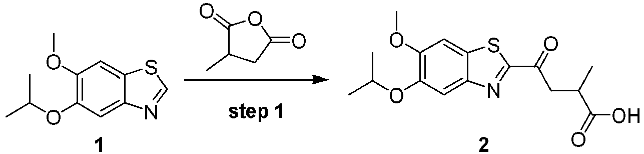

5-Isopropoxy-6-methoxybenzo[*d*]thiazole (3 g, 13.4 mmol) was dissolved in THF (60 mL). The mixture was purged with nitrogen three times and cooled to -78°C. A solution of LDA (7.2 g, 67.2 mmol) in THF was then added dropwise and stirred at -78°C for 0.5 h. Methylsuccinic anhydride (4.6 g, 40.3 mmol) was dissolved in THF (10 mL) and added dropwise to the reaction solution. After addition, the mixture was stirred at -78°C for 0.5 h. After completion of the reaction, the reaction was quenched by the dropwise addition of water (500 mL), and the reaction solution was adjusted to pH = 5 with aqueous hydrochloric acid solution. The mixture was extracted with EA, and the layers were separated. The organic phase was washed twice with water (500 mL), washed with saturated brine (500 mL), dried over Na₂SO₄, and filtered. The filtrate was concentrated under reduced pressure to give a crude product. The crude product was further purified by column chromatography (SiO₂, EA/petroleum ether = 3/1) to give Intermediate 2 (1.4 g, 31%) as a yellow solid. ESI MS[M + H]⁺ for C₁₆H₁₉NO₅S, calcd 338.4, found 338.0.

### Step (II): Synthesis of 4-(5-hydroxy-6-methoxybenzo[d]thiazol-2-yl)-2-methyl-4-oxobutanoic acid (Intermediate 3)

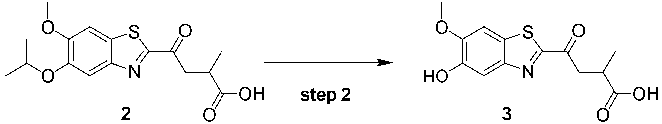

4-(5-Isopropoxy-6-methoxybenzo[*d*]thiazol-2-yl)-2-methyl-4-oxobutanoic acid (1.4 g, 4.2 mmol) was dissolved in DCM (14 mL). The mixture was purged with nitrogen three times and cooled to 0°C. Titanium tetrachloride (3.9 g, 20.8 mmol) was then added dropwise and stirred at room temperature overnight. After completion of the reaction, the reaction solution was poured into ice water (50 mL) to quench the reaction. The mixture was extracted with DCM (50 mL), and the layers were separated. The aqueous phase was extracted twice with DCM (50 mL). The organic phases were combined, dried over Na₂SO₄, and filtered. The filtrate was concentrated under reduced pressure to give a crude product. The crude product was further purified by column chromatography (SiO₂, EA/petroleum ether = 1/1) to give Intermediate 3 (900 mg, 73%) as a yellow solid. ESI MS[M + H]⁺ for C₁₃H₁₃NO₅S, calcd 296.3, found 295.9.

### Step (III): Synthesis of 4-(5-hydroxy-6-methoxy-4-nitrobenzo[d]thiazol-2-yl)-2-methyl-4-oxobutanoic acid (Intermediate 4)

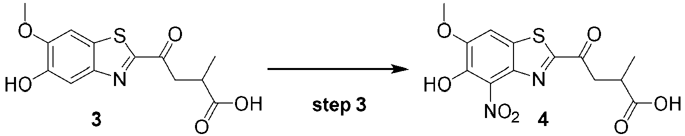

4-(5-Hydroxy-6-methoxybenzo[d]thiazol-2-yl)-2-methyl-4-oxobutanoic acid (800 mg, 2.7 mmol) was dissolved in EA (20 mL), and nitric acid (512 mg, 8.1 mmol) was added dropwise. The mixture was stirred at room temperature for 2 hours. After the reaction was monitored to be complete by LCMS, the reaction solution was diluted with water (50 mL) and extracted with EtOAc (50 mL). The organic phase was washed twice with water (50 mL) and washed with saturated aqueous sodium chloride solution (50 mL). The organic phase was collected and filtered over Na₂SO₄. The filtrate was concentrated under reduced pressure to give crude Intermediate 4 as a brown solid (800 mg, 87%), which was used directly in the next step, ESI MS[M + H]⁺ for C₁₃H₁₂N₂O₇S, calcd 341.3, found 341.0.

### Step (IV): Synthesis of 4-(4-amino-5-hydroxy-6-methoxybenzo[d]thiazol-2-yl)-2-methyl-4-oxobutanoic acid (Intermediate 5)

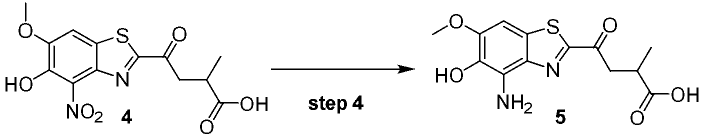

4-(5-Hydroxy-6-methoxy-4-nitrobenzo[d]thiazol-2-yl)-2-methyl-4-oxobutanoic acid (800 mg, 2.35 mmol) was dissolved in EA (20 mL), and palladium on carbon (10%, 80 mg, 0.08 mmol) was added. The mixture was purged with hydrogen three times and stirred at room temperature for 2 hours. After the reaction was monitored to be complete by LCMS, the reaction solution was filtered by suction under reduced pressure. The filter cake was washed with a small amount of EA, and the filtrate was concentrated to dryness under reduced pressure to give crude Intermediate 9 as a brown solid (620 mg, 85%), which was used directly in the next step, ESI MS[M + H]⁺ for C₁₃H₁₄N₂O₅S, calcd 311.3, found 311.0.

### Step (V): Synthesis of 4-(4-methoxy-2-methylthiazolo[5',4':5,6]benzo[1,2-d]oxazol-7-yl)-2-methyl-4-oxobutanoic acid (Example Compound 33)

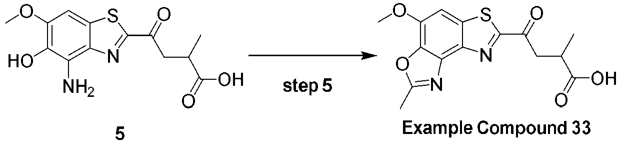

4-(4-Amino-5-hydroxy-6-methoxybenzo[d]thiazol-2-yl)-2-methyl-4-oxobutanoic acid (620 mg, 2.0 mmol) was dissolved in methanol (10 mL), and triethyl orthoacetate (1.9 g, 12.0 mmol) was added. The mixture was purged with nitrogen three times. The reaction solution was warmed, refluxed, and stirred overnight. After completion of the reaction, the reaction mixture was concentrated under vacuum to give a crude sample. The crude product was further purified by reverse-phase HPLC (C18 column, 50-60% gradient of MeCN and water containing 0.1% TFA) to give the product as a white solid (100 mg, 15%), ESI MS [M + H]⁺ for C₁₅H₁₄N₂O₅S, calcd 335.4, found 335.0, ¹H NMR (400 MHz, DMSO-d6) δ 12.27 (s, 1H), 7.86 (s, 1H), 4.06 (s, 3H), 3.66 (dd, J = 18.0, 8.8 Hz, 1H), 3.27 (dd, J = 18.0, 5.1 Hz, 1H), 3.00 (ddd, J = 8.8, 7.1, 5.1 Hz, 1H), 2.73 (s, 3H), 1.24 (d, J = 7.2 Hz, 3H).

### Example 34

### Synthesis of 4-(4-methoxy-2-methylthiazolo[5',4':5,6]benzo[1,2-d]oxazol-7-yl)-4-oxobutanoic acid (Example Compound 34)

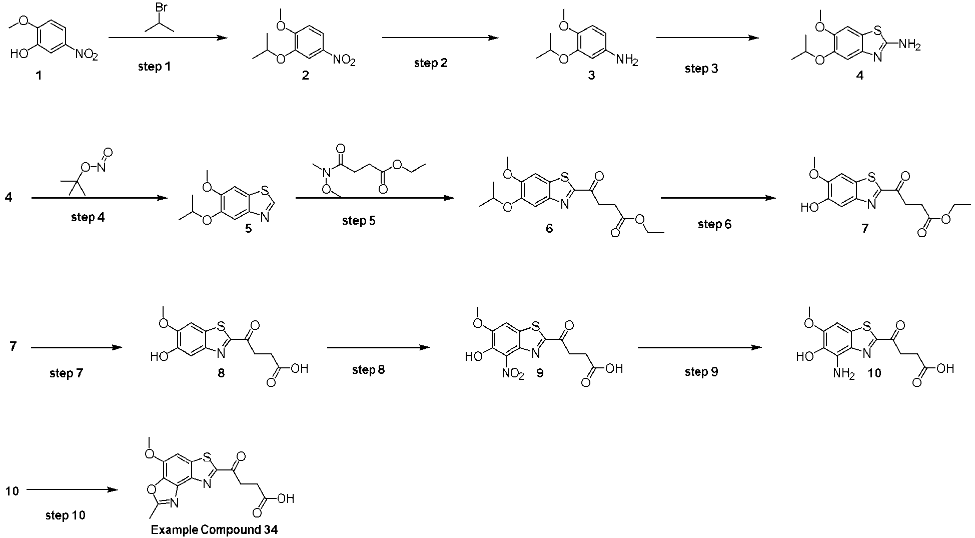

### Step (I): Synthesis of 2-isopropoxy-1-methoxy-4-nitrobenzene (Intermediate 2)

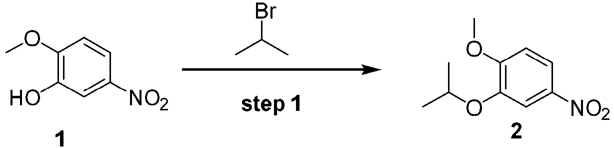

2-Methoxy-5-nitrophenol (20 g, 118.25 mmol) was dissolved in DMF (200 mL), and then bromoisopropane (30.15 g, 177.38 mmol) and potassium carbonate (40.03 g, 354.76 mmol) were added. The reaction mixture was then placed under a N₂ atmosphere and stirred at room temperature for 12 hours. After completion of the reaction, the reaction solution was diluted with ethyl acetate (1 L), washed twice with water (2 L) and twice with saturated sodium chloride solution (2 L), dried over Na₂SO₄, and filtered. The filtrate was concentrated under vacuum to give a crude product. The crude product was triturated with petroleum ether (200 mL) and then filtered. The filter cake was collected to give Compound 2 as a pale yellow solid (18.77 g, 75%): ¹H NMR (400 MHz, DMSO- d6) δ 7.90 (dd, *J =* 9.0, 2.7 Hz, 1H), 7.73 (d, *J =* 2.7 Hz, 1H), 7.18 (d, *J =* 9.0 Hz, 1H), 4.73 (p, *J =* 6.0 Hz, 1H), 3.90 (s, 3H), 1.30 (d, *J=* 6.0 Hz, 6H).

### Step (II): Synthesis of 3-isopropoxy-4-methoxyaniline (Intermediate 3)

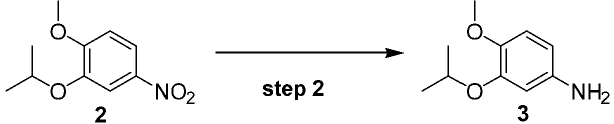

2-Isopropoxy-1-methoxy-4-nitrobenzene (18.77 g, 88.87 mmol) was dissolved in THF (180 mL), and then palladium on carbon (2 g, 18.79 mmol) was added. The reaction mixture was then sealed well, purged with H₂, and stirred at room temperature under H₂ protection for 3 hours. The reaction mixture was filtered. The filter cake was washed with methanol (100 mL) and then collected to give the crude product 3 as a brown oil (15.87 g, 98%): ¹H NMR (400 MHz, DMSO-*d* 6) δ 6.64 (d, *J =* 8.5 Hz, 1H), 6.26 (d, *J =* 2.6 Hz, 1H), 6.08 (dd, *J =* 8.5,2.6 Hz, 1H), 4.61 (s, 2H), 4.38 (p, *J =* 6.1 Hz, 1H), 3.60 (s, 3H), 1.23 (d, *J =* 6.1 Hz, 6H).

### Step (III): Synthesis of 5-isopropoxy-6-methoxybenzo[d]thiazol-2-amine (Intermediate 4)

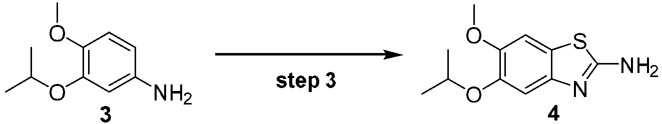

3-Isopropoxy-4-methoxyaniline (15.87 g, 87.57 mmol) was dissolved in acetic acid (150 mL), and then KSCN (18.72 g, 192.65 mmol) was added. The reaction mixture was then placed under a N₂ atmosphere, and Br₂ (13.99 g, 87.57 mmol) was added dropwise at 0°C. The reaction mixture was stirred at room temperature for 16 hours. The reaction solution was diluted with water (2 L). The reaction mixture was adjusted to pH = 8 with saturated sodium hydroxide solution and extracted with ethyl acetate (2 L). The organic phase was dried over Na₂SO₄ and filtered. The filtrate was concentrated under vacuum to give a crude sample. The crude product was further purified by column chromatography (SiO₂, petroleum ether:ethyl acetate = 1:1) to give the product 4 as a yellow solid (18.7 g, 78%): ¹H NMR (400 MHz, DMSO- *d*6) δ 7.28 (s, 1H), 7.17 (s, 2H), 6.94 (s, 1H), 4.48 (h, *J =* 6.0 Hz, 1H), 3.72 (s, 3H), 1.24 (d, *J =* 6.1 Hz, 6H).

### Step (IV): Synthesis of 5-isopropoxy-6-methoxybenzo[d]thiazole (Intermediate 5)

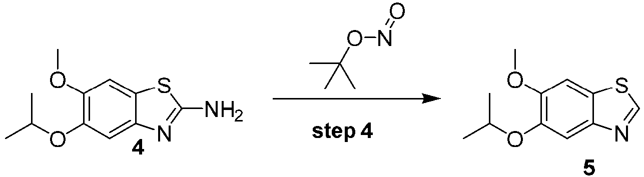

5-Isopropoxy-6-methoxybenzo[*d*]thiazol-2-amine (12.7 g, 53.29 mmol) was dissolved in THF (150 mL), and then tert-butyl nitrite (10.99 g, 106.58 mmol) was added. The reaction mixture was placed under a N₂ atmosphere and stirred at 70°C for 2 hours. The reaction mixture was cooled to room temperature, then diluted with ethyl acetate (1000 mL), and washed three times with water (500 mL). The organic phase was dried over Na₂SO₄ and filtered. The filtrate was concentrated under vacuum to give a crude sample. The crude product was further purified by column chromatography (SiO₂, petroleum ether:ethyl acetate = 20:1) to give the product 5 as a yellow solid (9.2 g, 77%): ¹H NMR (400 MHz, DMSO- d6) δ 9.15 (s, 1H), 7.69 (s, 1H), 7.61 (s, 1H), 4.68 (p, *J =* 6.0 Hz, 1H), 3.84 (s, 3H), 1.30 (d, *J =* 6.0 Hz, 6H).

### Step (V): Synthesis of ethyl 4-(5-isopropoxy-6-methoxybenzo[d]thiazol-2-yl)-4-oxobutanoate (Intermediate 6)

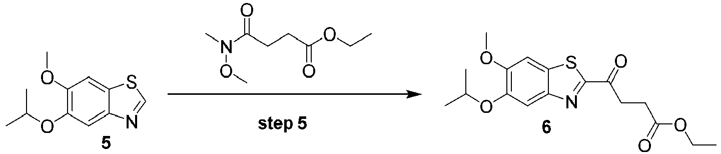

5-Isopropoxy-6-methoxybenzo[*d*]thiazole (4.0 g, 17.91 mmol) was dissolved in THF (40 mL), and LDA (9.59 g, 89.57 mmol) was added at -78°C under N₂ protection. The mixture was stirred under these conditions for 0.5 h, and then ethyl 4-(methoxy(methyl)amino)-4-oxobutanoate (10.51 g, 55.53 mmol) was added. The mixture was stirred at -78°C for 1 hour. The reaction mixture was quenched into ice-water (200 mL). The mixture was extracted with ethyl acetate (500 mL). The organic phase was washed three times with water (200 mL), dried over Na₂SO₄, and filtered. The filtrate was concentrated under vacuum to give a crude sample. The crude product was triturated with petroleum ether (100 mL) and then filtered. The filter cake was collected to give Compound 6 as a pale yellow solid (1.4 g, 22%): ¹H NMR (400 MHz, DMSO-*d*6) δ 7.76 (d, J = 1.9 Hz, 2H), 4.76 (p, *J =* 6.0 Hz, 1H), 4.06 (q, *J =* 7.1 Hz, 2H), 3.88 (s, 3H), 3.46 (t, *J =* 6.5 Hz, 2H), 2.72 (t, *J =* 6.5 Hz, 2H), 1.32 (d, *J =* 6.0 Hz, 6H), 1.17 (t, *J =* 7.1 Hz, 3H).

### Step (VI): Synthesis of ethyl 4-(5-hydroxy-6-methoxybenzo[d]thiazol-2-yl)-4-oxobutanoate (Compound 7)

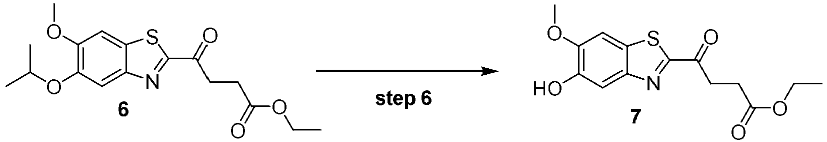

Ethyl 4-(5-isopropoxy-6-methoxybenzo[*d*]thiazol-2-yl)-4-oxobutanoate (720 mg, 2.05 mmol) was dissolved in dichloromethane (8 mL), and titanium tetrachloride (1.94 g, 10.24 mmol) was added at 0°C under N₂ protection. The reaction mixture was stirred at room temperature for 48 hours. The reaction mixture was quenched into ice-water (100 mL). The mixture was extracted with dichloromethane (200 mL). The organic phase was washed twice with water (100 mL), dried over Na₂SO₄, and filtered. The filtrate was concentrated under vacuum to give a crude sample. The crude product was further purified by column chromatography (SiO₂, petroleum ether:ethyl acetate = 2:1) to give the product 7 as a pale yellow solid (530 mg, 83%): ESI MS [M + H]⁺ for C₁₄H₁₅NO₅S, calcd 310.3, found 310.0.

### Step (VII): Synthesis of 4-(5-hydroxy-6-methoxybenzo[d]thiazol-2-yl)-4-oxobutanoic acid (Intermediate 8)

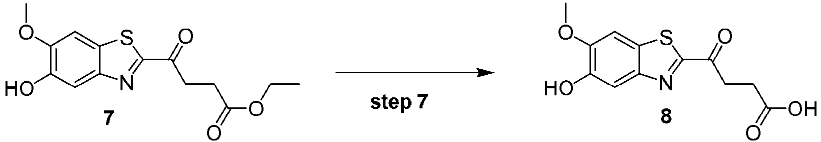

Ethyl 4-(5-hydroxy-6-methoxybenzo[*d*]thiazol-2-yl)-4-oxobutanoate (330 mg, 1.07 mmol) was dissolved in MeOH (5 mL) and H₂O (1 mL), and then sodium hydroxide (214 mg, 5.35 mmol) was added. The reaction mixture was then stirred at room temperature for ten minutes. The reaction mixture was adjusted to pH = 5 with hydrochloric acid and diluted with water (200 mL). The mixture was extracted with EtOAc (200 mL). The organic phase was then washed with water (200 mL) and brine (200 mL). The organic phase was collected, dried over Na₂SO₄, and concentrated under vacuum to give the product 8 as a yellow solid (280 mg, 93%): ESI MS [M + H]⁺ for C₁₂H₁₁NO₅S, calcd 282.2, found 282.0.

### Step (VIII): Synthesis of 4-(5-hydroxy-6-methoxy-4-nitrobenzo[d]thiazol-2-yl)-4-oxobutanoic acid (Intermediate 9)

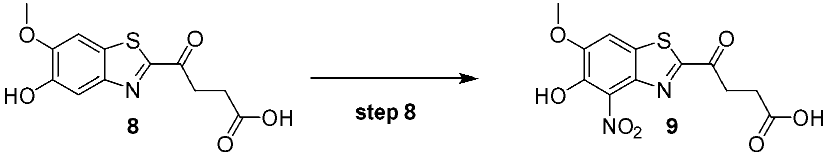

4-(5-Hydroxy-6-methoxybenzo[d]thiazol-2-yl)-4-oxobutanoic acid (110 mg, 0.39 mmol) was dissolved in acetic acid (2 mL), and nitric acid (39.96 mg, 0.59 mmol) was added dropwise. The reaction mixture was stirred at room temperature for 1 hour. The reaction mixture was quenched by the addition of water (50 mL) and extracted with ethyl acetate (150 mL). The organic phase was washed with water (100 mL) and brine (100 mL), dried over Na₂SO₄, and filtered. The filtrate was concentrated under vacuum to give the crude product 9 as a brown solid (80 mg, 62%): ESI MS [M + H]⁺ for C₁₂H₁₀N₂O₇S, calcd 327.2, found 326.9.

### Step (IX): Synthesis of 4-(4-amino-5-hydroxy-6-methoxybenzo[d]thiazol-2-yl)-4-oxobutanoic acid (Intermediate 10)

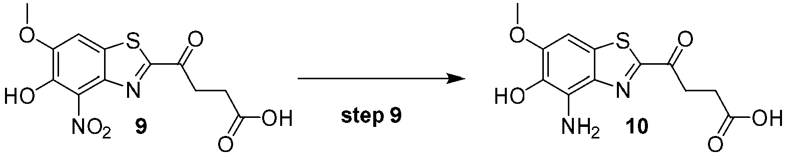

4-(5-Hydroxy-6-methoxy-4-nitrobenzo[*d*]thiazol-2-yl)-4-oxobutanoic acid (80 mg, 0.25 mmol) was dissolved in ethyl acetate (20 mL). Palladium on carbon (10 mg, 0.01 mmol) was then added. The reaction mixture was purged with H₂ and stirred at room temperature for 2 hours. After completion of the reaction, the reaction solution was filtered by suction under reduced pressure. The filtrate was concentrated under vacuum to give the crude product 10 as a brown solid (60 mg, 82%): ESI MS [M + H]⁺ for C₁₂H₁₀N₂O₇S, calcd 311.33, found 311.09.

### Step (X): Synthesis of 4-(4-methoxy-2-methylthiazolo[5',4':5,6]benzo[1,2-d]oxazol-7-yl)-4-oxobutanoic acid (Example Compound 34)

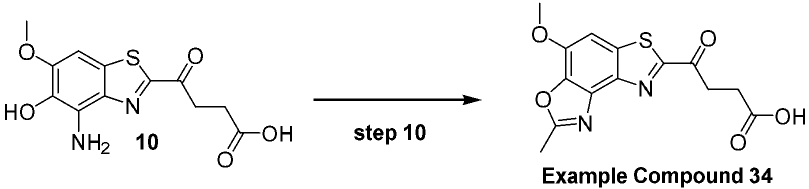

4-(4-Amino-5-hydroxy-6-methoxybenzo[*d*]thiazol-2-yl)-4-oxobutanoic acid (60 mg, 0.2 mmol) was dissolved in methanol (20 mL), and then 1,1,1-triethoxyethane (197.11 mg, 1.21 mmol) was added. The mixture was purged with N₂ and stirred at 70°C overnight. The reaction mixture was cooled to room temperature, concentrated under reduced pressure, and purified by prep-HPLC (column: Hanbang DAC-50, fillers: NanoMicro UniHybrid 10-120 C18, mobile phase: 0.1% FA water and ACN, eluent: 30-60% ACN) to give the product 1 as a white solid (4.3 mg, 6%): ¹H NMR (400 MHz, DMSO- *d*₆) δ 7.87 (s, 1H), 4.06 (s, 3H), 3.48 (t, *J =* 6.5 Hz, 2H), 2.73 (s, 3H), 2.68 (t, *J =* 6.4 Hz, 2H). ESI MS [M + H]⁺ for C₁₄H₁₂N₂O₅S, calcd 321.3, found 321.1.

### Example 35

### Synthesis of 4-(4-methoxy-2-methylthiazolo[5',4':5,6]benzo[1,2-d]oxazol-7-yl)-3-methyl-4-oxobutanoic acid (Example Compound 35)

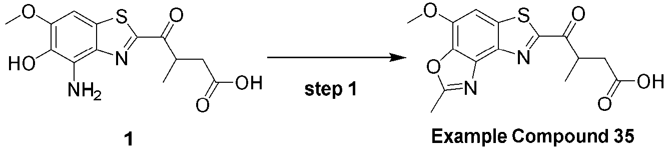

### Step (I): Synthesis of 4-(4-methoxy-2-methylthiazolo[5',4':5,6]benzo[1,2-d]oxazol-7-yl)-3-methyl-4-oxobutanoic acid (Example Compound 35)

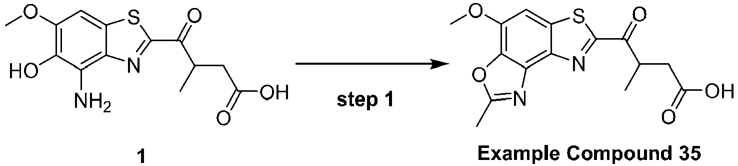

4-(4-Amino-5-hydroxy-6-methoxybenzo[*d*]thiazol-2-yl)-3-methyl-4-oxobutanoic acid (620 mg, 2.0 mmol) was dissolved in methanol (10 mL), and triethyl orthoacetate (1.9 g, 12.0 mmol) was added. The mixture was purged with nitrogen three times. The reaction solution was warmed, refluxed, and stirred overnight. After completion of the reaction, the reaction mixture was concentrated under vacuum to give a crude sample. The crude product was further purified by reverse-phase HPLC (C18 column, 50-60% gradient of MeCN and water containing 0.1% TFA) to give the product as a white solid (17 mg, 2.5%), ESI MS [M + H]⁺ for C₁₅H₁₄N₂O₅S, calcd 335.4, found 335.0; ¹H NMR (400 MHz, DMSO-d6) δ 12.28 (s, 1H), 7.86 (s, 1H), 4.17 (ddd, *J =* 9.3, 7.3, 5.4 Hz, 1H), 4.06 (s, 3H), 2.84 (dd, *J =* 17.0, 9.3 Hz, 1H), 2.73 (s, 3H), 2.61 (dd, *J* = 17.1, 5.3 Hz, 1H), 1.26(d, *J* = 7.1 Hz, 3H).

### Example 36

### Synthesis of (R)-2-ethyl-4-(4-methoxy-2-methylthieno[3,2-e]benzofuran-7-yl)-4-oxobutanoic acid (Example Compound 36)

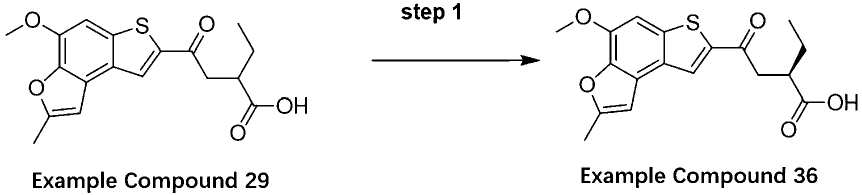

### Step (I): Synthesis of (R)-2-ethyl-4-(4-methoxy-2-methylthieno[3,2-e]benzofuran-7-yl)-4-oxobutanoic acid (Example Compound 36)

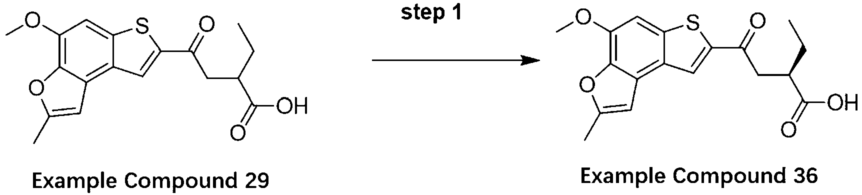

2-Ethyl-4-(4-methoxy-2-methylthieno[3,2-e]benzofuran-7-yl)-4-oxobutanoic acid (1 g, 2.9 mmol) was dissolved in EA (160 mL), and then (1S,2R)-2-amino-1,2-diphenylethan-1-ol (0.6 g, 2.9 mmol) was added. The mixture was warmed to 80°C for dissolution, then slowly cooled to room temperature, stirred overnight, and filtered. The filter cake was dried by rotary evaporation, and then 100v of EA was added. The mixture was warmed to 80°C for dissolution, then slowly cooled to room temperature, and stirred overnight. The reaction solution was filtered, and the filter cake was dried by rotary evaporation. Dilute hydrochloric acid (1M, 100 mL) and EA (160 mL) were then added and stirred for 0.5 h. The mixture was extracted, and the layers were separated. Dilute hydrochloric acid (1M, 100 mL) was then added to the organic phase and stirred for 0.5 h. The mixture was extracted, and the layers were separated. The organic phase was washed once with clean water (100 mL), then dried by rotary evaporation, and purified by prep-HPLC (column: Hanbang DAC-50, fillers: NanoMicro UniHybrid 10-120 C18, mobile phase: 0.1% FA water and ACN, eluent: 30-50% ACN) to give the pure product (97.0 mg, 10%), ESI MS[M + H ]⁺ for C₁₈H₁₈O₅S, calcd 347.4, found 347.1. ¹H NMR (400 MHz, DMSO-d6) δ 12.21 (s, 1H), 8.62 (s, 1H), 7.51 (s, 1H), 7.04 (d, J = 1.5 Hz, 1H), 4.01 (s, 3H), 3.52 - 3.40 (m, 1H), 3.14 (dd,J = 17.4, 4.6 Hz, 1H), 2.83 (dtd, J = 9.5, 6.5, 4.5 Hz, 1H), 2.57 - 2.53 (m, 3H), 1.63 (ddt, J = 13.6, 10.3, 6.8 Hz, 2H), 0.95 (t, J = 7.4 Hz, 3H).

### Example 37

### Synthesis of (S)-2-ethyl-4-(4-methoxy-2-methylthieno[3,2-e]benzofuran-7-yl)-4-oxobutanoic acid (Example Compound 37)

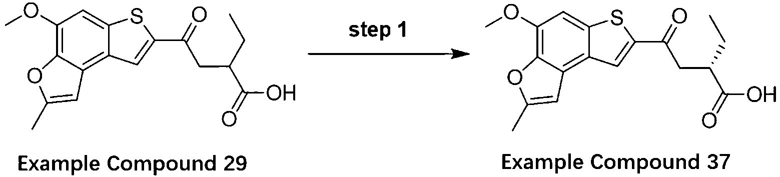

2-Ethyl-4-(4-methoxy-2-methylthieno[3,2-e]benzofuran-7-yl)-4-oxobutanoic acid (2.5 g, 7.2 mmol) was dissolved in EA (400 mL), and then (1S,2R)-2-amino-1,2-diphenylethan-1-ol (1.54 g, 7.2 mmol) was added. The mixture was warmed to 80°C for dissolution, then slowly cooled to room temperature, stirred overnight, and then filtered. The filter cake was dried by rotary evaporation, and then 100v of EA was added for dissolution. The mixture was warmed to 80°C for dissolution, then slowly cooled to room temperature, and stirred overnight. The reaction solution was filtered, and the filter cake was dried by rotary evaporation. Dilute hydrochloric acid (1M, 100 mL) and EA (160 mL) were then added and stirred for 0.5 h. The mixture was extracted, and the layers were separated. Dilute hydrochloric acid (1M, 100 mL) was then added to the organic phase and stirred for 0.5 h. The mixture was extracted, and the layers were separated. The organic phase was washed once with clean water (100 mL), then dried by rotary evaporation, and triturated with 10 mL of mixed solvent (PE:EA = 10:1) to give the product (430 mg, 17%), ESI MS[M + H ]⁺ for C₁₈H₁₉O₅S, calcd 347.4, found 347.1. ¹H NMR (400 MHz, Chloroform-d) δ 8.08 (s, 1H), 7.11 (s, 1H), 6.67 (d, J = 1.2 Hz, 1H), 4.07 (s, 3H), 3.48 (dd, J = 16.7, 8.0 Hz, 1H), 3.22 - 3.02 (m, 2H), 2.55 (d, J = 1.0 Hz, 3H), 1.89 - 1.66 (m, 2H), 1.04 (t, J = 7.4 Hz, 3H).

### Example 38

### Synthesis of 4-(4-methoxy-2-methylthiazolo[5',4':5,6]benzo[1,2-d]oxazol-7-yl)-2-methyl-4-oxobutanoic acid (Example Compound 38)

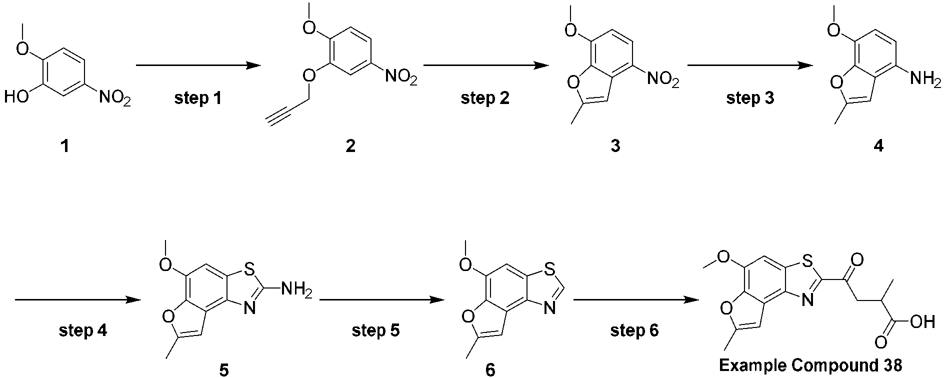

### Step (I): Synthesis of 1-methoxy-4-nitro-2-(prop-2-yn-1-yloxy)benzene (Intermediate 1)

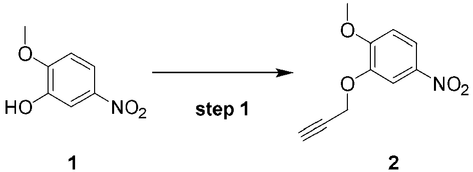

2-Methoxy-5-nitrophenol (7.8 g, 46.1 mmol) was dissolved in acetone (78 mL), and potassium carbonate (9.6 g, 46.1 mmol) and propargyl bromide (8.2 g, 46.1 mmol) were added. The mixture was purged with nitrogen three times, warmed to 60°C, and stirred for 4 h. After completion of the reaction, the reaction solution was cooled to room temperature and poured into ice water (500 mL) to quench the reaction. The mixture was extracted with EA (500 mL), and the layers were separated. The organic phase was washed twice with water (500 mL), washed with saturated brine (500 mL), dried over Na₂SO₄, and filtered. The filtrate was concentrated under reduced pressure to give Intermediate 2 (8 g, 84%) as a pale yellow solid. ¹H NMR (400 MHz, Chloroform-d) δ 7.97 (dd, J = 9.0, 2.6 Hz, 1H), 7.92 (d, J = 2.6 Hz, 1H), 6.95 (d, J = 9.0 Hz, 1H), 4.85 (d, J = 2.4 Hz, 2H), 3.98 (s, 3H), 2.58 (t, J = 2.4 Hz, 1H).

### Step (II): Synthesis of 7-methoxy-2-methyl-4-nitrobenzofuran (Intermediate 2)

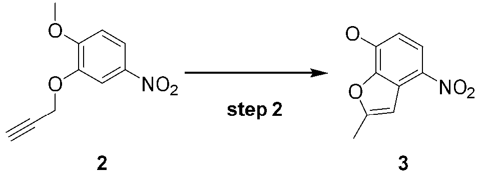

1-Methoxy-4-nitro-2-(prop-2-yn-1-yloxy)benzene (7 g, 33.8 mmol) was dissolved in N,N-diethylaniline (70 mL), and cesium fluoride (10.3 g, 67.6 mmol) was added. The mixture was purged with nitrogen three times, warmed to 200°C, and stirred for 4 h. After completion of the reaction, the reaction solution was poured into ice water (500 mL) to quench the reaction and adjusted to pH = 6 with concentrated hydrochloric acid. The mixture was extracted with EA (500 mL), and the layers were separated. The organic phase was washed twice with water (500 mL), washed with saturated brine (500 mL), dried over Na₂SO₄, and filtered. The filtrate was concentrated under reduced pressure to give a crude product. The crude product was further purified by column chromatography (SiO₂, EA/petroleum ether = 20/1) to give Intermediate 3 (1.9 g, 27%) as a white solid. ¹H NMR (400 MHz, Chloroform-d) δ 8.17 (d, J = 9.0 Hz, 1H), 7.14 (s, 1H), 6.78 (d, J = 9.0 Hz, 1H), 4.11 (s, 3H), 2.55 (s, 3H).

### Step (III): Synthesis of 7-methoxy-2-methylbenzofuran-4-amine (Intermediate 3)

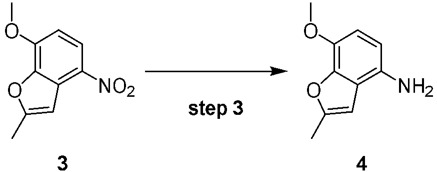

7-Methoxy-2-methyl-4-nitrobenzofuran (2.2 g, 10.6 mmol) was dissolved in EA (22 mL), and palladium on carbon (10%, 220 mg, 0.21 mmol) was added. The mixture was purged with hydrogen three times and stirred at room temperature for 2 hours. After the reaction was monitored to be complete by LCMS, the reaction solution was filtered by suction under reduced pressure. The filter cake was washed with a small amount of EA, and the filtrate was concentrated to dryness under reduced pressure to give crude Intermediate 4 as a brown solid (1.5 g, 80%), which was used directly in the next step, ESI MS[M + H]⁺ for C₁₀H₁₁NO₂, calcd 178.2, found 178.0.

### Step (IV): Synthesis of 5-methoxy-7-methylbenzofuro[4,5-d]thiazol-2-amine (Intermediate 5)

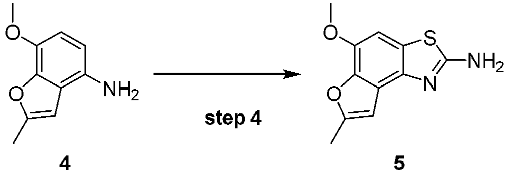

7-Methoxy-2-methylbenzofuran-4-amine (1.5 g, 8.5 mmol) was dissolved in acetic acid (30 mL), and potassium thiocyanate (1.8 g, 18.6 mmol) was added. The mixture was purged with nitrogen three times and cooled to 0°C. Bromine (1.4 g, 8.5 mmol) was added dropwise and stirred at room temperature overnight. After the reaction was monitored to be complete by LCMS, the reaction solution was poured into ice water to quench and adjusted to pH = 8 with aqueous sodium hydroxide solution. The mixture was extracted with EA (200 mL), and the layers were separated. The organic phase was washed twice with water (200 mL), washed with saturated brine (200 mL), dried over Na₂SO₄, and filtered. The filtrate was concentrated under reduced pressure to give a crude product. The crude product was further purified by column chromatography (SiO₂, EA/petroleum ether = 3/1) to give Intermediate 5 (1.8 g, 91%) as a yellow solid. ESI MS[M + H]⁺ for C₁₁H₁₀N₂O₂S, calcd 235.3, found 235.1.

### Step (V): Synthesis of 5-methoxy-7-methylbenzofuro[4,5-d]thiazol-2-amine (Intermediate 5)

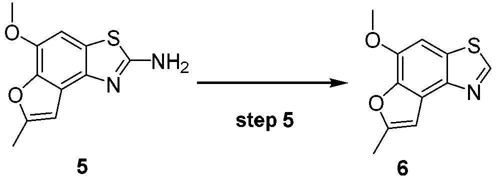

5-Methoxy-7-methylbenzofuro[4,5-*d*]thiazol-2-amine (1.8 g, 7.7 mmol) was dissolved in THF (20 mL), and tert-butyl nitrite (1.6 g, 15.4 mmol) was added. The mixture was purged with nitrogen three times, warmed to 70°C, and stirred for 2 h. After the reaction was monitored to be complete by LCMS, the reaction solution was cooled to room temperature, poured into ice water (200 mL) to quench, and adjusted to pH = 8 with aqueous sodium hydroxide solution. The mixture was extracted with EA (200 mL), and the layers were separated. The organic phase was washed twice with water (200 mL), washed with saturated brine (200 mL), dried over Na₂SO₄, and filtered. The filtrate was concentrated under reduced pressure to give a crude product. The crude product was further purified by column chromatography (SiO₂, EA/petroleum ether = 10/1) to give Intermediate 6 (1.1 g, 65%) as a yellow solid. ESI MS[M + H]⁺ for C₁₁H₉NO₂S, calcd 220.3, found 220.0.

### Step (VI): Synthesis of 4-(5-methoxy-7-methylbenzofuran[4,5-d]thiazol-2-yl)-2-methyl-4-oxobutanoic acid (Example Compound 38)

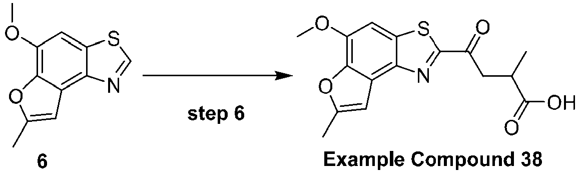

5-Methoxy-7-methylbenzofuro[4,5-d]thiazole (200 mg, 0.91 mmol) was dissolved in THF (10 mL). The mixture was purged with nitrogen three times and cooled to -78°C. A solution of LDA (2.4 g, 4.6 mmol) in THF was then added dropwise and stirred at -78°C for 0.5 h. Methylsuccinic anhydride (312 mg, 2.74 mmol) was dissolved in THF (2 mL) and added dropwise to the reaction solution. After addition, the mixture was stirred at -78°C for 0.5 h. After completion of the reaction, the reaction was quenched by the dropwise addition of water (50 mL), and the reaction solution was adjusted to pH = 5 with aqueous hydrochloric acid solution. The mixture was extracted with EA, and the layers were separated. The organic phase was washed twice with water (50 mL), washed with saturated brine (50 mL), dried over Na₂SO₄, and filtered. The filtrate was concentrated under reduced pressure to give a crude product. The crude product was further purified by reverse-phase HPLC (C18 column, 50-60% gradient of MeCN and 0.1% TFA containing water) to give the crude product as a yellow solid (17 mg, 5.6%), ESI MS [M + H]⁺ for C₁₆H₁₅NO₅S, calcd 334.4, found 334.0, ¹H NMR (400 MHz, DMSO-d6) δ 12.28 (s, 1H), 7.70 (d, *J* = 2.5 Hz, 1H), 7.11 (s, 1H), 4.03 (d, *J =* 1.3 Hz, 3H), 3.65 (dd, *J =* 18.1, 8.8 Hz, 1H), 3.26 (dd, *J =* 18.1, 5.1 Hz, 2H), 3.03 - 2.93 (m, 1H), 1.24 (dd, *J* = 7.2, 5.2 Hz, 3H).

### Example 39

### Synthesis of 4-oxo-4-(6H-thieno[2,3-e]indazol-2-yl)butanoic acid (Example Compound 39)

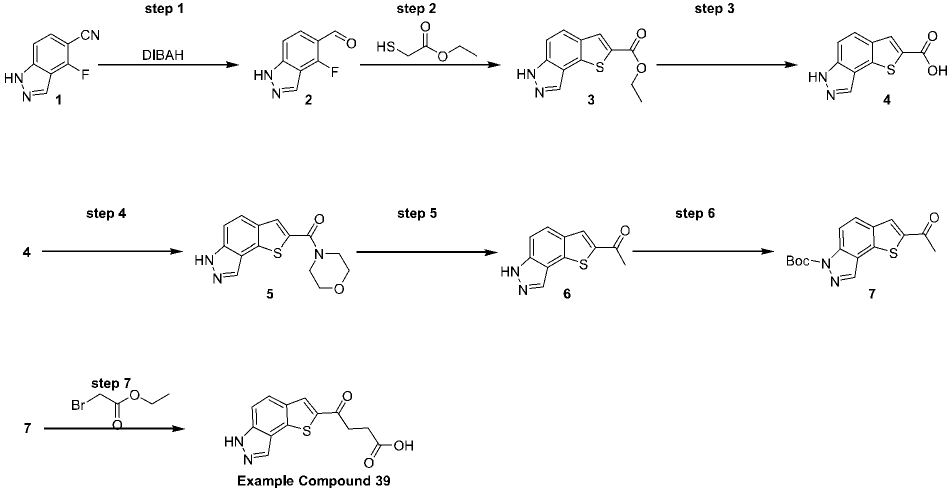

### Step (I): Synthesis of 4-fluoro-1H-indazole-5-carbaldehyde (Intermediate 2)

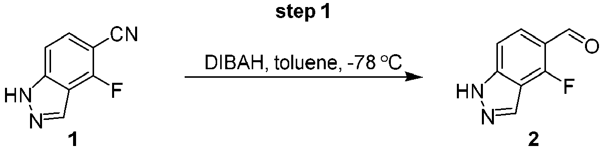

A mixture of 4-fluoro-1H-indazole-5-carbonitrile (749.3 mg, 4.7 mmol) and toluene (10 mL) was cooled to -78°C. Diisobutylaluminium hydride (661.3 mg, 4.7 mmol) was then added dropwise to the reaction mixture and stirred at -78°C under N₂ protection for 2 hours. LCMS at this point indicated that the starting material had disappeared. The reaction mixture was quenched with EtOAc (50 mL) and adjusted to pH = 6 with aqueous D (-)-tartaric acid solution (50 mL). The mixture was extracted twice with EtOAc (100 mL). The organic phase was concentrated under vacuum to give a yellow solid (820 mg, 100%): ESI MS [M + H]⁺ for C₈H₆FN₂O, calcd 165.0, found 165.1, which was used in the next step without further purification.

### Step (II): Synthesis of ethyl 6H-thieno[2,3-e]indazole-2-carboxylate (Intermediate 3)

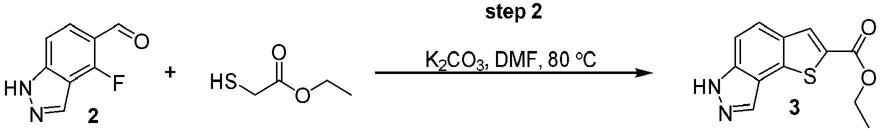

4-Fluoro-1H-indazole-5-carbaldehyde (820 mg, 5.0 mmol) was dissolved in DMF (20 mL), and then ethyl thioglycolate (3.0 g, 25.0 mmol) was added. The mixture was cooled to 0°C. K₂CO₃ (3.5 g, 25.0 mmol) was added to the mixture at 0°C. The reaction mixture was then sealed well and stirred at 80°C under N₂ protection for 3 hours. The reaction mixture was cooled to room temperature, quenched into ice-water (100 mL), and then diluted with EtOAc (100 mL). The resulting mixture was extracted with EtOAc (100 mL). The organic phase was then washed with water (100 mL) and brine (100 mL), dried over Na₂SO₄, and filtered. The filtrate was concentrated under vacuum to give a crude sample. The crude product was further purified by column chromatography (SiO₂, petroleum ether:EtOAc = 5:1) to give the title compound 3 as a white solid (480.0 mg, 39%): ESI MS [M + H]⁺ for C₁₂H₁₁N₂O₂S, calcd 247.1, found 247.1; ¹H NMR (400 MHz, DMSO-d6) δ 13.59 (s, 1H), 7.43 (s, 1H), 7.32 (s, 1H), 7.93-7.91 (d, *J =* 8.0, 1H), 7.65-7.63 (d, *J =* 8.0, 1H), 4.39-4.33 (q, *J =* 12.0 Hz, 2H), 1.37-1.34 (t, *J =* 12.0 Hz, 3H).

### Step (III): Synthesis of 6H-thieno[2,3-e]indazole-2-carboxylic acid (Intermediate 4)

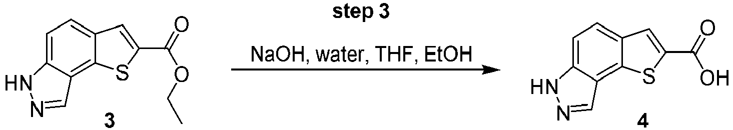

Ethyl 6H-thieno[2,3-e]indazole-2-carboxylate (480.0 mg, 2.0 mmol) was dissolved in a solution of water (2.5 mL), ethyl alcohol (2 mL), tetrahydrofuran (2.5 mL), and sodium hydroxide (779.6 mg, 19.5 mmol). The reaction mixture was stirred at 60°C for 0.5 h. LCMS indicated that the starting material had disappeared. The reaction mixture was adjusted to pH = 5-6 to give a white precipitate. The precipitate was filtered and dried under vacuum to give the product 4 as a white solid (350.0 mg, 82%): ESI MS [M + H]⁺ for C₁₀H₇N₂O₂S, calcd 219.0, found 219.0; ¹H NMR (400 MHz, DMSO-d6) δ 13.56 (b, 2H), 8.42 (s, 1H), 8.22 (s, 1H), 7.91-7.89 (d, *J=* 8.0 Hz, 1H), 7.64-7.62 (d, *J =* 8.0 Hz, 1H).

### Step (IV): Synthesis of morpholinyl (6H-thieno[2,3-e]indazol-2-yl)methanone (Intermediate 5)

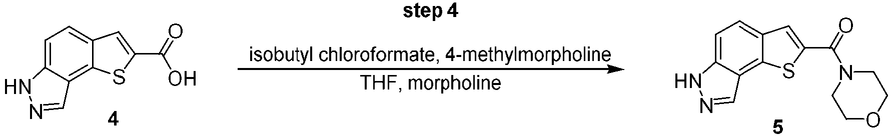

To a solution of 6H-thieno[2,3-e]indazole-2-carboxylic acid (50 mg, 0.23 mmol) in THF (3 mL) was added dropwise 4-methylmorpholine (25.5 mg, 0.3 mmol) and isobutyl chloroformate (65.7 mg, 0.5 mmol) at room temperature. The reaction mixture was then stirred at room temperature under N₂ protection for 10 minutes. Afterwards, a mixture of morpholine (199.6 mg, 2.3 mmol) was added dropwise and stirred at room temperature for 1 hour. LCMS at this point indicated no more starting material. The reaction mixture was quenched into water (50 g) and then diluted with EtOAc (50 mL). The resulting mixture was extracted with EtOAc (100 mL). The organic phase was then washed three times with water (50 mL), dried over Na₂SO₄, and filtered. The filtrate was concentrated under vacuum to give crude Compound 5 as a pale yellow solid (200 mg, 100%), ESI MS [M + H]⁺ for C₁₄H₁₄N₃O₂S, calcd 288.1, found 288.0.

### Step (V): Synthesis of 1-(6H-thieno[2,3-e]indazol-2-yl)ethan-1-one (Intermediate 6)

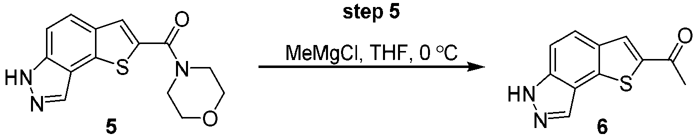

To a solution of morpholinyl (6H-thieno[2,3-e]indazol-2-yl)methanone was added dropwise 3.0M methylmagnesium chloride in THF (1.0 mL, 3.0 mmol) at 0°C. The reaction mixture was stirred at room temperature for 1 hour. LCMS at this point indicated no more starting material. The reaction mixture was quenched into water (50 g) and then diluted with EtOAc (50 mL). The resulting mixture was extracted with EtOAc (100 mL). The organic phase was then washed three times with water (50 mL), dried over Na₂SO₄, and filtered. The filtrate was concentrated under vacuum to give crude Compound 6 as a pale yellow solid (180 mg, 100%): ESI MS [M + H]⁺ for C₁₁H₉N₂OS, calcd 217.0, found 217.1.

### Step (VI): Synthesis of tert-butyl 2-acetyl-6H-thieno[2,3-e]indazole-6-carboxylate (Intermediate 7)

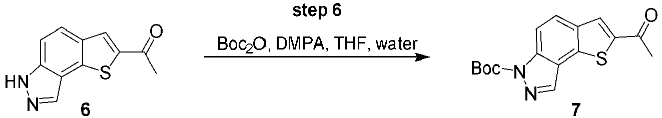

1-(6H-Thieno[2,3-e]indazol-2-yl)ethan-1-one (118.9 mg, 0.6 mmol) and DMPA (1.3 mg, 0.01 mmol) were dissolved in a solution of THF (5 mL), water (0.5 mL), and di-tert-butyl dicarbonate (144.0 mg, 0.7 mmol). The mixture was stirred at room temperature for 2 hours. The reaction mixture was poured into water (20 g) and then diluted with EtOAc (50 mL). The resulting mixture was extracted with EtOAc (50 mL). The organic phase was washed three times with water (50 mL), dried over Na₂SO₄, and filtered. The filtrate was concentrated under vacuum to give a crude sample. The crude product was further purified by column chromatography (SiO₂, petroleum ether:EtOAc = 10:1) to give the title compound 7 as a white solid (100 mg, 57%).

### Step (VII): Synthesis of 4-oxo-4-(6H-thieno[2,3-e]indazol-2-yl)butanoic acid (Example Compound 39)

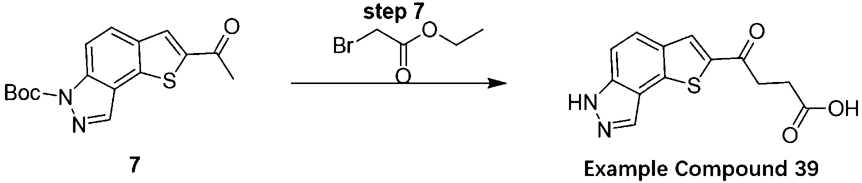

Tert-butyl 2-acetyl-6H-thieno[2,3-e]indazole-6-carboxylate (101.2 mg, 0.3 mmol) was dissolved in THF (4 mL), and then [bis(dimethylamino)phosphato]dimethylamine (200.7 mg, 1.1 mmol) was added. The mixture was purged with N₂ three times and cooled to -78°C. The reaction mixture was then added to 1M lithium (1+) bis(trimethylsilyl)amide in THF (0.6 mL, 0.6 mmol) and stirred at -78°C under N₂ protection for 0.5 h. Finally, ethyl 2-bromoacetate (106.9 mg, 0.6 mmol) was added. The reaction mixture was then sealed well and stirred at room temperature under N₂ protection for 2 h. LCMS indicated that the reaction was complete.

The reaction mixture was quenched with ice-water (100 g) and then diluted with EtOAc (100 mL). The resulting mixture was extracted with EtOAc (100 mL). The organic phase was dried over Na₂SO₄ and filtered. The filtrate was concentrated under vacuum to give a crude product. Aqueous NaOH solution (10 mL) and THF (5 mL) were added to the residue. The mixture was adjusted to pH = 4 and purified by reverse-phase HPLC (C18 column, 40-60% gradient of MeCN and water containing 0.1% FA) to give the product 4-oxo-4-(6H-thieno[2,3-e]indazol-2-yl)butanoic acid (5.0 mg, 5.7%): ESI MS[M + H]⁺ for C₁₃H₁₁N₂O₃S, calcd 275.0, found 275.1; ¹H NMR (400 MHz, DMSO-d6) δ 13.59 (s, 1H), 12.07 (s, 1H), 8.51 (s, 1H), 8.44 (s, 1H), 7.90 (d, *J =* 8.9 Hz, 1H), 7.64 (d, *J =* 9.0 Hz, 1H), 3.31 (s, 2H), 2.62 (t*, J =* 6.4 Hz, 2H).

### Example 40

### Synthesis of 4-oxo-4-(3-(2-(trimethylsilyl)ethoxy)methyl)-3H-thieno[3,2-e]indazol-7-yl)butanoic acid (Example Compound 40)

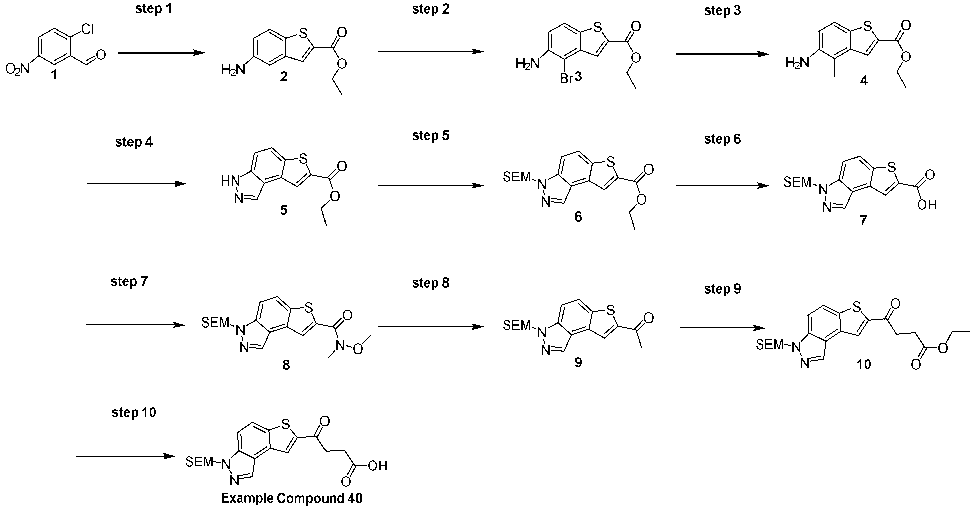

### Step (I): Synthesis of ethyl 5-aminobenzo[b]thiophene-2-carboxylate (Intermediate 2)

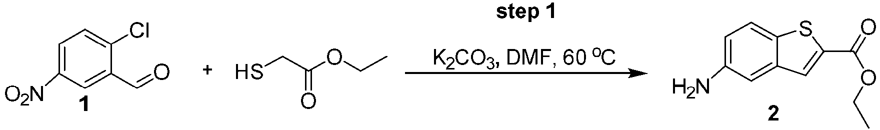

2-Chloro-5-nitrobenzaldehyde (5.0 g, 27.0 mmol) was dissolved in DMF (50 mL), and ethyl thioglycolate (16.2 g, 134.7 mmol) and K₂CO₃ (11.2 g, 80.8 mmol) were added. The mixture was purged with N₂ three times. The reaction mixture was then sealed well and stirred at 60°C under N₂ protection for 12 hours. LCMS at this point indicated that the reaction was complete. The reaction mixture was cooled to room temperature. The mixture was quenched into water (200 mL), extracted with EtOAc (300 mL), and washed three times with brine (300 mL). The organic phase was collected, dried over Na₂SO₄, and concentrated under vacuum to give a crude sample. The crude product was further purified by trituration (petroleum ether:EtOAc = 8:1) to give the pure title compound 2 as a red oil (2.9 g, 49%): ESI MS [M + H]⁺ for C₁₁H₁₂NO₂S, calcd 222.3, found 222.3.

### Step (II): Synthesis of ethyl 5-amino-4-bromobenzothiophene-2-carboxylate (Intermediate 3)

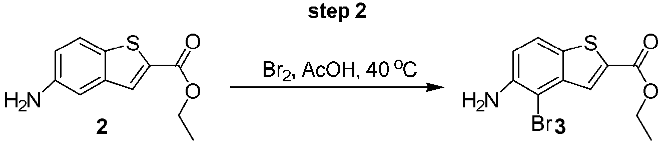

To a solution of ethyl 5-aminobenzo[b]thiophene-2-carboxylate (7.2 g, 32.5 mmol) in AcOH (200 mL) was slowly added Br₂ (3.6 g, 22.8 mmol). The reaction mixture was then stirred at 40°C under N₂ protection for 10 minutes. LCMS at this point indicated that the reaction was complete. The reaction mixture was adjusted to pH = 8 with Na₂CO₃ solution and extracted three times with EtOAc (400 mL). The organic phase was collected, dried over Na₂SO₄, and concentrated under vacuum to give the crude product 3 as a red solid (8.0 g, 82%): ESI MS [M + H]⁺ for C₁₁H₁₁BrNO₂S, calcd 300.2, found 300.2.

### Step (III): Synthesis of ethyl 5-amino-4-methylbenzo[b]thiophene-2-carboxylate (Intermediate 4)

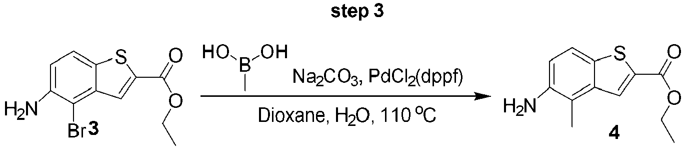

Ethyl 5-amino-4-bromobenzothiophene-2-carboxylate (2.6 g, 8.7 mmol), dioxane (26 mL), and H₂O (2.6 mL) were added under N₂, followed by Na₂CO₃ (2.8 g, 26.0 mmol), methylboronic acid (1.8 g, 30.1 mmol), and Pd(dppf)Cl₂ (0.6 g, 0.9 mmol). The reaction mixture was then stirred at 110°C overnight. LCMS at this point indicated that the reaction was complete. The reaction mixture was quenched into water (100 mL) and then extracted three times with EtOAc (100 mL). The organic phase was then dried over Na₂SO₄ and filtered. The filtrate was concentrated under vacuum to give a crude sample. The crude product was further purified by trituration (petroleum ether:EtOAc = 3:1) to give the pure title compound 4 as a brown solid (1.6 g, 79%): ESI MS [M + H]⁺ for C₁₂H₁₄NO₂S, calcd 236.3, found 236.3.

### Step (IV): Synthesis of ethyl 3H-thieno[3,2-e]indazole-7-carboxylate (Intermediate 5)

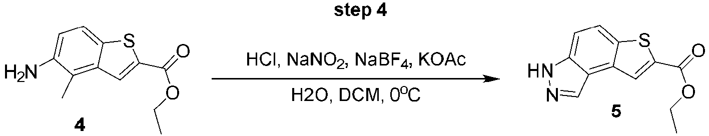

Ethyl 5-amino-4-methylbenzo[b]thiophene-2-carboxylate (1.5 g, 6.4 mmol) was dissolved in a solution of H₂O (10 mL) and HCl (1.6 mL, 19.1 mmol). Na₂NO₂ (0.4 g, 6.4 mmol) was added at 0°C and stirred in air at 0°C for 30 minutes. NaBF₄ (0.7 g, 6.4 mmol) was then added to the mixture and stirred at 0°C for 1 hour. Afterwards, the mixture was filtered to obtain the diazonium salt and rinsed with EtOH. The diazonium salt was dissolved in DCM (15 mL) at 0°C, and then KOAc (0.6 g, 6.4 mmol) was added. The mixture was stirred at 25°C for 1 h. LCMS at this point indicated that the reaction was complete. The reaction mixture was quenched by the addition of water (50 mL), then extracted three times with EtOAc (50 mL), dried over Na₂SO₄, and filtered. The filtrate was concentrated under vacuum to give the crude product 5 as a red solid (0.6 g, 38%): ESI MS [M + H]⁺ for C₁₂H₁₁N₂O₂S, calcd 247.3, found 247.3.

### Step (V): Synthesis of ethyl 3-((2-(trimethylsilyl)ethoxy)methyl)-3H-thieno[3,2-e]indazole-7-carboxylate (Intermediate 6)

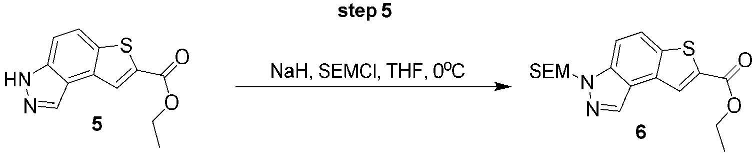

Ethyl 3H-thieno[3,2-e]indazole-7-carboxylate (800.0 mg, 3.3 mmol) was dissolved in THF (2 mL). NaH (233.9 mg, 9.8 mmol) was then slowly added at 0°C and stirred at 0°C for 30 minutes. SEMCl (812.3 mg, 4.9 mmol) was added to the mixture and stirred at 25°C for 1 hour. LCMS at this point indicated that the reaction was complete. The reaction mixture was poured into water (50 mL) to quench and then extracted three times with EtOAc (50 mL). The organic phase was then dried over Na₂SO₄ and filtered. The filtrate was concentrated under vacuum to give the crude product 6 as a brown oil (1.2 g, 98%): ESI MS [M + H]⁺ for C₁₈H₂₅N₂O₃SSi, calcd 377.6, found 377.6.

### Step (VI): Synthesis of 3-((2-(trimethylsilyl)ethoxy)methyl)-3H-thieno[3,2-e]indazole-7-carboxylic acid (Intermediate 7)

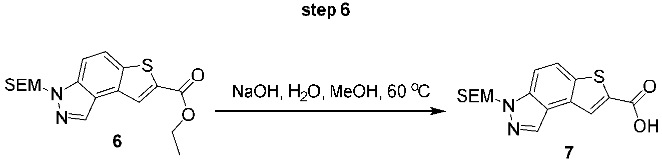

Ethyl 3-((2-(trimethylsilyl)ethoxy)methyl)-3H-thieno[3,2-e]indazole-7-carboxylate (2.1 g, 5.6 mmol) was dissolved in H₂O (10 mL) and THF (10 mL), and then NaOH (1.1 g, 27.9 mmol) was added. The mixture was stirred at 60°C for 1 hour. LCMS at this point indicated that the reaction was complete. The reaction mixture was poured into water (50 mL) to quench, then extracted three times with EtOAc (50 mL), dried over Na₂SO₄, and filtered. The filtrate was concentrated under vacuum to give a crude sample. The crude product was further purified by trituration (petroleum ether:EtOAc = 1:1) to give the pure title compound 7 as a yellow oil (1.8 g, 93%): ESI MS [M + H]⁺ for C₁₆H₂₁N₂O₃SSi, calcd 349.5, found 349.5.

### Step (VII): Synthesis of N-methoxy-N-methyl-3-((2-(trimethylsilyl)ethoxy)methyl)-3H-thieno[3,2-e]indazole-7-carb oxamide (Intermediate 8)

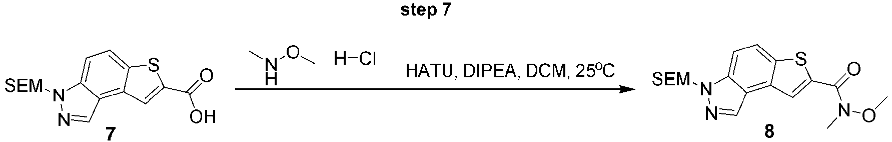

3-((2-(Trimethylsilyl)ethoxy)methyl)-3H-thieno[3,2-e]indazole-7-carboxylic acid (2.0 g, 5.7 mmol) was dissolved in DCM (20.0 mL) under N₂, and HATU (3.3 g, 8.6 mmol), DIPEA (2.2 g, 17.2 mmol), and N,O-dimethylhydroxylamine hydrochloride (0.62 g, 6.3 mmol) were added. The reaction mixture was stirred at 25°C for 3 hours. LCMS indicated that the reaction was complete. The reaction mixture was poured into water (50 mL), then extracted three times with EtOAc (50 mL), dried over Na₂SO₄, and filtered. The filtrate was concentrated under vacuum to give a crude sample. The crude product was further purified by trituration (petroleum ether:EtOAc = 5:1) to give the pure title compound 8 as a brown oil (2.0 g, 89%): ESI MS [M + H]⁺ for C₁₈H₂₆N₃O₃SSi, calcd 392.6, found 392.6.

### Step (VIII): Synthesis of 1-(3-((2-(trimethylsilyl)ethoxy)methyl)-3H-thieno[3,2-e]indazol-7-yl)ethan-1-one (Intermediate 9)

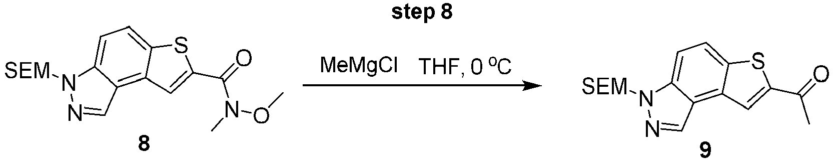

N-Methoxy-N-methyl-3-((2-(trimethylsilyl)ethoxy)methyl)-3H-thieno[3,2-e]indazole-7-carboxa mide (1.0 g, 2.6 mmol) was added to anhydrous THF (20 mL) at 0°C under a N₂ atmosphere. The reaction mixture was stirred at 30°C for 2 hours. LCMS indicated that the reaction was complete. The reaction mixture was poured into water (50 mL) and then diluted with EtOAc (50 mL). The resulting mixture was extracted three times with EtOAc (50 mL), dried over Na₂SO₄, and filtered. The filtrate was concentrated under vacuum to give a crude sample. The crude product was further purified by trituration (petroleum ether:EtOAc = 10:1) to give the pure title compound 9 as a brown oil (750.0 mg, 85%): ESI MS [M + H]⁺ for C₁₇H₂₃N₂O₂SSi, calcd 347.5, found 347.5.

### Step (IX): Synthesis of ethyl 4-oxo-4-(3-((2-(trimethylsilyl)ethoxy)methyl)-3H-thieno[3,2-e]indazol-7-yl)butanoate (Intermediate 10)

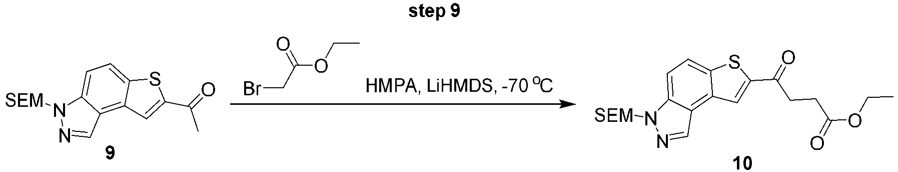

1-(3-((2-(Trimethylsilyl)ethoxy)methyl)-3H-thieno[3,2-e]indazol-7-yl)ethan-1-one (500.0 mg, 1.4 mmol) and HMPA (905.0 mg, 5.1 mmol) were dissolved in anhydrous THF (20 mL), and the mixture was purged with N₂ three times. The reaction mixture was cooled to -70°C under N₂ protection using a bath. A 1M solution of HMDSLi in THF (5.8 mL, 5.8 mmol) was then added dropwise and stirred at -70°C for 1 hour. Afterwards, ethyl 2-bromoacetate (720.0 mg, 4.3 mmol) was added to the reaction mixture and stirred at 25°C under a N₂ atmosphere for 10 minutes. LCMS at this point indicated that the reaction was complete. The reaction mixture was poured into water (50 mL) and then diluted with EtOAc (100 mL). The resulting mixture was extracted with EtOAc (100 mL). The organic phase was washed three times with brine (50 mL), dried over Na₂SO₄, and filtered. The filtrate was concentrated under vacuum to give a crude sample. The crude product was further purified by trituration (petroleum ether:EtOAc = 10:1) to give the pure title compound 10 as a brown oil (420.0 mg, 67%): ESI MS [M + H]⁺ for C₂₁H₂₉N₂O₄SSi, calcd 433.6, found 433.6.

### Step (X): Synthesis of 4-oxo-4-(3-(2-(trimethylsilyl)ethoxy)methyl)-3H-thieno[3,2-e]indazol-7-yl)butanoic acid (Example Compound 40)

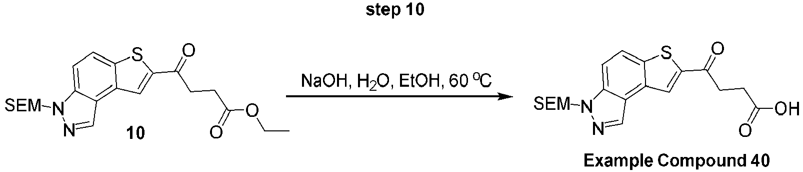

Ethyl 4-oxo-4-(3-((2-(trimethylsilyl)ethoxy)methyl)-3H-thieno[3,2-e]indazol-7-yl)butanoate (200.0 mg, 0.5 mmol) and NaOH (92.5 mg, 2.3 mmol) were dissolved in H₂O (5.0 mL). The reaction mixture was stirred at 60°C for 1 hour. LCMS at this point indicated that the reaction was complete. The reaction mixture was quenched with HCl (1 mol/L) and adjusted to pH = 6. The mixture was extracted three times with EtOAc (30 mL). The organic phase was collected, dried over Na₂SO₄, and concentrated under vacuum to give a crude sample. The crude product was further purified by reverse-phase HPLC (C18 column, 50-60% gradient of MeCN and water containing 0.1% TFA) to give the product as a white solid (28.0 mg, 15%): ESI MS [M + H]⁺ for C₁₉H₂₅N₂O₄SSi, calcd 405.6, found 405.6. ¹H NMR (400 MHz, DMSO-d₆) δ 12.23 (s, 1H), 8.84 (d, *J =* 0.7 Hz, 1H), 8.55 (d, *J =* 0.8 Hz, 1H), 8.06 (d, *J* = 9.0 Hz, 1H), 7.96 (d, *J =* 9.0 Hz, 1H), 5.85 (s, 2H), 3.58 - 3.50 (m, 2H), 3.39 (t, *J =* 6.5 Hz, 2H), 2.66 (t, *J =* 6.4 Hz, 2H), 0.80 (dd, *J =* 8.4, 7.5 Hz, 2H), -0.12 (s, 9H).

### Example 41

### Synthesis of 4-oxo-4-(3H-thieno[3,2-e]indazol-7-yl)butanoic acid (Example Compound 41)

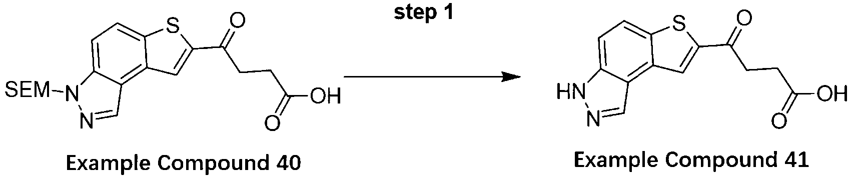

### Step (I): Synthesis of 4-oxo-4-(3H-thieno[3,2-e]indazol-7-yl)butanoic acid (Example Compound 41)

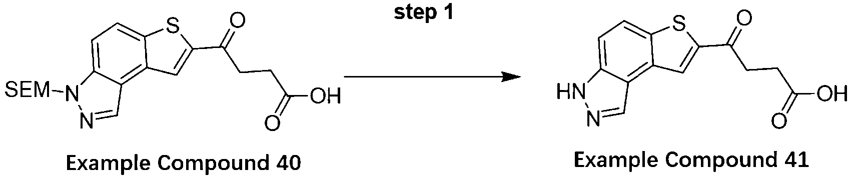

4-Oxo-4-((2-((trimethylsilyl)ethoxy)methyl)-3H-thieno[3,2-e]indazol-7-yl)butanoic acid (200.0 mg, 0.08 mmol) was dissolved in TFA (1.5 g, 13.3 mmol). The reaction mixture was stirred at 25°C for 40 minutes. LCMS at this point indicated that the reaction was complete. The reaction mixture was quenched with NaHCO₃ solution. The mixture was further purified by reverse-phase HPLC (C18 column, 40-50% gradient of MeCN and water containing 0.1% TFA) to give the product as a white solid (6.0 mg, 4%): ESI MS [M + H]⁺ for C₁₃H₁₁N₂O₃S, calcd 275.3, found 275.3. ¹H NMR (400 MHz, DMSO-d₆) δ 13.47 (s, 1H), 8.81 (s, 1H), 8.48 (s, 1H), 7.94 (d, *J =* 9.0 Hz, 1H), 7.74 (d, *J =* 9.0 Hz, 1H), 3.37 t, *J =* 6.4 Hz, 2H), 2.64 (t, *J =* 6.4 Hz, 2H).

### Example 42

### Synthesis of 4-oxo-4-(2-(2-(trimethylsilyl)ethoxy)methyl)-2H-thieno[3,2-e]indazol-7-yl)butanoic acid (Example Compound 42)

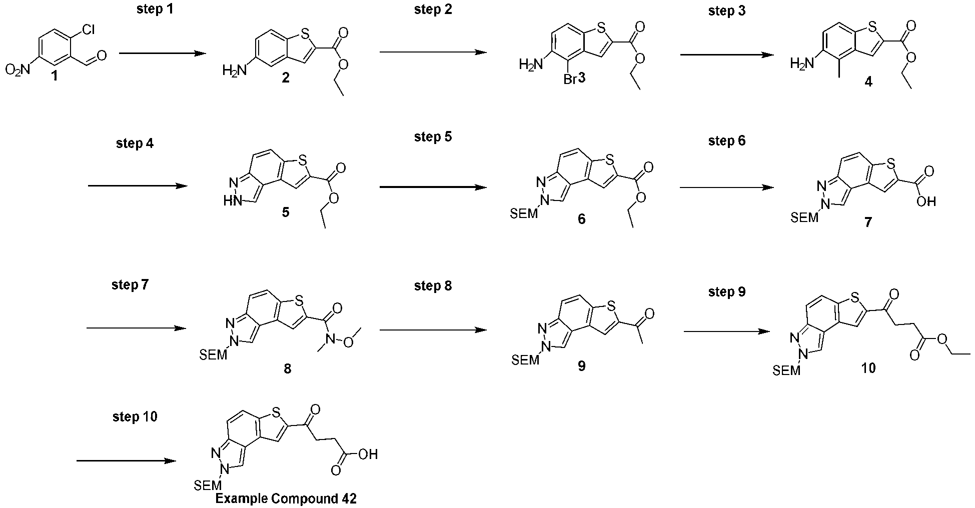

For the first three steps of the synthesis, refer to Example 40.

### Step (IV): Synthesis of ethyl 2H-thieno[3,2-e]indazole-7-carboxylate (Intermediate 5)

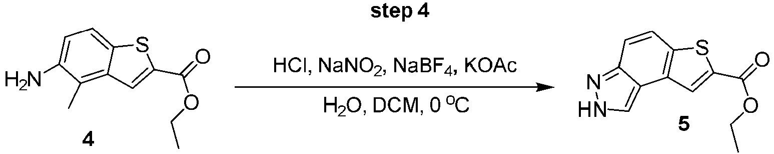

Ethyl 5-amino-4-methylbenzo[b]thiophene-2-carboxylate (1.5 g, 6.4 mmol) was dissolved in H₂O (10 mL) and HCl (1.6 mL, 19.1 mmol). NaNO₂ (0.4 g, 6.4 mmol) was added at 0°C and stirred at 0°C in air for 30 minutes. NaBF₄ (0.7 g, 6.4 mmol) was then added to the mixture and stirred at 0°C for 1 hour. The mixture was filtered to obtain the diazonium salt. The diazonium salt was dissolved in DCM (15 mL) at 0°C, and KOAc (0.6 g, 6.4 mmol) was added. The mixture was stirred at 25°C for 1 h. LCMS indicated that the reaction was complete. The reaction mixture was poured into water (50 mL), then extracted three times with EtOAc (50 mL), dried over Na₂SO₄, and filtered. The filtrate was concentrated under vacuum to give the crude product 5 as a red solid (0.6 g, 38%): ESI MS [M + H]⁺ for C₁₂H₁₁N₂O₂S, calcd 247.3, found 247.3.

### Step (V): Synthesis of ethyl 2-((2-(trimethylsilyl)ethoxy)methyl -2H-thieno[3,2-e]indazole-7-carboxylate (Intermediate 6)

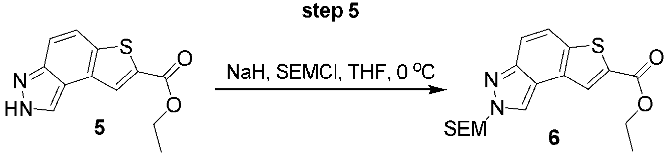

Ethyl 2H-thieno[3,2-e]indazole-7-carboxylate (800.0 mg, 3.3 mmol) was dissolved in THF (2 mL). NaH (233.9 mg, 9.8 mmol) was slowly added at 0°C and stirred at 0°C for 30 minutes. SEMCl (812.3 mg, 4.9 mmol) was then added to the mixture and stirred at 25°C for 1 hour. LCMS indicated that the reaction was complete. The reaction mixture was poured into water (50 mL) and then extracted three times with EtOAc (50 mL). The organic phase was then dried over Na₂SO₄ and filtered. The filtrate was concentrated under vacuum to give the crude product 6 as a brown oil (1.2 g, 98%): ESI MS [M + H]⁺ for C₁₈H₂₅N₂O₃SSi, calcd 377.6, found 377.6.

### Step (VI): Synthesis of 2-((2-(trimethylsilyl)ethoxy)methyl)-2H-thieno[3,2-e]indazole-7-carboxylic acid (Intermediate 7)

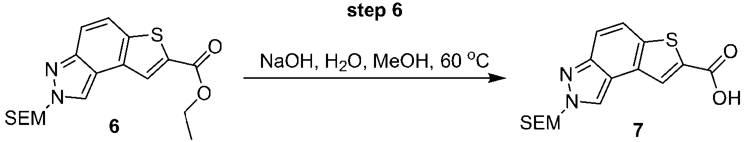

Ethyl 2-((2-(trimethylsilyl)ethoxy)methyl-2H-thieno[3,2-e]indazole-7-carboxylate (2.1 g, 5.6 mmol) was dissolved in H₂O (10 mL) and THF (10 mL), and NaOH (1.1 g, 27.9 mmol) was added. The mixture was stirred at 60°C for 1 hour. LCMS indicated that the reaction was complete. The reaction mixture was poured into water (50 mL), then extracted three times with EtOAc (50 mL), dried over Na₂SO₄, and filtered. The filtrate was concentrated under vacuum to give a crude sample. The crude product was further purified by trituration (petroleum ether:EtOAc = 1:1) to give the pure title compound 7 as a yellow oil (1.8 g, 93%): ESI MS [M + H]⁺ for C₁₆H₂₁N₂O₃SSi, calcd 349.5, found 349.5.

### Step (VII): Synthesis of N-methoxy-N-methyl-2-((2-(trimethylsilyl)ethoxy)methyl)-2H-thieno[3,2-e]indazole-7-carb oxamide (Intermediate 8)

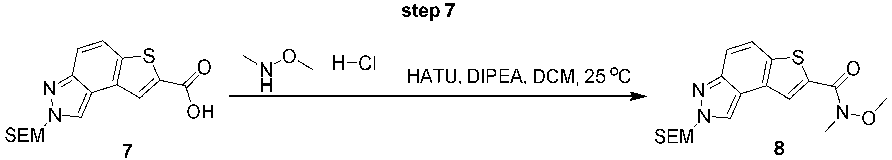

2-((2-(Trimethylsilyl)ethoxy)methyl)-2H-thieno[3,2-e]indazole-7-carboxylic acid (2.0 g, 5.7 mmol) was dissolved in DCM (20.0 mL) under N₂, and HATU (3.3 g, 8.6 mmol), DIPEA (2.2 g, 17.2 mmol), and N,O-dimethylhydroxylamine hydrochloride (0.62 g, 6.3 mmol) were added. Then, the reaction mixture was stirred at 25°C for 3 hours. LCMS indicated that the reaction was complete. The reaction mixture was poured into water (50 mL), then extracted three times with EtOAc (50 mL), dried over Na₂SO₄, and filtered. The filtrate was concentrated under vacuum to give a crude sample. The crude product was further purified by trituration (petroleum ether:EtOAc = 5:1) to give the pure title compound 8 as a brown oil (2.0 g, 89%): ESI MS [M + H]⁺ for C₁₈H₂₆N₃O₃SSi, calcd 392.6, found 392.6.

### Step (VIII): Synthesis of 1-(2-(2-(trimethylsilyl)ethoxy)methyl)-2H-thieno[3,2-e]indazol-7-yl)ethan-1-one (Intermediate 9)

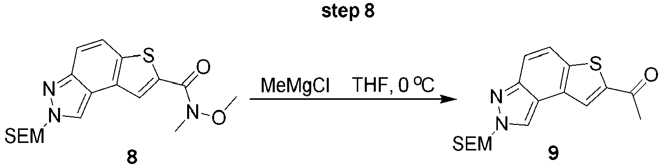

N-Methoxy-N-methyl-2-((2-(trimethylsilyl)ethoxy)methyl)-2H-thieno[3,2-e]indazole-7-carboxa mide (1.0 g, 2.6 mmol) was added to a solution of anhydrous THF (20 mL) at 0°C under a N₂ atmosphere. The reaction mixture was stirred at 30°C for 2 hours. LCMS indicated that the reaction was complete. The reaction mixture was poured into water (50 mL) and then diluted with EtOAc (50 mL). The resulting mixture was extracted three times with EtOAc (50 mL), dried over Na₂SO₄, and filtered. The filtrate was concentrated under vacuum to give a crude sample. The crude product was further purified by trituration (petroleum ether:EtOAc = 10:1) to give the pure title compound 9 as a brown oil (750.0 mg, 85%): ESI MS [M + H]⁺ for C₁₇H₂₃N₂O₂SSi, calcd 347.5, found 347.5.

### Step (IX): Synthesis of ethyl 4-oxo-4-(2-((2-(trimethylsilyl)ethoxy)methyl)-2H-thieno[3,2-e]indazol-7-yl)butanoate (Intermediate 10)

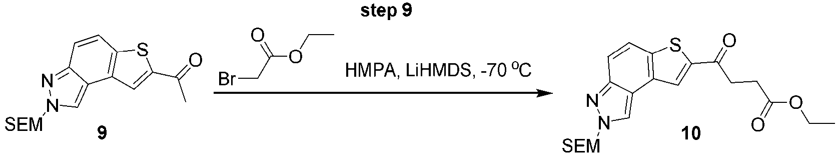

1-(2-(2-(Trimethylsilyl)ethoxy)methyl)-2H-thieno[3,2-e]indazol-7-yl)ethan-1-one (500.0 mg, 1.4 mmol) and HMPA (905.0 mg, 5.1 mmol) were added to anhydrous THF (20 mL). The mixture was purged with N₂ three times. The reaction mixture was cooled to -70°C under N₂ protection. A 1M solution of HMDSLi (5.8 mL, 5.8 mmol) in THF was then added dropwise and stirred at -70°C for 1 hour. Then, ethyl 2-bromoacetate (720.0 mg, 4.3 mmol) was added to the reaction mixture and stirred at 25°C under a N₂ atmosphere for 10 minutes. The reaction mixture was quenched into water (50 mL) to quench and then diluted with EtOAc (100 mL). The resulting mixture was extracted with EtOAc (100 mL). The organic phase was washed three times with brine (50 mL), dried over Na₂SO₄, and filtered. The filtrate was concentrated under vacuum to give a crude sample. The crude product was further purified by trituration (petroleum ether:EtOAc = 10:1) to give the pure title compound 10 as a brown oil (420.0 mg, 67%): ESI MS [M + H]⁺ for C₂₁H₂₉N₂O₄SSi, calcd 433.6, found 433.6.

### Step (X): Synthesis of 4-oxo-4-(2-(2-(trimethylsilyl)ethoxy)methyl)-2H-thieno[3,2-e]indazol-7-yl)butanoic acid (Example Compound 42)

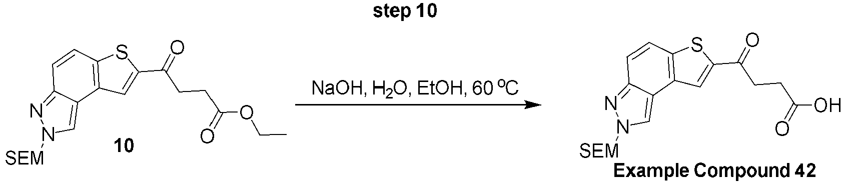

Ethyl 4-oxo-4-(2-((2-(trimethylsilyl)ethoxy)methyl)-2H-thieno[3,2-e]indazol-7-yl)butanoate (200.0 mg, 0.5 mmol) and NaOH (92.5 mg, 2.3 mmol) were dissolved in H₂O (5.0 mL). The reaction mixture was stirred at 60°C for 1 hour. HCl (1 mol/L) was added dropwise to the reaction mixture to adjust pH = 6. The mixture was extracted three times with EtOAc (30 mL). The organic phase was collected, dried over Na₂SO₄, and concentrated under vacuum to give a crude sample. The crude product was further purified by reverse-phase HPLC (C18 column, 50-60% gradient of MeCN and water containing 0.1% TFA) to give the product as a white solid (28.0 mg, 15%): ESI MS [M + H]⁺ for C₁₉H₂₅N₂O₄SSi, calcd 405.6, found 405.6; ¹H NMR (400 MHz, DMSO-d₆) δ 12.22 (s, 1H), 8.82 (d, *J =* 0.8 Hz, 1H), 8.73 (s, 1H), 7.86 (d, *J* = 9.3 Hz, 1H), 7.77 (d, *J =* 9.2 Hz, 1H), 5.78 (s, 2H), 3.67 - 3.58 (t, *J =* 7.5 Hz, 2H), 3.36 (t, *J =* 6.4 Hz, 2H), 2.65 (t, *J =* 6.4 Hz, 2H), 0.87 (t, *J =* 7.5 Hz, 2H), 0.06 (s, 9H).

### Example 43

### Synthesis of 4-(3-methyl-3H-thieno[3,2-e]indazol-7-yl)-4-oxobutanoic acid (Example Compound 43)

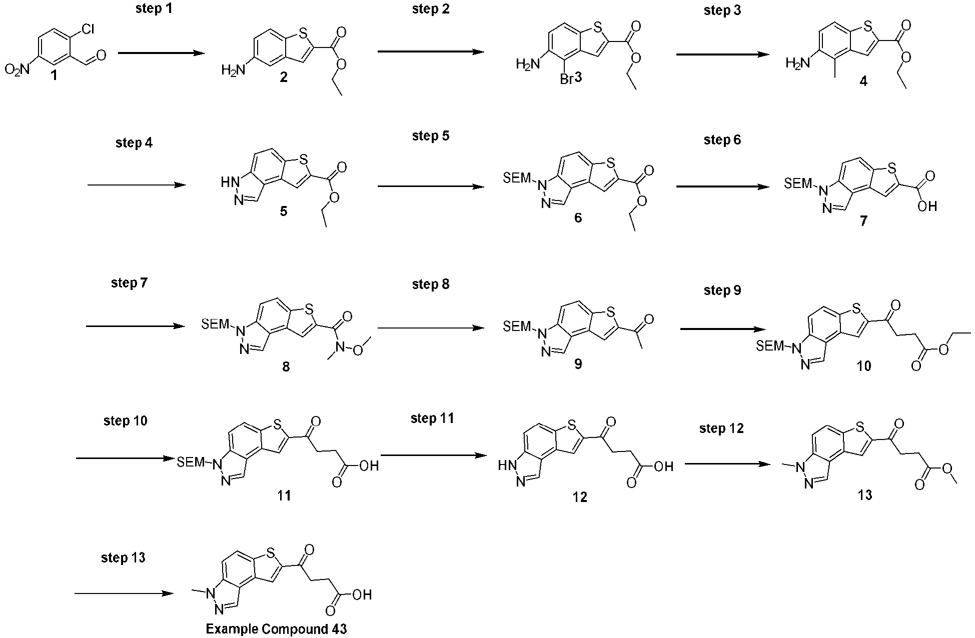

**For the first eleven steps of the synthesis, refer to Example 41.**

### Step (XII): Synthesis of methyl 4-(3-methyl-3H-thieno[3,2-e]indazol-7-yl)-4-oxobutanoate (Intermediate 13)

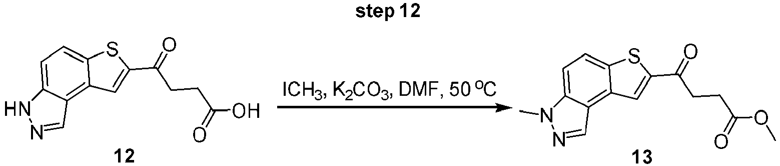

4-Oxo-4-(3H-thieno[3,2-e]indazol-7-yl)butanoic acid (50.0 mg, 0.2 mmol) was dissolved in DMF (1 mL), and then CH₃I (142.0 mg, 0.2 mmol) and K₂CO₃ (138.2 mg, 0.6 mmol) were added. The reaction mixture was stirred at 50°C in a sealed tube for 4 hours. The reaction mixture was cooled to room temperature, poured into water (10 mL), extracted with EtOAc (20 mL), and washed three times with brine (20 mL). The organic phase was collected, dried over Na₂SO₄, and concentrated under vacuum to give the crude product 13 as a brown solid (40.0 mg, 74%): ESI MS [M + H]⁺ for C₁₅H₁₅N₂O₃S, calcd 303.4, found 303.4.

### Step (XIII): Synthesis of 4-(3-methyl-3H-thieno[3,2-e]indazol-7-yl)-4-oxobutanoic acid (Example Compound 43)

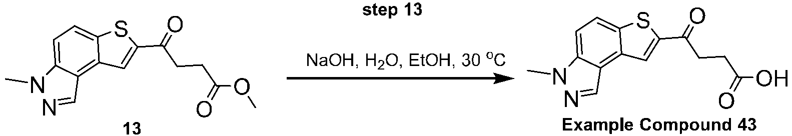

Methyl 4-(3-methyl-3H-thieno[3,2-e]indazol-7-yl)-4-oxobutanoate (30.0 mg, 0.1 mmol) was dissolved in EtOH (1 mL) and H₂O (1 mL), and NaOH (20.0 mg, 0.5 mmol) was added. The reaction mixture was stirred at 30°C for 2 hours. HCl (1 mol/L) was added to the reaction mixture to adjust pH = 6. The mixture was extracted three times with EtOAc (30 mL). The organic phase was collected, dried over Na₂SO₄, and concentrated under vacuum to give a crude sample. The crude product was further purified by reverse-phase HPLC (C18 column, 40-50% gradient of MeCN and water containing 0.1% TFA) to give the product as a white solid (3.0 mg, 10%): ESI MS [M + H]⁺ for C₁₄H₁₃N₂O₃S, calcd 289.3, found 289.3. ¹H NMR (400 MHz, DMSO-*d*₆) δ 8.81 (s, 1H), 8.44 (s, 1H), 8.01 (d, *J =* 9.1 Hz, 1H), 7.88 (d, *J =* 9.0 Hz, 1H), 4.15 (s, 3H), 2.64 (t, *J =* 6.4 Hz, 2H).

### Example 44

### Synthesis of 4-(2-methyl-2H-thieno[3,2-e]indazol-7-yl)-4-oxobutanoic acid (Example Compound 44)

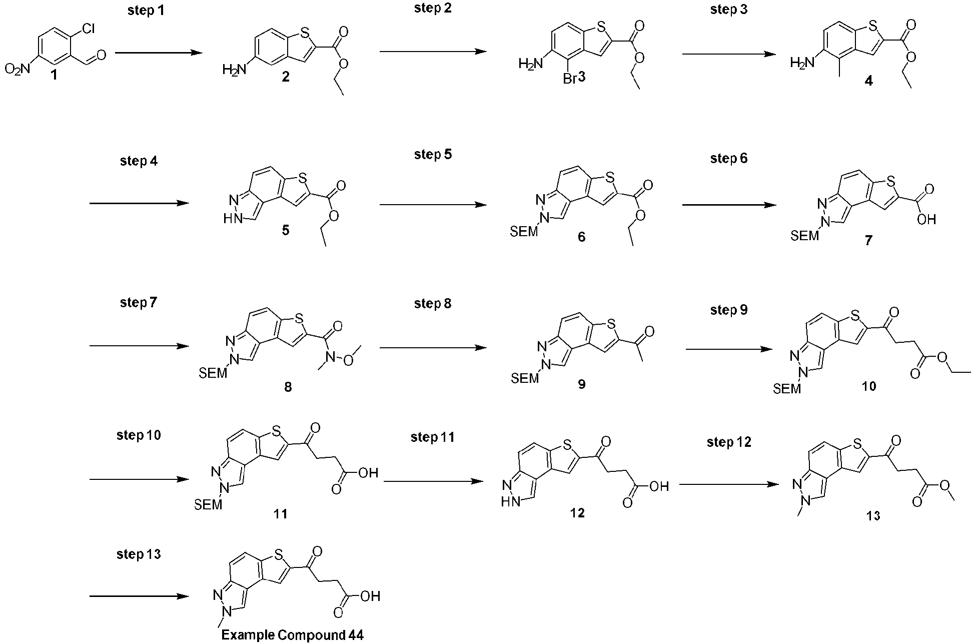

**For the first eleven steps of the synthesis, refer to Example 42.**

### Step (XII): Synthesis of methyl 4-(2-methyl-2H-thieno[3,2-e]indazol-7-yl)-4-oxobutanoate (Intermediate 13)

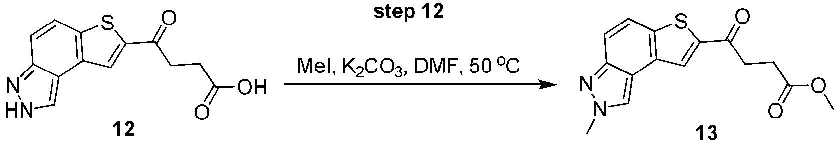

4-Oxo-4-(2H-thieno[3,2-e]indazol-7-yl)butanoic acid (50.0 mg, 0.2 mmol) was added to a solution of DMF (1 mL), followed by MeI (142.0 mg, 0.2 mmol) and K₂CO₃ (138.2 mg, 0.6 mmol). The reaction mixture was stirred at 50°C in a sealed tube for 4 hours. The reaction mixture was cooled to room temperature, poured into water (10 mL), extracted with EtOAc (20 mL), and washed three times with brine (20 mL). The organic phase was collected, dried over Na₂SO₄, and concentrated under vacuum to give the crude product 13 as a brown solid (40.0 mg, 74%): ESI MS [M + H]⁺ for C₁₅H₁₅N₂O₃S, calcd 303.4, found 303.4.

### Step (XIII): Synthesis of 4-(2-methyl-2H-thieno[3,2-e]indazol-7-yl)-4-oxobutanoic acid (Example Compound 44)

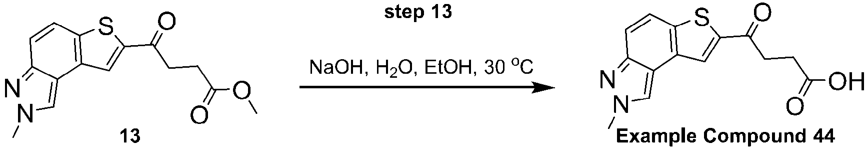

Methyl 4-(2-methyl-2H-thieno[3,2-e]indazol-7-yl)-4-oxobutanoate (30.0 mg, 0.1 mmol) was dissolved in a solution of EtOH (1 mL) and H₂O (1 mL), and then NaOH (20.0 mg, 0.5 mmol) was added. The reaction mixture was stirred at 30°C for 2 hours. HCl (1 mol/L) was added dropwise to the mixture to adjust pH = 6. The mixture was extracted three times with EtOAc (30 mL). The organic phases were collected, dried over Na₂SO₄, and concentrated under vacuum to give a crude sample. The crude product was further purified by reverse-phase HPLC (C18 column, 40-50% gradient of MeCN and water containing 0.1% TFA) to give the product as a white solid (3.5 mg, 12%): ESI MS [M + H]⁺ for C₁₄H₁₃N₂O₃S, calcd 289.3, found 289.3. ¹H NMR (400 MHz, DMSO-d₆) δ 8.67 (s, 1H), 8.63 (s, 1H), 7.81 (d, *J =* 9.2 Hz, 1H), 7.73 (d, *J* = 9.2 Hz, 1H), 4.22 (s, 3H), 2.62 (t, *J =* 6.5 Hz, 2H).

### Example 45

### Synthesis of 2-ethyl-4-(4-methoxythieno[2,3:5,6]benzo[1,2-d]oxazol-7-yl)-4-oxobutanoic acid (Example Compound 45)

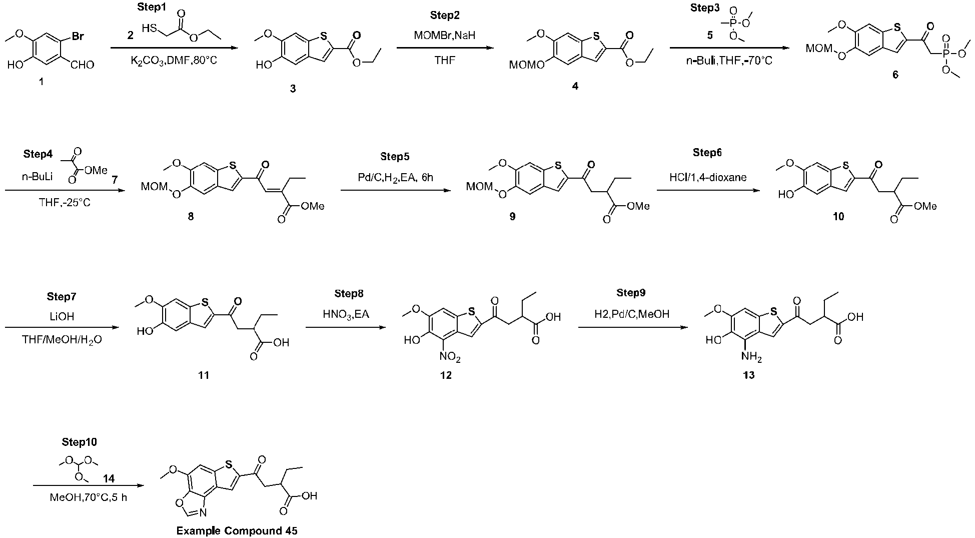

### Step (I): Synthesis of ethyl 5-hydroxy-6-methoxybenzo[b]thiophene-2-carboxylate (Intermediate 3)

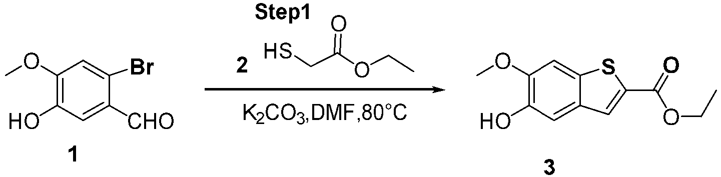

2-Bromo-5-hydroxy-4-methoxybenzaldehyde (20.0 g, 86.57 mmol) and potassium carbonate (16.39 g, 118.59 mmol) were dissolved in DMF (300 mL), and ethyl thioglycolate (11.96 g, 99.55 mmol) was added dropwise under nitrogen protection in an ice bath. The mixture was slowly warmed to room temperature and stirred for 0.5 hour, and finally warmed to 80°C for 4 h. TLC indicated that the reaction was complete with a small amount of starting material remaining. The reaction solution was added to saturated ammonium chloride solution. The mixture was extracted three times with ethyl acetate (100 mL). The organic phase was washed twice with saturated brine, dried over anhydrous sodium sulfate, and concentrated under reduced pressure. The resulting solid was purified on a silica gel column (EA:PE = 0 to 10%) in three batches to give the pure title compound 3 as a light brown solid (15.2 g, 69.6%).

### Step (II): Synthesis of ethyl 6-methoxy-5-(methoxymethoxy)benzo[b]thiophene-2-carboxylate (Intermediate 4)

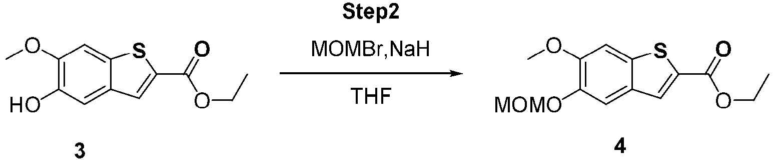

Ethyl 5-hydroxy-6-methoxybenzo[b]thiophene-2-carboxylate (15.2 g, 60.25 mmol) was dissolved in THF (200 mL). NaH (2.77 g, 72.3 mmol) was added in an ice bath and stirred for half an hour. MOMBr (9.03 g, 72.3 mmol) was then added in an ice bath. The mixture was returned to room temperature and stirred for 1 hour. TLC indicated that the starting material had completely reacted. The reaction solution was poured into sodium chloride solution, extracted three times with ethyl acetate (100 mL), washed with saturated brine, dried over anhydrous sodium sulfate, and concentrated. The resulting solid was purified on a silica gel column (EA:PE = 0 to 10%) to give the pure title compound 3 as a light brown solid (13.5 g, 75.61%).

### Step (III): Synthesis of dimethyl (2-(6-methoxy-5-(methoxymethoxy)benzo[b]thiophen-2-yl)-2-oxoethyl)phosphonate (Intermediate 6)

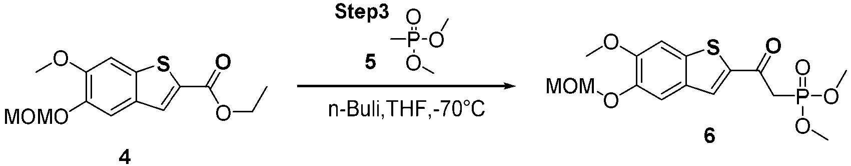

Dimethyl methylphosphonate (11.3 g, 91.11 mmol) was dissolved in anhydrous THF (200 mL), and n-BuLi (2.5M, 5.84 mL, 91.11 mmol) was added dropwise at -70°C. The mixture was then stirred at -70°C for 1 hour. Ethyl 6-methoxy-5-(methoxymethoxy)benzo[b]thiophene-2-carboxylate (13.5 g, 45.56 mmol) was then dissolved in THF (100 mL), added dropwise to the system at -70°C, and stirred for 1 hour. TLC indicated that most of the starting material had completely reacted. Aqueous ammonium chloride solution was added dropwise to the system, and the mixture was extracted three times with ethyl acetate (100 mL). The organic phase was washed with saturated brine, dried over anhydrous sodium sulfate, concentrated, and purified on a silica gel column (EA:PE = 0 to 100%) to give the pure title compound 6 as a white solid (8.6 g, 50.43%): ESI MS [M + H]⁺ for C₁₅H₂₀O₇PS, calcd 375.06, found 375.1.

### Step (IV): Synthesis of methyl 2-ethyl-4-(6-methoxy-5-methoxymethoxy)benzo[b]thiophen-2-yl)-4-oxobut-2-enoate (Intermediate 8)

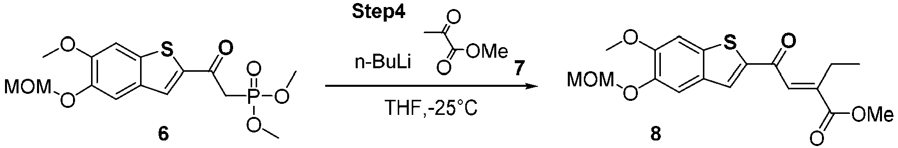

Dimethyl (2-(6-methoxy-5-(methoxymethoxy)benzo[b]thiophen-2-yl)-2-oxoethyl)phosphonate (1.00 g, 2.67 mmol) was dissolved in THF (20 mL). n-BuLi (0.19 mL, 2.94 mmol) was slowly added at -25°C under a nitrogen atmosphere and stirred for 1 hour. Methyl 2-oxopropanoate (310.19 mg, 2.67 mmol) was then dissolved in THF (5 mL), added dropwise to the system, and stirred for 10 minutes. The mixture was then returned to room temperature and stirred for 2 hours. TLC indicated that most of the starting material had completely reacted. The reaction solution was added dropwise to saturated aqueous ammonium chloride solution (100 mL) and extracted three times with ethyl acetate (30 mL). The organic phase was washed with saturated brine, dried over anhydrous sodium sulfate, concentrated under reduced pressure, and purified on a silica gel column (EA:PE = 0 to 50%) to give the pure title compound 8 as a yellow solid (860 mg, 88.39%): ESI MS [M + H]⁺ for C₁₈H₂₁O₆S, calcd 365.10, found 365.1.

### Step (V): Synthesis of methyl 2-ethyl-4-(6-methoxy-5-methoxymethoxy)benzo[b]thiophen-2-yl)-4-oxobutanoate

### (Intermediate 9)

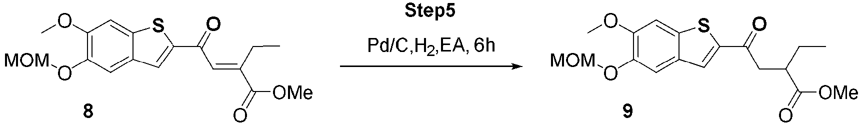

Methyl 2-ethyl-4-(6-methoxy-5-methoxymethoxy)benzo[b]thiophen-2-yl)-4-oxobut-2-enoate (860 mg, 2.36 mmol) was dissolved in ethyl acetate (10 mL). 10% Wet Pd/C (255.41 mg, 0.24 mmol) was added, and a hydrogen balloon was connected. The mixture was purged with hydrogen and reacted at room temperature for 5 h. TLC indicated that the starting material had completely reacted. The reaction solution was filtered. The filtrate was concentrated and then purified on a silica gel column (EA:PE = 0 to 30%) to give the crude title compound 9 as a yellow solid (380 mg, 43.94%): ESI MS [M + H]⁺ for C₁₈H₂₃O₆S, calcd 367.10, found 367.1.

### Step (VI): Synthesis of methyl 2-ethyl-4-(5-hydroxy-6-methoxybenzo[b]thiophen-2-yl)-4-oxobutanoate (Intermediate 10)

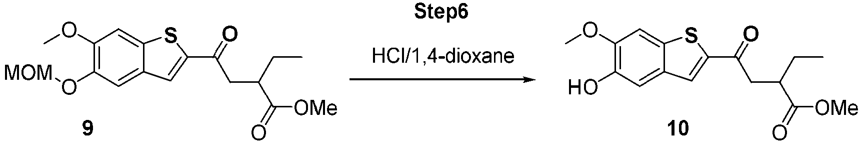

Methyl 2-ethyl-4-(6-methoxy-5-methoxymethoxy)benzo[b]thiophen-2-yl)-4-oxobutanoate (380 mg, 1.04 mmol) was dissolved in a solution of HCl/1,4-dioxane (2 M, 6 mL) and stirred at room temperature for 1 hour. TLC indicated that the starting material had completely reacted. The reaction solution was concentrated to give the crude title compound 10 as a yellow solid (320 mg, 99%).

### Step (VII): Synthesis of 2-ethyl-4-(5-hydroxy-6-methoxybenzo[b]thiophen-2-yl)-4-oxobutanoic acid (Intermediate 11)

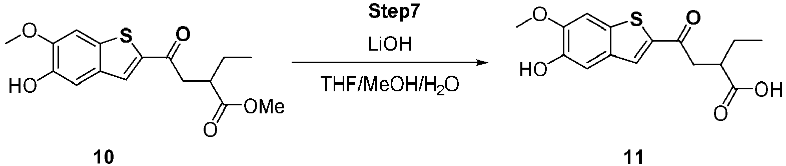

Methyl 2-ethyl-4-(5-hydroxy-6-methoxybenzo[b]thiophen-2-yl)-4-oxobutanoate (320 mg, 0.99 mmol) was dissolved in a mixed solvent of tetrahydrofuran/water/methanol (2:1:1, 8 mL), and lithium hydroxide monohydrate (47.55 mg, 1.99 mmol) was added. The reaction solution was stirred at room temperature for 4 hours. TLC indicated that the reaction was complete. The reaction solution was diluted with water (20 mL), adjusted to pH 3 with 1M HCl, extracted three times with ethyl acetate (20 mL), washed with saturated brine, dried over anhydrous sodium sulfate, and concentrated under reduced pressure to give the crude title compound 11 as a yellow solid (280 mg, 91.48%).

### Step (VIII): Synthesis of 2-ethyl-4-(5-hydroxy-6-methoxy-4-nitrobenzo[b]thiophen-2-yl)-4-oxobutanoic acid (Intermediate 12)

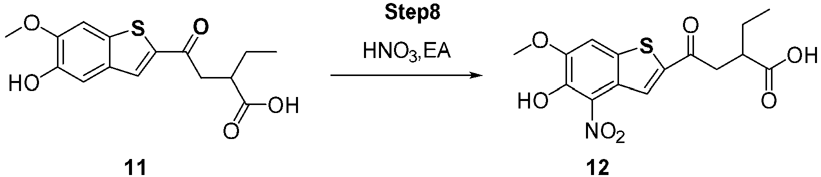

2-Ethyl-4-(5-hydroxy-6-methoxybenzo[b]thiophen-2-yl)-4-oxobutanoic acid (280 mg, 0.91 mmol) was dissolved in ethyl acetate (10 mL), and concentrated nitric acid (171.65 mg, 2.72 mmol) was added dropwise in an ice bath. The mixture was then returned to room temperature and stirred for 1.5 hours. TLC indicated that the starting material had completely reacted. The reaction solution was dropped into water, extracted three times with ethyl acetate (20 mL), washed with saturated brine, dried over anhydrous sodium sulfate, concentrated under reduced pressure, and purified on a silica gel column (EA:PE = 0 to 70%) to give the pure title compound 12 as a yellow solid (140 mg, 43.63%).

### Step (IX): Synthesis of 4-(4-amino-5-hydroxy-6-methoxybenzo[b]thiophen-2-yl)-2-ethyl-4-oxobutanoic acid (Intermediate 13)

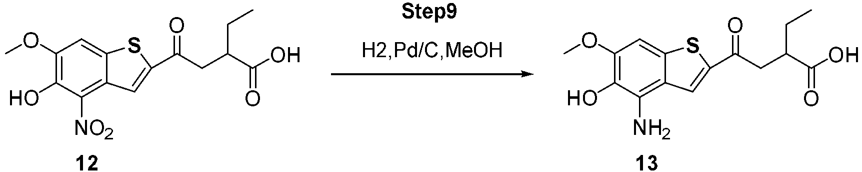

2-Ethyl-4-(5-hydroxy-6-methoxy-4-nitrobenzo[b]thiophen-2-yl)-4-oxobutanoic acid (140 mg, 0.4 mmol) was dissolved in methanol (10 mL). 10% Wet Pd/C (8.43 mg, 0.08 mmol) was added, and a hydrogen balloon was connected. The mixture was purged with hydrogen and reacted at room temperature for 3 h. The reaction solution was then filtered, and the filtrate was concentrated to give the crude title compound 13 as a brown solid (126 mg, 99.8%): ESI MS [M + H]⁺ for C₁₅H₁₈NO₅S, calcd 324.07, found 324.2.

### Step (X): Synthesis of 2-ethyl-4-(4-methoxythieno[2,3:5,6]benzo[1,2-d]oxazol-7-yl)-4-oxobutanoic acid (Example Compound 45)

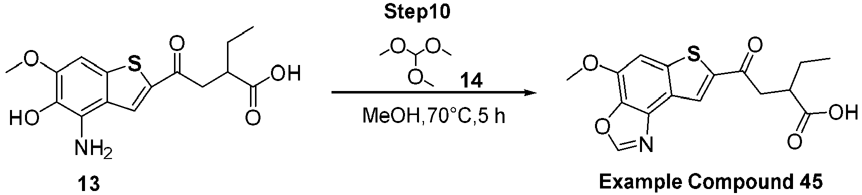

4-(4-Amino-5-hydroxy-6-methoxybenzo[b]thiophen-2-yl)-2-ethyl-4-oxobutanoic acid (126 mg, 0.4 mmol) was dissolved in methanol (5 mL), and trimethoxymethane (361 mg, 3.4 mmol) was added. The mixture was reacted at 70°C in a sealed tube for 5 hours. The reaction solution was filtered. The solid was triturated with methanol (5 mL) overnight, filtered, and washed three times with methanol to give the product as a white solid (77.6 mg, 58%): ESI MS [M + H]⁺ for C₁₆H₁₆NO₅S, calcd 334.07, found 334.07. ¹H NMR (400 MHz, DMSO-*d*₆) δ 12.18 (s, 1H), 8.92 (s, 1H), 8.61 (s, 1H), 7.78 (s, 1H), 4.07 (s, 3H), 3.54 (dd, J = 17.7, 9.7 Hz, 1H), 3.26 - 3.15 (m, 1H), 2.86 - 2.75 (m, 1H), 1.64 (pd, J = 7.1, 3.7 Hz, 2H), 0.95(t, J = 7.4 Hz, 1H).

### Example 47

### Synthesis of (R)-4-(4-methoxythieno[2',3':5,6]benzo[1,2-d]oxazol-7-yl)-2-methyl-4-oxobutanoic acid (Example Compound 47)

### Example 46

### Synthesis of (S)-4-(4-methoxythieno[2',3':5,6]benzo[1,2-d]oxazol-7-yl)-2-methyl-4-oxobutanoic acid (Example Compound 46)

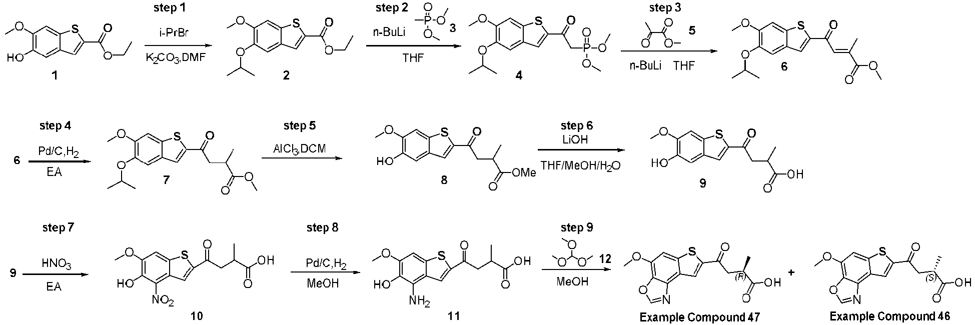

### Step (I): Synthesis of ethyl 5-isopropoxy-6-methoxybenzo[b]thiophene-2-carboxylate (Intermediate 2)

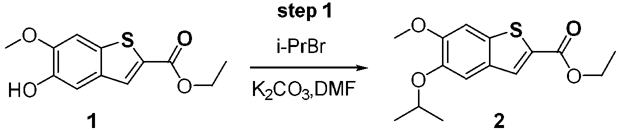

Ethyl 5-hydroxy-6-methoxybenzo[b]thiophene-2-carboxylate (18 g, 71.3 mmol) was dissolved in DMF (200 mL). Potassium carbonate (24.6 g, 178.3 mmol) and isopropyl bromide (17.54 g, 142.7 mmol) were added at room temperature and stirred at room temperature for 48 hours. TLC indicated partial remaining starting material. The reaction solution was filtered. The filtrate was poured into saturated ammonium chloride solution, extracted three times with ethyl acetate (100 mL), washed with saturated brine, dried over anhydrous sodium sulfate, and concentrated. The resulting solid was purified on a silica gel column (EA:PE = 0 to 10%) to give the pure title compound 2 as a white solid (11.6 g, 55.24%): ESI MS [M + H]⁺ for C₁₅H₁₉O₄S, calcd 295.09, found 295.1.

### Step (II): Synthesis of dimethyl (2-(5-isopropoxy-6-methoxybenzo[b]thiophen-2-yl)-2-oxoethyl)phosphonate (Intermediate 4)

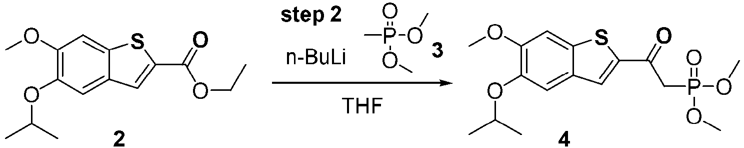

Dimethyl methylphosphonate (6.15 g, 49.6 mmol) was dissolved in anhydrous tetrahydrofuran (50 mL), and n-BuLi (2.5M, 3.18 mL, 49.6 mmol) was added dropwise at -70°C. The mixture was then stirred at -70°C for 1 hour. Ethyl 5-isopropoxy-6-methoxybenzo[b]thiophene-2-carboxylate (7.3 g, 24.8 mmol) was then dissolved in THF (50 mL), added dropwise to the system at -70°C, and stirred for 1 hour. TLC indicated that most of the starting material had completely reacted. Aqueous ammonium chloride solution was added dropwise to the system, and the mixture was extracted three times with ethyl acetate (100 mL). The organic phase was washed with saturated brine, dried over anhydrous sodium sulfate, concentrated, and purified on a silica gel column (EA:PE = 0 to 100%) to give the pure title compound 4 as a white solid (4.7 g, 50.9%): ESI MS [M + H]⁺ for C₁₆H₂₂O₆PS, calcd 373.08, found 373.1.

### Step (III): Synthesis of methyl (E)-4-(5-isopropoxy-6-methoxybenzo[b]thiophen-2-yl)-2-methyl-4-oxobut-2-enoate (Intermediate 6)

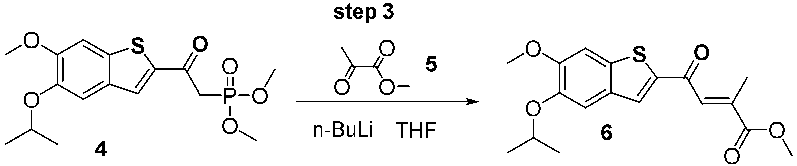

Dimethyl (2-(5-isopropoxy-6-methoxybenzo[b]thiophen-2-yl)-2-oxoethyl)phosphonate (4.7 g, 12.62 mmol) was dissolved in anhydrous tetrahydrofuran (30 mL). n-BuLi (0.89 mL, 13.88 mmol) was slowly added at -25°C under a nitrogen atmosphere and stirred for 1 hour. Methyl 2-oxopropanoate (1.42 g, 13.88 mmol) was then dissolved in anhydrous tetrahydrofuran (30 mL), added dropwise to the system, and stirred for 10 minutes. The mixture was then returned to room temperature and stirred for 2 hours. TLC indicated that most of the starting material had completely reacted. The reaction solution was added dropwise to saturated aqueous ammonium chloride solution (100 mL) and extracted three times with ethyl acetate (100 mL). The organic phase was washed with saturated brine, dried over anhydrous sodium sulfate, concentrated under reduced pressure, and purified on a silica gel column (EA:PE = 0 to 50%) to give the pure title compound 6 as a yellow oil (3.6 g, 81.86%): ESI MS [M + H]⁺ for C₁₈H₂₁O₅S, calcd 349.10, found 349.1.

### Step (IV): Synthesis of methyl 4-(5-isopropoxy-6-methoxybenzo[b]thiophen-2-yl)-2-methyl-4-oxobutanoate (Intermediate 7)

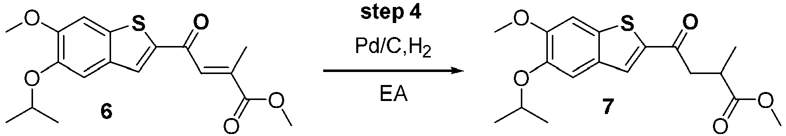

Methyl (E)-4-(5-isopropoxy-6-methoxybenzo[b]thiophen-2-yl)-2-methyl-4-oxobut-2-enoate (3.6 g, 10.33 mmol) was dissolved in ethyl acetate (40 mL). Wet Pd/C (10%, 109.9 mg, 1.03 mmol) was added, and a hydrogen balloon was connected. The mixture was purged with hydrogen and reacted at room temperature for 4 h. The reaction solution was then filtered. The filtrate was concentrated and then purified on a silica gel column (EA:PE = 0 to 30%) to give the pure title compound 7 as a white solid (2.4 g, 66.3%): ESI MS[M+H]⁺ for C₁₈H₂₃O₅S, calcd 351.12, found:351.1.

### Step (V): Synthesis of methyl 4-(5-hydroxy-6-methoxybenzo[b]thiophen-2-yl)-2-methyl-4-oxobutanoate (Intermediate 8)

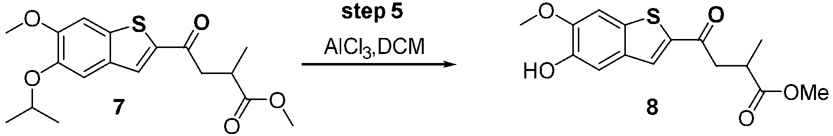

Methyl 4-(5-isopropoxy-6-methoxybenzo[b]thiophen-2-yl)-2-methyl-4-oxobutanoate (1.6 g, 4.57 mmol) was dissolved in a solution of dichloromethane (20 mL), and aluminum trichloride (1.8 g, 13.7 mmol) was added in an ice bath. The mixture was then heated to 40°C and stirred for 4 h. The reaction solution was then added dropwise to a 0.5M HCl solution and extracted three times with ethyl acetate. The organic phase was washed with saturated brine, dried, concentrated, and purified on a silica gel column (EA:PE = 0 to 30%) to give the pure title compound 8 as a white solid (1.1 g, 78.13%).

### Step (VI): Synthesis of 4-(5-hydroxy-6-methoxybenzo[b]thiophen-2-yl)-2-methyl-4-oxobutanoic acid (Intermediate 9)

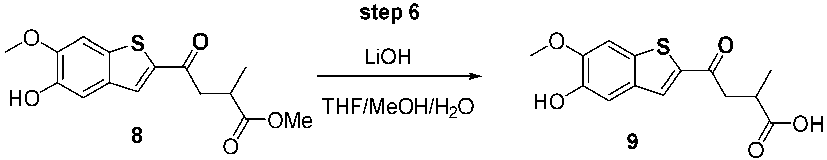

Methyl 4-(5-hydroxy-6-methoxybenzo[b]thiophen-2-yl)-2-methyl-4-oxobutanoate (1.64 g, 5.32 mmol) was dissolved in a mixed solvent of tetrahydrofuran/water/methanol (2:1:1, 20 mL), and lithium hydroxide (254.7 mg, 10.64 mmol) was added. The reaction solution was stirred at room temperature for 18 hours. The reaction solution was then adjusted to pH 3, extracted with ethyl acetate, washed with saturated brine, dried, and concentrated to give the pure Intermediate 9 as a white solid (1.4 g, 89.44%).

### Step (VII): Synthesis of 4-(5-hydroxy-6-methoxy-4-nitrobenzo[b]thiophen-2-yl)-2-methyl-4-oxobutanoic acid (Intermediate 10)

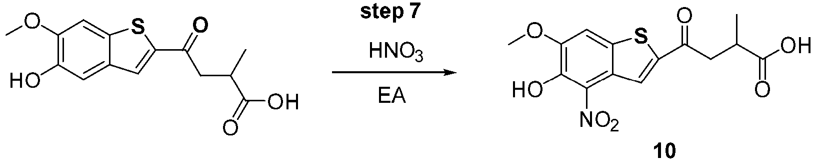

4-(5-Hydroxy-6-methoxybenzo[b]thiophen-2-yl)-2-methyl-4-oxobutanoic acid (1.4 g, 4.76 mmol) was dissolved in ethyl acetate (20 mL), and concentrated nitric acid (899 mg, 14.27 mmol) was added in an ice bath. The mixture was stirred at room temperature for 1.5 hours. TLC indicated that the starting material had completely reacted. The reaction solution was immediately poured into water and extracted with ethyl acetate. The organic phase was washed three times with saturated brine and concentrated at room temperature. The resulting solid was purified on a silica gel column (EA:PE = 0 to 100%) to give the pure title compound 10 as a brown solid (680 mg, 42.13%).

### Step (VIII): Synthesis of 4-(4-amino-5-hydroxy-6-methoxybenzothiophen-2-yl)-2-methyl-4-oxobutanoic acid (Intermediate 11)

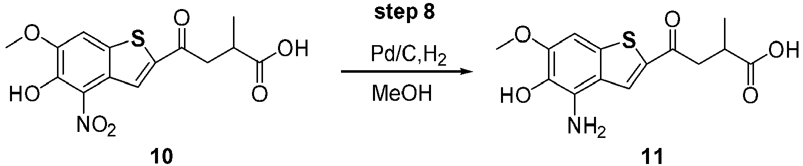

4-(5-Hydroxy-6-methoxy-4-nitrobenzo[b]thiophen-2-yl)-2-methyl-4-oxobutanoic acid (680 mg, 2 mmol) was dissolved in methanol (10 mL). Wet Pd/C (10%, 42.6 mg, 0.4 mmol) was added, and a hydrogen balloon was connected. The mixture was purged with hydrogen and reacted at room temperature for 1 h. The reaction solution was then filtered, and the filtrate was concentrated to give the pure title compound 11 as a brown solid (618 mg, 99.8%): ESI MS [M + H]⁺ for C₁₄H₁₆NO₅S, calcd 310.07, found 310.2.

### Step (IX): Synthesis of (R)-4-(4-methoxythieno[2',3':5,6]benzo[1,2-d]oxazol-7-yl)-2-methyl-4-oxobutanoic acid and (S)-4-(4-methoxythieno[2,3:5,6]benzo[1,2-d]oxazol-7-yl)-2-methyl-4-oxobutanoic acid (Example Compound 46 and Example Compound 47)

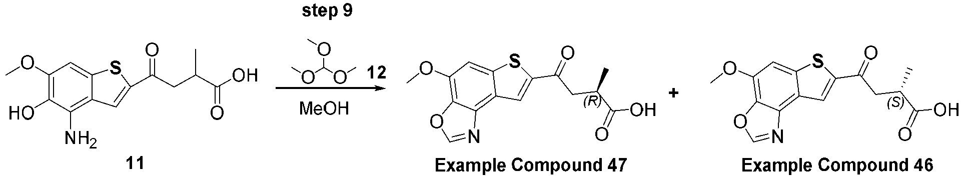

4-(4-Amino-5-hydroxy-6-methoxybenzothiophen-2-yl)-2-methyl-4-oxobutanoic acid (620 mg, 2 mmol) was dissolved in methanol (10 mL), and trimethoxymethane (2.12 g, 20 mmol) was added. The mixture was reacted at 70°C in a sealed tube for 7 hours. The reaction solution was then filtered, and the resulting solid was resolved by chiral column (column: CHIRALAPK IC-3 (IC30CE-KJ008), mobile phase: DCM/IPA/Hac = 75/25/0.1 (V/V/V)) to give the product Example Compound 47 (108.4 mg, 16.97%): R.T=3.968 min, ESI MS [M + H]⁺ for C₁₅H₁₄NO₅S, calcd 320.05, found 320.1. ¹H NMR (400 MHz, DMSO-*d*₆) δ 12.20 (s, 1H), 8.93 (s, 1H), 8.57 (s, 1H), 7.78 (s, 1H), 4.07 (s, 3H), 3.53 (dd, J = 17.5, 8.6 Hz, 1H), 3.20 (dd, J = 17.5, 5.0 Hz, 1H), 2.97 - 2.84 (m, 1H), 1.20 (d, J = 7.2 Hz, 3H) and Example Compound 46 (108.6 mg, 17.04%): R.T=5.144 min, ESI MS [M + H]⁺ for C₁₅H₁₄NO₅S, calcd 320.05, found 320.1. ¹H NMR (400 MHz, DMSO-*d*₆) δ 12.20 (s, 1H), 8.93 (s, 1H), 8.57 (s, 1H), 7.78 (s, 1H), 4.07 (s, 3H), 3.53 (dd, J = 17.5, 8.6 Hz, 1H), 3.20 (dd, J = 17.5, 5.0 Hz, 1H), 2.97 - 2.84 (m, 1H), 1.20 (d, J = 7.2 Hz, 3H) as white solids.

### Example 48

### Synthesis of 4-(2-fluoro-4-methoxythieno[3,2-e]benzofuran-7-yl)-4-oxobutanoic acid (Example Compound 48)

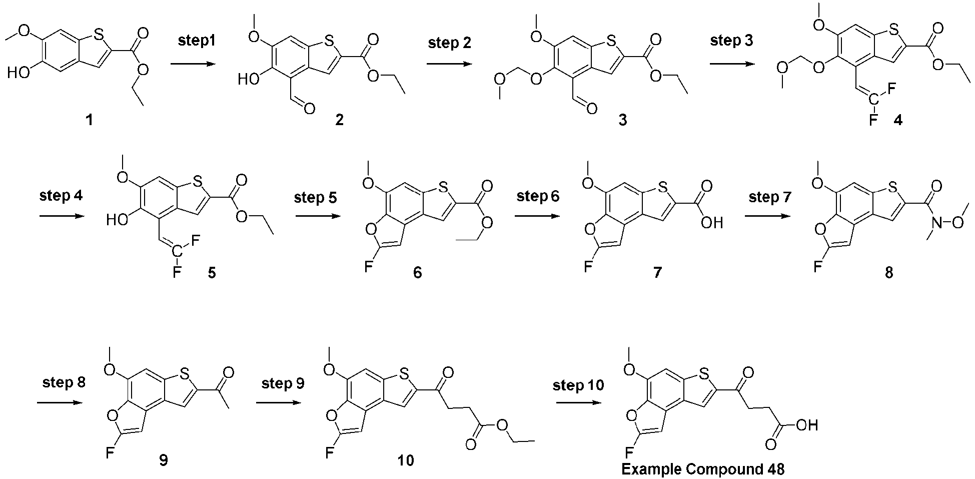

### Step (I): Synthesis of ethyl 4-formyl-5-hydroxy-6-methoxybenzo[b]thiophene-2-carboxylate (Intermediate 2)

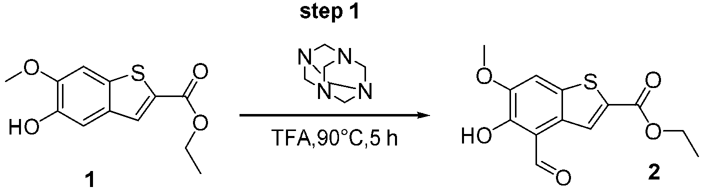

Ethyl 5-hydroxy-6-methoxybenzo[b]thiophene-2-carboxylate (2.5 g, 9.91 mmol) was dissolved in trifluoroacetic acid (25 mL), and urotropine (6.94 g, 49.55 mmol) was added portionwise in an ice bath. The reaction mixture was warmed to 90°C under nitrogen protection and stirred overnight. TLC indicated that most of the starting material had completely reacted. The reaction solution was diluted with water (200 mL) and extracted three times with DCM (50 mL). The organic phase was washed with brine (50 mL), dried over anhydrous sodium sulfate, and filtered. The filtrate was concentrated under vacuum to give a crude sample. The crude product was purified by silica gel column chromatography (EA:PE = 0 to 30%) to give Compound 2 as a yellow solid (1.0 g, 36%): ESI MS [M + H]⁺ for C₁₃H₁₃O₅S, calcd 281.04, found 281.1.

### Step (II): Synthesis of ethyl 4-formyl-6-methoxy-5-(methoxymethoxy)benzo[b]thiophene-2-carboxylate (Intermediate 3)

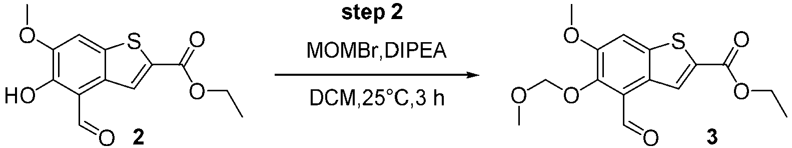

Ethyl 4-formyl-5-hydroxy-6-methoxybenzo[b]thiophene-2-carboxylate (1.0 g, 3.57 mmol) was dissolved in DCM (20 mL), and MOMBr (668.71 mg, 5.35 mmol) and DIPEA (922.15 mg, 7.14 mmol) were added in an ice bath. The mixture was returned to room temperature and stirred for 4 hours. TLC indicated that the starting material had completely reacted. The reaction solution was poured into ammonium chloride solution (50 mL), extracted three times with ethyl acetate (50 mL), washed with saturated brine, dried over anhydrous sodium sulfate, and concentrated. The resulting solid was purified on a silica gel column (EA:PE = 0 to 10%) to give Compound 3 as a colorless oil (1.1 g, 95%): ESI MS [M + H]⁺ for C₁₅H₁₇O₆S, calcd 325.07, found 325.1.

### Step (III): Synthesis of ethyl 4-(2,2-difluorovinyl)-6-methoxy-5-(methoxymethoxy)benzo[b]thiophene-2-carboxylate (Intermediate 4)

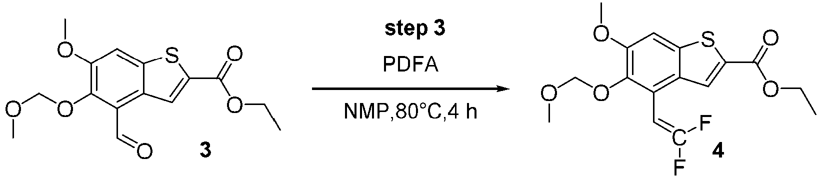

Ethyl 4-formyl-6-methoxy-5-(methoxymethoxy)benzo[b]thiophene-2-carboxylate (750 mg, 2.31 mmol) was dissolved in N-methylpyrrolidinone (10 mL), and (triphenylphosphonio)difluoroacetate (1.65 g, 4.62 mmol) was added. The system was purged with nitrogen, warmed to 80°C, and stirred for 4 hours.

TLC indicated that the reaction was complete. The reaction solution was poured into saturated ammonium chloride solution (50 mL) and extracted three times with ethyl acetate (50 mL). The organic phase was washed with brine, dried over anhydrous sodium sulfate, concentrated, and purified on a silica gel column (EA:PE = 0 to 20%) to give Compound 4 as a white solid (640 mg, 77.23%): ESI MS [M + H]⁺ for C₁₆H₁₇F₂O₅S, calcd 359.07, found 359.1.

### Step (IV): Synthesis of ethyl 4-(2,2-difluorovinyl)-5-hydroxy-6-methoxybenzo[b]thiophene-2-carboxylate (Intermediate 5)

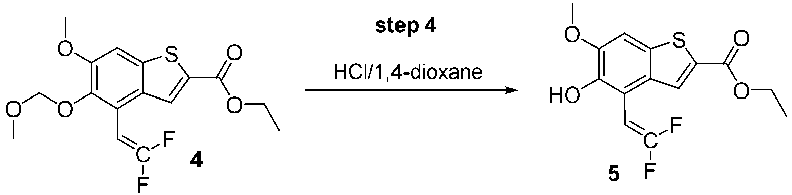

Ethyl 4-(2, 2-difluorovinyl)-6-methoxy-5-(methoxymethoxy)benzo[b]thiophene-2-carboxylate (640 mg, 1.79 mmol) was dissolved in 1,4-dioxane (5 mL), and HCl/1,4-dioxane solution (4M, 5.8 mL, 159.59 mmol) was added. The reaction solution was stirred at room temperature for 0.5 hour. TLC indicated that the starting material had completely reacted. The reaction solution was concentrated to give Compound 5 as a white solid (560 mg, 99.77%): ESI MS [M + H]⁺ for C₁₄H₁₃F₂O₄S, calcd 315.04, found 315.1.

### Step (V): Synthesis of ethyl 2-fluoro-4-methoxythieno[3,2-e]benzofuran-7-carboxylate (Intermediate 6)

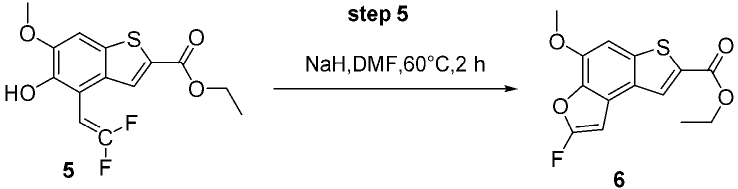

Ethyl 4-(2,2-difluorovinyl)-5-hydroxy-6-methoxybenzo[b]thiophene-2-carboxylate (560 mg, 1.78 mmol) was dissolved in DMF (10 mL). NaH (49.15 mg, 2.14 mmol) was added in an ice bath and stirred for 10 min. The mixture was then warmed to 60°C and stirred for 2 h. The reaction solution was then poured into aqueous ammonium chloride solution (50 mL), extracted three times with ethyl acetate (50 mL), washed with saturated brine, dried over anhydrous sodium sulfate, and concentrated. The resulting solid was purified on a silica gel column (EA:PE = 0 to 20%) to give Compound 6 (380 mg, 72.47%) as a white solid: ESI MS [M + H]⁺ for C₁₄H₁₂FO₄S, calcd 295.04, found 295.1.

### Step (VI): Synthesis of 2-fluoro-4-methoxythieno[3,2-e]benzofuran-7-carboxylic acid (Intermediate 7)

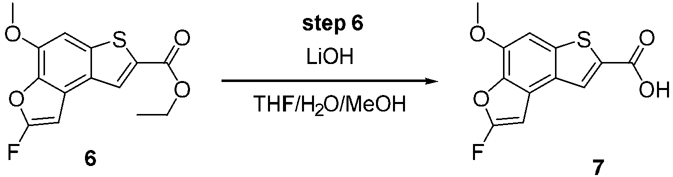

Ethyl 2-fluoro-4-methoxythieno[3,2-e]benzofuran-7-carboxylate (200 mg, 0.68 mmol) was dissolved in H₂O (1 mL), MeOH (1 mL), and THF (2 mL), and then LiOH (32.5 mg, 1.36 mmol) was added. The reaction mixture was stirred at 25°C for 3 hours. The reaction mixture was then adjusted to pH = 3, then extracted three times with EtOAc (50 mL), dried over Na₂SO₄, and filtered. The filtrate was concentrated under vacuum to give crude Compound 7 as a white solid (180.0 mg, 99%).

### Step (VII): Synthesis of 2-fluoro-N,4-dimethoxy-N-methylthieno[3,2-e]benzofuran-7-carboxamide (Intermediate 8)

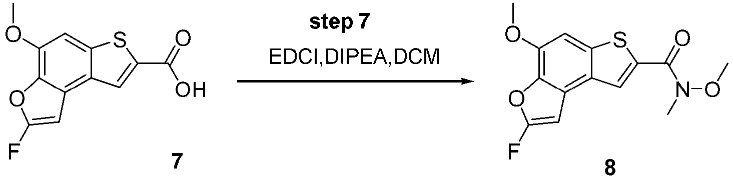

2-Fluoro-4-methoxythieno[3,2-e]benzofuran-7-carboxylic acid (180 mg, 0.68 mmol) was dissolved in DCM (10 mL). N,O-dimethylhydroxylamine hydrochloride (79.1 mg, 0.81 mmol), DIPEA (113.5 mg, 0.88 mmol), and EDCI (130.9 mg, 0.68 mmol) were added, followed by nitrogen protection. The mixture was stirred at room temperature for 18 hours. The system was then added dropwise to ammonium chloride solution, extracted with ethyl acetate, washed with brine, dried, concentrated, and purified on a silica gel column (EA:PE = 0% to 30%) to give the pure title compound 8 as a white solid (130 mg, 62.17%): ESI MS [M + H]⁺ for C₁₄H₁₃FNO₄S, calcd 310.05, found 310.1.

### Step (VIII): Synthesis of 1-(2-fluoro-4-methoxythieno[3,2-e]benzofuran-7-yl)ethan-1-one (Intermediate 9)

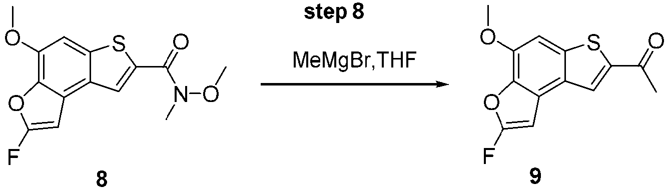

2-Fluoro-N,4-dimethoxy-N-methylthieno[3,2-e]benzofuran-7-carboxamide (130.0 mg, 0.42 mmol) was dissolved in anhydrous THF (5 mL), and methylmagnesium bromide (3 M, 0.15 ml, 1.26 mmol) was added at 0°C under N₂ protection. The reaction mixture was stirred at 25°C for 1 hour. The reaction mixture was quenched into ice water (25 mL) and then diluted with EtOAc (50 mL). The resulting mixture was extracted three times with EtOAc (50 mL), washed three times with brine (50 mL), dried over Na₂SO₄, and filtered. The filtrate was concentrated under vacuum, and the resulting solid was purified on a silica gel column (EA:PE = 0 to 20%) to give the pure title compound 9 as a yellow oil (100.0 mg, 90.03%).

### Step (IX): Synthesis of ethyl 4-(2-fluoro-4-methoxythieno[3,2-E]benzofuran-7-yl)-4-oxobutanoate (Intermediate 11)

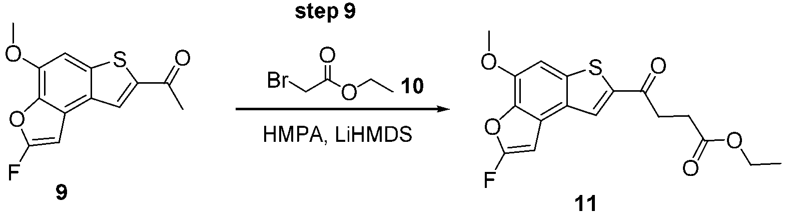

1-(2-Fluoro-4-methoxythieno[3,2-e]benzofuran-7-yl)ethan-1-one (100.0 mg, 0.38 mmol) and HMPA (237.3 mg, 1.32 mmol) were dissolved in a solution of tetrahydrofuran (10 mL), and a solution of LiHMDS in tetrahydrofuran (1M, 0.49 ml, 0.49 mmol) was added dropwise at -70°C. The reaction solution was stirred at -40°C for 1 h. The system was then cooled to -70°C, and ethyl bromoacetate (126.3 mg, 0.76 mmol) was added. The system was slowly warmed to room temperature and stirred for 1 hour. The reaction solution was then added dropwise to saturated ammonium chloride solution. The resulting mixture was extracted three times with EtOAc (50 mL), washed three times with brine (50 mL), dried over Na₂SO₄, filtered, and concentrated. The resulting solid was purified on a silica gel column (EA:PE = 0 to 30%) to give the pure title compound 11 as a white oil (40.0 mg, 30.17%): ESI MS [M + H]⁺ for C₁₇H₁₆FO₅S, calcd 351.06, found 351.1.

### Step (X): Synthesis of 4-(2-fluoro-4-methoxythieno[3,2-e]benzofuran-7-yl)-4-oxobutanoic acid (Example Compound 48)

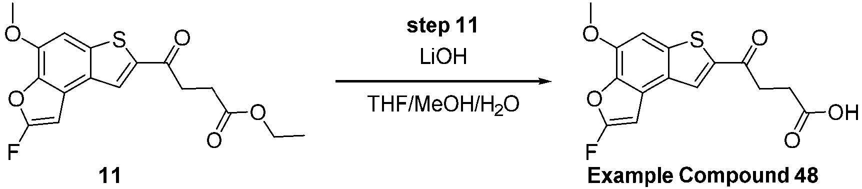

Ethyl 4-(2-fluoro-4-methoxythieno[3,2-e]benzofuran-7-yl)-4-oxobutanoate (40 mg, 0.11 mmol) was dissolved in H₂O (1 mL), MeOH (1 mL), and THF (2 mL), and then LiOH (5.3 mg, 0.23 mmol) was added. The reaction mixture was stirred at 25°C for 1 hour. The reaction solution was then adjusted to pH 3 with 1M HCl and concentrated. The resulting solid was purified by high-performance liquid chromatography (C18 column, mobile phase gradient: 0 to 50% MeCN and 0.1% TFA in water) to give the product as a white solid (20 mg, 54.35%). ¹H NMR (400 MHz, DMSO-d6) δ12.22 (s, 1H), 8.60 (s, 1H), 7.65 (s, 1H), 6.82 (d, J = 6.6 Hz, 1H), 4.02 (s, 3H), 3.31 (t, J = 6.5 Hz, 2H), 2.64 (t, J = 6.5 Hz, 2H). ESI MS [M + H]⁺ for C₁₅H₁₂FO₅S, calcd 323.03, found 323.1.

### Example 49

### Synthesis of 4-(4-methoxy-3H-thieno [3',2':3,4]benzo[1,2-d]imidazol-7-yl)-4-oxobutanoic acid (Example Compound 49)

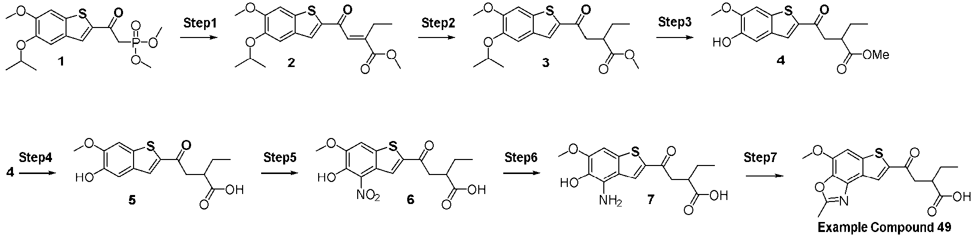

### Step (I): Synthesis of methyl 2-ethyl-4-(5-isopropoxy-6-methoxybenzo[b]thiophen-2-yl)-4-oxobut-2-enoate (Intermediate 2)

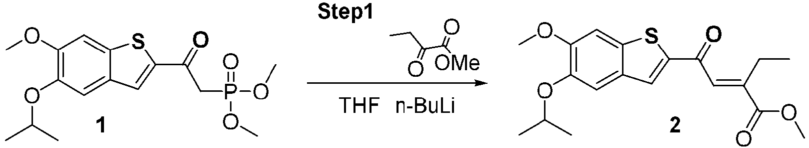

Dimethyl (2-(5-isopropoxy-6-methoxybenzo[b]thiophen-2-yl)-2-oxoethyl)phosphonate (5.6 g, 14.96 mmol) was dissolved in anhydrous tetrahydrofuran (50 mL). n-BuLi (6.5 mL, 16.46 mmol) was slowly added at -25°C under a nitrogen atmosphere and stirred for 1 hour. Methyl 2-oxobutanoate (1.73 g, 14.96 mmol) was then dissolved in anhydrous tetrahydrofuran (30 mL), slowly added dropwise to the system, and stirred for 30 minutes. The mixture was then returned to room temperature and stirred for 1 hour. The reaction solution was then added dropwise to saturated aqueous ammonium chloride solution (100 mL) and extracted three times with ethyl acetate (100 mL). The organic phase was washed with saturated brine, dried over anhydrous sodium sulfate, concentrated under reduced pressure, and purified on a silica gel column (EA:PE = 0 to 50%) to give the pure title compound 2 as a yellow oil (4.6 g, 84.38%): ESI MS [M + H]⁺ for C₁₉H₂₃O₅S, calcd 363.10, found 363.1.

### Step (II): Synthesis of methyl 2-ethyl-4-(5-isopropoxy-6-methoxybenzo[b]thiophen-2-yl)-4-oxobutanoate (Intermediate 3)

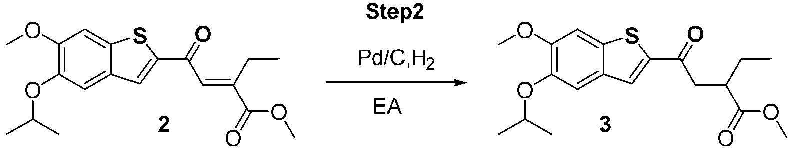

Methyl (E)-4-(5-isopropoxy-6-methoxybenzo[b]thiophen-2-yl)-2-methyl-4-oxobut-2-enoate (4.3 g, 11.86 mmol) was dissolved in ethyl acetate (60 mL). Wet Pd/C (10%, 126.9 mg, 1.19 mmol) was added, and a hydrogen balloon was connected. The mixture was purged with hydrogen and reacted at room temperature for 4 h. The reaction solution was then filtered. The filtrate was concentrated and then purified on a silica gel column (EA:PE = 0 to 30%) to give the pure title compound 3 as a white solid (2.1 g, 48.57%): ESI MS[M+H]⁺ for C₁₉H₂₅O₅S, calcd 365.12, found:365.1.

### Step (III): Synthesis of methyl 2-ethyl-4-(5-hydroxy-6-methoxybenzo[b]thiophen-2-yl)-4-oxobutanoate (Intermediate 4)

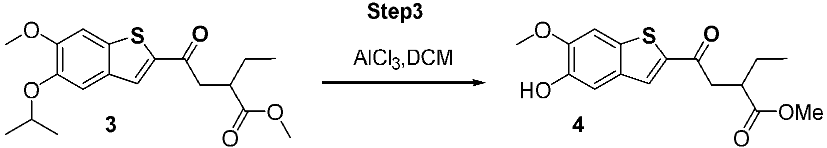

Methyl 2-ethyl-4-(5-isopropoxy-6-methoxybenzo[b]thiophen-2-yl)-4-oxobutanoate (2.1 g, 5.76 mmol) was dissolved in dichloromethane (30 mL), and aluminum trichloride (2.3 g, 17.29 mmol) was added in an ice bath. The mixture was then heated to 40°C and stirred for 4 h. The reaction solution was then added dropwise to a 0.5M HCl solution and extracted three times with ethyl acetate. The organic phase was washed with saturated brine, dried, concentrated, and purified on a silica gel column (EA:PE = 0 to 30%) to give the pure title compound 4 as a white solid (1.4 g, 75.39 %).

### Step (IV): Synthesis of 2-ethyl-4-(5-hydroxy-6-methoxybenzothiophen-2-yl)-4-oxobutanoic acid (Intermediate 5)

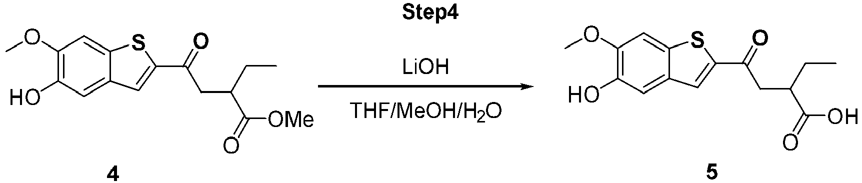

Methyl 2-ethyl-4-(5-hydroxy-6-methoxybenzo[b]thiophen-2-yl)-4-oxobutanoate (1.4 g, 4.34 mmol) was dissolved in a mixed solvent of tetrahydrofuran/water/methanol (2:1:1, 20 mL), and lithium hydroxide (208.7 mg, 8.69 mmol) was added. The reaction solution was stirred at room temperature for 18 hours. The reaction solution was then adjusted to pH 3, extracted with ethyl acetate, washed with saturated brine, dried, and concentrated to give the crude title compound 5 as a white solid (1.2 g, 89.61%).

### Step (V): Synthesis of 2-ethyl-4-(5-hydroxy-6-methoxy-4-nitrobenzo[b]thiophen-2-yl)-4-oxobutanoic acid (Intermediate 6)

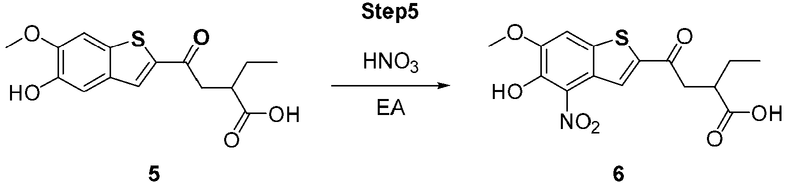

2-Ethyl-4-(5-hydroxy-6-methoxybenzothiophen-2-yl)-4-oxobutanoic acid (1.1 g, 3.57 mmol) was dissolved in ethyl acetate (80 mL), and concentrated nitric acid (674 mg, 10.7 mmol) was added in an ice bath. The mixture was stirred at room temperature for 1.5 hours. TLC indicated that the starting material had completely reacted. The reaction solution was immediately poured into water and extracted three times with ethyl acetate (100 mL). The organic phase was washed three times with saturated brine and concentrated at room temperature to give the crude title compound 6 as a brown solid (1.26 g, 100%).

### Step (VI): Synthesis of 4-(4-amino-5-hydroxy-6-methoxybenzo[b]thiophen-2-yl)-2-ethyl-4-oxobutanoic acid (Intermediate 6)

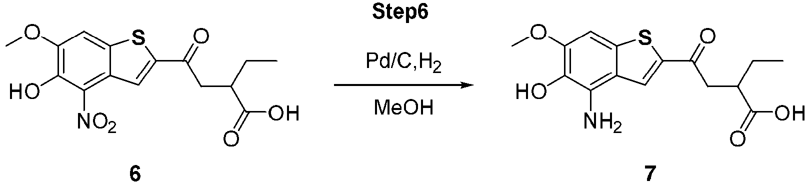

2-Ethyl-4-(5-hydroxy-6-methoxy-4-nitrobenzo[b]thiophen-2-yl)-4-oxobutanoic acid (1.26 g, 3.57 mmol) was dissolved in methanol (10 mL). Wet Pd/C (10%, 75.9 mg, 0.71 mmol) was added, and a hydrogen balloon was connected. The mixture was purged with hydrogen and reacted at room temperature for 1 h. The reaction solution was then filtered, and the filtrate was concentrated to give the crude title compound 7 as a brown solid (1.15 g, 100%): ESI MS [M + H]⁺ for C₁₅H₁₈NO₅S, calcd 324.08, found 324.1.

### Step (VII): Synthesis of 2-ethyl-4-(4-methoxy-2-methylthieno[2',3':5,6]benzo[1,2-d]oxazol-7-yl)-4-oxobutanoic acid (Example Compound 49)

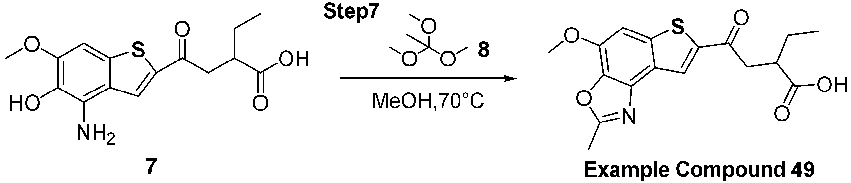

4-(4-Amino-5-hydroxy-6-methoxybenzo[b]thiophen-2-yl)-2-ethyl-4-oxobutanoic acid (104 mg, 0.32 mmol) was dissolved in methanol (10 mL), and trimethyl orthoacetate (384 mg, 3.2 mmol) was added. The mixture was reacted at 70°C in a sealed tube for 7 hours. The reaction solution was then filtered, and the filtrate was washed with methanol to give the product as a white solid (50.9 mg, 45.79%): ESI MS [M + H]⁺ for C₁₇H_{1S}NO₅S, calcd 348.08, found 348.1. ¹H NMR (400 MHz, DMSO-*d*₆) δ 12.17 (s, 1H), 8.55 (s, 1H), 7.69 (s, 1H), 4.04 (s, 3H), 3.48 (d, J = 9.7 Hz, 1H), 3.17 (dd, J = 17.6, 4.3 Hz, 1H), 2.79 (m, 1H), 2.71 (s, 3H), 1.64 (td, J = 7.1, 3.9 Hz, 2H), 0.94, (t, J = 7.4 Hz, 3H).

### Example 50

### Synthesis of (R)-2-ethyl-4-(4-methoxythieno[2',3':5,6]benzo[1,2-d]oxazol-7-yl)-4-oxobutanoic acid (Example Compound 50)

### Example 51

### Synthesis of (S)-2-ethyl-4-(4-methoxythieno[2',3':5,6]benzo[1,2-d]oxazol-7-yl)-4-oxobutanoic acid (Example Compound 51)

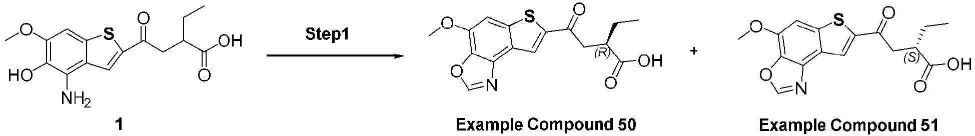

### Step (I): Synthesis of (R)-2-ethyl-4-(4-methoxythieno[2',3':5,6]benzo[1,2-d]oxazol-7-yl)-4-oxobutanoic acid and (S)-2-ethyl-4-(4-methoxythieno[2',3':5,6]benzo[1,2-d]oxazol-7-yl)-4-oxobutanoic acid (Example Compound 50 and Example Compound 51)

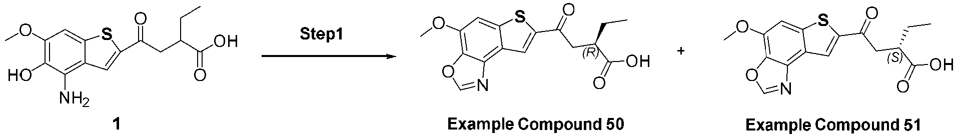

4-(4-Amino-5-hydroxy-6-methoxybenzo[b]thiophen-2-yl)-2-ethyl-4-oxobutanoic acid (1153.54 mg, 3.57 mmol) was dissolved in methanol (10 mL), and trimethoxymethane (3788.48 mg, 35.7 mmol) was added. The mixture was reacted at 70°C in a sealed tube for 7 hours. The reaction solution was then filtered to give 475 mg of solid, of which 300 mg was resolved by chiral column (column: CHIRALAPK IC-3 (IC30CE-KJ008), mobile phase: DCM/IPA/HAc = 80/20/0.1 (V/V/V)) to give the product Example Compound 50 (114.1 mg, 9.59%): R.T=4.269 min, ESI MS [M + H]⁺ for C₁₆H₁₆NO₅S, calcd 334.05, found 334.1. ¹H NMR (400 MHz, DMSO-*d*₆) δ 12.18 (s, 1H), 8.93 (s, 1H), 8.69 - 8.56 (m, 1H), 7.78 (s, 1H), 4.07 (s, 3H), 3.54 (dd, J = 17.7, 9.7 Hz, 1H), 3.20 (dd, J = 17.6, 4.3 Hz, 1H), 2.85 - 2.74 (m, 1H), 1.64 (pd, J = 7.0, 3.8 Hz, 2H), 0.95 (t, J = 7.4 Hz, 3H) and Example Compound 51 (111.8 mg, 9.39%): R.T=5.623 min, ESI MS [M + H]⁺ for C₁₆H₁₆NO₅S, calcd 334.05, found 334.1. ¹H NMR (400 MHz, DMSO-*d*₆) δ 12.18 (s, 1H), 8.92 (s, 1H), 8.61 (s, 1H), 7.78 (s, 1H), 4.07 (s, 3H), 3.54 (dd, J = 17.7, 9.7 Hz, 1H), 3.26 - 3.15 (m, 1H), 2.86 - 2.75 (m, 1H), 1.64 (pd, J = 7.1, 3.7 Hz, 2H), 0.95(t, J = 7.4 Hz, 1H) as white solids.

### Example 52

### Synthesis of (R)-2-ethyl-4-(4-methoxy-2-methylthieno[2,3:5,6]benzo[1,2-d]oxazol-7-yl)-4-oxobutanoic acid (Example Compound 52)

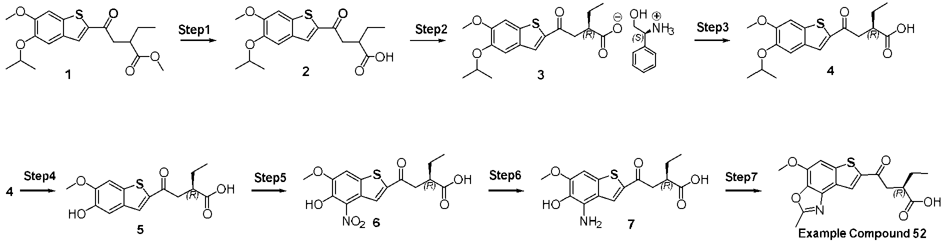

### Step (I): Synthesis of 2-ethyl-4-(5-isopropoxy-6-methoxybenzo[b]thiophen-2-yl)-4-oxobutanoic acid (Intermediate 2)

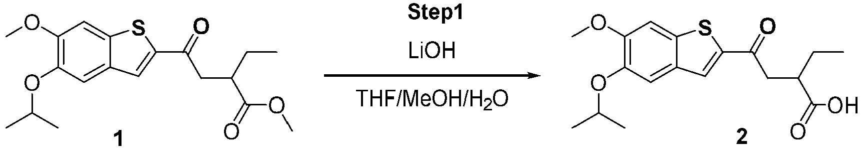

Methyl 2-ethyl-4-(5-isopropoxy-6-methoxybenzo[b]thiophen-2-yl)-4-oxobutanoate (4.8 g, 13.17 mmol) was dissolved in a mixed solvent of tetrahydrofuran/water/methanol (2:1:1, 20 mL), and lithium hydroxide (630 mg, 26.34 mmol) was added. The reaction solution was stirred at room temperature for 2 hours. The reaction solution was then adjusted to pH 3, extracted three times with ethyl acetate (100 mL), washed with saturated brine, dried, and concentrated to give crude Compound 2 as a yellow solid (4.6 g, 99.67%).

### Step (II): Synthesis of (S)-2-hydroxy-1-phenylethyl-1-amine (R)-2-ethyl-4-(5-isopropoxy-6-methoxybenzo[b]thiophen-2-yl)-4-oxobutanoate (Intermediate 3)

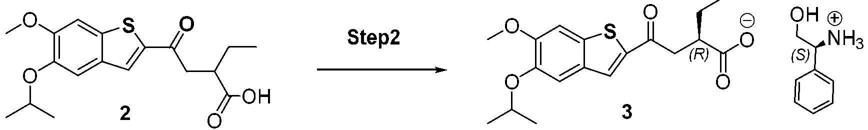

2-Ethyl-4-(5-isopropoxy-6-methoxybenzo[b]thiophen-2-yl)-4-oxobutanoic acid (2.6 g, 7.42 mmol) was dissolved in ethyl acetate (50 mL), and (S)-2-hydroxy-1-phenylethyl-1-amine (1.01 g, 7.42 mmol) was added. The mixture was stirred at room temperature overnight. A large amount of solid precipitated from the reaction solution, which was filtered to give 1.2 g of a white crude solid (89% purity). 1.2 g of the crude solid was added to ethyl acetate (50 mL). The mixture was heated to 60°C for dissolution, and then returned to room temperature and stirred for 18 h. A large amount of solid precipitated again, which was filtered to give the title compound 3 as a white solid (1.2 g, 33.17%).

### Step (III): Synthesis of (R)-2-ethyl-4-(5-isopropoxy-6-methoxybenzo[b]thiophen-2-yl)-4-oxobutanoic acid (Intermediate 4)

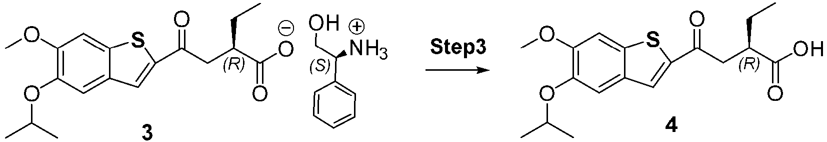

### (S)-2-Hydroxy-1-phenylethyl-1-amine

(R)-2-ethyl-4-(5-isopropoxy-6-methoxybenzo[b]thiophen-2-yl)-4-oxobutanoate (640 mg, 1.31 mmol) was dissolved in ethyl acetate (20 mL). 1M HCl (10 mL) was added and stirred for 1 h. The mixture was then extracted three times with ethyl acetate (50 mL). The organic phase was washed with saturated brine, dried over anhydrous sodium sulfate, and concentrated to give the crude title compound 4 as a white solid (450 mg, 97.84%).

### Step (IV): Synthesis of (R)-2-ethyl-4-(5-hydroxy-6-methoxybenzo[b]thiophen-2-yl)-4-oxobutanoic acid (Intermediate 5)

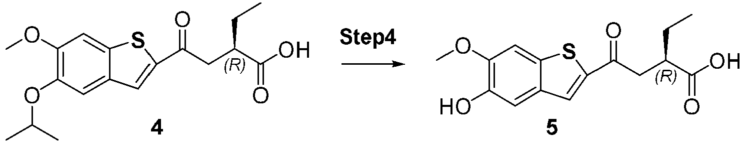

(R)-2-Ethyl-4-(5-isopropoxy-6-methoxybenzo[b]thiophen-2-yl)-4-oxobutanoic acid (450 mg, 1.28 mmol) was dissolved in a solution of dichloromethane (20 mL), and aluminum trichloride (513 mg, 3.85 mmol) was added in an ice bath. The mixture was then heated to 40°C and stirred for 2 h. The reaction solution was then added dropwise to a 0.5M HCl solution and extracted three times with ethyl acetate (100 mL). The organic phase was washed with saturated brine, dried, concentrated, and purified on a silica gel column (EA:PE = 0 to 30%) to give the pure title compound 5 as a white solid (380 mg, 95.97%).

### Step (V): Synthesis of (R)-2-ethyl-4-(5-hydroxy-6-methoxy-4-nitrobenzothiophen-2-yl)-4-oxobutanoic acid (Intermediate 6)

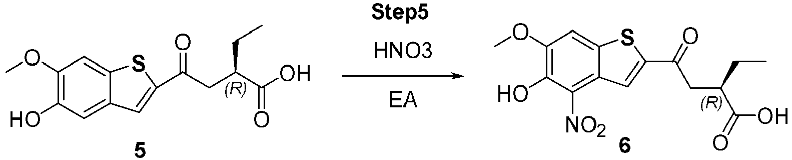

(R)-2-Ethyl-4-(5-hydroxy-6-methoxybenzo[b]thiophen-2-yl)-4-oxobutanoic acid (380 mg, 1.23 mmol) was dissolved in ethyl acetate (30 mL), and concentrated nitric acid (232 mg, 3.7 mmol) was added in an ice bath. The mixture was stirred at room temperature for 0.5 hour. TLC indicated that the starting material had completely reacted. The reaction solution was immediately poured into water and extracted three times with ethyl acetate (100 mL). The organic phase was washed three times with saturated brine and concentrated at room temperature to give the crude title compound 6 as a brown solid (435 mg, 100%).

### Step (VI): Synthesis of (R)-4-(4-amino-5-hydroxy-6-methoxybenzo[b]thiophen-2-yl)-2-ethyl-4-oxobutanoic acid (Intermediate 6)

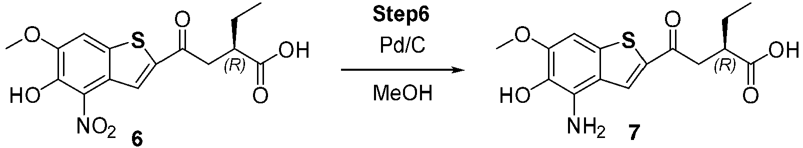

(R)-2-Ethyl-4-(5-hydroxy-6-methoxy-4-nitrobenzothiophen-2-yl)-4-oxobutanoic acid (435 mg, 1.28 mmol) was dissolved in methanol (5 mL). Wet Pd/C (10%, 27 mg, 0.26 mmol) was added, and a hydrogen balloon was connected. The mixture was purged with hydrogen and reacted at room temperature for 1 h. The reaction solution was then filtered, and the filtrate was concentrated to give the crude title compound 7 as a brown solid (396.57 mg, 100%): ESI MS [M + H]⁺ for C₁₅H₁₈NO₅S, calcd 324.08, found 324.1.

### Step (VII): Synthesis of (R)-2-ethyl-4-(4-methoxy-2-methylthieno[2,3:5,6]benzo[1,2-d]oxazol-7-yl)-4-oxobutanoic acid (Example Compound 52)

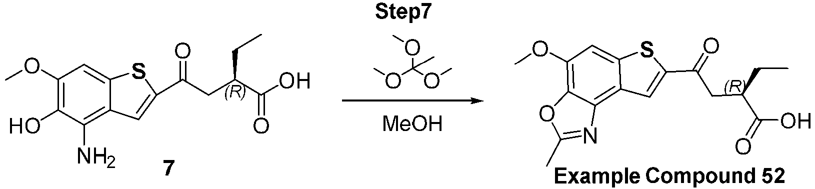

(R)-4-(4-Amino-5-hydroxy-6-methoxybenzo[b]thiophen-2-yl)-2-ethyl-4-oxobutanoic acid (396 mg, 1.22 mmol) was dissolved in methanol (10 mL), and triethyl orthoacetate (1.46 g, 12.2 mmol) was added. The mixture was reacted at 70°C in a sealed tube for 7 hours. The reaction solution was then cooled and filtered. The solid was washed with methanol (10 mL) to give the product Example Compound 52 as a white solid (151 mg, 35.63%): ESI MS [M + H]⁺ for C₁₇H₁₈NO₅S, calcd 348.08, found 348.1. ¹H NMR (400 MHz, DMSO-*d*₆)δ12.17 (s, 1H), 8.55 (s, 1H), 7.69 (s, 1H), 4.04 (s, 3H), 3.51 (dd, J = 17.6, 9.6 Hz, 1H), 3.17 (dd, J = 17.6, 4.3 Hz, 1H), 2.85 - 2.74 (m, 1H), 2.71 (s, 3H), 1.73 - 1.54 (m,2H), 0.94 (t, J = 7.4 Hz, 3H).

### Example 53

### Synthesis of (S)-2-ethyl-4-(4-methoxy-2-methylthieno[2',3':5,6]benzo[1,2-d]oxazol-7-yl)-4-oxobutanoic acid (Example Compound 53)

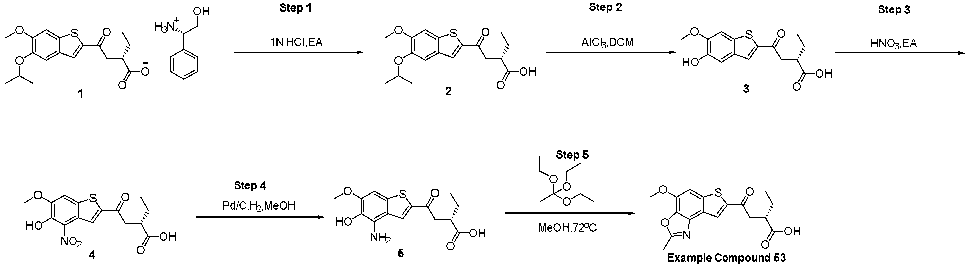

### Step (I): Synthesis of (S)-2-ethyl-4-(5-isopropoxy-6-methoxybenzo[b]thiophen-2-yl)-4-oxobutanoic acid (Intermediate 2)

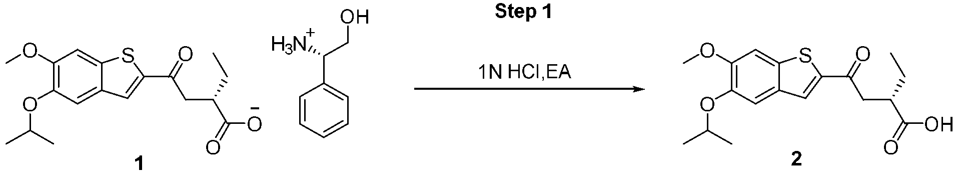

The (R)-phenylglycinol salt of the isomer (1.2 g, 2.46 mmol) was dissolved in 100 mL of EA, and 100 mL of 1N aqueous HCl solution was added. The mixture was stirred until completely dissolved, allowed to stand for liquid-liquid separation, and washed with 1N aqueous HCl solution (100 mL * 2). The aqueous phase was extracted with EA (100 mL * 1) and washed with 1N aqueous HCl solution (100 mL * 1). The EA phases were combined, washed once with brine, dried over anhydrous sodium sulfate, filtered, concentrated, and dried under vacuum to give 0.96 g of Intermediate 2 as a yellow oil. The crude product was used directly in the next step for deprotection, ESI MS[M + H]⁺ for C₁₈H₂₃O₅S, calcd 350.9, found: 350.9.

### Step (II): Synthesis of (S)-2-ethyl-4-(5-hydroxy-6-methoxybenzo[b]thiophen-2-yl)-4-oxobutanoic acid (Intermediate 3)

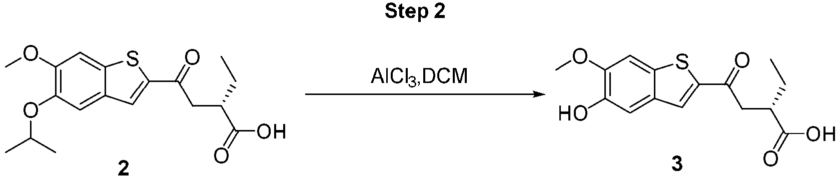

(S)-2-Ethyl-4-(5-hydroxy-6-methoxybenzo[b]thiophen-2-yl)-4-oxobutanoic acid (0.96 g, 2.74 mmol) was dissolved in 30 mL of anhydrous DCM and placed in an ice bath under a nitrogen atmosphere. Aluminum trichloride (1.1 g, 8.22 mmol) was added, and the mixture was placed in an ice bath for 10 min. The mixture was warmed to 40°C and reacted for 2 h. TLC monitoring indicated that the starting material had completely reacted. Subsequently, 50 mL of 1N aqueous HCl solution was placed in an ice bath. The reaction solution was slowly poured into the ice-bathed 1N aqueous HCl solution with stirring to quench and extracted with EA (100 mL * 3). The EA phases were combined, washed with 1N aqueous HCl solution (100 mL * 2), washed once with brine, dried over anhydrous sodium sulfate, filtered, concentrated, and dried under vacuum to give 0.8 g of an off-white solid. The solid was dissolved in 6 mL of DMF, filtered through a 0.22 µ m filter, and purified in 2 portions (column: C18, 10 µm, 30 * 250 mm; mobile phase: water (containing 0.1% TFA)/acetonitrile; procedure: 10% acetonitrile for 5 min, gradient from 10% to 95% acetonitrile over 30 min, 90% acetonitrile for 5 min, flow rate: 20 mL/min, 48%-57% acetonitrile) to give a product. The product was lyophilized to give 660 mg of Intermediate 3 as a white solid, yield: 78.2%; ESI MS[M + H]⁺ for C₁₅H₁₇O₅S, calcd 309.0, found: 308.9.

### Step (III): Synthesis of (S)-2-ethyl-4-(5-hydroxy-6-methoxy-4-nitrobenzo[b]thiophen-2-yl)-4-oxobutanoic acid (Intermediate 4)

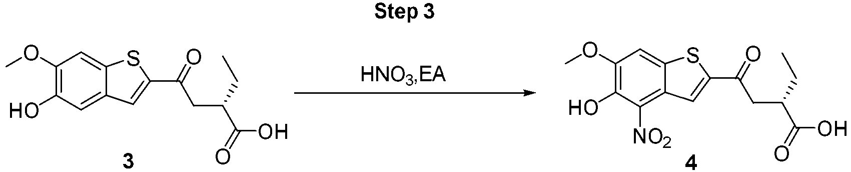

(S)-2-Ethyl-4-(5-hydroxy-6-methoxybenzo[b]thiophen-2-yl)-4-oxobutanoic acid (660 mg, 2.14 mmol) was dissolved in 120 mL of EA, and concentrated nitric acid (2.32 mL, 15.0 mmol) was added dropwise in an ice bath. The mixture was reacted at 0°C for 5 min, and the solution began to turn red. The reaction was continued for 10 min. A sample was collected for LCMS monitoring, which indicated that the starting material had completely reacted. The reaction mixture was washed three times with ice water, washed once with brine, and concentrated to give 700 mg of Intermediate 4 as a reddish-gray solid, yield: 92.6%; ESI MS[M + H]⁺ for C₁₅H₁₆NO₇S, calcd 354.0, found: 353.9.

### Step (IV): Synthesis of (S)-4-(4-amino-5-hydroxy-6-methoxybenzo[b]thiophen-2-yl)-2-ethyl-4-oxobutanoic acid (Intermediate 5)

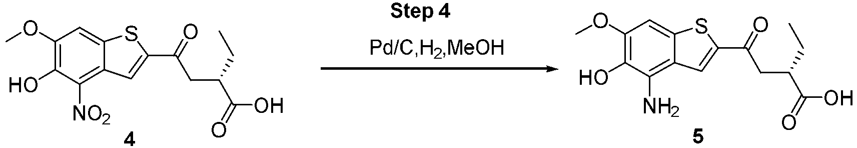

(S)-2-Ethyl-4-(5-hydroxy-6-methoxy-4-nitrobenzo[b]thiophen-2-yl)-4-oxobutanoic acid (700 mg, 1.98 mmol) was dissolved in 25 mL of methanol, and 200 mg of palladium on carbon was added. The mixture was subjected to catalytic hydrogenation at room temperature under a hydrogen atmosphere for 20 min. LCMS monitoring indicated that the starting material had completely reacted. ESI MS[M + H]⁺ for C₁₅H₁₈NO₅S, calcd 324.0, found: 323.9. The reaction mixture was filtered, and the filtrate was used directly in the next step.

### Step (V): Synthesis of (S)-2-ethyl-4-(4-methoxy-2-methylthieno[2',3':5,6]benzo[1,2-d]oxazol-7-yl)-4-oxobutanoic acid (Example Compound 53)

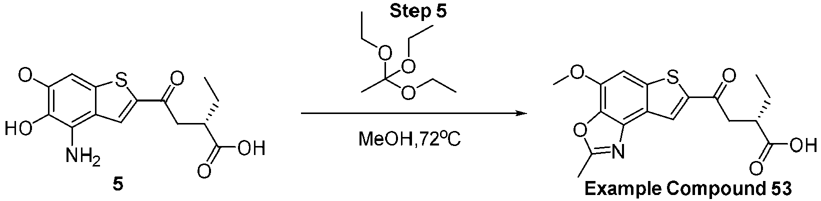

The filtrate of (S)-4-(4-amino-5-hydroxy-6-methoxybenzo[b]thiophen-2-yl)-2-ethyl-4-oxobutanoic acid was transferred to a 100 mL sealed tube under nitrogen protection, and triethyl orthoacetate (3.2 g, 19.8 mmol) was added. The mixture was reacted at 72°C overnight, and a white solid precipitated. The mixture was cooled, filtered, washed with a small amount of methanol, and dried under vacuum to give 306.8 mg of Example Compound 53 as a white solid. HPLC purity: 99.5%; ee value: 98.0%; ESI MS[M + H]⁺ for C₁₇H₁₈NO₅S, calcd 348.0, found: 347.9; ¹H NMR (400 MHz, DMSO) δ 12.18 (s, 1H), 8.55 (s, 1H), 7.68 (s, 1H), 4.04 (s, 3H), 3.51 (dd, J = 17.6, 9.7 Hz, 1H), 3.32 (s, 3H), 3.17 (dd, J = 17.6, 4.3 Hz, 1H), 2.84 - 2.74 (m, 1H), 2.70 (s, 3H), 1.70 - 1.55 (m, 2H), 0.94 (t, J = 7.4 Hz, 3H).

### Example 54

### Synthesis of 4-(2-amino-4-methoxythieno[2',3':5,6]benzo[1,2-d]oxazol-7-yl)-2-methyl-4-oxobutanoic acid (Example Compound 54)

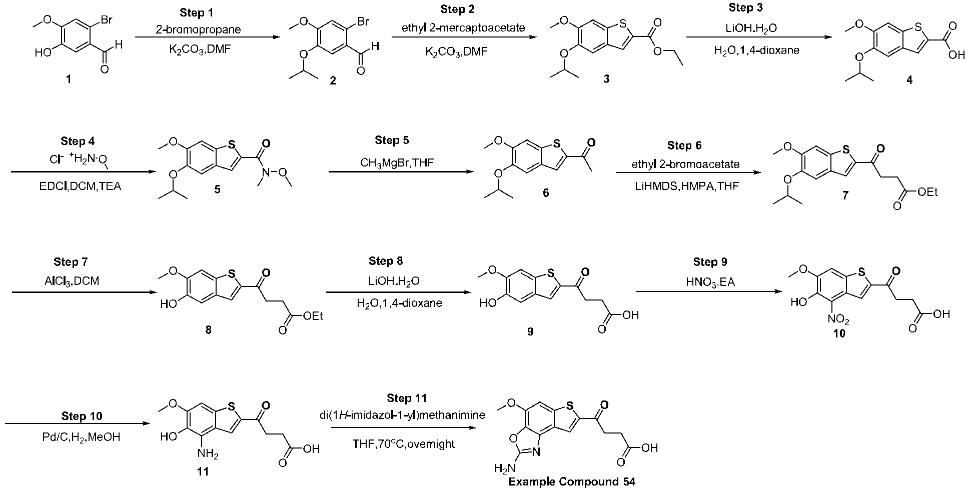

### Step (I): Synthesis of 2-bromo-5-isopropoxy-4-methoxybenzaldehyde (Intermediate 2)

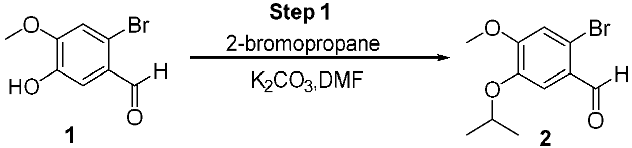

2-Bromo-5-hydroxy-4-methoxybenzaldehyde (23.1 g, 100 mmol) was dissolved in 200 mL of anhydrous DMF and placed in an ice bath under a nitrogen atmosphere. Potassium carbonate (22.1 g, 160 mmol) was added portionwise, and 2-bromopropane (18.40 g, 150 mmol) was added dropwise. The mixture was reacted at 30°C overnight. TLC monitoring indicated that a small amount of starting material had not completely reacted. An additional 2-bromopropane (10.0 g, 81.3 mmol) was added, and the mixture was reacted at 30°C for 3 h. A sample was collected for TLC monitoring, which indicated that the starting material had completely reacted. The reaction mixture was filtered, concentrated to remove DMF, diluted with EA, washed with water and brine, dried over anhydrous sodium sulfate, filtered, concentrated, and purified on a flash column using PE/EA as the mobile phase and 2%-10% EA to give a product. The product was concentrated to give 24.5 g of Intermediate 2 as a white solid, yield: 89.7%; ¹H NMR (400 MHz, CDCl₃) δ 10.10 (s, 1H), 7.35 (s, 1H), 6.98 (s, 1H), 4.55 (hept, J = 6.1 Hz, 1H), 3.86 (s, 3H), 1.31 (d, J = 6.1 Hz, 6H).

### Step (II): Synthesis of ethyl 5-isopropoxy-6-methoxybenzo[b]thiophene-2-carboxylate (Intermediate 3)

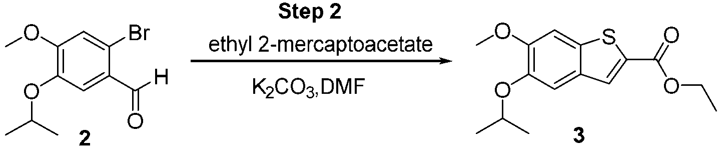

2-Bromo-5-isopropoxy-4-methoxybenzaldehyde (24.5 g, 89.70 mmol) was dissolved in 200 mL of anhydrous DMF and placed in an ice bath under a nitrogen atmosphere. Potassium carbonate (37.2 g, 269.11 mmol) was added portionwise, and ethyl 2-mercaptoacetate (16.2 g, 134.56 mmol) was added dropwise. The mixture was stirred at room temperature for 1 h, and the solution turned from light yellow to dark yellow. The reaction was carried out at 90°C for 5 h, and the solution turned red. TLC monitoring indicated that the starting material had completely reacted. The reaction mixture was filtered. The filtrate was concentrated to remove most of the DMF and dissolved and diluted with EA. The filter cake was washed with EA. The EA phases were combined, washed with water and brine, dried over anhydrous sodium sulfate, concentrated, and purified on a silica gel column using PE/DCM as the mobile phase and 10%-20% DCM to give a product. The product was concentrated and dried under vacuum to give 17.2 g of Intermediate 3 as a yellow solid, yield: 65.1%; ¹H NMR (400 MHz, CDCl₃) δ 7.93 (s, 1H), 7.29 (s, 1H), 7.27 (s, 1H), 4.60 (dt, J = 12.2, 6.1 Hz, 1H), 4.40 (q, J = 7.1 Hz, 2H), 3.96 (s, 3H), 1.44 - 1.40 (m, 9H).

### Step (III): Synthesis of 5-isopropoxy-6-methoxybenzo[b]thiophene-2-carboxylic acid (Intermediate 4)

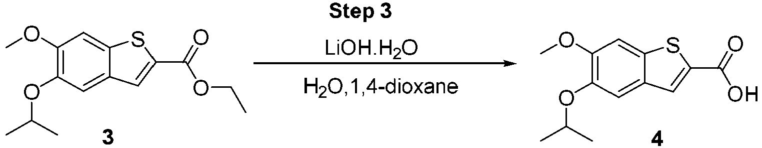

2-Bromo-5-isopropoxy-4-methoxybenzaldehyde (6.0 g, 19.46 mmol) was dissolved in 75 mL of 1,4-dioxane and 25 mL of water, and lithium hydroxide monohydrate (4.9 g, 116.76 mmol) was added. The mixture was reacted at 30°C overnight. TLC monitoring indicated that the starting material had completely reacted. The reaction mixture was concentrated to remove the organic solvent, diluted with water, and extracted with EA. The EA phase was washed with water. The aqueous phases were combined and adjusted to pH 4 with 1N HCl. A large amount of white solid precipitated. The solid was filtered, washed with water, and dried under vacuum to give 2.7 g of Intermediate 4 as a white solid, yield: 49.5%; ESI MS [M + H]⁺ for C₁₃H_{1S}O₄S, calcd 267.1, found 267.1; ¹H NMR (400 MHz, DMSO) δ 13.14 (s, 1H), 7.92 (s, 1H), 7.57 (s, 1H), 7.50 (s, 1H), 4.59 (dt, J = 12.1, 6.0 Hz, 1H), 3.84 (s, 3H), 1.30 (s, 3H), 1.29 (s, 3H).

### Step (IV): Synthesis of 5-isopropoxy-N,6-dimethoxy-N-methylbenzo[b]thiophene-2-carboxamide (Intermediate 5)

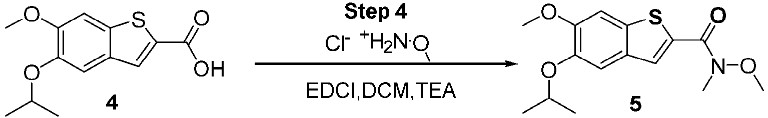

5-Isopropoxy-6-methoxybenzo[b]thiophene-2-carboxylic acid (2.7 g, 10.14 mmol) was dissolved in 100 mL of anhydrous DCM and placed in an ice bath under a nitrogen atmosphere. Dimethylhydroxylamine hydrochloride (1.2 g, 12.17 mmol) and EDCI (2.95 g, 15.21 mmol) were added, and TEA (2.56 g, 25.35 mmol) was added dropwise. The mixture was placed in an ice bath for 10 min and reacted at room temperature overnight. TLC monitoring indicated that the starting material had completely reacted. The reaction mixture was diluted with 100 mL of DCM, washed with water (100 mL * 4) and brine (100 mL * 1), dried over anhydrous sodium sulfate, filtered, concentrated, and purified on a silica gel column using PE/DCM as the mobile phase and 30%-100% DCM to give a product. The product was concentrated to give 2.0 g of Intermediate 5 as a yellow oil, yield: 63.7%.

### Step (V): Synthesis of 1-(5-isopropoxy-6-methoxybenzo[b]thiophen -2-yl)ethan-1-one (Intermediate 6)

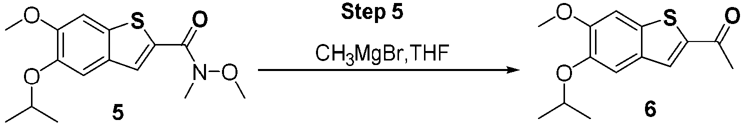

5-Isopropoxy-N,6-dimethoxy-N-methylbenzo[b]thiophene-2-carboxamide (2.0 g, 6.46 mmol) was dissolved in 20 mL of anhydrous THF and placed in an ice bath under a nitrogen atmosphere. Methylmagnesium bromide (6.5 mL, 3.0M/L in 2-methyl THF) was added dropwise, and the mixture was reacted in an ice bath for 1 h. TLC monitoring indicated that the starting material had completely reacted. The reaction was quenched by the addition of saturated aqueous ammonium chloride solution, and the reaction mixture was extracted with EA. The EA phases were combined, washed with water and brine, dried over anhydrous sodium sulfate, filtered, concentrated, and purified on a silica gel column using PE/DCM as the mobile phase and 30%-100% DCM to give a product. The product was concentrated and dried under vacuum to give 1.56 g of Intermediate 6 as a white solid, yield: 91.3%; ¹H NMR (400 MHz, CDCl₃) δ 7.82 (s, 1H), 7.31 (s, 1H), 7.28 (s, 1H), 4.60 (hept, J = 6.1 Hz, 1H), 3.97 (s, 3H), 2.64 (s, 3H), 1.45 (s, 3H), 1.43 (s, 3H).

### Step (VI): Synthesis of ethyl 4-(5-isopropoxy-6-methoxybenzo[b]thiophen-2-yl)-4-oxobutanoate (Intermediate 7)

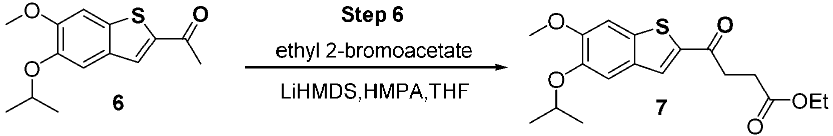

1-(5-Isopropoxy-6-methoxybenzo[b]thiophen-2-yl)ethan-1-one (1.2 g, 4.54 mmol) was dissolved in 40 mL of anhydrous THF, placed under a nitrogen atmosphere, and cooled to -78°C. HMPA (2.85 g, 15.89 mmol) was added dropwise, followed by LiHMDS (9.1 mL, 1.0M/L in THF), and the solution turned from colorless to yellow-brown. The mixture was warmed to -50°C, stirred for 1 h, and then cooled to -78°C. Ethyl bromoacetate (1.97g, 11.80 mmol) was added dropwise, and then the mixture was slowly warmed to room temperature and reacted overnight. The reaction was quenched by the addition of saturated aqueous ammonium chloride solution, and the reaction mixture was extracted with EA. The EA phases were combined, washed with water and brine, dried over anhydrous sodium sulfate, concentrated, and purified on a silica gel column using PE/EA as the mobile phase and 20% EA to give a product. The product was concentrated and dried under vacuum to give 1.2 g of Intermediate 7 as a yellow oil, yield: 75.4%; ESI MS [M + H]⁺ for C₁₈H₂₃O₅S, calcd 351.1, found 351.1.

### Step (VII): Synthesis of ethyl 4-(5-hydroxy-6-methoxybenzo[b]thiophen-2-yl)-4-oxobutanoate (Intermediate 8)

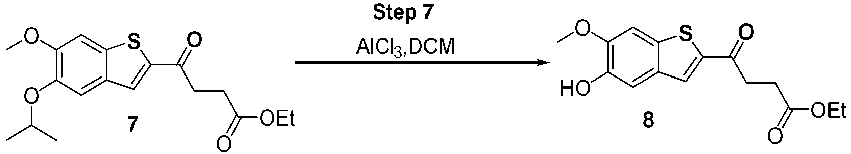

Ethyl 4-(5-isopropoxy-6-methoxybenzo[b]thiophen-2-yl)-4-oxobutanoate (800 mg, 2.28 mmol) was dissolved in 35 mL of anhydrous DCM and placed in an ice bath under a nitrogen atmosphere. Anhydrous aluminum trichloride (610 mg, 4.56 mmol) was added, and the mixture was slowly warmed to room temperature and reacted overnight. TLC monitoring indicated that the starting material had completely reacted. The reaction was quenched by the addition of water. The reaction mixture was extracted with EA, washed with water and brine, dried over anhydrous sodium sulfate, filtered, concentrated, and purified on a silica gel column using PE/EA as the mobile phase and 25%-30% EA to give a product. The product was concentrated and dried under vacuum to give 530 mg of Intermediate 8 as a light yellow solid, yield: 75.4%; ESI MS [M + H]⁺ for C₁₅H₁₇O₅S, calcd 309.1, found 309.1.

### Step (VIII): Synthesis of 4-(5-hydroxy-6-methoxybenzo[b]thiophen-2-yl)-4-oxobutanoic acid (Intermediate 9)

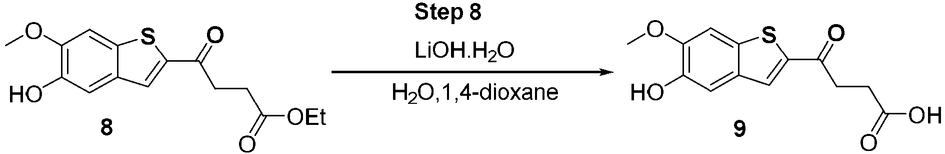

Ethyl 4-(5-hydroxy-6-methoxybenzo[b]thiophen-2-yl)-4-oxobutanoate (530 mg, 2.28 mmol) was dissolved in 30 mL of 1,4-dioxane and 30 mL of water, and lithium hydroxide monohydrate (0.72 g, 22.8 mmol) was added. The mixture was reacted at room temperature overnight. TLC monitoring indicated that the starting material had completely reacted. The reaction mixture was concentrated to remove the organic solvent, diluted with water, and adjusted to pH 4 with 1N HCl. A large amount of white solid precipitated. The solid was filtered, washed with water, and dried under vacuum to give 400 mg of Intermediate 9 as a white solid, yield: 83.0%; ESI MS [M + H]⁺ for C₁₃H₁₃O₅S, calcd 281.0, found 281.0.

### Step (IX): Synthesis of 4-(5-hydroxy-6-methoxy-4-nitrobenzo[b]thiophen-2-yl)-4-oxobutanoic acid (Intermediate 10)

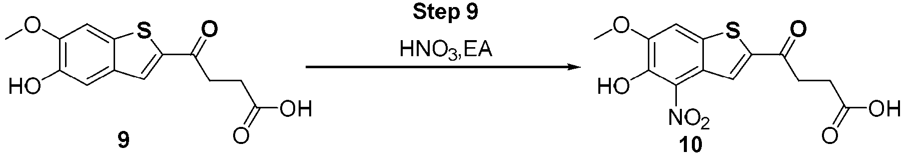

4-(5-Hydroxy-6-methoxybenzo[b]thiophen-2-yl)-4-oxobutanoic acid (400 mg, 1.43 mmol) was dissolved in 40 mL of EA, and concentrated nitric acid (2.2 mL, 14.3 mmol) was added dropwise in an ice bath. The mixture was warmed to room temperature (22°C) and reacted for 30 min to give a red suspension. LCMS monitoring indicated that the starting material had completely reacted. The reaction mixture was filtered, washed with a small amount of EA and a large amount of PE, and dried under vacuum to give 80 mg of Intermediate 10 as a yellow solid, yield: 17.2%; A sample was collected, ESI MS [M + H]⁺ for C₁₃H₁₂NO₇S, calcd 326.1, found 326.1, and the filter cake was used directly in the next step for direct reduction.

### Step (X): Synthesis of 4-(4-amino-5-hydroxy-6-methoxybenzo[b]thiophen-2-yl)-4-oxobutanoic acid (Intermediate 10)

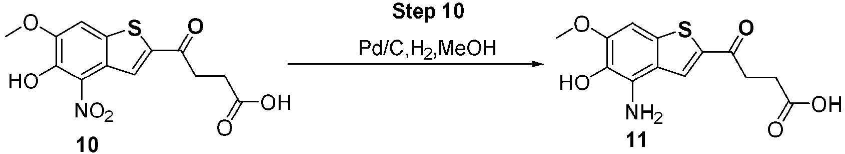

4-(5-Hydroxy-6-methoxy-4-nitrobenzo[b]thiophen-2-yl)-4-oxobutanoic acid (80 mg, 0.25 mmol) was dissolved in 10 mL of methanol, and 20 mg of palladium on carbon was added. The mixture was subjected to catalytic hydrogenation at room temperature under a hydrogen atmosphere for 40 min. LCMS monitoring indicated that the starting material had completely reacted, ESI MS [M + H]⁺ for C₁₃H₁₄NO₅S, calcd 296.1, found 296.1. The reaction mixture was filtered. The filtrate was concentrated at low temperature, dried under vacuum, dissolved in anhydrous THF, and then used directly in the next step.

### Step (XI): Ssynthesis of 4-(2-amino-4-methoxythieno[2',3':5,6]benzo[1,2-d]oxazol-7-yl)-2-methyl-4-oxobutanoic acid (Example Compound 54)

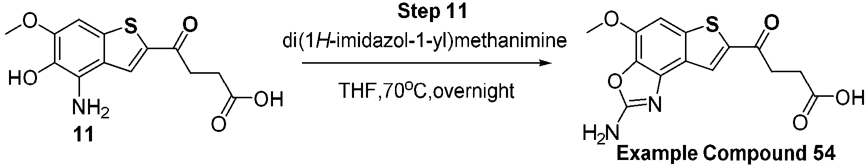

The THF filtrate of 4-(4-amino-5-hydroxy-6-methoxybenzo[b]thiophen-2-yl)-4-oxobutanoic acid was transferred to a 100 mL sealed tube under nitrogen protection, and di(1H-imidazol-1-yl)methanimine (196 mg, 1.22 mmol) was added. The mixture was reacted at room temperature for 2 h and at 70°C overnight. LCMS monitoring indicated that the starting material had completely reacted. The reaction mixture was concentrated, dissolved in DMF, filtered, and purified on a preparative column (column: C18, 10 µ m, 30 * 250 mm; mobile phase: water (containing 0.1% formic acid)/acetonitrile; procedure: 10% acetonitrile for 5 min, gradient from 10% to 90% acetonitrile over 30 min, 90% acetonitrile for 5 min, flow rate: 20 mL/min, 55% acetonitrile) to give a product. A solid precipitated from the prepared solution. The solid was filtered, washed with CH₃CN/H₂O (1:1), dissolved in CH₃CN/H₂O (1:1), and lyophilized to give 4.4 mg of Example Compound 54 as a light yellow solid, yield: 5.6%; ESI MS[M + H]⁺ for C₁₄H₁₃N₂O₅S, calcd 321.1, found: 321.1; ¹H NMR (400 MHz, DMSO-CD3OD) δ 8.29 (s, 1H), 7.29 (s, 1H), 4.01 (s, 3H), 3.38 - 3.28 (m, 2H), 2.71 - 2.63 (m, 2H).

### Example 55

### Synthesis of 4-(2-amino-4-methoxythieno[2',3':5,6]benzo[1,2-d]oxazol-7-yl)-2-methyl-4-oxobutanoic acid (Example Compound 55)

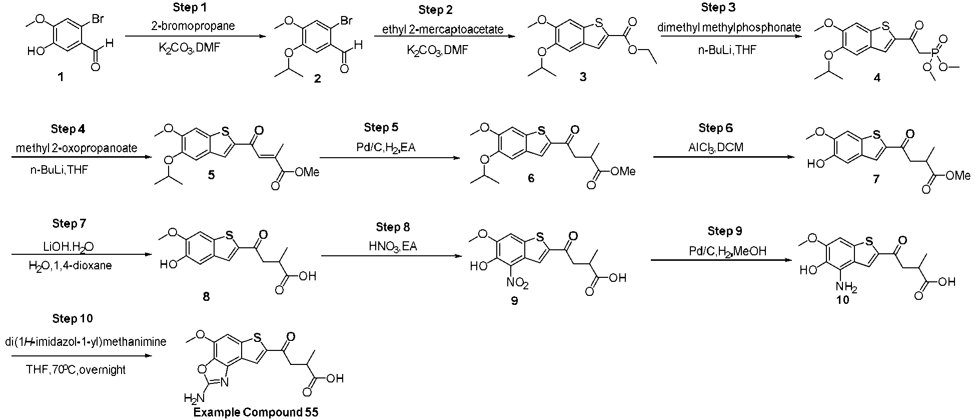

The first two steps refer to Example 54.

### Step (III): Synthesis of dimethyl 2-(5-isopropoxy-6-methoxybenzo[b]thiophen-2-yl)-2-oxoethyl)phosphonate (Intermediate 4)

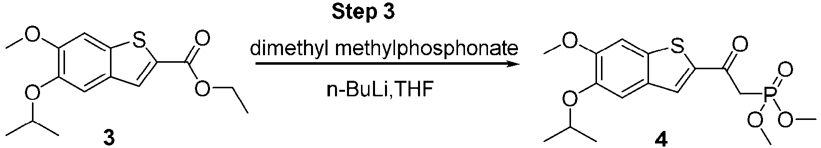

Dimethyl methylphosphonate (3.02 g, 24.32 mmol) was dissolved in 85 mL of anhydrous THF, placed under a nitrogen atmosphere, and cooled to -78°C. n-Butyllithium (10 mL, 2.5M/L in hexane) was added dropwise, and the mixture was reacted at -78°C for 1 h. A 15 mL solution of ethyl 5-isopropoxy-6-methoxybenzo[b]thiophene-2-carboxylate (3.58 g, 12.16 mmol) in anhydrous THF was added dropwise, and the mixture was reacted at -78°C for 1 h. TLC monitoring indicated that the starting material had completely reacted. The reaction was quenched with saturated aqueous ammonium chloride solution. The reaction mixture was extracted with EA, washed with brine, dried over anhydrous sodium sulfate, filtered, concentrated, and purified on a silica gel column using PE:EA as the mobile phase and 50%-100% EA to give 3.41 g of Intermediate 4 as a yellow solid, yield: 75.3%; ¹H NMR (400 MHz, CDCl₃) δ 7.96 (s, 1H), 7.32 (s, 1H), 7.26 (s, 1H), 4.65 - 4.54 (m, 1H), 3.97 (s, 3H), 3.84 (d, J = 3.1 Hz, 3H), 3.81 (d, J = 3.2 Hz, 3H), 3.67 (s, 1H), 3.61 (s, 1H), 1.45 (s, 3H), 1.43 (s, 3H).

### Step (IV): Synthesis of methyl 4-(5-isopropoxy-6-methoxybenzo[b]thiophen-2-yl)-2-methyl-4-oxobut-2-enoate (Intermediate 5)

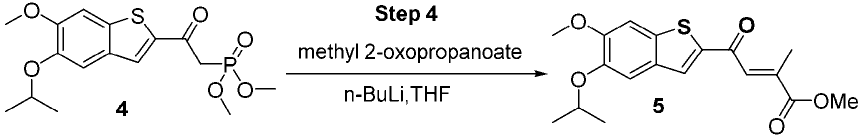

Dimethyl 2-(5-isopropoxy-6-methoxybenzo[b]thiophen-2-yl)-2-oxoethyl)phosphonate (2.5 g, 6.71 mmol) was dissolved in 60 mL of anhydrous THF, placed under a nitrogen atmosphere, and cooled to -20°C. n-Butyllithium (4.0 mL, 2.5M/L in hexane) was added dropwise, and the mixture was reacted at -20°C for 1 h. Methyl 2-oxopropanoate (0.96 g, 9.40 mmol) was added dropwise, and the mixture was slowly warmed to 0°C and reacted for 1 h. TLC monitoring indicated that the starting material had completely reacted. The reaction was quenched by the addition of water, and the reaction mixture was extracted with EA. The EA phases were combined, washed with brine, concentrated, and purified on a silica gel column using PE:EA as the mobile phase and 20% EA to give 1.06 g of Intermediate 5 as a yellow solid, yield: 45.3%: ESI MS[M + H]⁺ for C₁₈H₂₁O₅S, calcd 349.1, found: 349.1.

### Step (V): Synthesis of methyl 4-(5-isopropoxy-6-methoxybenzo[b]thiophen-2-yl)-2-methyl-4-oxobutanoate (Intermediate 6)

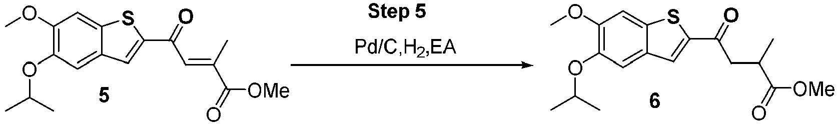

Methyl 4-(5-isopropoxy-6-methoxybenzo[b]thiophen-2-yl)-2-methyl-4-oxobut-2-enoate (1.06 g, 3.05 mmol) was dissolved in 60 mL of ethyl acetate, and Pd/C (160 mg) was added. The mixture was subjected to catalytic hydrogenation at 25°C under a hydrogen atmosphere for 4 h. The reaction mixture was filtered, washed with EA, combined, concentrated, and purified using PE/EA as the mobile phase and 15%-20% EA to give a product. The product was concentrated and dried under vacuum to give 460 mg of Intermediate 6 as an orange solid, yield: 43.4%.

### Step (VI): Synthesis of methyl 4-(5-hydroxy-6-methoxybenzo[b]thiophen-2-yl)-2-methyl-4-oxobutanoate (Intermediate 7)

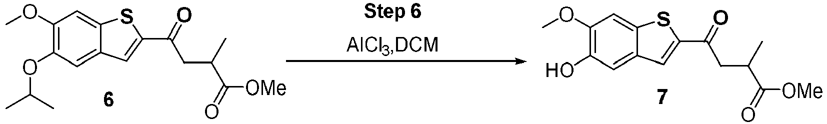

Methyl 4-(5-isopropoxy-6-methoxybenzo[b]thiophen-2-yl)-2-methyl-4-oxobutanoate (460 mg, 1.31 mmol) was dissolved in 15 mL of anhydrous DCM, and anhydrous aluminum trichloride (700 mg, 5.25 mmol) was added in an ice bath. The mixture was slowly warmed to room temperature and reacted overnight. TLC monitoring indicated that the starting material had completely reacted. The reaction was quenched by the addition of water. The reaction mixture was diluted with EA, washed with water and brine, dried over anhydrous sodium sulfate, concentrated, and purified on a silica gel column using PE/EA as the mobile phase and 25%-30% EA to give a product. The product was concentrated and dried under vacuum to give 280 mg of Intermediate 7 as a light yellow solid, yield: 69.3%, ESI MS[M + H]⁺ for C₁₅H₁₇O₅S, calcd 309.1, found: 309.1.

### Step (VII): Synthesis of 4-(5-hydroxy-6-methoxybenzo[b]thiophen-2-yl)-2-methyl-4-oxobutanoic acid (Intermediate 8)

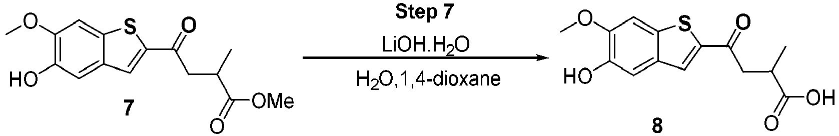

Methyl 4-(5-hydroxy-6-methoxybenzo[b]thiophen-2-yl)-2-methyl-4-oxobutanoate (280 mg, 0.91 mmol) was dissolved in 10 mL of 1,4-dioxane and 10 mL of water, and lithium hydroxide monohydrate (0.38 g, 9.10 mmol) was added. The mixture was reacted at 35°C for 5 h. TLC monitoring indicated that the starting material had completely reacted. The reaction mixture was concentrated to remove the organic solvent, diluted with water, and adjusted to pH 4 with 1N HCl. A large amount of white solid precipitated. The solid was filtered, washed with water, and dried under vacuum to give 240 mg of Intermediate 8 as a white solid, ESI MS[M + H]⁺ for C₁₄H₁₅O₅S, calcd 294.9, found: 294.9, yield: 89.6%.

### Step (VIII): Synthesis of 4-(5-hydroxy-6-methoxy-4-nitrobenzo[b]thiophen-2-yl)-2-methyl-4-oxobutanoic acid (Intermediate 9)

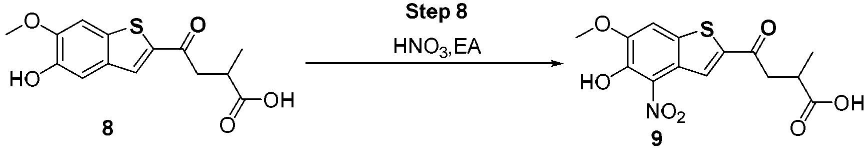

4-(5-Hydroxy-6-methoxybenzo[b]thiophen-2-yl)-2-methyl-4-oxobutanoic acid (250 mg, 0.82 mmol) was dissolved in 40 mL of EA, and concentrated nitric acid (1.3 mL, 8.2 mmol) was added dropwise in an ice bath. The mixture was warmed to room temperature and reacted for 1 h to give a clear red solution. LCMS monitoring indicated that the starting material had completely reacted. The reaction mixture was washed four times with ice water and once with brine, and concentrated at low temperature to give 250 mg of a gray-red solid. A sample was collected, ESI MS[M + H]⁺ for C₁₄H₁₄NO₇S, calcd 340.1, found: 340.1, yield: 67.7%, and used directly in the next step for reduction.

### Step (IX): Synthesis of 4-(4-amino-5-hydroxy-6-methoxybenzo[b]thiophen-2-yl)-2-methyl-4-oxobutanoic acid (Intermediate 10)

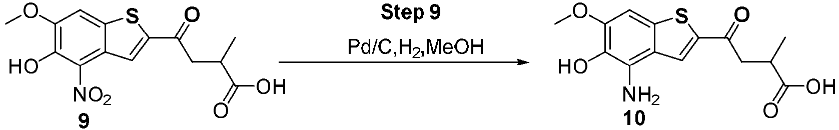

4-(5-Hydroxy-6-methoxy-4-nitrobenzo[b]thiophen-2-yl)-2-methyl-4-oxobutanoic acid (250 mg, 0.82 mmol) was dissolved in 10 mL of methanol, and 50 mg of palladium on carbon was added. The mixture was subjected to catalytic hydrogenation at room temperature under a hydrogen atmosphere for 35 min. LCMS monitoring indicated that the starting material had completely reacted, ESI MS[M + H]⁺ for C₁₄H₁₆NO₅S, calcd 340.1, found: 340.1. The reaction mixture was filtered. The filtrate was concentrated at low temperature, concentrated, dried under vacuum, dissolved in anhydrous THF, and then used directly in the next step.

### Step (X): Synthesis of 4-(2-amino-4-methoxythieno[2',3':5,6]benzo[1,2-d]oxazol-7-yl)-2-methyl-4-oxobutanoic acid (Example Compound 55)

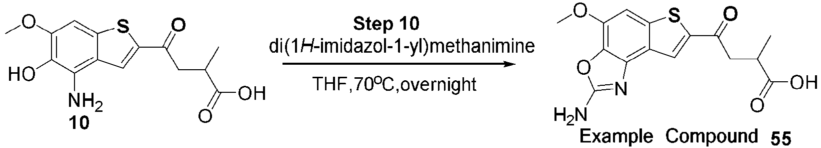

The THF filtrate of 4-(4-amino-5-hydroxy-6-methoxybenzo[b]thiophen-2-yl)-2-methyl-4-oxobutanoic acid was transferred to a 100 mL sealed tube under nitrogen protection, and di(1H-imidazol-1-yl)methanimine (281mg, 1.75 mmol) was added. The mixture was reacted at room temperature for 2 h and at 70°C overnight. The reaction mixture was cooled, concentrated, dissolved in DMF, filtered, and purified on a preparative column (column: C18, 10 µm, 30 * 250 mm; mobile phase: water (containing 0.1% formic acid)/acetonitrile; procedure: 10% acetonitrile for 5 min, gradient from 10% to 90% acetonitrile over 30 min, 90% acetonitrile for 5 min, flow rate: 20 mL/min, 55% acetonitrile) to give a product. A white solid precipitated from the prepared solution. The solid was filtered, washed with a small amount of solvent (water (containing 0.1% formic acid)/acetonitrile = 1:1), dissolved in a 1:1 mixed solvent of water (containing 0.1% formic acid)/acetonitrile, and lyophilized to give 8.2 mg of Example Compound 55 as a light yellow solid, yield: 8.1%; ESI MS[M + H]⁺ for C₁₅H₁₅N₂O₅S, calcd 335.1, found: 335.1; ¹H NMR (400 MHz, DMSO) δ 12.17 (s, 1H), 8.29 (s, 1H), 7.67 (s, 2H), 7.34 (s, 1H), 3.97 (s, 3H), 3.46 (dd, J = 17.4, 8.5 Hz, 1H), 3.13 (dd, J = 17.4, 5.1 Hz, 1H), 2.96 - 2.83 (m, 1H), 1.19 (d, J = 7.2 Hz, 3H).

### Example 56

### Synthesis of 4-(2-fluoro-4-methoxythieno[3,2-e]benzofuran-7-yl)-2-methyl-4-oxobutanoic acid (Example Compound 56)

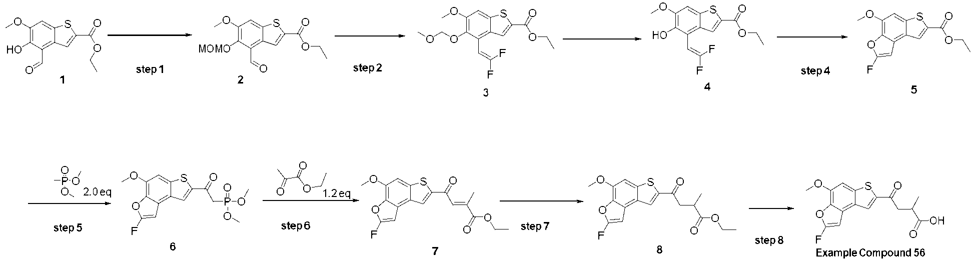

### Step (I): Synthesis of ethyl 4-formyl-6-methoxy-5-(methoxymethoxy)-1-benzothiophene-2-carboxylate (Intermediate 2)

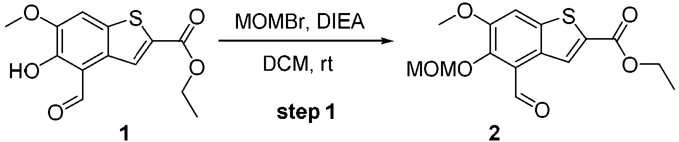

Ethyl 4-formyl-5-hydroxy-6-methoxy-1-benzothiophene-2-carboxylate (1 g, 3.57 mmol, 1.0 eq) was dissolved in DCM (10 mL), and bromo(methoxy)methane (892.2 mg, 7.14 mmol, 2.0 eq) and ethyl bis(propan-2-yl)amine (1384.2 mg, 10.71 mmol, 3.0 eq) were added to the above mixture. The reaction solution was stirred at room temperature for 6 h and then monitored by LCMS to confirm the formation of the desired product. The resulting mixture was extracted with DCM (2 * 30 mL), and the combined organic layer was dried over anhydrous Na₂SO₄. After filtration, the filtrate was concentrated under reduced pressure to give Compound 2 as a yellow solid (1 g, 86.9%, crude). ESI MS [M + H]⁺ for C₁₅H₁₇O₆S₃, calcd 325.3, found for 325.1.

### Step (II): Synthesis of ethyl 4-(2,2-difluorovinyl)-6-methoxy-5-(methoxymethoxy)-1-benzothiophene-2-carboxylate (Intermediate 3)

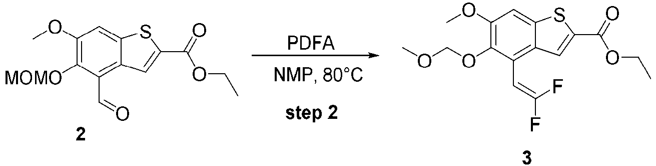

Ethyl 4-formyl-6-methoxy-5-(methoxymethoxy)-1-benzothiophene-2-carboxylate (1 g, 3.08 mmol, 1.0 eq) was dissolved in NMP (10 mL), and 2,2-difluoro-2-(triphenylphosphino)acetate (2.19 g, 6.16 mmol, 2.0 eq) was added to the above mixture. The reaction was stirred at 80°C for 4 hours, then cooled to room temperature, and monitored by LCMS. The resulting mixture was extracted with EA (2 * 30 mL), and the combined organic layer was dried over anhydrous Na₂SO₄. After filtration, the filtrate was concentrated under reduced pressure and purified by silica gel column chromatography (SiO₂, 5% EtOAc/Hex) to give Compound 3 as a yellow solid (800 mg, 72.7%). ESI MS [M + H]⁺ for C₁₆H₁₇F₂O₅S, calcd 359.4, found for 359.1.

### Step (III): Synthesis of ethyl 4-(2,2-difluorovinyl)-5-hydroxy-6-methoxy-1-benzothiophene-2-carboxylate (Intermediate 4)

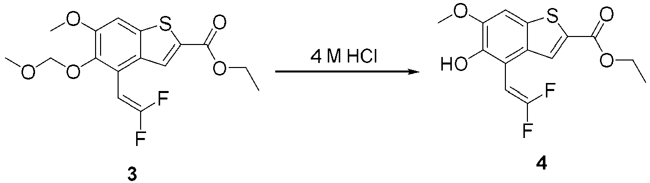

4-(2,2-Difluorovinyl)-6-methoxy-5-(methoxymethoxy)-1-benzothiophene-2-carboxylate (800 mg) was dissolved in 4M HCl (10 mL). The mixture was stirred at room temperature for 4 h and monitored by LCMS. The mixture was then concentrated under reduced pressure and purified by silica gel column chromatography (SiO₂, 20% EtOAc/Hex) to give Compound 4 as an off-white solid (400 mg, 56.8%). ESI MS [M + H]⁺ for C₁₄H₁₃F₂O₄S, calcd 315.3, found for 315.1.

### Step (IV): Synthesis of ethyl 2-fluoro-4-methoxythieno[3,2-E]benzofuran-7-carboxylate (Intermediate 5)

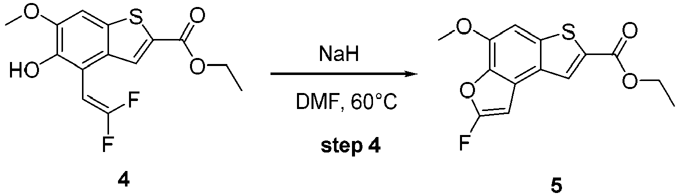

Ethyl 4-(2,2-difluorovinyl)-5-hydroxy-6-methoxy-1-benzothiophene-2-carboxylate (400 mg, 1.27 mmol, 1.0 eq) was dissolved in DMF (8 mL), and 60% NaH (63.3 mg, 1.65 mmol, 1.3 eq) was added to the above mixture. The reaction solution was stirred at 60°C for 1 h, then cooled to room temperature, and monitored by LCMS. The resulting mixture was extracted with EA (2 * 80 mL), and the combined organic layer was dried over anhydrous Na₂SO₄. After filtration, the filtrate was concentrated under reduced pressure and purified by silica gel column chromatography (SiO₂, 10% EtOAc/Hex) to give Compound 5 as a yellow solid (280 mg, 75%). ¹HNMR(300 MHz, DMSO) δ 8.39 (s, 1H), 7.64 (s, 1H), 6.89 (d,J = 6.5 Hz, 1H), 4.34 (q,J = 7.1 Hz, 2H), 4.00 (s, 3H), 1.32 (dd,J = 14.1, 7.0 Hz, 3H). ESI MS [M + H]⁺ for C₁₄H₁₂FO₄S, calcd 295.3, found for 295.1.

### Step (V): Synthesis of dimethyl (2-(2-fluoro-4-methoxythieno[3,2-e]benzofuran-7-yl)-2-oxoethyl)phosphonate (Intermediate 6)

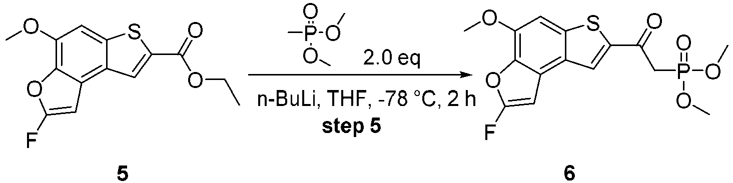

Ethyl 2-fluoro-4-methoxythieno[3,2-E]benzofuran-7-carboxylate (280 mg, 0.95 mmol, 1.0 eq) and dimethyl methylphosphonate (236.1 mg, 1.9 mmol, 2.0 eq) were dissolved in THF (5 mL) and cooled to -78°C. Then, 2M/L n-butyllithium (0.95 ml, 1.9 mmol, 2.0 eq) was added dropwise. The reaction mixture was stirred at -78°C for 0.5 hour and at room temperature for 0.5 hour. The reaction mixture was quenched into ice water (10 mL) and then diluted with EtOAc (10 mL). The resulting mixture was extracted with EtOAc (10 mL). The organic phase was washed three times with brine (20 mL), dried over Na₂SO₄, and filtered. The filtrate was concentrated under vacuum, and the crude product was further purified by column chromatography (SiO₂, 10% EtOAc/Hex) to give Compound 6 as a yellow solid (200 mg, 56.3%), ESI MS [M + H]⁺ for C₁₅H₁₄FO₆PS, calcd 373.3, found for 373.0.

### Step (VI): Synthesis of dimethyl (2-(2-fluoro-4-methoxythieno[3,2-e]benzofuran-7-yl)-2-oxoethyl)phosphonate (Intermediate 7)

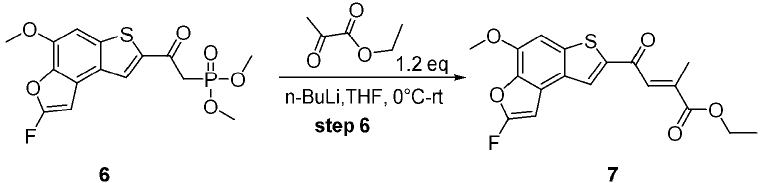

Dimethyl (2-(2-fluoro-4-methoxythieno[3,2-e]benzofuran-7-yl)-2-oxoethyl)phosphonate (400 mg, 1.07 mmol, 1.0 eq) was dissolved in THF (8 mL), and 2M/L n-butyllithium (0.65 mL, 1.28 mmol, 1.2 eq) was added to the mixture at 0°C. The mixture was stirred for 30 minutes, and then ethyl 2-oxopropanoate (149.1 mg, 1.28 mmol, 1.2 eq) was added to the mixture. The reaction was stirred at room temperature for 4 hours. The resulting mixture was extracted with EA (2 * 50 mL), and the combined organic layer was dried over anhydrous Na₂SO₄. After filtration, the filtrate was concentrated under reduced pressure and purified by silica gel column chromatography (SiO₂, 20% EtOAc/Hex) to give Compound 7 as a yellow solid (220 mg, 56.5%): ESI MS [M + H]⁺ for C₁₈H₁₆FO₅S, calcd 363.4, found for 363.1.

### Step (VII): Synthesis of ethyl 4-(2-fluoro-4-methoxythieno[3,2-E]benzofuran-7-yl)-2-methyl-4-oxobutanoate (Intermediate 8)

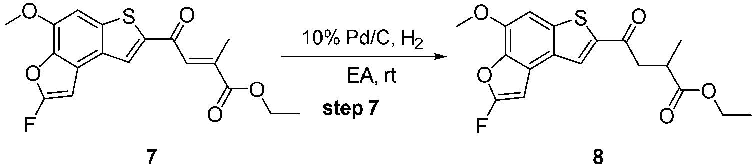

Dimethyl (2-(2-fluoro-4-methoxythieno[3,2-e]benzofuran-7-yl)-2-oxoethyl)phosphonate (220 mg, 0.61 mmol, 1.0 eq) was dissolved in ethanol (5 mL), and 10% palladium hydroxide (130 mg, 0.12 mmol, 0.2 eq) was added to the above mixture under H₂. The reaction was stirred at 60°C for 2 hours, and the mixture was monitored by LCMS. The resulting mixture was filtered, concentrated under reduced pressure, and purified by silica gel column chromatography (SiO₂, 10% EtOAc/Hex) to give Compound 8 as a yellow solid (90 mg, 40.7%). ESI MS [M + H]⁺ for C₁₈H₁₈FO₅S, calcd 365.4, found for 365.1.

### Step (VIII): Synthesis of 4-(2-fluoro-4-methoxythieno[3,2-e]benzofuran-7-yl)-2-methyl-4-oxobutanoic acid (Example Compound 56)

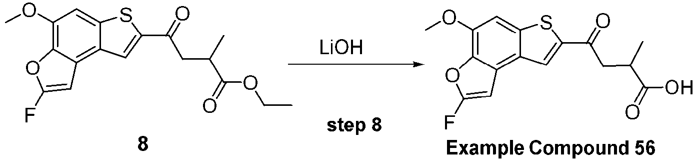

Ethyl 4-(2-fluoro-4-methoxythieno[3,2-E]benzofuran-7-yl)-2-methyl-4-oxobutanoate (90 mg, 0.25 mmol, 1.0 eq) and lithium hydroxide (60 mg, 2.5 mmol, 10 eq) were dissolved in THF:H₂O (5 mL, v:v = 4:1). The mixture was stirred at 50°C for 16 hours, then cooled to room temperature, monitored by LCMS, and adjusted to pH 2 with HCl. The resulting mixture was then extracted with EA (2 * 30 mL), and the combined organic layer was dried over anhydrous Na₂SO₄. After filtration, the filtrate was concentrated under reduced pressure to give the compound 4-(2-fluoro-4-methoxythieno[3,2-e]benzofuran-7-yl)-2-methyl-4-oxobutanoic acid as a yellow solid (48.3 mg, 58.2%). ¹H NMR (300 MHz, DMSO) δ 12.20 (s, 1H), 8.59 (s, 1H), 7.62 (s, 1H), 6.79 (d, J = 6.6 Hz, 1H), 4.01 (s, 3H), 3.44 (dd, J = 17.3, 8.4 Hz, 1H), 3.12 (dd, J = 17.3, 5.3 Hz, 1H), 2.92 (dd, J = 13.1, 7.6 Hz, 1H), 1.20 (d, J = 7.2 Hz, 3H). ESI MS [M + H]⁺ for C₁₆H₁₄FO₅S, calcd 337.3, found for 337.0.

### Example 57

### Synthesis of 2-ethyl-4-(2-fluoro-4-methoxythieno[3,2-e]benzofuran-7-yl)-4-oxobutanoic acid (Example Compound 57)

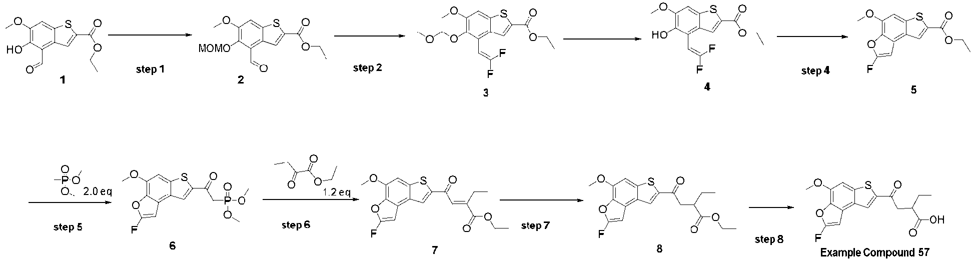

### Step (I): Synthesis of ethyl 4-formyl-6-methoxy-5-(methoxymethoxy)-1-benzothiophene-2-carboxylate (Intermediate 2)

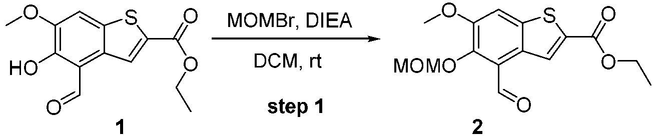

Ethyl 4-formyl-5-hydroxy-6-methoxy-1-benzothiophene-2-carboxylate (10 g, 35.7 mmol, 1.0 eq) was dissolved in DCM (100 mL), and bromo(methoxy)methane (8.9 g, 71.4 mmol, 2.0 eq) and ethyl bis(propan-2-yl)amine (13.8 g, 107.1 mmol, 3.0 eq) were added to the above mixture. The reaction solution was stirred at room temperature for 6 h and then monitored by LCMS to confirm the formation of the desired product. The resulting mixture was extracted with DCM (2 * 300 mL), and the combined organic layer was dried over anhydrous Na₂SO₄. After filtration, the filtrate was concentrated under reduced pressure to give Compound 2 (10 g, 86.4%, crude) as a yellow solid. ESI MS [M + H]⁺ for C₁₅H₁₇O₆S₃, calcd 325.3, found for 325.1.

### Step (II): Synthesis of ethyl 4-(2,2-difluorovinyl)-6-methoxy-5-(methoxymethoxy)-1-benzothiophene-2-carboxylate (Intermediate 3)

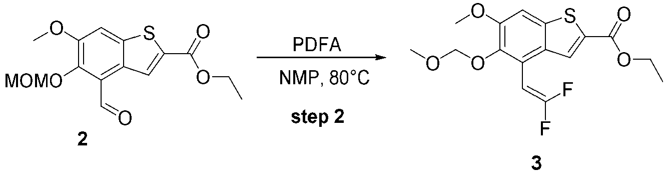

Ethyl 4-formyl-6-methoxy-5-(methoxymethoxy)-1-benzothiophene-2-carboxylate (10 g, 30.8 mmol, 1.0 eq) was dissolved in NMP (100 mL), and 2,2-difluoro-2-(triphenylphosphino)acetate (21.9 g, 61.6 mmol, 2.0 eq) was added to the above mixture. The reaction was stirred at 80°C for 4 hours, then cooled to room temperature, and monitored by LCMS. The resulting mixture was extracted with EA (2 * 300 mL), and the combined organic layer was dried over anhydrous Na₂SO₄. After filtration, the filtrate was concentrated under reduced pressure and purified by silica gel column chromatography (SiO₂, 5% EtOAc/Hex) to give Compound 3 as a yellow solid (7 g, 63.6%). ESI MS [M + H]⁺ for C₁₆H₁₇F₂O₅S, calcd 359.4, found for 359.1.

### Step (III): Synthesis of ethyl 4-(2,2-difluorovinyl)-5-hydroxy-6-methoxy-1-benzothiophene-2-carboxylate (Intermediate 4)

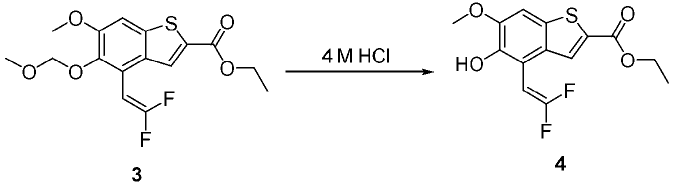

4-(2,2-Difluorovinyl)-6-methoxy-5-(methoxymethoxy)-1-benzothiophene-2-carboxylate (7 g) was dissolved in 4M HCl (100 mL). The mixture was stirred at room temperature for 4 h and monitored by LCMS. The mixture was then concentrated under reduced pressure and purified by silica gel column chromatography (SiO₂, 20% EtOAc/Hex) to give Compound 4 as an off-white solid (6 g, 97.8%). ESI MS [M + H]⁺ for C₁₄H₁₃F₂O₄S, calcd 315.3, found for 315.1.

### Step (IV): Synthesis of ethyl 2-fluoro-4-methoxythieno[3,2-E]benzofuran-7-carboxylate (Intermediate 5)

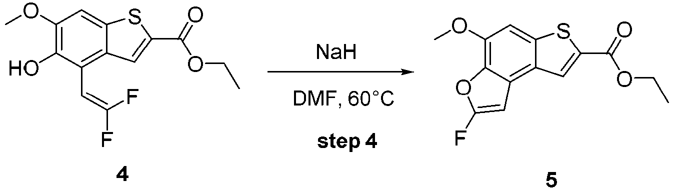

Ethyl 4-(2,2-difluorovinyl)-5-hydroxy-6-methoxy-1-benzothiophene-2-carboxylate (6 g, 19.0 mmol, 1.0 eq) was dissolved in DMF (60 mL), and 60% NaH (990.5 mg, 24.8 mmol, 1.3 eq) was added to the above mixture. The reaction solution was stirred at 60°C for 1 h, then cooled to room temperature, and monitored by LCMS. The resulting mixture was extracted with EA (2 * 500 mL), and the combined organic layer was dried over anhydrous Na₂SO₄. After filtration, the filtrate was concentrated under reduced pressure and purified by silica gel column chromatography (SiO₂, 10% EtOAc/Hex) to give Compound 5 as a yellow solid (5 g, 89.2%). ESI MS [M + H]⁺ for C₁₄H₁₂FO₄S, calcd 295.3, found for 295.1.

### Step (V): Synthesis of dimethyl (2-(2-fluoro-4-methoxythieno[3,2-e]benzofuran-7-yl)-2-oxoethyl)phosphonate (Intermediate 6)

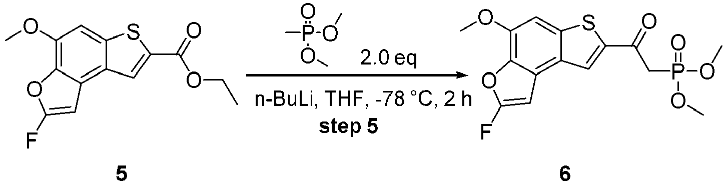

Ethyl 2-fluoro-4-methoxythieno[3,2-E]benzofuran-7-carboxylate (5 g, 17 mmol, 1.0 eq) and dimethyl methylphosphonate (4.2 g, 34 mmol, 2.0 eq) were dissolved in THF (50 mL) and cooled to -78°C. Then, 2M/L n-butyllithium (17 ml, 34 mmol, 2.0 eq) was added dropwise. The reaction mixture was stirred at -78°C for 0.5 hour and at room temperature for 0.5 hour. The reaction mixture was quenched into ice water (100 mL) and then diluted with EtOAc (100 mL). The resulting mixture was extracted with EtOAc (100 mL). The organic phase was washed three times with brine (200 mL), dried over Na₂SO₄, and filtered. The filtrate was concentrated under vacuum, and the crude product was further purified by column chromatography (SiO₂, 10% EtOAc/Hex) to give Compound 6 as a yellow solid (3.6 g, 57.1%), ESI MS [M + H]⁺ for C₁₅H₁₄FO₆PS, calcd 273.3, found for 373.0.

### Step (VI): Synthesis of ethyl 2-ethyl-4-(2-fluoro-4-methoxythieno[3,2-E]benzofuran-7-yl)-4-oxobut-2-enoate (Intermediate 7)

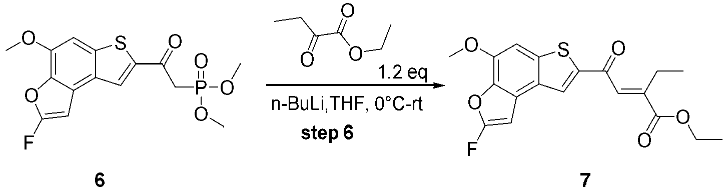

Dimethyl (2-(2-fluoro-4-methoxythieno[3,2-e]benzofuran-7-yl)-2-oxoethyl)phosphonate (3.4 g, 9.13 mmol, 1.0 eq) was dissolved in THF (20 mL), and 2M/L n-butyllithium (5.5 mL, 10.96 mmol, 1.2 eq) was added to the mixture at 0°C. The mixture was stirred for 30 minutes, and then ethyl 2-oxobutanoate (1.43 g, 10.96 mmol, 1.2 eq) was added to the mixture. The reaction was stirred at room temperature for 4 hours. The resulting mixture was extracted with EA (2 * 50 mL), and the combined organic layer was dried over anhydrous Na₂SO₄. After filtration, the filtrate was concentrated under reduced pressure and further purified by column chromatography (SiO₂, 15% EtOAc/Hex) to give Compound 7 as a yellow solid (2 g, 54.8%), ESI MS [M + H]⁺ for C₁₉H₁₇FO₅S, calcd 377.4, found for 377.1.

### Step (VII): Synthesis of ethyl 2-ethyl-4-(2-fluoro-4-methoxythieno[3,2-e]benzofuran-7-yl)-4-oxobutanoate (Intermediate 8)

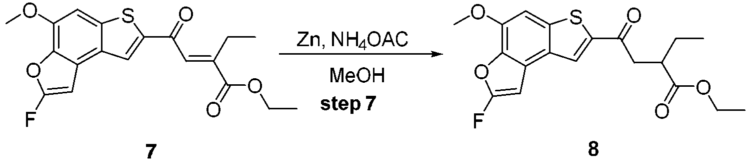

Ethyl 2-ethyl-4-(2-fluoro-4-methoxythieno[3,2-E]benzofuran-7-yl)-4-oxobut-2-enoate (2 g, 5.31 mmol, 1.0 eq) was dissolved in MeOH (8 mL), and ammonium acetate (1.33 g, 17.25 mmol, 5.0 eq) and zinc (676.7 mg, 10.35 mmol, 3.0 eq) were added to the above mixture. The reaction was stirred at room temperature for 4 h, and the mixture was monitored by LCMS. The resulting mixture was filtered, concentrated under reduced pressure, and further purified by column chromatography (SiO₂, 15% EtOAc/Hex) to give Compound 8 as a yellow solid (900 mg, 42.8%), ESI MS [M + H]⁺ for C₁₉H₁₉FO₅S, calcd 379.4, found for 379.1.

### Step (VIII): Synthesis of 2-ethyl-4-(2-fluoro-4-methoxythieno[3,2-e]benzofuran-7-yl)-4-oxobutanoic acid (Example Compound 57)

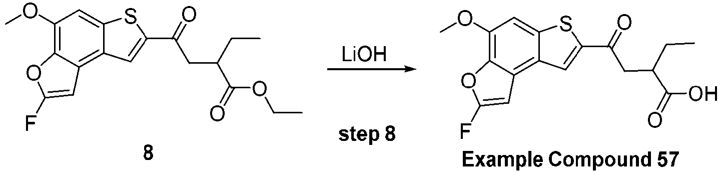

Ethyl 2-ethyl-4-(2-fluoro-4-methoxythieno[3,2-e]benzofuran-7-yl)-4-oxobutanoate (900 mg, 2.38 mmol, 1.0 eq) and lithium hydroxide (569.6 mg, 23.78 mmol, 10 eq) were dissolved in THF:H₂O (5 ml, v:v = 4:1). The mixture was stirred at 50°C for 16 h, then cooled to room temperature, and monitored by LCMS. The mixture was acidified to pH 2 with HCl and then extracted with EA (2 * 30 mL). The combined organic layer was dried over anhydrous Na₂SO₄. After filtration, the filtrate was concentrated under reduced pressure to give a yellow solid compound (458 mg, 55.0%). ¹H NMR (300 MHz, DMSO) *δ* 12.20 (s, 1H), 8.61 (s, 1H), 7.63 (s, 1H), 6.80 (d, J = 6.6 Hz, 1H), 4.01 (s, 3H), 3.44 (dd, J = 17.5, 9.4 Hz, 1H), 3.13 (dd, J = 17.5, 4.6 Hz, 1H), 2.89 - 2.76 (m, 1H), 1.62 (dd, J = 14.1, 7.0 Hz, 2H), 0.93 (t, J = 7.4 Hz, 3H).ESI MS [M + H]⁺ for C₁₇H₁₅FO₅S, calcd 351.4, found for 351.1.

### Example 58

### Synthesis of (S)-4-(4-methoxy-2-methylthieno[3,2-e]benzofuran-7-yl-1-D)-2-methyl-4-oxobutanoic acid (Example Compound 58)

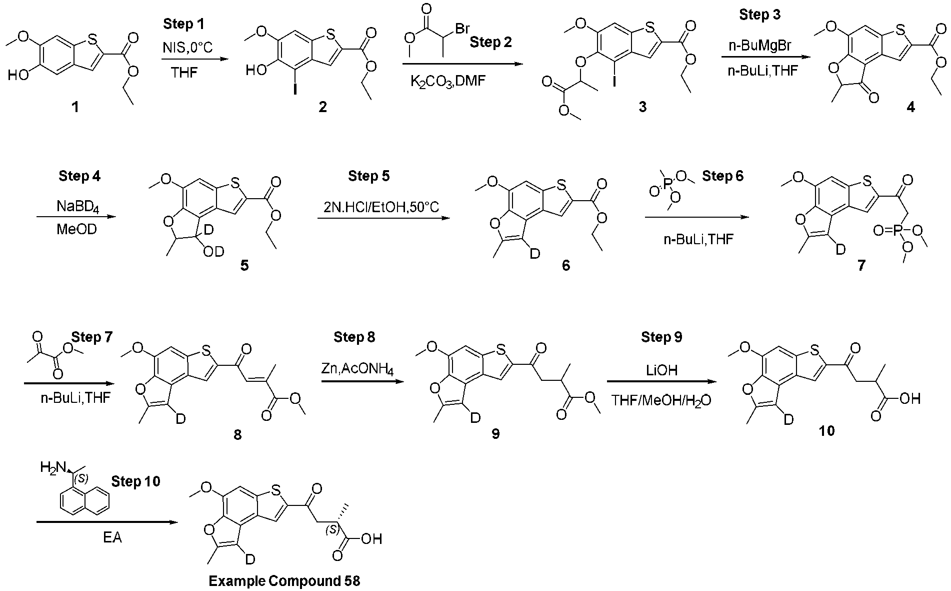

### Step (I): Synthesis of ethyl 5-hydroxy-4-iodo-6-methoxybenzo[b]thiophene-2-carboxylate (Intermediate 2)

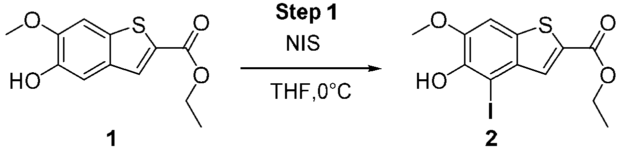

Ethyl 5-hydroxy-6-methoxybenzo[b]thiophene-2-carboxylate (1 g, 3.96 mmol, 1 equiv.) was dissolved in THF (20 mL), and N-iodosuccinimide (980.93 mg, 4.36 mmol, 1.1 equiv.) was added portionwise in an ice bath. The mixture was reacted at a maintained temperature for 2 h. TLC monitoring indicated that the starting material had completely reacted. The reaction was quenched by the addition of 5% aqueous sodium sulfite solution (20 mL), and the mixture was extracted with EA. The organic phase was washed with brine, dried over anhydrous sodium sulfate, concentrated, triturated with EA, and filtered. The filter cake was dried to give ethyl 5-hydroxy-4-iodo-6-methoxybenzo[b]thiophene-2-carboxylate (1.3 g, 3.44 mmol, 86.73%) as an off-white solid, ESI MS[M+H]⁺ for C₁₂H₁₁IO₄S, calcd 379.18, found: 378.9.

### Step (II): Synthesis of ethyl 4-iodo-6-methoxy-5-((1-methoxy-1-oxopropan-2-yl)oxy)benzo[b]thiophene-2-carboxylate (Intermediate 3)

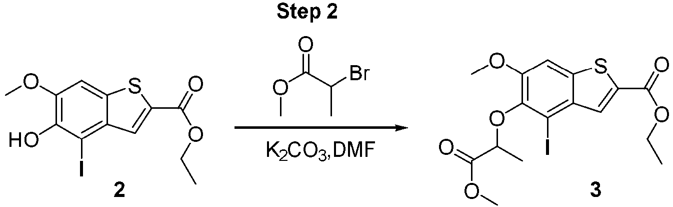

Ethyl 5-hydroxy-4-iodo-6-methoxybenzo[b]thiophene-2-carboxylate (1 g, 2.64 mmol, 1 equiv.) was dissolved in DMF (20 mL), and methyl 2-bromopropionate (574.07 mg, 3.44 mmol, 1.3 equiv.) and potassium carbonate (548.19 mg, 3.97 mmol, 2 equiv.) were added. The mixture was warmed to 80°C and reacted at a maintained temperature for 1.5 h. TLC monitoring indicated that the starting material had completely reacted. The reaction mixture was cooled to room temperature and filtered. The filtrate was diluted with water and extracted with EA. The organic phase was washed with brine, dried over anhydrous sodium sulfate, concentrated, and triturated with EA to give ethyl 4-iodo-6-methoxy-5-((1-methoxy-1-oxopropan-2-yl)oxy)benzo[b]thiophene-2-carboxylate (1.1 g, 2.37 mmol, 89.6%) as an off-white solid, ESI MS[M+H]⁺ for C₁₆H₁₇IO₆S, calcd 465.27, found: 464.9.

### Step (III): Synthesis of ethyl 4-methoxy-2-methyl-1-oxo-1,2-dihydrothieno [3,2-e] benzofuran-7-carboxylate (Intermediate 4)

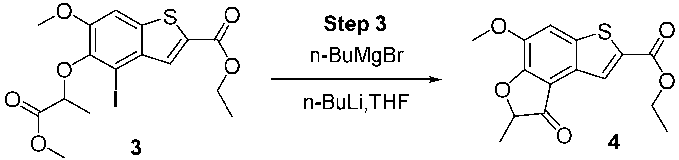

Under nitrogen protection at room temperature, n-butyllithium (0.47 mL, 2.5M, 1.18 mmol, 1.5 equiv.) was placed in an ice bath, and n-butylmagnesium bromide (1.18 mL, 1M, 1.18 mmol, 1.5 equiv.) was added dropwise. The mixture was reacted at a maintained temperature in an ice bath for 30 min and then cooled to -78°C. A solution of ethyl 4-iodo-6-methoxy-5-((1-methoxy-1-oxopropan-2-yl)oxy)benzo[b]thiophene-2-carboxylate (0.5 g, 1.08 mmol, equiv.)/THF (10 mL) was added dropwise, and the mixture was reacted at a maintained temperature for 0.5 h. LC-MS monitoring indicated partial remaining starting material. The reaction solution was added dropwise to saturated ammonium chloride to quench, extracted with EA, washed with saturated brine, dried, concentrated, and purified on a silica gel column (EA:PE = 0 to 20%) to give ethyl 4-methoxy-2-methyl-1-oxo-1,2-dihydrothieno[3,2-e]benzofuran-7-carboxylate (110 mg, 0.36 mmol, 33.34%) as a yellowish solid. ESI MS[M+H]⁺ for C₁₅H₁₄O₅S, calcd 307.33, found: 306.9.

### Step (IV): Synthesis of ethyl 1-hydroxy-D-4-methoxy-2-methyl-1,2-dihydrothieno[3,2-E]benzofuran-7-carboxylate-1-D

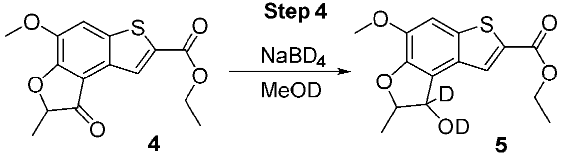

Ethyl 4-methoxy-2-methyl-1-oxo-1,2-dihydrothieno[3,2-e]benzofuran-7-carboxylate (1.5 g, 4.9 mmol, 1 equiv.) was dissolved in methanol-d (30 mL, 100.0%), and sodium deuterated borohydride (555.72 mg, 14.69 mmol, 3 equiv.) was added. The mixture was stirred for 1 h. LCMS indicated that the starting material had completely reacted. The reaction solution was quenched by the addition of 1 mL of acetone, concentrated, and purified on a silica gel column (EA:PE = 0 to 20%) to give ethyl 1-hydroxy-D-4-methoxy-2-methyl-1,2-dihydrothieno[3,2-E]benzofuran-7-carboxylate-1-D as a solid (1.4 g, 4.51 mmol, 92.06%). ESI MS[M+H]⁺ for C₁₅H₁₄D₂O₅S, calcd 311.36, found: 310.9.

### Step (V): Synthesis of ethyl 4-methoxy-2-methylthieno[3,2-E]benzofuran-7-carboxylate-1-D

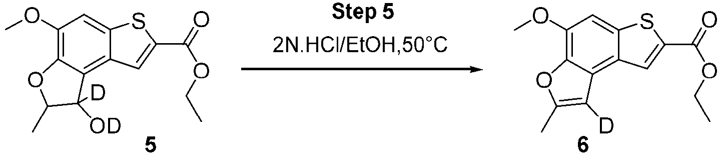

Ethyl 1-hydroxy-D-4-methoxy-2-methyl-1,2-dihydrothieno[3,2-E]benzofuran-7-carboxylate-1-D (1400 mg, 4.51 mmol, 1 equiv.) was dissolved in a solution of 2N HCl/EtOH (22.55 mL, 45.11 mmol, 10 equiv.), heated to 50°C, and stirred for 3 h. LCMS indicated that the starting material had completely reacted. The reaction solution was concentrated to give ethyl 4-methoxy-2-methylthieno[3,2-E]benzofuran-7-carboxylate-1-D (1200 mg, 4.12 mmol, 91.33%). ESI MS[M+H]⁺ for C₁₅H₁₃DO₄S, calcd 292.34, found: 291.9.

### Step (VI): Synthesis of dimethyl (2-(4-methoxy-2-methylthieno[3,2-e]benzofuran-7-yl-1-D)-2-oxoethyl)phosphonate

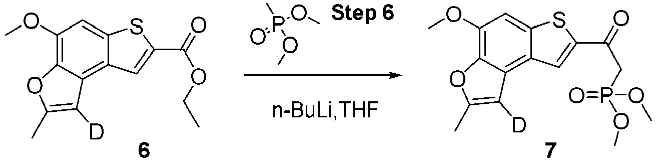

Dimethyl methylphosphonate (1022.15 mg, 8.24 mmol, 2 equiv.) was dissolved in tetrahydrofuran (30 mL, 100.0%), and then n-butyllithium (0.53 mL, 8.24 mmol, 2 equiv.) was slowly added dropwise at -70°C. The system was stirred at -70°C for 1 h. Ethyl 4-methoxy-2-methylthieno[3,2-E]benzofuran-7-carboxylate-1-D (1.2 g, 4.12 mmol, 1 equiv.) was then dissolved in THF and slowly added dropwise to the system. The mixture was then stirred for 1 hour. TLC indicated partial remaining starting material. The reaction solution was added dropwise to saturated ammonium chloride to quench, extracted with EA, washed with saturated brine, dried, concentrated, and purified on a silica gel column (EA:PE = 0 to 100%) to give dimethyl (2-(4-methoxy-2-methylthieno[3,2-e]benzofuran-7-yl-1-D)-2-oxoethyl)phosphonate as a solid (1300 mg, 3.52 mmol, 85.45%). ESI MS[M+H]⁺ for C₁₆H₁₆DO₆PS, calcd 370.35, found: 369.9.

### Step (VII): Synthesis of methyl 4-(4-methoxy-2-methylthieno[3,2-e]benzofuran-7-yl-1-D)-2-methyl-4-oxobut-2-enoate

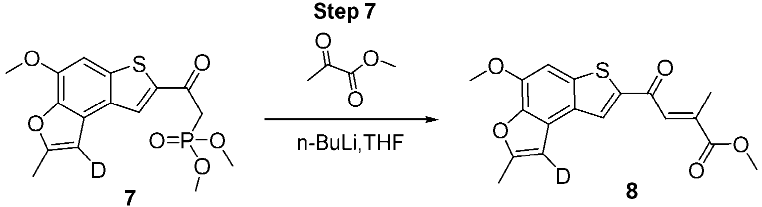

Dimethyl (2-(4-methoxy-2-methylthieno[3,2-e]benzofuran-7-yl-1-D)-2-oxoethyl)phosphonate (1300 mg, 3.52 mmol, 1 equiv.) was dissolved in tetrahydrofuran (30 mL, 100.0%), and then n-butyllithium (0.13 mL, 1.99 mmol, 2 equiv.) was slowly added dropwise at -25°C. The system was stirred at -25°C for 1 h. Methyl 2-oxopropanoate (538.99 mg, 5.28 mmol, 1.5 equiv.) was then dissolved in THF and slowly added dropwise to the system. The mixture was stirred for 0.5 hour. The mixture was then returned to room temperature and stirred for 1 hour. TLC indicated a small amount of starting material remaining. The reaction solution was added dropwise to saturated ammonium chloride to quench, extracted with EA, washed with saturated brine, dried, concentrated, and purified on a silica gel column (EA:PE = 0 to 20%) to give methyl 4-(4-methoxy-2-methylthieno[3,2-e]benzofuran-7-yl-1-D)-2-methyl-4-oxobut-2-enoate as a solid (1200 mg, 3.34 mmol, 94.86%), ESI MS[M+H]⁺ for C₁₈H₁₅DO₅S, calcd 346.39, found: 345.9.

### Step (VIII): Synthesis of methyl 4-(4-methoxy-2-methylthieno[3,2-e]benzofuran-7-yl-1-D)-2-methyl-4-oxobutanoate

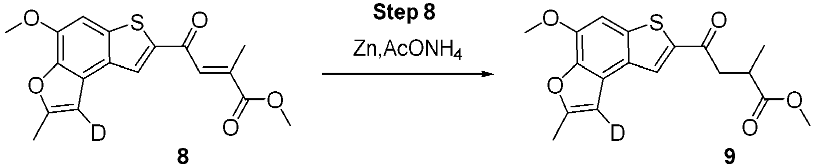

Methyl 4-(4-methoxy-2-methylthieno[3,2-e]benzofuran-7-yl-1-D)-2-methyl-4-oxobut-2-enoate (1200 mg, 3.47 mmol, 1 equiv.) was dissolved in methanol (30 mL, 83.33%) and water (6 mL, 16.67%), and zinc powder (1135.76 mg, 17.37 mmol, 5 equiv.) and ammonium acetate (1339.01 mg, 17.37 mmol, 5 equiv.) were added. The system was heated to 50°C and stirred for 1 h. TLC indicated that the starting material had completely reacted. The reaction solution was poured into water and extracted with EA. The organic phase was washed with brine, dried over anhydrous sodium sulfate, concentrated, and purified on a silica gel column (EA:PE = 0 to 20%) to give methyl 4-(4-methoxy-2-methylthieno[3,2-e]benzofuran-7-yl-1-D)-2-methyl-4-oxobutanoate as a solid (1200 mg, 3.45 mmol, 99.42%). ESI MS[M+H]⁺ for C₁₈H₁₇DO₅S, calcd 348.4, found: 347.9.

### Step (IX): Synthesis of 4-(4-methoxy-2-methylthieno[3,2-e]benzofuran-7-yl-1-D)-2-methyl-4-oxobutanoic acid

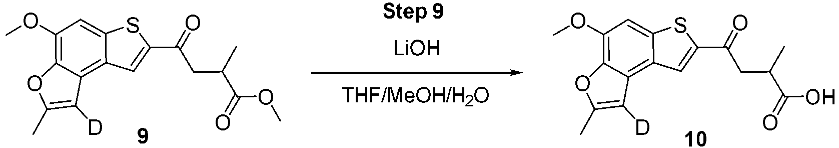

Methyl 4-(4-methoxy-2-methylthieno[3,2-e]benzofuran-7-yl-1-D)-2-methyl-4-oxobutanoate (1200 mg, 3.45 mmol, 1 equiv.) was dissolved in a mixed solution of tetrahydrofuran (10 mL, 50.0%), methanol (5 mL, 25.0%), and water (5 mL, 25.0%), and lithium hydroxide (165.46 mg, 6.91 mmol, 2 equiv.) was added. The mixture was stirred at room temperature for 1 h. TLC indicated that the starting material had completely reacted. The reaction solution was adjusted to pH = 7 with 1N HCl and extracted with EA. The organic phase was washed with saturated brine, dried over anhydrous sodium sulfate, and concentrated to give 4-(4-methoxy-2-methylthieno[3,2-e]benzofuran-7-yl-1-D)-2-methyl-4-oxobutanoic acid as a solid (1100 mg, 3.3 mmol, 95.52%). ESI MS[M+H]⁺ for C₁₇H₁₅DO₅S, calcd 334.38, found: 333.9.

### Step (X): Synthesis of (S)-4-(4-methoxy-2-methylthieno[3,2-e]benzofuran-7-yl-1-D)-2-methyl-4-oxobutanoic acid

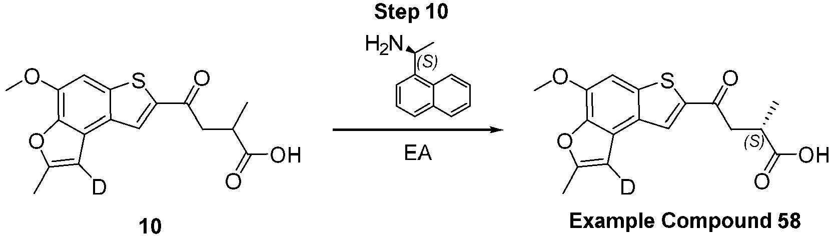

4-(4-Methoxy-2-methylthieno[3,2-e]benzofuran-7-yl-1-D)-2-methyl-4-oxobutanoic acid (1000 mg, 3 mmol, 1 equiv.) and 1S-1-naphthylethan-1-amine (513.65 mg, 3 mmol, 1 equiv.) were dissolved in ethyl acetate (150 mL, 100.0%). The system was heated to 70°C until completely dissolved. Then, the heating was stopped, and the system was slowly returned to room temperature and stirred overnight. A large amount of solid precipitated from the system. The solid was filtered, and the filter cake was dried to give 630 mg. The filter cake was then added to ethyl acetate (50 mL, 100.0%). The system was heated to 70°C until completely dissolved. Then, the heating was stopped, and the system was slowly returned to room temperature and stirred overnight. A large amount of solid precipitated from the system. The solid was filtered, and the filter cake was washed with ethyl acetate and dried to give 440 mg of solid. The solid obtained above was purified on a column (column: C18, 10 µm, 20 * 250 mm; mobile phase: water (containing 0.1% formic acid)/acetonitrile; procedure: gradient from 10% to 60% acetonitrile over 60 min, 60% acetonitrile) to give a product. The product was lyophilized to give (S)-4-(4-methoxy-2-methylthieno[3,2-e]benzofuran-7-yl-1-D)-2-methyl-4-oxobutanoic acid (240 mg, yield: 39.39%). ee% = 98%. ESI MS[M+H]⁺ for C₁₇H₁₅DO₅S, calcd 334.38, found: 333.9; ¹H NMR (400 MHz, DMSO-d6) δ 12.21 (s, 1H), 8.60 (s, 1H), 7.51 (s, 1H), 4.00 (s, 3H), 3.46 (dd, J = 17.4, 8.5 Hz, 1H), 3.13 (dd, J = 17.3, 5.3 Hz, 1H), 2.93 (ddd, J = 8.5, 7.1, 5.3 Hz, 1H), 2.53 (s, 3H), 1.20 (d, J = 7.2 Hz, 3H).

### Example 59

### Synthesis of (S)-4-(1-fluoro-4-methoxy-2-methylthieno[3,2-e]benzofuran-7-yl)-2-methyl-4-oxobutanoic acid (Example Compound 59)

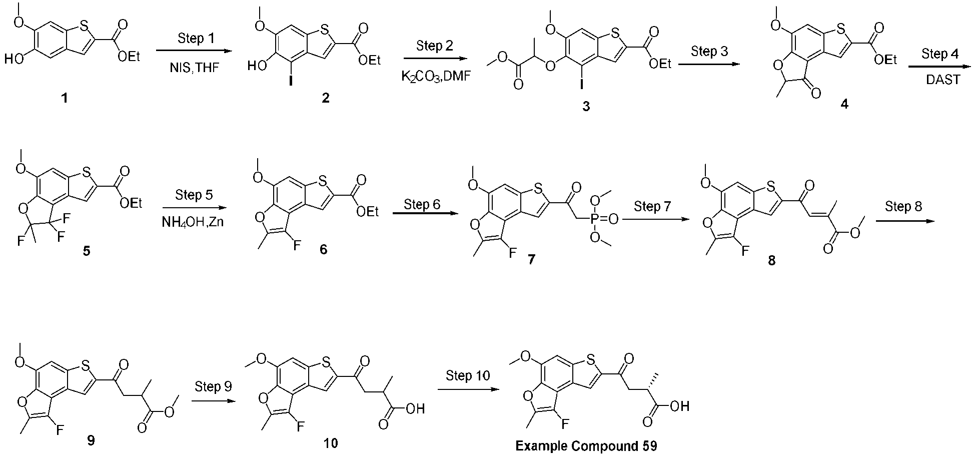

### Step (I): Synthesis of ethyl 5-hydroxy-4-iodo-6-methoxybenzo[b]thiophene-2-carboxylate (Intermediate 2)

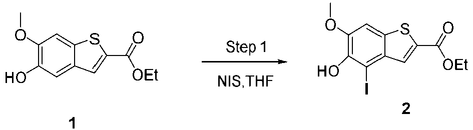

Ethyl 5-hydroxy-6-methoxybenzo[b]thiophene-2-carboxylate (32.5 g, 128.82 mmol, 1 equiv.) was dissolved in THF (500 mL), and N-iodosuccinimide (30.43 g, 135.26 mmol, 1.05 equiv.) was added portionwise in an ice bath. The mixture was reacted at a maintained temperature for 2 h. TLC monitoring indicated that the starting material had completely reacted. The reaction was quenched by the addition of 5% aqueous sodium sulfite solution (200 mL), and the mixture was extracted with EA. The organic phase was washed with brine, dried over anhydrous sodium sulfate, concentrated, triturated with EA, and filtered. The filter cake was dried to give ethyl 5-hydroxy-4-iodo-6-methoxybenzo[b]thiophene-2-carboxylate (42.33 g, 111.93 mmol, 86.89%) as an off-white solid, LC-MS m/z = 378.8 (M+1).

### Step (II): Synthesis of ethyl 4-iodo-6-methoxy-5-((1-methoxy-1-oxopropan-2-yl)oxy)benzo[b]thiophene-2-carboxylate (Intermediate 3)

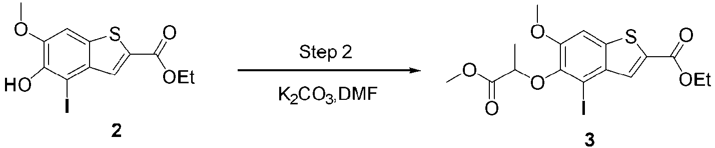

Ethyl 5-hydroxy-4-iodo-6-methoxybenzo[b]thiophene-2-carboxylate (42.33 g, 111.93 mmol, 1 equiv.) was dissolved in DMF (350 mL), and methyl 2-bromopropionate (24.3 g, 145.51 mmol, 1.3 equiv.) and potassium carbonate (30.94 g, 223.86 mmol, 2 equiv.) were added. The mixture was warmed to 80°C and reacted at a maintained temperature for 1.5 h. TLC monitoring indicated that the starting material had completely reacted. The reaction mixture was cooled to room temperature and filtered. The filtrate was diluted with water and extracted with EA. The organic phase was washed with brine, dried over anhydrous sodium sulfate, concentrated, and triturated with EA to give ethyl 4-iodo-6-methoxy-5-((1-methoxy-1-oxopropan-2-yl)oxy)benzo[b]thiophene-2-carboxylate (49.48 g, 106.58 mmol, 95.22%) as an off-white solid, LC-MS m/z = 464.8 (M+1).

### Step (III): Synthesis of ethyl 4-methoxy-2-methyl-1-oxo-1,2-dihydrothieno[3,2-e]benzofuran-7-carboxylate (Intermediate 4)

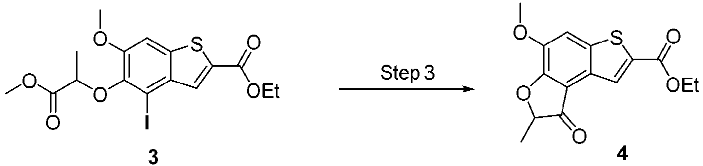

Under nitrogen protection at room temperature, n-butyllithium (35 mL, 2.5M, 85.94 mmol, 1.5 equiv.) was placed in an ice bath, and n-butylmagnesium bromide (86 mL, 1M, 85.94 mmol, 1.5 equiv.) was added dropwise. The mixture was reacted at a maintained temperature in an ice bath for 30 min and then cooled to -78°C. A solution of ethyl 4-iodo-6-methoxy-5-((1-methoxy-1-oxopropan-2-yl)oxy)benzo[b]thiophene-2-carboxylate (26.6 g, 57.29 mmol, equiv.)/THF (250 mL) was added dropwise, and the mixture was reacted at a maintained temperature for 0.5 h. LC-MS monitoring indicated that about 80% of the starting material had reacted. The reaction was quenched by the addition of saturated aqueous ammonium chloride solution (600 mL), and the reaction mixture was extracted with DCM. The organic phase was washed with water, dried over anhydrous sodium sulfate, concentrated, and triturated with EA to give ethyl 4-methoxy-2-methyl-1-oxo-1,2-dihydrothieno[3,2-e]benzofuran-7-carboxylate (6.7 g, 21.87 mmol, 38.17%) as a yellowish solid, LC-MS m/z = 306.8 (M+1).

### Step (IV): Synthesis of ethyl 1,1,2-trifluoro-4-methoxy-2-methyl-1,2-dihydrothieno[3,2-e]benzofuran-7-carboxylate (Intermediate 5)

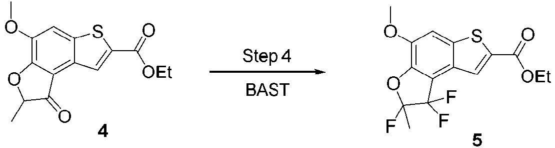

Under nitrogen protection at room temperature, ethyl 4-methoxy-2-methyl-1-oxo-1,2-dihydrothieno[3,2-e]benzofuran-7-carboxylate (2.5 g, 8.16 mmol, 1 equiv.) was added to a sealed tube, followed by BAST (19.86 g, 89.77 mmol, 11 equiv.). The mixture was warmed to 80°C and reacted at a maintained temperature for 6 h. LC-MS monitoring indicated that the starting material had completely reacted. The reaction mixture was cooled to room temperature. The reaction was quenched by the dropwise addition of ice water, and the reaction mixture was extracted with EA. The organic layer was washed with water, dried over anhydrous sodium sulfate, concentrated, purified on a column (eluent: 0-30% DCM/100-70% PE) to give ethyl 1,1,2-trifluoro-4-methoxy-2-methyl-1,2-dihydrothieno[3,2-e]benzofuran-7-carboxylate (2 g, 5.78 mmol, 70.76%) as a white solid, LC-MS m/z = 346.8 (M+1).

### Step (V): Synthesis of ethyl 1-fluoro-4-methoxy-2-methylthieno[3,2-e]benzofuran-7-carboxylate (Intermediate 6)

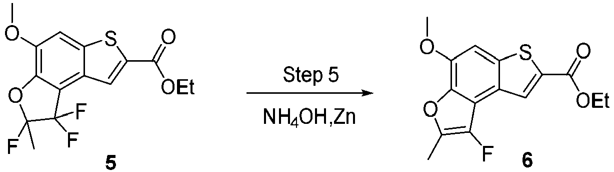

Under nitrogen protection at room temperature, ethyl 1,1,2-trifluoro-4-methoxy-2-methyl-1,2-dihydrothieno[3,2-e]benzofuran-7-carboxylate (2 g, 5.78 mmol, 1 equiv.) was dissolved in THF (40 mL), and zinc powder (5.66 g, 86.63 mmol, 15 equiv.) and aqueous ammonia (80 mL, 28%) were added. The mixture was reacted at room temperature for 3 days. The reaction mixture was filtered, and the filtrate was extracted with DCM. The organic layer was washed with water, dried over anhydrous sodium sulfate, concentrated, and triturated with EA/PE = 1:2 to give ethyl 1-fluoro-4-methoxy-2-methylthieno[3,2-e]benzofuran-7-carboxylate (1.47 g, 4.77 mmol, 82.56%) as a white solid, LC-MS m/z = 308.9 (M+1).

### Step (VI): Synthesis of dimethyl (2-(1-fluoro-4-methoxy-2-methylthieno[3,2-e]benzofuran-7-yl)-2-oxoethyl)phosphonate (Intermediate 7)

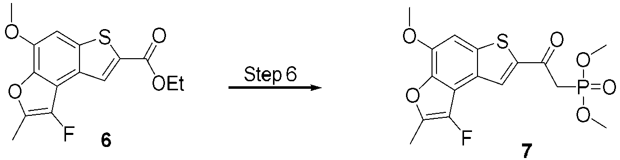

Under nitrogen protection at room temperature, dimethyl methylphosphonate (1.48 g, 11.92 mmol, 2.5 equiv.) was dissolved in THF (15 mL) and then cooled to -78°C. n-Butyllithium (4.8 mL, 2.5M, 11.92 mmol, 2.5 equiv.) was added dropwise, and the mixture was reacted at a maintained temperature for 1 h. A solution of ethyl 1-fluoro-4-methoxy-2-methylthieno[3,2-e]benzofuran-7-carboxylate (1.47 g, 4.77 mmol, equiv.)/THF (30 mL) was added dropwise, and the mixture was reacted at a maintained temperature for 1.5 h. The reaction was quenched by the addition of saturated aqueous ammonium chloride solution (50 mL), and the reaction mixture was extracted with EA. The organic layer was washed with water, dried over anhydrous sodium sulfate, concentrated, and purified on a column (mobile phase: 0-100% EA/100-0% PE) to give dimethyl 2-(1-fluoro-4-methoxy-2-methylthieno[3,2-e]benzofuran-7-yl)-2-oxoethyl)phosphonate (1.48 g, 3.83 mmol, 80.35%) as a pale yellow solid, LC-MS m/z = 386.8 (M+1).

### Step (VII): Synthesis of methyl (E)-4-(1-fluoro-4-methoxy-2-methylthieno[3,2-e]benzofuran-7-yl)-2-methyl-4-oxobut-2-eno ate (Intermediate 8)

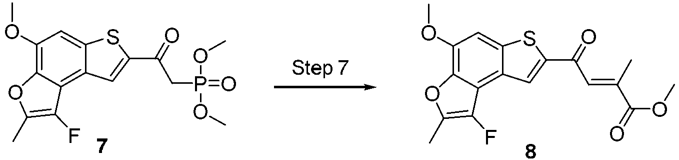

Under nitrogen protection at room temperature, dimethyl 2-(1-fluoro-4-methoxy-2-methylthieno[3,2-e]benzofuran-7-yl)-2-oxoethyl)phosphonate (1.48 g, 3.83 mmol, 1 equiv.) was dissolved in THF (30 mL) and then cooled to -25°C. n-Butyllithium (2.3 mL, 2.5M, 5.75 mmol, 1.5 equiv.) was added dropwise, and the mixture was reacted at a maintained temperature for 1 h. A solution of methyl 2-oxopropanoate (0.7 g, 6.9 mmol, 1.8 equiv.)/THF (5 mL) was added dropwise, and the mixture was reacted at a maintained temperature for 0.5 h. The reaction mixture was warmed to room temperature and reacted for another 0.5 h. The reaction was quenched by the addition of saturated aqueous ammonium chloride solution (40 mL), and the reaction mixture was extracted with EA. The organic layer was washed with water, dried over anhydrous sodium sulfate, and concentrated to give methyl (E)-4-(1-fluoro-4-methoxy-2-methylthieno[3,2-E]benzofuran-7-yl)-2-methyl-4-oxobut-2-enoate (1.3 g, 3.59 mmol, 93.65%) as a yellow solid. LC-MS m/z = 362.8 (M+1).

### Step (VIII): Synthesis of methyl 4-(1-fluoro-4-methoxy-2-methylthieno[3,2-e]benzofuran-7-yl)-2-methyl-4-oxobutanoate (Intermediate 9)

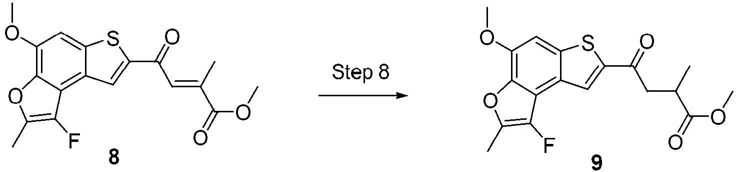

Under nitrogen protection at room temperature, methyl (E)-4-(1-fluoro-4-methoxy-2-methylthieno[3,2-e]benzofuran-7-yl)-2-methyl-4-oxobut-2-enoate (1.3 g, 3.59 mmol, 1 equiv.) was dissolved in methanol (32 mL) and water (3.2 mL), and zinc powder (1.17 g, 17.94 mmol, 5 equiv.) and ammonium acetate (1.38 g, 17.94 mmol, 5 equiv.) were added. The mixture was refluxed for 0.5 h. TLC monitoring indicated that the starting material had completely reacted. The reaction mixture was filtered, and the filter cake was washed with DCM. The filtrate was diluted with water and extracted with DCM. The organic layer was washed with water, dried over anhydrous sodium sulfate, concentrated, and triturated with EA/PE = 1:2 to give methyl 4-(1-fluoro-4-methoxy-2-methylthieno[3,2-e]benzofuran-7-yl)-2-methyl-4-oxobutanoate (0.83 g, 2.28 mmol, 63.49%) as a white solid, LC-MS m/z = 364.8 (M+1).

### Step (IX): Synthesis of 4-(1-fluoro-4-methoxy-2-methylthieno[3,2-e]benzofuran-7-yl)-2-methyl-4-oxobutanoic acid (Intermediate 10)

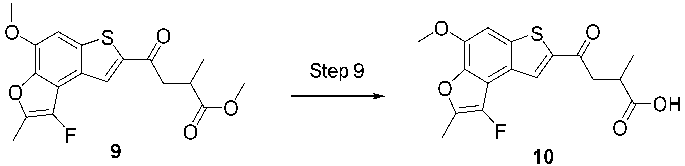

Methyl 4-(1-fluoro-4-methoxy-2-methylthieno[3,2-e]benzofuran-7-yl)-2-methyl-4-oxobutanoate (0.83 g, 2.29 mmol, 1 equiv.) was dissolved in THF (6 mL), MeOH (3 mL), and water (3 mL) at room temperature, and lithium hydroxide monohydrate (288.11 mg, 6.87 mmol, 3 equiv.) was added. The mixture was reacted at room temperature for 1.5 h. TLC monitoring indicated that the starting material had completely reacted. The reaction mixture was concentrated, diluted with water, and extracted with DCM to remove impurities. The aqueous phase was adjusted to pH = 5 with dilute hydrochloric acid. A large amount of solid precipitated. The solid was filtered, and the filter cake was washed to neutral with water and dried under vacuum to give 4-(1-fluoro-4-methoxy-2-methylthieno[3,2-e]benzofuran-7-yl)-2-methyl-4-oxobutanoic acid (742 **mg,** 2.12 mmol, 92.53%) as a yellowish solid, LC-MS m/z = 350.8 (M+1).

### Step (X): Synthesis of (S)-4-(1-fluoro-4-methoxy-2-methylthieno[3,2-e]benzofuran-7-yl)-2-methyl-4-oxobutanoic acid (Example Compound 59)

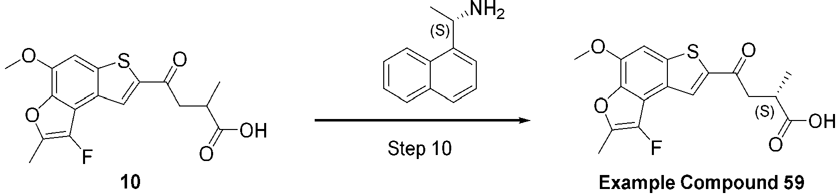

4-(1-Fluoro-4-methoxy-2-methylthieno[3,2-e]benzofuran-7-yl)-2-methyl-4-oxobutanoic acid (308.32 mg, 0.88 mmol, 1 equiv.) and (S)-1-(naphthalen-1-yl)ethan-1-amine (147.68 mg, 0.86 mmol, 0.98 equiv.) were added at room temperature, followed by ethyl acetate (61 mL). The mixture was warmed to 75°C, stirred until completely dissolved, then slowly cooled to room temperature, and stirred overnight. A solid precipitated. The solid was filtered, and the filter cake was purified by pre-HPLC (mobile phase: 0.1% TFA/MeCN) and lyophilized to give (S)-4-(1-fluoro-4-methoxy-2-methylthieno[3,2-e]benzofuran-7-yl)-2-methyl-4-oxobutanoic acid (115 mg, 0.33 mmol, 37.3%) as a white solid, ee = 97%, MS m/z = 350.9 (M+1), ¹H NMR (400 MHz, DMSO) δ 12.21 (s, 1H), 8.30 (s, 1H), 7.58 (s, 1H), 4.00 (s, 3H), 3.50 (dd, J = 17.6, 8.6 Hz, 1H), 3.16 (dd, J = 17.6, 4.9 Hz, 1H), 2.97 - 2.86 (m, 1H), 2.50 (s, 3H), 1.22 (d, J = 7.2 Hz, 3H).

### Example 60

### Synthesis of (R)-4-(1-fluoro-4-methoxy-2-methylthieno[3,2-e]benzofuran-7-yl)-2-methyl-4-oxobutanoic acid (Example Compound 60)

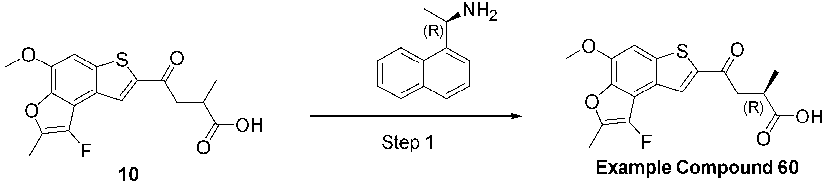

### Step (I): Synthesis of (R)-4-(1-fluoro-4-methoxy-2-methylthieno[3,2-e]benzofuran-7-yl)-2-methyl-4-oxobutanoic acid (Example Compound 60)

4-(1-Fluoro-4-methoxy-2-methylthieno[3,2-e]benzofuran-7-yl)-2-methyl-4-oxobutanoic acid (50 mg, 0.14 mmol, 1 equiv.) and (R)-1-(naphthalen-1-yl)ethan-1-amine (24.44 mg, 0.14 mmol, 1 equiv.) were added at room temperature, followed by EA (7.5 mL, 100.0%). The mixture was warmed to 75°C, stirred until completely dissolved, then slowly cooled to room temperature, and stirred overnight. A solid precipitated. The solid was filtered, and the filter cake was purified by pre-HPLC (mobile phase: 0.1% TFA/MeCN) and lyophilized to give (R)-4-(1-fluoro-4-methoxy-2-methylthieno[3,2-e]benzofuran-7-yl)-2-methyl-4-oxobutanoic acid (17.1 mg, 0.05 mmol, 34.2%) as a white solid, ee = 94.5%, MS m/z = 350.9 (M+1), ¹H NMR (400 MHz, DMSO) δ 12.21 (s, 1H), 8.33 (s, 1H), 7.60 (s, 1H), 4.01 (s, 3H), 3.51 (dd, J = 17.6, 8.6 Hz, 1H), 3.17 (dd, J = 17.6, 5.0 Hz, 1H), 2.96 - 2.87 (m, 1H), 2.51 (d, J = 1.8 Hz, 3H), 1.21 (d, J = 7.2 Hz, 3H).

### Example 61

### Synthesis of (S)-4-(2-fluoro-4-(methoxy-d3)thieno[3,2-e]benzofuran-7-yl)-2-methyl-4-oxobutanoic acid (Example Compound 61)

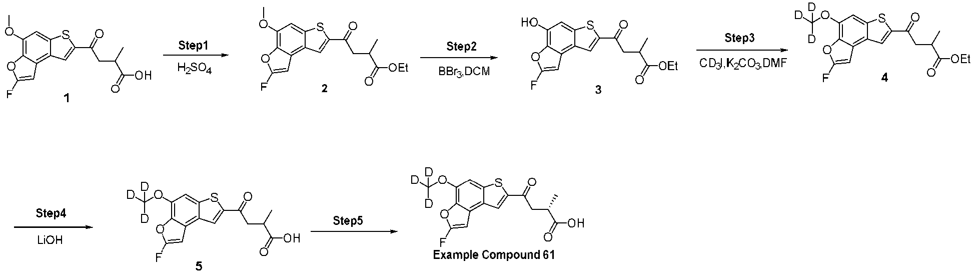

### Step (I): Synthesis of ethyl 4-(2-fluoro-4-methoxythieno[3,2-e]benzofuran-7-yl)-2-methyl-4-oxobutanoate (Intermediate 2)

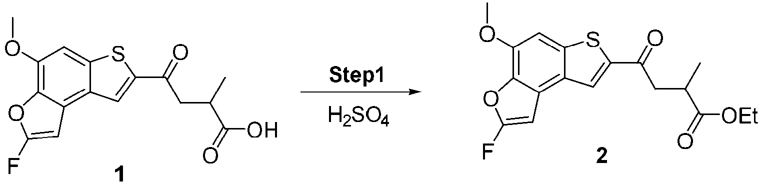

4-(2-Fluoro-4-methoxythieno[3,2-e]benzofuran-7-yl)-2-methyl-4-oxobutanoic acid (1.08 g, 3.21 mmol, 1 equiv.) was uniformly mixed in EtOH (11 mL, 100.0%), and concentrated sulfuric acid (0.31 g, 3.21 mmol, 1 equiv.) was added. The mixture was warmed to 85°C and refluxed for 3 h. TLC monitoring indicated that the starting material had completely reacted. The reaction mixture was cooled in an ice bath, diluted with 8 mL of water, and filtered. The filter cake was washed to neutral with water and dried under vacuum to give ethyl 4-(2-fluoro-4-methoxythieno[3,2-e]benzofuran-7-yl)-2-methyl-4-oxobutanoate (1.04 g, 2.85 mmol, 88.88%) as a white solid, LC-MS m/z = 364.9 (M+1).

### Step (II): Synthesis of ethyl 4-(2-fluoro-4-hydroxythieno[3,2-e]benzofuran-7-yl)-2-methyl-4-oxobutanoate (Intermediate 3)

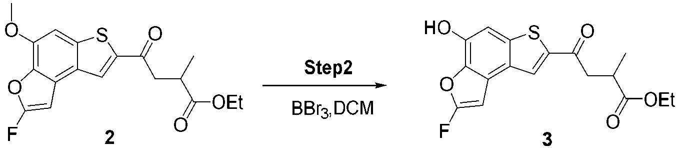

Under nitrogen protection at room temperature, ethyl 4-(2-fluoro-4-methoxythieno[3,2-e]benzofuran-7-yl)-2-methyl-4-oxobutanoate (1.04 g, 2.85 mmol, 1 equiv.) was dissolved in DCM (10 mL, 100.0%), and boron tribromide (4.29 g, 17.12 mmol, 6 equiv.) was added. The mixture was reacted at room temperature for 3 h. The reaction was quenched by the dropwise addition of ice water. The reaction mixture was extracted with EA (50 ml * 2) and washed twice with hydrochloric acid. The organic layer was dried over anhydrous sodium sulfate, concentrated, and purified by pre-TLC (eluent: EA/PE = 1:2) to give ethyl 4-(2-fluoro-4-hydroxythieno[3,2-e]benzofuran-7-yl)-2-methyl-4-oxobutanoate (178 mg, 0.51 mmol, 17.8%), LC-MS m/z = 350.9 (M+1).

### Step (III): Synthesis of ethyl 4-(2-fluoro-4-(methoxy-d3)thieno[3,2-e]benzofuran-7-yl)-2-methyl-4-oxobutanoate (Intermediate 4)

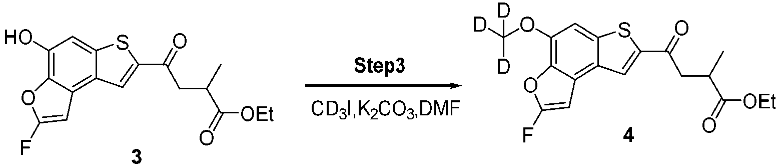

Under nitrogen protection at room temperature, ethyl 4-(2-fluoro-4-hydroxythieno[3,2-e]benzofuran-7-yl)-2-methyl-4-oxobutanoate (178 mg, 0.51 mmol, 1 equiv.) was dissolved in DMF (4 mL, 100.0%), and deuterated iodomethane (144.22 mg, 1.02 mmol, 2 equiv.) and potassium carbonate (210.65 mg, 1.52 mmol, 3 equiv.) were added. The mixture was reacted at room temperature for 2 h. TLC monitoring indicated that the starting material had completely reacted. The reaction mixture was diluted with DCM (20 mL) and washed with water. The organic layer was dried over anhydrous sodium sulfate, concentrated, and purified by pre-TLC (eluent: EA/PE = 1:5) to give ethyl 4-(2-fluoro-4-(methoxy-d3)thieno[3,2-e]benzofuran-7-yl)-2-methyl-4-oxobutanoate (159 mg, 0.43 mmol, 100%), LC-MS m/z = 367.9 (M+1).

### Step (IV): Synthesis of 4-(2-fluoro-4-(methoxy-d3)thieno[3,2-e]benzofuran-7-yl)-2-methyl-4-oxobutanoic acid (Intermediate 5)

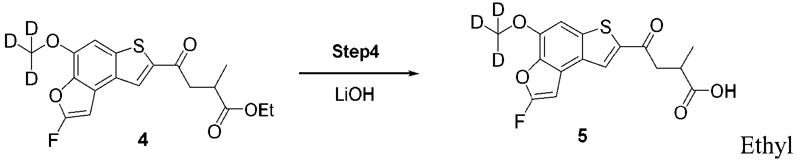

4-(2-fluoro-4-(methoxy-d3)thieno[3,2-e]benzofuran-7-yl)-2-methyl-4-oxobutanoate (159 mg, 0.43 mmol, 1 equiv.) was dissolved in 1,4-dioxane (2.4 mL, 70.59%) and H₂O (1 mL, 29.41%) at room temperature, and lithium hydroxide monohydrate (108.26 mg, 2.58 mmol, 6 equiv.) was added. The mixture was reacted at room temperature for 2 h. TLC monitoring indicated that the starting material had completely reacted. The system was adjusted to pH = 4-5 with 3N hydrochloric acid, extracted with EA (20 mL), and washed with water. The organic layer was dried over anhydrous sodium sulfate, concentrated, and purified by pre-TLC to give 4-(2-fluoro-4-(methoxy-d3)thieno[3,2-e]benzofuran-7-yl)-2-methyl-4-oxobutanoic acid (150 mg, 0.44 mmol, 102.8%), LC-MS m/z = 339.9 (M+1).

### Step (V): Synthesis of (S)-4-(2-fluoro-4-(methoxy-d3)thieno[3,2-e]benzofuran-7-yl)-2-methyl-4-oxobutanoic acid (Example Compound 61)

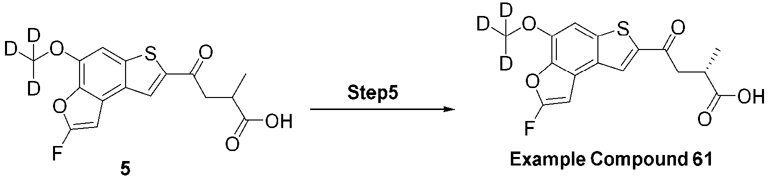

4-(2-Fluoro-4-(methyloxy-d3)thieno[3,2-e]benzofuran-7-yl)-2-methyl-4-oxobutanoic acid (150 mg, 0.44 mmol, 1 equiv.) was uniformly mixed in EA (4.5 mL), and (2S)-2-amino-2-phenylethan-1-ol (60.64 mg, 0.44 mmol, 1 equiv.) was added. The mixture was heated until completely dissolved, then cooled to room temperature, and stirred overnight. A large amount of solid precipitated. The solid was filtered to give 170 mg of filter cake, S/R = 88:12. Subsequently, 170 mg of the above solid was uniformly mixed in EA (12 mL). The mixture was heated until completely dissolved, then cooled to room temperature, and stirred overnight. A large amount of solid precipitated. The solid was filtered to give the filter cake S/R = 94.5:5.5. The filter cake was purified by pre-HPLC (mobile phase: 0.1% TFA/MeCN) and lyophilized to give (S)-4-(2-fluoro-4-(methoxy-d3)thieno[3,2-e]benzofuran-7-yl)-2-methyl-4-oxobutanoic acid (86.7 mg, 0.26 mmol, 57.8%) as a white solid. MS m/z = 340.0 (M+1), HPLC = 98.7%, ¹H NMR (400 MHz, DMSO) δ 12.25 (s, 1H), 8.60 (s, 1H), 7.63 (s, 1H), 6.80 (d, J = 6.6 Hz, 1H), 3.49 - 3.44 (m, 1H), 3.13 (dd, J = 17.4, 5.3 Hz, 1H), 2.99 - 2.88 (m, 1H), 1.21 (d, J = 7.2 Hz, 3H).

### Example 62

### Preparation of (S)-4-(2-fluoro-4-methoxythieno[3,2-e]benzofuran-7-yl)-2-methyl-4-oxobutanoic acid (Example Compound 62)

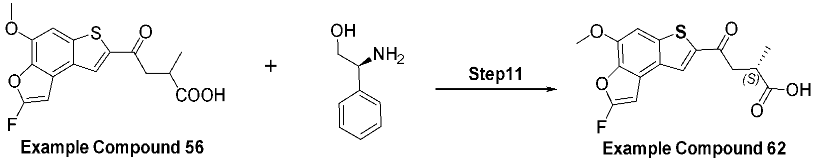

4-(2-Fluoro-4-methoxythieno[3,2-e]benzofuran-7-yl)-2-methyl-4-oxobutanoic acid (1.13 g, 1 equiv.) and (2S)-2-amino-2-phenylethan-1-ol (0.456 g, 0.99 equiv.) were added at room temperature, followed by EA (48 mL). The mixture was warmed to 70°C, stirred until completely dissolved, then slowly cooled to room temperature, and stirred overnight. A large amount of solid precipitated. The solid was filtered, and the filter cake was dried under vacuum to give 0.97 g of an off-white solid. Subsequently, 0.97 g of the above solid was added to EA (95 mL). The mixture was warmed to 70°C, stirred until completely dissolved, concentrated to a volume of about 60 mL, then cooled to room temperature, and stirred overnight. A large amount of solid precipitated. The solid was filtered, and the filter cake was dried under vacuum to give 0.66 g of an off-white solid, MS m/z = 336.9 (M+1). ¹H NMR (400 MHz, DMSO) δ 12.29 (s, 1H), 8.56 (s, 1H), 7.59 (s, 1H), 6.77 (d, J = 6.6 Hz, 1H), 4.01 (s, 3H), 3.45 (d, J = 8.9 Hz, 1H), 3.12 (dd, J = 17.4, 5.3 Hz, 1H), 3.00 - 2.89 (m, 1H), 1.22 (d, J = 7.2 Hz, 3H).

### Example 63

### Preparation of (R)-4-(2-fluoro-4-methoxythieno[3,2-e]benzofuran-7-yl)-2-methyl-4-oxobutanoic acid (Example 63)

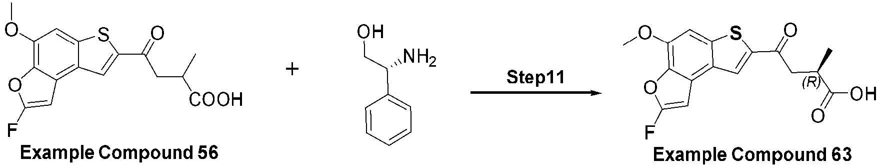

4-(2-Fluoro-4-methoxythieno[3,2-e]benzofuran-7-yl)-2-methyl-4-oxobutanoic acid (1.13 g, 1 equiv.) and (2S)-2-amino-2-phenylethan-1-ol (0.456 g, 0.99 equiv.) were added at room temperature, followed by EA (48 mL). The mixture was warmed to 70°C, stirred until completely dissolved, then slowly cooled to room temperature, and stirred overnight. A large amount of solid precipitated. The solid was filtered, and the filter cake was dried under vacuum to give 0.95 g of an off-white solid. Subsequently, 0.95 g of the above solid was added to EA (90 mL). The mixture was warmed to 70°C, stirred until completely dissolved, concentrated to a volume of about 55 mL, then cooled to room temperature, and stirred overnight. A large amount of solid precipitated. The solid was filtered, and the filter cake was dried under vacuum to give 0.51 g of an off-white solid, MS m/z = 336.9 (M+1). ¹H NMR (400 MHz, DMSO-d6) δ 12.23 (s, 1H), 8.61 (s, 1H), 7.64 (s, 1H), 6.81 (d, J = 6.6 Hz, 1H), 4.02 (s, 3H), 3.45 (dd, J = 17.3, 8.5 Hz, 1H), 3.13 (dd, J = 17.4, 5.3 Hz, 1H), 3.02 - 2.84 (m, 1H), 1.21 (d, J = 7.2 Hz, 3H).

### Example 64

### Synthesis of (S)-4-(2-fluoro-4-hydroxythieno[3,2-e]benzofuran-7-yl)-2-methyl-4-oxobutanoic acid (Example Compound 64)

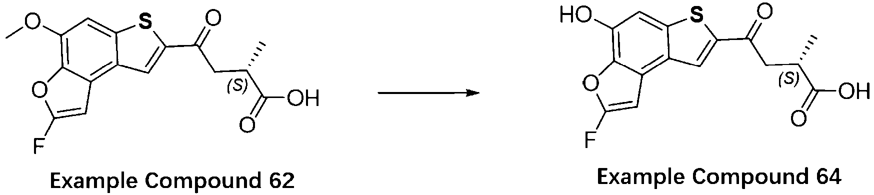

**Step (I): Synthesis of (S)-4-(2-fluoro-4-hydroxythieno[3,2-e]benzofuran-7-yl)-2-methyl-4-oxobutanoic acid (Example Compound 64**)(S)-4-(2-Fluoro-4-methoxythieno[3,2-e]benzofuran-7-yl)-2-methyl-4-oxobutanoic acid (300 mg, 0.89 mmol, 1 equiv.) was dissolved in DCM (3 mL, 100.0%) at room temperature, and boron tribromide (1787.67 mg, 7.14 mmol, 8 equiv.) was added. The mixture was reacted at room temperature for 3 h. The reaction was quenched by the dropwise addition of ice water, and the reaction mixture was extracted with EA (50 mL). The organic layer was washed with water, dried over anhydrous sodium sulfate, concentrated, purified by pre-HPLC (mobile phase: 0.1% TFA/MeCN), and lyophilized to give (S)-4-(2-fluoro-4-hydroxythieno[3,2-e]benzofuran-7-yl)-2-methyl-4-oxobutanoic acid (80.9 mg, 0.25 mmol, 28.14%) as a white solid. MS m/z = 322.9 (M+1), HPLC = 98.0%, ¹H NMR (400 MHz, DMSO) δ 12.23 (s, 1H), 11.07 (s, 1H), 8.56 (s, 1H), 7.28 (s, 1H), 6.77 (d, J = 6.6 Hz, 1H), 3.43 (d, J = 8.5 Hz, 1H), 3.11 (dd, J = 17.3, 5.3 Hz, 1H), 2.98 - 2.88 (m, 1H), 1.20 (d, J = 7.2 Hz, 3H).

### Detection of the Effect of Compounds on Reporter Gene Activity Using Reporter Gene Cell Line

**Test Principle:** The HEK293-HAQ Luc reporter cell line utilizes the chemiluminescent reaction property of luciferase binding to its substrate beetle luciferin. The regulatory elements of the Sting-HAQ gene transcription are cloned upstream/downstream of the firefly luciferase gene to construct a luciferase recombinant plasmid. The recombinant plasmid is then transfected into HEK293 cells. After treatment with compounds, the cells are lysed, and the luciferase activity is measured using the Steady-Glo^{®} detection system (Cat#E2550) manufactured by Promega Corporation. The effect of compounds on the Sting-HAQ regulatory elements is determined based on the level of luciferase activity. The Steady Glo^{®} Luciferase Assay System enables high-throughput evaluation of the degree of activation of reporter cell lines by compounds through the activity of luciferase in mammalian cells, and provides long-term stable luminescence when used with reporter cell lines. The detection system can sustain a signal half-life of more than 5 hours in the test cell culture medium, enabling uniform measurement without sample pretreatment.

**Test Method:** Complete medium (DMEM + 10% FBS + 1% P/S) was prepared. The Hek293-HAQ Luc reporter cell line was resuscitated and passaged twice. Cells in logarithmic growth phase were collected by centrifugation, counted, and resuspended to an appropriate concentration. The cell suspension was seeded into a 96-well plate at a density of 20000 cells/well, with 100 µL of the cell suspension added per well. Test compounds were prepared into stock solutions with DMSO, and serially diluted 3-fold with DMSO starting from the highest concentration of 100 mM to obtain 9 concentration gradients. The test compounds were diluted 47.62-fold with culture medium, and 5 µL of each dilution was added to the 96-well plate containing 100 µL of cells. To Min control wells were added 5 µL of DMSO-cell culture medium mixture (with a final DMSO concentration of 0.1%), while Max control wells received 5 µL of a positive reference compound. The plate was incubated at 37°C in an incubator with 5% carbon dioxide and relative humidity above 90% for 24 h. Subsequently, 75 µL/well of Dual-Glo was added to terminate the reaction. After shaking at 305 rpm for 10 min, the plates were detected on Envision. The fluorescence value RLU of each well was read, and the activation rate was calculated according to the formula: Activation% = (Sample - Ave_L) / (Ave_H - Ave_L) × 100, where Ave_L: RLU Min (0.1% DMSO), Ave_H: RLU Max (positive reference compound). The activation rate, Activation% (Y), corresponding to each concentration (X), was entered in EXCEL. The half-maximal effective concentration EC50 value of each compound was calculated according to the built-in four-parameter fitting formula Y = Bottom + (Top - Bottom) / (1 + 10 ^ ((LogEC50 - X) * HillSlope)) using the XLfit plug-in. The test results are shown in Table 1.

**Table 1**

| Compound structure | HEK293-HAQ Luc Report Assay (24 h) EC50 (µM) |
|---|---|
| 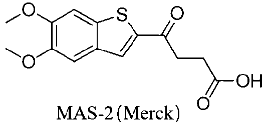 | 2.59 |
| 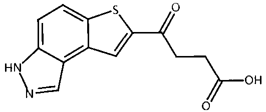 | >100 |
| 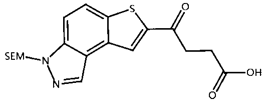 | >100 |
| 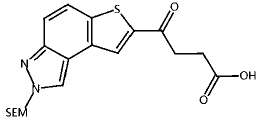 | >100 |
| 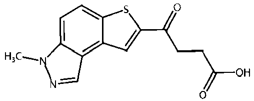 | >100 |
| 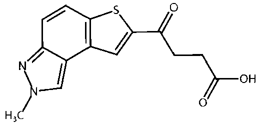 | 41.81 |
| 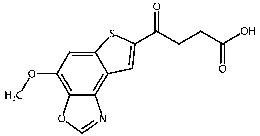 | 1.23 |
| 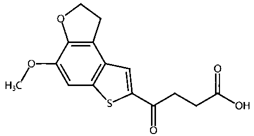 | 7.87 |
| 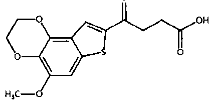 | >100 |
| 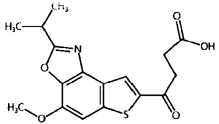 | >100 |
| 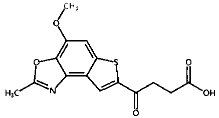 | 1.58 |
| 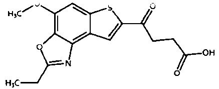 | 36 |
| 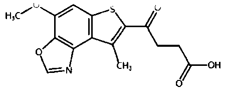 | 58 |
| 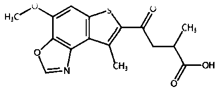 | 92 |
| 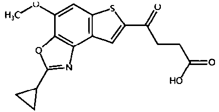 | 90.67 |
| 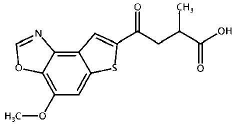 | 1.12 |
| 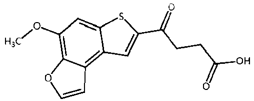 | 2.52 |
| 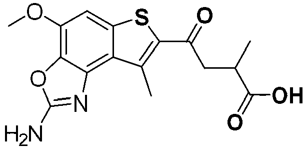 | >100 |
| 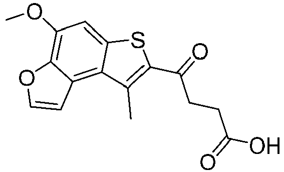 | 29.81 |
| 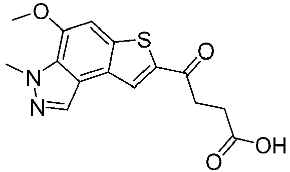 | >100 |
| 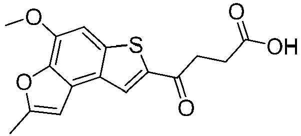 | 1.04 |
| 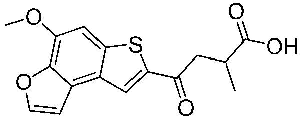 | 2.2 |
| 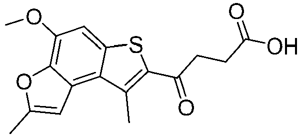 | 19 |
| 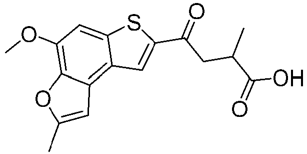 | 1.2 |
| 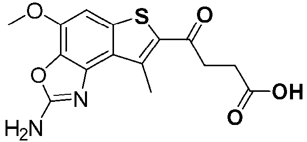 | >100 |
| 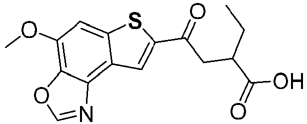 | 0.308 |
| 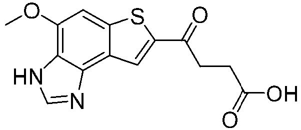 | >100 |
| 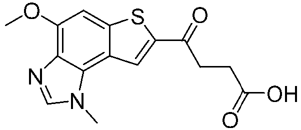 | >100 |
| 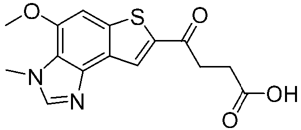 | >100 |
| 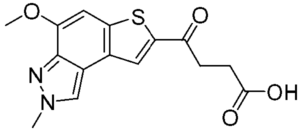 | 49 |
| 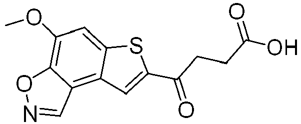 | >100 |
| 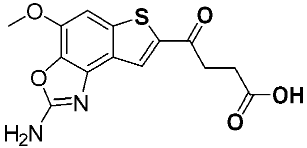 | >100 |
| 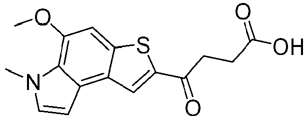 | >100 |
| 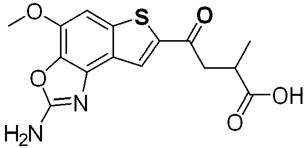 | >100 |
| 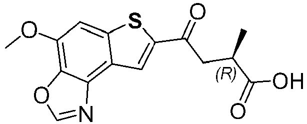 | 5.81 |
| 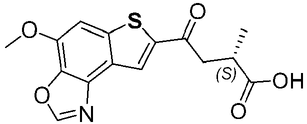 | 0.16 |
| 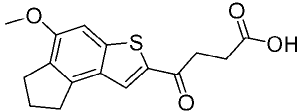 | >100 |
| 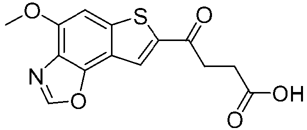 | 2.503 |
| 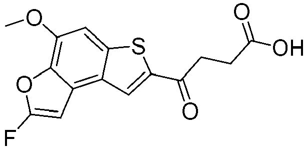 | 0.792 |
| 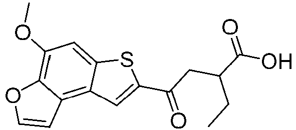 | 0.979 |
| 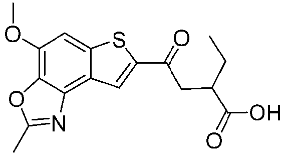 | 0.326 |
| 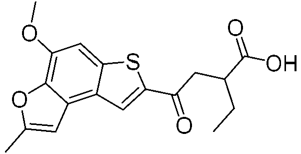 | 0.565 |
| 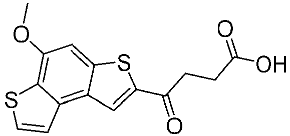 | 10.73 |
| 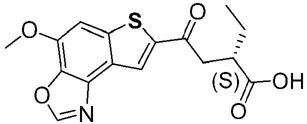 | 0.302 |
| 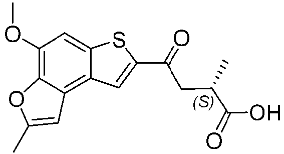 | 0.341 |
| 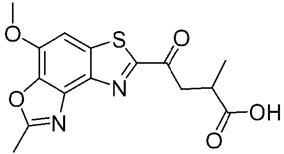 | 3.322 |
| 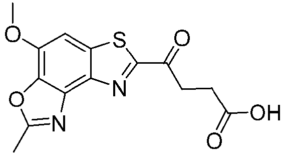 | 17.98 |
| 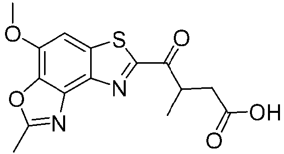 | 59.89 |
| 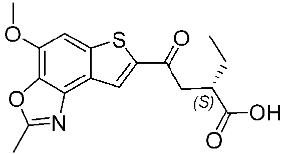 | 2.75 |
| 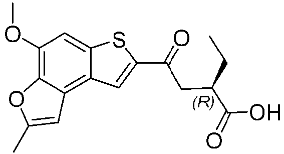 | 4.46 |
| 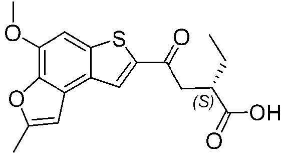 | 0.24 |
| 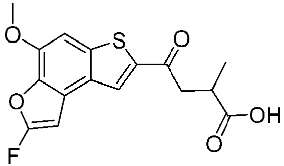 | 0.57 |
| 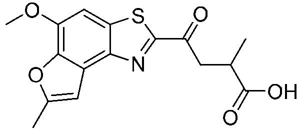 | 2.337 |
| 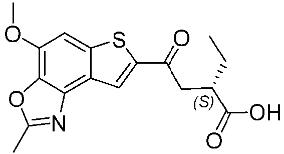 | 0.158 |
| 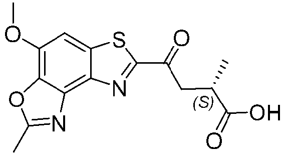 | 4.181 |
| 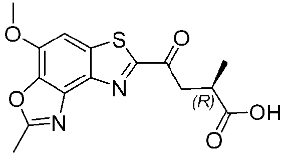 | 67.71 |
| 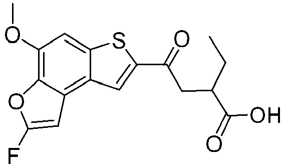 | 0.823 |
| 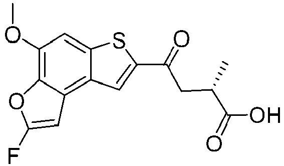 | 0.465 |
| 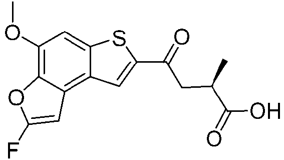 | 6.872 |
| 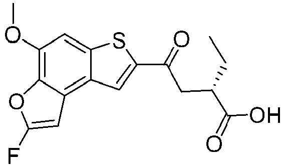 | 0.352 |
| 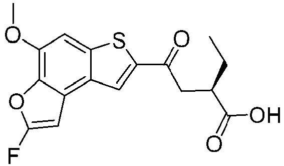 | 4.104 |
| 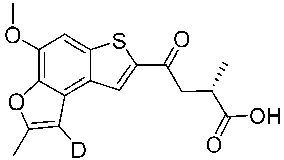 | 0.313 |
| 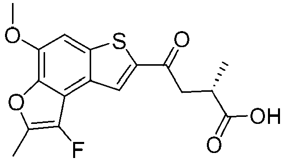 | 0.19 |
| 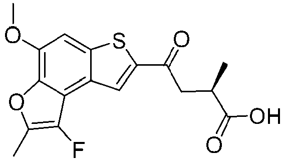 | 1.51 |
| 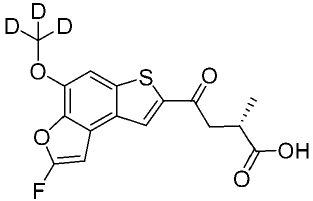 | 0.263 |
| 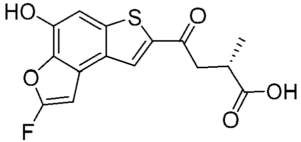 | 29.313 |

### Study on the Pharmacokinetic (PK) Properties of Compounds of the Present Disclosure

The compounds of the present disclosure were administered to ICR mice via intravenous injection (IV) and oral gavage (PO), respectively, and compared with the Merck compound MSA-2 to investigate their pharmacokinetic properties. The doses for IV and PO were 2 mg/kg and 5 mg/kg, respectively. Blood was collected at different time points after IV and PO administration and placed in heparinized tubes. Plasma was separated and frozen in a -20°C refrigerator. Plasma was processed and then analyzed by LCMS/MS. Pharmacokinetic parameters of drug administration were calculated using a non-compartmental model in WinNonlin 8.2 software. The results are shown in the table below.

**Table 2**

| Name | Parameters | | MSA-2 | Example Compound 1 | Example Compound 6 | Example Compound 12 | Example Compound 51 |
|---|---|---|---|---|---|---|---|
| PK | iv, 2mpk | CL (mL/kg/min) | 33 | 50.3 | 7.85 | 21.2 | 10.2 |
| | | AUC(0-t) (h*ng/mL) | 1010 | 660 | 4244 | 1572 | 3244 |
| | | Vss (L/kg) | 0.343 | 0.76 | 0.264 | 0.49 | 1.28 |
| | | T1/2 (h) | 0.431 | 0.658 | 1.113 | 1.5 | 4.36 |
| | PO, 5mpk | %F | 61.23 | 46.98 | 42.5 | 104 | 105.4 |
| | | Cmax (ng/mL) | 2807 | 1273 | 4277 | 6023 | 4546 |
| | | AUC(0-t) (h*ng/mL) | 1532 | 757 | 3678 | 4055 | 8549 |
| | | T1/2 (h) | 1.63 | 1.56 | 2.64 | 2 | 3.1 |

| Name | Parameters | | Example Compound 32 | Example Compound 46 | Example Compound 58 | Example Compound 59 | Example Compound 62 |
|---|---|---|---|---|---|---|---|
| PK | iv, 2mpk | CL (mL/kg/min) | 8.98 | 9.38 | 8.43 | 6.03 | 7.56 |
| | | AUC(0-t) (h*ng/mL) | 3705 | 3540 | 3937 | 5501 | 4305 |
| | | Vss (L/kg) | 0.252 | 0.32 | 0.263 | 0.191 | 0.49 |
| | | T1/2 (h) | 0.821 | 1.722 | 2.08 | 2.35 | 2.68 |
| | PO, | %F | 37.4 | 64.6 | 57.07 | 37.98 | 92.89 |
| | 5mpk | Cmax (ng/mL) | 5360 | 6477 | 7496 | 7703 | 10130 |
| | | AUC(0-t) (h*ng/mL) | 3450 | 5709 | 5559 | 5181 | 10228 |
| | | T1/2 (h) | 0.925 | 1.61 | 7.51 | 1.1 | 3.05 |

Conclusion: In the mouse PK study with injection administration at 2 mg/kg and oral administration at 5 mg/kg, Example Compounds 6, 12, 32, 46, 51, 58, 59, and 62 exhibited higher exposure and lower clearance *in vivo* compared to MAS-2. The oral availabilities of Example Compounds 12, 46, 51, and 62 were 100%, 64.6%, 100%, and 92.89%, respectively, which were superior to that of the comparative compound MSA-2 (61.23%), demonstrating excellent pharmacokinetic properties.

### Pharmacodynamic Evaluation of Test Compounds in a Subcutaneous Tumor Model of Female Balb/c Mice Transplanted with Mouse Colorectal Cancer CT26 Cell Line

CT-26 tumor cells were cultured in a suitable culture medium. Cells in the exponential growth phase were collected and subcutaneously inoculated into the right back of experimental mice. Tumor growth was observed regularly after inoculation. When the tumor reached an average volume of approximately 100 mm³, mice were randomly grouped based on tumor size and body weight for administration. The day of grouping was defined as Day 0. Test compounds were administered orally once weekly for 4 times. The efficacy was evaluated by calculating the tumor growth inhibition (TGI) based on the tumor volume, and the safety was evaluated based on the changes in animal body weight and death. Example Compounds 46 and 32 exhibited significant antitumor effects in the CT-26 model at a dose of 20 mg/kg, with TGI > 90%, and their antitumor effects were superior to MSA-2 at 100 mg/kg. All test compounds were well-tolerated. Moreover, five compounds, Example Compounds 51, 32, 37, 53, and 62, were evaluated at a dose of 20 mg/kg in a separate CT26 model cohort, demonstrating TGI values of 99%, 100%, 96%, 101%, and 100%, respectively, indicating significant antitumor effects.

## Claims

1. A compound having the structure of formula (I) or a pharmaceutically acceptable salt thereof: 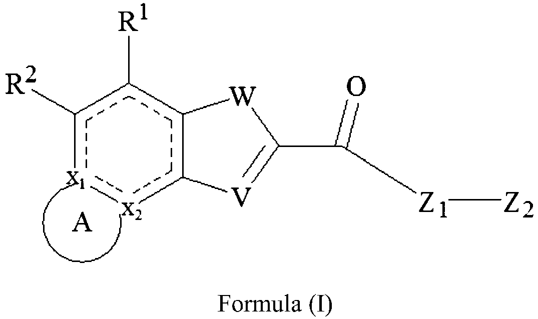
wherein R¹ represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein R² represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
or, R¹ and R², together with the atom to which they are attached, form a 5-8-membered ring, which may optionally contain 0, 1, or 2 heteroatoms selected from O, N, and S;
wherein R³ represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein Z₁ represents -C₁-C₆ alkylene-, -C₂-C₆ alkenylene-, -(C₀-C₆ alkylene)-(C₃-C₆ cycloalkyl)-(C₀-C₆ alkylene)-, -(C₀-C₆ alkylene)-O-(C₀-C₆ alkylene)-, -(C₀-C₆ alkylene)-(3-6-membered heterocycloalkyl)-(C₀-C₆ alkylene)-, -(C₀-C₆ alkylene)-NR^{a}-(C₀-C₆ alkylene)-, -(C₀-C₆ alkylene)-C(O)NR^{a}-(C₀-C₆ alkylene)-, -(C₀-C₆ alkylene)-NR^{a}C(O)-(C₀-C₆ alkylene)-, -(C₀-C₆ alkylene)-S(O)₂NR^{a}-(C₀-C₆ alkylene)-; and any hydrogen atom in the said Z₁ may be substituted with 0-3 substituents selected from halogen, cyano, -OR^{a}, -SR^{a}, -NR^{a}R^{b}, C₁-C₆ alkyl, C₁-C₆ haloalkyl, C₃-C₁₀ cycloalkyl; or any CH₂ in Z₁ is replaced by 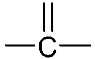 or 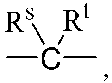 wherein R^{s} and R^{t}, together with the C atom to which they are attached, form a 3-6-membered ring;
wherein W represents S or Se;
wherein V represents CR³ or N;
wherein X₁ and X₂ represent C or N;
wherein Z₂ represents C(O)OR^{L}, C(O)SR^{L}, C(S)OR^{L}, S(O)₂R^{L};
wherein R^{L} represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein ring A represents a substituted or unsubstituted saturated or unsaturated 5-10-membered ring or 5-10-membered heterocyclic ring;
wherein the dotted line represents a single bond or a double bond;
the "substituted or unsubstituted" as defined in the above substituents means optionally substituted with 0, 1, 2, or 3 substituents selected from deuterium, halogen, cyano, C₁-C₆ alkyl, C₂-C₆ alkenyl, C₂-C₆ alkynyl, C₁-C₆ haloalkyl, C₁-C₆ alkoxy, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein R^{a} and R^{b} each independently represent hydrogen, deuterium, C₁-C₆ alkyl, C₁-C₆ haloalkyl, hydroxy-substituted C₁-C₆ alkyl, C₃-C₁₀ cycloalkyl; or R^{a} and R^{b}, together with the atom to which they are attached, form a 3-6-membered ring, which may optionally contain 0, 1, or 2 heteroatoms selected from O, N, and S.

2. The compound or a pharmaceutically acceptable salt thereof according to claim 1, having
the structure of formula I-1 below: 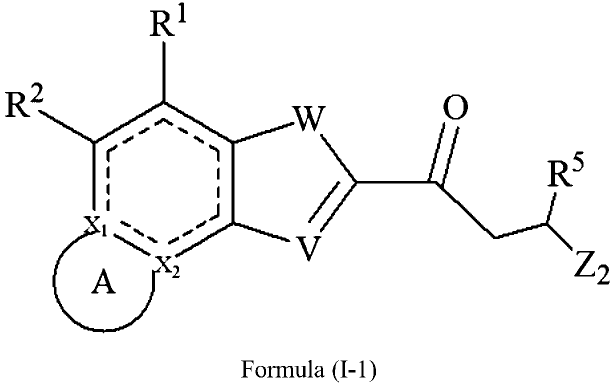 wherein R¹, R², W, V, X₁, X₂, Z₂, and ring A are as defined in claim 1, wherein R⁵ represents C₁-C₆ alkyl, preferably methyl and ethyl; wherein the dotted line represents a single bond or a double bond.

3. The compound or a pharmaceutically acceptable salt thereof according to claim 1, having the structure of formula II below: 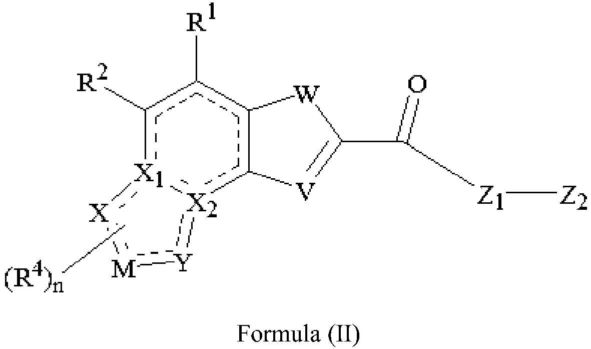
wherein R¹ represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein R² represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
or, R¹ and R², together with the atom to which they are attached, form a 5-8-membered ring, which may optionally contain 0, 1, or 2 heteroatoms selected from O, N, and S;
wherein R³ represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein R⁴ represents hydrogen, deuterium, halogen, cyano, C₁-C₆ alkyl, C₂-C₆ alkenyl, C₂-C₆ alkynyl, C₁-C₆ haloalkyl, C₁-C₆ alkoxy, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein \Z₁ represents -C₁-C₆ alkylene-, -C₂-C₆ alkenylene-, -(C₀-C₆ alkylene)-(C₃-C₆ cycloalkyl)-(C₀-C₆ alkylene)-, -(C₀-C₆ alkylene)-O-(C₀-C₆ alkylene)-, -(C₀-C₆ alkylene)-(3-6-membered heterocycloalkyl)-(C₀-C₆ alkylene)-, -(C₀-C₆ alkylene)-NR^{a}-(C₀-C₆ alkylene)-, -(C₀-C₆ alkylene)-C(O)NR^{a}-(C₀-C₆ alkylene)-, -(C₀-C₆ alkylene)-NR^{a}C(O)-(C₀-C₆ alkylene)-, -(C₀-C₆ alkylene)-S(O)₂NR^{a}-(C₀-C₆ alkylene)-; and any hydrogen atom in the said Z₁ may be substituted with 0-3 substituents selected from halogen, cyano, -OR^{a}, -SR^{a}, -NR^{a}R^{b}, C₁-C₆ alkyl, C₁-C₆ haloalkyl, C₃-C₁₀ cycloalkyl; or any CH₂ in Z₁ is replaced by 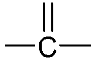 or 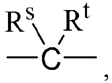 wherein R^{s} and R^{t}, together with the C atom to which they are attached, form a 3-6-membered ring;
wherein Z₂ represents C(O)OR^{L}, C(O)SR^{L}, C(S)OR^{L}, S(O)₂R^{L};
wherein R^{L} represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein W represents S or Se;
wherein V represents CR³ or N;
wherein X, Y, and M each independently represent CR^{X}, O, S, or NR⁵;
wherein X₁ and X₂ represent C or N, provided that at least one is C;
wherein R^{X} represents hydrogen, deuterium, C₁-C₆ alkyl, C₂-C₆ alkenyl, C₂-C₆ alkynyl, C₁-C₆ haloalkyl, hydroxy C₁-C₆ alkyl, halogen, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein R⁵ represents hydrogen, deuterium, C₁-C₆ alkyl, C₂-C₆ alkenyl, C₂-C₆ alkynyl, C₁-C₆ haloalkyl, hydroxy C₁-C₆ alkyl, halogen, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein n is 0, 1, 2;
the "substituted or unsubstituted" as defined in the above substituents means optionally substituted with 0, 1, 2, or 3 substituents selected from halogen, cyano, C₁-C₆ alkyl, C₁-C₆ haloalkyl, C₁-C₆ alkoxy, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein R^{a} and R^{b} each independently represent hydrogen, deuterium, C₁-C₆ alkyl, C₁-C₆ haloalkyl, hydroxy-substituted C₁-C₆ alkyl, C₃-C₁₀ cycloalkyl; or R^{a} and R^{b}, together with the atom to which they are attached, form a 3-6-membered ring, which may optionally contain 0, 1, or 2 heteroatoms selected from O, N, and S;
wherein the dotted line represents a single bond or a double bond.

4. The compound or a pharmaceutically acceptable salt thereof according to claim 3, having the structure of formula II-2 below: 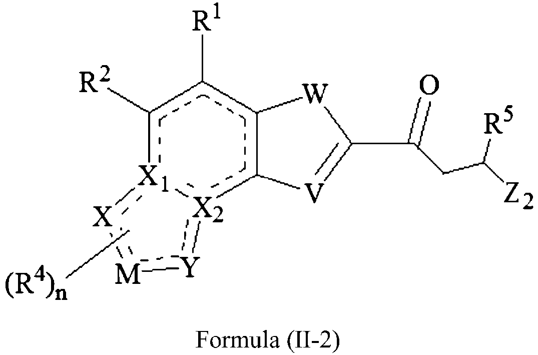 wherein R¹, R², X₁, X₂, X, Y, M, W, V, R₄, n, and Z₂ are as defined in claim 3, wherein R⁵ represents C₁-C₆ alkyl, preferably methyl and ethyl; wherein the dotted line represents a single bond or a double bond.

5. The compound or a pharmaceutically acceptable salt thereof according to any one of claims 1-4, wherein R¹ represents hydrogen, deuterium, halogen, C₁-C₆ alkyl, C₁-C₆ haloalkyl, hydroxy C₁-C₆ alkyl, mercapto C₁-C₆ alkyl, C₂-C₆ alkenyl, C₂-C₆ haloalkenyl, hydroxy C₂-C₆ alkenyl, C₂-C₆ alkenyl, C₂-C₆ haloalkenyl, hydroxy C₂-C₆ alkenyl, mercapto C₂-C₆ alkenyl, C₂-C₆ alkynyl, C₂-C₆ haloalkynyl, hydroxy C₂-C₆ alkynyl, mercapto C₂-C₆ alkynyl, C₃-C₆ saturated or unsaturated cycloalkyl, 3-6-membered saturated or unsaturated heterocycloalkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

6. The compound or a pharmaceutically acceptable salt thereof according to claim 5, wherein R¹ represents hydrogen, halogen, C₁-C₆ alkyl, C₁-C₆ haloalkyl, hydroxy C₁-C₆ alkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

7. The compound or a pharmaceutically acceptable salt thereof according to any one of claims 1-6, wherein R² represents hydrogen, deuterium, halogen, C₁-C₆ alkyl, C₁-C₆ haloalkyl, hydroxy C₁-C₆ alkyl, mercapto C₁-C₆ alkyl, C₂-C₆ alkenyl, C₂-C₆ haloalkenyl, hydroxy C₂-C₆ alkenyl, C₂-C₆ alkenyl, C₂-C₆ haloalkenyl, hydroxy C₂-C₆ alkenyl, mercapto C₂-C₆ alkenyl, C₂-C₆ alkynyl, C₂-C₆ haloalkynyl, hydroxy C₂-C₆ alkynyl, mercapto C₂-C₆ alkynyl, C₃-C₆ saturated or unsaturated cycloalkyl, 3-6-membered saturated or unsaturated heterocycloalkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

8. The compound or a pharmaceutically acceptable salt thereof according to any one of claims 1-7, wherein R² represents hydrogen, deuterium, halogen, C₁-C₆ alkyl, C₁-C₆ haloalkyl, hydroxy C₁-C₆ alkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

9. The compound or a pharmaceutically acceptable salt thereof according to claim 1 or 3, wherein R¹ and R², together with the atom to which they are attached, form a 5-6-membered ring, which may optionally contain 0, 1, or 2 heteroatoms selected from O, N, and S.

10. The compound or a pharmaceutically acceptable salt thereof according to any one of claims 1-9, wherein R³ represents hydrogen, deuterium, halogen, -OR^{a}, cyano, C₁-C₆ alkyl, hydroxy-substituted C₁-C₆ alkyl, C₁-C₆ haloalkyl, C₃-C₆ cycloalkyl.

11. The compound or a pharmaceutically acceptable salt thereof according to any one of claims 1-10, wherein X, Y, and M are each independently selected from O, S, NR⁵, or CR^{X}.

12. The compound or a pharmaceutically acceptable salt thereof according to any one of claims 1-11, wherein W represents S.

13. The compound or a pharmaceutically acceptable salt thereof according to any one of claims 1-12, wherein R⁴ represents hydrogen, deuterium, halogen, -OR^{a}, cyano, C₁-C₆ alkyl, hydroxy-substituted C₁-C₆ alkyl, C₁-C₆ haloalkyl, C₃-C₆ cycloalkyl.

14. The compound or a pharmaceutically acceptable salt thereof according to any one of claims 1-13, wherein Z₁ represents -C₁-C₆ alkylene-, -C₂-C₆ alkenylene-, -(C₀-C₆ alkylene)-(C₃-C₆ cycloalkyl)-(C₀-C₆ alkylene)-, -(C₀-C₆ alkylene)-O-(C₀-C₆ alkylene)-; and any hydrogen atom in the said Z₁ may be substituted with 0-3 substituents selected from halogen, cyano, -OR^{a}, -SR^{a}, -NR^{a}R^{b}, C₁-C₆ alkyl, C₁-C₆ haloalkyl, C₃-C₁₀ cycloalkyl; or any CH₂ in Z₁ is replaced by 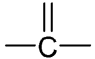 or 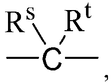 wherein R^{s} and R^{t}, together with the C atom to which they are attached, form a 3-6-membered ring.

15. The compound or a pharmaceutically acceptable salt thereof according to claim 14, wherein Z₁ represents -C₁-C₆ alkylene- or -(C₀-C₆ alkylene)-(C₃-C₆ cycloalkyl)-(C₀-C₆ alkylene)-; and any hydrogen atom in the said Z₁ may be substituted with 0-3 substituents selected from halogen, cyano, -OR^{a}, -SR^{a}, -NR^{a}R^{b}, C₁-C₆ alkyl, C₁-C₆ haloalkyl, C₃-C₁₀ cycloalkyl; or any CH₂ in Z₁ is replaced by 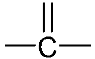 or 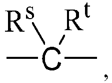 wherein R^{s} and R^{t}, together with the C atom to which they are attached, form a 3-6-membered ring.

16. The compound or a pharmaceutically acceptable salt thereof according to any one of claims 1-15, wherein Z₂ represents C(O)OR^{L}, wherein R^{L} represents hydrogen or C₁-C₆ alkyl, preferably hydrogen.

17. The compound or a pharmaceutically acceptable salt thereof according to any one of claims 1-16, wherein R^{a} and R^{b} each independently represent hydrogen, C₁-C₆ alkyl, C₁-C₆ haloalkyl, C₃-C₆ cycloalkyl.

18. The compound or a pharmaceutically acceptable salt thereof according to claim 4, having the structure of formula II-3 below: 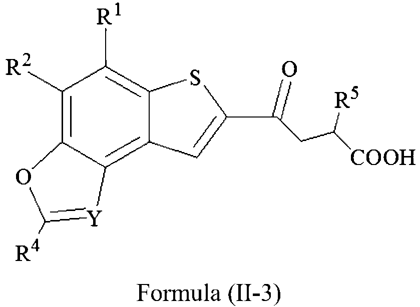
wherein Y represents CH or N;
wherein R¹ and R² each independently represent hydrogen, deuterium, halogen, C₁-C₆ alkyl, C₁-C₆ haloalkyl, hydroxy C₁-C₆ alkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}; or R¹ and R², together with the atom to which they are attached, form a 5-6-membered saturated or unsaturated ring, which may optionally contain 0, 1, or 2 heteroatoms selected from O, S, and N;
wherein Y represents CH or N;
wherein R⁴ represents hydrogen, deuterium, halogen, C₁-C₆ alkyl;
wherein R⁵ represents C₁-C₆ alkyl, preferably methyl and ethyl;
wherein R^{a} and R^{b} each independently represent hydrogen or C₁-C₆ alkyl.

19. The compound or a pharmaceutically acceptable salt thereof according to claim 18, wherein Y represents CH.

20. The compound or a pharmaceutically acceptable salt thereof according to claim 18, wherein Y represents N.

21. The compound or a pharmaceutically acceptable salt thereof according to claim 18, wherein R¹ represents hydrogen, deuterium, halogen, C₁-C₆ alkyl.

22. The compound or a pharmaceutically acceptable salt thereof according to claim 18, wherein R² represents hydrogen, deuterium, halogen, C₁-C₆ alkyl, C₁-C₆ haloalkyl, hydroxy C₁-C₆ alkyl, cyano, -OR^{a};
wherein R^{a} represents hydrogen or C₁-C₆ alkyl.

23. The compound or a pharmaceutically acceptable salt thereof according to claim 18, wherein R² represents hydrogen, halogen, C₁-C₆ haloalkyl, C₁-C₆ alkoxy.

24. The compound or a pharmaceutically acceptable salt thereof according to claim 18, wherein R⁴ represents hydrogen or C₁-C₆ alkyl.

25. The compound or a pharmaceutically acceptable salt thereof according to claim 18, wherein R⁵ represents methyl.

26. The compound or a pharmaceutically acceptable salt thereof according to claim 1, having the structure of formula III below: 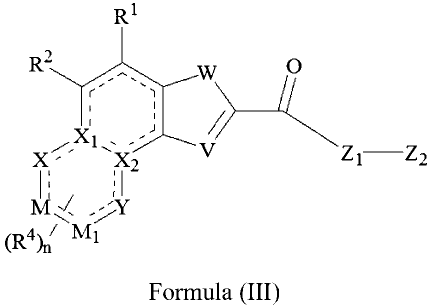
wherein R¹ represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein R² represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
or, R¹ and R², together with the atom to which they are attached, form a 5-8-membered ring,
which may optionally contain 0, 1, or 2 heteroatoms selected from O, N, and S;
wherein R³ represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein R⁴ represents hydrogen, deuterium, halogen, cyano, C₁-C₆ alkyl, C₂-C₆ alkenyl, C₂-C₆ alkynyl, C₁-C₆ haloalkyl, C₁-C₆ alkoxy, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein Z₁ represents -C₁-C₆ alkylene-, -C₂-C₆ alkenylene-, -(C₀-C₆ alkylene)-(C₃-C₆ cycloalkyl)-(C₀-C₆ alkylene)-, -(C₀-C₆ alkylene)-O-(C₀-C₆ alkylene)-, -(C₀-C₆ alkylene)-(3-6-membered heterocycloalkyl)-(C₀-C₆ alkylene)-, -(C₀-C₆ alkylene)-NR^{a}-(C₀-C₆ alkylene)-, -(C₀-C₆ alkylene)-C(O)NR^{a}-(C₀-C₆ alkylene)-, -(C₀-C₆ alkylene)-NR^{a}C(O)-(C₀-C₆ alkylene)-, -(C₀-C₆ alkylene)-S(O)₂NR^{a}-(C₀-C₆ alkylene)-; and any hydrogen atom in the said Z₁ may be substituted with 0-3 substituents selected from halogen, cyano, -OR^{a}, -SR^{a}, -NR^{a}R^{b}, C₁-C₆ alkyl, C₁-C₆ haloalkyl, C₃-C₁₀ cycloalkyl; or any CH₂ in Z₁ is replaced by 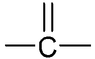 or 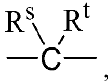 wherein R^{s} and R^{t}, together with the C atom to which they are attached, form a 3-6-membered ring;
wherein Z₂ represents C(O)OR^{L}, C(O)SR^{L}, C(S)OR^{L}, S(O)₂R^{L};
wherein R^{L} represents hydrogen, deuterium, halogen, cyano, nitro, -OR^{a}, NR^{a}R^{b}, -SR^{a}, substituted or unsubstituted C₁-C₆ alkyl, substituted or unsubstituted C₂-C₆ alkenyl, substituted or unsubstituted C₂-C₆ alkynyl, substituted or unsubstituted C₁-C₆ alkoxy, substituted or unsubstituted saturated or unsaturated C₃-C₁₀ cycloalkyl, substituted or unsubstituted saturated or unsaturated 3-10-membered heterocycloalkyl, substituted or unsubstituted 5-10-membered heteroaryl, substituted or unsubstituted C₆-C₁₀ aryl, -C(O)OR^{a}, -C(O)R^{a}, -C(O)NR^{a}R^{b}, -NR^{a}C(O)R^{a}, -S(O)₃R^{a}, -S(O)₂R^{a};
wherein W represents S or Se;
wherein V represents CR³ or N;
wherein X, Y, M, and M₁ each independently represent CR^{X}, O, S, or NR⁵;
wherein X₁ and X₂ represent C or N, provided that at least one is C;
wherein R^{X} represents hydrogen, deuterium, C₁-C₆ alkyl, C₂-C₆ alkenyl, C₂-C₆ alkynyl, C₁-C₆ haloalkyl, hydroxy C₁-C₆ alkyl, halogen, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein R⁵ represents hydrogen, deuterium, C₁-C₆ alkyl, C₂-C₆ alkenyl, C₂-C₆ alkynyl, C₁-C₆ haloalkyl, hydroxy C₁-C₆ alkyl, halogen, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein n is 0, 1, 2;
the "substituted or unsubstituted" as defined in the above substituents means optionally substituted with 0, 1, 2, or 3 substituents selected from deuterium, halogen, cyano, C₁-C₆ alkyl, C₁-C₆ haloalkyl, C₁-C₆ alkoxy, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a};
wherein R^{a} and R^{b} each independently represent hydrogen, C₁-C₆ alkyl, C₁-C₆ haloalkyl, hydroxy-substituted C₁-C₆ alkyl, C₃-C₁₀ cycloalkyl; or R^{a} and R^{b}, together with the atom to which they are attached, form a 3-6-membered ring, which may optionally contain 0, 1, or 2 heteroatoms selected from O, N, and S;
wherein the dotted line represents a single bond or a double bond.

27. The compound or a pharmaceutically acceptable salt thereof according to claim 26, having the structure of formula III-2 below: 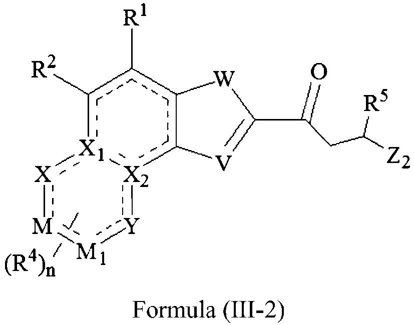 wherein R¹, R², X₁, X₂, R⁴, R⁵, X, Y, M, M₁, W, V, Z₂, and n are as defined in claim 26, wherein the dotted line represents a single bond or a double bond.

28. The compound or a pharmaceutically acceptable salt thereof according to claim 26 or 27, wherein R¹ represents hydrogen, halogen, C₁-C₆ alkyl, C₁-C₆ haloalkyl, hydroxy C₁-C₆ alkyl, mercapto C₁-C₆ alkyl, C₂-C₆ alkenyl, C₂-C₆ haloalkenyl, hydroxy C₂-C₆ alkenyl, C₂-C₆ alkenyl, C₂-C₆ haloalkenyl, hydroxy C₂-C₆ alkenyl, mercapto C₂-C₆ alkenyl, C₂-C₆ alkynyl, C₂-C₆ haloalkynyl, hydroxy C₂-C₆ alkynyl, mercapto C₂-C₆ alkynyl, C₃-C₆ saturated or unsaturated cycloalkyl, 3-6-membered saturated or unsaturated heterocycloalkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

29. The compound or a pharmaceutically acceptable salt thereof according to claim 26 or 27, wherein R¹ represents hydrogen, halogen, C₁-C₆ alkyl, C₁-C₆ haloalkyl, hydroxy C₁-C₆ alkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

30. The compound or a pharmaceutically acceptable salt thereof according to any one of claims 26-29, wherein R² represents hydrogen, deuterium, halogen, C₁-C₆ alkyl, C₁-C₆ haloalkyl, hydroxy C₁-C₆ alkyl, mercapto C₁-C₆ alkyl, C₂-C₆ alkenyl, C₂-C₆ haloalkenyl, hydroxy C₂-C₆ alkenyl, C₂-C₆ alkenyl, C₂-C₆ haloalkenyl, hydroxy C₂-C₆ alkenyl, mercapto C₂-C₆ alkenyl, C₂-C₆ alkynyl, C₂-C₆ haloalkynyl, hydroxy C₂-C₆ alkynyl, mercapto C₂-C₆ alkynyl, C₃-C₆ saturated or unsaturated cycloalkyl, 3-6-membered saturated or unsaturated heterocycloalkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

31. The compound or a pharmaceutically acceptable salt thereof according to any one of claims 26-30, wherein R² represents hydrogen, deuterium, halogen, C₁-C₆ alkyl, C₁-C₆ haloalkyl, hydroxy C₁-C₆ alkyl, cyano, nitro, -OR^{a}, NR^{a}R^{b}, -SR^{a}, -C(O)OR^{a}, -C(O)R^{a}, -C(O)NR^{a}R^{b}, -NR^{a}C(O)R^{a}, -S(O)₃R^{a}, -S(O)₂R^{a}.

32. The compound or a pharmaceutically acceptable salt thereof according to claim 36 or 31, wherein R¹ and R², together with the atom to which they are attached, form a 5-6-membered ring, which may optionally contain 0, 1, or 2 heteroatoms selected from O, N, and S.

33. The compound or a pharmaceutically acceptable salt thereof according to any one of claims 26-32, wherein R³ represents hydrogen, halogen, -OR^{a}, cyano, C₁-C₆ alkyl, hydroxy-substituted C₁-C₆ alkyl, C₁-C₆ haloalkyl, C₃-C₆ cycloalkyl.

34. The compound or a pharmaceutically acceptable salt thereof according to any one of claims 26-33, wherein X, Y, M, and M₁ are each independently selected from O, S, NR⁵, or CR^{X}.

35. The compound or a pharmaceutically acceptable salt thereof according to any one of claims 26-34, wherein R⁴ represents hydrogen, halogen, -OR^{a}, cyano, C₁-C₆ alkyl, hydroxy-substituted C₁-C₆ alkyl, C₁-C₆ haloalkyl, C₃-C₆ cycloalkyl.

36. The compound or a pharmaceutically acceptable salt thereof according to any one of claims 26-35, wherein Z₁ represents -C₁-C₆ alkylene-, -C₂-C₆ alkenylene-, -(C₀-C₆ alkylene)-(C₃-C₆ cycloalkyl)-(C₀-C₆ alkylene)-, -(C₀-C₆ alkylene)-O-(C₀-C₆ alkylene)-; and any hydrogen atom in the said Z₁ may be substituted with 0-3 substituents selected from halogen, cyano, -OR^{a}, -SR^{a}, -NR^{a}R^{b}, C₁-C₆ alkyl, C₁-C₆ haloalkyl, C₃-C₁₀ cycloalkyl; or any CH₂ in Z₁ is replaced by 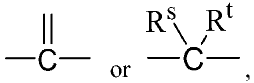 wherein R^{s} and R^{t}, together with the C atom to which they are attached, form a 3-6-membered ring.

37. The compound or a pharmaceutically acceptable salt thereof according to claim 26, wherein Z₁ represents -C₁-C₆ alkylene- or -(C₀-C₆ alkylene)-(C₃-C₆ cycloalkyl)-(C₀-C₆ alkylene)-; and any hydrogen atom in the said Z₁ may be substituted with 0-3 substituents selected from halogen, cyano, -OR^{a}, -SR^{a}, -NR^{a}R^{b}, C₁-C₆ alkyl, C₁-C₆ haloalkyl, C₃-C₁₀ cycloalkyl; or any CH₂ in Z₁ is replaced by 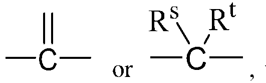 wherein R^{s} and R^{t}, together with the C atom to which they are attached, form a 3-6-membered ring.

38. The compound or a pharmaceutically acceptable salt thereof according to any one of claims 26-37, wherein Z₂ represents C(O)OR^{L}, wherein R^{L} represents hydrogen or C₁-C₆ alkyl, preferably hydrogen.

39. The compound or a pharmaceutically acceptable salt thereof according to any one of claims 26-38, wherein R^{a} and R^{b} each independently represent hydrogen, C₁-C₆ alkyl, C₁-C₆ haloalkyl, C₃-C₆ cycloalkyl.

40. A compound or a pharmaceutically acceptable salt thereof, having the following structure:
| Compound No. | Compound structure | Compound No. | Compound structure |
|---|---|---|---|
| 1 | 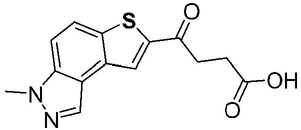 | 2 | 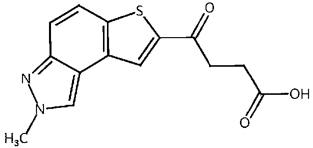 |
| 3 | 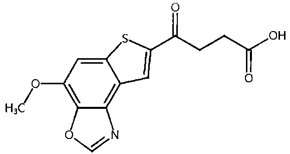 | 4 | 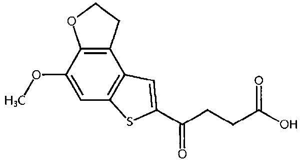 |
| 5 | 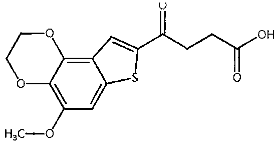 | 6 | 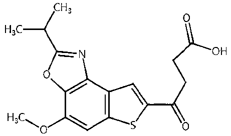 |
| 7 | 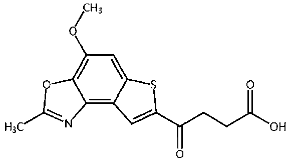 | 8 | 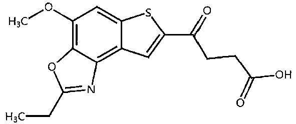 |
| 9 | 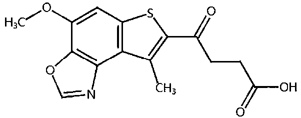 | 10 | 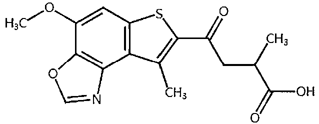 |
| 11 | 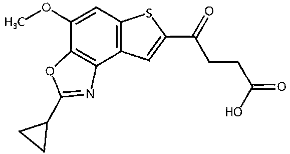 | 12 | 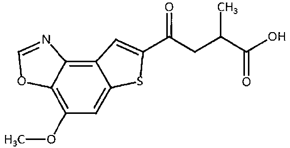 |
| 13 | 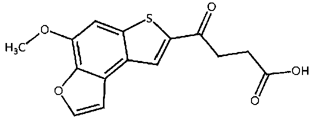 | 14 | 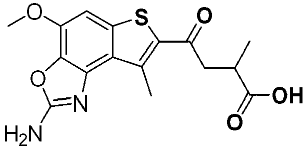 |
| 15 | 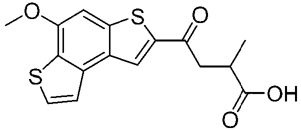 | 16 | 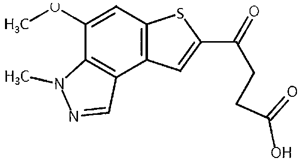 |
| 17 | 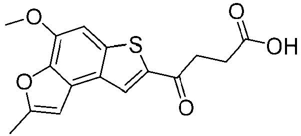 | 18 | 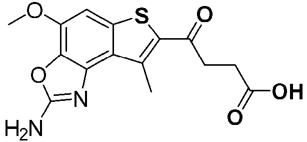 |
| 19 | 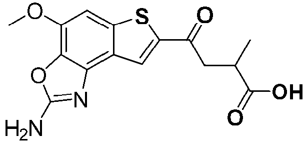 | 20 | 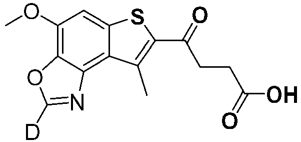 |
| 21 | 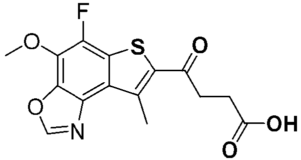 | 22 | 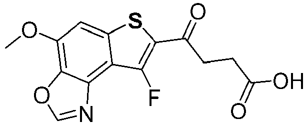 |
| 23 | 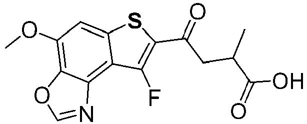 | 24 | 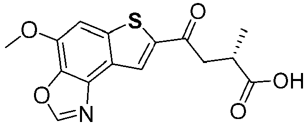 |
| 25 | 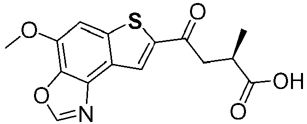 | 26 | 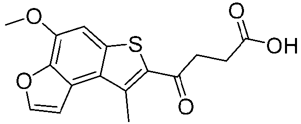 |
| 27 | 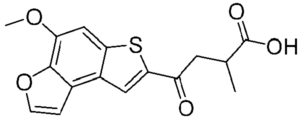 | 28 | 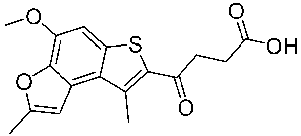 |
| 29 | 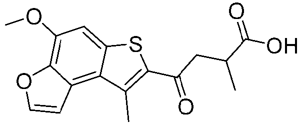 | 30 | 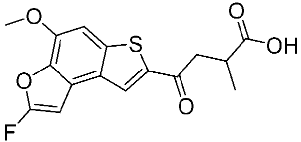 |
| 31 | 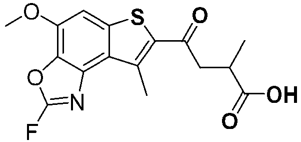 | 32 | 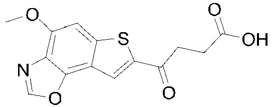 |
| 33 | 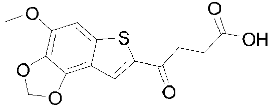 | 34 | 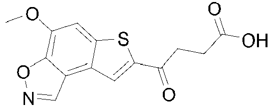 |
| 35 | 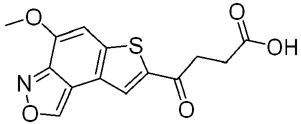 | 36 | 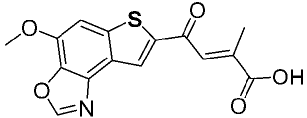 |
| 37 | 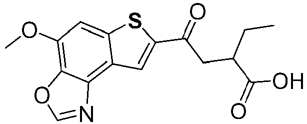 | 38 | 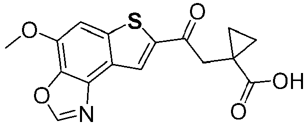 |
| 39 | 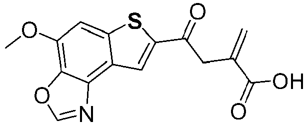 | 40 | 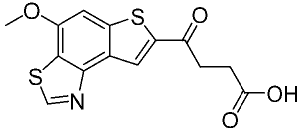 |
| 41 | 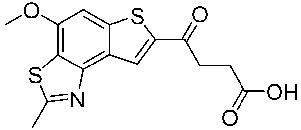 | 42 | 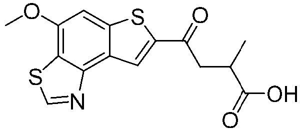 |
| 43 | 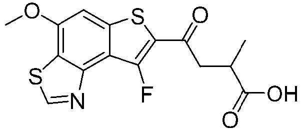 | 44 | 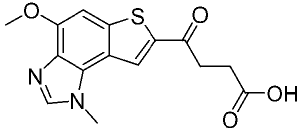 |
| 45 | 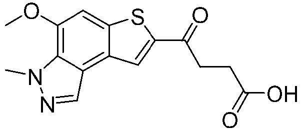 | 46 | 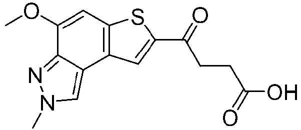 |
| 47 | 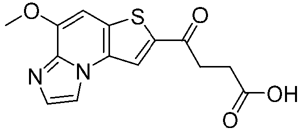 | 48 | 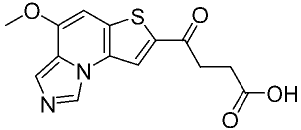 |
| 49 | 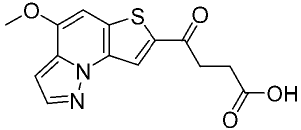 | 50 | 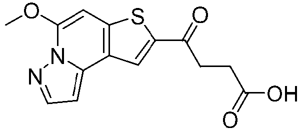 |
| 51 | 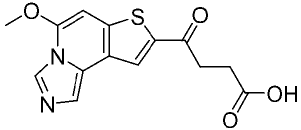 | 52 | 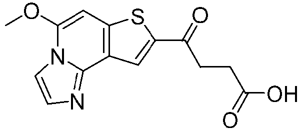 |
| 53 | 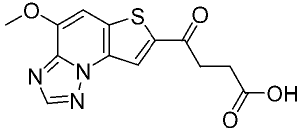 | 54 | 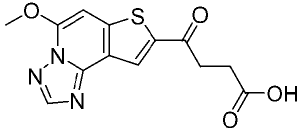 |
| 55 | 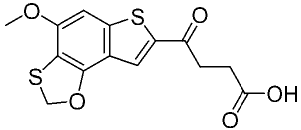 | 56 | 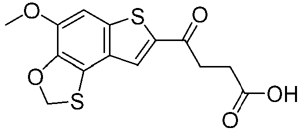 |
| 57 | 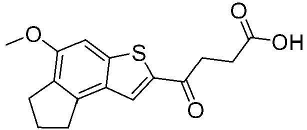 | 58 | 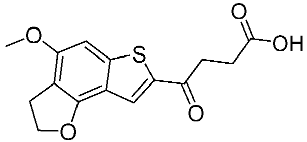 |
| 59 | 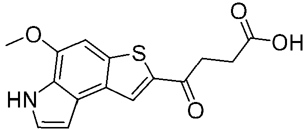 | 60 | 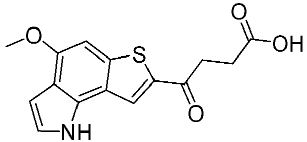 |
| 61 | 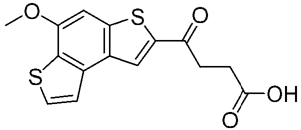 | 62 | 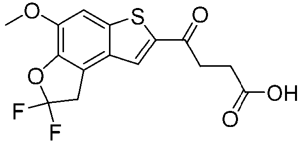 |
| 63 | 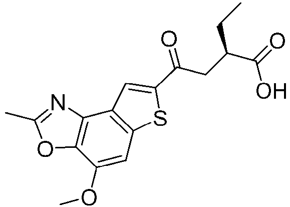 | 64 | 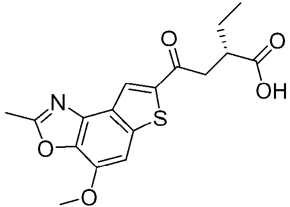 |
| 65 | 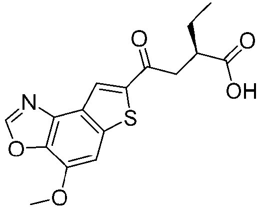 | 66 | 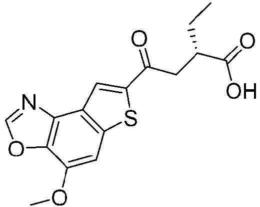 |
| 67 | 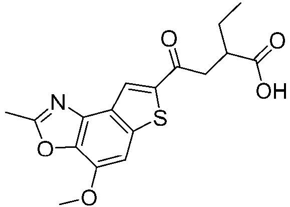 | 68 | 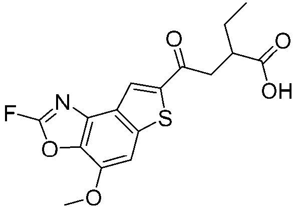 |
| 69 | 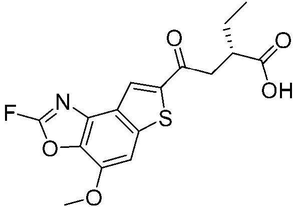 | 70 | 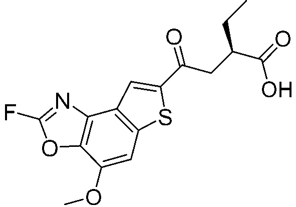 |
| 71 | 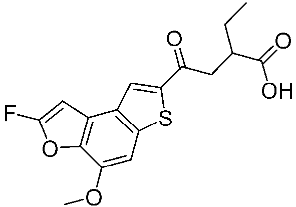 | 72 | 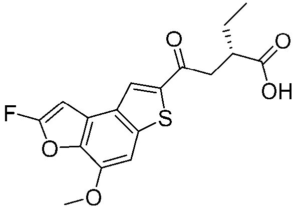 |
| 73 | 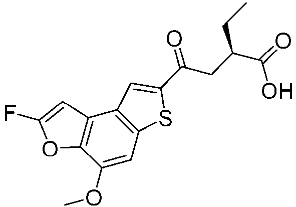 | 74 | 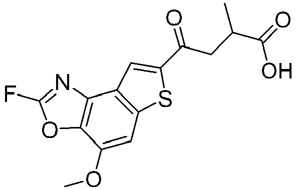 |
| 75 | 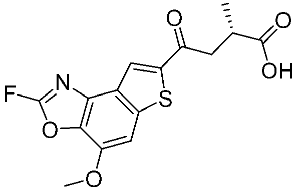 | 76 | 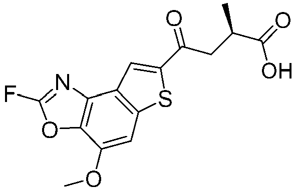 |
| 77 | 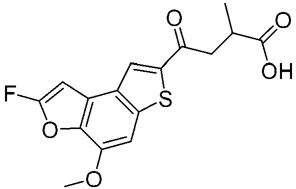 | 78 | 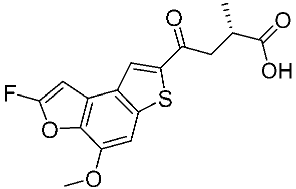 |
| 79 | 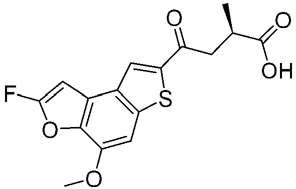 | 80 | 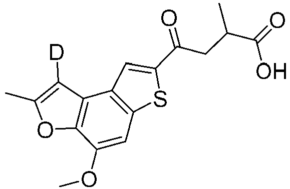 |
| 81 | 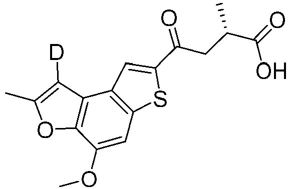 | 82 | 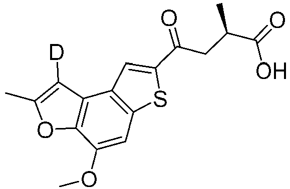 |
| 83 | 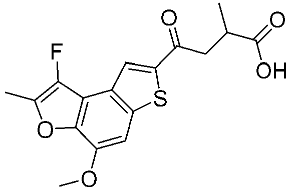 | 84 | 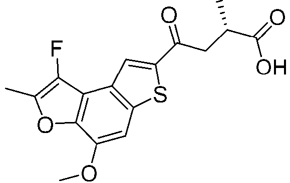 |
| 85 | 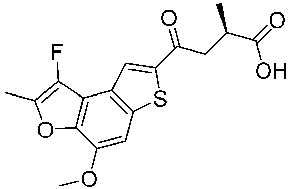 | 86 | 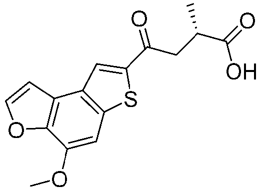 |
| 87 | 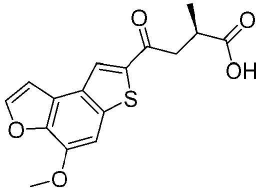 | 88 | 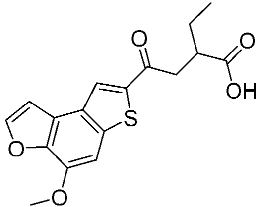 |
| 89 | 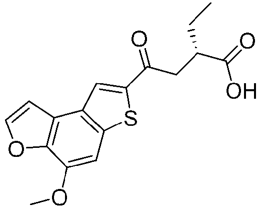 | 90 | 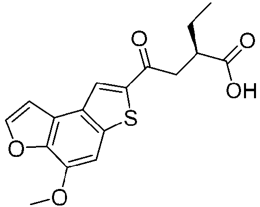 |
| 91 | 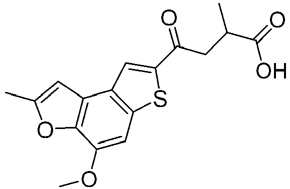 | 92 | 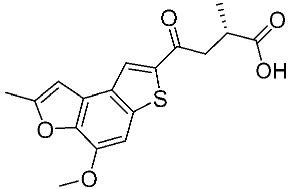 |
| 93 | 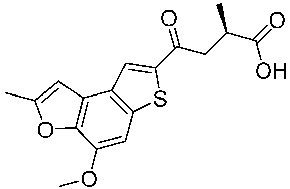 | 94 | 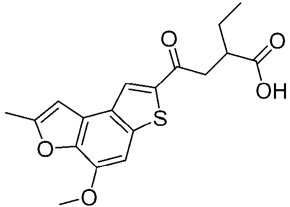 |
| 95 | 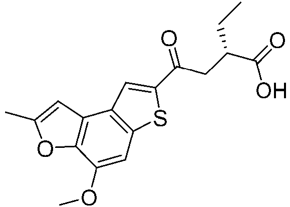 | 96 | 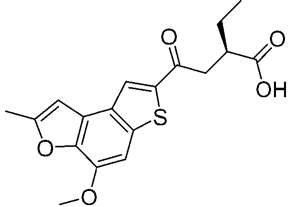 |
| 97 | 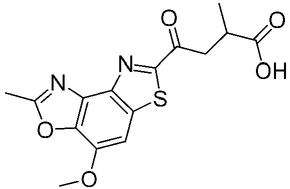 | 98 | 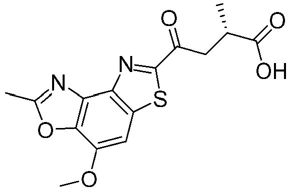 |
| 99 | 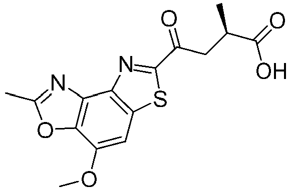 | 100 | 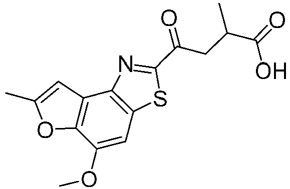 |
| 101 | 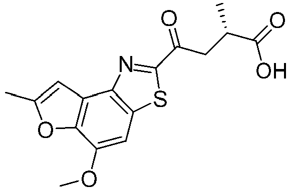 | 102 | 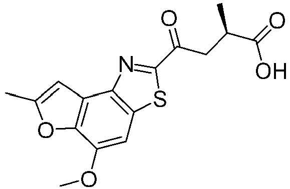 |
| 103 | 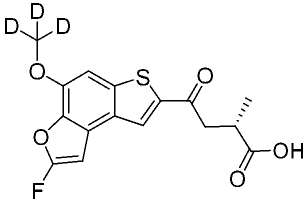 | 104 | 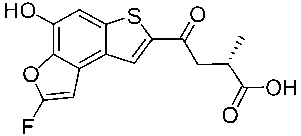 |
